# EUROPEAN PATENT APPLICATION

(11) **EP 3 889 152 A1**
(43) Date of publication of application: **06.10.2021**
(21) Application number: 19890520.0
(22) Date of filing: 29.11.2019
(51) Int. Cl.: C07D 451/14, C07D 451/04, A61K 31/506, A61P 35/00

(54) **HETEROAROMATIC DERIVATIVES FOR USE AS REGULATOR, PREPARATION METHOD THEREFOR AND USE THEREOF**

(30) Priority: 30.11.2018 CN 201811455357; 28.04.2019 CN 201910351595; 26.11.2019 CN 201911175587
(71) Applicant: Jiangsu Hansoh Pharmaceutical Group Co., Ltd., Lianyungang, Jiangsu 222069 (CN); Shanghai Hansoh Biomedical Co., Ltd., Shanghai 201203 (CN)
(72) Inventor: ZENG, Mi, Shanghai 201203 (CN); GAO, Peng, Shanghai 201203 (CN); XU, Peng, Shanghai 201203 (CN); CHENG, Yu, Shanghai 201203 (CN); LI, Jian, Shanghai 201203 (CN); CAI, Jiaqiang, Shanghai 201203 (CN); BAO, Rudi, Shanghai 201203 (CN)
(74) Representative: Hoffmann Eitle
(86) International application number: PCT/CN2019/121944
(87) International publication number: WO 2020/108613

(57) **Abstract**

The present invention relates to heteroaromatic derivatives for use as a regulator, a preparation method therefor and a use thereof. In particular, disclosed are compounds represented by general formula (I), preparation methods therefor, pharmaceutical compositions comprising said compounds, and a use thereof as Janus kinase inhibitors in treating inflammatory diseases and tumor-related diseases.

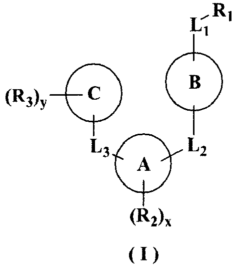

## Description

The present application claims the benefits of Chinese patent application CN201811455357.3 filed on November 30, 2018, Chinese patent application CN201910351595.8 filed on April 28, 2019, and Chinese patent application CN201911175587.9 filed on November 26, 2019. The contents of the aforementioned Chinese patent applications are incorporated herein by reference in their entireties.

### Field of the invention

The present disclosure belongs to the field of pharmaceutical synthesis, and specifically relates to a JAK inhibitor, a preparation method thereof and use thereof.

### Background

Janus kinase (JAK) is an intracellular non-receptor tyrosine kinase that mediates the signal transduction and activation of various cytokines. The JAK kinase family contains four subfamily members, JAK1, JAK2, JAK3 and TYK2, each of which mediates a different type of cytokine signaling pathway. JAK1, JAK2 and TYK2 are expressed in all tissue cells of the human body, and JAK3 is mainly expressed in various hematopoietic tissue cells. The common feature of cytokine receptors is that the receptor itself does not possess kinase activity, but the intracellular segment of the receptor has a binding site for the tyrosine kinase JAK. When the cytokine receptor binds to its ligand, the receptor-coupled JAKs are activated, which in turn phosphorylates the receptor; the phosphorylated tyrosine sites may bind to STAT proteins containing the SH2 domain, thereby recruiting STAT to the receptor and phosphorylating the STAT by JAKs; then phosphotyrosine mediates the dimerization of the STAT, and the activated STAT dimer is transferred to the nucleus and activates the transcription of its target genes, which in turn regulates the growth, activation, differentiation and other functions of various cells.

The JAK/STAT signaling pathway mediates the signal transduction of most intracellular cytokines and plays a key role in the biological processes such as immune regulation and immune cell proliferation, *etc.* The JAK/STAT signaling pathway has a wide range of functions and is involved in many important biological processes such as cell proliferation, differentiation, apoptosis and immune regulation, *etc.,* and is closely associated with various inflammatory diseases such as rheumatoid arthritis, dermatitis, psoriasis, inflammatory bowel disease (ulcerative colitis and Crohn's disease), *etc.;* additionally, the JAK/STAT signaling pathway is closely associated with neoplastic diseases such as myelofibrosis, polycythemia vera and essential thrombocythemia, *etc.;* and mutations in the JAK molecule itself may lead to neoplastic diseases such as acute myeloid leukemia (AML), acute lymphocytic leukemia (ALL), breast ductal carcinoma and non-small cell lung cancer (NSCLC).

Inflammatory bowel disease is a chronic intestinal inflammatory disease, including ulcerative colitis (UC) and Crohn's disease (CD). The main drugs used for the treatment of inflammatory bowel disease are aminosalicylic acid, glucocorticoids, immunosuppressants, and antibiotics, *etc.* The main principle of UC treatment is to modulate the immune response and suppress inflammation. At present, in clinical practice, sulfasalazine is mainly used for the treatment of mild to moderate UC. Glucocorticoids are commonly used for moderate to severe UC, but are not used as a long-term treatment because the risks outweigh the benefits. Monoclonal antibodies have problems such as high drug costs, the production of anti-drug antibodies that compromise the safety and efficacy of the drugs, and the lack of convenience of intravenous administration. Therefore, there is still a significant unmet medical need in this area. Many treated patients have not achieved remission, and up to 80% of Crohn's patients and 30% of UC patients eventually need surgery.

Tofacitinib (Xeljanz) is the first oral JAK inhibitor for the treatment of adult patients with moderate to severe active UC, with significant inhibitory activities against JAK subtypes 1, 2, and 3, which increases the efficacy of tofacitinib, but also brings more serious side effects. Adverse effects include infections, tuberculosis, tumors, anemia, liver damage, and cholesterol increase. The instruction of tofacitinib lists a number of black box warnings: severe infections (tuberculosis, bacterial, fungal, viral) and malignancies (lymphoma, *etc.*). Because of the wide range of functions mediated by individual JAKs, these side effects are caused by the drug's simultaneous inhibition of multiple JAKs. Because of the extensive involvement of JAKs in the regulation of immune cells, JAK inhibitors inevitably cause immunosuppression-related side effects such as severe infections and even tumorigenesis, *etc.* Even with many highly selective inhibitors currently in development, such side effects caused by target inhibition are inevitable.

In view of the good efficacy of JAK inhibitors and the serious side effects related to multiple targets, the development of a safer JAK inhibitor drug is an urgent issue to be solved. Since inflammatory bowel disease occurs on the luminal surface of the gastrointestinal tract and does not require drugs to enter the blood system to be effective, developing a drug that reduces systemic exposure in the blood circulation while increasing local exposure at the site of inflammation is a good strategy to improve safety. International application WO2016191524A1 reports the synthesis of a series of compounds by Theravance company with very low systemic exposure and enrichment at the inflammatory site of the intestine, which may effectively treat intestinal inflammation without causing serious side effects, suggesting that this strategy is highly feasible and may have significant clinical application value.

### Content of the invention

The technical problem to be solved by the present disclosure is to overcome the shortcomings of existing JAK inhibitors, which have serious side effects while exerting therapeutic effects, thereby providing a heteroaromatic derivative modulator, a preparation method thereof and a use thereof herein. The heteroaromatic derivative of the present disclosure has a good inhibitory effect on JAK kinase, and can significantly increase the local exposure at the treatment site with good targeting.

The present disclosure solves the above technical problem by the following technical solutions.

A compound of general formula (I), a stereoisomer thereof or a pharmaceutically acceptable salt thereof: 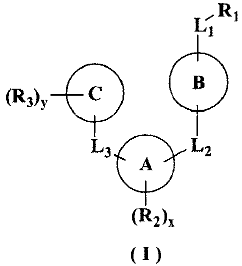 wherein:
L₁ is selected from bond, alkylene, alkenylene, alkynyl, cycloalkyl, heterocyclyl,-(CH₂)ₙ₁C(O)(CH₂)ₘ₁-, -(CH₂)ₙ₁C(O)(CH₂)ₘ₁NRₐₐ(CH₂)ₘ₂-, -NRₐₐ(CH₂)ₙ₁-, -(CH₂)ₙ₁S(O)ₘ₁-, or -(CH₂)ₙ₁S(O)ₘ₁NRₐₐ-;
L₂ is selected from bond, oxygen atom, sulfur atom, -CRₐₐR_{bb}-, -(CH₂)ₙ₁C(O)-, -NR₄-, or -(CH₂)ₙ₁S(O)ₘ₁-;
L₃ is selected from bond, -NRₐₐ-, or -C(O)NRₐₐ-;
ring A is 6-14 membered heteroaryl, wherein the 6-14 membered heteroaryl is selected from 6-14 membered fused heteroaryl; preferably 5,5 fused heteroaryl, 5,6 fused heteroaryl or 6,6 fused heteroaryl;
ring B is selected from 3-10 membered monocyclic heterocyclyl, 6-14 membered bridged heterocyclyl, 6-14 membered fused heterocyclyl or 6-14 membered spiro heterocyclyl;
ring C is heteroaryl;
R₁ is selected from hydrogen atom, deuterium atom, oxo, alkyl, deuterated alkyl, haloalkyl, alkoxy, haloalkoxy, hydroxyalkyl, halogen, amino, nitro, hydroxyl, cyano, alkenyl, alkynyl, cycloalkyl, heterocyclyl, oxoheterocyclyl, thioxoheterocyclyl, aryl, heteroaryl,-(CH₂)ₙ₁Rₐₐ, -(CH₂)ₙ₁ORₐₐ, -NRₐₐC(O)(CH₂)ₙ₁ORₐₐ, -NRₐₐC(=S)(CH₂)ₙ₁OR_{bb}, -(CH₂)ₙ₁SRₐₐ,-(CH₂)ₙ₁C(O)Rₐₐ, -(CH₂)ₙC(O)ORₐₐ, -(CH₂)ₙ₁S(O)ₘ₁Rₐₐ, -(CH₂)ₙ₁NRₐₐR_{bb},-(CH₂)ₙ₁C(O)NRₐₐR_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, alkyl, haloalkyl, halogen, amino, oxo, nitro, cyano, hydroxyl, alkenyl, alkynyl, alkoxy, haloalkoxy, hydroxyalkyl, cycloalkyl, heterocyclyl, aryl, heteroaryl,-(CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc},-(CH₂)ₙ₁S(O)ₘ₁R_{cc}, -(CH₂)ₙ]NR_{cc}R_{dd}, -(CH₂)ₙ₁C(O)NR_{cc}R_{dd}, -(CH₂)ₙ₁NR_{cc}C(O)R_{dd} or-(CH₂)ₙ₁NR_{cc}S(O)ₘ₁R_{dd};
R₂ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl, heteroaryl, -(CH₂)ₙ₁Rₐₐ,-(CH₂)ₙ₁O(CH₂)ₘ₁Rₐₐ, -(CH₂)ₙ₁ORₐₐ, -(CH₂)ₙ₁NRₐₐ(CH₂)ₘ₁Rₐₐ, -NRₐₐC(O)(CH₂)ₙ₁ORₐₐ,-NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁S(O)ₘ₁NRₐₐR_{bb}, -(CH₂)ₙ₁P(O)ₘ₂RₐₐR_{bb}, -(CH₂)ₙ₁NRₐₐR_{bb}, -(CH₂)ₙ₁S-,-(CH₂)ₙ₁C(O)NRₐₐR_{bb}, -(CH₂)ₙ₁NRₐₐC(O)R_{bb}, or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted or unsubstituted heteroaryl,-(CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc},-(CH₂)ₙ₁S(O)ₘ₁R_{cc}, -(CH₂)ₙ₁S(O)ₘ₁NR_{cc}R_{dd}, -(CH₂)ₙ₁NR_{cc}R_{dd}, -(CH₂)ₙ₁C(O)NR_{cc}R_{dd},-(CH₂)ₙ₁C(O)NR_{cc}S(O)ₘ₁R_{dd}, -(CH₂)ₙ₁NR_{cc}S(O)ₘ₁R_{dd} or -(CH₂)ₙ₁NR_{cc}S(O)ₘ₁R_{dd};
R₃ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl, heteroaryl, -(CH₂)ₙ₁Rₐₐ,-(CH₂)ₙ₁ORₐₐ, -NRₐₐC(O)(CH₂)ₙ₁ORₐₐ, -NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁S(O)ₘ₁NRₐₐR_{bb}, -(CH₂)ₙ₁P(O)ₘ₂RₐₐR_{bb},-(CH₂)ₙ₁NRₐₐR_{bb}, -(CH₂)ₙ₁C(O)NRₐₐR_{bb}, -(CH₂)ₙ₁NRₐₐC(O)R_{bb}, or -(CH₂)ₙ₁NRₐₐS(O)ₘ₁R_{bb}, wherein the alkyl, haloalkyl; alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted or unsubstituted heteroaryl, -(CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc},-(CH₂)ₙ₁C(O)OR_{cc}, -(CH₂)ₙ₁S(O)ₘ₁R_{cc}, -(CH₂)ₙ₁S(O)ₘ₁NR_{cc}R_{dd}, -(CH₂)ₙ₁NR_{cc}R_{dd},-(CH₂)ₙ₁C(O)NR_{cc}R_{dd}, -(CH₂)ₙ₁C(O)NR_{cc}S(O)ₘ₁R_{dd}, -(CH₂)ₙ₁NR_{cc}S(O)ₘ₁R_{dd}, or-(CH₂)ₙ₁NR_{cc}S(O)ₘ₁R_{dd};
R₄ is selected from hydrogen atom, deuterium atom, alkyl, deuterated alkyl, haloalkyl, alkoxy, hydroxyalkyl, haloalkoxy, halogen, amino, nitro, hydroxyl, cyano, alkenyl or alkynyl;
Rₐₐ, R_{bb}, R_{cc}, and R_{dd} are identical or different and are each independently selected from hydrogen atom, deuterium atom, alkyl, deuterated alkyl, haloalkyl, alkoxy, hydroxyalkyl, haloalkoxy, halogen, cyano, nitro, hydroxyl, amino, alkenyl, alkynyl, cycloalkyl, heterocyclyl, aryl and heteroaryl, wherein the alkyl, deuterated alkyl, haloalkyl, alkoxy, hydroxyalkyl, haloalkoxy, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl, or substituted or unsubstituted heteroaryl;
x is an integer of 0, 1, 2 or 3;
y is an integer of 0, 1, 2, 3, 4 or 5;
m₁ is an integer of 0, 1 or 2;
m₂ is an integer of 0, 1 or 2; and
n₁ is an integer of 0, 1, 2, 3, 4 or 5.

In some technical solutions of the present disclosure, in the compound of general formula (I), certain groups are as defined below (unspecified groups are as defined above):
L₁ is selected from bond, alkylene, cycloalkyl, heterocyclyl, -(CH₂)ₙ₁C(O)(CH₂)ₘ₁-,-(CH₂)ₙ₁C(O)(CH₂)ₘ₁NRₐₐ(CH₂)ₘ₂-, -NRₐₐ(CH₂)ₙ₁-, -(CH₂)ₙ₁S(O)ₘ₁-, or -(CH₂)ₙ₁S(O)ₘ₁NRₐₐ-;
L₂ is selected from bond, oxygen atom, -CRₐₐR_{bb}-, -(CH₂)ₙ₁C(O)-, -NR4-, or-(CH₂)ₙ₁S(O)ₘ₁-;
R₄ is selected from hydrogen atom, or alkyl;
L₃ is selected from bond, -NRₐₐ-, or -C(O)NRₐₐ-;
ring A is 6-14 membered fused heteroaryl;
ring B is selected from 3-10 membered monocyclic heterocyclyl, 6-14 membered bridged heterocyclyl, 6-14 membered fused heterocyclyl or 6-14 membered spiro heterocyclyl;
ring C is heteroaryl;
R₁ is selected from hydrogen atom, alkyl, haloalkyl, alkoxy, halogen, amino, hydroxyl, cyano, cycloalkyl, heterocyclyl, aryl, or heteroaryl; wherein the alkyl, amino, heterocyclyl, aryl, or heteroaryl is optionally further substituted with one or more substituents selected from halogen, cyano, alkyl, or alkoxy;
R₂ is selected from hydrogen atom, alkyl, alkoxy, halogen, amino, hydroxyl, cyano, cycloalkyl, heterocyclyl, aryl, heteroaryl, -(CH₂)ₙ₁C(O)R_{cc}, -(CH₂)ₙ₁SRₐₐ, or -(CH₂)ₙ₁NRₐₐR_{bb}; wherein the alkyl, alkoxy, amino, cycloalkyl, heterocyclyl, aryl, or heteroaryl is optionally further substituted with one or more substituents selected from substituted or unsubstituted alkyl, unsubstituted alkoxy, unsubstituted cycloalkyl, substituted or unsubstituted amino, substituted or unsubstituted heterocyclyl, or unsubstituted heteroaryl; wherein "substituted" refers to be substituted with alkyl, or halogen;
R_{cc} is heterocyclyl; the heterocyclyl is optionally substituted with unsubstituted alkyl;
R₃ is selected from hydrogen atom, alkyl, haloalkyl, hydroxyalkyl, alkoxy, halogen, hydroxyl, cyano, cycloalkyl, heterocyclyl, -(CH₂)ₙ₁C(O)Rₐₐ, -(CH₂)ₙ₁C(O)NRₐₐR_{bb},-(CH₂)ₙ₁S(O)ₘ₁Rₐₐ, or -(CH₂)ₙ₁S(O)ₘ₁NRₐₐR_{bb}; wherein the alkyl, alkoxy, cycloalkyl, or heterocyclyl is optionally further substituted with one or more substituents selected from halogen, hydroxyl, unsubstituted alkyl, or unsubstituted alkoxy;
Rₐₐ and R_{bb} are identical or different and are each independently selected from hydrogen atom, alkyl, amino, or heteroaryl; wherein the alkyl, amino are optionally further substituted with one or more substituents selected from unsubstituted alkyl, substituted or unsubstituted heterocyclyl, unsubstituted heteroaryl; wherein "substituted" refers to be substituted with alkyl, or halogen;
x is 0, 1, 2 or 3;
y is 0, 1, 2 or 3;
m₁ is 0, 1 or 2;
m₂ is 0, 1 or 2; and
n₁ is 0, 1 or 2.
   In some technical solutions of the present disclosure, in the compound of general formula (I), certain groups are as defined below (unspecified groups are as defined above):
L₁ is selected from bond, alkylene, -(CH₂)ₙ₁C(O)(CH₂)ₘ₁-,-(CH₂)ₙ₁C(O)(CH₂)ₘ₁NRₐₐ(CH₂)ₘ₂-, or -(CH₂)ₙ₁S(O)ₘ₁-;
L₂ is selected from bond, oxygen atom, or -NR₄-;
R₄ is selected from hydrogen atom, or alkyl;
L₃ is -NRₐₐ-;
ring A is 6-14 membered fused heteroaryl;
ring B is selected from 3-10 membered monocyclic heterocyclyl, 6-14 membered bridged heterocyclyl, or 6-14 membered fused heterocyclyl;
ring C is heteroaryl;
R₁ is selected from hydrogen atom, alkyl, haloalkyl, amino, heterocyclyl, or heteroaryl; wherein the alkyl, amino, heterocyclyl, or heteroaryl is optionally further substituted with one or more substituents selected from halogen, cyano, alkyl, or alkoxy;
R₂ is selected from hydrogen atom, alkyl, alkoxy, halogen, cycloalkyl, heterocyclyl, or heteroaryl; wherein the heteroaryl is optionally further substituted with one or more substituents selected from unsubstituted alkyl, unsubstituted alkoxy, or unsubstituted cycloalkyl;
R₃ is selected from hydrogen atom, alkyl, hydroxyalkyl, halogen, cyano, cycloalkyl, - (CH₂)ₙ₁C(O)Rₐₐ, or -(CH₂)ₙ₁C(O)NRₐₐR_{bb}; wherein the alkyl is optionally further substituted with hydroxyl;
Rₐₐ and R_{bb} are identical or different and are each independently selected from hydrogen atom, alkyl, or amino;
x is 0, 1, 2 or 3;
y is 0, 1, 2 or 3;
m₁ is 0, 1 or 2;
m₂ is 0, 1 or 2; and
n₁ is 0, 1 or 2.

In some technical solutions of the present disclosure, in the compound of general formula (I), certain group is as defined below (unspecified groups are as defined above): L₁ is selected from bond, alkylene, -(CH₂)ₙ₁C(O)(CH₂)ₘ₁-, -(CH₂)ₙ₁C(O)(CH₂)ₘ₁NRₐₐ(CH₂)ₘ₂-, or-(CH₂)ₙ₁S(O)ₘ₁-.

In some technical solutions of the present disclosure, in the compound of general formula (I), certain group is as defined below (unspecified groups are as defined above): L₂ is selected from bond, oxygen atom, or -NR₄-.

In some technical solutions of the present disclosure, in the compound of general formula (I), certain group is as defined below (unspecified groups are as defined above): R₄ is selected from hydrogen atom, or alkyl.

In some technical solutions of the present disclosure, in the compound of general formula (I), certain group is as defined below (unspecified groups are as defined above): L₃ is - NRₐₐ-.

In some technical solutions of the present disclosure, in the compound of general formula (I), certain group is as defined below (unspecified groups are as defined above): ring A is 6-14 membered fused heteroaryl.

In some technical solutions of the present disclosure, in the compound of general formula (I), certain group is as defined below (unspecified groups are as defined above): ring B is selected from 3-10 membered monocyclic heterocyclyl, 6-14 membered bridged heterocyclyl, or 6-14 membered fused heterocyclyl.

In some technical solutions of the present disclosure, in the compound of general formula (I), certain group is as defined below (unspecified groups are as defined above): ring C is heteroaryl.

In some technical solutions of the present disclosure, in the compound of general formula (I), certain group is as defined below (unspecified groups are as defined above): R₁ is selected from hydrogen atom, alkyl, haloalkyl, amino, heterocyclyl, or heteroaryl; wherein the alkyl, amino, heterocyclyl, or heteroaryl is optionally further substituted with one or more substituents selected from halogen, cyano, alkyl, or alkoxy.

In some technical solutions of the present disclosure, in the compound of general formula (I), certain group is as defined below (unspecified groups are as defined above): R₂ is selected from hydrogen atom, alkyl, alkoxy, halogen, cycloalkyl, heterocyclyl, or heteroaryl; wherein the heteroaryl is optionally further substituted with one or more substituents selected from unsubstituted alkyl, unsubstituted alkoxy, or unsubstituted cycloalkyl.

In some technical solutions of the present disclosure, in the compound of general formula (I), certain group is as defined below (unspecified groups are as defined above): R₃ is selected from hydrogen atom, alkyl, hydroxyalkyl, halogen, cyano, cycloalkyl, -(CH₂)ₙ₁C(O)Rₐₐ, or -(CH₂)ₙ₁C(O)NRₐₐR_{bb}; wherein the alkyl is optionally further substituted with hydroxyl.

In some technical solutions of the present disclosure, in the compound of general formula (I), certain groups are as defined below (unspecified groups are as defined above): Rₐₐ and R_{bb} are identical or different and are each independently selected from hydrogen atom, alkyl, or amino.

In some technical solutions of the present disclosure, in the compound of general formula (I), certain group is as defined below (unspecified groups are as defined above): x is 0, 1, 2 or 3.

In some technical solutions of the present disclosure, in the compound of general formula (I), certain group is as defined below (unspecified groups are as defined above): y is 0, 1, 2 or 3.

In some technical solutions of the present disclosure, in the compound of general formula (I), certain group is as defined below (unspecified groups are as defined above): m₁ is 0, 1 or 2.

In some technical solutions of the present disclosure, in the compound of general formula (I), certain group is as defined below (unspecified groups are as defined above): m₂ is 0, 1 or 2.

In some technical solutions of the present disclosure, in the compound of general formula (I), certain group is as defined below (unspecified groups are as defined above): n₁ is 0, 1 or 2.

In some technical solutions of the present disclosure, in the compound of general formula (I), certain group is as defined below (unspecified groups are as defined above): when L₁ is alkylene, the alkylene is preferably C₁-C₄ alkylene, e.g., -CH₂-, -(CH₂)₂-, -(CH₂)₃- or-(CH₂)₄-, more preferably -CH₂-, or -(CH₂)₂-.

In some technical solutions of the present disclosure, in the compound of general formula (I), certain group is as defined below (unspecified groups are as defined above): when L₁ is cycloalkyl, the cycloalkyl is preferably C₃-C₈ cycloalkyl, more preferably C₃-C₆ cycloalkyl, *e.g.,*

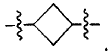

In some technical solutions of the present disclosure, in the compound of general formula (I), certain group is as defined below (unspecified groups are as defined above): when L₁ is heterocyclyl, the heterocyclyl is preferably 3-8 membered monocyclic heterocyclyl with "heteroatom selected from one or more of N, O and S, and the number of the heteroatom being 1-4", more preferably 4-6 membered monocyclic heterocyclyl with "heteroatom selected from one or more of N, O and S, and the number of the heteroatom being 1-3", *e.g.,*

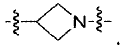

In some technical solutions of the present disclosure, in the compound of general formula (I), certain group is as defined below (unspecified groups are as defined above): when L₁ is -(CH₂)ₙ₁C(O)(CH₂)ₘ₁-, the -(CH₂)ₙ₁C(O)(CH₂)ₘ₁- is preferably -C(O)-, -C(O)CH₂-, or-CH₂C(O)-.

In some technical solutions of the present disclosure, in the compound of general formula (I), certain group is as defined below (unspecified groups are as defined above): when L₁ is -(CH₂)ₙ₁C(O)(CH₂)ₘ₁NRₐₐ(CH₂)ₘ₂-, the -(CH₂)ₙ₁C(O)(CH₂)ₘ₁NRₐₐ(CH₂)ₘ₂- is preferably-C(O)CH₂NH-, -C(O)CH₂N(CH₃)-, -C(O)CH₂NHCH₂-, -C(O)CH₂NH(CH₂)₂-, or-CH₂C(O)N(CH₃)-.

In some technical solutions of the present disclosure, in the compound of general formula (I), certain group is as defined below (unspecified groups are as defined above): when L₁ is -NRₐₐ(CH₂)ₙ₁-, the -NRₐₐ(CH₂)ₙ₁- is preferably -NH(CH2)2-.

In some technical solutions of the present disclosure, in the compound of general formula (I), certain group is as defined below (unspecified groups are as defined above): when L₁ is -(CH₂)ₙ₁S(O)ₘ₁-, the -(CH₂)ₙ₁S(O)ₘ₁- is preferably -S(O)₂-.

In some technical solutions of the present disclosure, in the compound of general formula (I), certain group is as defined below (unspecified groups are as defined above): when L₁ is -(CH₂)ₙ₁S(O)ₘ₁NRₐₐ-, the -(CH₂)ₙ₁S(O)ₘ₁NRₐₐ- is preferably -S(O)₂NH-.

In some technical solutions of the present disclosure, in the compound of general formula (I), certain group is as defined below (unspecified groups are as defined above): the left end of L₁ is connected with ring B, and the right end of L₁ is connected with R₁.

In some technical solutions of the present disclosure, in the compound of general formula (I), certain group is as defined below (unspecified groups are as defined above): when L₂ is -CRₐₐR_{bb}-, the -CRₐₐR_{bb}- is preferably -CH₂- or -CH(OH)-.

In some technical solutions of the present disclosure, in the compound of general formula (I), certain group is as defined below (unspecified groups are as defined above): when L₂ is -(CH₂)ₙ₁C(O)-, the -(CH₂)ₙ₁C(O)- is preferably -C(O)-.

In some technical solutions of the present disclosure, in the compound of general formula (I), certain group is as defined below (unspecified groups are as defined above): when L₂ is -NR₄-, the -NR₄- is preferably -NH- or -N(CH₃)-.

In some technical solutions of the present disclosure, in the compound of general formula (I), certain group is as defined below (unspecified groups are as defined above): when L₂ is -(CH₂)ₙ₁S(O)ₘ₁-, the -(CH₂)ₙ₁S(O)ₘ₁- is preferably -S(O)₂-.

In some technical solutions of the present disclosure, in the compound of general formula (I), certain group is as defined below (unspecified groups are as defined above): the left end of L₂ is connected with ring A, and the right end of L₂ is connected with ring B.

In some technical solutions of the present disclosure, in the compound of general formula (I), certain group is as defined below (unspecified groups are as defined above): when R₄ is alkyl, the alkyl is preferably C₁-C₈ alkyl, more preferably C₁-C₆ alkyl, further preferably C₁-C₃ alkyl, e.g., methyl, ethyl, propyl or isopropyl, further preferably methyl.

In some technical solutions of the present disclosure, in the compound of general formula (I), certain group is as defined below (unspecified groups are as defined above): when L₃ is -NRₐₐ-, the -NRaa- is preferably -NH-.

In some technical solutions of the present disclosure, in the compound of general formula (I), certain group is as defined below (unspecified groups are as defined above): when L₃ is -C(O)NRₐₐ-, the -C(O)NRₐₐ- is preferably -C(O)NH-.

In some technical solutions of the present disclosure, in the compound of general formula (I), certain group is as defined below (unspecified groups are as defined above): the left end of L₃ is connected with ring C, and the right end of L₃ is connected with ring B.

In some technical solutions of the present disclosure, in the compound of general formula (I), certain group is as defined below (unspecified groups are as defined above): when the ring A is 6-14 membered fused heteroaryl, the 6-14 membered fused heteroaryl is preferably 6-14 membered fused heteroaryl with "heteroatom selected from one or more of N, O and S, and the number of the heteroatom being 1-4", more preferably 5,6 fused heteroaryl with" heteroatom selected from one or more of N, O and S, and the number of the heteroatom being 1-4", or 6,6 fused heteroaryl with "heteroatom selected from one or more of N, O and S, and the number of the heteroatom being 1-4". The 5,6 fused heteroaryl is preferably thienopyrimidinyl or pyrazolopyrimidinyl, benzopyrrolyl, pyrrolopyridyl, imidazolopyridyl, triazolopyridyl, imidazolopyridazinyl, pyrrolopyrimidinyl, pyrazolopyrimidinyl, imidazolopyrimidinyl, triazolopyrimidinyl, thienopyrimidinyl, thiazolopyrimidinyl, pyrrolopyrazinyl, pyrazolopyrazinyl, imidazolopyrazinyl, triazolopyrazinyl, pyrrolotriazinyl, imidazolotriazinyl or imidazolopyrazinonyl, more preferably 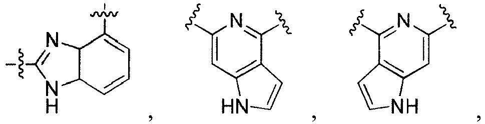 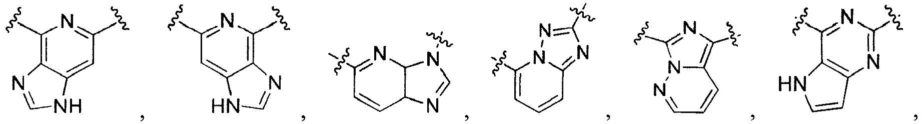 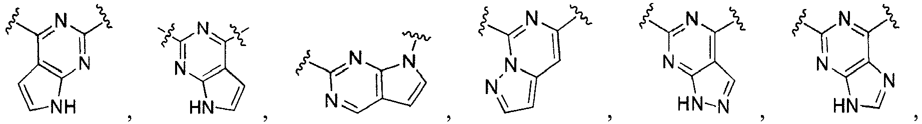 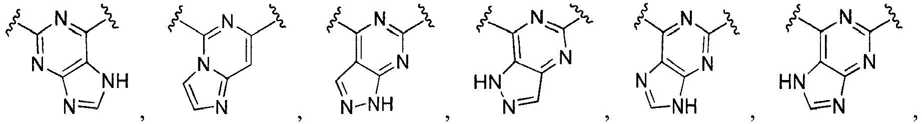 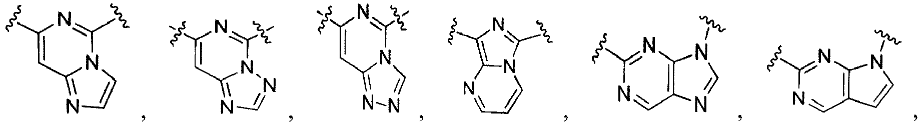 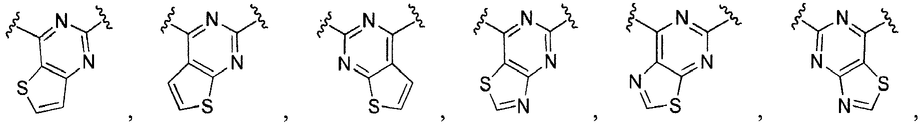 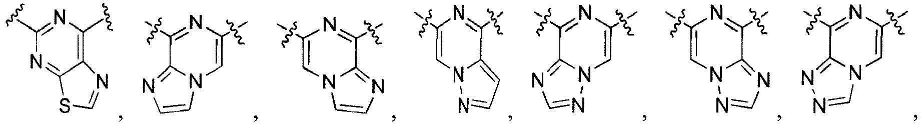 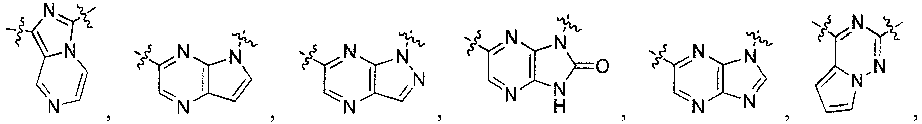 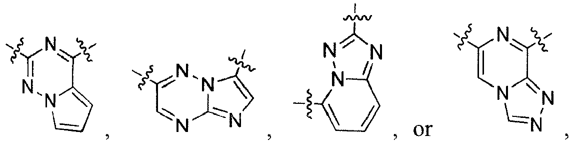 further preferably 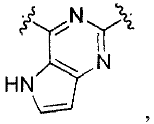 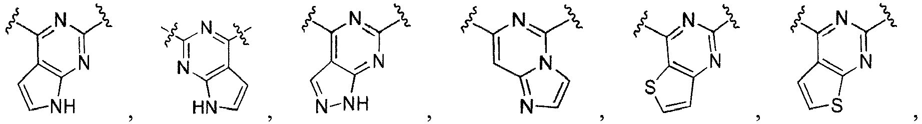 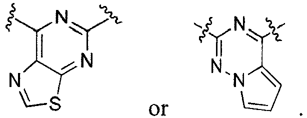 The 6,6 fused heteroaryl is preferably isoquinolinyl, naphthyridinyl, pyridopyrazinyl, pyridopyrimidinyl, quinazolinyl, pteridinyl, quinoxalinyl, dihydropyranopyrimidinyl, dihydrodioxinopyrimidinyl, 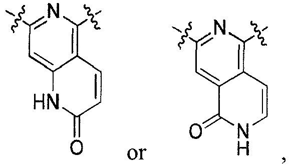 more preferably 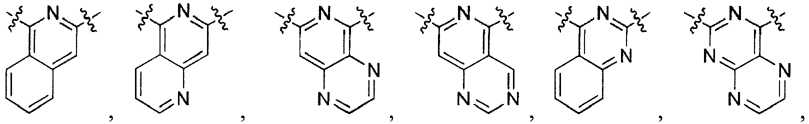 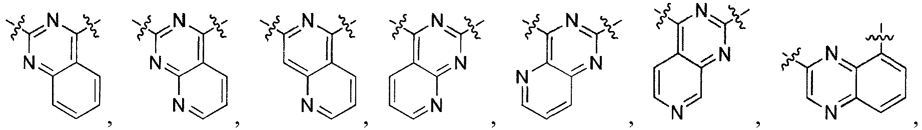 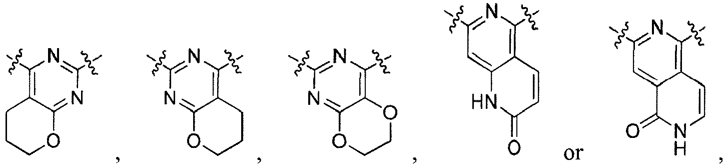 further preferably 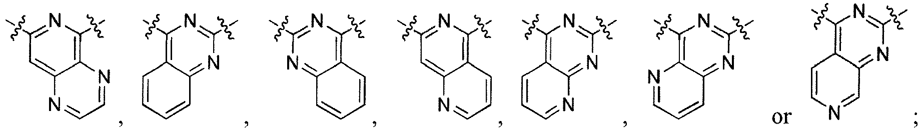 the left end of ring A is connected with L₃, and the right end of ring A is connected with L₂.

In some technical solutions of the present disclosure, in the compound of general formula (I), certain group is as defined below (unspecified groups are as defined above): when the ring B is 3-10 membered monocyclic heterocyclyl, the 3-10 membered monocyclic heterocyclyl is preferably 3-8 membered monocyclic heterocyclyl with "heteroatom selected from one or more of N, O and S, and the number of the heteroatom being 1-4", more preferably 4-6 membered monocyclic heterocyclyl with "heteroatom selected from one or more of N, O and S, and the number of the heteroatom being 1-3", *e.g.,*

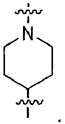

In some technical solutions of the present disclosure, in the compound of general formula (I), certain group is as defined below (unspecified groups are as defined above): when the ring B is 6-14 membered bridged heterocyclyl, the 6-14 membered bridged heterocyclyl is preferably 6-14 membered bridged heterocyclyl with "heteroatom selected from one or more of N, O and S, and the number of the heteroatom being 1-4", more preferably 7-10 membered bridged heterocyclyl with "heteroatom selected from one or more of N, O and S, and the number of the heteroatom being 1-3", *e.g.,*

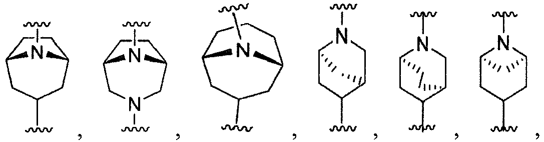

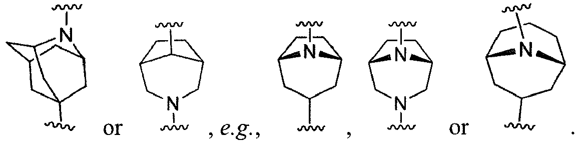

In some technical solutions of the present disclosure, in the compound of general formula (I), certain group is as defined below (unspecified groups are as defined above): when the ring B is 6-14 membered fused heterocyclyl, the 6-14 membered fused heterocyclyl is preferably 6-14 membered fused heterocyclyl with "heteroatom selected from one or more of N, O and S, and the number of the heteroatom being 1-4", more preferably 7-10 membered fused heterocyclyl with "heteroatom selected from one or more of N, O and S, and the number of the heteroatom being 1-3", *e.g.,*

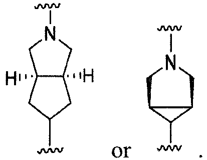

In some technical solutions of the present disclosure, in the compound of general formula (I), certain group is as defined below (unspecified groups are as defined above): when the ring B is 6-14 membered spiro heterocyclyl, the 6-14 membered spiro heterocyclyl is preferably 6-14 membered spiro heterocyclyl with "heteroatom selected from one or more of N, O and S, and the number of the heteroatom being 1-4", more preferably 7-10 membered spiro heterocyclyl with "heteroatom selected from one or more of N, O and S, and the number of the heteroatom being 1-3", *e.g.,*

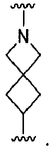

In some technical solutions of the present disclosure, in the compound of general formula (I), certain group is as defined below (unspecified groups are as defined above): the upper end of ring B is connected with L₁, and the lower end of ring B is connected with L₂.

In some technical solutions of the present disclosure, in the compound of general formula (I), certain group is as defined below (unspecified groups are as defined above): when the ring C is heteroaryl, the heteroaryl is preferably 6-14 membered heteroaryl with "heteroatom selected from one or more of N, O and S, and the number of the heteroatom being 1-4", more preferably 5-6 membered monocyclic heteroaryl with "heteroatom selected from one or more of N, O and S, and the number of the heteroatom being 1-3", or 8-10 membered fused heteroaryl with "heteroatom selected from one or more of N, O and S, and the number of the heteroatom being 1-3". The 5-6 membered monocyclic heteroaryl is preferably pyrazolyl, imidazolyl, thiazolyl, triazolyl, pyridazinyl, pyrimidinyl or pyrazinyl, e.g., 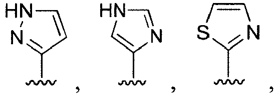 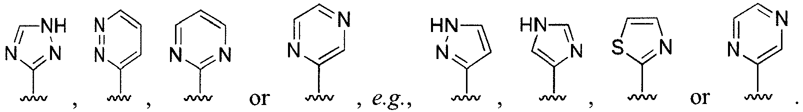 The 8-10 membered fused heteroaryl is preferably indazolyl, or pyrazolopyridyl, e.g., 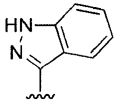 or

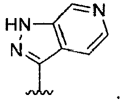

In some technical solutions of the present disclosure, in the compound of general formula (I), certain group is as defined below (unspecified groups are as defined above): when R₁ is alkyl, the alkyl is preferably C₁-C₈ alkyl, more preferably C₁-C₆ alkyl, further preferably C₁-C₃ alkyl, e.g., methyl, ethyl, propyl or isopropyl, further preferably methyl or ethyl.

In some technical solutions of the present disclosure, in the compound of general formula (I), certain group is as defined below (unspecified groups are as defined above): when R₁ is haloalkyl, the haloalkyl is preferably C₁-C₈ haloalkyl, more preferably C₁-C₆ haloalkyl, further preferably C₁-C₃ haloalkyl, e.g., -CHF₂ or CF₃.

In some technical solutions of the present disclosure, in the compound of general formula (I), certain group is as defined below (unspecified groups are as defined above): when R₁ is alkoxy, the alkoxy is preferably C₁-C₈ alkoxy, more preferably C₁-C₆ alkoxy, further preferably C₁-C₃ alkoxy, *e*.*g*., methoxy, ethoxy, propoxy or isopropoxy, further preferably methoxy or ethoxy.

In some technical solutions of the present disclosure, in the compound of general formula (I), certain group is as defined below (unspecified groups are as defined above): when R₁ is halogen, the halogen is preferably fluorine.

In some technical solutions of the present disclosure, in the compound of general formula (I), certain group is as defined below (unspecified groups are as defined above): when R₁ is cycloalkyl, the cycloalkyl is preferably C₃-C₈ cycloalkyl, more preferably C₃-C₆ cycloalkyl, *e*.*g*.,

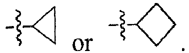

In some technical solutions of the present disclosure, in the compound of general formula (I), certain group is as defined below (unspecified groups are as defined above): when R₁ is heterocyclyl, the heterocyclyl is preferably 3-8 membered monocyclic heterocyclyl with "heteroatom selected from one or more of N, O and S, and the number of the heteroatom being 1-4", more preferably 4-6 membered monocyclic heterocyclyl with "heteroatom selected from one or more of N, O and S, and the number of the heteroatom being 1-3", *e.g.,* 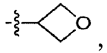 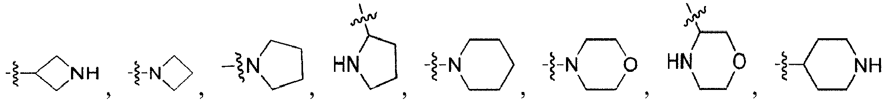 or

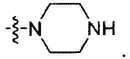

In some technical solutions of the present disclosure, in the compound of general formula (I), certain group is as defined below (unspecified groups are as defined above): when R₁ is aryl, the aryl is preferably 6-10 membered aryl, more preferably phenyl.

In some technical solutions of the present disclosure, in the compound of general formula (I), certain group is as defined below (unspecified groups are as defined above): when R₁ is heteroaryl, the heteroaryl is preferably 5-10 membered heteroaryl with "heteroatom selected from one or more of N, O and S, and the number of the heteroatom being 1-4", more preferably 5-6 membered heteroaryl with "heteroatom selected from one or more of N, O and S, and the number of the heteroatom being 1-3", *e.g.,*

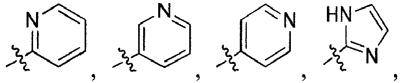

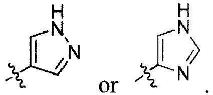

In some technical solutions of the present disclosure, in the compound of general formula (I), certain group is as defined below (unspecified groups are as defined above): when R₁ is alkyl, amino, heterocyclyl, aryl or heteroaryl, and the alkyl, amino, heterocyclyl, aryl or heteroaryl is optionally further substituted with halogen, then the halogen is preferably fluorine.

In some technical solutions of the present disclosure, in the compound of general formula (I), certain group is as defined below (unspecified groups are as defined above): when R₁ is amino, heterocyclyl, aryl or heteroaryl, and the amino, heterocyclyl, aryl or heteroaryl is optionally further substituted with alkyl, then the alkyl is preferably C₁-C₈ alkyl, more preferably C₁-C₆ alkyl, further preferably C₁-C₃ alkyl, e.g., methyl, ethyl, propyl or isopropyl, further preferably methyl.

In some technical solutions of the present disclosure, in the compound of general formula (I), certain group is as defined below (unspecified groups are as defined above): when R₁ is alkyl, amino, heterocyclyl, aryl or heteroaryl, and the alkyl, amino, heterocyclyl, aryl or heteroaryl is optionally further substituted with alkoxy, then the alkoxy is preferably C₁-C₈ alkoxy, more preferably C₁-C₆ alkoxy, further preferably C₁-C₃ alkoxy, e.g., methoxy, ethoxy, propoxy or isopropoxy, further preferably methoxy.

In some technical solutions of the present disclosure, in the compound of general formula (I), certain group is as defined below (unspecified groups are as defined above): when R₂ is alkyl, the alkyl is preferably C₁-C₈ alkyl, more preferably C₁-C₆ alkyl, further preferably C₁-C₃ alkyl, e.g., methyl, ethyl, propyl or isopropyl, further preferably methyl or ethyl.

In some technical solutions of the present disclosure, in the compound of general formula (I), certain group is as defined below (unspecified groups are as defined above): when R₂ is alkoxy, the alkoxy is preferably C₁-C₈ alkoxy, more preferably C₁-C₆ alkoxy, further preferably C₁-C₃ alkoxy, e.g., methoxy, ethoxy, propoxy or isopropoxy, further preferably methoxy or ethoxy.

In some technical solutions of the present disclosure, in the compound of general formula (I), certain group is as defined below (unspecified groups are as defined above): when R₂ is halogen, the halogen is preferably fluorine, chlorine or bromine.

In some technical solutions of the present disclosure, in the compound of general formula (I), certain group is as defined below (unspecified groups are as defined above): when R₂ is cycloalkyl, the cycloalkyl is preferably C₃-C₈ cycloalkyl, more preferably C₃-C₆ cycloalkyl, *e.g.,*

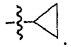

In some technical solutions of the present disclosure, in the compound of general formula (I), certain group is as defined below (unspecified groups are as defined above): when R₂ is heterocyclyl, the heterocyclyl is preferably 3-8 membered monocyclic heterocyclyl with "heteroatom selected from one or more of N, O and S, and the number of the heteroatom being 1-4", more preferably 4-6 membered monocyclic heterocyclyl with "heteroatom selected from one or more of N, O and S, and the number of the heteroatom being 1-3", *e.g.,*

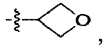

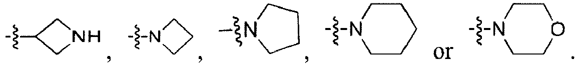

In some technical solutions of the present disclosure, in the compound of general formula (I), certain group is as defined below (unspecified groups are as defined above): when R₂ is aryl, the aryl is preferably 6-10 membered aryl, more preferably phenyl.

In some technical solutions of the present disclosure, in the compound of general formula (I), certain group is as defined below (unspecified groups are as defined above): when R₂ is heteroaryl, the heteroaryl is preferably 5-10 membered heteroaryl with "heteroatom selected from one or more of N, O and S, and the number of the heteroatom being 1-4", more preferably 5-6 membered heteroaryl with "heteroatom selected from one or more of N, O and S, and the number of the heteroatom being 1-3", *e.g.,*

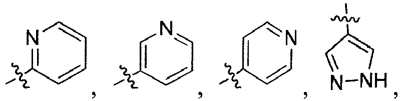

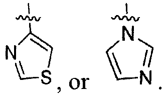

In some technical solutions of the present disclosure, in the compound of general formula (I), certain group is as defined below (unspecified groups are as defined above): when R₂ is -(CH₂)ₙ₁C(O)R_{cc}, the -(CH₂)ₙ₁C(O)R_{cc} is preferably

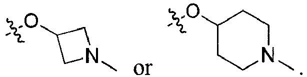

In some technical solutions of the present disclosure, in the compound of general formula (I), certain group is as defined below (unspecified groups are as defined above): when R₂ is -(CH₂)ₙ₁SRₐₐ, the -(CH₂)ₙ₁SRₐₐ is preferably

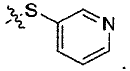

In some technical solutions of the present disclosure, in the compound of general formula (I), certain group is as defined below (unspecified groups are as defined above): when R₂ is -(CH₂)ₙ₁NRₐₐR_{bb}, the -(CH₂)ₙ₁NRₐₐR_{bb} is preferably

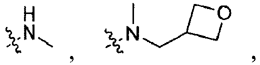

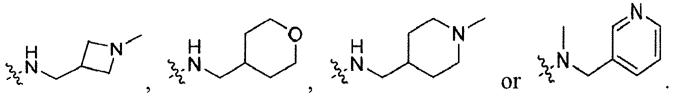

In some technical solutions of the present disclosure, in the compound of general formula (I), certain group is as defined below (unspecified groups are as defined above): when R₂ is alkyl, and the alkyl is optionally further substituted with unsubstituted alkoxy, then the alkoxy is preferably C₁-C₈ alkoxy, more preferably C₁-C₆ alkoxy, further preferably C₁-C₃ alkoxy, e.g., methoxy, ethoxy, propoxy or isopropoxy, further preferably methoxy.

In some technical solutions of the present disclosure, in the compound of general formula (I), certain group is as defined below (unspecified groups are as defined above): when R₂ is alkyl, and the alkyl is optionally further substituted with substituted or unsubstituted heterocyclyl, then the heterocyclyl is preferably 3-8 membered monocyclic heterocyclyl with "heteroatom selected from one or more of N, O and S, and the number of the heteroatom being 1-4", more preferably 4-6 membered monocyclic heterocyclyl with "heteroatom selected from one or more of N, O and S, and the number of the heteroatom being 1-3", *e.g.,* 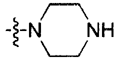 or

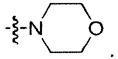

In some technical solutions of the present disclosure, in the compound of general formula (I), certain group is as defined below (unspecified groups are as defined above): when R₂ is alkyl, and the alkyl is optionally further substituted with substituted or unsubstituted heterocyclyl, then the heterocyclyl is preferably substituted with alkyl, the alkyl is preferably C₁-C₈ alkyl, more preferably C₁-C₆ alkyl, further preferably C₁-C₃ alkyl, e.g., methyl, ethyl, propyl or isopropyl, further preferably methyl.

In some technical solutions of the present disclosure, in the compound of general formula (I), certain group is as defined below (unspecified groups are as defined above): when R₂ is alkoxy, and the alkoxy is optionally further substituted with unsubstituted alkoxy, then the unsubstituted alkoxy is preferably C₁-C₈ alkoxy, more preferably C₁-C₆ alkoxy, further preferably C₁-C₃ alkoxy, *e*.*g*., methoxy, ethoxy, propoxy or isopropoxy, further preferably methoxy.

In some technical solutions of the present disclosure, in the compound of general formula (I), certain group is as defined below (unspecified groups are as defined above): when R₂ is alkoxy, and the alkoxy is optionally further substituted with substituted or unsubstituted heterocyclyl, then the heterocyclyl is preferably 3-8 membered monocyclic heterocyclyl with "heteroatom selected from one or more of N, O and S, and the number of the heteroatom being 1-4", more preferably 4-6 membered monocyclic heterocyclyl with "heteroatom selected from one or more of N, O and S, and the number of the heteroatom being 1-3", *e*.*g*., 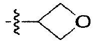 or

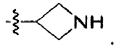

In some technical solutions of the present disclosure, in the compound of general formula (I), certain group is as defined below (unspecified groups are as defined above): when R₂ is alkoxy, and the alkoxy is optionally further substituted with substituted or unsubstituted heterocyclyl, then the heterocyclyl is preferably substituted with alkyl, the alkyl is preferably C₁-C₈ alkyl, more preferably C₁-C₆ alkyl, further preferably C₁-C₃ alkyl, e.g., methyl, ethyl, propyl or isopropyl, further preferably methyl.

In some technical solutions of the present disclosure, in the compound of general formula (I), certain group is as defined below (unspecified groups are as defined above): when R₂ is alkoxy, and the alkoxy is optionally further substituted with unsubstituted heteroaryl, then the heteroaryl is preferably 5-10 membered heteroaryl with "heteroatom selected from one or more of N, O and S, and the number of the heteroatom being 1-4", more preferably 5-6 membered heteroaryl with "heteroatom selected from one or more of N, O and S, and the number of the heteroatom being 1-3", *e*.*g*.,

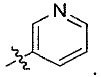

In some technical solutions of the present disclosure, in the compound of general formula (I), certain group is as defined below (unspecified groups are as defined above): when R₂ is amino, and the amino is optionally further substituted with unsubstituted alkyl, then the alkyl is preferably C₁-C₈ alkyl, more preferably C₁-C₆ alkyl, further preferably C₁-C₃ alkyl, *e.g.,* methyl, ethyl, propyl or isopropyl, further preferably methyl.

In some technical solutions of the present disclosure, in the compound of general formula (I), certain group is as defined below (unspecified groups are as defined above): when R₂ is heterocyclyl, and the heterocyclyl is optionally further substituted with unsubstituted alkyl, then the alkyl is preferably C₁-C₈ alkyl, more preferably C₁-C₆ alkyl, further preferably C₁-C₃ alkyl, e.g., methyl, ethyl, propyl or isopropyl, further preferably methyl.

In some technical solutions of the present disclosure, in the compound of general formula (I), certain group is as defined below (unspecified groups are as defined above): when R₂ is heterocyclyl, and the heterocyclyl is optionally further substituted with unsubstituted alkoxy, then the alkoxy is preferably C₁-C₈ alkoxy, more preferably C₁-C₆ alkoxy, further preferably C₁-C₃ alkoxy, e.g., methoxy, ethoxy, propoxy or isopropoxy, further preferably methoxy.

In some technical solutions of the present disclosure, in the compound of general formula (I), certain group is as defined below (unspecified groups are as defined above): when R₂ is heterocyclyl, and the heterocyclyl is optionally further substituted with substituted or unsubstituted amino, then the amino is preferably substituted with alkyl, the alkyl is preferably C₁-C₈ alkyl, more preferably C₁-C₆ alkyl, further preferably C₁-C₃ alkyl, e.g., methyl, ethyl, propyl or isopropyl, further preferably methyl.

In some technical solutions of the present disclosure, in the compound of general formula (I), certain group is as defined below (unspecified groups are as defined above): when R₂ is heteroaryl, and the heteroaryl is optionally further substituted with substituted or unsubstituted alkyl, then the alkyl is preferably C₁-C₈ alkyl, more preferably C₁-C₆ alkyl, further preferably C₁-C₃ alkyl, e.g., methyl, ethyl, propyl or isopropyl, further preferably methyl.

In some technical solutions of the present disclosure, in the compound of general formula (I), certain group is as defined below (unspecified groups are as defined above): when R₂ is heteroaryl, and the heteroaryl is optionally further substituted with substituted or unsubstituted alkyl, then the alkyl is preferably substituted with halogen, the halogen is preferably fluorine.

In some technical solutions of the present disclosure, in the compound of general formula (I), certain group is as defined below (unspecified groups are as defined above): when R₂ is heteroaryl, and the heteroaryl is optionally further substituted with unsubstituted alkoxy, then the alkoxy is preferably C₁-C₈ alkoxy, more preferably C₁-C₆ alkoxy, further preferably C₁-C₃ alkoxy, e.g., methoxy, ethoxy, propoxy or isopropoxy, further preferably methoxy.

In some technical solutions of the present disclosure, in the compound of general formula (I), certain group is as defined below (unspecified groups are as defined above): when R₂ is heteroaryl, and the heteroaryl is optionally further substituted with unsubstituted cycloalkyl, then the cycloalkyl is preferably C₃-C₈ cycloalkyl, more preferably C₃-C₆ cycloalkyl, e.g.,

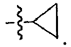

In some technical solutions of the present disclosure, in the compound of general formula (I), certain group is as defined below (unspecified groups are as defined above): when R_{cc} is heterocyclyl, the heterocyclyl is preferably 3-8 membered monocyclic heterocyclyl with "heteroatom selected from one or more of N, O and S, and the number of the heteroatom being 1-4", more preferably 4-6 membered monocyclic heterocyclyl with "heteroatom selected from one or more of N, O and S, and the number of the heteroatom being 1-3", *e*.*g*., 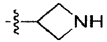 or

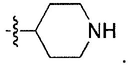

In some technical solutions of the present disclosure, in the compound of general formula (I), certain group is as defined below (unspecified groups are as defined above): when R_{cc} is heterocyclyl, and the heterocyclyl is optionally substituted with unsubstituted alkyl, then the alkyl is preferably C₁-C₈ alkyl, more preferably C₁-C₆ alkyl, further preferably C₁-C₃ alkyl, e.g., methyl, ethyl, propyl or isopropyl, further preferably methyl.

In some technical solutions of the present disclosure, in the compound of general formula (I), certain group is as defined below (unspecified groups are as defined above): when R₃ is alkyl, the alkyl is preferably C₁-C₈ alkyl, more preferably C₁-C₆ alkyl, further preferably C₁-C₃ alkyl, e.g., methyl, ethyl, propyl or isopropyl, further preferably methyl or ethyl.

In some technical solutions of the present disclosure, in the compound of general formula (I), certain group is as defined below (unspecified groups are as defined above): when R₃ is hydroxyalkyl, the alkyl is preferably C₁-C₈ hydroxyalkyl, more preferably C₁-C₆ hydroxyalkyl, further preferably C₁-C₃ hydroxyalkyl, e.g., hydroxymethyl, hydroxyethyl, hydroxypropyl or hydroxyisopropyl, further preferably hydroxymethyl.

In some technical solutions of the present disclosure, in the compound of general formula (I), certain group is as defined below (unspecified groups are as defined above): when R₃ is haloalkyl, the haloalkyl is preferably C₁-C₈ haloalkyl, more preferably C₁-C₆ haloalkyl, further preferably C₁-C₃ haloalkyl, e.g., -CHF₂ or CF₃.

In some technical solutions of the present disclosure, in the compound of general formula (I), certain group is as defined below (unspecified groups are as defined above): when R₃ is alkoxy, the alkoxy is preferably C₁-C₈ alkoxy, more preferably C₁-C₆ alkoxy, further preferably C₁-C₃ alkoxy, *e*.*g*., methoxy, ethoxy, propoxy or isopropoxy, further preferably methoxy.

In some technical solutions of the present disclosure, in the compound of general formula (I), certain group is as defined below (unspecified groups are as defined above): when R₃ is halogen, the halogen is preferably fluorine.

In some technical solutions of the present disclosure, in the compound of general formula (I), certain group is as defined below (unspecified groups are as defined above): when R₃ is cycloalkyl, the cycloalkyl is preferably C₃-C₈ cycloalkyl, more preferably C₃-C₆ cycloalkyl, *e*.*g*.,

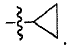

In some technical solutions of the present disclosure, in the compound of general formula (I), certain group is as defined below (unspecified groups are as defined above): when R₃ is heterocyclyl, the heterocyclyl is preferably 3-8 membered monocyclic heterocyclyl with "heteroatom selected from one or more of N, O and S, and the number of the heteroatom being 1-4", more preferably 4-6 membered monocyclic heterocyclyl with "heteroatom selected from one or more of N, O and S, and the number of the heteroatom being 1-3", *e.g.,* 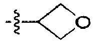 or

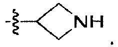

In some technical solutions of the present disclosure, in the compound of general formula (I), certain group is as defined below (unspecified groups are as defined above): when R₃ is alkyl, and the alkyl is optionally further substituted with halogen, then the halogen is preferably fluorine.

In some technical solutions of the present disclosure, in the compound of general formula (I), certain group is as defined below (unspecified groups are as defined above): when R₃ is alkyl, and the alkyl is optionally further substituted with unsubstituted alkoxy, then the alkoxy is preferably C₁-C₈ alkoxy, more preferably C₁-C₆ alkoxy, further preferably C₁-C₃ alkoxy, *e*.*g*., methoxy, ethoxy, propoxy or isopropoxy, further preferably methoxy.

In some technical solutions of the present disclosure, in the compound of general formula (I), certain group is as defined below (unspecified groups are as defined above): when R₃ is alkoxy, and the alkoxy is optionally further substituted with unsubstituted alkyl, then the alkyl is preferably C₁-C₈ alkyl, more preferably C₁-C₆ alkyl, further preferably C₁-C₃ alkyl, *e.g.,* methyl, ethyl, propyl or isopropyl, further preferably methyl.

In some technical solutions of the present disclosure, in the compound of general formula (I), certain group is as defined below (unspecified groups are as defined above): when R₃ is cycloalkyl, the cycloalkyl is preferably substituted with hydroxyl.

In some technical solutions of the present disclosure, in the compound of general formula (I), certain group is as defined below (unspecified groups are as defined above): when R₃ is heterocyclyl, and the heterocyclyl is optionally further substituted with unsubstituted alkyl, then the alkyl is preferably C₁-C₈ alkyl, more preferably C₁-C₆ alkyl, further preferably C₁-C₃ alkyl, e.g., methyl, ethyl, propyl or isopropyl, further preferably methyl.

In some technical solutions of the present disclosure, in the compound of general formula (I), certain group is as defined below (unspecified groups are as defined above): when R₃ is alkyl, and the alkyl is substituted with halogen, then R₃ is -CHF₂ or CF₃.

In some technical solutions of the present disclosure, in the compound of general formula (I), certain group is as defined below (unspecified groups are as defined above): when R₃ is alkyl, and the alkyl is substituted with hydroxyl, then R₃ is

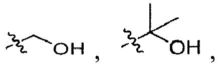

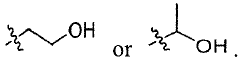

In some technical solutions of the present disclosure, in the compound of general formula (I), certain group is as defined below (unspecified groups are as defined above): when R₃ is alkyl, and the alkyl is substituted with unsubstituted alkoxy, then R₃ is

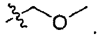

In some technical solutions of the present disclosure, in the compound of general formula (I), certain group is as defined below (unspecified groups are as defined above): when R₃ is cycloalkyl, and the cycloalkyl is optionally further substituted with hydroxyl, then R₃ is

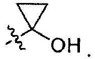

In some technical solutions of the present disclosure, in the compound of general formula (I), certain group is as defined below (unspecified groups are as defined above): when R₃ is heterocyclyl, and the heterocyclyl is optionally further substituted with unsubstituted alkyl, then R₃ is

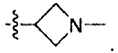

In some technical solutions of the present disclosure, in the compound of general formula (I), certain group is as defined below (unspecified groups are as defined above): when R₃ is -(CH₂)ₙ₁C(O)Rₐₐ, the -(CH₂)ₙ₁C(O)Rₐₐ is preferably

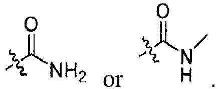

In some technical solutions of the present disclosure, in the compound of general formula (I), certain group is as defined below (unspecified groups are as defined above): when R₃ is -(CH₂)ₙ₁C(O)NRₐₐR_{bb}, the -(CH₂)ₙ₁C(O)NRₐₐR_{bb} is preferably

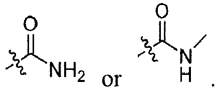

In some technical solutions of the present disclosure, in the compound of general formula (I), certain group is as defined below (unspecified groups are as defined above): when R₃ is -(CH₂)ₙ₁S(O)ₘ₁Rₐₐ, the -(CH₂)ₙ₁S(O)ₘ₁Rₐₐ is preferably

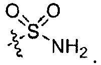

In some technical solutions of the present disclosure, in the compound of general formula (I), certain group is as defined below (unspecified groups are as defined above): when R₃ is -(CH₂)ₙ₁S(O)ₘ₁NRₐₐR_{bb}, the -(CH₂)ₙ₁S(O)ₘ₁NRₐₐR_{bb} is preferably

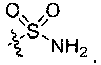

In some technical solutions of the present disclosure, in the compound of general formula (I), certain group is as defined below (unspecified groups are as defined above): when Rₐₐ and R_{bb} are independently alkyl, the alkyl is preferably C₁-C₈ alkyl, more preferably C₁-C₆ alkyl, further preferably C₁-C₃ alkyl, e.g., methyl, ethyl, propyl or isopropyl, further preferably methyl.

In some technical solutions of the present disclosure, in the compound of general formula (I), certain group is as defined below (unspecified groups are as defined above): when Rₐₐ and R_{bb} are independently heteroaryl, the heteroaryl is preferably 5-10 membered heteroaryl with "heteroatom selected from one or more of N, O and S, and the number of the heteroatom being 1-4", more preferably 5-6 membered heteroaryl with "heteroatom selected from one or more of N, O and S, and the number of the heteroatom being 1-3", *e.g.,*

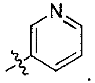

In some technical solutions of the present disclosure, in the compound of general formula (I), certain group is as defined below (unspecified groups are as defined above): when Rₐₐ and R_{bb} are independently alkyl, and the alkyl is optionally further substituted with substituted or unsubstituted heterocyclyl, then the heterocyclyl is preferably 3-8 membered monocyclic heterocyclyl with "heteroatom selected from one or more of N, O and S, and the number of the heteroatom being 1-4", more preferably 4-6 membered monocyclic heterocyclyl with "heteroatom selected from one or more of N, O and S, and the number of the heteroatom being 1-3", *e.g.,*

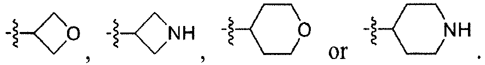

In some technical solutions of the present disclosure, in the compound of general formula (I), certain group is as defined below (unspecified groups are as defined above): when Rₐₐ and R_{bb} are independently alkyl, and the alkyl is optionally further substituted with substituted or unsubstituted heterocyclyl, then the heterocyclyl is preferably substituted with alkyl, the alkyl is preferably C₁-C₈ alkyl, more preferably C₁-C₆ alkyl, further preferably C₁-C₃ alkyl, *e*.*g*., methyl, ethyl, propyl or isopropyl, further preferably methyl.

In some technical solutions of the present disclosure, in the compound of general formula (I), certain group is as defined below (unspecified groups are as defined above): when Rₐₐ and R_{bb} are independently alkyl, and the alkyl is optionally further substituted with unsubstituted heteroaryl, then the heteroaryl is preferably 5-10 membered heteroaryl with "heteroatom selected from one or more of N, O and S, and the number of the heteroatom being 1-4", more preferably 5-6 membered heteroaryl with "heteroatom selected from one or more of N, O and S, and the number of the heteroatom being 1-3, *e.g.,*

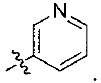

In some technical solutions of the present disclosure, in the compound of general formula (I), certain group is as defined below (unspecified groups are as defined above): when Rₐₐ and R_{bb} are independently amino, and the amino is optionally further substituted with unsubstituted alkyl, then the alkyl is preferably C₁-C₈ alkyl, more preferably C₁-C₆ alkyl, further preferably C₁-C₃ alkyl, e.g., methyl, ethyl, propyl or isopropyl, further preferably methyl.

In some technical solutions of the present disclosure, in the compound of general formula (I), certain group is as defined below (unspecified groups are as defined above): -L₁- is preferably bond, -CH₂-, -(CH₂)₂-, 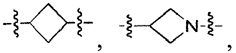 -C(O)-, -C(O)CH₂-, -CH₂C(O)-,-C(O)CH₂NH-, -C(O)CH₂N(CH₃)-, -C(O)CH₂NHCH₂-, -C(O)CH₂NH(CH₂)₂-,-CH₂C(O)N(CH₃)-, -NH(CH₂)₂-, -S(O)₂-, or -S(O)₂NH-, more preferably bond, -CH₂-, -(CH₂)₂-, -C(O)-, -C(O)CH₂-, -C(O)CH₂NH-, -C(O)CH₂N(CH₃)-, -C(O)CH₂NH(CH₂)₂-,-CH₂C(O)N(CH₃)-, or -S(O)₂-; the left end of L₁ is connected with ring B, and the right end of L₁ is connected with R₁.

In some technical solutions of the present disclosure, in the compound of general formula (I), certain group is as defined below (unspecified groups are as defined above): L₂ is preferably bond, oxygen atom, -CH₂-, -CH(OH)-, -C(O)-, -NH-, -N(CH₃)- or -S(O)₂-, more preferably bond, oxygen atom, -NH-, or -N(CH₃)-; the left end of L₂ is connected with ring A, and the right end of L₂ is connected with ring B.

In some technical solutions of the present disclosure, in the compound of general formula (I), certain group is as defined below (unspecified groups are as defined above): L₃ is preferably bond, -NH- or -C(O)NH-, more preferably -NH-; the left end of L₃ is connected with ring C, and the right end of L₃ is connected with ring B.

In some technical solutions of the present disclosure, in the compound of general formula (I), certain group is as defined below (unspecified groups are as defined above): the ring A is preferably the following group, 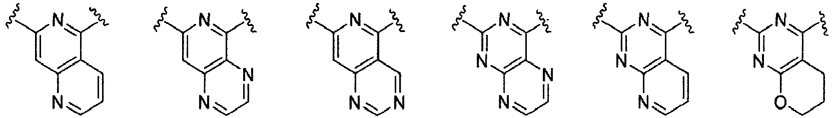 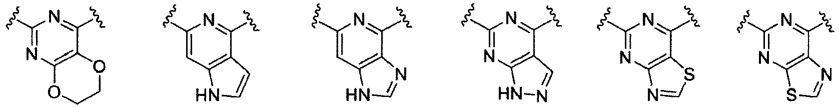 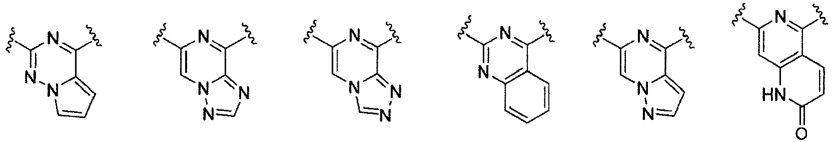 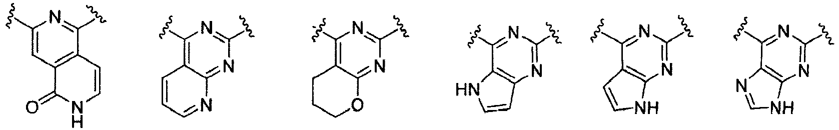 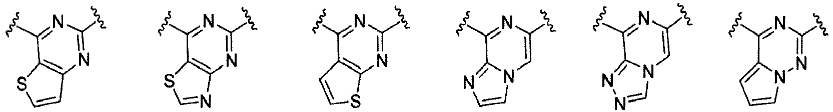 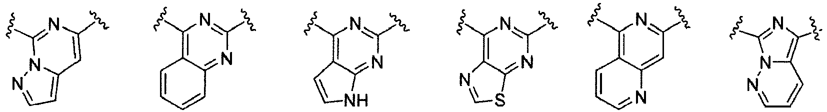 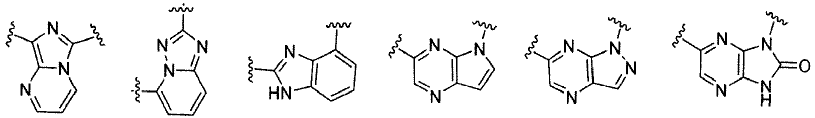 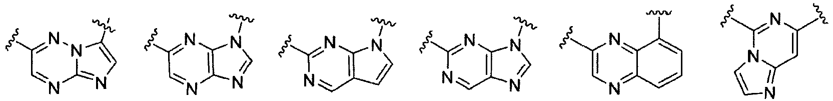 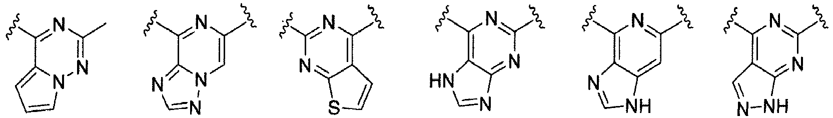 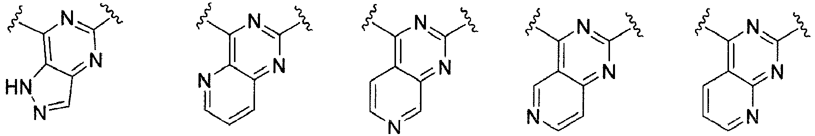 or 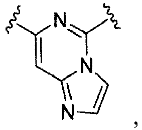 more preferably 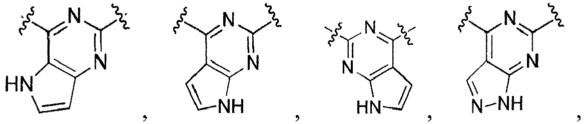 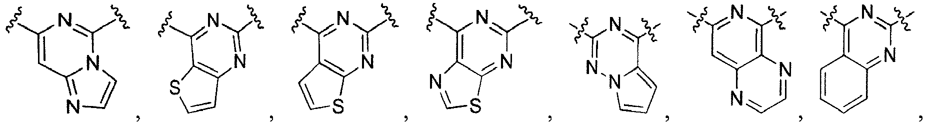 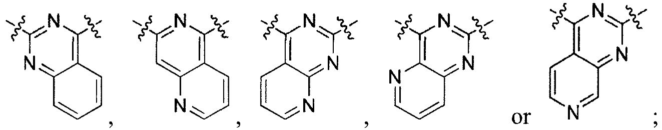 the left end of ring A is connected with L₃, and the right end of ring A is connected with L₂.

In some technical solutions of the present disclosure, in the compound of general formula (I), certain group is as defined below (unspecified groups are as defined above): the ring B is preferably the following group, 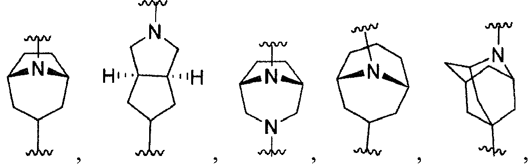 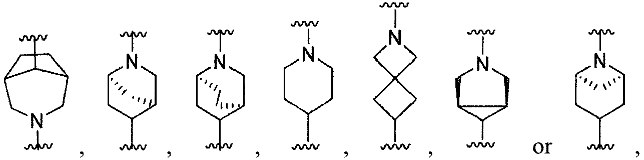 more preferably 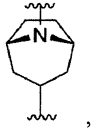 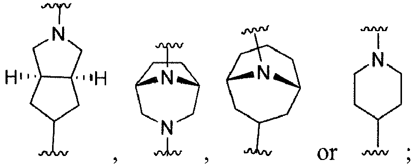 the upper end of ring B is connected with L₁, and the lower end of ring B is connected with L₂.

In some technical solutions of the present disclosure, in the compound of general formula (I), certain group is as defined below (unspecified groups are as defined above): the ring C is preferably the following group, 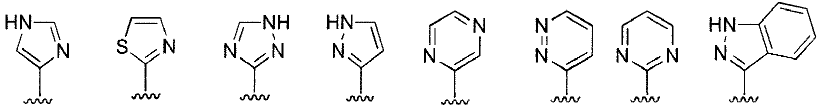 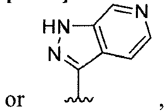 more preferably

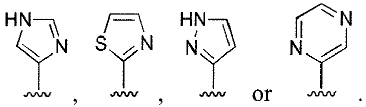

In some technical solutions of the present disclosure, in the compound of general formula (I), certain group is as defined below (unspecified groups are as defined above): R₁ is preferably hydrogen atom, methyl, ethyl, fluorine, methoxy, ethoxy, phenyl, cyano, -CHF₂,-CH₂CH₂CN, -CH₂CH₂F, -NHCH₂CH₂CN, 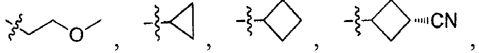 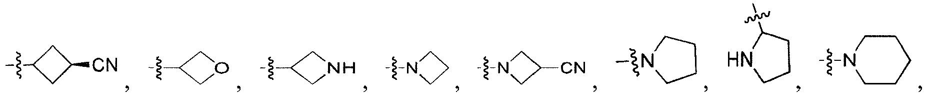 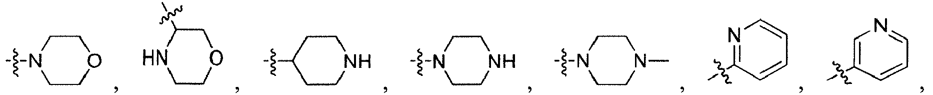 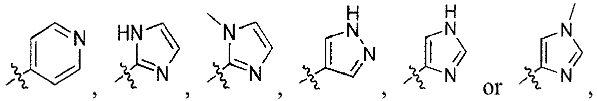 more preferably hydrogen atom, methyl, ethyl, fluorine, cyano, -CHF₂, -CH₂CH₂CN, -CH₂CH₂F,

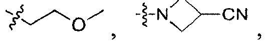

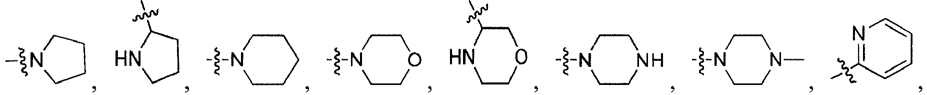

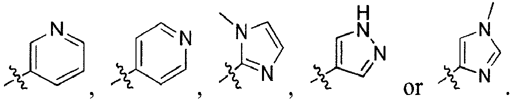

In some technical solutions of the present disclosure, in the compound of general formula (I), certain group is as defined below (unspecified groups are as defined above): R₂ is preferably hydrogen atom, fluorine, chlorine, bromine, amino, hydroxyl, cyano, methyl, methoxy, 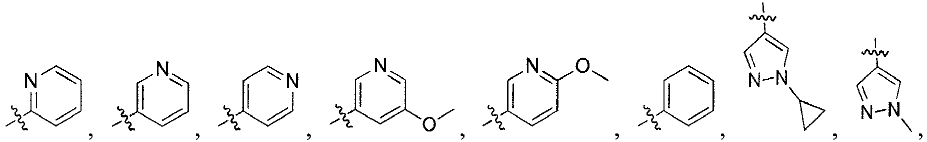 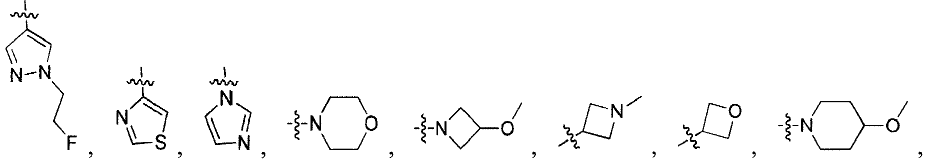 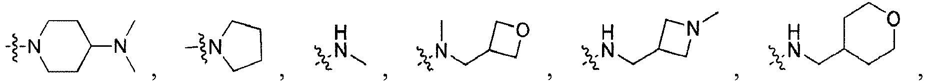 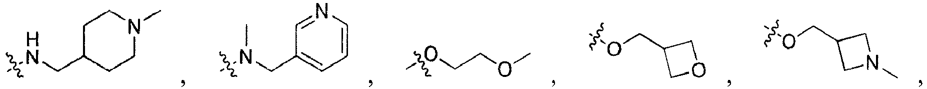 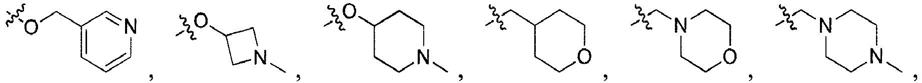 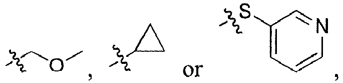 more preferably hydrogen atom, fluorine, chlorine, bromine, methyl, methoxy,

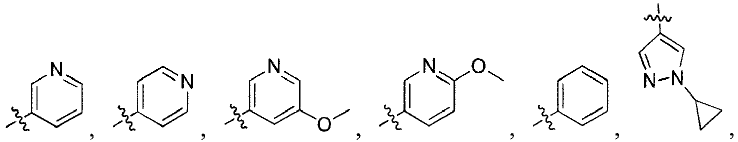

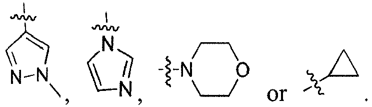

In some technical solutions of the present disclosure, in the compound of general formula (I), certain group is as defined below (unspecified groups are as defined above): R₃ is preferably hydrogen atom, methyl, ethyl, fluorine, cyano, -CHF₂, CF₃, 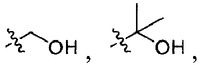 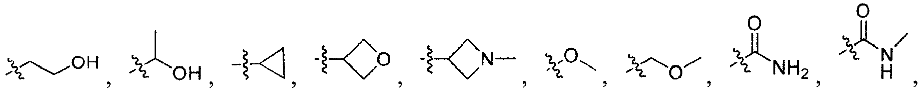 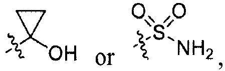 more preferably hydrogen atom, methyl, ethyl, fluorine, cyano,

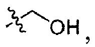

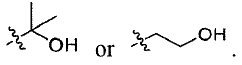

In some technical solutions of the present disclosure, in the compound of general formula (I), certain group is as defined below (unspecified groups are as defined above): structure -L₁-R₁ is preferably 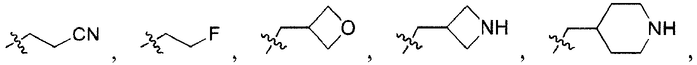 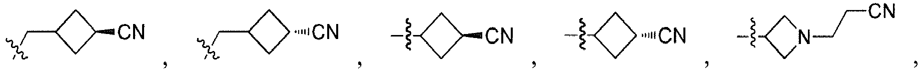 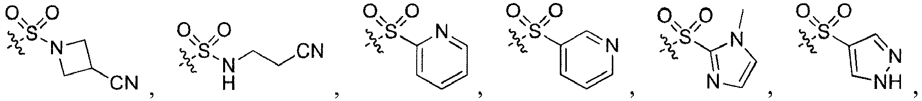 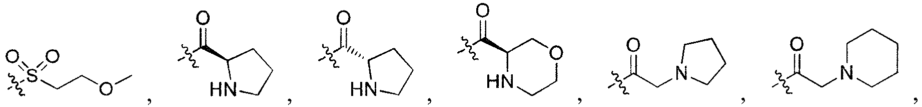 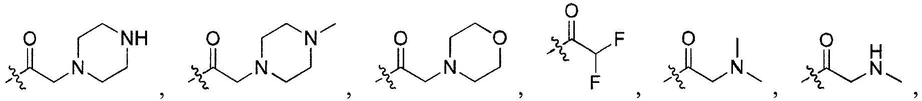 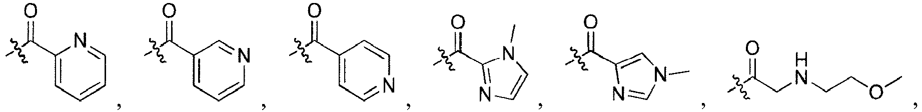 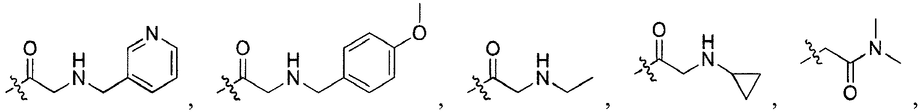 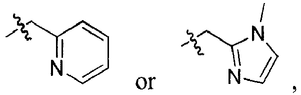 more preferably

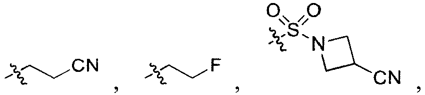

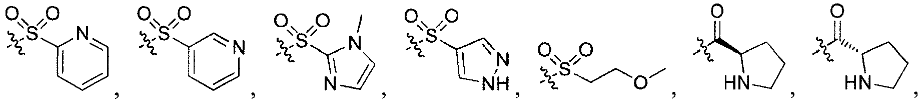

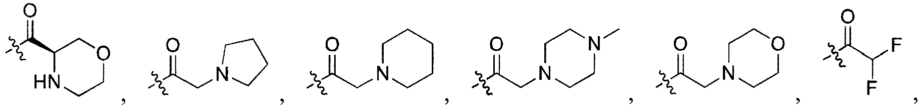

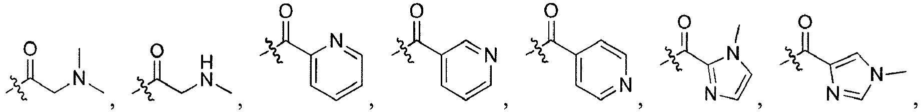

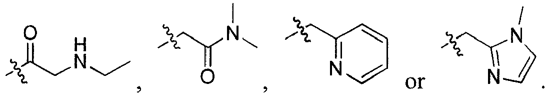

In some technical solutions of the present disclosure, in the compound of general formula (I), certain groups are as defined below (unspecified groups are as defined above):
L₁ and R₁ are defined as any one of the following combinations of definitions:
(1) L₁ is alkylene, R₁ is cycloalkyl, heterocyclyl or 5 membered heteroaryl; wherein the cycloalkyl or heterocyclyl is optionally further substituted with cyano; the 5 membered heteroaryl is optionally further substituted with alkyl;
(2) L₁ is cycloalkyl or heterocyclyl, R₁ is cyano or alkyl; the alkyl is optionally further substituted with cyano;
(3) L₁ is -(CH₂)ₙ₁C(O)(CH₂)ₘ₁-, R₁ is heterocyclyl or 5 membered heteroaryl; the heterocyclyl or 5 membered heteroaryl is optionally further substituted with alkyl;
(4) L₁ is -C(O)(CH₂)ₘ₁NH(CH₂)ₘ₂-, R₁ is hydrogen atom, alkoxy, cycloalkyl, aryl or heteroaryl; the aryl is optionally further substituted with alkoxy;
(5) L₁ is -(CH₂)ₙ₁S(O)ₘ₁-, R₁ is 5 membered heteroaryl; the 5 membered heteroaryl is optionally further substituted with alkyl;
(6) L₁ is -(CH₂)ₙ₁S(O)ₘ₁NH-, R₁ is alkyl; the alkyl is optionally further substituted with cyano.

In some technical solutions of the present disclosure, in the compound of general formula (I), certain groups are as defined below (unspecified groups are as defined above):
L₁ and R₁ are defined as any one of the following combinations of definitions:
   (1) L₁ is alkylene, R₁ is cycloalkyl, heterocyclyl, or 5 membered heteroaryl; wherein the cycloalkyl or heterocyclyl is optionally further substituted with cyano; the 5 membered heteroaryl is optionally further substituted with alkyl;
   (2) L₁ is cycloalkyl, or heterocyclyl, R₁ is cyano, or alkyl; the alkyl is optionally further substituted with cyano;
   (3) L₁ is -(CH₂)ₙ₁C(O)(CH₂)ₘ₁-, R₁ is heterocyclyl, or 5 membered heteroaryl; the heterocyclyl, or 5 membered heteroaryl is optionally further substituted with alkyl;
   (4) L₁ is -C(O)(CH₂)ₘ₁NH(CH₂)ₘ₂-, R₁ is hydrogen atom, alkoxy, cycloalkyl, aryl, or heteroaryl; the aryl is optionally further substituted with alkoxy;
   (5) L₁ is -(CH₂)ₙ₁S(O)ₘ₁-, R₁ is 5 membered heteroaryl; the 5 membered heteroaryl is optionally further substituted with alkyl;
   (6) L₁ is -(CH₂)ₙ₁S(O)ₘ₁NH-, R₁ is alkyl; the alkyl is optionally further substituted with cyano;
L₂ is -NR₄-;
R₄ is selected from hydrogen atom, or alkyl;
L₃ is -NH-;
ring A is 6-14 membered fused heteroaryl;
ring B is selected from 3-10 membered monocyclic heterocyclyl, or 6-14 membered bridged heterocyclyl;
ring C is heteroaryl;
R₂ is selected from hydrogen atom, or alkyl;
R₃ is selected from hydrogen atom, alkyl, or hydroxyalkyl.

In some technical solutions of the present disclosure, in the compound of general formula (I), certain groups are as defined below (unspecified groups are as defined above):
L₁ and R₁ are defined as any one of the following combinations of definitions:
   (1) L₁ is alkylene, R₁ is 5 membered heteroaryl; wherein the 5 membered heteroaryl is optionally further substituted with alkyl;
   (2) L₁ is -(CH₂)ₙ₁C(O)(CH₂)ₘ₁-, R₁ is heterocyclyl or 5 membered heteroaryl; the heterocyclyl or 5 membered heteroaryl is optionally further substituted with alkyl;
   (3) L₁ is -C(O)(CH₂)ₘ₁NH(CH₂)ₘ₂-, R₁ is hydrogen atom;
   (4) L₁ is -(CH₂)ₙ₁S(O)ₘ₁-, R₁ is 5 membered heteroaryl; the 5 membered heteroaryl is optionally further substituted with alkyl;
L₂ is -NR₄-;
R₄ is selected from hydrogen atom, or alkyl;
L₃ is -NH-;
ring A is 6-14 membered fused heteroaryl;
ring B is 6-14 membered bridged heterocyclyl;
ring C is heteroaryl;
R₂ is selected from hydrogen atom, or alkyl;
R₃ is selected from hydrogen atom, or alkyl.

In some technical solutions of the present disclosure, in the compound of general formula (I), certain groups are as defined below (unspecified groups are as defined above): L₁ is alkylene, R₁ is cycloalkyl, heterocyclyl or 5 membered heteroaryl; wherein the cycloalkyl or heterocyclyl is optionally further substituted with cyano; the 5 membered heteroaryl is optionally further substituted with alkyl.

In some technical solutions of the present disclosure, in the compound of general formula (I), certain groups are as defined below (unspecified groups are as defined above): L₁ is cycloalkyl or heterocyclyl, R₁ is cyano or alkyl; the alkyl is optionally further substituted with cyano.

In some technical solutions of the present disclosure, in the compound of general formula (I), certain groups are as defined below (unspecified groups are as defined above): L₁ is -(CH₂)ₙ₁C(O)(CH₂)ₘ₁-, R₁ is heterocyclyl or 5 membered heteroaryl; the heterocyclyl or 5 membered heteroaryl is optionally further substituted with alkyl.

In some technical solutions of the present disclosure, in the compound of general formula (I), certain groups are as defined below (unspecified groups are as defined above): L₁ is -C(O)(CH₂)ₘ₁NH(CH₂)ₘ₂-, R₁ is hydrogen atom, alkoxy, cycloalkyl, aryl or heteroaryl; the aryl is optionally further substituted with alkoxy.

In some technical solutions of the present disclosure, in the compound of general formula (I), certain groups are as defined below (unspecified groups are as defined above): L₁ is -(CH₂)ₙ₁S(O)ₘ₁-, R₁ is 5 membered heteroaryl; the 5 membered heteroaryl is optionally further substituted with alkyl.

In some technical solutions of the present disclosure, in the compound of general formula (I), certain groups are as defined below (unspecified groups are as defined above): L₁ is -(CH₂)ₙ₁S(O)ₘ₁NH-, R₁ is alkyl; the alkyl is optionally further substituted with cyano.

In some technical solutions of the present disclosure, the compound of formula (I) may be any one of the following structures,

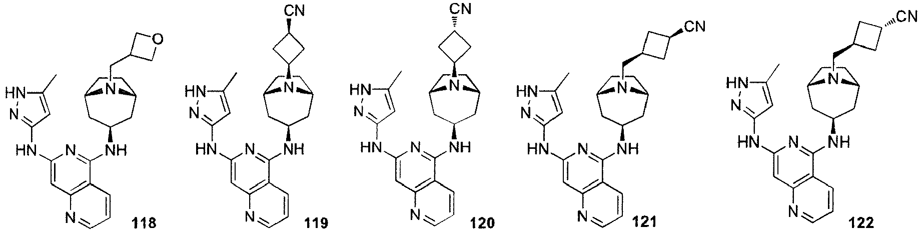

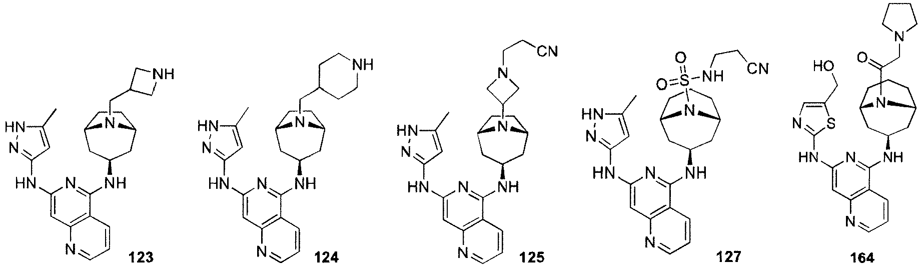

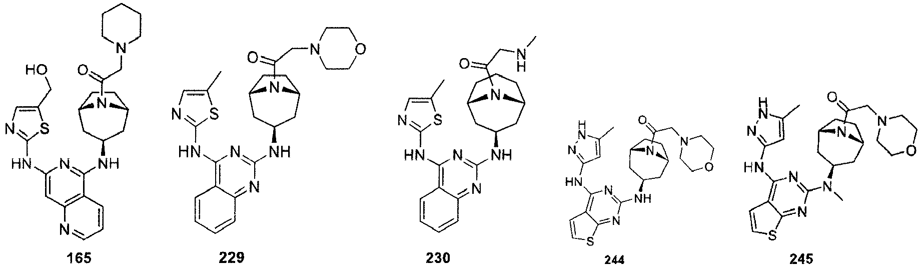

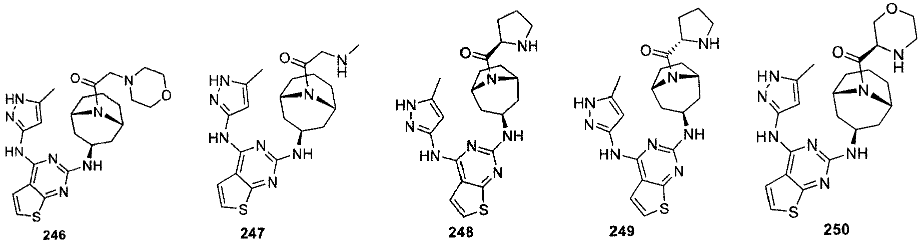

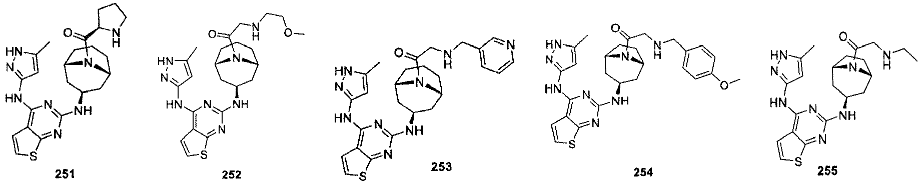

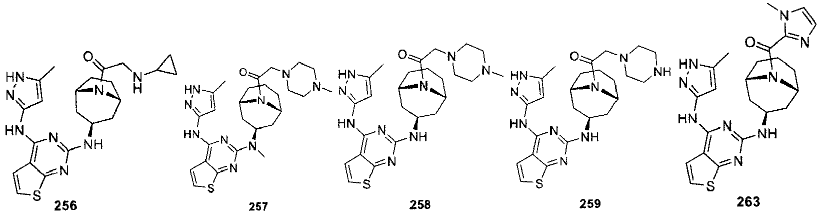

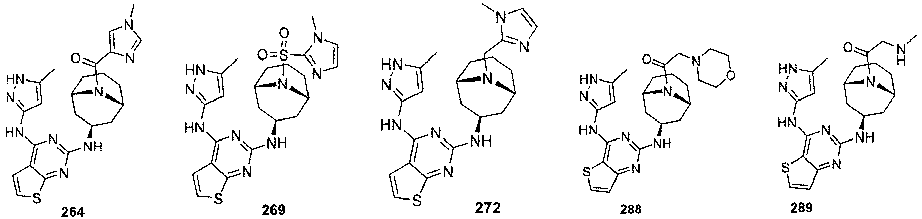

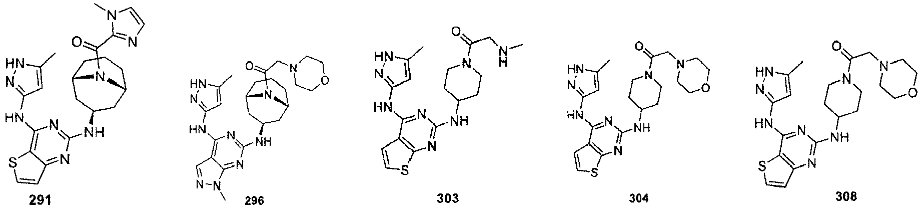

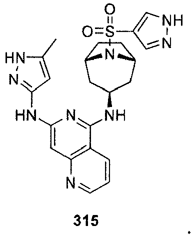

In some technical solutions of the present disclosure, in the compound of general formula (I), certain group is as defined below (unspecified groups are as defined above):

ring B is selected from 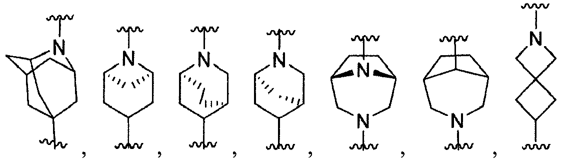 or

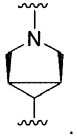

In some technical solutions of the present disclosure, in the compound of general formula (I), certain groups are as defined below (unspecified groups are as defined above):
ring B is selected from 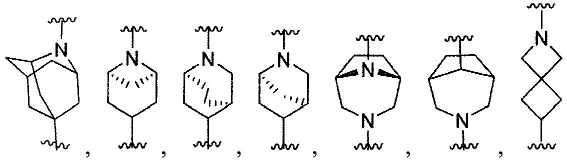 or 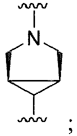
L₁ is alkylene or -NH(CH₂)ₙ₁-;
L₂ is selected from bond, or -NH-;
L₃ is NH;
ring A is 6-14 membered fused heteroaryl;
ring C is heteroaryl;
R₁ is cyano;
R₂ is hydrogen atom;
R₃ is selected from hydrogen atom, or alkyl;
n₁ is 0, 1 or 2.

In some technical solutions of the present disclosure, in the compound of general formula (I), certain groups are as defined below (unspecified groups are as defined above):
ring B is 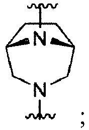
L₁ is alkylene;
L₂ is bond;
L₃ is NH;
ring A is 6-14 membered fused heteroaryl;
ring C is heteroaryl;
R₁ is cyano;
R₂ is hydrogen atom;
R₃ is hydrogen atom, or alkyl.

In some technical solutions of the present disclosure, the compound of formula (I) may be any one of the following structures, 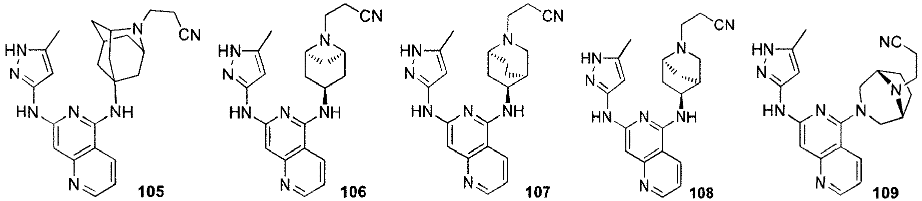 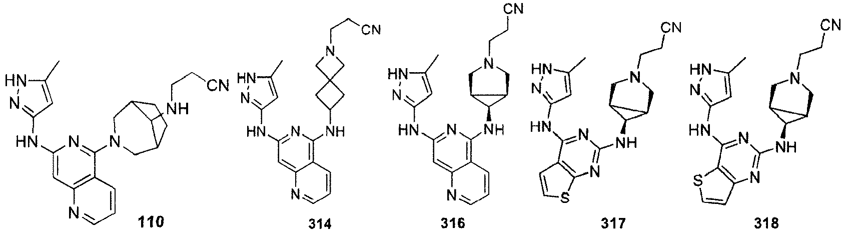 or

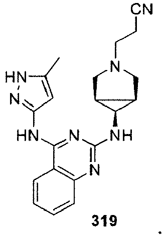

In some technical solutions of the present disclosure, in the compound of general formula (I), certain groups are as defined below (unspecified groups are as defined above):
L₂ is selected from bond, -CHRₐₐ-, -(CH₂)ₙ₁C(O)-, or -(CH₂)ₙ₁S(O)₂-; Rₐₐ is selected from hydrogen atom, or hydroxyl;
n₁ is 0, 1 or 2.

In some technical solutions of the present disclosure, in the compound of general formula (I), certain groups are as defined below (unspecified groups are as defined above):
L₂ is selected from bond, -CHRₐₐ-, -(CH₂)ₙ₁C(O)- or -(CH₂)ₙ₁S(O)₂-; Rₐₐ is selected from hydrogen atom or hydroxyl;
n₁ is 0, 1 or 2;
L₁ is alkylene;
L₃ is NH;
ring A is 6-14 membered fused heteroaryl;
ring B is 6-14 membered bridged heterocyclyl;
ring C is heteroaryl;
R₁ is cyano;
R₂ is selected from hydrogen atom, alkyl, alkoxy, or heterocyclyl;
R₃ is selected from hydrogen atom, or alkyl.

In some technical solutions of the present disclosure, the compound of formula (I) may be any of the following structures,

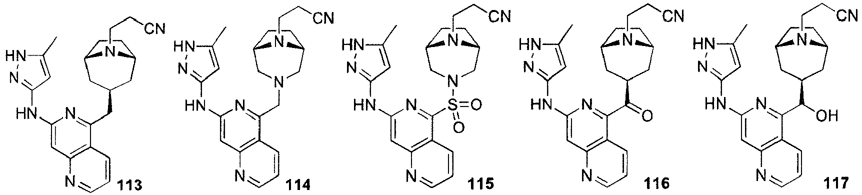

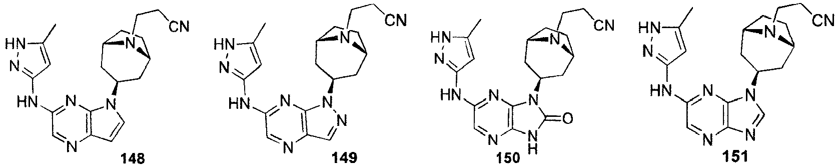

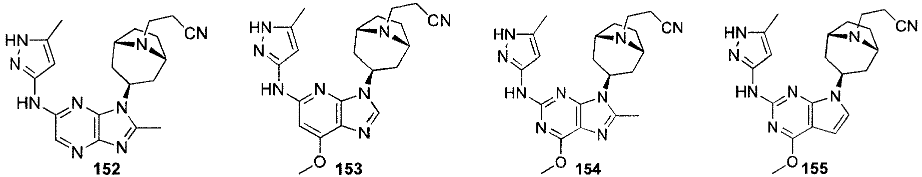

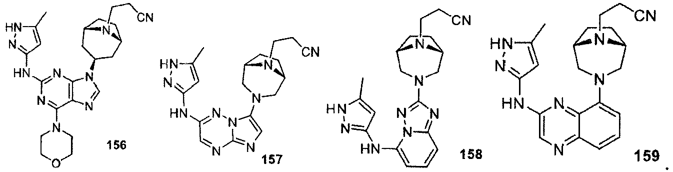

In some technical solutions of the present disclosure, in the compound of general formula (I), certain groups are as defined below (unspecified groups are as defined above):

ring A is 6-14 membered fused heteroaryl, and ring A is not 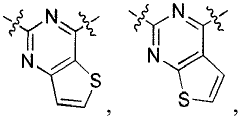 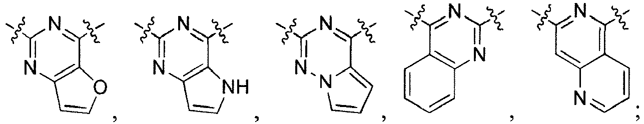 wherein, the left end of ring A is connected with L₃, and the right end of ring A is connected with L₂.

In some technical solutions of the present disclosure, in the compound of general formula (I), certain groups are as defined below (unspecified groups are as defined above):

ring A is 6-14 membered fused heteroaryl, and ring A is not 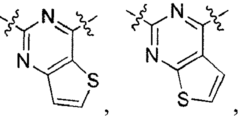 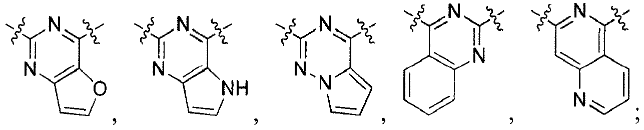 wherein, the left end of ring A is connected with L₃, and the right end of ring A is connected with L₂;
L₁ is selected from alkylene, -(CH₂)ₙ₁C(O)(CH₂)ₘ₁-,-(CH₂)ₙ₁C(O)(CH₂)ₘ₁NRₐₐ(CH₂)ₘ₂-, or -(CH₂)ₙ₁S(O)ₘ₁-;
L₂ is selected from bond, oxygen atom, or -NR₄-;
R₄ is selected from hydrogen atom, or alkyl;
L₃ is -NH-;
ring B is selected from 3-10 membered monocyclic heterocyclyl, 6-14 membered bridged heterocyclyl, or 6-14 membered fused heterocyclyl;
ring C is heteroaryl;
R₁ is selected from hydrogen atom, alkyl, alkoxy, cyano, cycloalkyl, heterocyclyl, aryl, or heteroaryl; the heterocyclyl or heteroaryl is optionally further substituted with cyano or alkyl; the aryl is optionally further substituted with alkoxy;
R₂ is selected from hydrogen atom, alkyl, alkoxy, halogen, or heterocyclyl; the alkyl is optionally further substituted with unsubstituted alkoxy, heterocyclyl unsubstituted or substituted with alkyl; the heterocyclyl is optionally further substituted with alkyl;
R₃ is selected from hydrogen atom, alkyl, or hydroxyalkyl;
Rₐₐ is selected from hydrogen atom, or alkyl.

In some technical solutions of the present disclosure, in the compound of general formula (I), certain groups are as defined below (unspecified groups are as defined above):
ring A is 6-14 membered fused heteroaryl, and ring A is not 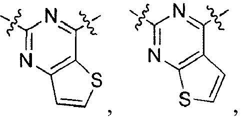 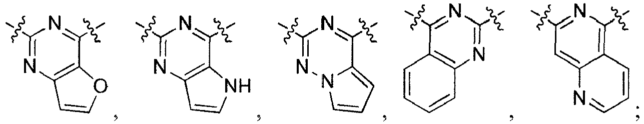 wherein, the left end of ring A is connected with L₃, and the right end of ring A is connected with L₂;
L₁ is selected from alkylene, -(CH₂)ₙ₁C(O)(CH₂)ₘ₁-,-(CH₂)ₙ₁C(O)(CH₂)ₘ₁NRₐₐ(CH₂)ₘ₂-, or -(CH₂)ₙ₁S(O)ₘ₁-;
L₂ is selected from oxygen atom, or -NR₄-;
R₄ is selected from hydrogen atom, or alkyl;
L₃ is -NH-;
ring B is selected from 3-10 membered monocyclic heterocyclyl, 6-14 membered bridged heterocyclyl, or 6-14 membered fused heterocyclyl;
ring C is heteroaryl;
R₁ is selected from hydrogen atom, alkyl, alkoxy, cyano, heterocyclyl, or heteroaryl; the heterocyclyl or heteroaryl is optionally further substituted with cyano or alkyl;
R₂ is selected from hydrogen atom, alkyl, alkoxy, halogen, or heterocyclyl; the alkyl is optionally further substituted with alkoxy, heterocyclyl unsubstituted or substituted with alkyl; the heterocyclyl is optionally further substituted with alkyl;
R₃ is selected from hydrogen atom, alkyl, or hydroxyalkyl;
Rₐₐ is selected from hydrogen atom, or alkyl.

In some technical solutions of the present disclosure, in the compound of general formula (I), certain group is as defined below (unspecified groups are as defined above): when the ring A is 6-14 membered fused heteroaryl, the 6-14 membered fused heteroaryl is preferably 6-14 membered fused heteroaryl with "heteroatom selected from one or more of N, O and S, and the number of the heteroatom being 1-4", more preferably 5,6 fused heteroaryl with "heteroatom selected from one or more of N, O and S, and the number of the heteroatom being 1-4" or 6,6 fused heteroaryl with "heteroatom selected from one or more of N, O and S, and the number of the heteroatom being 1-4". The 5,6 fused heteroaryl is preferably thienopyrimidinyl or pyrazolopyrimidinyl, benzopyrrolyl, pyrrolopyridyl, imidazolopyridyl, triazolopyridyl, imidazolopyridazinyl, pyrrolopyrimidinyl, pyrazolopyrimidinyl, imidazolopyrimidinyl, triazolopyrimidinyl, thienopyrimidinyl, thiazolopyrimidinyl, pyrrolopyrazinyl, pyrazolopyrazinyl, imidazolopyrazinyl, triazolopyrazinyl, pyrrolotriazinyl, imidazolotriazinyl or imidazolopyrazinonyl, more preferably 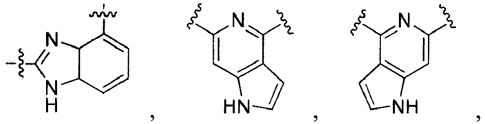 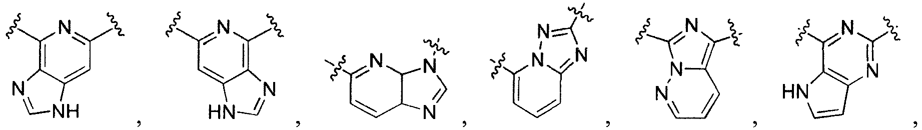 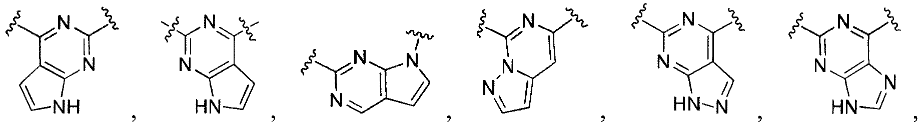 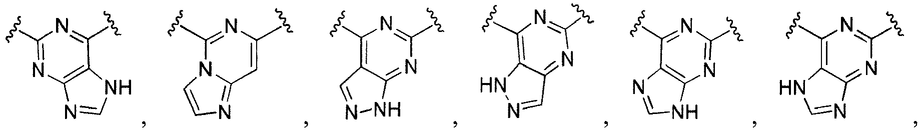 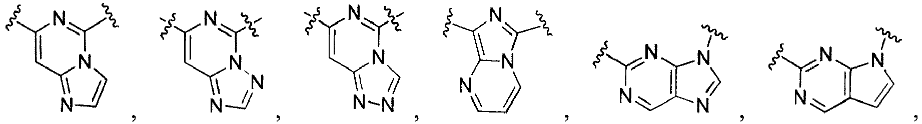 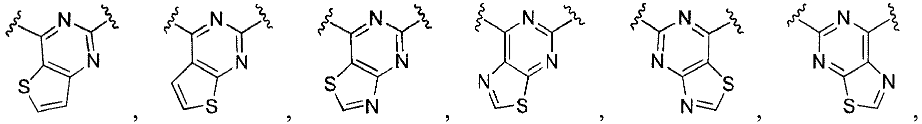 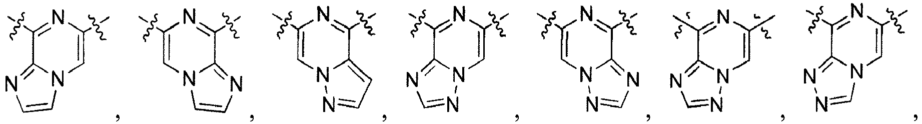 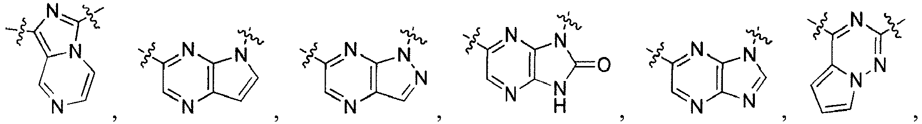 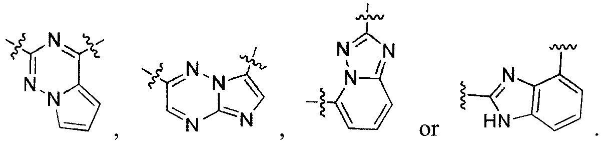 The 6,6 fused heteroaryl is preferably isoquinolinyl, naphthyridinyl, pyridopyrazinyl, pyridopyrimidinyl, quinazolinyl, pteridinyl, quinoxalinyl, dihydropyranopyrimidinyl, dihydrodioxinopyrimidinyl, 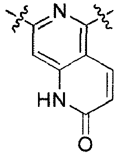 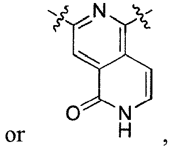 more preferably 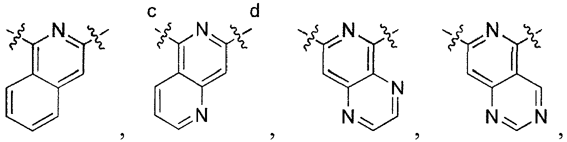 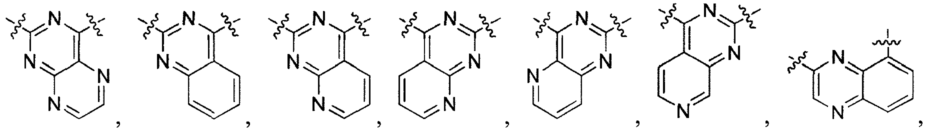 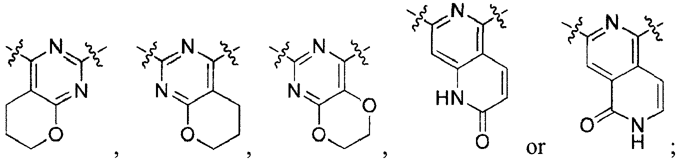 wherein, the left end of ring A is connected with L₃, and the right end of ring A is connected with L₂.

In some technical solutions of the present disclosure, in the compound of general formula (I), certain group is as defined below (unspecified groups are as defined above): the ring A is preferably pyrrolopyrazinyl, pyrazolopyrazinyl, imidazolopyrazinyl, triazolopyrazinyl, 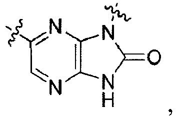 pyrrolopyrazinyl, pteridinyl or quinoxalinyl, more preferably

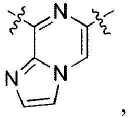

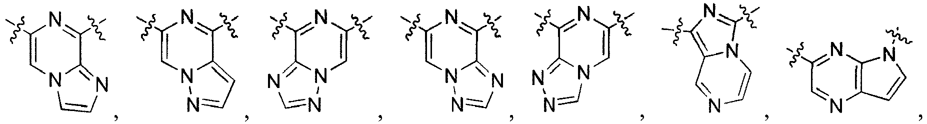

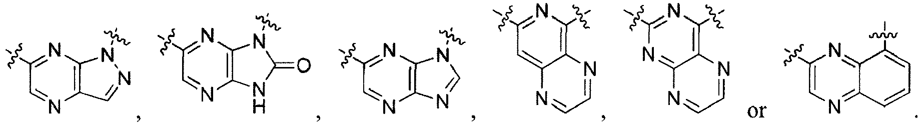

In some technical solutions of the present disclosure, in the compound of general formula (I), certain group is as defined below (unspecified groups are as defined above): the ring A is preferably pyrazolopyrimidinyl or pyrazolopyrazinyl, more preferably

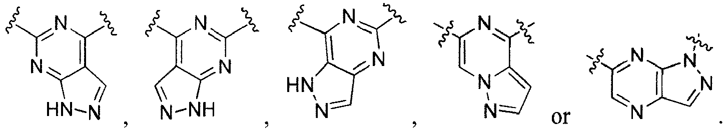

In some technical solutions of the present disclosure, in the compound of general formula (I), certain group is as defined below (unspecified groups are as defined above): the ring A is preferably imidazopyridyl, imidazopyridazinyl, imidazopyrimidinyl, imidazopyrazinyl or imidazotriazinyl, more preferably

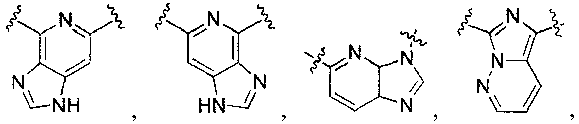

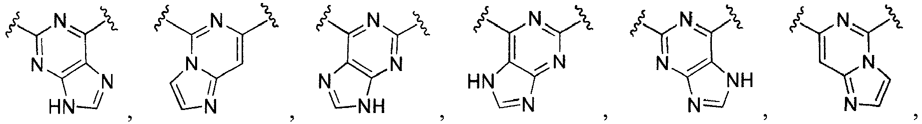

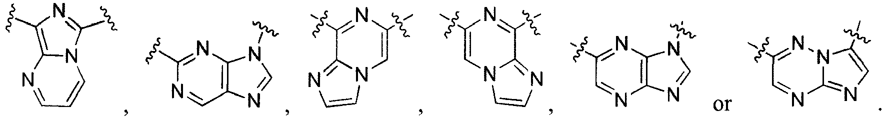

In some technical solutions of the present disclosure, in the compound of general formula (I), certain group is as defined below (unspecified groups are as defined above): the ring A is preferably thiazolopyrimidinyl, more preferably

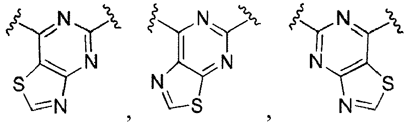

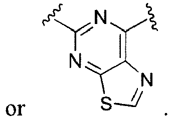

In some technical solutions of the present disclosure, in the compound of general formula (I), certain group is as defined below (unspecified groups are as defined above): the ring A is preferably triazolopyridyl, triazolopyrimidinyl or triazolopyrazinyl, more preferably

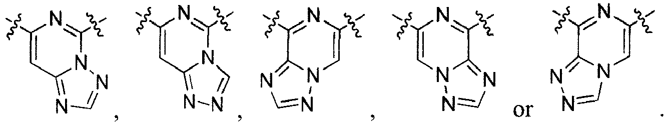

In some technical solutions of the present disclosure, the compound of formula (I) may be any one of the following structures,

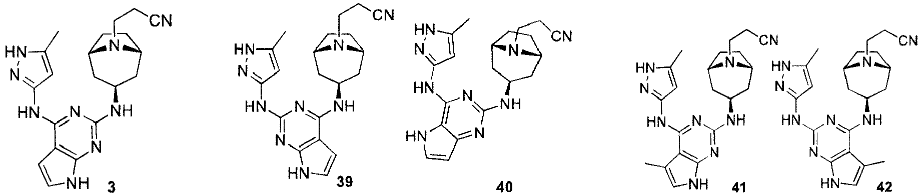

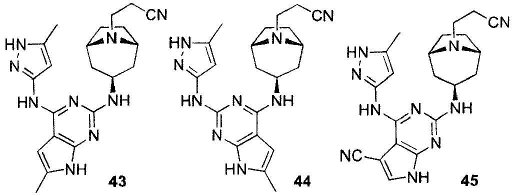

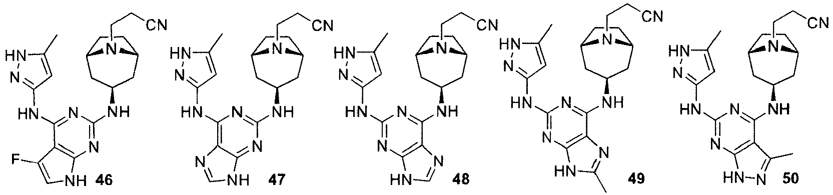

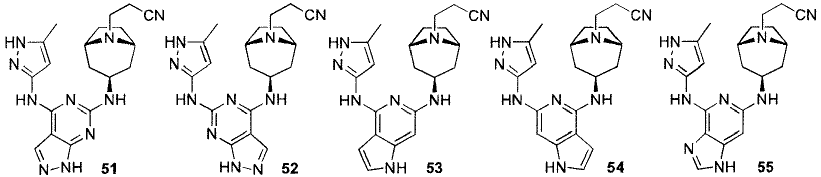

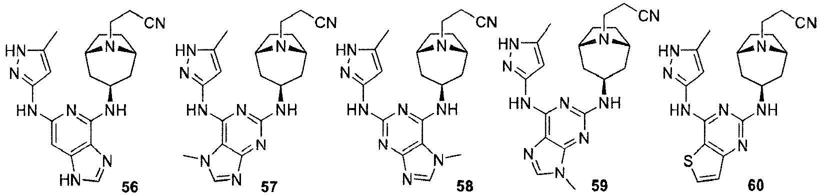

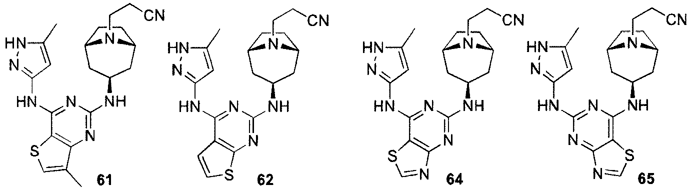

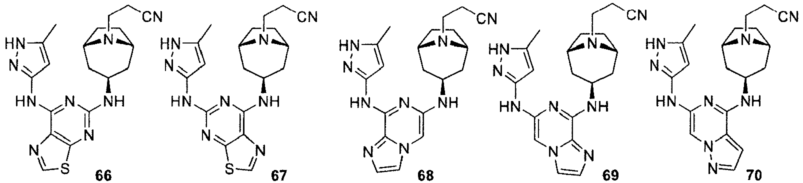

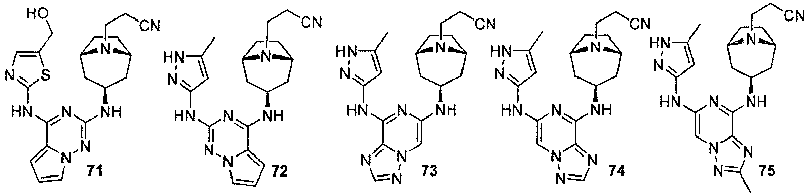

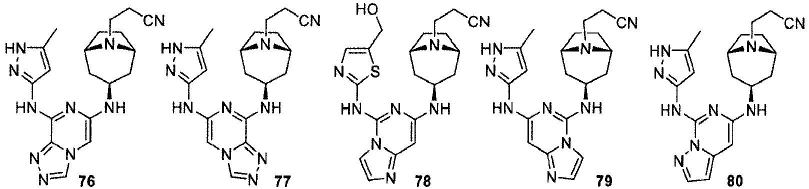

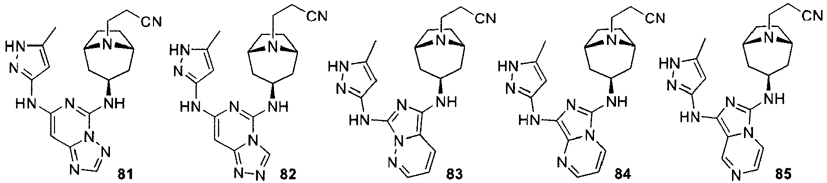

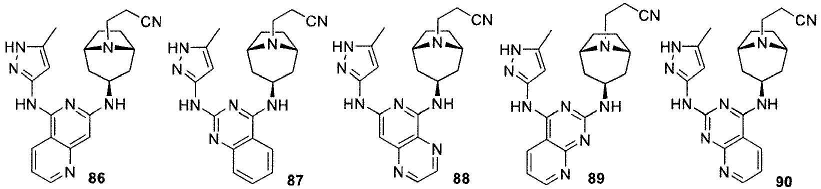

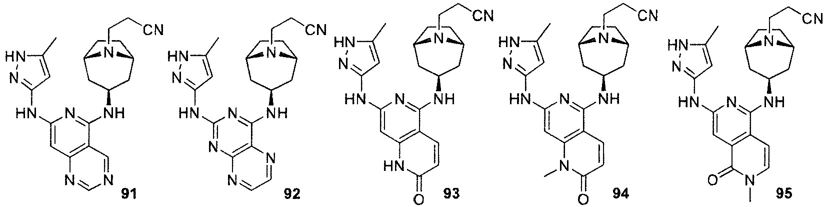

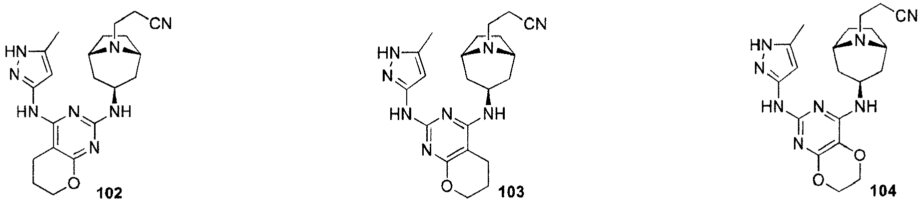

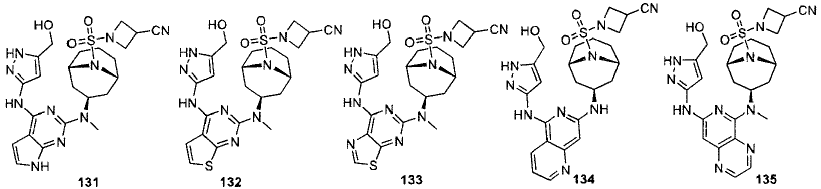

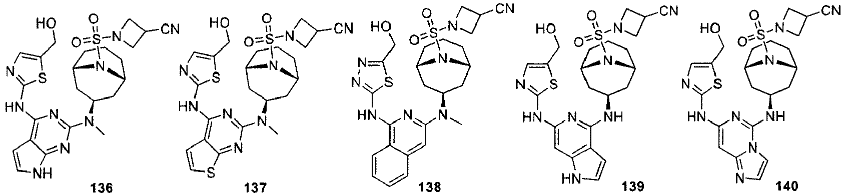

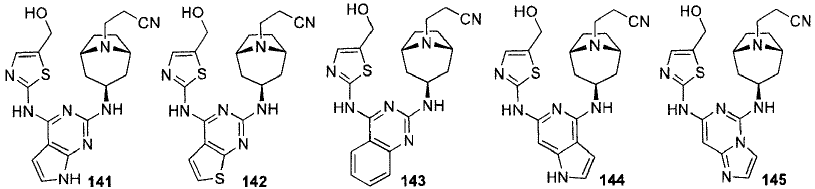

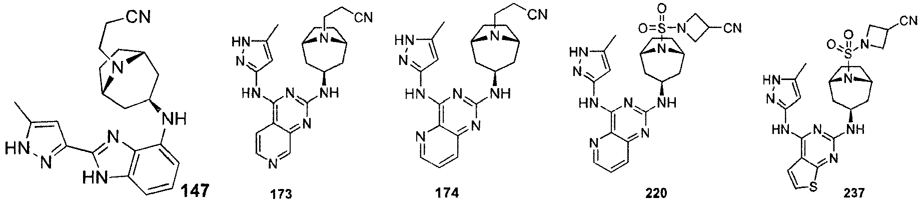

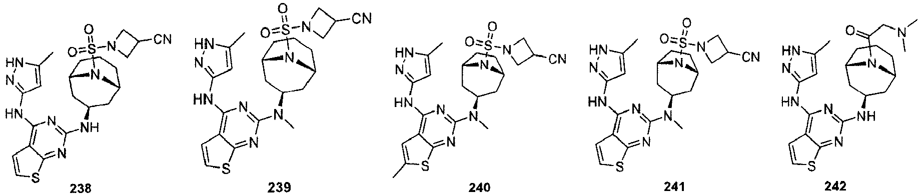

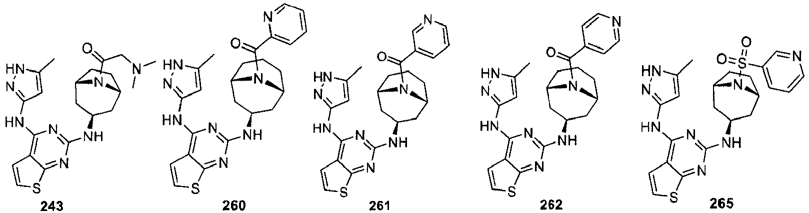

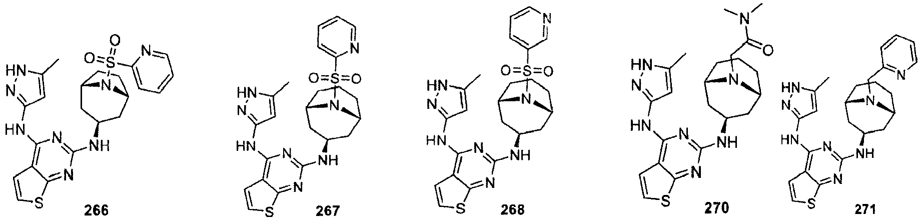

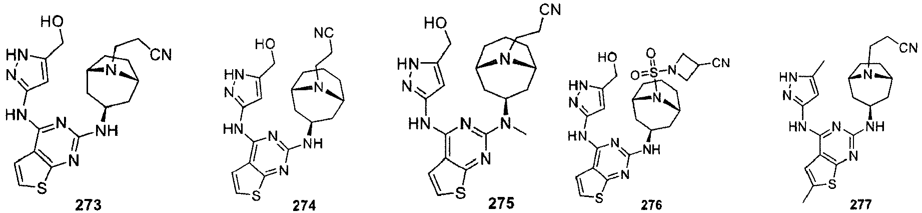

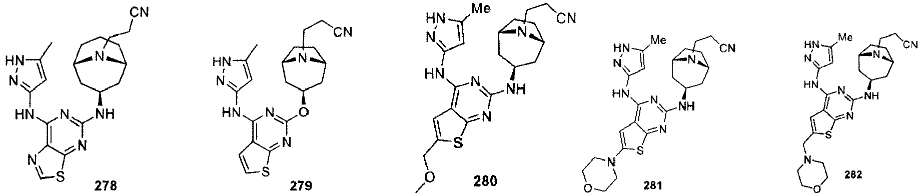

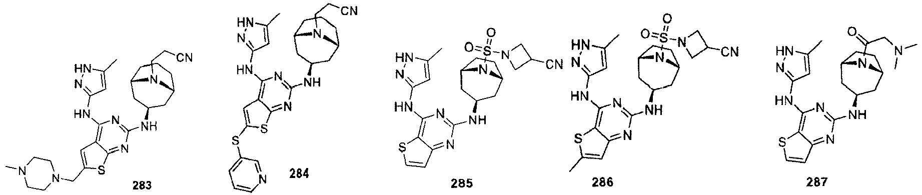

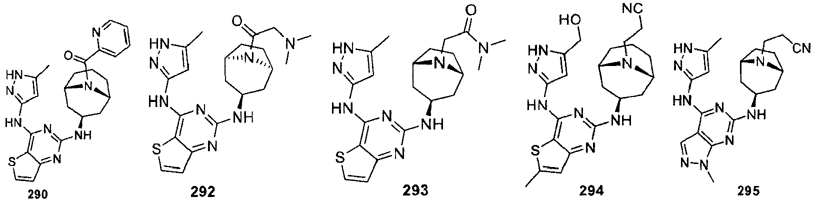

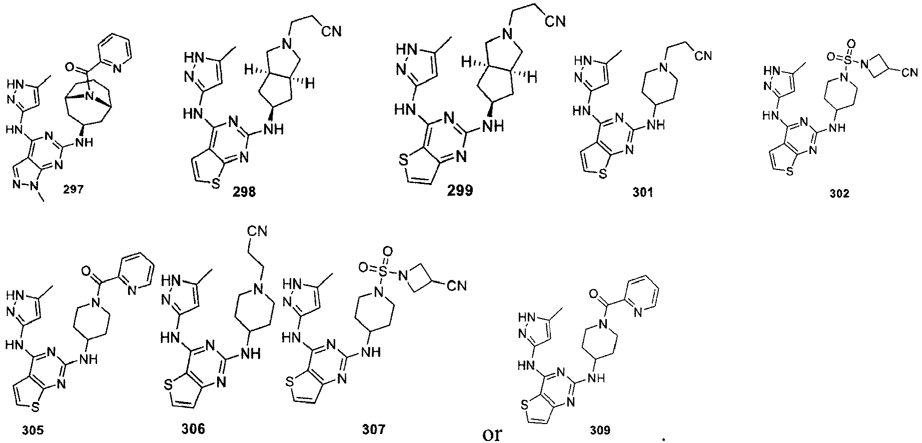

In some technical solutions of the present disclosure, the compound of formula (I) is preferably a compound of formula (I-1), 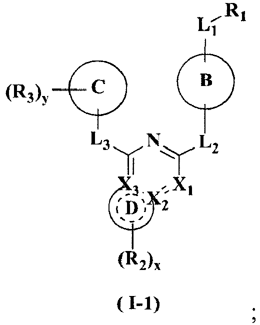
wherein, ring D is heterocycloalkenyl, aryl, or heteroaryl;
X₁, X₂ and X₃ are independently CH or N;
L₁ is selected from alkylene, -(CH₂)ₙ₁C(O)(CH₂)ₘ₁-,-(CH₂)ₙ₁C(O)(CH₂)ₘ₁NRₐₐ(CH₂)ₘ₂-, or -(CH₂)ₙ₁S(O)ₘ₁-;
R₁ is selected from halogen, cyano, alkyl, alkoxy, cycloalkyl, heterocyclyl, or heteroaryl; the alkyl is optionally further substituted with halogen or alkoxy; the heterocyclyl is optionally further substituted with cyano; the heteroaryl is optionally further substituted with alkyl;
when L₁ is -(CH₂)ₙ₁C(O)(CH₂)ₘ₁-, R₁ is alkyl, heterocyclyl, or heteroaryl; the alkyl is optionally further substituted with halogen; the heterocyclyl or heteroaryl is optionally further substituted with alkyl;
L₂, L₃, R₂, R₃, ring B, ring C, x and y are as defined above.

In some technical solutions of the present disclosure, in the compound of general formula (1-1), certain groups are as defined below (unspecified groups are as defined above):
ring D is heterocycloalkenyl, aryl, or heteroaryl;
X₁, X₂ and X₃ are independently CH or N;
L₁ is selected from alkylene, -(CH₂)ₙ₁C(O)(CH₂)ₘ₁-,-(CH₂)ₙ₁C(O)(CH₂)ₘ₁NRₐₐ(CH₂)ₘ₂-, or -(CH₂)ₙ₁S(O)ₘ₁-;
R₁ is selected from halogen, cyano, alkyl, alkoxy, cycloalkyl, heterocyclyl, or heteroaryl; the alkyl is optionally further substituted with halogen or alkoxy; the heterocyclyl is optionally further substituted with cyano; the heteroaryl is optionally further substituted with alkyl;
when L₁ is -(CH₂)ₙ₁C(O)(C_{H2})ₘ₁-, R₁ is alkyl, heterocyclyl, or heteroaryl; the alkyl is optionally further substituted with halogen; the heterocyclyl or heteroaryl is optionally further substituted with alkyl;
L₂ is selected from oxygen atom, or -NR₄-;
R₄ is selected from hydrogen atom, or alkyl;
L₃ is -NH-;
ring B is selected from 3-10 membered monocyclic heterocyclyl, 6-14 membered fused heterocyclyl, or 6-14 membered bridged heterocyclyl;
ring C is heteroaryl;
R₂ is selected from hydrogen atom, alkyl, alkoxy, halogen, cycloalkyl, heterocyclyl, aryl, heteroaryl, -(CH₂)ₙ₁OR_{cc}, or -(CH₂)ₙ₁NRₐₐR_{bb}; the alkyl is optionally further substituted with heterocyclyl unsubstituted or substituted with alkyl; the alkoxy is optionally further substituted with unsubstituted alkoxy, heterocyclyl unsubstituted or substituted with alkyl, or unsubstituted heteroaryl; the heterocyclyl is optionally further substituted with unsubstituted alkyl, unsubstituted alkoxy, or amino substituted with alkyl; the heteroaryl is optionally further substituted with alkyl unsubstituted or substituted with halogen, unsubstituted alkoxy, or unsubstituted cycloalkyl;
Rₐₐ and R_{bb} are identical or different, and are each independently selected from hydrogen atom, or alkyl; the alkyl is optionally further substituted with heterocyclyl unsubstituted or substituted with alkyl, or unsubstituted heteroaryl;
R_{cc} is heterocyclyl; the heterocyclyl is optionally further substituted with unsubstituted alkyl;
R₃ is selected from hydrogen atom, alkyl, or hydroxyalkyl.
In some technical solutions of the present disclosure, in the compound of general formula (1-1), certain groups are as defined below (unspecified groups are as defined above):
ring D is heterocycloalkenyl, aryl, or heteroaryl;
X₁, X₂ and X₃ are independently CH or N;
L₁ is selected from alkylene, -(CH₂)ₙ₁C(O)(CH₂)ₘ₁-,-(CH₂)ₙ₁C(O)(CH₂)ₘ₁NRₐₐ(CH₂)ₘ₂-, or -(CH₂)ₙ₁S(O)ₘ₁-;
R₁ is selected from halogen, cyano, alkyl, heterocyclyl, or heteroaryl; the alkyl is optionally further substituted with halogen or alkoxy; the heterocyclyl is optionally further substituted with cyano; the heteroaryl is optionally further substituted with alkyl;
when L₁ is -(CH₂)ₙ₁C(O)(CH₂)ₘ₁-, R₁ is alkyl, heterocyclyl, or heteroaryl; the alkyl is optionally further substituted with halogen; the heterocyclyl or heteroaryl is optionally further substituted with alkyl;
L₂ is selected from oxygen atom, or -NR₄-;
R₄ is selected from hydrogen atom, or alkyl;
L₃ is -NH-;
ring B is selected from 3-10 membered monocyclic heterocyclyl, 6-14 membered fused heterocyclyl, or 6-14 membered bridged heterocyclyl;
ring C is heteroaryl;
R₂ is selected from hydrogen atom, alkyl, alkoxy, halogen, cycloalkyl, heterocyclyl, aryl, or heteroaryl; the heteroaryl is optionally further substituted with unsubstituted alkyl, unsubstituted alkoxy, or unsubstituted cycloalkyl;
Rₐₐ is selected from hydrogen atom, or alkyl;
R₃ is selected from hydrogen atom, alkyl, or hydroxyalkyl.

In some technical solutions of the present disclosure, in the compound of general formula (I-1), certain group is as defined below (unspecified groups are as defined above): ring D is heterocycloalkenyl, aryl, or heteroaryl.

In some technical solutions of the present disclosure, in the compound of general formula (I-1), certain groups are as defined below (unspecified groups are as defined above): X₁, X₂ and X₃ are independently CH or N.

In some technical solutions of the present disclosure, in the compound of general formula (I-1), certain group is as defined below (unspecified groups are as defined above): L₁ is selected from alkylene, -(CH₂)ₙ₁C(O)(CH₂)ₘ₁-, -(CH₂)ₙ₁C(O)(CH₂)ₘ₁NRₐₐ(CH₂)ₘ₂-, or-(CH₂)ₙ₁S(O)ₘ₁-.

In some technical solutions of the present disclosure, in the compound of general formula (I-1), certain groups are as defined below (unspecified groups are as defined above): R₁ is selected from halogen, cyano, alkyl, heterocyclyl, or heteroaryl; the alkyl is optionally further substituted with halogen or alkoxy; the heterocyclyl is optionally further substituted with cyano; the heteroaryl is optionally further substituted with alkyl.

In some technical solutions of the present disclosure, in the compound of general formula (I-1), certain groups are as defined below (unspecified groups are as defined above): when L₁ is -(CH₂)ₙ₁C(O)(CH₂)ₘ₁-, R₁ is alkyl, heterocyclyl, or heteroaryl; the alkyl is optionally further substituted with halogen; the heterocyclyl or heteroaryl is optionally further substituted with alkyl.

In some technical solutions of the present disclosure, in the compound of general formula (I-1), certain groups are as defined below (unspecified groups are as defined above): L₂ is selected from oxygen atom, or -NR₄-.

In some technical solutions of the present disclosure, in the compound of general formula (I-1), certain group is as defined below (unspecified groups are as defined above): R₄ is selected from hydrogen atom, or alkyl.

In some technical solutions of the present disclosure, in the compound of general formula (I-1), certain group is as defined below (unspecified groups are as defined above): L₃ is -NH-.

In some technical solutions of the present disclosure, in the compound of general formula (I-1), certain group is as defined below (unspecified groups are as defined above): ring B is selected from 3-10 membered monocyclic heterocyclyl, 6-14 membered fused heterocyclyl, or 6-14 membered bridged heterocyclyl.

In some technical solutions of the present disclosure, in the compound of general formula (I-1), certain group is as defined below (unspecified groups are as defined above): ring C is heteroaryl.

In some technical solutions of the present disclosure, in the compound of general formula (I-1), certain group is as defined below (unspecified groups are as defined above): R₂ is selected from hydrogen atom, alkyl, alkoxy, halogen, cycloalkyl, heterocyclyl, aryl, or heteroaryl; the heteroaryl is optionally further substituted with unsubstituted alkyl, unsubstituted alkoxy, or unsubstituted cycloalkyl.

In some technical solutions of the present disclosure, in the compound of general formula (1-1), certain group is as defined below (unspecified groups are as defined above): Rₐₐ is selected from hydrogen atom, or alkyl.

In some technical solutions of the present disclosure, in the compound of general formula (I-1), certain group is as defined below (unspecified groups are as defined above): R₃ is selected from hydrogen atom, alkyl, or hydroxyalkyl.

In some technical solutions of the present disclosure, in the compound of general formula (I-1), certain group is as defined below (unspecified groups are as defined above): when ring D is heterocycloalkenyl, the heterocycloalkenyl is preferably 3-8 membered heterocycloalkenyl with "heteroatom selected from one or more of N, O and S, and the number of the heteroatom being 1-3", more preferably 4-6 membered heterocycloalkenyl with "heteroatom selected from one or more of N, O and S, and the number of the heteroatom being 1-3", *e.g.,*

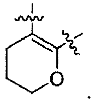

In some technical solutions of the present disclosure, in the compound of general formula (1-1), certain group is as defined below (unspecified groups are as defined above): when ring D is aryl, the aryl is preferably 6-10 membered aryl, more preferably phenyl.

In some technical solutions of the present disclosure, in the compound of general formula (I-1), certain group is as defined below (unspecified groups are as defined above): when ring D is heteroaryl, the heteroaryl is preferably 5-10 membered heteroaryl with "heteroatom selected from one or more of N, O and S, and the number of the heteroatom being 1-4", more preferably 5-6 membered heteroaryl with "heteroatom selected from one or more of N, O and S, and the number of the heteroatom being 1-3", *e.g.,* 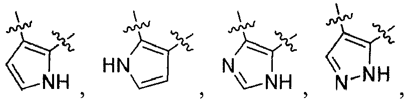 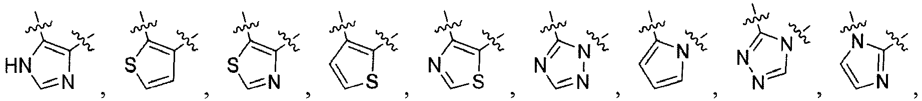 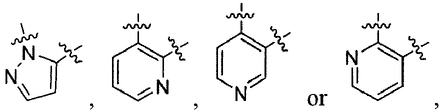 more preferably

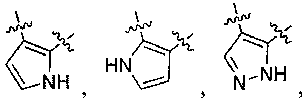

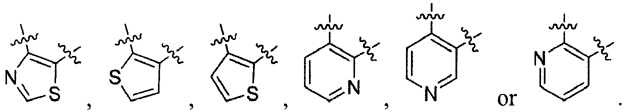

In some technical solutions of the present disclosure, the compound of formula (I) is preferably a compound of formula (1-2), 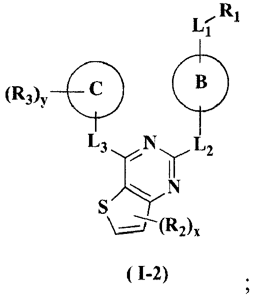
wherein, L₁, L₂, L₃, R₁, R₂, R₃, ring B, ring C, y are as defined above;
x is 0, 1 or 2.
In some technical solutions of the present disclosure, in the compound of general formula (I-2), certain group is as defined below (unspecified groups are as defined above):
L₁ is alkylene, -(CH₂)ₙ₁C(O)(CH₂)ₘ₁-, -(CH₂)ₙ₁C(O)(CH₂)ₘ₁NRₐₐ(CH₂)ₘ₂-, or - (CH₂)ₙ₁S(O)ₘ₁-;
Rₐₐ is selected from hydrogen atom, or alkyl;
L₂ is -NH-;
L₃ is -NH-;
ring B is selected from 3-10 membered monocyclic heterocyclyl, 6-14 membered bridged heterocyclyl, or 6-14 membered fused heterocyclyl;
ring C is heteroaryl;
R₁ is alkyl, cyano, heterocyclyl, or heteroaryl; the heterocyclyl is optionally further substituted with cyano;
R₂ is selected from hydrogen atom, or alkyl;
R₃ is selected from hydrogen atom, alkyl, or hydroxyalkyl;
x is 0, 1 or 2.

In some technical solutions of the present disclosure, in the compound of general formula (I-2), certain groups are as defined below (unspecified groups are as defined above):
L₁ is alkylene, -(CH₂)ₙ₁C(O)(CH₂)ₘ₁-, -(CH₂)ₙ₁C(O)(CH₂)ₘ₁NRₐₐ(CH₂)ₘ₂-, - (CH₂)ₙ₁S(O)ₘ₁-;
Rₐₐ is selected from hydrogen atom, or alkyl;
L₂ is -NH-;
L₃ is -NH-;
ring B is selected from 6-14 membered bridged heterocyclyl, or 6-14 membered fused heterocyclyl;
ring C is heteroaryl;
R₁ is alkyl, cyano, or heterocyclyl;
R₂ is selected from hydrogen atom, or alkyl;
R₃ is selected from hydrogen atom, alkyl, or hydroxyalkyl;
x is 0, 1 or 2.

In some technical solutions of the present disclosure, in the compound of general formula (1-2), certain groups are as defined below (unspecified groups are as defined above):
L₁ is C₁-C₄ alkylene, -(CH₂)ₙ₁C(O)(CH₂)ₘ₁-, -(CH₂)ₙ₁C(O)(CH₂)ₘ₁NRₐₐ(CH₂)ₘ₂-, - (CH₂)ₙ₁S(O)ₘ₁-;
Rₐₐ is selected from hydrogen atom, or C₁-C₃ alkyl;
L₂ is -NH-;
L₃ is -NH-;
ring B is selected from 7-10 membered bridged heterocyclyl with "heteroatom selected from one or more of N, O and S, and the number of the heteroatom being 1-3", or 7-10 membered fused heterocyclyl with "heteroatom selected from one or more of N, O and S, and the number of the heteroatom being 1-3";
ring C is 5-6 membered monocyclic heteroaryl with "heteroatom selected from one or more of N, O and S, and the number of the heteroatom being 1-3";
R₁ is C₁-C₄ alkyl, cyano, or 4-6 membered monocyclic heterocyclyl with "heteroatom selected from one or more of N, O and S, and the number of the heteroatom being 1-3";
R₂ is selected from hydrogen atom, or C₁-C₃ alkyl;
R₃ is selected from hydrogen atom, C₁-C₃ alkyl, or C₁-C₃ hydroxyalkyl;
x is 0, 1 or 2.

In some technical solutions of the present disclosure, in the compound of general formula (1-2), certain group is as defined below (unspecified groups are as defined above): when L₁ is alkylene, the alkylene is preferably C₁-C₄ alkylene, *e.g.,* -CH₂-, -(CH₂)₂-, -(CH₂)₃- or - (CH₂)₄-, more preferably -CH₂-, or -(CH₂)₂-.

In some technical solutions of the present disclosure, in the compound of general formula (I-2), certain group is as defined below (unspecified groups are as defined above): when L₁ is -(CH₂)ₙ₁C(O)(CH₂)ₘ₁-, the -(CH₂)ₙ₁C(O)(CH₂)ₘ₁- is preferably -C(O)-, -C(O)CH₂-, or - CH₂C(O)-.

In some technical solutions of the present disclosure, in the compound of general formula (1-2), certain group is as defined below (unspecified groups are as defined above): when L₁ is -(CH₂)ₙ₁C(O)(CH₂)ₘ₁NRₐₐ(CH₂)ₘ₂-, the -(CH₂)ₙ₁C(O)(CH₂)ₘ₁NRₐₐ(CH₂)ₘ₂- is preferably - C(O)CH₂NH-, -C(O)CH₂N(CH₃)-, or -CH₂C(O)N(CH₃)-.

In some technical solutions of the present disclosure, in the compound of general formula (1-2), certain group is as defined below (unspecified groups are as defined above): when L₁ is -(CH₂)ₙ₁S(O)ₘ₁-, the -(CH₂)ₙ₁S(O)ₘ₁- is preferably -S(O)₂-.

In some technical solutions of the present disclosure, in the compound of general formula (I-2), certain group is as defined below (unspecified groups are as defined above): when Rₐₐ is alkyl, the alkyl is preferably C₁-C₈ alkyl, more preferably C₁-C₆ alkyl, further preferably C₁-C₃ alkyl, *e.g.,* methyl, ethyl, propyl or isopropyl, further preferably methyl.

In some technical solutions of the present disclosure, in the compound of general formula (1-2), certain group is as defined below (unspecified groups are as defined above): when the ring B is 3-10 membered monocyclic heterocyclyl, the 3-10 membered monocyclic heterocyclyl is preferably 3-8 membered monocyclic heterocyclyl with "heteroatom selected from one or more of N, O and S, and the number of the heteroatom being 1-4", more preferably 4-6 membered monocyclic heterocyclyl with "heteroatom selected from one or more of N, O and S, and the number of the heteroatom being 1-3", *e.g.,*

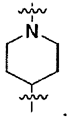

In some technical solutions of the present disclosure, in the compound of general formula (1-2), certain group is as defined below (unspecified groups are as defined above): when the ring B is 6-14 membered bridged heterocyclyl, the 6-14 membered bridged heterocyclyl is preferably 6-14 membered bridged heterocyclyl with "heteroatom selected from one or more of N, O and S, and the number of the heteroatom being 1-4", more preferably 7-10 membered bridged heterocyclyl with "heteroatom selected from one or more of N, O and S, and the number of the heteroatom being 1-3", *e.g.,*

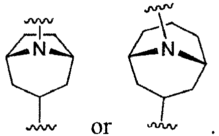

In some technical solutions of the present disclosure, in the compound of general formula (1-2), certain group is as defined below (unspecified groups are as defined above): when the ring B is 6-14 membered fused heterocyclyl, the 6-14 membered fused heterocyclyl is preferably 6-14 membered fused heterocyclyl with "heteroatom selected from one or more of N, O and S, and the number of the heteroatom being 1-4", more preferably 7-10 membered fused heterocyclyl with "heteroatom selected from one or more of N, O and S, and the number of the heteroatom being 1-3", *e.g.,*

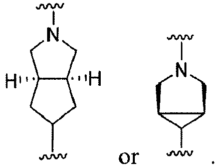

In some technical solutions of the present disclosure, in the compound of general formula (I-2), certain group is as defined below (unspecified groups are as defined above): when the ring C is heteroaryl, the heteroaryl is preferably 6-14 membered heteroaryl with "heteroatom selected from one or more of N, O and S, and the number of the heteroatom being 1-4", more preferably 5-6 membered monocyclic heteroaryl with "heteroatom selected from one or more of N, O and S, and the number of the heteroatom being 1-3", or 8-10 membered fused heteroaryl with "heteroatom selected from one or more of N, O and S, and the number of the heteroatom being 1-3". The 5-6 membered monocyclic heteroaryl is preferably

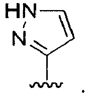

In some technical solutions of the present disclosure, in the compound of general formula (1-2), certain group is as defined below (unspecified groups are as defined above): when R₁ is alkyl, the alkyl is preferably C₁-C₈ alkyl, more preferably C₁-C₆ alkyl, further preferably C₁-C₃ alkyl, *e.g.*, methyl, ethyl, propyl or isopropyl, further preferably methyl.

In some technical solutions of the present disclosure, in the compound of general formula (I-2), certain group is as defined below (unspecified groups are as defined above): when R₁ is heterocyclyl, the heterocyclyl is preferably 3-8 membered monocyclic heterocyclyl with "heteroatom selected from one or more of N, O and S, and the number of the heteroatom being 1-4", more preferably 4-6 membered monocyclic heterocyclyl with "heteroatom selected from one or more of N, O and S, and the number of the heteroatom being 1-3", *e*.*g*., 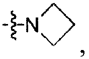 or

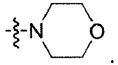

In some technical solutions of the present disclosure, in the compound of general formula (1-2), certain group is as defined below (unspecified groups are as defined above): when R₁ is heteroaryl, the heteroaryl is preferably 5-10 membered heteroaryl with "heteroatom selected from one or more of N, O and S, and the number of the heteroatom being 1-4", more preferably 5-6 membered heteroaryl with "heteroatom selected from one or more of N O and S, and the number of the heteroatom being 1-3", *e.g.,*

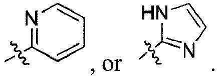

In some technical solutions of the present disclosure, in the compound of general formula (1-2), certain group is as defined below (unspecified groups are as defined above): when R₂ is alkyl, the alkyl is preferably C₁-C₈ alkyl, more preferably C₁-C₆ alkyl, further preferably C₁-C₃ alkyl, *e.g.*, methyl, ethyl, propyl or isopropyl, further preferably methyl.

In some technical solutions of the present disclosure, in the compound of general formula (1-2), certain group is as defined below (unspecified groups are as defined above): when R₃ is alkyl, the alkyl is preferably C₁-C₈ alkyl, more preferably C₁-C₆ alkyl, further preferably C₁-C₃ alkyl, *e.g.*, methyl, ethyl, propyl or isopropyl, further preferably methyl.

In some technical solutions of the present disclosure, in the compound of general formula (1-2), certain group is as defined below (unspecified groups are as defined above): when R₃ is hydroxyalkyl, the alkyl is preferably C₁-C₈ hydroxyalkyl, more preferably C₁-C₆ hydroxyalkyl, further preferably C₁-C₃ hydroxyalkyl, *e*.*g*., hydroxymethyl, hydroxyethyl, hydroxypropyl or hydroxyisopropyl, further preferably hydroxymethyl.

In some technical solutions of the present disclosure, the compound of formula (I) is preferably a compound of formula (I-3), 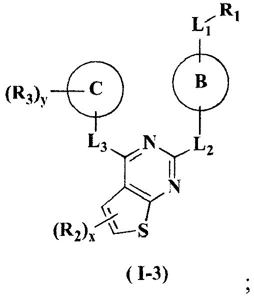
wherein, L₁, L₂, L₃, R₁, R₂, R₃, ring B, ring C, y are as defined above;
x is 0, 1 or 2.

In some technical solutions of the present disclosure, in the compound of general formula (1-3), certain groups are as defined below (unspecified groups are as defined above):
L₁ is alkylene, -(CH₂)ₙ₁C(O)(CH₂)ₘ₁-, -(CH₂)ₙ₁C(O)(CH₂)ₘ₁NRₐₐ(CH₂)ₘ₂-, or - (CH₂)ₙ₁S(O)ₘ₁-;
L₂ is oxygen atom, or -NR₄-;
R₄ is selected from hydrogen atom, or alkyl;
L₃ is -NH-;
ring B is selected from 3-10 membered monocyclic heterocyclyl, 6-14 membered bridged heterocyclyl, or 6-14 membered fused heterocyclyl;
ring C is heteroaryl;
R₁ is hydrogen atom, alkyl, alkoxy, cyano, cycloalkyl, heterocyclyl, aryl, or heteroaryl; the heterocyclyl, or heteroaryl is optionally further substituted with unsubstituted alkyl, or cyano; the aryl is optionally further substituted with unsubstituted alkoxy;
R₂ is selected from hydrogen atom, alkyl, heterocyclyl, or -(CH₂)ₙ₁SRₐₐ; the alkyl is optionally further substituted with unsubstituted alkoxy, or heterocyclyl unsubstituted or substituted with alkyl; the heterocyclyl is optionally further substituted with alkyl;
R₃ is selected from hydrogen atom, alkyl, or hydroxyalkyl;
Rₐₐ is selected from hydrogen atom, or alkyl;
x is 0, 1 or 2.

In some technical solutions of the present disclosure, in the compound of general formula (1-3), certain groups are as defined below (unspecified groups are as defined above):
L₁ is alkylene, -(CH₂)ₙ₁C(O)(CH₂)ₘ₁-, -(CH₂)ₙ₁C(O)(CH₂)ₘ₁NRₐₐ(CH₂)ₘ₂-, or - (CH₂)ₙ₁S(O)ₘ₁-;
L₂ is oxygen atom, or -NR₄-;
R₄ is selected from hydrogen atom, or alkyl;
L₃ is -NH-;
ring B is selected from 3-10 membered monocyclic heterocyclyl, 6-14 membered bridged heterocyclyl, or 6-14 membered fused heterocyclyl;
ring C is heteroaryl;
R₁ is hydrogen atom, alkyl, cyano, heterocyclyl, or heteroaryl; the heterocyclyl or heteroaryl is optionally further substituted with unsubstituted alkyl, or cyano;
R₂ is selected from hydrogen atom, or alkyl;
R₃ is selected from hydrogen atom, alkyl, or hydroxyalkyl;
Rₐₐ is selected from hydrogen atom, or alkyl;
x is 0, 1 or 2.

In some technical solutions of the present disclosure, in the compound of general formula (1-3), certain groups are as defined below (unspecified groups are as defined above):
L₁ is C₁-C₄ alkylene, -(CH₂)ₙ₁C(O)(CH₂)ₘ₁-, -(CH₂)ₙ₁C(O)(CH₂)ₘ₁NRₐₐ(CH₂)ₘ₂-, or - (CH₂)ₙ₁S(O)ₘ₁-;
L₂ is oxygen atom, or -NR₄-;
R₄ is selected from hydrogen atom, or C₁-C₃ alkyl;
L₃ is -NH-;
ring B is selected from 4-6 membered monocyclic heterocyclyl with "heteroatom selected from one or more of N, O and S, and the number of the heteroatom being 1-3", 7-10 membered bridged heterocyclyl with "heteroatom selected from one or more of N, O and S, and the number of the heteroatom being 1-3", or 7-10 membered fused heterocyclyl with "heteroatom selected from one or more of N, O and S, and the number of the heteroatom being 1-3";
ring C is 5-6 membered monocyclic heteroaryl with "heteroatom selected from one or more of N, O and S, and the number of the heteroatom being 1-3";
R₁ is hydrogen atom, C₁-C₃ alkyl, cyano, 4-6 membered monocyclic heterocyclyl with "heteroatom selected from one or more of N, O and S, and the number of the heteroatom being 1-3", or 5-6 membered heteroaryl with "heteroatom selected from one or more of N, O and S, and the number of the heteroatom being 1-3"; the monocyclic heterocyclyl, or heteroaryl is optionally further substituted with C₁-C₃ alkyl, or cyano;
R₂ is selected from hydrogen atom, or C₁-C₃ alkyl;
R₃ is selected from hydrogen atom, C₁-C₃ alkyl, or C₁-C₃ hydroxyalkyl;
Rₐₐ is selected from hydrogen atom, or C₁-C₃ alkyl;
x is 0, 1 or 2.
preferably:
L₁ is alkylene, -(CH₂)ₙ₁C(O)(CH₂)ₘ₁-, or -(CH₂)ₙ₁C(O)(CH₂)ₘ₁NRₐₐ(CH₂)ₘ₂-;
R₁ is alkyl, cyano, heterocyclyl or heteroaryl; the heterocyclyl is optionally further substituted with alkyl;
L₂ is oxygen atom, or -NR₄-;
R₄ is selected from hydrogen atom, or alkyl;
L₃ is -NH-;
ring B is 6-14 membered bridged heterocyclyl;
ring C is heteroaryl;
R₂ is selected from hydrogen atom, or alkyl;
R₃ is selected from hydrogen atom, or alkyl;
Rₐₐ is selected from hydrogen atom, or alkyl.

In some technical solutions of the present disclosure, in the compound of general formula (1-3), certain group is as defined below (unspecified groups are as defined above): when L₁ is alkylene, the alkylene is preferably C₁-C₄ alkylene, *e.g.*, -CH₂-, -(CH₂)₂-, -(CH₂)₃- or - (CH₂)₄-, more preferably -CH₂-, or -(CH₂)₂-.

In some technical solutions of the present disclosure, in the compound of general formula (1-3), certain group is as defined below (unspecified groups are as defined above): when L₁ is -(CH₂)ₙ₁C(O)(CH₂)ₘ₁-, the -(CH₂)ₙ₁C(O)(CH₂)ₘ₁- is preferably -C(O)-, -C(O)CH₂-, or - CH₂C(O)-.

In some technical solutions of the present disclosure, in the compound of general formula (1-3), certain group is as defined below (unspecified groups are as defined above): when L₁ is -(CH₂)ₙ₁C(O)(CH₂)ₘ₁NRₐₐ(CH₂)ₘ₂-, the -(CH₂)ₙ₁C(O)(CH₂)ₘ₁NRₐₐ(CH₂)ₘ₂- is preferably - C(O)CH₂NH-, -C(O)CH₂N(CH₃)-, -C(O)CH₂NHCH₂-, -C(O)CH₂NH(CH₂)₂-, or - CH₂C(O)N(CH₃)-.

In some technical solutions of the present disclosure, in the compound of general formula (1-3), certain group is as defined below (unspecified groups are as defined above): when L₁ is -(CH₂)ₙ₁S(O)ₘ₁-, the -(CH₂)ₙ₁S(O)ₘ₁- is preferably -S(O)₂-.

In some technical solutions of the present disclosure, in the compound of general formula (1-3), certain group is as defined below (unspecified groups are as defined above): when R₄ is alkyl, the alkyl is preferably C₁-C₈ alkyl, more preferably C₁-C₆ alkyl, further preferably C₁-C₃ alkyl, *e.g.*, methyl, ethyl, propyl or isopropyl, further preferably methyl.

In some technical solutions of the present disclosure, in the compound of general formula (1-3), certain group is as defined below (unspecified groups are as defined above): when the ring B is 3-10 membered monocyclic heterocyclyl, the 3-10 membered monocyclic heterocyclyl is preferably 3-8 membered monocyclic heterocyclyl with "heteroatom selected from one or more of N, O and S, and the number of the heteroatom being 1-4", more preferably 4-6 membered monocyclic heterocyclyl with "heteroatom selected from one or more of N, O and S, and the number of the heteroatom being 1-3", *e.g.,*

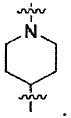

In some technical solutions of the present disclosure, in the compound of general formula (1-3), certain group is as defined below (unspecified groups are as defined above): when the ring B is 6-14 membered bridged heterocyclyl, the 6-14 membered bridged heterocyclyl is preferably 6-14 membered bridged heterocyclyl with "heteroatom selected from one or more of N, O and S, and the number of the heteroatom being 1-4", more preferably 7-10 membered bridged heterocyclyl with "heteroatom selected from one or more of N, O and S, and the number of the heteroatom being 1-3", *e.g.,*

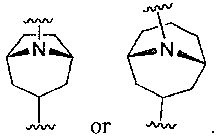

In some technical solutions of the present disclosure, in the compound of general formula (1-3), certain group is as defined below (unspecified groups are as defined above): when the ring B is 6-14 membered fused heterocyclyl, the 6-14 membered fused heterocyclyl is preferably 6-14 membered fused heterocyclyl with "heteroatom selected from one or more of N, O and S, and the number of the heteroatom being 1-4", more preferably 7-10 membered fused heterocyclyl with "heteroatom selected from one or more of N, O and S, and the number of the heteroatom being 1-3", *e.g.,*

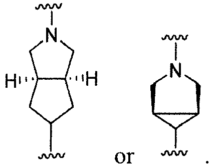

In some technical solutions of the present disclosure, in the compound of general formula (1-3), certain group is as defined below (unspecified groups are as defined above): when the ring C is heteroaryl, the heteroaryl is preferably 6-14 membered heteroaryl with "heteroatom selected from one or more of N, O and S, and the number of the heteroatom being 1-4", more preferably 5-6 membered monocyclic heteroaryl with "heteroatom selected from one or more of N, O and S, and the number of the heteroatom being 1-3", *e.g.,*

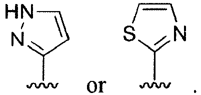

In some technical solutions of the present disclosure, in the compound of general formula (1-3), certain group is as defined below (unspecified groups are as defined above): when R₁ is alkyl, the alkyl is preferably C₁-C₈ alkyl, more preferably C₁-C₆ alkyl, further preferably C₁-C₃ alkyl, *e.g.*, methyl, ethyl, propyl or isopropyl, further preferably methyl or ethyl.

In some technical solutions of the present disclosure, in the compound of general formula (1-3), certain group is as defined below (unspecified groups are as defined above): when R₁ is alkoxy, the alkoxy is preferably C₁-C₈ alkoxy, more preferably C₁-C₆ alkoxy, further preferably C₁-C₃ alkoxy, *e.g.*, methoxy, ethoxy, propoxy or isopropoxy, further preferably methoxy.

In some technical solutions of the present disclosure, in the compound of general formula (1-3), certain group is as defined below (unspecified groups are as defined above): when R₁ is cycloalkyl, the cycloalkyl is preferably C₃-C₈ cycloalkyl, more preferably C₃-C₆ cycloalkyl, *e.g.,*

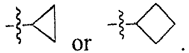

In some technical solutions of the present disclosure, in the compound of general formula (1-3), certain group is as defined below (unspecified groups are as defined above): when R₁ is heterocyclyl, the heterocyclyl is preferably 3-8 membered monocyclic heterocyclyl with "heteroatom selected from one or more of N, O and S, and the number of the heteroatom being 1-4", more preferably 4-6 membered monocyclic heterocyclyl with "heteroatom selected from one or more of N, O and S, and the number of the heteroatom being 1-3", *e.g.,*

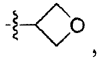

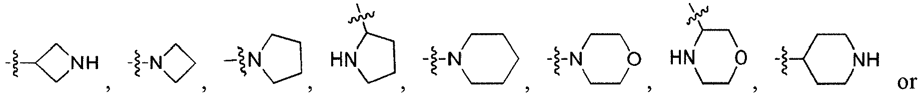

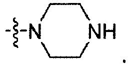

In some technical solutions of the present disclosure, in the compound of general formula (1-3), certain group is as defined below (unspecified groups are as defined above): when R₁ is aryl, the aryl is preferably 6-10 membered aryl, more preferably phenyl.

In some technical solutions of the present disclosure, in the compound of general formula (1-3), certain group is as defined below (unspecified groups are as defined above): when R₁ is heteroaryl, the heteroaryl is preferably 5-10 membered heteroaryl with "heteroatom selected from one or more of N, O and S, and the number of the heteroatom being 1-4", more preferably 5-6 membered heteroaryl with "heteroatom selected from one or more of N, O and S, and the number of the heteroatom being 1-3", *e.g.,*

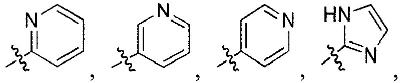

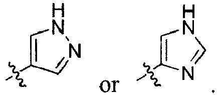

In some technical solutions of the present disclosure, in the compound of general formula (1-3), certain group is as defined below (unspecified groups are as defined above): when the heterocyclyl, or heteroaryl is optionally further substituted with alkyl, the alkyl is preferably C₁-C₈ alkyl, more preferably C₁-C₆ alkyl, further preferably C₁-C₃ alkyl, *e.g.*, methyl, ethyl, propyl or isopropyl, further preferably methyl.

In some technical solutions of the present disclosure, in the compound of general formula (1-3), certain group is as defined below (unspecified groups are as defined above): when the aryl is optionally further substituted with alkoxy, the alkoxy is preferably C₁-C₈ alkoxy, more preferably C₁-C₆ alkoxy, further preferably C₁-C₃ alkoxy, *e.g.*, methoxy, ethoxy, propoxy or isopropoxy, further preferably methoxy.

In some technical solutions of the present disclosure, in the compound of general formula (1-3), certain group is as defined below (unspecified groups are as defined above): when R₂ is alkyl, the alkyl is preferably C₁-C₈ alkyl, more preferably C₁-C₆ alkyl, further preferably C₁-C₃ alkyl, *e.g.*, methyl, ethyl, propyl or isopropyl, further preferably methyl or ethyl.

In some technical solutions of the present disclosure, in the compound of general formula (1-3), certain group is as defined below (unspecified groups are as defined above): when R₂ is heterocyclyl, the heterocyclyl is preferably 3-8 membered monocyclic heterocyclyl with "heteroatom selected from one or more of N, O and S, and the number of the heteroatom being 1-4", more preferably 4-6 membered monocyclic heterocyclyl with "heteroatom selected from one or more of N, O and S, and the number of the heteroatom being 1-3", *e.g.,*

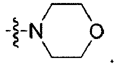

In some technical solutions of the present disclosure, in the compound of general formula (1-3), certain group is as defined below (unspecified groups are as defined above): when R₂ is -(CH₂)ₙ₁SRₐₐ, the -(CH₂)ₙ₁SRₐₐ is preferably

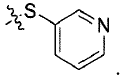

In some technical solutions of the present disclosure, in the compound of general formula (1-3), certain group is as defined below (unspecified groups are as defined above): when R₂ is alkyl, the alkyl is optionally further substituted with unsubstituted alkoxy, the alkoxy is preferably C₁-C₈ alkoxy, more preferably C₁-C₆ alkoxy, further preferably C₁-C₃ alkoxy, *e.g.*, methoxy, ethoxy, propoxy or isopropoxy, further preferably methoxy.

In some technical solutions of the present disclosure, in the compound of general formula (1-3), certain group is as defined below (unspecified groups are as defined above): when R₂ is alkyl, the alkyl is optionally further substituted with substituted or unsubstituted heterocyclyl, the heterocyclyl is preferably 3-8 membered monocyclic heterocyclyl with "heteroatom selected from one or more of N, O and S, and the number of the heteroatom being 1-4", more preferably 4-6 membered monocyclic heterocyclyl with "heteroatom selected from one or more of N, O and S, and the number of the heteroatom being 1-3", *e.g.,* 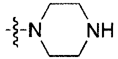 or

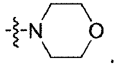

In some technical solutions of the present disclosure, in the compound of general formula (1-3), certain group is as defined below (unspecified groups are as defined above): when the heterocyclyl is optionally further substituted with alkyl, the alkyl is preferably C₁-C₈ alkyl, more preferably C₁-C₆ alkyl, further preferably C₁-C₃ alkyl, *e*.*g*., methyl, ethyl, propyl or isopropyl, further preferably methyl.

In some technical solutions of the present disclosure, in the compound of general formula (1-3), certain group is as defined below (unspecified groups are as defined above): when R3 is alkyl, the alkyl is preferably C₁-C₈ alkyl, more preferably C₁-C₆ alkyl, further preferably C₁-C₃ alkyl, *e.g.*, methyl, ethyl, propyl or isopropyl, further preferably methyl or ethyl.

In some technical solutions of the present disclosure, in the compound of general formula (1-3), certain group is as defined below (unspecified groups are as defined above): when R₃ is hydroxyalkyl, the alkyl is preferably C₁-C₈ hydroxyalkyl, more preferably C₁-C₆ hydroxyalkyl, further preferably C₁-C₃ hydroxyalkyl, *e.g.*, hydroxymethyl, hydroxyethyl, hydroxypropyl or hydroxyisopropyl, further preferably hydroxymethyl.

In some technical solutions of the present disclosure, in the compound of general formula (1-3), certain group is as defined below (unspecified groups are as defined above): when Rₐₐ is alkyl, the alkyl is preferably C₁-C₈ alkyl, more preferably C₁-C₆ alkyl, further preferably C₁-C₃ alkyl, *e.g.*, methyl, ethyl, propyl or isopropyl, further preferably methyl.

In some technical solutions of the present disclosure, the compound of formula (I) is preferably a compound of formula (1-4): 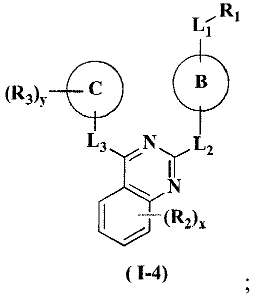
wherein, L₁ is selected from alkylene, -(CH₂)ₙ₁C(O)(CH₂)ₘ₁-, - (CH₂)ₙ₁C(O)(CH₂)ₘ₁NRₐₐ(CH₂)ₘ₂-, or -(CH₂)ₙ₁S(O)ₘ₁-;
R₁ is selected from halogen, cyano, alkyl, alkoxy, heterocyclyl, or heteroaryl; the alkyl is optionally further substituted with halogen or alkoxy; the heterocyclyl is optionally further substituted with cyano;
when L₁ is -(CH₂)ₙ₁C(O)(CH₂)ₘ₁-, R₁ is alkyl, heterocyclyl, or heteroaryl; the alkyl is optionally further substituted with halogen; the heterocyclyl or heteroaryl is optionally further substituted with alkyl;
L₂, L₃, R₂, R₃, ring B, ring C, x and y are as defined above.

In some technical solutions of the present disclosure, in the compound of general formula (1-4), certain groups are as defined below (unspecified groups are as defined above):
L₁ is selected from alkylene, -(CH₂)ₙ₁C(O)(CH₂)ₘ₁-, - (CH₂)ₙ₁C(O)(CH₂)ₘ₁NRₐₐ(CH₂)ₘ₂-, or -(CH₂)ₙ₁S(O)ₘ₁-;
R₁ is selected from halogen, cyano, alkyl, alkoxy, heterocyclyl, or heteroaryl; the alkyl is optionally further substituted with halogen or alkoxy; the heterocyclyl is optionally further substituted with cyano;
when L₁ is -(CH₂)ₙ₁C(O)(CH₂)ₘ₁-, R₁ is alkyl, heterocyclyl, or heteroaryl; the alkyl is optionally further substituted with halogen; the heterocyclyl or heteroaryl is optionally further substituted with alkyl;
L₂ is -NR₄-;
R₄ is selected from hydrogen atom, or alkyl;
L₃ is -NH-;
ring B is selected from 3-10 membered monocyclic heterocyclyl, 6-14 membered bridged heterocyclyl, or 6-14 membered fused heterocyclyl;
ring C is heteroaryl;
R₂ is selected from hydrogen atom, alkyl, alkoxy, halogen, cycloalkyl, heterocyclyl, aryl, heteroaryl, -(CH₂)ₙ₁OR_{cc}, or -(CH₂)ₙ₁NRₐₐR_{bb}; the alkyl is optionally further substituted with heterocyclyl unsubstituted or substituted with alkyl; the alkoxy is optionally further substituted with unsubstituted alkoxy, heterocyclyl unsubstituted or substituted with alkyl, or unsubstituted heteroaryl; the heterocyclyl is optionally further substituted with unsubstituted alkyl, unsubstituted alkoxy, or amino substituted with alkyl; the heteroaryl is optionally further substituted with alkyl unsubstituted or substituted with halogen, unsubstituted alkoxy, or unsubstituted cycloalkyl;
Rₐₐ and R_{bb} are identical or different and are each independently selected from hydrogen atom, or alkyl; the alkyl is optionally further substituted with heterocyclyl unsubstituted or substituted with alkyl, or unsubstituted heteroaryl;
R_{cc} is heterocyclyl; the heterocyclyl is optionally further substituted with unsubstituted alkyl;
R₃ is selected from hydrogen atom, alkyl, or hydroxyalkyl.

In some technical solutions of the present disclosure, in the compound of general formula (1-4), certain groups are as defined below (unspecified groups are as defined above):
L₁ is selected from alkylene, -(CH₂)ₙ₁C(O)(CH₂)ₘ₁-, - (CH₂)ₙ₁C(O)(CH₂)ₘ₁NRₐₐ(CH₂)ₘ₂-, or -(CH₂)ₙ₁S(O)ₘ₁-;
R₁ is selected from halogen, cyano, alkyl, heterocyclyl, or heteroaryl; the alkyl is optionally further substituted with halogen or alkoxy; the heterocyclyl is optionally further substituted with cyano; the heteroaryl is optionally further substituted with alkyl;
when L₁ is -(CH₂)ₙ₁C(O)(CH₂)ₘ₁-, R₁ is alkyl, heterocyclyl, or heteroaryl; the alkyl is optionally further substituted with halogen; the heterocyclyl or heteroaryl is optionally further substituted with alkyl;
L₂ is -NR₄-;
R₄ is selected from hydrogen atom, or alkyl;
L₃ is -NH-;
ring B is selected from 3-10 membered monocyclic heterocyclyl, 6-14 membered bridged heterocyclyl, or 6-14 membered fused heterocyclyl;
ring C is heteroaryl;
R₂ is selected from hydrogen atom, alkyl, alkoxy, halogen, cycloalkyl, heterocyclyl, aryl, or heteroaryl; the heteroaryl is optionally further substituted with unsubstituted alkyl, unsubstituted alkoxy, or unsubstituted cycloalkyl;
R₃ is selected from hydrogen atom, alkyl, or hydroxyalkyl.

In some technical solutions of the present disclosure, in the compound of general formula (1-4), certain groups are as defined below (unspecified groups are as defined above):
L₁ is selected from C₁-C₄ alkylene, -(CH₂)ₙ₁C(O)(CH₂)ₘ₁-, - (CH₂)ₙ₁C(O)(CH₂)ₘ₁NRₐₐ(CH₂)ₘ₂-, or -(CH₂)ₙ₁S(O)ₘ₁-;
R₁ is selected from halogen, cyano, C₁-C₃ alkyl, 4-6 membered monocyclic heterocyclyl with "heteroatom selected from one or more of N, O and S, and the number of the heteroatom being 1-3", or 5-6 membered monocyclic heteroaryl with "heteroatom selected from one or more of N, O and S, and the number of the heteroatom being 1-3"; the C₁-C₃ alkyl is optionally further substituted with halogen or C₁-C₃ alkoxy; the heterocyclyl is optionally further substituted with cyano; the heteroaryl is optionally further substituted with C₁-C₃ alkyl;
when L₁ is -(CH₂)ₙ₁C(O)(CH₂)ₘ₁-, R₁ is C₁-C₃ alkyl, 4-6 membered monocyclic heterocyclyl with "heteroatom selected from one or more of N, O and S, and the number of the heteroatom being 1-3", or 5-6 membered monocyclic heteroaryl with "heteroatom selected from one or more of N, O and S, and the number of the heteroatom being 1-3"; the C₁-C₃ alkyl is optionally further substituted with halogen; the heterocyclyl or heteroaryl is optionally further substituted with C₁-C₃ alkyl;
L₂ is -NR₄-;
R₄ is selected from hydrogen atom, or C₁-C₃ alkyl;
L₃ is -NH-;
ring B is selected from 4-6 membered monocyclic heterocyclyl with "heteroatom selected from one or more of N, O and S, and the number of the heteroatom being 1-3", 7-10 membered bridged heterocyclyl with "heteroatom selected from one or more of N, O and S, and the number of the heteroatom being 1-3", or 7-10 membered fused heterocyclyl with "heteroatom selected from one or more of N, O and S, and the number of the heteroatom being 1-3";
ring C is 5-6 membered monocyclic heteroaryl with "heteroatom selected from one or more of N, O and S, and the number of the heteroatom being 1-3";
R₂ is selected from hydrogen atom, C₁-C₃ alkyl, C₁-C₃ alkoxy, halogen, cycloalkyl, 4-6 membered monocyclic heterocyclyl with "heteroatom selected from one or more of N, O and S, and the number of the heteroatom being 1-3", 6-10 membered aryl, or 5-6 membered heteroaryl with "heteroatom selected from one or more of N, O and S, and the number of the heteroatom being 1-3"; the heteroaryl is optionally further substituted with one or more substituents selected from unsubstituted C₁-C₃ alkyl, unsubstituted C₁-C₃ alkoxy, or unsubstituted 4-6 membered monocyclic heterocyclyl with "heteroatom selected from one or more of N, O and S, and the number of the heteroatom being 1-3";
R₃ is selected from hydrogen atom, C₁-C₃ alkyl, or C₁-C₃ hydroxyalkyl.
L₁ is alkylene, or -(CH₂)ₙ₁S(O)ₘ₁-;
R₁ is cyano, or heterocyclyl; the heterocyclyl is optionally further substituted with cyano;
L₂ is -NH-;
L₃ is -NH-;
R₂ is selected from hydrogen atom, halogen, or alkoxy;
R₃ is selected from hydrogen atom, or alkyl.

In some technical solutions of the present disclosure, in the compound of general formula (1-4), certain group is as defined below (unspecified groups are as defined above): when L₁ is alkylene, the alkylene is preferably C₁-C₄ alkylene, *e.g.,* -CH₂-, -(CH₂)₂-, -(CH₂)₃- or - (CH₂)₄-, more preferably -CH₂-, or -(CH₂)₂-.

In some technical solutions of the present disclosure, in the compound of general formula (1-4), certain group is as defined below (unspecified groups are as defined above): when L₁ is -(CH₂)ₙ₁C(O)(CH₂)ₘ₁-, the -(CH₂)ₙ₁C(O)(CH₂)ₘ₁- is preferably -C(O)-, -C(O)CH₂-, or - CH₂C(O)-.

In some technical solutions of the present disclosure, in the compound of general formula (1-4), certain group is as defined below (unspecified groups are as defined above): when L₁ is -(CH₂)ₙ₁C(O)(CH₂)ₘ₁NRₐₐ(CH₂)ₘ₂-, the -(CH₂)ₙ₁C(O)(CH₂)ₘ₁NRₐₐ(CH₂)ₘ₂- is preferably - C(O)CH₂NH-, or -C(O)CH₂N(CH₃)-.

In some technical solutions of the present disclosure, in the compound of general formula (1-4), certain group is as defined below (unspecified groups are as defined above): when L₁ is -(CH₂)ₙ₁S(O)ₘ₁-, the -(CH₂)ₙ₁S(O)ₘ₁- is preferably -S(O)₂-.

In some technical solutions of the present disclosure, in the compound of general formula (1-4), certain group is as defined below (unspecified groups are as defined above): when R₄ is alkyl, the alkyl is preferably C₁-C₈ alkyl, more preferably C₁-C₆ alkyl, further preferably C₁-C₃ alkyl, *e.g.*, methyl, ethyl, propyl or isopropyl, further preferably methyl.

In some technical solutions of the present disclosure, in the compound of general formula (1-4), certain group is as defined below (unspecified groups are as defined above): when the ring B is 3-10 membered monocyclic heterocyclyl, the 3-10 membered monocyclic heterocyclyl is preferably 3-8 membered monocyclic heterocyclyl with "heteroatom selected from one or more of N, O and S, and the number of the heteroatom being 1-4", more preferably 4-6 membered monocyclic heterocyclyl with "heteroatom selected from one or more of N, O and S, and the number of the heteroatom being 1-3", *e.g.,*

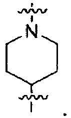

In some technical solutions of the present disclosure, in the compound of general formula (1-4), certain group is as defined below (unspecified groups are as defined above): when the ring B is 6-14 membered bridged heterocyclyl, the 6-14 membered bridged heterocyclyl is preferably 6-14 membered bridged heterocyclyl with "heteroatom selected from one or more of N, O and S, and the number of the heteroatom being 1-4", more preferably 7-10 membered bridged heterocyclyl with "heteroatom selected from one or more of N, O and S, and the number of the heteroatom being 1-3", *e.g.,*

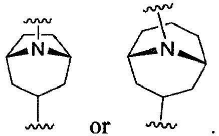

In some technical solutions of the present disclosure, in the compound of general formula (1-4), certain group is as defined below (unspecified groups are as defined above): when the ring B is 6-14 membered fused heterocyclyl, the 6-14 membered fused heterocyclyl is preferably 6-14 membered fused heterocyclyl with "heteroatom selected from one or more of N, O and S, and the number of the heteroatom being 1-4", more preferably 7-10 membered fused heterocyclyl with "heteroatom selected from one or more of N, O and S, and the number of the heteroatom being 1-3", *e.g.,*

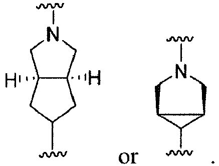

In some technical solutions of the present disclosure, in the compound of general formula (1-4), certain group is as defined below (unspecified groups are as defined above): when the ring C is heteroaryl, the heteroaryl is preferably 6-14 membered heteroaryl with "heteroatom selected from one or more of N, O and S, and the number of the heteroatom being 1-4", more preferably 5-6 membered monocyclic heteroaryl with "heteroatom selected from one or more of N, O and S, and the number of the heteroatom being 1-3", *e.g.,*

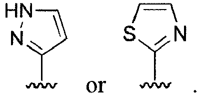

In some technical solutions of the present disclosure, in the compound of general formula (1-4), certain group is as defined below (unspecified groups are as defined above): when R₁ is halogen, the halogen is preferably fluorine.

In some technical solutions of the present disclosure, in the compound of general formula (1-4), certain group is as defined below (unspecified groups are as defined above): when R₁ is alkyl, the alkyl is preferably C₁-C₈ alkyl, more preferably C₁-C₆ alkyl, further preferably C₁-C₃ alkyl, *e.g.*, methyl, ethyl, propyl or isopropyl, further preferably methyl or ethyl.

In some technical solutions of the present disclosure, in the compound of general formula (1-4), certain group is as defined below (unspecified groups are as defined above): when R₁ is alkoxy, the alkoxy is preferably C₁-C₈ alkoxy, more preferably C₁-C₆ alkoxy, further preferably C₁-C₃ alkoxy, *e.g.*, methoxy, ethoxy, propoxy or isopropoxy, further preferably methoxy or ethoxy.

In some technical solutions of the present disclosure, in the compound of general formula (1-4), certain group is as defined below (unspecified groups are as defined above): when R₁ is heterocyclyl, the heterocyclyl is preferably 3-8 membered monocyclic heterocyclyl with "heteroatom selected from one or more of N, O and S, and the number of the heteroatom being 1-4", more preferably 4-6 membered monocyclic heterocyclyl with "heteroatom selected from one or more of N, O and S, and the number of the heteroatom being 1-3", *e.g.,*

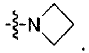

In some technical solutions of the present disclosure, in the compound of general formula (1-4), certain group is as defined below (unspecified groups are as defined above): when R₁ is heteroaryl, the heteroaryl is preferably 5-10 membered heteroaryl with "heteroatom selected from one or more of N, O and S, and the number of the heteroatom being 1-4", more preferably 5-6 membered heteroaryl with "heteroatom selected from one or more of N, O and S, and the number of the heteroatom being 1-3", *e.g.,*

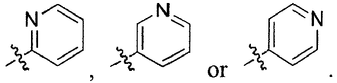

In some technical solutions of the present disclosure, in the compound of general formula (1-4), certain group is as defined below (unspecified groups are as defined above): when R₂ is alkyl, the alkyl is preferably C₁-C₈ alkyl, more preferably C₁-C₆ alkyl, further preferably C₁-C₃ alkyl, *e.g.*, methyl, ethyl, propyl or isopropyl, further preferably methyl or ethyl.

In some technical solutions of the present disclosure, in the compound of general formula (1-4), certain group is as defined below (unspecified groups are as defined above): when R₂ is alkoxy, the alkoxy is preferably C₁-C₈ alkoxy, more preferably C₁-C₆ alkoxy, further preferably C₁-C₃ alkoxy, *e.g.*, methoxy, ethoxy, propoxy or isopropoxy, further preferably methoxy or ethoxy.

In some technical solutions of the present disclosure, in the compound of general formula (1-4), certain group is as defined below (unspecified groups are as defined above): when R₂ is halogen, the halogen is preferably fluorine, chlorine or bromine.

In some technical solutions of the present disclosure, in the compound of general formula (1-4), certain group is as defined below (unspecified groups are as defined above): when R₂ is heterocyclyl, the heterocyclyl is preferably 3-8 membered monocyclic heterocyclyl with "heteroatom selected from one or more of N, O and S, and the number of the heteroatom being 1-4", more preferably 4-6 membered monocyclic heterocyclyl with "heteroatom selected from one or more of N, O and S, and the number of the heteroatom being 1-3", *e.g.,*

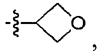

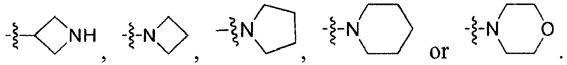

In some technical solutions of the present disclosure, in the compound of general formula (1-4), certain group is as defined below (unspecified groups are as defined above): when R₂ is aryl, the aryl is preferably 6-10 membered aryl, more preferably phenyl.

In some technical solutions of the present disclosure, in the compound of general formula (1-4), certain group is as defined below (unspecified groups are as defined above): when R₂ is heteroaryl, the heteroaryl is preferably 5-10 membered heteroaryl with "heteroatom selected from one or more of N, O and S, and the number of the heteroatom being 1-4", more preferably 5-6 membered heteroaryl with "heteroatom selected from one or more of N, O and S, and the number of the heteroatom being 1-3, *e*.*g*.,

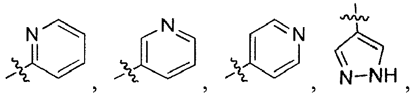

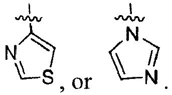

In some technical solutions of the present disclosure, in the compound of general formula (1-4), certain group is as defined below (unspecified groups are as defined above): when R2 is -(CH₂)ₙ₁NRₐₐR_{bb}, -(CH₂)ₙ₁NRₐₐR_{bb} is preferably

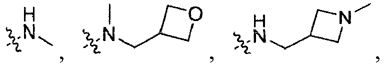

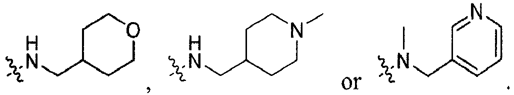

In some technical solutions of the present disclosure, in the compound of general formula (1-4), certain group is as defined below (unspecified groups are as defined above): when R₂ is alkyl, and the alkyl is optionally further substituted with substituted or unsubstituted heterocyclyl, then the heterocyclyl is preferably 3-8 membered monocyclic heterocyclyl with "heteroatom selected from one or more of N, O and S, and the number of the heteroatom being 1-4", more preferably 4-6 membered monocyclic heterocyclyl with "heteroatom selected from one or more of N, O and S, and the number of the heteroatom being 1-3", *e.g.,* 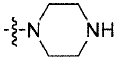 or

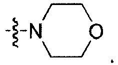

In some technical solutions of the present disclosure, in the compound of general formula (1-4), certain group is as defined below (unspecified groups are as defined above): when R₂ is alkoxy, and the alkoxy is optionally further substituted with unsubstituted alkoxy, then the unsubstituted alkoxy is preferably C₁-C₈ alkoxy, more preferably C₁-C₆ alkoxy, further preferably C₁-C₃ alkoxy, *e*.*g*., methoxy, ethoxy, propoxy or isopropoxy, further preferably methoxy.

In some technical solutions of the present disclosure, in the compound of general formula (1-4), certain group is as defined below (unspecified groups are as defined above): when R₂ is alkoxy, and the alkoxy is optionally further substituted with heterocyclyl unsubstituted or substituted with alkyl, then the heterocyclyl is preferably 3-8 membered monocyclic heterocyclyl with "heteroatom selected from one or more of N, O and S, and the number of the heteroatom being 1-4", more preferably 4-6 membered monocyclic heterocyclyl with "heteroatom selected from one or more of N, O and S, and the number of the heteroatom being 1-3", *e.g.,*

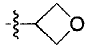

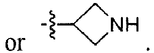

In some technical solutions of the present disclosure, in the compound of general formula (1-4), certain group is as defined below (unspecified groups are as defined above): when R₂ is alkoxy, and the alkoxy is optionally further substituted with heterocyclyl unsubstituted or substituted with alkyl, then the alkyl is preferably C₁-C₈ alkyl, more preferably C₁-C₆ alkyl, further preferably C₁-C₃ alkyl, *e.g.,* methyl, ethyl, propyl or isopropyl, further preferably methyl.

In some technical solutions of the present disclosure, in the compound of general formula (1-4), certain group is as defined below (unspecified groups are as defined above): when R₂ is alkoxy, and the alkoxy is optionally further substituted with unsubstituted heteroaryl, the heteroaryl is preferably 5-10 membered heteroaryl with "heteroatom selected from one or more of N, O and S, and the number of the heteroatom being 1-4", more preferably 5-6 membered heteroaryl with "heteroatom selected from one or more of N, O and S, and the number of the heteroatom being 1-3, *e.g.,*

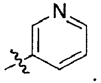

In some technical solutions of the present disclosure, in the compound of general formula (1-4), certain group is as defined below (unspecified groups are as defined above): when R₂ is heterocyclyl, and the heterocyclyl is optionally further substituted with unsubstituted alkyl, then the alkyl is preferably C₁-C₈ alkyl, more preferably C₁-C₆ alkyl, further preferably C₁-C₃ alkyl, *e.g.*, methyl, ethyl, propyl or isopropyl, further preferably methyl.

In some technical solutions of the present disclosure, in the compound of general formula (1-4), certain group is as defined below (unspecified groups are as defined above): when R₂ is heterocyclyl, and the heterocyclyl is optionally further substituted with unsubstituted alkoxy, then the alkoxy is preferably C₁-C₈ alkoxy, more preferably C₁-C₆ alkoxy, further preferably C₁-C₃ alkoxy, *e.g.*, methoxy, ethoxy, propoxy or isopropoxy, further preferably methoxy.

In some technical solutions of the present disclosure, in the compound of general formula (1-4), certain group is as defined below (unspecified groups are as defined above): when R₂ is heterocyclyl, and the heterocyclyl is optionally further substituted with amino substituted with alkyl, then the alkyl is preferably C₁-C₈ alkyl, more preferably C₁-C₆ alkyl, further preferably C₁-C₃ alkyl, *e.g.*, methyl, ethyl, propyl or isopropyl, further preferably methyl.

In some technical solutions of the present disclosure, in the compound of general formula (1-4), certain group is as defined below (unspecified groups are as defined above): when R₂ is heteroaryl, and the heteroaryl is optionally further substituted with alkyl unsubstituted or substituted with halogen, then the alkyl is preferably C₁-C₈ alkyl, more preferably C₁-C₆ alkyl, further preferably C₁-C₃ alkyl, *e.g.*, methyl, ethyl, propyl or isopropyl, further preferably methyl.

In some technical solutions of the present disclosure, in the compound of general formula (1-4), certain group is as defined below (unspecified groups are as defined above): when R₂ is heteroaryl, and the heteroaryl is optionally further substituted with alkyl unsubstituted or substituted with halogen, then the halogen is preferably fluorine.

In some technical solutions of the present disclosure, in the compound of general formula (1-4), certain group is as defined below (unspecified groups are as defined above): when R₂ is heteroaryl, and the heteroaryl is optionally further substituted with unsubstituted alkoxy, then the alkoxy is preferably C₁-C₈ alkoxy, more preferably C₁-C₆ alkoxy, further preferably C₁-C₃ alkoxy, *e*.*g*., methoxy, ethoxy, propoxy or isopropoxy, further preferably methoxy.

In some technical solutions of the present disclosure, in the compound of general formula (1-4), certain group is as defined below (unspecified groups are as defined above): when R₂ is heteroaryl, and the heteroaryl is optionally further substituted with unsubstituted cycloalkyl, then the cycloalkyl is preferably C₃-C₈ cycloalkyl, more preferably C₃-C₆ cycloalkyl, *e.g.*,

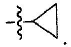

In some technical solutions of the present disclosure, in the compound of general formula (1-4), certain group is as defined below (unspecified groups are as defined above): when Rₐₐ and R_{bb} are independently alkyl, the alkyl is preferably C₁-C₈ alkyl, more preferably C₁-C₆ alkyl, further preferably C₁-C₃ alkyl, *e.g.*, methyl, ethyl, propyl or isopropyl, further preferably methyl.

In some technical solutions of the present disclosure, in the compound of general formula (1-4), certain group is as defined below (unspecified groups are as defined above): when Rₐₐ and R_{bb} are independently alkyl, and the alkyl is optionally further substituted with heterocyclyl unsubstituted or substituted with alkyl, then the heterocyclyl is preferably 3-8 membered monocyclic heterocyclyl with "heteroatom selected from one or more of N, O and S, and the number of the heteroatom being 1-4", more preferably 4-6 membered monocyclic heterocyclyl with "heteroatom selected from one or more of N, O and S, and the number of the heteroatom being 1-3", *e*.*g*.,

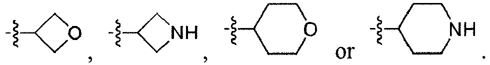

In some technical solutions of the present disclosure, in the compound of general formula (1-4), certain group is as defined below (unspecified groups are as defined above): when Rₐₐ and R_{bb} are independently alkyl, the alkyl is optionally further substituted with heterocyclyl unsubstituted or substituted with alkyl, the alkyl is preferably C₁-C₈ alkyl, more preferably C₁-C6 alkyl, further preferably C₁-C₃ alkyl, *e.g.*, methyl, ethyl, propyl or isopropyl, further preferably methyl.

In some technical solutions of the present disclosure, in the compound of general formula (1-4), certain group is as defined below (unspecified groups are as defined above): when Rₐₐ and R_{bb} are independently alkyl, the alkyl is optionally further substituted with unsubstituted heteroaryl, the heteroaryl is preferably 5-10 membered heteroaryl with "heteroatom selected from one or more of N, O and S, and the number of the heteroatom being 1-4", more preferably 5-6 membered heteroaryl with "heteroatom selected from one or more of N, O and S, and the number of the heteroatom being 1-3, *e.g.,*

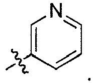

In some technical solutions of the present disclosure, in the compound of general formula (1-4), certain group is as defined below (unspecified groups are as defined above): when R₃ is alkyl, the alkyl is preferably C₁-C₈ alkyl, more preferably C₁-C₆ alkyl, further preferably C₁-C₃ alkyl, *e.g.*, methyl, ethyl, propyl or isopropyl, further preferably methyl or ethyl.

In some technical solutions of the present disclosure, in the compound of general formula (1-4), certain group is as defined below (unspecified groups are as defined above): when R₃ is hydroxyalkyl, the alkyl is preferably C₁-C₈ hydroxyalkyl, more preferably C₁-C₆ hydroxyalkyl, further preferably C₁-C₃ hydroxyalkyl, *e.g.*, hydroxymethyl, hydroxyethyl, hydroxypropyl or hydroxyisopropyl, further preferably hydroxymethyl.

In some technical solutions of the present disclosure, the compound of formula (I) may be any one of the following structures,

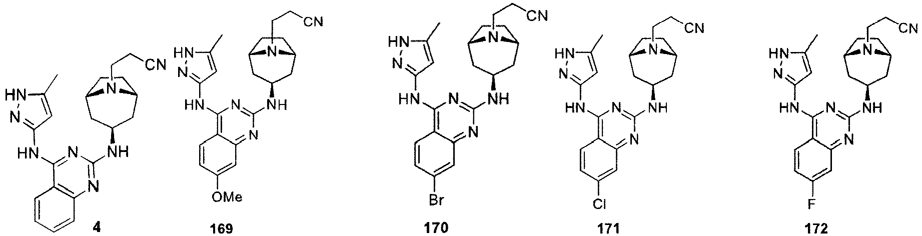

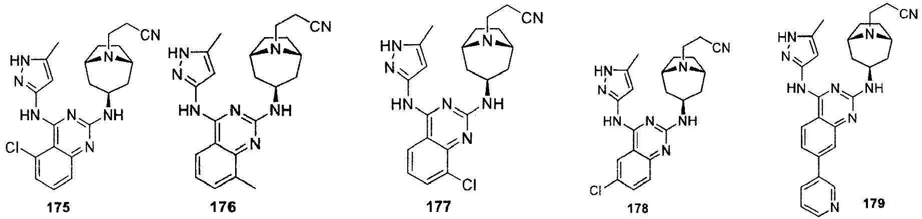

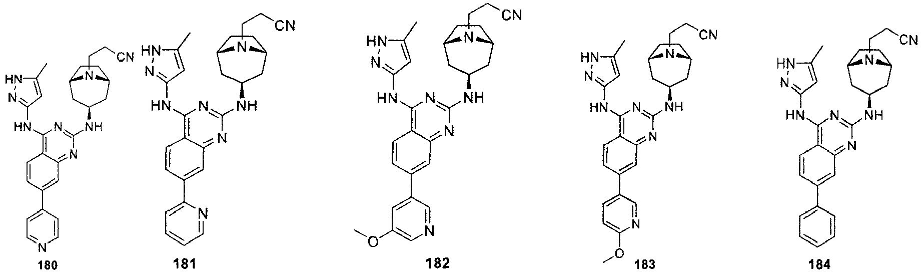

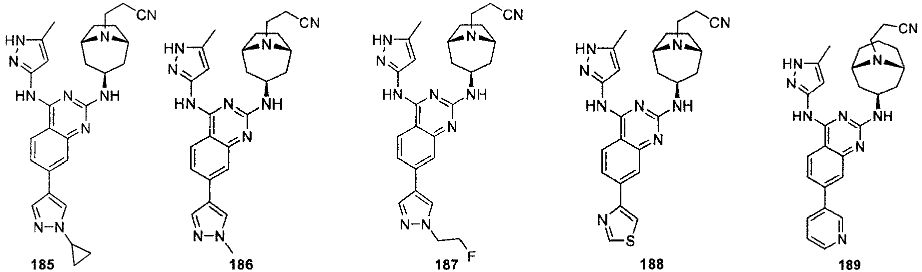

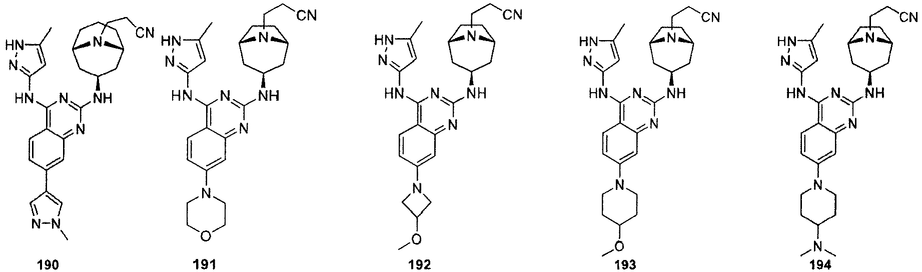

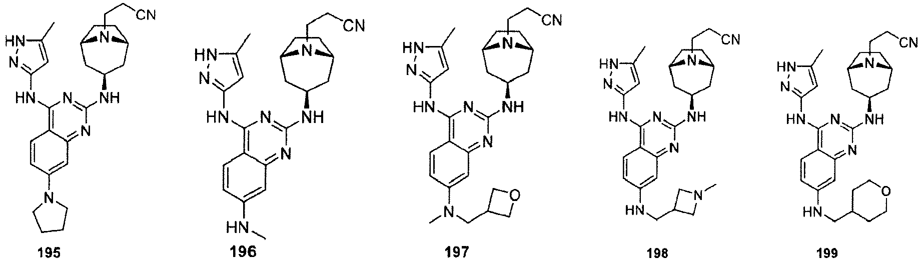

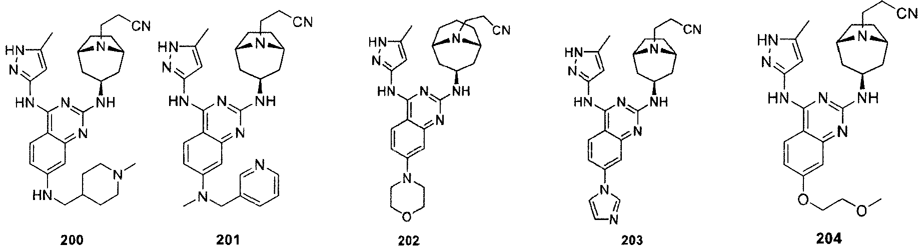

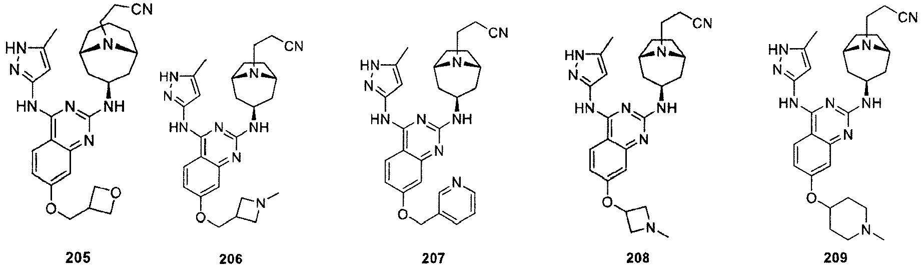

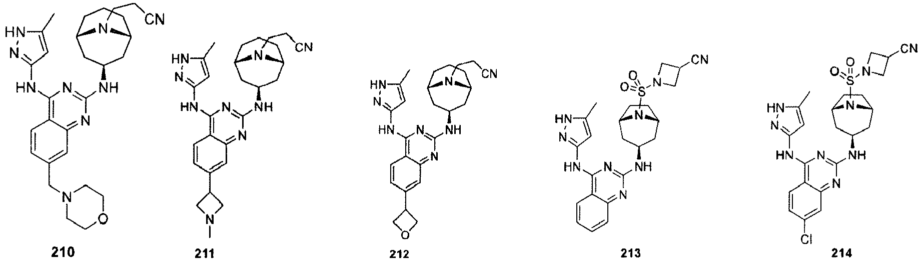

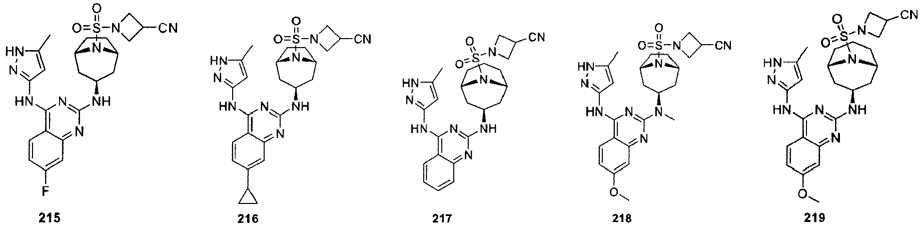

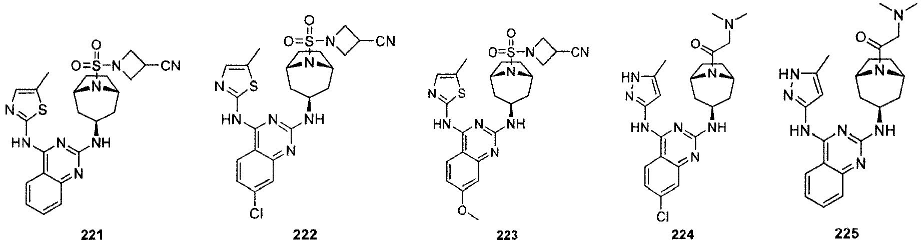

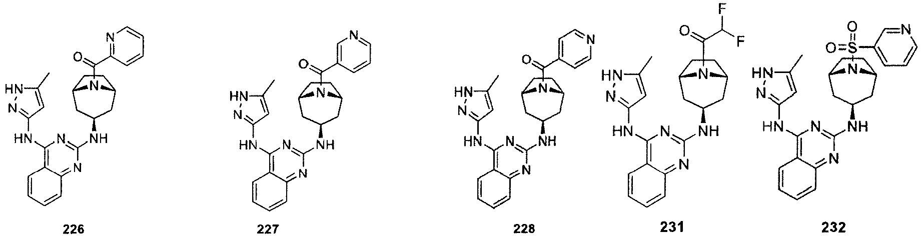

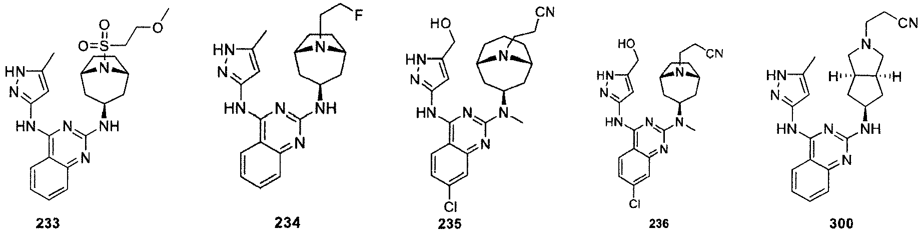

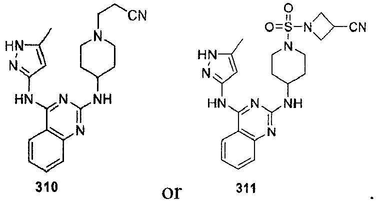

In some technical solutions of the present disclosure, the compound of formula (I) is preferably a compound of formula (1-5): 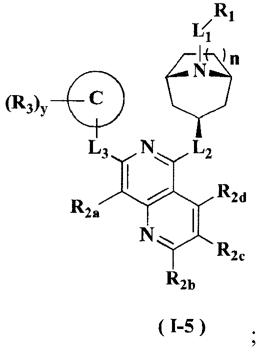 wherein,
R₂ₐ is hydrogen atom, cyano, alkyl or alkoxy;
R_{2b} is hydrogen atom or cyano;
R_{2c} is hydrogen atom or halogen;
R_{2d} is hydrogen atom or hydroxyl;
n is 1 or 2;
L₁, L₂, L₃, R₁, R₃, ring C, y are as defined above.

In some technical solutions of the present disclosure, the compound of formula (1-5) is preferably a compound of formula (I-5-1), 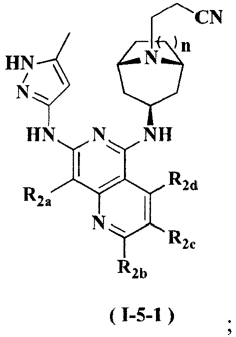 wherein, n, R₂ₐ, R_{2b}, R_{2c} and R_{2d} are as defined above.

In some technical solutions of the present disclosure, the compound of formula (I) may be any one of the following structures,

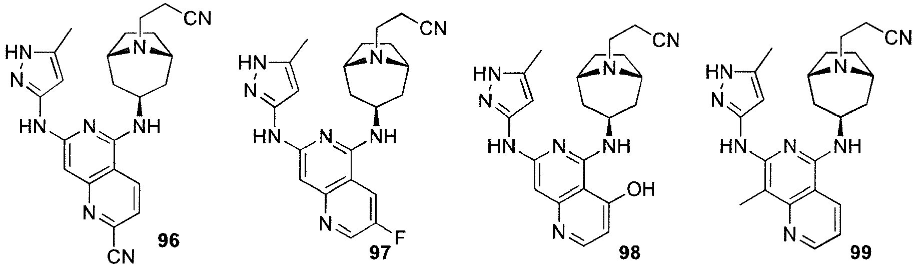

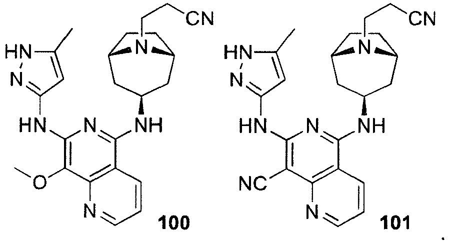

In some technical solutions of the present disclosure, in the compound of general formula (I), certain groups are as defined below (unspecified groups are as defined above):
L₃ is selected from bond, or -C(O)NRₐₐ-;
Rₐₐ is selected from hydrogen atom, or alkyl.

In some technical solutions of the present disclosure, the compound of formula (I) is preferably a compound of formula (1-6), 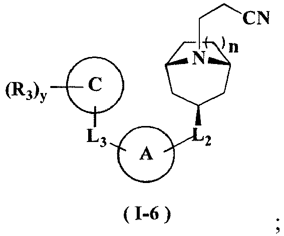
wherein, L₂ is selected from -NH-;
L₃ is selected from bond, or -C(O)NH-;
ring A is selected from 6-14 membered fused heteroaryl;
ring C is selected from heteroaryl;
y is 0, 1, 2 or 3;
n is 1 or 2.

In some technical solutions of the present disclosure, the compound of formula (I) may be the following structure,

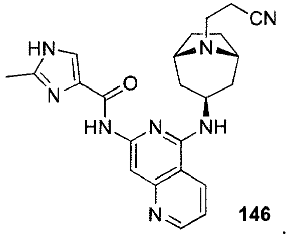

In some technical solutions of the present disclosure, the compound of formula (I) may be a compound of formula (1-7): 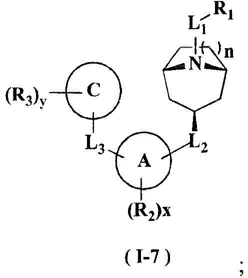
wherein, ring C is heteroaryl, and ring C is not 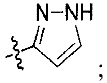
L₁, L₂, L₃, R₁, R₂, R₃, ring A, x and y are as defined above.

In some technical solutions of the present disclosure, in the compound of general formula (1-7), certain groups are as defined below (unspecified groups are as defined above):
ring C is heteroaryl, and ring C is not 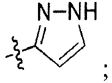
L₁ is selected from alkylene, -(CH₂)ₙ₁C(O)(CH₂)ₘ₁-, - (CH₂)ₙ₁C(O)(CH₂)ₘ₁NRₐₐ(CH₂)ₘ₂-, or -(CH₂)ₙ₁S(O)ₘ₁-;
L₂ is selected from oxygen atom, or -NR₄-;
R₄ is selected from hydrogen atom, or alkyl;
L₃ is hydrogen atom;
ring A is 6-14 membered fused heteroaryl;
R₁ is hydrogen atom, halogen, alkyl, cyano, or heterocyclyl; the heterocyclyl is optionally further substituted with cyano;
R₂ is hydrogen atom;
R₃ is selected from hydrogen atom, alkyl, hydroxyalkyl, cycloalkyl, - (CH₂)ₙ₁C(O)NRₐₐR_{bb}, or -(CH₂)ₙ₁S(O)ₘ₁NRₐₐR_{bb};
Rₐₐ and R_{bb} are independently selected from hydrogen atom, or alkyl.

In some technical solutions of the present disclosure, in the compound of general formula (1-7), certain group are as defined below (unspecified groups are as defined above):
ring C is heteroaryl, and ring C is not 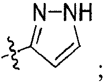
L₁ is selected from alkylene, -(CH₂)ₙ₁C(O)(CH₂)ₘ₁-, - (CH₂)ₙ₁C(O)(CH₂)ₘ₁NRₐₐ(CH₂)ₘ₂-;
L₂ is selected from oxygen atom, or -NR₄-;
R₄ is selected from hydrogen atom, or alkyl;
L₃ is hydrogen atom;
ring A is 6-14 membered fused heteroaryl;
R₁ is hydrogen atom, halogen, alkyl, cyano, or heterocyclyl;
R₂ is hydrogen atom;
R₃ is selected from hydrogen atom, alkyl, hydroxyalkyl, -(CH₂)ₙ₁C(O)NRₐₐR_{bb};
Rₐₐ and R_{bb} are independently selected from hydrogen atom, or alkyl.

In some technical solutions of the present disclosure, in the compound of general formula (1-7), certain group is as defined below (unspecified groups are as defined above): when the ring C is heteroaryl, the heteroaryl is preferably 6-14 membered heteroaryl with "heteroatom selected from one or more of N, O and S, and the number of the heteroatom being 1-4", more preferably 5-6 membered monocyclic heteroaryl with "heteroatom selected from one or more of N, O and S, and the number of the heteroatom being 1-3". The 5-6 membered monocyclic heteroaryl is preferably

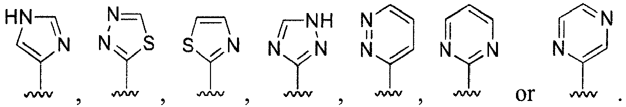

In some technical solutions of the present disclosure, the compound of formula (I) may be any one of the following structures,

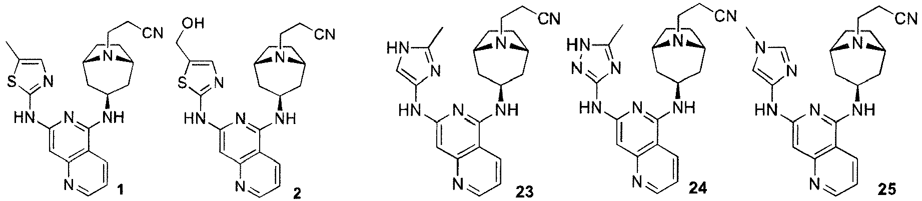

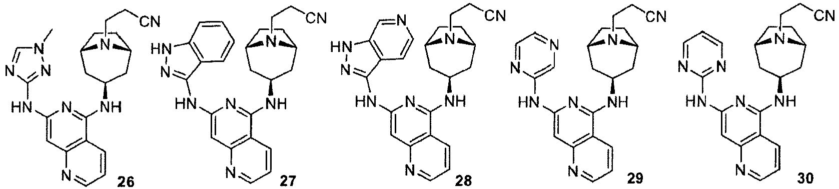

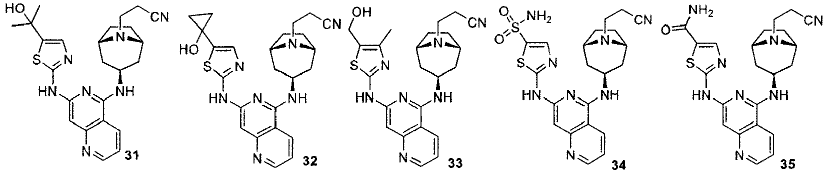

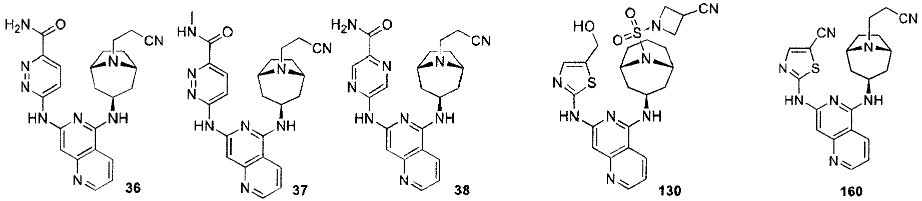

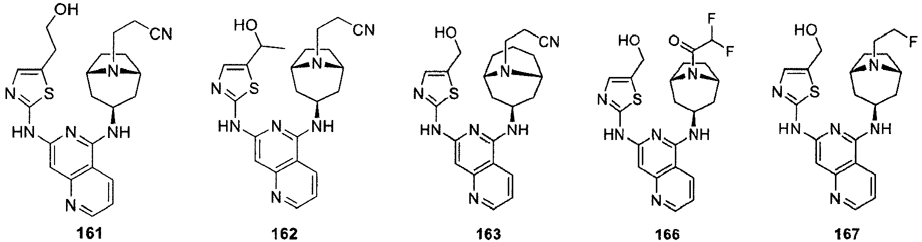

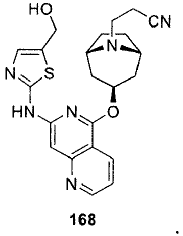

In some technical solutions of the present disclosure, the compound of formula (I) may be a compound of formula (I-8): 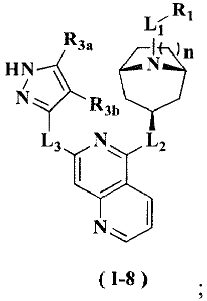
wherein, R₃ₐ and R_{3b} are independently hydrogen atom, halogen, hydroxyl, cyano, alkyl, hydroxyalkyl, alkoxy, cycloalkyl, heterocyclyl or -(CH₂)ₙ₁C(O)NRₐₐR_{bb}; the alkyl, cycloalkyl or heterocyclyl is optionally further substituted with halogen, hydroxyl, unsubstituted alkyl;
Rₐₐ and R_{bb} are identical or different, and are each independently selected from hydrogen atom, or alkyl;
when R₃ₐ is methyl, R_{3b} is not hydrogen atom;
R₁, L₁, L₂ and L₃ are as defined above;
n is 1 or 2.

In some technical solutions of the present disclosure, in the compound of general formula (1-8), certain groups are as defined below (unspecified groups are as defined above):
R₃ₐ and R_{3b} are independently hydrogen atom, halogen, hydroxyl, cyano, alkyl, hydroxyalkyl, alkoxy, cycloalkyl, heterocyclyl or -(CH₂)ₙ₁C(O)NRₐₐR_{bb}; the alkyl, cycloalkyl or heterocyclyl is optionally further substituted with halogen, hydroxyl, unsubstituted alkyl;
Rₐₐ and R_{bb} are identical or different, and are each independently selected from hydrogen atom, or alkyl;
when R₃ₐ is methyl, R_{3b} is not hydrogen atom;
L₁ is selected from alkylene, or -(CH₂)ₙ₁S(O)ₘ₁-;
L₂ is -NR₄-;
L₃ is -NH-;
R₁ is selected from cyano, or heterocyclyl; the heterocyclyl is optionally further substituted with cyano;
n is 1 or 2.

In some technical solutions of the present disclosure, in the compound of general formula (I-8), certain groups are as defined below (unspecified groups are as defined above):
R₃ₐ and R_{3b} are independently hydrogen atom, halogen, alkyl, hydroxyalkyl, or cycloalkyl;
when R₃ₐ is methyl, R_{3b} is not hydrogen atom;
L₁ is selected from alkylene;
L₂ is -NH-;
L₃ is -NH-;
R₁ is cyano;
n is 1 or 2.

In some technical solutions of the present disclosure, the compound of formula (I) may be any one of the following structures,

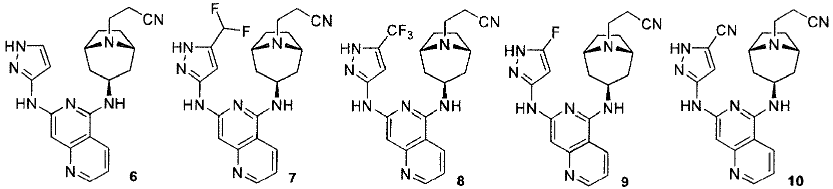

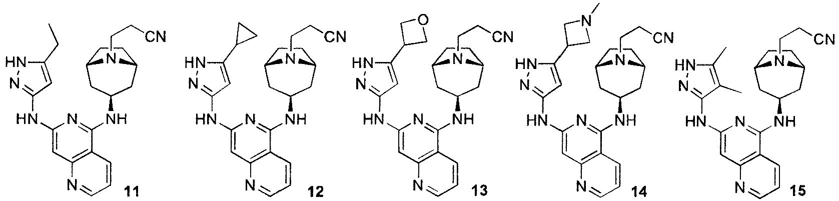

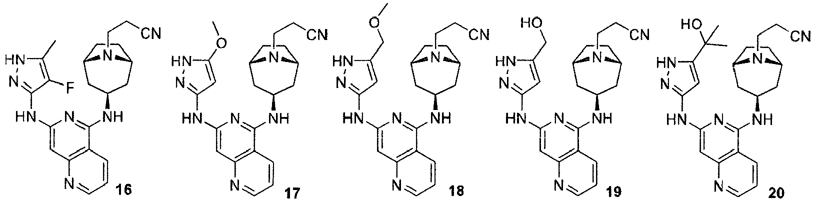

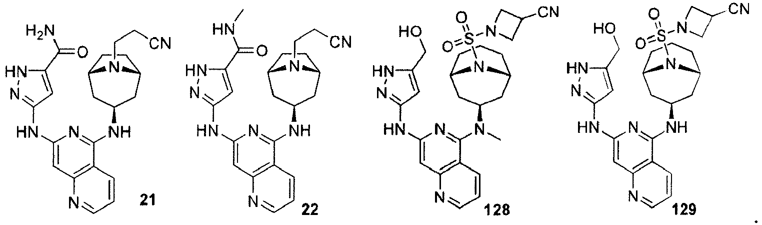

In some technical solutions of the present disclosure, in the compound of general formula (I), the compound of general formula (I) may be a compound of formula (I-9), certain group is as defined below (unspecified groups are as defined above): 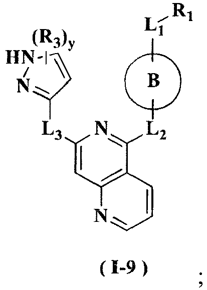
wherein, ring B is selected from 3-10 membered monocyclic heterocyclyl, or 6-14 membered fused heterocyclyl;
R₁, R₃, L₁, L₂, L₃ and y are as defined above.

In some technical solutions of the present disclosure, in the compound of general formula (1-8), certain groups are as defined below (unspecified groups are as defined above):
ring B is selected from 3-10 membered monocyclic heterocyclyl, or 6-14 membered fused heterocyclyl;
L₁ is selected from alkylene, or -(CH₂)ₙ₁S(O)ₘ₁-;
L₂ is -NH-;
L₃ is -NH-;
R₁ is selected from cyano, or heterocyclyl; the heterocyclyl is optionally further substituted with cyano;
R₃ is selected from hydrogen atom, or alkyl;
y is 0, 1 or 2.

In some technical solutions of the present disclosure, the compound of formula (I) may be any one of the following structures,

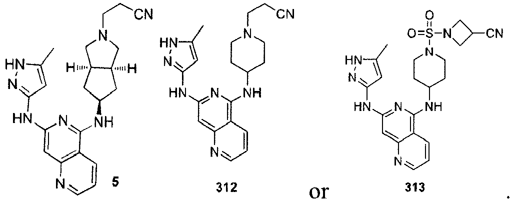

In some technical solutions of the present disclosure, the compound of general formula (I) may be a compound of formula (I-10), certain groups are as defined below (unspecified groups are as defined above): 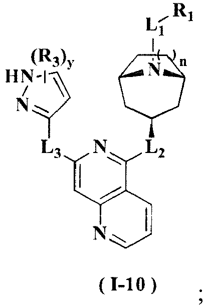
wherein, L₂ is selected from oxygen atom, or -NR₄-;
R₄ is alkyl;
R₁, R₃, L₁, L₃ and y are as defined above.

In some technical solutions of the present disclosure, in the compound of general formula (I-10), certain groups are as defined below (unspecified groups are as defined above):
L₂ is selected from oxygen atom, or -NR₄-;
R₄ is alkyl;
L₁ is selected from alkylene, or -(CH₂)ₙ₁S(O)ₘ₁-;
L₃ is -NH-;
R₁ is selected from cyano, or heterocyclyl; the heterocyclyl is optionally further substituted with cyano;
R₃ is selected from hydrogen atom, or alkyl;
y is 0, 1 or 2.

In some technical solutions of the present disclosure, the compound of formula (I) may be any one of the following structures,

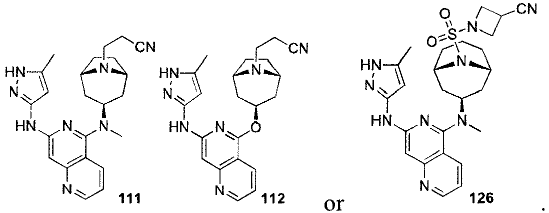

In some technical solutions of the present disclosure, the compound of formula (I) may be any one of the following structures,

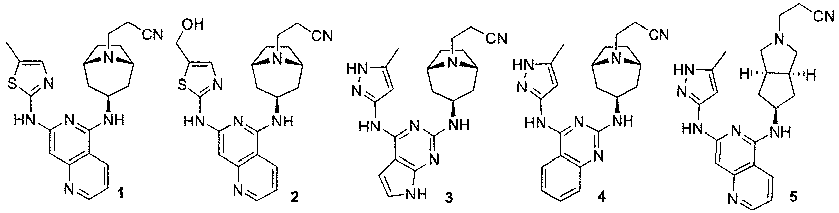

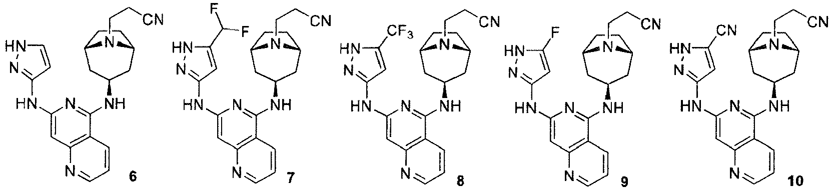

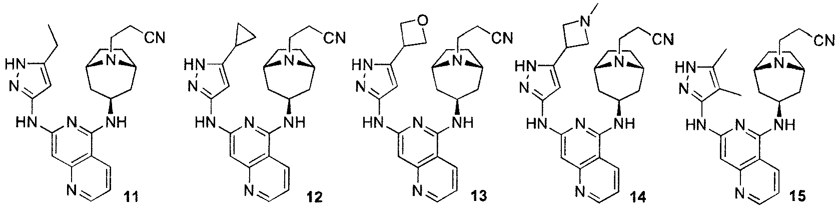

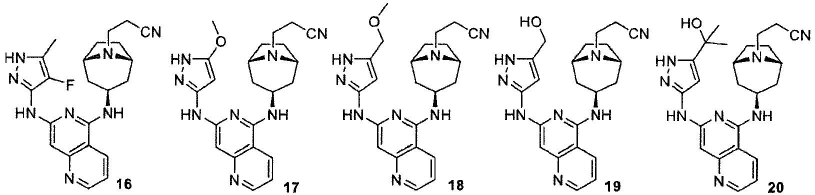

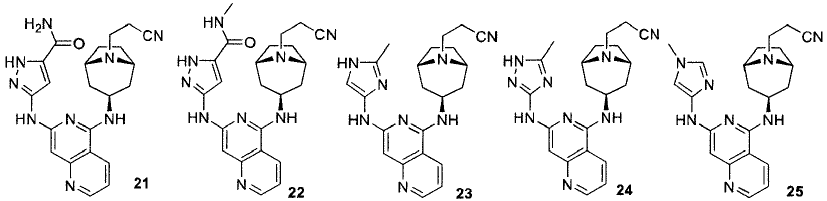

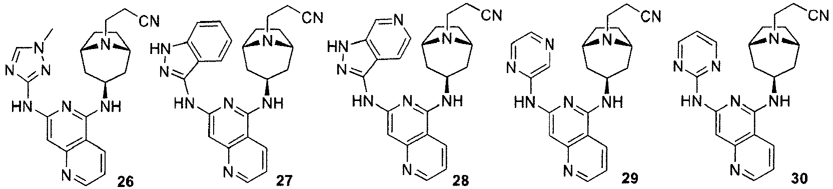

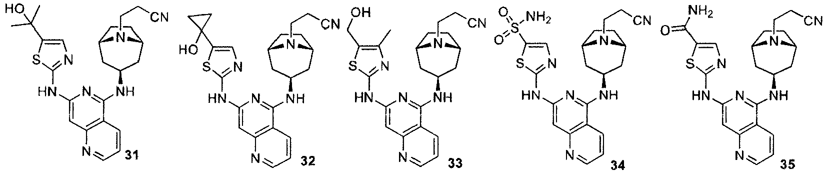

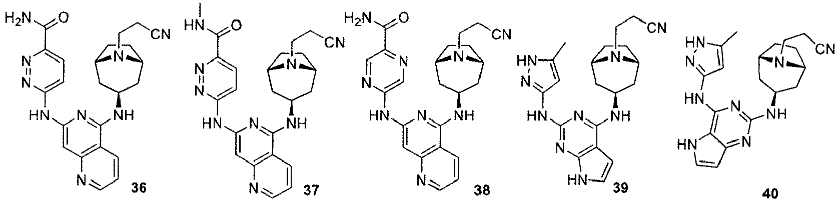

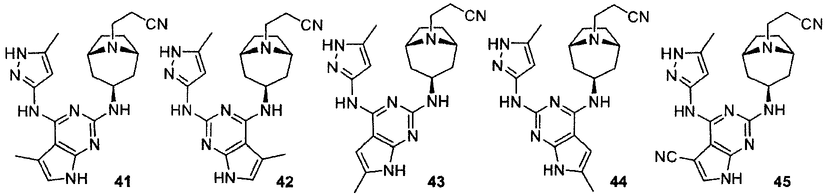

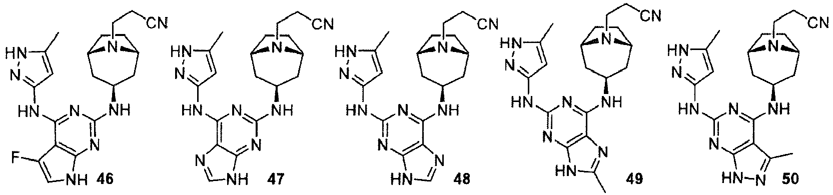

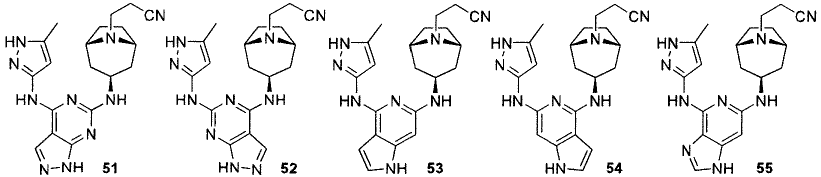

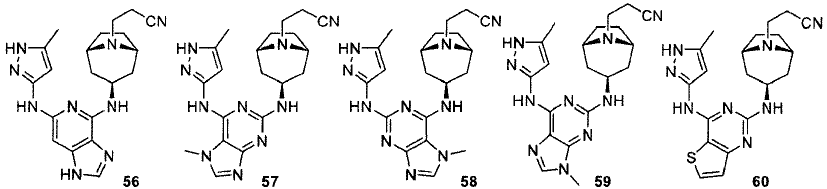

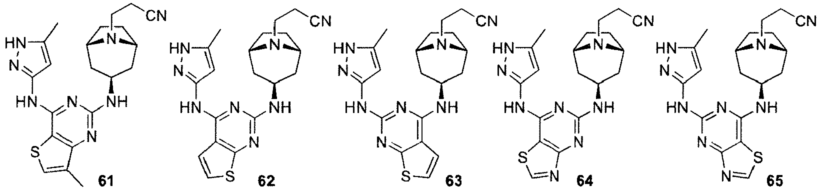

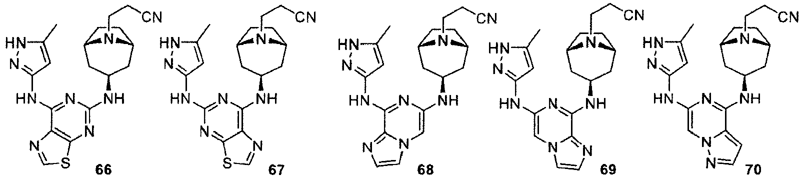

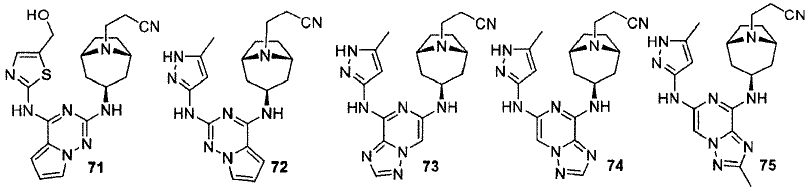

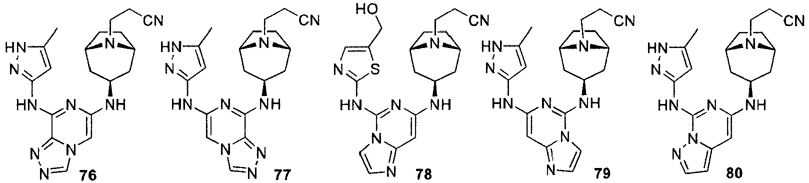

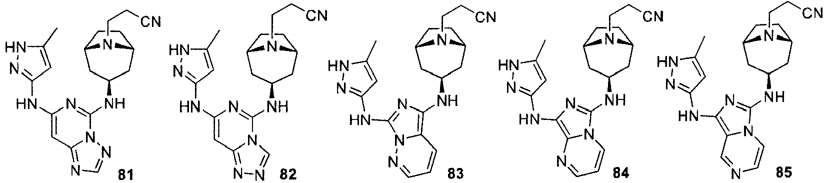

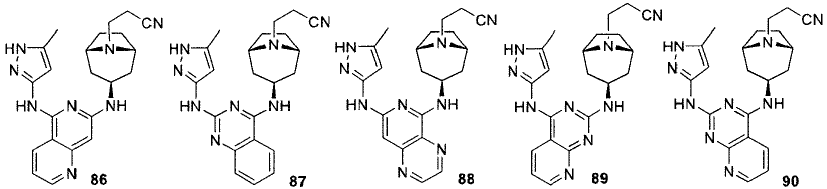

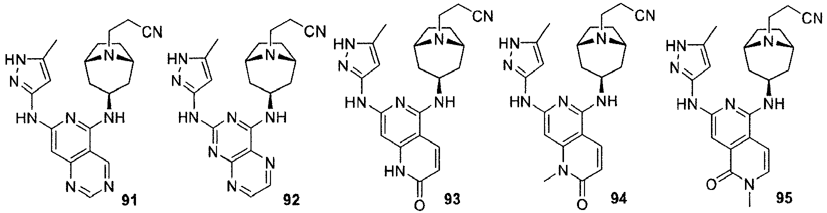

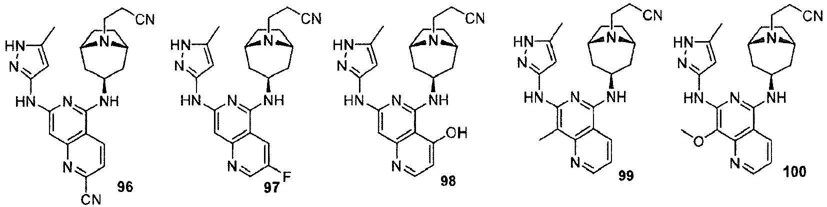

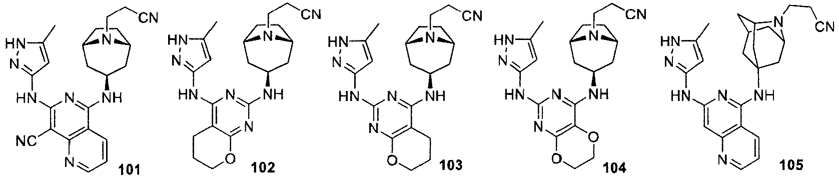

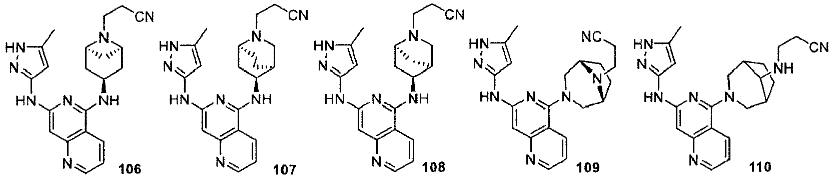

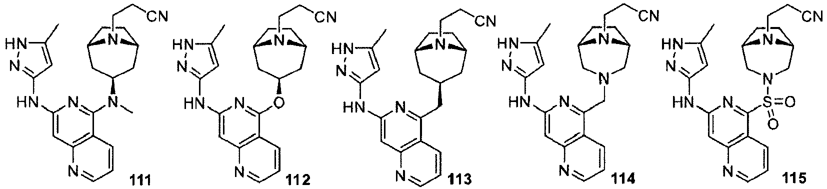

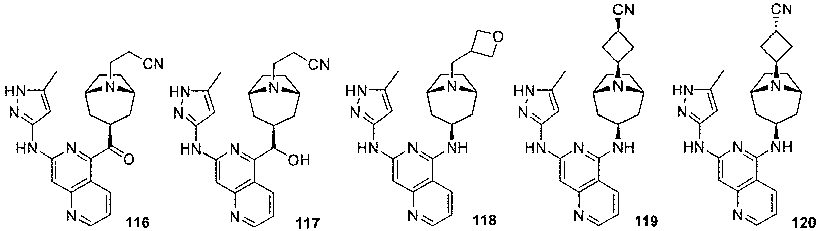

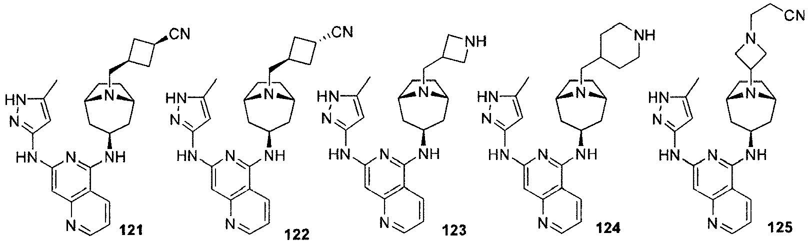

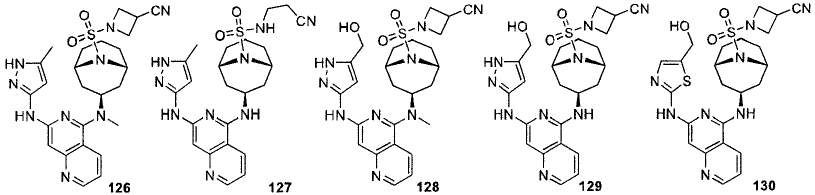

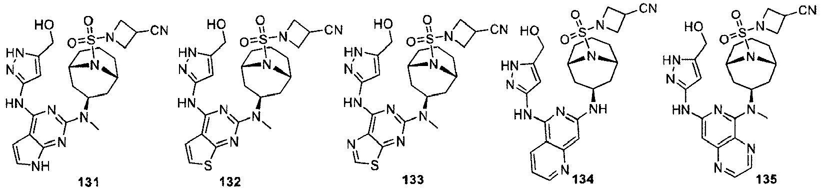

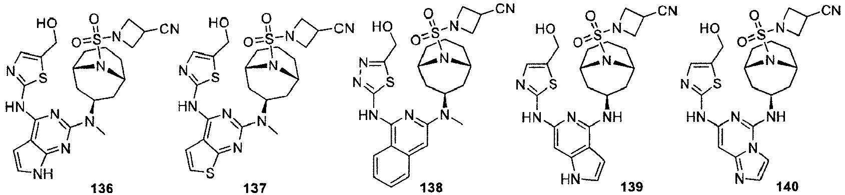

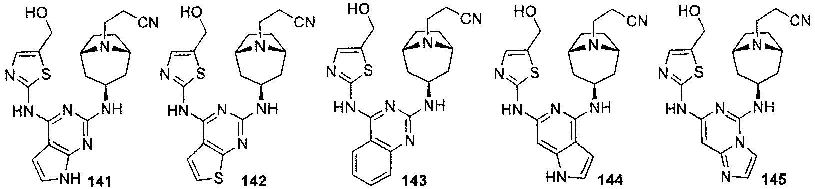

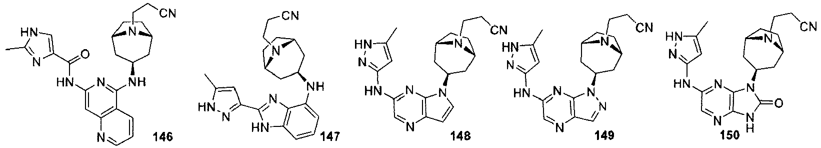

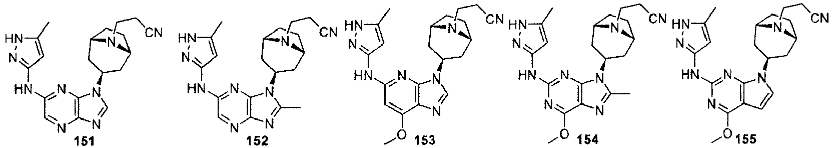

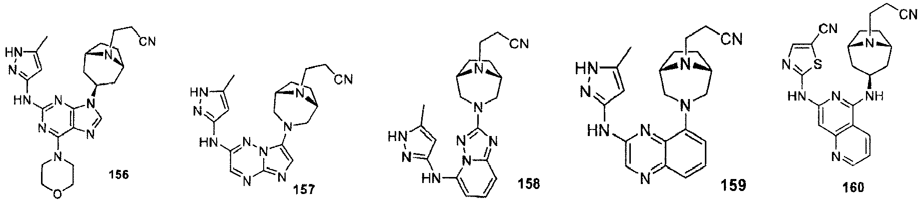

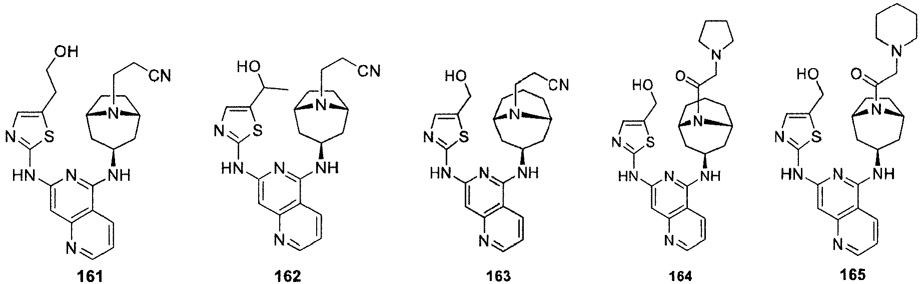

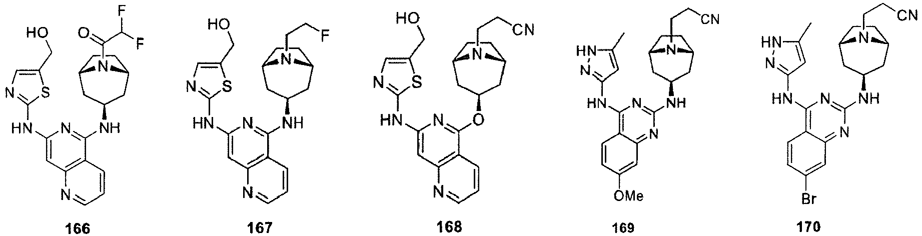

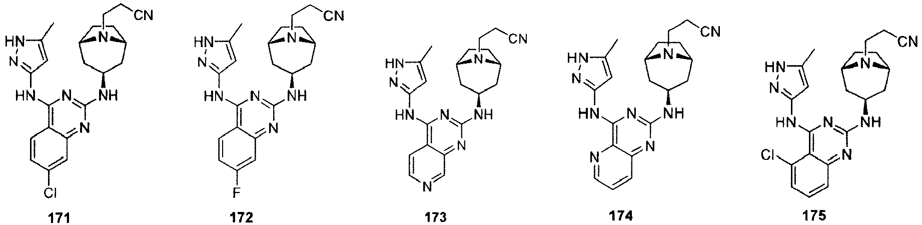

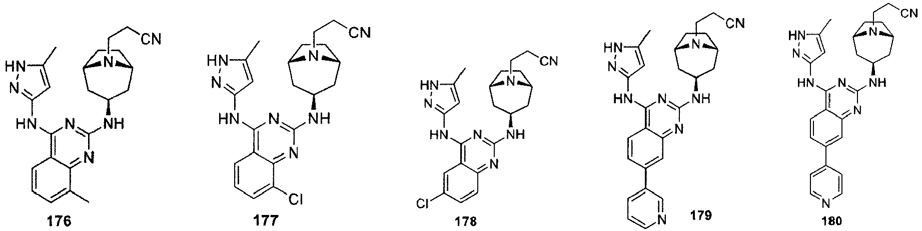

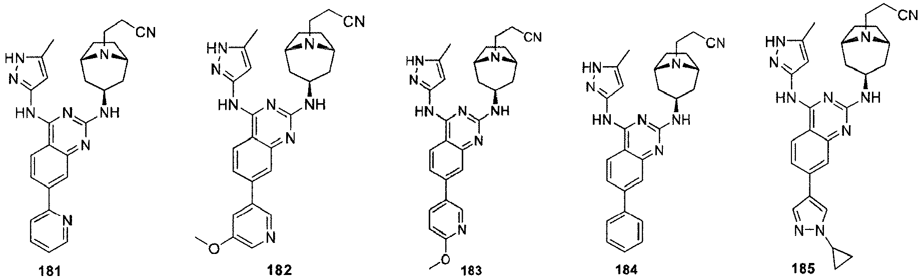

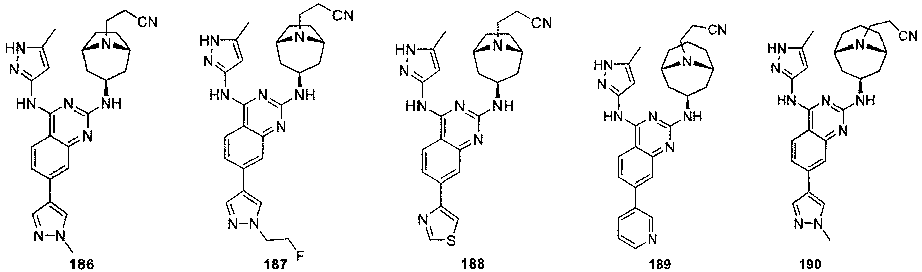

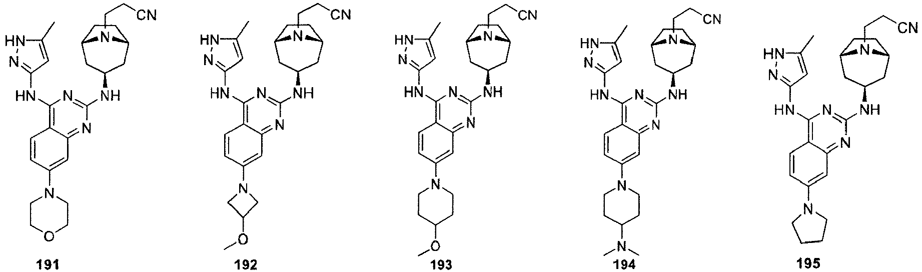

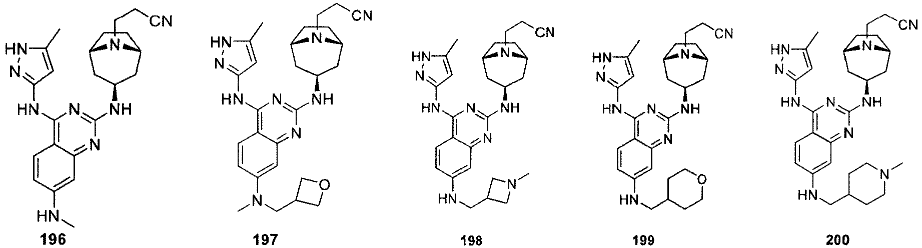

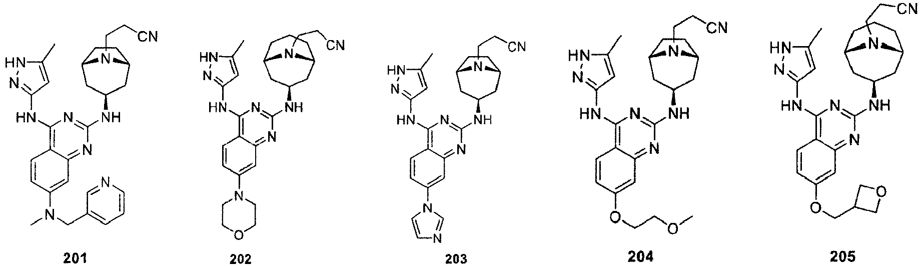

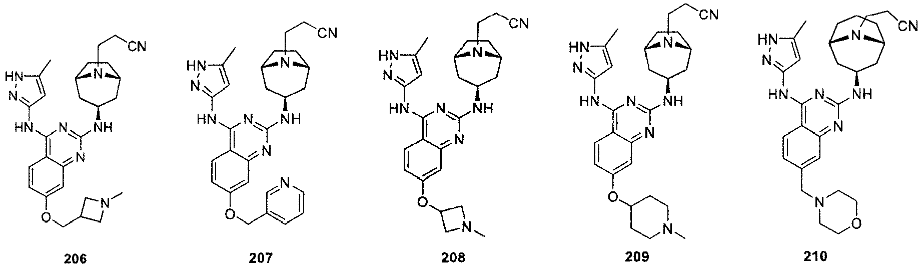

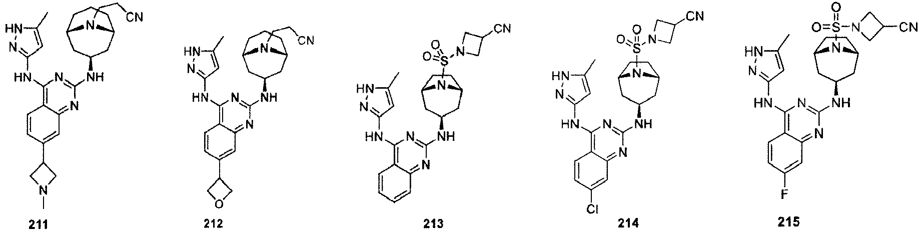

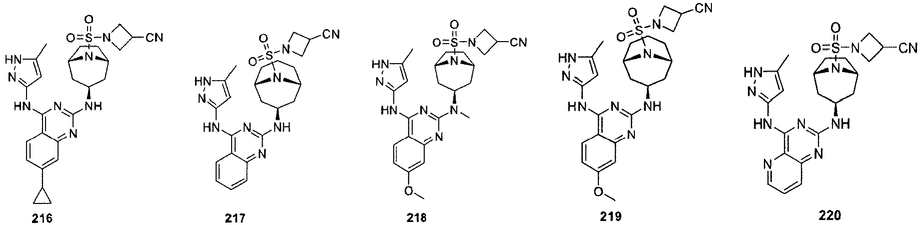

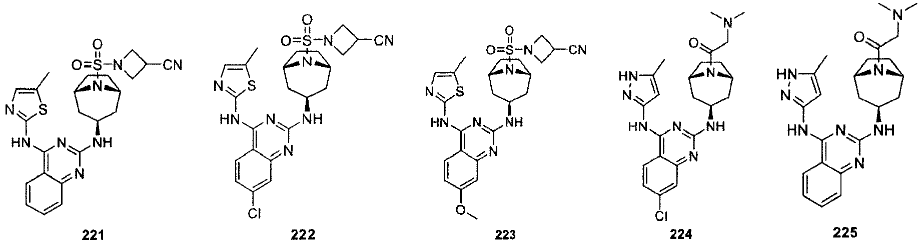

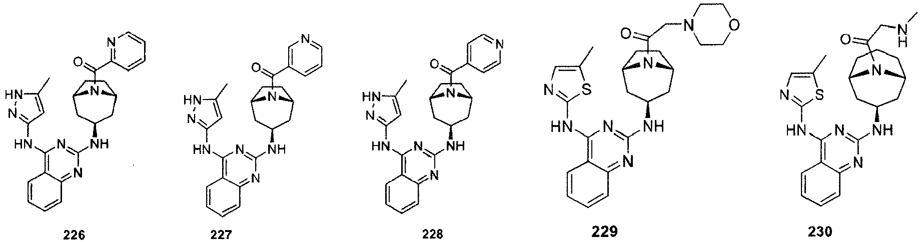

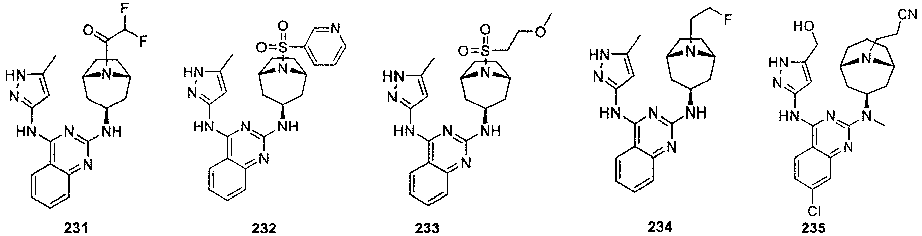

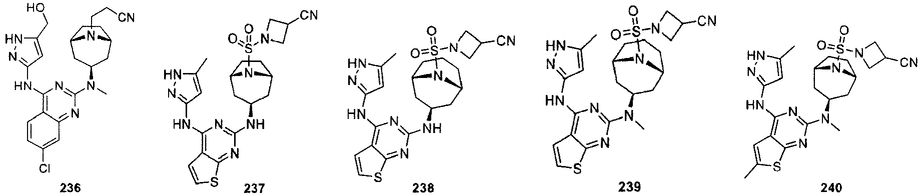

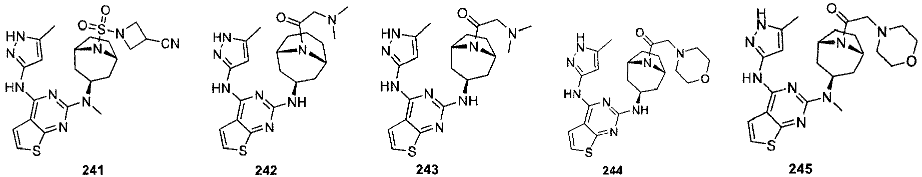

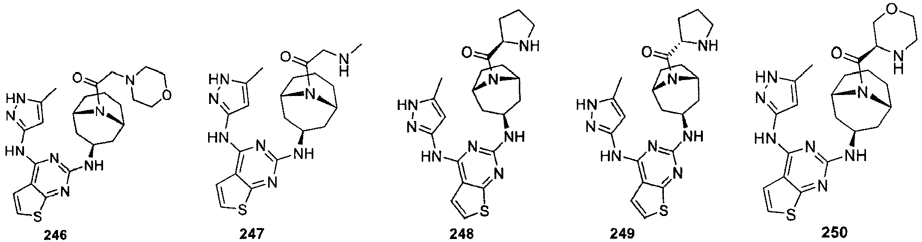

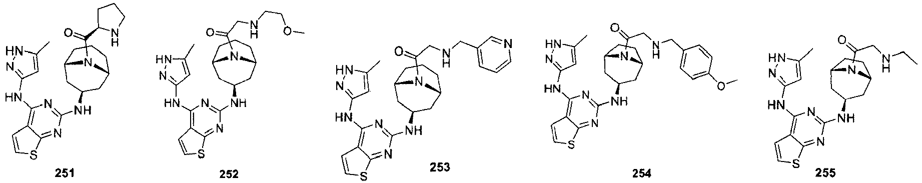

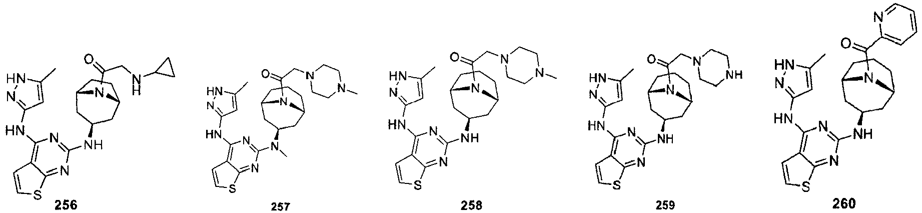

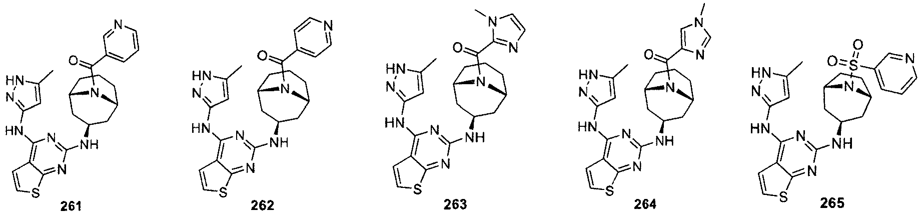

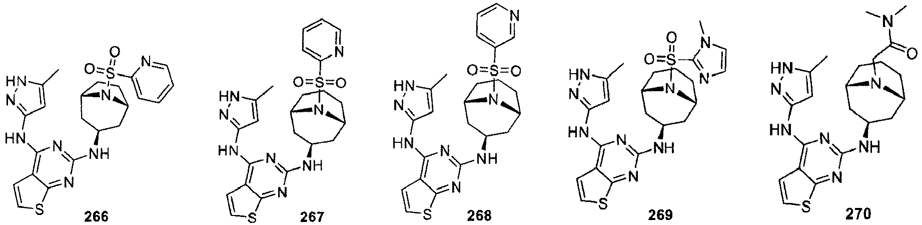

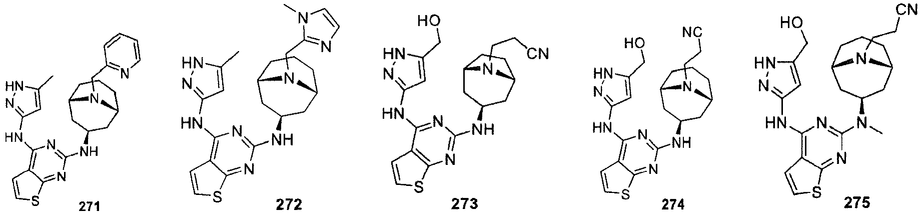

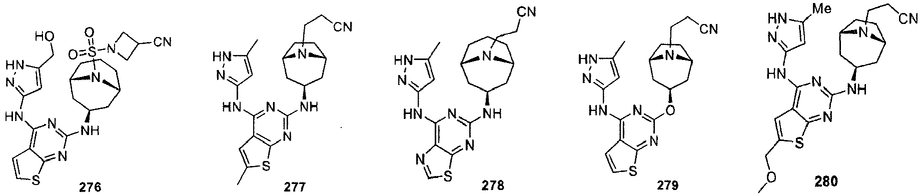

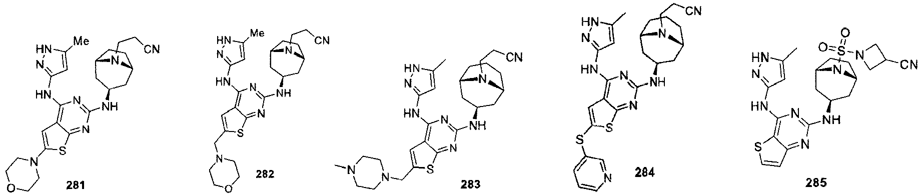

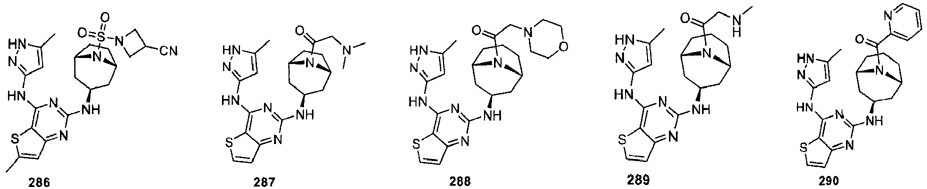

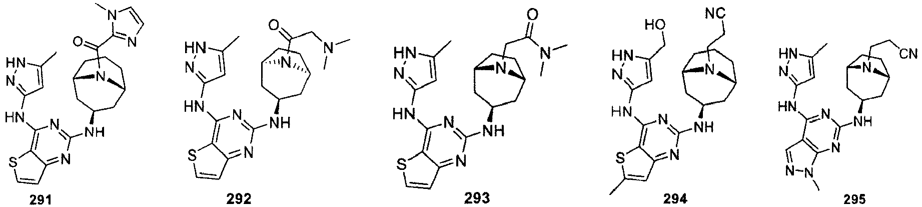

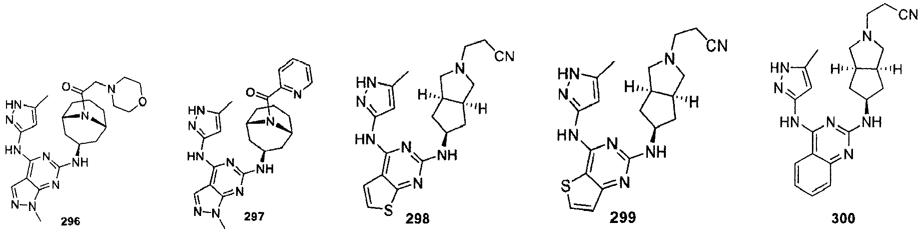

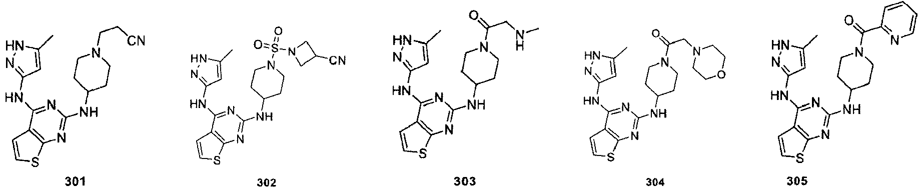

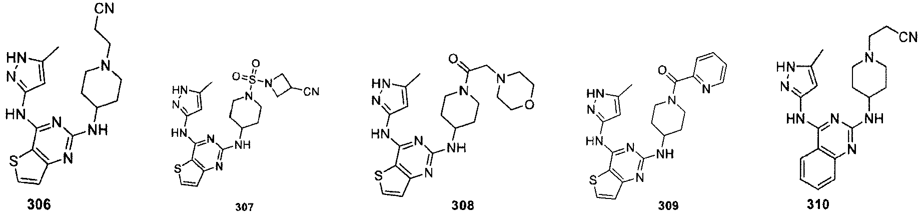

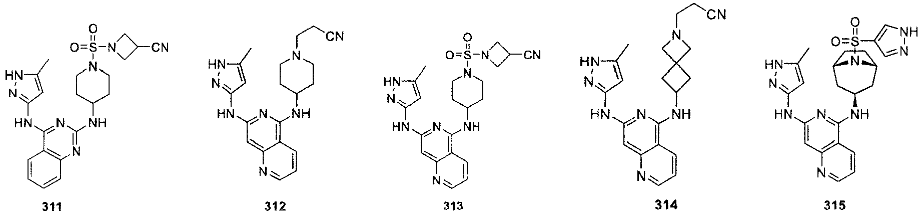

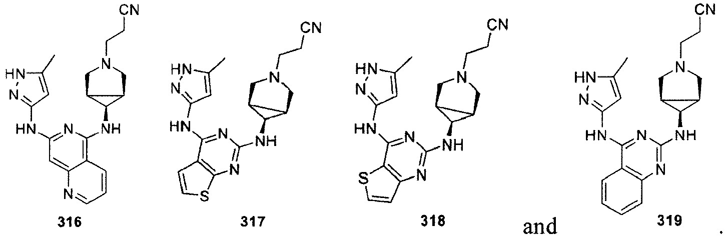

An object of the present disclosure is to provide a compound of general formula (I), a stereoisomer thereof or a pharmaceutically acceptable salt thereof, the structure of the compound of general formula (I) is as follows: 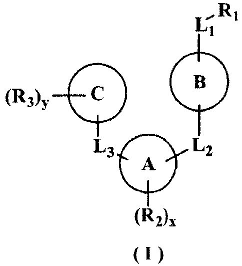 wherein:
L₁ is selected from bond, alkylene, alkenylene, alkynyl, cycloalkyl, heterocyclyl, - (CH₂)ₙ₁C(O)(CH₂)ₘ₁-, -(CH₂)ₙ₁C(O)(CH₂)ₘ₁NRₐₐ(CH₂)ₘ₂-, -NRₐₐ(CH₂)ₙ₁-, -(CH₂)ₙ₁S(O)ₘ₁- or - (CH₂)ₙ₁S(O)ₘ₁NRₐₐ-;
L₂ is selected from bond, oxygen atom, sulfur atom, -CRₐₐR_{bb}-, -(CH₂)ₙ₁C(O)-, -NR₄- or -(CH₂)ₙ₁S(O)ₘ₁-;
L₃ is selected from bond, -NRaa- or -C(O)NRₐₐ-;
ring A is selected from 6-14 membered heteroaryl, wherein the 6-14 membered heteroaryl is selected from 6-14 membered fused heteroaryl; preferably 5,5 fused heteroaryl, 5,6 fused heteroaryl or 6,6 fused heteroaryl;
ring B is selected from 3-10 membered monocyclic heterocyclyl, 6-14 membered bridged heterocyclyl or 6-14 membered fused heterocyclyl;
ring C is selected from heteroaryl;
R₁ is selected from hydrogen atom, deuterium atom, oxo, alkyl, deuterated alkyl, haloalkyl, alkoxy, haloalkoxy, hydroxyalkyl, halogen, amino, nitro, hydroxyl, cyano, alkenyl, alkynyl, cycloalkyl, heterocyclyl, oxoheterocyclyl, thioxoheterocyclyl, aryl, heteroaryl, - (CH₂)ₙ₁Rₐₐ, -(CH₂)ₙ₁ORₐₐ, -NRₐₐC(O)(CH₂)ₙ₁ORₐₐ, -NRₐₐC(=S)(CH₂)ₙ₁OR_{bb}, -(CH₂)ₙ₁SRₐₐ, - (CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, - (CH₂)ₙ₁C(O)NRₐₐR_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, alkyl, haloalkyl, halogen, amino, oxo, nitro, cyano, hydroxyl, alkenyl, alkynyl, alkoxy, haloalkoxy, hydroxyalkyl, cycloalkyl, heterocyclyl, aryl, heteroaryl, - (CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc}, - (CH₂)ₙ₁S(O)ₘ₁R_{cc}, -(CH₂)ₙ₁NR_{cc}R_{dd}, -(CH₂)ₙ₁C(O)NR_{cc}R_{dd}, -(CH₂)ₙ₁NR_{cc}C(O)R_{dd} or - (CH₂)ₙ₁NR_{cc}S(O)ₘ₁R_{dd};
R₂ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl, heteroaryl, -(CH₂)ₙ₁Rₐₐ, - (CH₂)ₙ₁O(CH₂)ₘ₁Rₐₐ, -(CH₂)ₙ₁ORₐₐ, -(CH₂)ₙ₁NRₐₐ(CH₂)ₘ₁Rₐₐ, -NRₐₐC(O)(CH₂)ₙ₁ORₐₐ, - NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁S(O)ₘ₁NRₐₐR_{bb}, -(CH₂)ₙ₁P(O)ₘ₂RₐₐR_{bb}, -(CH₂)ₙ₁NRₐₐR_{bb}, -(CH₂)ₙ₁S-, - (CH₂)ₙ₁C(O)NRₐₐR_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted, unsubstituted heteroaryl, - (CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc}, - (CH₂)ₙ₁S(O)ₘ]R_{cc}, -(CH₂)ₙ₁S(O)ₘ₁NR_{cc}R_{dd}, -(CH₂)ₙ₁NR_{cc}R_{dd}, -(CH₂)ₙ₁C(O)NR_{cc}R_{dd}, - (CH₂)ₙ₁C(O)N_{Rcc}S(O)ₘ₁R_{dd}, -(CH₂)ₙ₁NR_{cc}S(O)ₘ₁R_{dd} or -(CH₂)ₙ₁NR_{cc}S(O)ₘ₁R_{dd};
R₃ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl, heteroaryl, -(CH₂)ₙ₁Rₐₐ, - (CH₂)ₙ₁ORₐₐ, -NRₐₐC(O)(CH₂)ₙ₁ORₐₐ, -NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁S(O)ₘ₁NRₐₐR_{bb}, -(CH₂)ₙ₁P(O)ₘ₂RₐₐR_{bb}, - (CH₂)ₙ₁NRₐₐR_{bb}, -(CH₂)ₙ₁C(O)NRₐₐR_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted, unsubstituted heteroaryl, -(CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, - (CH₂)ₙ₁(O)OR_{cc}, -(CH₂)ₙ₁S(O)ₘ₁R_{cc}, -(CH₂)ₙ₁S(O)ₘ₁NR_{cc}R_{dd}, -(CH₂)ₙ₁NR_{cc}R_{dd}, - (CH₂)ₙ₁C(O)NR_{cc}R_{dd}, -(CH₂)ₙ₁C(O)NR_{cc}S(O)ₘ₁R_{dd}, -(CH₂)ₙ₁NR_{cc}S(O)ₘ₁R_{dd} or - (CH₂)ₙ₁NR_{cc}S(O)ₘ₁R_{dd};
R₄ is selected from hydrogen atom, deuterium atom, alkyl, deuterated alkyl, haloalkyl, alkoxy, hydroxyalkyl, haloalkoxy, halogen, amino, nitro, hydroxyl, cyano, alkenyl, alkynyl;
Rₐₐ, R_{bb}, R_{cc} and R_{dd} are identical or different and are each independently selected from hydrogen atom, deuterium atom, alkyl, deuterated alkyl, haloalkyl, alkoxy, hydroxyalkyl, haloalkoxy, halogen, cyano, nitro, hydroxyl, amino, alkenyl, alkynyl, cycloalkyl, heterocyclyl, aryl and heteroaryl, wherein the alkyl, deuterated alkyl, haloalkyl, alkoxy, hydroxyalkyl, haloalkoxy, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted or unsubstituted heteroaryl;
x is an integer of 0, 1, 2 or 3;
y is an integer of 0, 1, 2, 3, 4 or 5;
m₁ is an integer of 0, 1 or 2;
m₂ is an integer of 0, 1 or 2; and
n₁ is an integer of 0, 1, 2, 3, 4 or 5.

The present disclosure also relates to a preferred embodiment of the general formula (I), the stereoisomer thereof or the pharmaceutically acceptable salt thereof as described above, wherein,
wherein:
L₁ is selected from bond, alkylene, alkenylene, alkynyl, cycloalkyl, heterocyclyl, - NRₐₐ(CH₂)ₙ₁-, -(CH₂)ₙ₁S(O)ₘ₁- or -(CH₂)ₙ₁S(O)ₘ₁NRₐₐ-;
L₂ is selected from bond, oxygen atom, sulfur atom, -CRaaRbb-, -(CH₂)ₙ₁C(O)-, -NR₄- or -(CH₂)ₙ₁S(O)ₘ₁-;
L₃ is selected from bond, -NRₐₐ- or -C(O)NRₐₐ-;
ring A is selected from 6-14 membered heteroaryl, wherein the 6-14 membered heteroaryl is selected from 6-14 membered fused heteroaryl; preferably 5,5 fused heteroaryl, 5,6 fused heteroaryl or 6,6 fused heteroaryl;
ring B is selected from 6-14 membered bridged heterocyclyl or 6-14 membered fused heterocyclyl;
ring C is selected from heteroaryl;
R₁ is selected from hydrogen atom, deuterium atom, oxo, alkyl, deuterated alkyl, haloalkyl, alkoxy, haloalkoxy, hydroxyalkyl, halogen, amino, nitro, hydroxyl, cyano, alkenyl, alkynyl, cycloalkyl, heterocyclyl, oxoheterocyclyl, thioxoheterocyclyl, aryl, heteroaryl, - (CH₂)ₙ₁Rₐₐ, -(CH₂)ₙ₁ORₐₐ, -NRₐₐC(O)(CH₂)ₙ₁ORₐₐ, -NRₐₐC(=S)(CH₂)ₙ₁OR_{bb}, -(CH₂)ₙ₁SRₐₐ, - (CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, - (CH₂)ₙ₁C(O)NRₐₐR_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, alkyl, haloalkyl, halogen, amino, oxo, nitro, cyano, hydroxyl, alkenyl, alkynyl, alkoxy, haloalkoxy, hydroxyalkyl, cycloalkyl, heterocyclyl, aryl, heteroaryl, - (CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc}, - (CH₂)ₙ₁S(O)ₘ₁R_{cc}, -(CH₂)ₙ₁NR_{cc}R_{dd}, -(CH₂)ₙ₁C(O)NR_{cc}R_{dd}, -(CH₂)ₙ₁NR_{cc}C(O)R_{dd} or - (CH₂)ₙ₁NR_{cc}S(O)ₘ₁R_{dd};
R₂ is selected from hydrogen atom, deuterium atom, alkyl, deuterated alkyl, oxo, haloalkyl, alkoxy, hydroxyalkyl, haloalkoxy, halogen, amino, nitro, hydroxyl, cyano, alkenyl or alkynyl;
R₃ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl, heteroaryl, -(CH₂)ₙ₁Rₐₐ, - (CH₂)ₙ₁ORₐₐ, -NRₐₐC(O)(CH₂)ₙ₁ORₐₐ, -NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁S(O)ₘ₁NRₐₐR_{bb}, -(CH₂)ₙ₁P(O)ₘ₂RₐₐR_{bb}, - (CH₂)ₙ₁NRₐₐR_{bb}, -(CH₂)ₙ₁C(O)NRₐₐR_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted, unsubstituted heteroaryl, -(CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, - (CH₂)ₙ₁C(O)OR_{cc}, -(CH₂)ₙ₁S(O)ₘ₁R_{cc}, -(CH₂)ₙ₁S(O)ₘ₁NR_{cc}R_{dd}, -(CH₂)ₙ₁NR_{cc}R_{dd}, - (CH₂)ₙ₁C(O)NR_{cc}R_{dd}, -(CH₂)ₙ₁C(O)NR_{cc}S(O)ₘ₁R_{dd}, -(CH₂)ₙ₁NR_{cc}S(O)ₘ₁R_{dd} or - (CH₂)ₙ₁NR_{cc}S(O)ₘ₁R_{dd};
R₄ is selected from hydrogen atom, deuterium atom, alkyl, deuterated alkyl, haloalkyl, alkoxy, hydroxyalkyl, haloalkoxy, halogen, amino, nitro, hydroxyl, cyano, alkenyl, alkynyl;
Rₐₐ, R_{bb}, R_{cc} and R_{dd} are identical or different and are each independently selected from hydrogen atom, deuterium atom, alkyl, deuterated alkyl, haloalkyl, alkoxy, hydroxyalkyl, haloalkoxy, halogen, cyano, nitro, hydroxyl, amino, alkenyl, alkynyl, cycloalkyl, heterocyclyl, aryl and heteroaryl, wherein the alkyl, deuterated alkyl, haloalkyl, alkoxy, hydroxyalkyl, haloalkoxy, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted or unsubstituted heteroaryl;
x is an integer of 0, 1, 2 or 3;
y is an integer of 0, 1, 2, 3, 4 or 5;
m₁ is an integer of 0, 1 or 2;
m₂ is an integer of 0, 1 or 2; and
n₁ is an integer of 0, 1, 2, 3, 4 or 5.

The present disclosure also relates to a preferred embodiment of the general formula (I), the stereoisomer thereof or the pharmaceutically acceptable salt thereof, which is a compound of general formula (II) and a compound of general formula (IIA), a stereoisomer thereof or a pharmaceutically acceptable salt thereof: 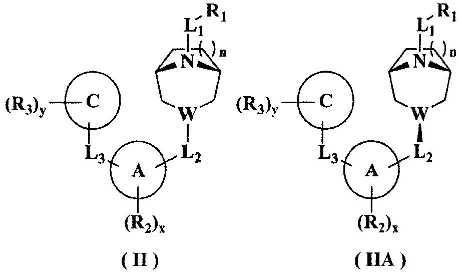 wherein:
W is selected from nitrogen atom or alkylene; and
n is an integer of 0, 1, 2 or 3;
L₁, L₂, L₃, ring A, ring C, R₁-R₃, x and y are as defined in general formula (I).

The present disclosure also relates to a preferred embodiment of the general formula (I), the stereoisomer thereof or the pharmaceutically acceptable salt thereof, which is a compound of general formula (IIIA), a stereoisomer thereof or a pharmaceutically acceptable salt thereof: 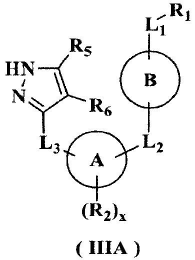 wherein:
R₅ is selected from hydrogen atom, halogen, cyano, alkyl, alkoxy, hydroxyalkyl, haloalkyl, -(CH₂)ₙ₁ORₐₐ, -(CH₂)ₙ₁C(O)NRₐₐR_{bb}, cycloalkyl, heterocyclyl, aryl or heteroaryl, wherein the alkyl, alkoxy, hydroxyalkyl, haloalkyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted, unsubstituted heteroaryl, -(CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc}, -(CH₂)ₙ₁S(O)ₘ₁R_{cc}, - (CH₂)ₙ₁S(O)ₘ₁NR_{cc}R_{dd}, -(CH₂)ₙ₁NR_{cc}R_{dd}, -(CH₂)ₙ₁C(O)NR_{cc}R_{dd}, -(CH₂)ₙ₁C(O)NR_{cc}S(O)ₘ₁R_{dd}, -(CH₂)ₙ₁NR_{cc}S(O)ₘ₁R_{dd} or -(CH₂)ₙ₁NR_{cc}S(O)ₘ₁R_{dd};
R₆ is selected from hydrogen atom, halogen, cyano, alkyl, alkoxy, hydroxyalkyl, haloalkyl, cycloalkyl, heterocyclyl or -(CH₂)ₙ₁C(O)NRₐₐR_{bb};
L₁, L₂, L₃, ring A, ring B, R₁, R₂ and x are as defined in general formula (I).

The present disclosure also relates to a preferred embodiment of the general formula (I), the stereoisomer thereof or the pharmaceutically acceptable salt thereof, which is a compound of general formula (III), a stereoisomer thereof or a pharmaceutically acceptable salt thereof: 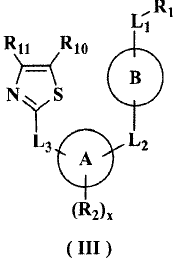 wherein:
R₁₀ and R₁₁ are identical or different and are independently selected from hydrogen atom, halogen, cyano, alkyl, alkoxy, hydroxyalkyl, haloalkyl, -(CH₂)ₙ₁ORₐₐ, -(CH₂)ₙ₁C(O)NRₐₐR_{bb}, - (CH₂)ₙ₁S(O)ₘ₁NRₐₐR_{bb}-, cycloalkyl, heterocyclyl, aryl or heteroaryl, wherein the alkyl, alkoxy, hydroxyalkyl, haloalkyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted, unsubstituted heteroaryl, -(CH₂)ₙ₁-, -(CH₂)ₙ₁R_{cc}, -(CH₂)ₙ₁OR_{cc}, - (CH₂)ₙ₁SR_{cc}, -(CH₂)ₙ₁C(O)R_{cc}, -(CH₂)ₙ₁C(O)OR_{cc}, -(CH₂)ₙ₁S(O)ₘ₁R_{cc}, -(CH₂)ₙ₁S(O)ₘ₁NR_{cc}R_{dd}, -(CH₂)ₙ₁NR_{cc}R_{dd}, -(CH₂)ₙ₁C(O)NR_{cc}R_{dd}, -(CH₂)ₙ₁C(O)NR_{cc}S(O)ₘ₁R_{dd}, -(CH₂)ₙ₁NR_{cc}S(O)ₘ₁R_{dd} or -(CH₂)ₙ₁NR_{cc}S(O)ₘ₁R_{dd};
L₁, L₂, L₃, ring A, ring B, R₁, R₂ and x are as defined in general formula (I).

The present disclosure also relates to a preferred embodiment of the general formula (I), the stereoisomer thereof or the pharmaceutically acceptable salt thereof, which is a compound of general formula (IV), a stereoisomer thereof or a pharmaceutically acceptable salt thereof: 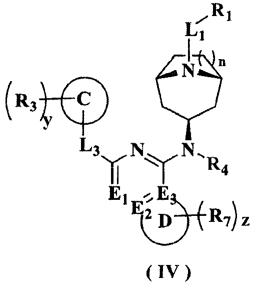 wherein:
ring D is selected from heterocyclyl, aryl or heteroaryl; preferably 5-6 membered heterocyclyl, 6-10 membered aryl or 5-6 membered heteroaryl;
E₁, E₂ and E₃ are identical or different and are each independently selected from nitrogen atom or -CRₐₐ-;
R₄ is selected from hydrogen atom, halogen, cyano, alkyl, alkenyl, alkynyl or alkoxy;
R₇ is selected from hydrogen atom, halogen, cyano, alkyl, alkenyl, alkynyl, alkoxy, oxo or thioxo; and
z is an integer of 0, 1, 2 or 3;
L₁, L₃, ring C, R₁, R₃, Rₐₐ, y and n are as defined in general formula (I).

The present disclosure also relates to a preferred embodiment of the general formula (I), the stereoisomer thereof or the pharmaceutically acceptable salt thereof, which is a compound of general formula (V), a stereoisomer thereof or a pharmaceutically acceptable salt thereof: 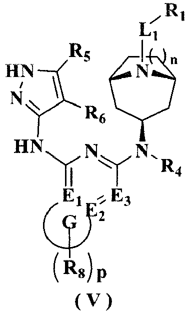 wherein:
ring G is selected from heterocyclyl, aryl or heteroaryl; preferably 5-6 membered heterocyclyl, 6-10 membered aryl or 5-6 membered heteroaryl;
R₈ is selected from hydrogen atom, halogen, cyano, alkyl, alkenyl, alkynyl, alkoxy, oxo or thioxo; and
p is an integer of 0, 1, 2 or 3;
R₅ and R₆ are as defined in claim 3;
L₁, E₁, E₂, E₃, R₄ and n are as defined in general formula (IV).

The present disclosure also relates to a preferred embodiment of the general formula (I), the stereoisomer thereof or the pharmaceutically acceptable salt thereof, which is a compound of general formula (VI), a stereoisomer thereof or a pharmaceutically acceptable salt thereof: 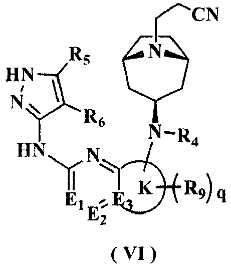 wherein:
ring K is selected from heterocyclyl, aryl or heteroaryl; preferably 5-6 membered heterocyclyl, 6-10 membered aryl, 5-6 membered heteroaryl;
R₉ is selected from hydrogen atom, halogen, cyano, alkyl, alkenyl, alkynyl, alkoxy, oxo or thioxo; and
q is an integer of 0, 1, 2 or 3;
R₅ and R₆ are as defined in claim 3;
E₁, E₂, E₃ and R₄ are as defined in general formula (IV).

The present disclosure also relates to a preferred embodiment of the general formula (I), the stereoisomer thereof or the pharmaceutically acceptable salt thereof, which is a compound of general formula (VII), a stereoisomer thereof or a pharmaceutically acceptable salt thereof: 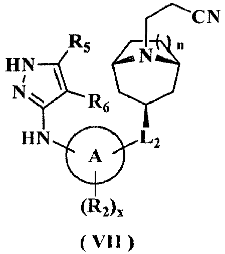 wherein:
n is an integer of 0, 1 or 2;
L₂, ring A, R₂, R₅, R₆ and x are as defined in general formula (IIIA).

The present disclosure also relates to a preferred embodiment of the general formula (III), the stereoisomer thereof or the pharmaceutically acceptable salt thereof, which is a compound of general formula (VIII), a stereoisomer thereof or a pharmaceutically acceptable salt thereof: 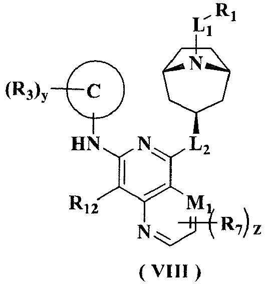 wherein:
M₁ is selected from nitrogen atom or -CRₐₐ-;
R₁₂ is selected from hydrogen atom, cyano, halogen, alkyl or alkoxy; and
z is an integer of 0, 1 or 2;
L₁, L₂, ring C, R₁, R₃, R₇, Rₐₐ and y are as defined in general formula (IV).

The present disclosure also relates to a preferred embodiment of the general formula (I), the stereoisomer thereof or the pharmaceutically acceptable salt thereof, which is a compound of the general formula (IX), a stereoisomer thereof or a pharmaceutically acceptable salt thereof: 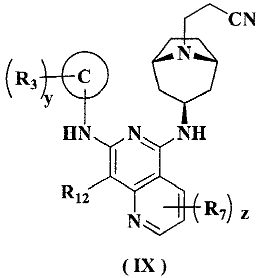 wherein:
ring C, R₃, R₁₂, R₇, y and z are as defined in general formula (VII).

The present disclosure also relates to a preferred embodiment of the general formula (I), the stereoisomer thereof or the pharmaceutically acceptable salt thereof, which is a compound of general formula (X), a stereoisomer thereof or a pharmaceutically acceptable salt thereof: 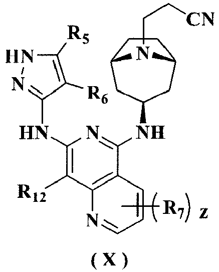 wherein:
R₅-R₆ are as defined in general formula (IIIA);
R₁₂, R₇ and z are as defined in general formula (VII).

The present disclosure also relates to a preferred embodiment of the general formula (I), the stereoisomer thereof or the pharmaceutically acceptable salt thereof, which is a compound of general formula (XI), a stereoisomer thereof or a pharmaceutically acceptable salt thereof: 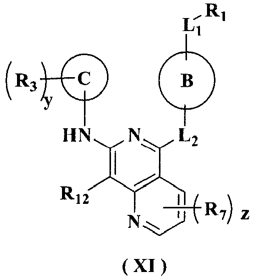 wherein:
L₁, L₂, ring B, ring C, R₃ and R₁₂ are as defined in general formula (I)
R₇, y and z are as defined in general formula (IX).

The present disclosure also relates to a preferred embodiment of the general formula (I), the stereoisomer thereof or the pharmaceutically acceptable salt thereof, which is a compound of general formula (XII), a stereoisomer thereof or a pharmaceutically acceptable salt thereof: 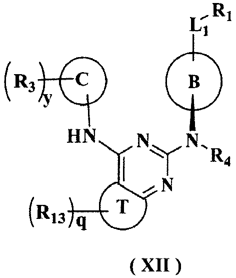 wherein:
L₁ is selected from bond, alkylene, alkenylene, alkynyl, cycloalkyl, heterocyclyl, - (CH₂)ₙ₁C(O)(CH₂)ₘ₁-, -(CH₂)ₙ₁C(O)(CH₂)ₘ₁NRₐₐ(CH₂)ₘ₂-, -(CH₂)ₙ₁S(O)ₘ₁- or - (CH₂)ₙ₁S(O)ₘ₁NRₐₐ-;
ring B is selected from 3-10 membered monocyclic heterocyclyl, 6-14 membered spiro heterocyclyl, 6-14 membered bridged heterocyclyl or 6-14 membered fused heterocyclyl;
ring T is selected from aryl or heteroaryl;
R₁ is selected from hydrogen atom, deuterium atom, oxo, alkyl, deuterated alkyl, haloalkyl, alkoxy, haloalkoxy, hydroxyalkyl, halogen, amino, nitro, hydroxyl, cyano, alkenyl, alkynyl, cycloalkyl, heterocyclyl, oxoheterocyclyl, thioxoheterocyclyl, aryl, heteroaryl, - (CH₂)ₙ₁Rₐₐ, -(CH₂)ₙ₁ORₐₐ, -NRₐₐC(O)(CH₂)ₙ₁ORₐₐ, -NRₐₐC(=S)(CH₂)ₙ₁OR_{bb}, -(CH₂)ₙ₁SRₐₐ, - (CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, - (CH₂)ₙ₁C(O)NRₐₐR_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, alkyl, haloalkyl, halogen, amino, oxo, nitro, cyano, hydroxyl, alkenyl, alkynyl, alkoxy, haloalkoxy, hydroxyalkyl, cycloalkyl, heterocyclyl, aryl, heteroaryl, - (CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc}, - (CH₂)ₙ₁S(O)ₘ₁R_{cc}, -(CH₂)ₙ₁NR_{cc}R_{dd}, -(CH₂)ₙ₁C(O)NR_{cc}R_{dd}, -(CH₂)ₙ₁NR_{cc}C(O)R_{dd} or - (CH₂)ₙ₁NR_{cc}S(O)ₘ₁R_{dd};
R₃ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl, heteroaryl, -(CH₂)ₙ₁Rₐₐ, - (CH₂)ₙ₁ORₐₐ, -NRₐₐC(O)(CH₂)ₙ₁ORₐₐ, -NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁S(O)ₘ₁NRₐₐR_{bb}, -(CH₂)ₙ₁P(O)ₘ₂RₐₐR_{bb}, - (CH₂)ₙ₁NRₐₐR_{bb}, -(CH₂)ₙ₁C(O)N_{Raa}R_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted, unsubstituted heteroaryl, -(CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, - (CH₂)ₙ₁C(O)OR_{cc}, -(CH₂)ₙ₁S(O)ₘ₁R_{cc}, -(CH₂)ₙ₁S(O)ₘ₁NR_{cc}R_{dd}, -(CH₂)ₙ₁NR_{cc}R_{dd}, - (CH₂)ₙ₁C(O)NR_{cc}R_{dd}, -(CH₂)ₙ₁C(O)NR_{cc}S(O)ₘ₁R_{dd}, -(CH₂)ₙ₁NR_{cc}S(O)ₘ₁R_{dd} or - (CH₂)ₙ₁NR_{cc}S(O)ₘ₁R_{dd};
R₄ is selected from hydrogen atom, deuterium atom, alkyl, deuterated alkyl, haloalkyl, alkoxy, hydroxyalkyl, haloalkoxy, halogen, amino, nitro, hydroxyl, cyano, alkenyl, alkynyl;
R₁₃ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl, heteroaryl, -(CH₂)ₙ₁Rₐₐ, - (CH₂)ₙ₁O(CH₂)ₘ₁Rₐₐ, -(CH₂)ₙ₁ORₐₐ, -(CH₂)ₙ₁NRₐₐ(CH₂)ₘ₁Rₐₐ, -NRₐₐC(O)(CH₂)ₙ₁ORₐₐ, - NRₐₐC(=S)(CH₂)ₙₜOR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁S(O)ₘ₁NRₐₐR_{bb}, -(CH₂)ₙ₁P(O)ₘ₂RₐₐR_{bb}, -(CH₂)ₙ₁NRₐₐR_{bb}, -(CH₂)ₙ₁S-, - (CH₂)ₙ₁C(O)NRₐₐR_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted, unsubstituted heteroaryl, - (CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc}, - (CH₂)ₙ₁S(O)ₘ₁R_{cc}, -(CH₂)ₙ₁S(O)ₘ₁NR_{cc}R_{dd}, -(CH₂)ₙ₁NR_{cc}R_{dd}, -(CH₂)ₙ₁C(O)NR_{cc}R_{dd}, - (CH₂)ₙ₁C(O)NR_{cc}S(O)ₘ₁R_{dd}, -(CH₂)ₙ₁NR_{cc}S(O)ₘ₁R_{dd} or -(CH₂)ₙ₁NR_{cc}S(O)ₘ₁R_{dd};
Rₐₐ, R_{bb}, R_{cc} and R_{dd} are identical or different and are each independently selected from hydrogen atom, deuterium atom, alkyl, deuterated alkyl, haloalkyl, alkoxy, hydroxyalkyl, haloalkoxy, halogen, cyano, nitro, hydroxyl, amino, alkenyl, alkynyl, cycloalkyl, heterocyclyl, aryl and heteroaryl, wherein the alkyl, deuterated alkyl, haloalkyl, alkoxy, hydroxyalkyl, haloalkoxy, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted or unsubstituted heteroaryl;
y is an integer of 0, 1, 2, 3, 4 or 5;
q is an integer of 0, 1, 2, 3, 4, or 5;
m₁ is an integer of 0, 1 or 2;
m₂ is an integer of 0, 1 or 2; and
n₁ is an integer of 0, 1, 2, 3, 4 or 5.

The present disclosure also relates to a preferred embodiment of the general formula (I), the stereoisomer thereof or the pharmaceutically acceptable salt thereof, which is a compound of general formula (XIII), a stereoisomer thereof or a pharmaceutically acceptable salt thereof: 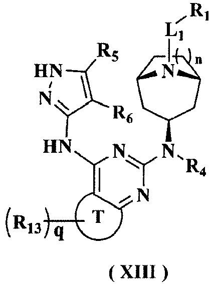 wherein:
R₅ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl or heteroaryl;
R₆ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl or heteroaryl;
n is an integer of 0, 1 or 2;
L₁, ring T, R₁, R₃, R₄, R₁₃ and q are as defined in general formula (XII).

The present disclosure also relates to a preferred embodiment of the general formula (I), the stereoisomer thereof or the pharmaceutically acceptable salt thereof, which is a compound of general formula (XIV), a stereoisomer thereof or a pharmaceutically acceptable salt thereof: 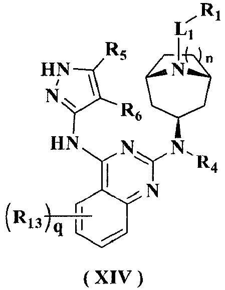 wherein:
L₁, R₁, R₄-R₆, R₁₃, q and n are as defined in general formula (XIII).

The present disclosure also relates to a preferred embodiment of the general formula (I), the stereoisomer thereof or the pharmaceutically acceptable salt thereof, which is a compound of general formula (XV), a stereoisomer thereof or a pharmaceutically acceptable salt thereof: 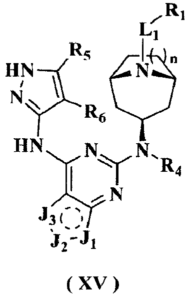 wherein:
L₁ is selected from bond, alkylene, alkenylene, alkynyl, cycloalkyl, heterocyclyl, - (CH₂)ₙ₁C(O)(CH₂)ₘ₁-, -(CH₂)ₙ₁C(O)(CH₂)ₘ₁NRₐₐ(CH₂)ₘ₂-, -(CH₂)ₙ₁S(O)ₘ₁- or - (CH₂)ₙ₁S(O)ₘ₁NRₐₐ-;
J₁, J₂ and J₃ are identical or different and are each independently selected from nitrogen atom, sulfur atom, oxygen atom, NRₐₐ or CR₁₄;
R₁ is selected from hydrogen atom, deuterium atom, oxo, alkyl, deuterated alkyl, haloalkyl, alkoxy, haloalkoxy, hydroxyalkyl, halogen, amino, nitro, hydroxyl, cyano, alkenyl, alkynyl, cycloalkyl, heterocyclyl, oxoheterocyclyl, thioxoheterocyclyl, aryl, heteroaryl, - (CH₂)ₙ₁Rₐₐ, -(CH₂)ₙ₁ORₐₐ, -NRₐₐC(O)(CH₂)ₙ₁ORₐₐ, -NRₐₐC(=S)(CH₂)ₙ₁OR_{bb}, -(CH₂)ₙ₁SRₐₐ, - (CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, - (CH₂)ₙ₁C(O)NRₐₐR_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, alkyl, haloalkyl, halogen, amino, oxo, nitro, cyano, hydroxyl, alkenyl, alkynyl, alkoxy, haloalkoxy, hydroxyalkyl, cycloalkyl, heterocyclyl, aryl, heteroaryl, - (CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc}, - (CH₂)ₙ₁S(O)ₘ₁R_{cc}, -(CH₂)ₙ₁NR_{cc}R_{dd}, -(CH₂)ₙ₁C(O)NR_{cc}R_{dd}, -(CH₂)ₙ₁NR_{cc}C(O)R_{dd} or - (CH₂)ₙ₁NR_{cc}S(O)ₘ₁R_{dd};
R₄ is selected from hydrogen atom, deuterium atom, alkyl, deuterated alkyl, haloalkyl, alkoxy, hydroxyalkyl, haloalkoxy, halogen, amino, nitro, hydroxyl, cyano, alkenyl, alkynyl;
R₅ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl or heteroaryl;
R₆ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl or heteroaryl;
R₁₄ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl, heteroaryl, -(CH₂)ₙ₁Rₐₐ, - (CH₂)ₙ₁O(CH₂)ₘ₁Rₐₐ, -(CH₂)ₙ₁ORₐₐ, -(CH₂)ₙ₁NRₐₐ(CH₂)ₘ₁Rₐₐ, -NRₐₐC(O)(CH₂)ₙ₁ORₐₐ, - NRₐₐC(=S)(CH₂)ₙₗOR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁S(O)ₘ₁NRₐₐR_{bb}, -(CH₂)ₙ₁P(O)ₘ₂RₐₐR_{bb}, -(CH₂)ₙ₁NRₐₐR_{bb}, -(CH₂)ₙ₁S-, - (CH₂)ₙ₁C(O)NRₐₐR_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted, unsubstituted heteroaryl, - (CH₂)n1-, -(CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc}, - (CH₂)ₙ₁S(O)ₘ₁R_{cc}, -(CH₂)ₙ₁S(O)ₘ₁NR_{cc}R_{dd}, -(CH₂)ₙ₁NR_{cc}R_{dd}, -(CH₂)ₙ₁C(O)NR_{cc}R_{dd}, - (CH₂)ₙ₁C(O)NR_{cc}S(O)ₘ₁R_{dd}, -(CH₂)ₙ₁NR_{cc}S(O)ₘ₁R_{dd} or -(CH₂)ₙ₁NR_{cc}S(O)ₘ₁R_{dd};
Rₐₐ, R_{bb}, R_{cc} and R_{dd} are identical or different and are each independently selected from hydrogen atom, deuterium atom, alkyl, deuterated alkyl, haloalkyl, alkoxy, hydroxyalkyl, haloalkoxy, halogen, cyano, nitro, hydroxyl, amino, alkenyl, alkynyl, cycloalkyl, heterocyclyl, aryl and heteroaryl, wherein the alkyl, deuterated alkyl, haloalkyl, alkoxy, hydroxyalkyl, haloalkoxy, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted or unsubstituted heteroaryl;
n is an integer of 0, 1 or 2;
m₁ is an integer of 0, 1 or 2;
m₂ is an integer of 0, 1 or 2; and
n₁ is an integer of 0, 1, 2, 3, 4 or 5.

The present disclosure also relates to a preferred embodiment of the general formula (I), the stereoisomer thereof or the pharmaceutically acceptable salt thereof, which is a compound of general formula (XVI), a stereoisomer thereof or a pharmaceutically acceptable salt thereof: 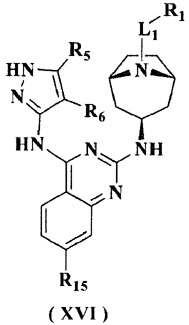 wherein:
R₁₅ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl, heteroaryl, -(CH₂)ₙ₁Rₐₐ, - (CH₂)ₙ₁(CH₂)ₘ₁Rₐₐ, -(CH₂)ₙ₁ORₐₐ, -(CH₂)ₙ₁NRₐₐ(CH₂)ₘ₁Rₐₐ, -NRₐₐC(O)(CH₂)ₙ₁ORₐₐ, - NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁S(O)ₘ₁NRₐₐR_{bb}, -(CH₂)ₙ₁P(O)ₘ₂RₐₐR_{bb}, -(CH₂)ₙ₁NRₐₐR_{bb}, -(CH₂)ₙ₁S-, - (CH₂)ₙ₁C(O)NRₐₐR_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted, unsubstituted heteroaryl, - (CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc}, - (CH₂)ₙ₁S(O)ₘ₁R_{cc}, -(CH₂)ₙ₁S(O)ₘ₁NR_{cc}R_{dd}, -(CH₂)ₙ₁NR_{cc}R_{dd}, -(CH₂)ₙ₁C(O)NR_{cc}R_{dd}, - (CH₂)ₙ₁C(O)NR_{cc}S(O)ₘ₁R_{dd}, -(CH₂)ₙ₁NR_{cc}S(O)ₘ₁R_{dd} or -(CH₂)ₙ₁NR_{cc}S(O)ₘ₁R_{dd};
L₁, R₁, R₅-R₆, Rₐₐ-R_{dd} and n are as defined in general formula (XIV).

The present disclosure also relates to a preferred embodiment of the general formula (I), the stereoisomer thereof or the pharmaceutically acceptable salt thereof, which is a compound of general formula (XVII), a stereoisomer thereof or a pharmaceutically acceptable salt thereof: 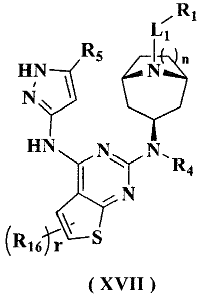 wherein:
R₁₆ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl, heteroaryl, -(CH₂)ₙ₁Rₐₐ, - (CH₂)ₙ₁O(CH₂)ₘ₁Rₐₐ, -(CH₂)ₙ₁ORₐₐ, -(CH₂)ₙ₁NRₐₐ(CH₂)ₘ₁Rₐₐ, -NRₐₐC(O)(CH₂)ₙ₁ORₐₐ, - NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁S(O)ₘ₁NRₐₐR_{bb}, -(CH₂)ₙ₁P(O)ₘ₂RₐₐR_{bb}, -(CH₂)ₙ₁NRₐₐR_{bb}, -(CH₂)ₙ₁S-, - (CH₂)ₙ₁C(O)NRₐₐR_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted, unsubstituted heteroaryl, - (CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc}, - (CH₂)ₙ₁S(O)ₘ₁R_{cc}, -(CH₂)ₙ₁S(O)ₘ₁NR_{cc}R_{dd}, -(CH₂)ₙ₁NR_{cc}R_{dd}, -(CH₂)ₙ₁C(O)NR_{cc}R_{dd}, - (CH₂)ₙ₁C(O)NR_{cc}S(O)ₘ₁R_{dd}, -(CH₂)ₙ₁NR_{cc}S(O)ₘ₁R_{dd} or -(CH₂)ₙ₁NR_{cc}S(O)ₘ₁R_{dd};
r is an integer of 0, 1 or 2; and
L₁, R₁, R₄-R₆, Rₐₐ-R_{dd}, n, n₁, m₁ and m₂ are as defined in general formula (XV).

The present disclosure also relates to a preferred embodiment of any one of the general formulas, the stereoisomer thereof or the pharmaceutically acceptable salt thereof, wherein,
ring A is selected from the following group: 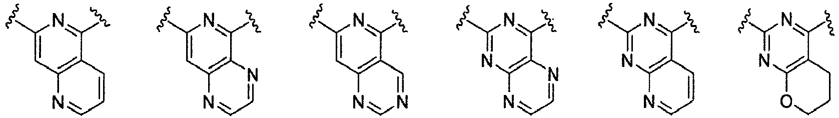 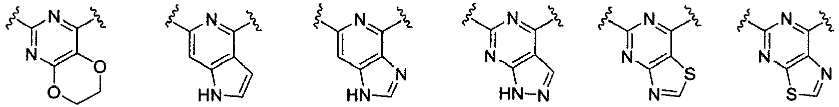 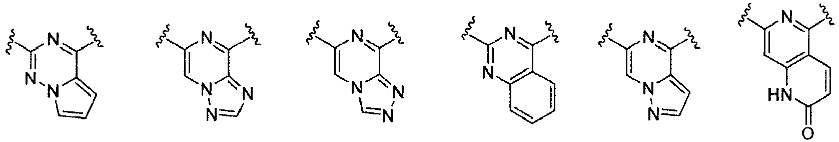 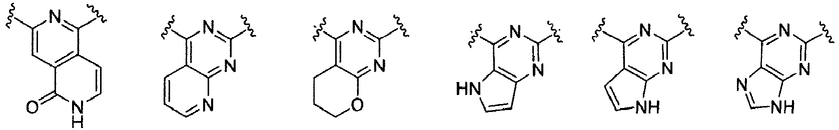 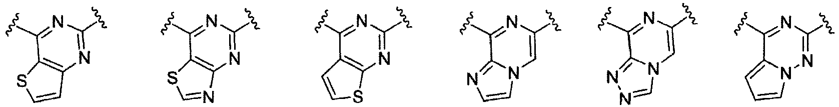 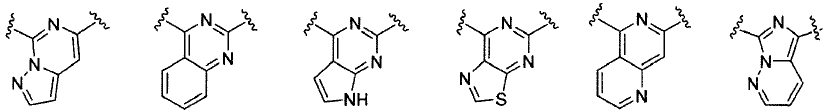 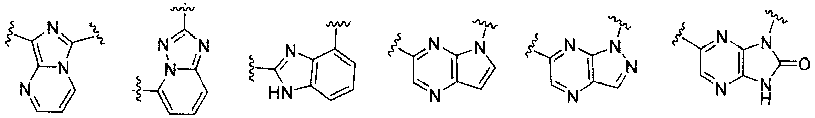 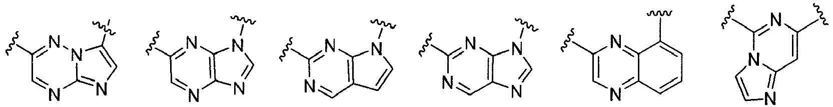 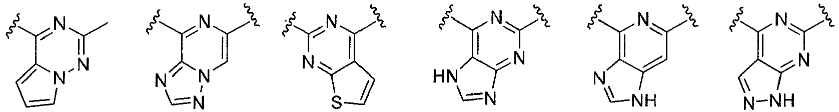 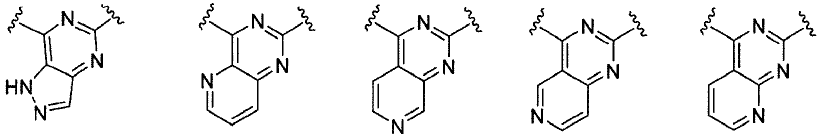 and 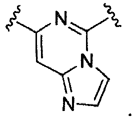
ring B is selected from the following group: 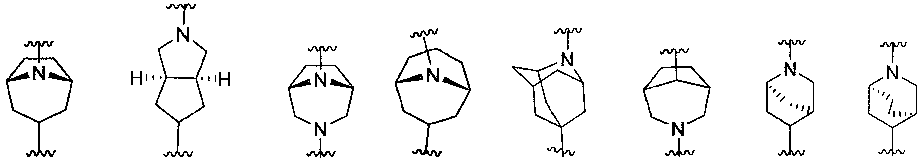 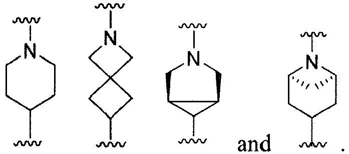
ring C is selected from the following group: 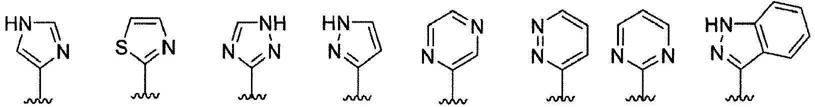 and

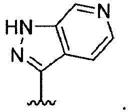

The present disclosure also relates to a preferred embodiment of any one of the general formulas, the stereoisomer thereof or the pharmaceutically acceptable salt thereof, wherein,
L₁ is C₃₋₈ cycloalkyl, C₃₋₈ heterocycloalkyl, -(CH₂)ₙ₁-, -(CH₂)ₙ₁C(O)(CH₂)ₘ₁-, - (CH₂)ₙ₁C(O)(CH₂)ₘ₁NRₐₐ(CH₂)ₘ₂-, -NRₐₐ(CH₂)ₙ₁-, -(CH₂)ₙ₁S(O)ₘ₁- or -(CH₂)ₙ₁S(O)ₘ₁NRₐₐ-;
L₂ is selected from bond, oxygen atom, -CRₐₐR_{bb}-, -(CH₂)ₙ₁C(O)-, -NR₄- or - (CH₂)ₙ₁S(O)ₘ₁-;
L₃ is selected from bond, -NRₐₐ- or -C(O)NRₐₐ-;
R₁ is selected from cyano, -(CH₂)ₙ₁Rₐₐ, -(CH₂)ₙ₁C(O)Rₐₐ, C₃₋₈ cycloalkyl, 3-10 membered heterocyclyl, C₆₋₁₀ aryl or 5-10 membered heteroaryl, wherein the C₃₋₈ cycloalkyl, 3-10 membered heterocyclyl, C₆₋₁₀ aryl and 5-10 membered heteroaryl are optionally further substituted with one or more substituents selected from hydrogen atom, cyano, C₁₋₆ alkyl, C₁₋₆ alkoxy;
R₂ is selected from hydrogen atom, halogen, cyano, hydroxyl, oxo, C₁₋₆ alkyl, C₁₋₆ alkoxy, C₃₋₈ cycloalkyl, 3-10 membered heterocyclyl, C₆₋₁₂ aryl, 5-10 membered heteroaryl, - (CH₂)ₙ₁Rₐₐ, -O(CH₂)ₙ₁Rₐₐ, -S(CH₂)ₙ₁Rₐₐ or -NRₐₐ(CH₂)ₙ₁R_{bb}, wherein the C₃₋₈ cycloalkyl, 3-10 membered heterocyclyl, C₆₋₁₀ aryl and 5-10 membered heteroaryl are optionally further substituted with one or more substituents selected from hydrogen atom, cyano, C₁₋₆ alkyl, C₁₋₆ alkoxy, C₃₋₈ cycloalkyl or -NR_{cc}R_{dd};
R₃ is selected from hydrogen atom, cyano, halogen, C₁₋₆ alkyl, C₁₋₆ haloalkyl, C₁₋₆ hydroxyalkyl, C₃₋₈ cycloalkyl, 3-10 membered heterocyclyl, -(CH₂)ₙ₁ORₐₐ, -C(O)NRₐₐR_{bb} or - (CH₂)ₙ₁S(O)ₘ₁Rₐₐ, wherein the cycloalkyl and heterocyclyl are optionally further substituted with one or more substituents selected from hydrogen atom, C₁₋₆ alkyl or hydroxyl;
R₄ is selected from hydrogen atom or methyl;
Rₐₐ, R_{bb}, R_{cc} and R_{dd} are identical or different and are each independently selected from hydrogen atom, C₁₋₆ alkyl, C₁₋₆ haloalkyl, cyano, hydroxyl, amino, C₃₋₈ cycloalkyl, 3-10 membered heterocyclyl, C_{6-1O} aryl or 5-10 membered heteroaryl; wherein the C₁₋₆ alkyl, C₁₋₆ haloalkyl, amino, C₃₋₈ cycloalkyl, 3-10 membered heterocyclyl, C₆₋₁₀ aryl and 5-10 membered heteroaryl are optionally further substituted with hydrogen atom, cyano, hydroxyl, amino, aminoalkyl, C₁₋₆ alkyl, C₁₋₆ alkoxy, C₃₋₈ cycloalkyl, 3-10 membered heterocyclyl, C₆₋₁₀ aryl or 5-10 membered heteroaryl.

An object of the present disclosure is to provide a compound of general formula (I), a stereoisomer thereof or a pharmaceutically acceptable salt thereof, wherein the structure of the compound of general formula (I) is as follows: 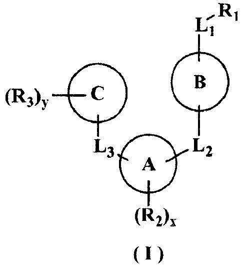 wherein:
L₁ is selected from bond, alkylene, alkenylene, alkynyl, cycloalkyl, heterocyclyl, - NRₐₐ(CH₂)ₙ₁-, -(CH₂)ₙ₁S(O)ₘ₁- or -(CH₂)ₙ₁S(O)ₘ₁NRₐₐ-;
L₂ is selected from bond, oxygen atom, sulfur atom, -CRₐₐR_{bb}-, -(CH₂)ₙ₁C(O)-, -NR₄- or -(CH₂)ₙ₁S(O)ₘ₁-;
L₃ is selected from bond, -NRₐₐ- or -C(O)NRₐₐ-;
ring A is selected from 6-14 membered heteroaryl, wherein the 6-14 membered heteroaryl is selected from 6-14 membered fused heteroaryl; preferably 5,5 fused heteroaryl, 5,6 fused heteroaryl or 6,6 fused heteroaryl;
ring B is selected from 6-14 membered bridged heterocyclyl or 6-14 membered fused heterocyclyl;
ring C is selected from heteroaryl;
R₁ is selected from hydrogen atom, deuterium atom, oxo, alkyl, deuterated alkyl, haloalkyl, alkoxy, haloalkoxy, hydroxyalkyl, halogen, amino, nitro, hydroxyl, cyano, alkenyl, alkynyl, cycloalkyl, heterocyclyl, oxoheterocyclyl, thioxoheterocyclyl, aryl, heteroaryl, - (CH₂)ₙ₁Rₐₐ, -(CH₂)ₙ₁ORₐₐ, -NRₐₐC(O)(CH₂)ₙ₁ORₐₐ, -NRₐₐC(=S)(CH₂)ₙ₁OR_{bb}, -(CH₂)ₙ₁SRₐₐ, - (CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, - (CH₂)ₙ₁C(O)NRₐₐR_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, alkyl, haloalkyl, halogen, amino, oxo, nitro, cyano, hydroxyl, alkenyl, alkynyl, alkoxy, haloalkoxy, hydroxyalkyl, cycloalkyl, heterocyclyl, aryl, heteroaryl, - (CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc}, - (CH₂)ₙ₁S(O)ₘ₁R_{cc}, -(CH₂)ₙ₁NR_{cc}R_{dd}, -(CH₂)ₙ₁C(O)NR_{cc}R_{dd}, -(CH₂)ₙ₁NR_{cc}C(O)R_{dd} or - (CH₂)ₙ₁NR_{cc}S(O)ₘ₁R_{dd};
R₂ is selected from hydrogen atom, deuterium atom, alkyl, deuterated alkyl, oxo, haloalkyl, alkoxy, hydroxyalkyl, haloalkoxy, halogen, amino, nitro, hydroxyl, cyano, alkenyl or alkynyl;
R₃ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl, heteroaryl, -(CH₂)ₙ₁Rₐₐ, - (CH₂)ₙ₁ORₐₐ, -NRₐₐC(O)(CH₂)ₙ₁ORₐₐ, -NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁S(O)ₘ₁NRₐₐR_{bb}, -(CH₂)ₙ₁P(O)ₘ₂RₐₐR_{bb}, - (CH₂)ₙ₁NRₐₐR_{bb}, -(CH₂)ₙ₁C(O)NRₐₐR_{bb}, -(CH₂)ₙ₁NRₐₐC(O)R_{bb} or -(CH₂)ₙ₁N_{Raa}S(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted, unsubstituted heteroaryl, -(CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, - (CH₂)ₙ₁C(O)OR_{cc}, -(CH₂)ₙ₁S(O)ₘ₁R_{cc}, -(CH₂)ₙ₁S(O)ₘ₁ NR_{cc}R_{dd}, -(CH₂)nlNR_{cc}R_{dd}, - (CH₂)ₙ₁C(O)NR_{cc}R_{dd}, -(CH₂)ₙ₁C(O)NR_{cc}S(O)ₘ₁R_{dd}, -(CH₂)ₙ₁NR_{cc}S(O)ₘ₁R_{dd} or - (CH₂)ₙ₁NR_{cc}S(O)ₘ₁R_{dd};
R₄ is selected from hydrogen atom, deuterium atom, alkyl, deuterated alkyl, haloalkyl, alkoxy, hydroxyalkyl, haloalkoxy, halogen, amino, nitro, hydroxyl, cyano, alkenyl, alkynyl;
Rₐₐ, R_{bb}, R_{cc} and R_{dd} are identical or different and are each independently selected from hydrogen atom, deuterium atom, alkyl, deuterated alkyl, haloalkyl, alkoxy, hydroxyalkyl, haloalkoxy, halogen, cyano, nitro, hydroxyl, amino, alkenyl, alkynyl, cycloalkyl, heterocyclyl, aryl and heteroaryl, wherein the alkyl, deuterated alkyl, haloalkyl, alkoxy, hydroxyalkyl, haloalkoxy, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted or unsubstituted heteroaryl;
x is an integer of 0, 1, 2 or 3;
y is an integer of 0, 1, 2, 3, 4 or 5;
m₁ is an integer of 0, 1 or 2;
m₂ is an integer of 0, 1 or 2; and
n₁ is an integer of 0, 1, 2, 3, 4, or 5.

The present disclosure also relates to a preferred embodiment of the general formula (I), the stereoisomer thereof or the pharmaceutically acceptable salt thereof, which is a compound of general formula (II) and the compound of general formula (IIA), a stereoisomer thereof or a pharmaceutically acceptable salt thereof: 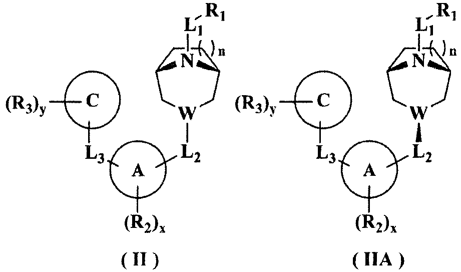 wherein:
W is selected from nitrogen atom or alkylene; and
n is an integer of 0, 1, 2 or 3;
L₁, L₂, L₃, ring A, ring C, R₁-R₃, x and y are as defined in general formula (I).

The present disclosure also relates to a preferred embodiment of the general formula (I), the stereoisomer thereof or the pharmaceutically acceptable salt thereof, which is a compound of general formula (IIIA), a stereoisomer thereof or a pharmaceutically acceptable salt thereof: 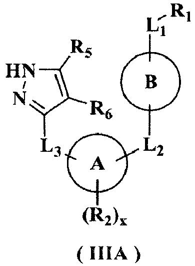 wherein:
R₅ is selected from hydrogen atom, halogen, cyano, alkyl, alkoxy, hydroxyalkyl, haloalkyl, -(CH₂)ₙ₁ORₐₐ, -(CH₂)ₙ₁C(O)NRₐₐR_{bb}, cycloalkyl, heterocyclyl, aryl or heteroaryl, wherein the alkyl, alkoxy, hydroxyalkyl, haloalkyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted, unsubstituted heteroaryl, -(CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc}, -(CH₂)ₙ₁S(O)ₘ₁R_{cc}, - (CH₂)ₙ₁S(O)ₘ₁NR_{cc}R_{dd}, -(CH₂)ₙ₁NR_{cc}R_{dd}, -(CH₂)ₙ₁C(O)NR_{cc}R_{dd}, -(CH₂)ₙ₁C(O)NR_{cc}S(O)ₘ₁R_{dd}, -(CH₂)ₙ₁NR_{cc}S(O)ₘ₁R_{dd} or -(CH₂)ₙ₁NR_{cc}S(O)ₘ₁R_{dd};
R₆ is selected from hydrogen atom, halogen, cyano, alkyl, alkoxy, hydroxyalkyl, haloalkyl, cycloalkyl, heterocyclyl or -(CH₂)ₙ₁C(O)NRₐₐR_{bb};
L₁, L₂, L₃, ring A, ring B, R₁, R₂ and x are as defined in general formula (I).

The present disclosure also relates to a preferred embodiment of the general formula (I), the stereoisomer thereof or the pharmaceutically acceptable salt thereof, which is a compound of general formula (III), a stereoisomer thereof or a pharmaceutically acceptable salt thereof: 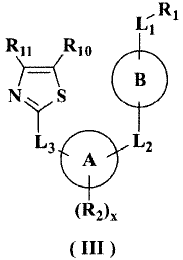 wherein:
R₁₀ and R₁₁ are identical or different and are each independently selected from hydrogen atom, halogen, cyano, alkyl, alkoxy, hydroxyalkyl, haloalkyl, -(CH₂)ₙ₁ORₐₐ, - (CH₂)ₙ₁C(O)NRₐₐR_{bb}, -(CH₂)ₙ₁S(O)ₘ₁NRₐₐR_{bb}-, cycloalkyl, heterocyclyl, aryl or heteroaryl, wherein the alkyl, alkoxy, hydroxyalkyl, haloalkyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted, unsubstituted heteroaryl, -(CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc}, -(CH₂)ₙ₁S(O)ₘ₁R_{cc}, - (CH₂)ₙ₁S(O)ₘ₁NR_{cc}R_{dd}, -(CH₂)ₙ₁NR_{cc}R_{dd}, -(CH₂)ₙ₁C(O)NR_{cc}R_{dd}, -(CH₂)ₙ₁C(O)NR_{cc}S(O)ₘ₁R_{dd}, -(CH₂)ₙ₁NR_{cc}S(O)ₘ₁R_{dd} or -(CH₂)ₙ₁NR_{cc}S(O)ₘ₁R_{dd};
L₁, L₂, L₃, ring A, ring B, R₁, R₂ and x are as defined in general formula (I).

The present disclosure also relates to a preferred embodiment of the general formula (I), the stereoisomer thereof or the pharmaceutically acceptable salt thereof, which is a compound of general formula (IV), a stereoisomer thereof or a pharmaceutically acceptable salt thereof: 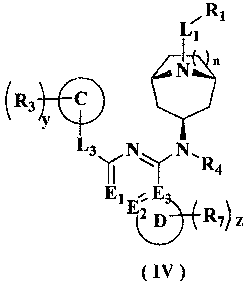 wherein:
ring D is selected from heterocyclyl, aryl or heteroaryl; preferably 5-6 membered heterocyclyl, 6-10 membered aryl or 5-6 membered heteroaryl;
E₁, E₂ and E₃ are identical or different and are each independently selected from nitrogen atom or -CRₐₐ-;
R₄ is selected from hydrogen atom, halogen, cyano, alkyl, alkenyl, alkynyl or alkoxy;
R₇ is selected from hydrogen atom, halogen, cyano, alkyl, alkenyl, alkynyl, alkoxy, oxo or thioxo; and
z is an integer of 0, 1, 2 or 3;
L₁, L₃, ring C, R₁, R₃, Rₐₐ, y and n are as defined in general formula (I).

The present disclosure also relates to a preferred embodiment of the general formula (I), the stereoisomer thereof or the pharmaceutically acceptable salt thereof, which is a compound of general formula (V), a stereoisomer thereof or a pharmaceutically acceptable salt thereof: 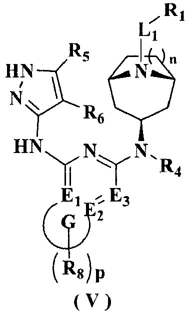 wherein:
ring G is selected from heterocyclyl, aryl or heteroaryl; preferably 5-6 membered heterocyclyl, 6-10 membered aryl or 5-6 membered heteroaryl;
R₈ is selected from hydrogen atom, halogen, cyano, alkyl, alkenyl, alkynyl, alkoxy, oxo or thioxo; and
p is an integer of 0, 1, 2 or 3;
R₅ and R₆ are as defined in claim 3;
L₁, E₁, E₂, E₃, R₄ and n as defined in general formula (IV).

The present disclosure also relates to a preferred embodiment of the general formula (I), the stereoisomer thereof or the pharmaceutically acceptable salt thereof, which is a compound of general formula (VI), a stereoisomer thereof or a pharmaceutically acceptable salt thereof: 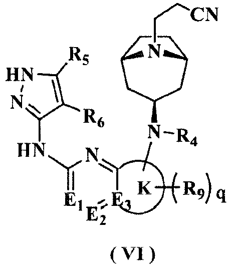 wherein:
ring K is selected from heterocyclyl, aryl or heteroaryl; preferably 5-6 membered heterocyclyl, 6-10 membered aryl, 5-6 membered heteroaryl;
R₉ is selected from hydrogen atom, halogen, cyano, alkyl, alkenyl, alkynyl, alkoxy, oxo or thioxo; and
q is an integer of 0, 1, 2 or 3;
R₅ and R₆ are as defined in claim 3;
E₁, E₂, E₃ and R₄ are as defined in general formula (IV).

The present disclosure also relates to a preferred embodiment of the general formula (I), the stereoisomer thereof or the pharmaceutically acceptable salt thereof, which is a compound of general formula (VII), a stereoisomer thereof or a pharmaceutically acceptable salt thereof: 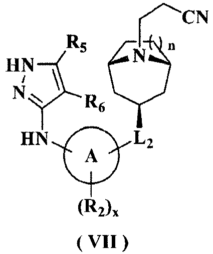 wherein:
n is an integer of 0, 1 or 2;
L₂, ring A, R₂, R₅, R₆ and x are as defined in general formula (IIIA).

The present disclosure also relates to a preferred embodiment of the general formula (III), the stereoisomer thereof or the pharmaceutically acceptable salt thereof, which is a compound of general formula (VIII), a stereoisomer thereof or a pharmaceutically acceptable salt thereof: 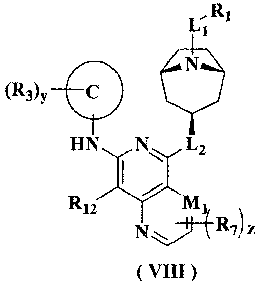 wherein:
M₁ is selected from nitrogen atom or -CRₐₐ-;
R₁₂ is selected from hydrogen atom, cyano, halogen, alkyl or alkoxy; and
z is an integer of 0, 1 or 2;
L₁, L₂, ring C, R₁, R₃, R₇, Rₐₐ and y are as defined in general formula (IV).

The present disclosure also relates to a preferred embodiment of the general formula (I), the stereoisomer thereof or the pharmaceutically acceptable salt thereof, which is a compound of general formula (IX), a stereoisomer thereof or a pharmaceutically acceptable salt thereof: 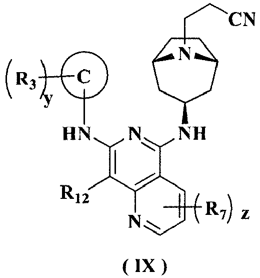 wherein:
ring C, R₃, R₁₂, R₇, y and z are as defined in general formula (VII).

The present disclosure also relates to a preferred embodiment of the general formula (I), the stereoisomer thereof or the pharmaceutically acceptable salt thereof, which is a compound of general formula (X), a stereoisomer thereof or a pharmaceutically acceptable salt thereof: 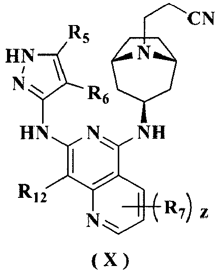 wherein:
R₅-R₆ are as defined in general formula (IIIA);
R₁₂, R₇ and z are as defined in general formula (VII).

The present disclosure also relates to a preferred embodiment of the general formula (I), the stereoisomer thereof or the pharmaceutically acceptable salt thereof, which is a compound of general formula (XI), a stereoisomer thereof or a pharmaceutically acceptable salt thereof: 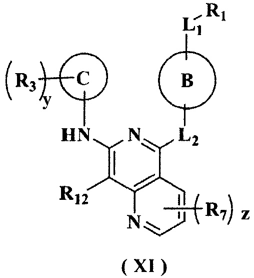 wherein:
L₁, L₂, ring B, ring C, R₃ and R₁₂ are as defined in general formula (I)
R₇, y and z are as defined in general formula (IX).

The present disclosure also relates to a preferred embodiment of any one of the general fonnulas, the stereoisomer thereof or the pharmaceutically acceptable salt thereof, wherein,
ring A is selected from the following group: 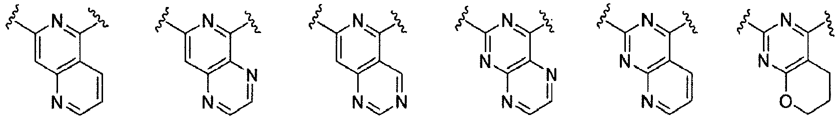 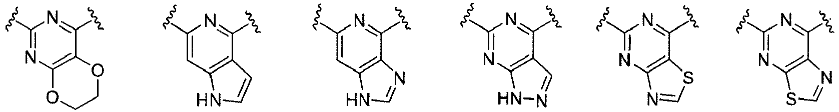 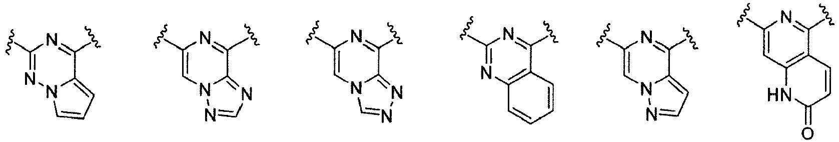 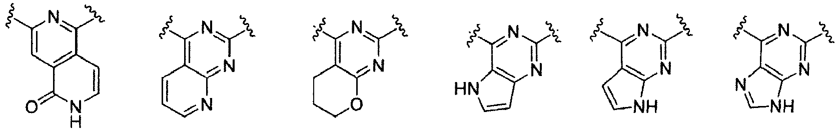 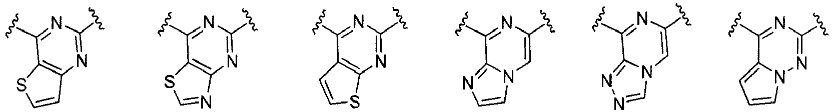 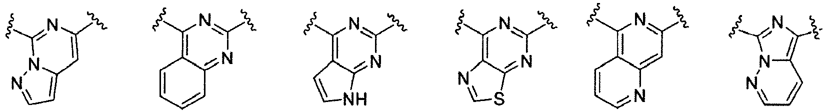 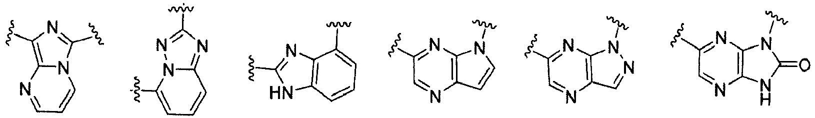 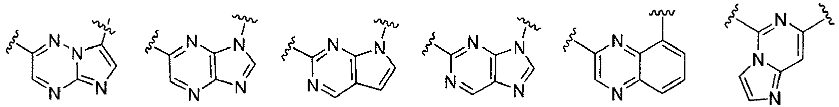 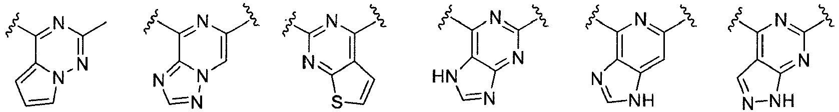 and 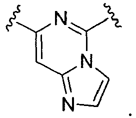
ring B is selected from the following group: 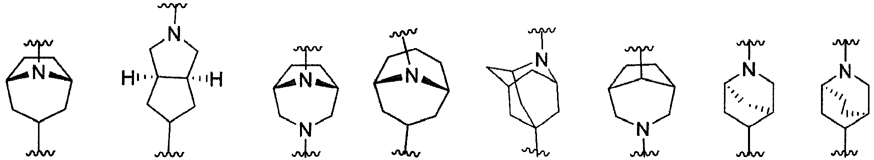 and 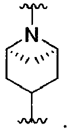
ring C is selected from the following group: 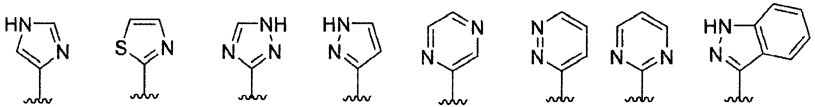 and

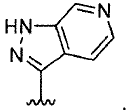

The present disclosure also relates to a preferred embodiment of any one of the general formulas, the stereoisomer thereof or the pharmaceutically acceptable salt thereof, wherein,
L₁ is C₃₋₈ cycloalkyl, C₃₋₈ heterocycloalkyl, -(CH₂)ₙ₁-, -NRₐₐ(CH₂)ₙ₁-, -(CH₂)ₙ₁S(O)ₘ₁- or -(CH₂)ₙ₁S(O)ₘ₁NRₐₐ-;
L₂ is selected from bond, oxygen atom, -CRₐₐR_{bb}-, -(CH₂)ₙ₁C(O)-, -NR₄- or - (CH₂)ₙ₁S(O)ₘ₁-;
L₃ is selected from bond, -NRₐₐ- or -C(O)NRₐₐ-;
R₁ is selected from cyano, -(CH₂)ₙ₁Rₐₐ, C₃₋₈ cycloalkyl or 3-10 membered heterocyclyl, wherein the cycloalkyl and heterocyclyl are optionally further substituted with one or more substituents selected from hydrogen atom or cyano;
R₂ is hydrogen atom, halogen, cyano, hydroxyl, oxo, C₁₋₆ alkyl, C₁₋₆ alkoxy or 3-10 membered heterocyclyl; preferably hydrogen atom, halogen, cyano, hydroxyl, oxo, C₁₋₃ alkoxy, C₁₋₃ alkyl or 3-8 membered heterocyclyl; more preferably hydrogen atom, fluorine, cyano, hydroxyl, oxo, methoxy, methyl or morpholinyl;
R₃ is selected from hydrogen atom, cyano, halogen, C₁₋₆ alkyl, C₁₋₆ haloalkyl, C₁₋₆ hydroxyalkyl, C₃₋₈ cycloalkyl, 3-10 membered heterocyclyl, -(CH₂)ₙ₁ORₐₐ, -C(O)NRₐₐR_{bb} or - (CH₂)ₙ₁S(O)ₘ₁Rₐₐ, wherein the cycloalkyl and heterocyclyl are optionally further substituted with one or more substituents selected from hydrogen atom, C₁₋₆ alkyl or hydroxyl;
R₄ is selected from hydrogen atom or methyl;
Rₐₐ, R_{bb}, R_{cc} and R_{dd} are identical or different and are each independently selected from hydrogen atom, C₁₋₆ alkyl, cyano, hydroxyl, amino or 3-10 membered heterocyclyl; wherein the C₁₋₆ alkyl, amino and 3-10 membered heterocyclyl is optionally further substituted with one or more substituents selected from hydrogen atom, cyano, hydroxyl or 5-10 membered heteroaryl.

The present disclosure further relates to a use of any one of the compounds of general formula (I), the stereoisomers thereof or the pharmaceutically acceptable salts thereof, or a pharmaceutical composition in the manufacture of a JAK kinase inhibitor medicament.

The present disclosure also relates to a method for the prevention and/or treatment, pretreatment of a condition mediated by JAK kinase inhibitor, which comprises administering to a patient a therapeutically effective dose of the compound of general formula (I), the stereoisomer thereof or the pharmaceutically acceptable salt thereof, or the pharmaceutical composition thereof.

The present disclosure also relates to a pharmaceutical composition, which comprises a therapeutically effective dose of the compound of general formula (I), the stereoisomer thereof or the pharmaceutically acceptable salt thereof and one or more pharmaceutically acceptable carriers, diluents or excipients.

The present disclosure also relates to a use of the compound of general formula (I), the stereoisomer thereof or the pharmaceutically acceptable salt thereof, or the pharmaceutical composition in the manufacture of a medicament for the treatment of an inflammatory disease and a neoplastic disease.

The present disclosure also relates to a method for the treatment of an inflammatory disease and a method for the treatment of a neoplastic disease, which comprises administering to a patient a therapeutically effective dose of the pharmaceutical composition, the inflammatory disease is selected from rheumatoid arthritis, dermatitis, psoriasis, inflammatory bowel disease; wherein gastrointestinal inflammatory disease is chronic inflammatory disease of the intestine, further preferably ulcerative colitis and Crohn's disease.

The present disclosure also relates to a method of treating a neoplastic disease, which comprises administering to a patient a therapeutically effective dose of the pharmaceutical composition, the neoplastic disease is selected from myelofibrosis, polycythemia vera and essential thrombocythemia, acute myeloid leukemia (AML), acute lymphoblastic leukemia (ALL), ductal carcinoma of the breast and non-small cell lung cancer (NSCLC).

The present disclosure also relates to a pharmaceutical composition, which comprises the compound of general formula (I), the stereoisomer thereof or the pharmaceutically acceptable salt thereof, and one or more pharmaceutically acceptable carriers, diluents or excipients, the amount of the compound of general formula (I), the stereoisomer thereof or the pharmaceutically acceptable salt thereof may be a therapeutically effective amount.

The present disclosure further relates to a use of any one of the compounds of general formula (I), the stereoisomers thereof or the pharmaceutically acceptable salts thereof, or the aforementioned pharmaceutical composition in the manufacture of a JAK kinase inhibitor.

The present disclosure also relates to a use of the aforementioned compound of general formula (I), the stereoisomer thereof or the pharmaceutically acceptable salt thereof, or the aforementioned pharmaceutical composition in the manufacture of a medicament for the prevention and/or treatment of a JAK kinase related disease. The JAK kinase related disease is preferably an inflammatory disease and/or a neoplastic disease; the inflammatory disease is preferably selected from rheumatoid arthritis, dermatitis, psoriasis, inflammatory bowel disease; wherein gastrointestinal inflammatory disease is chronic inflammatory disease of the intestine, further preferably ulcerative colitis and Crohn's disease. The neoplastic disease is preferably selected from myelofibrosis, polycythemia vera and essential thrombocythemia, acute myelomonocytic leukemia, acute lymphocytic leukemia, ductal carcinoma of the breast and non-small cell lung cancer.

The present disclosure also relates to a use of the aforementioned compound of general formula (I), the stereoisomer thereof or the pharmaceutically acceptable salt thereof, or the aforementioned pharmaceutical composition in the manufacture of a medicament, the medicament is preferably a medicament for the treatment of an inflammatory disease and/or a neoplastic disease. The inflammatory disease is preferably selected from rheumatoid arthritis, dermatitis, psoriasis, inflammatory bowel disease; wherein gastrointestinal inflammatory disease is chronic inflammatory disease of the intestine, further preferably ulcerative colitis and Crohn's disease. The neoplastic disease is preferably selected from myelofibrosis, polycythemia vera and essential thrombocythemia, acute myelomonocytic leukemia, acute lymphocytic leukemia, ductal carcinoma of the breast and non-small cell lung cancer.

The present disclosure also relates to a method for the treatment of an inflammatory disease, which comprises administering to a patient a therapeutically effective dose of the aforementioned compound of general formula (I), the stereoisomer thereof or the pharmaceutically acceptable salt thereof, or the aforementioned pharmaceutical composition, the inflammatory disease is selected from rheumatoid arthritis, dermatitis, psoriasis, inflammatory bowel disease; wherein gastrointestinal inflammatory disease is chronic inflammatory disease of the intestine, further preferably ulcerative colitis and Crohn's disease.

The present disclosure also relates to a method for the treatment of neoplastic disease, which comprises administering to a patient a therapeutically effective dose of the aforementioned compound of general formula (I), the stereoisomer thereof or the pharmaceutically acceptable salt thereof, or the aforementioned pharmaceutical composition, the neoplastic disease is selected from myelofibrosis, polycythemia vera and essential thrombocythemia, acute myelomonocytic leukemia, acute lymphocytic leukemia, ductal carcinoma of the breast and non-small cell lung cancer.

The present disclosure also relates to a method for the prevention and/or treatment of a disease mediated by JAK kinase, which comprises administering to a patient a therapeutically effective dose of the aforementioned compound of general formula (I), the stereoisomer thereof or the pharmaceutically acceptable salt thereof, or the aforementioned pharmaceutical composition. The disease mediated by JAK kinase is preferably an inflammatory disease and/or a neoplastic disease; the inflammatory disease is preferably selected from rheumatoid arthritis, dermatitis, psoriasis, inflammatory bowel disease; wherein gastrointestinal inflammatory disease is chronic inflammatory disease of the intestine, further preferably ulcerative colitis and Crohn's disease. The neoplastic disease is preferably selected from myelofibrosis, polycythemia vera and essential thrombocythemia, acute myelomonocytic leukemia, acute lymphocytic leukemia, ductal carcinoma of the breast and non-small cell lung cancer.

### Detailed description of the invention

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl comprising 1 to 8 carbon atoms, more preferably an alkyl comprising 1 to 6 carbon atoms, and most preferably an alkyl comprising 1 to 3 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof. Lower alkyl comprising 1 to 6 carbon atoms is more preferred, non-limiting examples include methyl, ethyl, *n*-propyl, isopropyl, *n*-butyl, isobutyl, *tert*-butyl, *sec*-butyl, *n-*pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *etc.* The alkyl group may be substituted or unsubstituted; when substituted, the substituent group may be substituted at any available connection point; the substituent group is preferably one or more groups independently selected from alkyl, alkenyl, alkynyl, alkoxy, alkylthio, alkylamino, halogen, thiol, hydroxyl, nitro, cyano, cycloalkyl, heterocycloalkyl, aryl, heteroaryl, cycloalkyloxy, heterocycloalkyloxy, cycloalkylthio, heterocycloalkylthio, oxo, carboxyl and carboxylate ester group. The alkyl of the present disclosure is preferably selected from methyl, ethyl, isopropyl, *tert*-butyl, haloalkyl, deuterated alkyl, alkyl substituted with alkoxy and alkyl substituted with hydroxyl.

The term "alkylene" refers to an alkyl of which a hydrogen atom is further substituted, *e.g.*, "methylene" refers to -CH₂-, "ethylene" refers to -(CH₂)₂-, "propylene" refers to -(CH₂)₃-, "butylene" refers to -(CH₂)₄-, *etc.* The term "alkenyl" refers to an alkyl as defined above that consists of at least two carbon atoms and at least one carbon-carbon double bond, e.g., ethenyl, 1-propenyl, 2-propenyl, 1-, 2- or 3-butenyl, *etc.* The alkenyl group may be substituted or unsubstituted; when substituted, the substituent group is preferably one or more independently selected from alkyl, alkenyl, alkynyl, alkoxy, alkylthio, alkylamino, halogen, thiol, hydroxyl, nitro, cyano, cycloalkyl, heterocycloalkyl, aryl, heteroaryl, cycloalkyloxy, heterocycloalkyloxy, cycloalkylthio, heterocycloalkylthio.

The term "cycloalkyl" refers to a saturated or partially unsaturated monocyclic or polycyclic hydrocarbon substituent group comprising 3 to 20 carbon atoms, preferably 3 to 12 carbon atoms, further preferably 3 to 8 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, *etc.*; polycyclic cycloalkyl includes spiro, fused and bridged cycloalkyl; preferably cyclopropyl, cyclobutyl, cyclohexyl, cyclopentyl and cycloheptyl, more preferably cyclopropyl, cyclobutyl and cyclopentyl.

The term "spiro cycloalkyl" refers to 5 to 20 membered polycyclic group with individual rings connected through one shared carbon atom (called a spiro atom), wherein the rings may contain one or more double bonds, but none of the rings has a completely conjugated π-electron system. The spiro cycloalkyl is preferably 6 to 14 membered spiro cycloalkyl, and more preferably 7 to 10 membered spiro cycloalkyl. According to the number of the spiro atoms shared between the rings, the spiro cycloalkyl may be divided into mono-spiro cycloalkyl, di-spiro cycloalkyl, or poly-spiro cycloalkyl, and preferably mono-spiro cycloalkyl or di-spiro cycloalkyl. The spiro cycloalkyl is more preferably 4 membered/4 membered, 4 membered/5 membered, 4 membered/6 membered, 5 membered/5 membered, or 5 membered/6 membered mono-spiro cycloalkyl. Non-limiting examples of spiro cycloalkyl include:

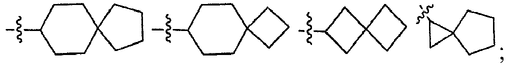

and also include spiro cycloalkyl in which mono-spiro cycloalkyl and heterocycloalkyl are connected through one spiro atom, non-limiting examples thereof include: 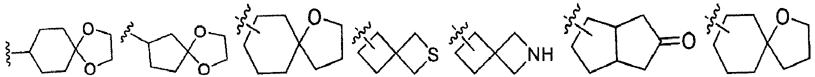 and

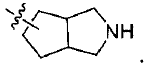

The term "fused cycloalkyl" refers to 5 to 20 membered all-carbon polycyclic group, wherein each ring in the system shares an adjacent pair of carbon atoms with another ring, wherein one or more rings may contain one or more double bonds, but none of the rings has a completely conjugated π-electron system. The fused cycloalkyl is preferably 6 to 14 membered fused cycloalkyl, and more preferably 7 to 10 membered fused cycloalkyl. According to the number of membered rings, the fused cycloalkyl may be divided into bicyclic, tricyclic, tetracyclic or polycyclic fused cycloalkyl, and the fused cycloalkyl is preferably bicyclic or tricyclic fused cycloalkyl, and more preferably 4 membered/4 membered, 5 membered/5 membered, or 5 membered/6 membered bicyclic fused cycloalkyl. Non-limiting examples of fused cycloalkyl include:

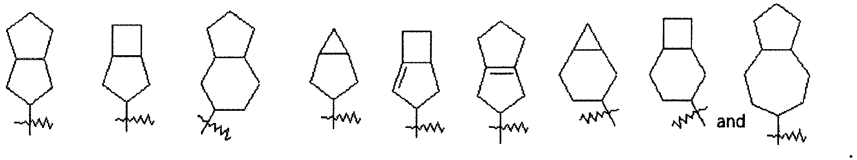

The term "bridged cycloalkyl" refers to 5 to 20 membered all-carbon polycyclic group, wherein every two rings in the system share two disconnected carbon atoms, wherein the rings may have one or more double bonds, but none of the rings has a completely conjugated π-electron system. The bridged cycloalkyl is preferably 6 to 14 membered bridged cycloalkyl, and more preferably 7 to 10 membered bridged cycloalkyl. According to the number of membered rings, the bridged cycloalkyl may be divided into bicyclic, tricyclic, tetracyclic or polycyclic bridged cycloalkyl, and the bridged cycloalkyl is preferably bicyclic, tricyclic or tetracyclic bridged cycloalkyl, and more preferably bicyclic or tricyclic bridged cycloalkyl. Non-limiting examples of bridged cycloalkyl include:

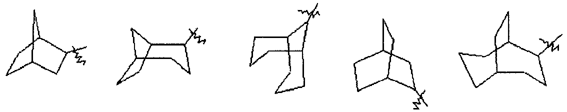

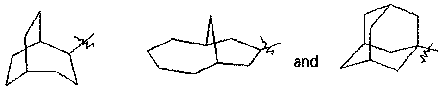

The cycloalkyl ring may be fused to the ring of aryl, heteroaryl or heterocycloalkyl, wherein the ring bound to the parent structure is cycloalkyl; non-limiting examples include indanyl, tetrahydronaphthyl, benzocycloheptyl, *etc.* The cycloalkyl may be optionally substituted or unsubstituted; when substituted, the substituent group is preferably one or more groups independently selected from alkyl, alkenyl, alkynyl, alkoxy, alkylthio, alkylamino, halogen, thiol, hydroxyl, nitro, cyano, cycloalkyl, heterocycloalkyl, aryl, heteroaryl, cycloalkyloxy, heterocycloalkyloxy, cycloalkylthio, heterocycloalkylthio, oxo, carboxyl or carboxylate ester group.

The term "heterocyclyl" refers to 3 to 20 membered saturated or partially unsaturated monocyclic or polycyclic hydrocarbon group, wherein one or more ring atoms are heteroatoms selected from N, O, B, P, S(O)ₘ (wherein m is an integer of 0 to 2) or P(O)ₙ (wherein n is an integer of 0 to 2), but excluding -O-O-, -O-S- or -S-S- in the ring, with the remaining ring atoms being carbon atoms. Preferably, the heterocyclyl comprises 3 to 12 ring atoms wherein 1 to 4 atoms are heteroatoms; more preferably, 3 to 10 ring atoms; and most preferably 3 to 8 ring atoms. Non-limiting examples of monocyclic heterocyclyl include oxacyclobutyl, azacyclobutyl, pyrrolidinyl, imidazolidinyl, tetrahydrofuranyl, tetrahydropyranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl, *etc.*, preferably oxycyclobutyl, azacyclobutyl, tetrahydrofuranyl, tetrahydropyranyl, pyrazolidinyl, morpholinyl, piperidinyl, piperazinyl and pyranyl, more preferably azacyclobutyl, oxacyclobutyl, pyrrolidinyl, tetrahydropyranyl, morpholinyl, piperidinyl and piperazinyl. Polycyclic heterocyclyl includes spiro, fused and bridged heterocyclyl. The heterocyclyl comprising spiro ring, fused ring or bridged ring is optionally bonded to other group via a single bond, or further bonded to other cycloalkyl, heterocyclyl, aryl and heteroaryl via any two or more atoms on the ring.

The term "spiro heterocyclyl" refers to 3 to 20 membered polycyclic heterocyclyl group with individual rings connected through one shared atom (called a spiro atom), wherein one or more ring atoms are heteroatoms selected from N, O, B, P, S(O)ₘ (wherein m is an integer of 0 to 2) or P(O)ₙ (wherein n is an integer of 0 to 2), with the remaining ring atoms being carbon atoms. The rings may contain one or more double bonds, but none of the rings has a completely conjugated π-electron system. The spiro heterocyclyl is preferably 6 to 14 membered spiro heterocyclyl, and more preferably 7 to 10 membered spiro heterocyclyl. According to the number of the spiro atoms shared between the rings, the spiro heterocyclyl may be divided into mono-spiro heterocyclyl, di-spiro heterocyclyl, or poly-spiro heterocyclyl, and the spiro heterocyclyl is preferably mono-spiro heterocyclyl or di-spiro heterocyclyl, and more preferably 3 membered/5 membered, 4 membered/5 membered, 4 membered/6 membered, 5 membered/5 membered, or 5 membered/6-membered mono-spiro heterocyclyl. Non-limiting examples of spiro heterocyclyl include: 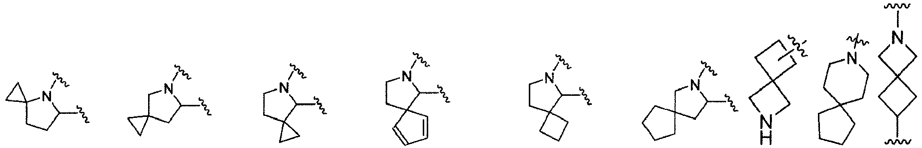 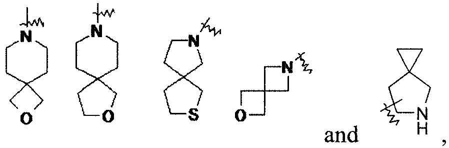 *etc.*

The term "fused heterocyclyl" refers to 5 to 20 membered polycyclic heterocyclyl group, wherein each ring in the system shares an adjacent pair of atoms with another ring, wherein one or more rings may contain one or more double bonds, but none of the rings has a completely conjugated π-electron system, and wherein one or more ring atoms are heteroatoms selected from N, O or S(O)ₘ (wherein m is an integer of 0 to 2), with the remaining ring atoms being carbon atoms. The fused heterocyclyl is preferably 6 to 14 membered fused heterocyclyl, and more preferably 7 to 10 membered fused heterocyclyl. According to the number of membered rings, the fused heterocyclyl may be divided into bicyclic, tricyclic, tetracyclic or polycyclic fused heterocyclyl, and preferably bicyclic or tricyclic fused heterocyclyl, and more preferably 3 membered/5 membered, 4 membered/5 membered or 5 membered/6 membered bicyclic fused heterocyclyl. Non-limiting examples of fused heterocyclyl include: 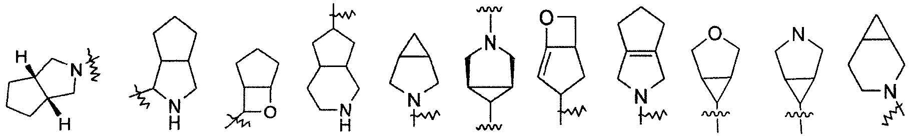 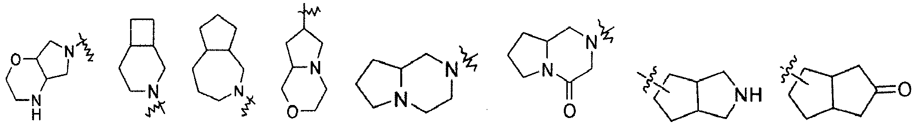 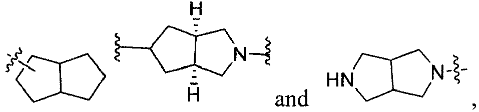 *etc.*

The term "bridged heterocyclyl" refers to 5 to 14 membered polycyclic heterocyclyl group, wherein two arbitrary rings in the system share two disconnected atoms, wherein the rings may have one or more double bonds, but none of the rings has a completely conjugated π-electron system, and wherein one or more ring atoms are heteroatoms selected from N, O and S(O)ₘ (wherein m is an integer of 0 to 2), with the remaining ring atoms being carbon atoms. The bridged heterocyclyl is preferably 6 to 14 membered bridged heterocyclyl, and more preferably 7 to 10 membered bridged heterocyclyl. According to the number of membered rings, the bridged heterocyclyl may be divided into bicyclic, tricyclic, tetracyclic or polycyclic bridged heterocyclyl, and the bridged heterocyclyl is preferably bicyclic, tricyclic or tetracyclic bridged heterocyclyl, and more preferably bicyclic or tricyclic bridged heterocyclyl. Non-limiting examples of bridged heterocyclyl include:

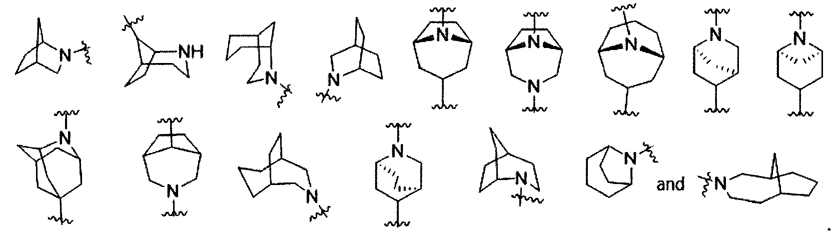

The heterocyclyl ring may be fused to the ring of aryl, heteroaryl or cycloalkyl, wherein the ring bound to the parent structure is heterocyclyl; non-limiting examples thereof include: 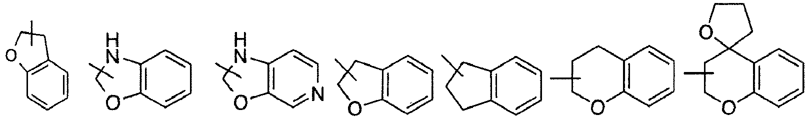 and 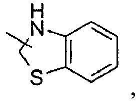 *etc.*

The heterocyclyl may be optionally substituted or unsubstituted; when substituted, the substituent group is preferably one or more groups independently selected from alkyl, alkenyl, alkynyl, alkoxy, alkylthio, alkylamino, halogen, thiol, hydroxyl, nitro, cyano, cycloalkyl, heterocycloalkyl, aryl, heteroaryl, cycloalkyloxy, heterocycloalkyloxy, cycloalkylthio, heterocycloalkylthio, oxo, carboxyl or carboxylate ester group.

The term "aryl" refers to 6 to 14 membered all-carbon monocyclic ring or polycyclic fused ring (i.e., each ring in the system shares an adjacent pair of carbon atoms with another ring in the system) comprising a conjugated π-electron system, preferably 6 to 10 membered aryl, e.g., phenyl and naphthyl. The aryl is more preferably phenyl. The aryl ring may be fused to the ring of heteroaryl, heterocyclyl or cycloalkyl, wherein the ring bound to the parent structure is aryl ring. Non-limiting examples thereof include: 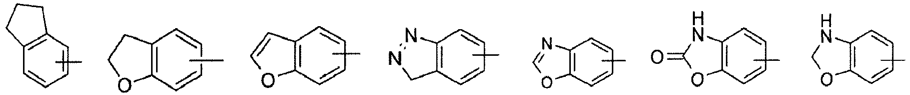 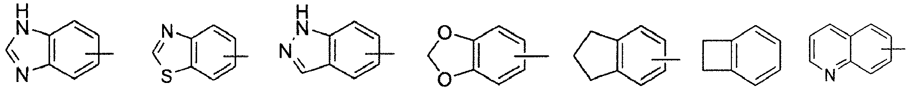 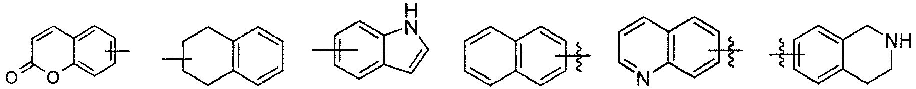 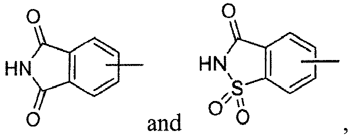 *etc.*

The aryl may be substituted or unsubstituted; when substituted, the substituent group is preferably one or more groups independently selected from alkyl, alkenyl, alkynyl, alkoxy, alkylthio, alkylamino, halogen, thiol, hydroxyl, nitro, cyano, cycloalkyl, heterocycloalkyl, aryl, heteroaryl, cycloalkyloxy, heterocycloalkyloxy, cycloalkylthio, heterocycloalkylthio, carboxyl or carboxylate ester group.

The term "heteroaryl" refers to 5 to 14 membered heteroaromatic system comprising 1 to 4 heteroatoms selected from O, S and N. The heteroaryl is preferably 5 to 10 membered heteroaryl, and more preferably 5 or 6 membered heteroaryl, e.g., imidazolyl, furyl, thienyl, thiazolyl, pyrazolyl, oxazolyl, pyrrolyl, triazolyl, tetrazolyl, pyridyl, pyrimidinyl, thiadiazolyl, oxadiazolyl, pyrazinyl, pyridazinyl *etc.*, preferably pyrazinyl, pyridazinyl, oxazolyl, oxadiazole, tetrazolyl, triazolyl, thienyl, imidazolyl, pyridinyl, pyrimidinyl, pyrazolyl, thiazolyl, thiadiazole, pyrazolyl and pyrimidinyl; more preferably pyrazinyl, pyridyl, pyridazinyl, pyrimidinyl, imidazolyl, triazolyl, pyrazolyl and thiazolyl.

The term "fused heteroaryl" refers to 4 to 20 membered heteroaryl system comprising 1 to 6 heteroatoms selected from O, S and N. The fused heteroaryl is preferably 6 to 14 membered, more preferably 6 membered or 10 membered. The fused heteroaryl ring refers to heteroaryl fused to the ring of aryl, heteroaryl or cycloalkyl; non-limiting examples thereof include:

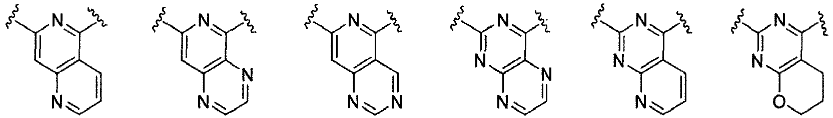

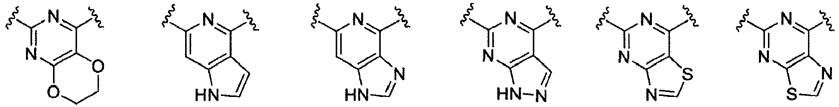

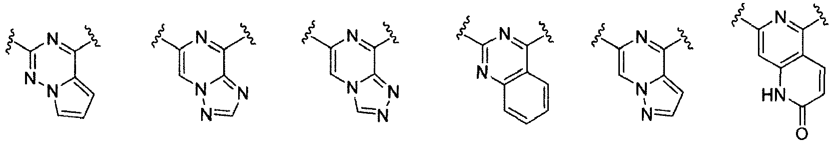

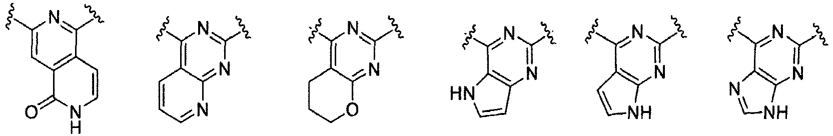

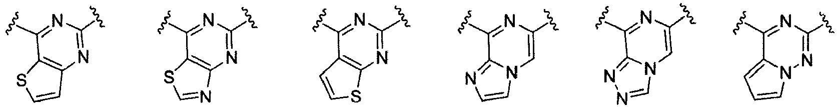

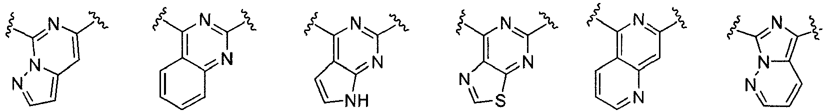

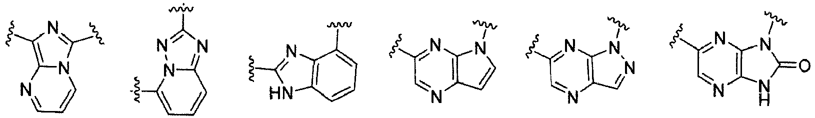

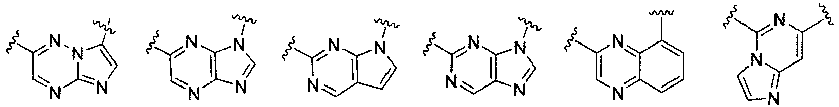

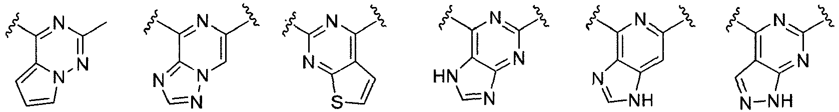

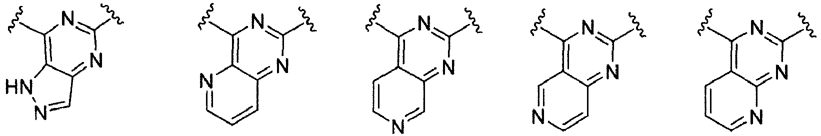

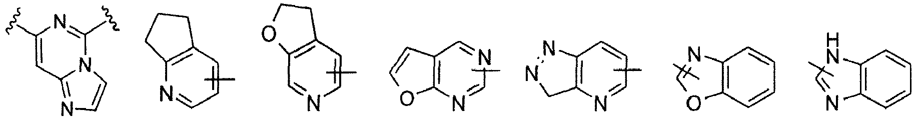

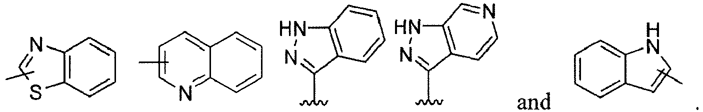

The heteroaryl or the fused heteroaryl may be optionally substituted or unsubstituted; when substituted, the substituent is preferably one or more groups independently selected from alkyl, alkenyl, alkynyl, alkoxy, alkylthio, alkylamino, halogen, thiol, hydroxyl, nitro, cyano, cycloalkyl, heterocycloalkyl, aryl, heteroaryl, cycloalkyloxy, heterocycloalkyloxy, cycloalkylthio, heterocycloalkylthio, carboxyl or carboxylate ester group.

The term "alkoxy" refers to -O-(alkyl) or -O-(unsubstituted cycloalkyl) group, wherein the alkyl is as defined above. Non-limiting examples of alkoxy include methoxy, ethoxy, propoxy, butoxy, cyclopropyloxy, cyclobutyloxy, cyclopentyloxy, cyclohexyloxy. The alkoxy may be optionally substituted or unsubstituted; when substituted, the substituent group is preferably one or more groups independently selected from alkyl, alkenyl, alkynyl, alkoxy, alkylthio, alkylamino, halogen, thiol, hydroxyl, nitro, cyano, cycloalkyl, heterocycloalkyl, aryl, heteroaryl, cycloalkyloxy, heterocycloalkyloxy, cycloalkylthio, heterocycloalkylthio, carboxyl or carboxylate ester group.

"Haloalkyl" refers to an alkyl group substituted with one or more halogens, wherein the alkyl is as defined above.

"Haloalkoxy" refers to an alkoxy group substituted with one or more halogens, wherein the alkoxy is as defined above.

"Hydroxyalkyl" refers to an alkyl group substituted with hydroxyl, wherein the alkyl is as defined above.

"Alkenyl" refers to a chain alkenyl, also known as an olefin group, wherein the alkenyl may be further substituted with other related group(s), *e.g.*, alkyl, alkenyl, alkynyl, alkoxy, alkylthio, alkylamino, halogen, thiol, hydroxyl, nitro, cyano, cycloalkyl, heterocycloalkyl, aryl, heteroaryl, cycloalkyloxy, heterocycloalkyloxy, cycloalkylthio, heterocycloalkylthio, carboxyl or carboxylate ester group.

"Alkynyl" refers to (CH=C- or -C=C-) , wherein the alkynyl may be further substituted by other related group(s), *e.g.*, alkyl, alkenyl, alkynyl, alkoxy, alkylthio, alkylamino, halogen, thiol, hydroxyl, nitro, cyano, cycloalkyl, heterocycloalkyl, aryl, heteroaryl, cycloalkyloxy, heterocycloalkyloxy, cycloalkylthio, heterocycloalkylthio, carboxyl or carboxylate ester group.

"Hydroxyl" refers to -OH group.

"Halogen" refers to fluorine, chlorine, bromine or iodine.

"Amino" refers to -NH₂.

"Cyano" refers to -CN.

"Nitro" refers to -NO₂.

"Carboxyl" refers to -C(O)OH.

"THF" refers to tetrahydrofuran.

"EtOAc" refers to ethyl acetate.

"MeOH" refers to methanol.

"DMF" refers to *N*,*N*-dimethylformamide.

"DIPEA" refers to diisopropylethylamine.

"AN" refers to acrylonitrile.

"TFA" refers to trifluoroacetic acid.

"MeCN" refers to acetonitrile.

"DMA" refers to *N*,*N-*dimethylacetamide.

"Et₂O" refers to diethyl ether.

"DCE" refers to 1,2-dichloroethane.

"DIPEA" refers to *N*,*N*-diisopropylethylamine.

"NBS" refers to *N*-bromosuccinimide.

"NIS" refers to *N*-iodosuccinimide.

"Cbz-Cl" refers to benzyl chloroformate.

"Pd₂(dba)₃" refers to tris(dibenzylideneacetone)dipalladium.

"Dppf' refers to 1,1'-bis(diphenylphosphino)ferrocene.

"HATU" refers to 2-(7-azabenzotriazol-1-yl)-N,N,N,N-tetramethyluronium hexafluorophosphate.

"KHMDS" refers to potassium hexamethyldisilazide.

"LiHMDS" refers to lithium bis(trimethylsilyl)amide.

"MeLi" refers to methyl lithium.

"n-BuLi" refers to *n*-butyl lithium.

"NaBH(OAc)₃" refers to sodium triacetoxyborohydride.

Different expressions such as "X is selected from A, B or C", "X is selected from A, B and C", "X is A, B or C", "X is A, B and C", *etc.*, express identical meaning, that is, X may be any one or more of A, B and C.

The hydrogen atom of the present disclosure may be substituted by its isotope deuterium. Any one of the hydrogen atoms in the compounds of the examples of the present disclosure may also be substituted by deuterium atom.

"Optional" or "optionally" refers to that the event or circumstance described subsequently may, but need not, occur, and such a description includes the situation in which the event or circumstance does or does not occur. For example, "heterocyclyl optionally substituted by alkyl" refers to that an alkyl group may be, but need not be, present, and such a description includes the situation of the heterocyclyl being substituted by alkyl and the heterocyclyl being not substituted by alkyl.

"Substituted" refers to one or more hydrogen atoms in a group, preferably up to 5, and more preferably 1 to 3 hydrogen atoms, independently substituted by a corresponding number of substituents. It goes without saying that the substituents only exist in their possible chemical position. The person skilled in the art is able to determine whether the substitution is possible or impossible by experiments or theory without undue efforts. For example, the combination of amino or hydroxyl comprising free hydrogen and carbon atoms comprising unsaturated bonds (such as olefinic) may be unstable.

A "pharmaceutical composition" refers to a mixture of one or more of the compounds according to the present disclosure or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to exert biological activity.

A "pharmaceutically acceptable salt" refers to a salt of the compound of the present disclosure, which is safe and effective in mammals and has the desired biological activity.

### Detailed description of the preferred embodiment

The present disclosure will be further described by the embodiments below, but the present disclosure is not limited to the scope of the described embodiments. In the following embodiments, the experimental methods without specific conditions are selected according to conventional methods and conditions, or according to the product specification.

### Embodiment

The structure of the compound of the present embodiment is determined by nuclear magnetic resonance (NMR) or/and liquid-mass spectrometry (LC-MS). The NMR chemical shift (δ) is given in units of parts per million (ppm). NMR was measured with Bruker AVANCE-400 nuclear magnetic instrument, the solvent was deuterated dimethyl sulfoxide (DMSO-d₆), deuterated methanol (CD₃OD) and deuterated chloroform (CDCl₃), and the internal standard was tetramethylsilane (TMS).

The liquid mass spectrometry LC-MS was measured with an Agilent 1200 Infinity Series mass spectrometer. HPLC was determined by Agilent 1200DAD high pressure liquid chromatograph (Sunfire C18 150 × 4.6 mm column) and Waters 2695-2996 high pressure liquid chromatograph (Gi minutes i C18 150 × 4.6 mm column).

Yantai Huanghai HSGF254 or Qingdao GF254 silica gel plate was used as the thin layer chromatography silica gel plate, the specification of TLC is 0.15 mm-0.20 mm, and the specification of the thin layer chromatography separation used for product purification is 0.4 mm-0.5 mm. 200-300 mesh silica gel of Yantai Huanghai silica gel was generally used as the carrier of the column chromatography.

The starting materials in the embodiment of the present disclosure are known and can be purchased on the market, or can be synthesized by adopting or following methods known in the art.

Unless otherwise specified, all reactions of the present embodiment were carried out under continuous magnetic stirring under dry nitrogen or argon atmosphere, the solvent was dry solvent, and the reaction temperature unit was degrees Celsius.

### Embodiment 1

### 3-((3-exo)-3-((7-((5-methylthioazol-2-yl)amino)-1,6-diazanaphthalen-5-yl)amino)-8-azabicyclo[3.2.1]oct-8-yl)propionitrile

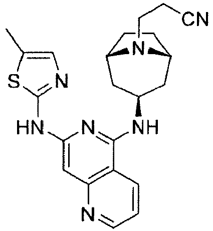

### Step 1: preparation oftert-butyl (3-exo)-3-((7-chloro-1,6-diazanaphthalen-5-yl)amino)-8-azabicyclo[3.2.1]oct-8-carboxylate

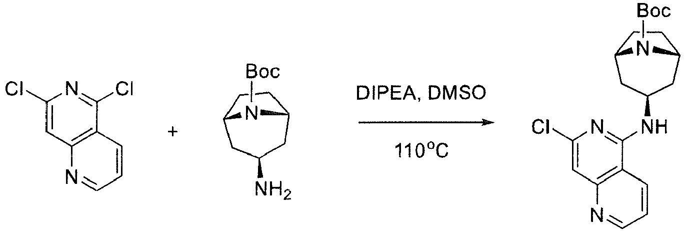

The compound 5,7-dichloro-1,6-diazanaphthalene (100 mg, 0.502 mmol), Tert-butyl (3-exo)-3-amino-8-azabicyclo[3.2.1]oct-8-carboxylate (119 mg, 0.527 mmol) and diisopropylethylamine (195 mg, 1.51 mmol) were dissolved in dimethyl sulfoxide (4 mL), and the reaction mixture was heated to 110°C and stirred for 22 hours. The reaction mixture was cooled to room temperature, diluted with ethyl acetate, and washed with saturated sodium chloride aqueous solution. The organic phase was collected, dried over anhydrous sodium sulfate, filtered, and concentrated under reduced pressure to remove the organic solvent. The resulting residue was separated and purified by silica gel column chromatography to obtain the title compound as a yellow-white solid (173 mg, 92%).

¹H NMR (400 MHz, DMSO-*d*₆) *δ* 8.89 (dd, *J* = 4.2, 1.3 Hz, 1H), 8.72 (d, *J* = 8.3 Hz, 1H), 7.74 (d, *J* = 7.5 Hz, 1H), 7.48 (dd, *J* = 8.4, 4.3 Hz, 1H), 6.96 (s, 1H), 4.70-4.56 (m, 1H), 4.16 (s, 2H), 2.05-1.87 (m, 4H), 1.81-1.57 (m, 4H), 1.44 (s, 9H).

MS *m*/*z* (ESI): 389.2 [M+H]⁺.

### Step 2: preparation of 3-((3-exo)-3-((7-chloro-1,6-diazanaphthalen-5-yl)amino)-8-azabicyclo[3.2.1]oct-8-yl) propionitrile

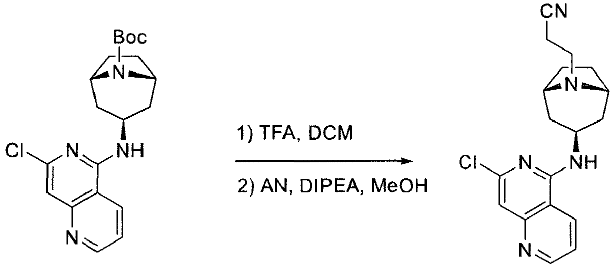

Tert-butyl (3-*exo*)-3-((7-chloro-1,6-diazanaphthalen-5-yl)amino)-8-azabicyclo[3.2.1]oct-8-carboxylate (100 mg, 0.257 mmol) was dissolved in dichloromethane (1 mL), and trifluoroacetic acid (0.5 mL) was added with stirring at room temperature. The resulting reaction mixture was stirred at room temperature for 30 minutes, and then concentrated under reduced pressure to remove the solvent. The mixture was dissolved by anhydrous methanol, and diisopropylethylamine (332 mg, 2.57 mmol) and acrylonitrile (16 mg, 0.31 mmol) were added successively. The reaction mixture was stirred at room temperature for 26.5 hours, and concentrated under reduced pressure to remove the solvent. The residue was separated and purified by silica gel column chromatography to obtain the title compound as a white powder (81 mg, 92%).

¹H NMR (400 MHz, DMSO-*d*₆) *δ* 8.90-8.87 (m, 1H), 8.72 (d, *J* = 8.3 Hz, 1H), 7.76 (d, *J* = 7.7 Hz, 1H), 7.47 (dd, *J* = 8.4, 4.3 Hz, 1H), 6.94 (s, 1H), 4.51-4.35 (m, 1H), 3.34 (s, 2H), 2.69-2.58 (m, 4H), 1.98-1.88 (m, 2H), 1.84-1.62 (m, 6H).

MS *m*/*z* (ESI): 342.1 [M+H]⁺.

### Step 3: preparation of 3-((3-exo)-3-((7-((5-methylthioazol-2-yl)amino)-1,6-diazanaphthalen-5-yl)amino)-8-azabicyclo[3.2.1]oct-8-yl)propionitrile

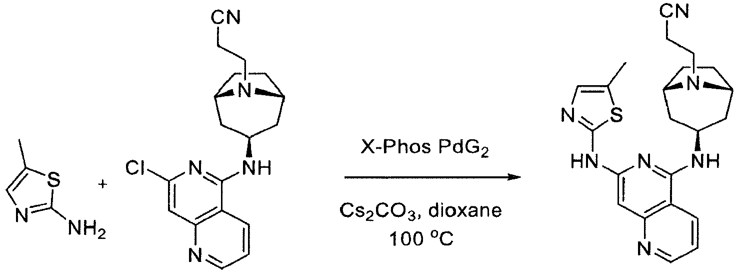

3-((3-*exo*)-3-((7-chloro-1,6-diazanaphthalen-5-yl)amino)-8-azabicyclo[3.2.1]oct-8-yl)propionitrile (50 mg, 0.146 mmol), 5-methylthioazol-2-amine (25 mg, 0.219 mmol), chloro(2-dicyclohexylphosphino-2',4',6'-triisopropyl-1,1'-biphenyl)[2-(2'-amino-1,1'-biphenyl)]palladium(II) (7 mg, 0.009 mmol) and cesium carbonate (86 mg, 0.263 mmol) were added to ultra-dry 1,4-dioxane (5 mL). The reaction mixture was filled with dry nitrogen. The reaction mixture was then sealed, heated to 110 °C and stirred for 24 hours, then cooled to room temperature, and filtered. The filtrate was concentrated under reduced pressure and purified by preparative TLC to obtain the title compound as a yellow-green solid (14 mg, 23%).

¹H NMR (400 MHz, DMSO-*d*₆) *δ* 10.77 (s, 1H), 8.71-8.63 (m, 1H), 8.55 (d, *J* = 8.3 Hz, 1H), 7.47 (d, *J* = 8.6 Hz, 1H), 7.10 (dd, *J* = 8.3, 4.3 Hz, 1H), 7.02 (d, *J* = 1.1 Hz, 1H), 6.46 (s, 1H), 4.96-4.77 (m, 1H), 3.36 (s, 2H), 2.65 (s, 4H), 2.32 (s, 3H), 2.04-1.72 (m, 8H).

MS *m*/*z* (ESI): 420.2 [M+H]⁺.

### Embodiment 2

### 3-(3-exo)-3-((7-((5-(hydroxymethyl)thiazol-2-yl)amino)-1,6-diazadiazanaphthalen-5-yl)amino)-8-azabicyclo[3.2.1]oct-8-yl)propionitrile

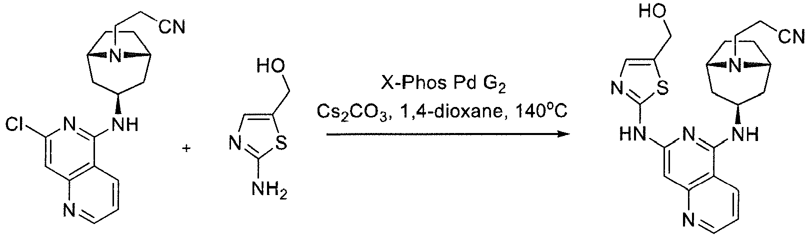

### 3-((3-exo)-3-((7-Chloro-1,6-diazanaphthalen-5-yl)amino)-8-azabicyclo[3.2.1]oct-8-yl)propionitrile (50 mg, 0.146 mmol), (2-aminothiazol-5-yl)methanol (38 mg, 0.292 mmol), chloro(2-dicyclohexylphosphino-2',4',6'-triisopropyl-1,1'-biphenyl)[2-(2'-amino-1,1'-biphenyl)]palladium(II) (58 mg, 0.073 mmol) and cesium carbonate (143 mg, 0.438 mmol) were added to ultra-dry 1,4-dioxane (5 mL). The reaction mixture was filled with dry nitrogen to remove oxygen, heated to 140°C with a microwave reactor, stirred for 2.5 hours, cooled to room temperature, and filtered. The filtrate was concentrated under reduced pressure, and purified by silica gel column chromatography and reverse phase HPLC to obtain the title compound (8.6 mg, 14%).

¹H NMR (400 MHz, DMSO-*d*₆) *δ* 10.85 (s, 1H), 8.70-8.65 (m, 1H), 8.60-8.53 (m, 1H), 7.47 (d, *J* = 8.6 Hz, 1H), 7.18 (s, 1H), 7.11 (dd, *J* = 8.3, 4.3 Hz, 1H), 6.50 (s, 1H), 5.16 (t, *J* = 5.2 Hz, 1H), 4.86 (dd, *J* = 16.7, 8.2 Hz, 1H), 4.56 (d, *J* = 5.2 Hz, 2H), 3.33 (s, 2H), 2.65 (s, 4H), 1.99-1.86 (m, 4H), 1.81 (d, *J* = 7.4 Hz, 4H).

MS *m*/*z* (ESI): 436.1 [M+H]⁺.

**Embodiment 3**

### 3-((3-exo)-3-((4-((5-methyl-1H-pyrazol-3-yl)amino)-7H-pyrrolo[2,3-d]pyrimidin-2-yl)amino)-8-azabicyclo[3.2.1]oct-8-yl)propionitrile

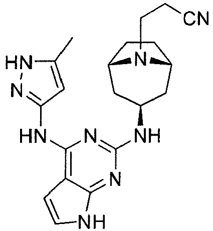

### Step 1: preparation of 2,4-dichloro-5-tosyl-7H-pyrrolo[2,3-d]pyrimidine

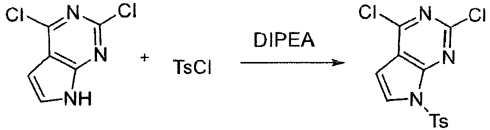

TsCl (242 mg, 1.27 mmol) and DIPEA (273 mg, 2.12 mmol) were added successively into the solution of 2,4-dichloropyrrole[3,2-d]pyrimidine (200 mg, 1.06 mmol) in dichloromethane (5 mL), and stirred overnight at room temperature. When the reaction was complete, the reaction mixture was extracted with DCM (15 mL x 3), and washed with saturated sodium chloride aqueous solution (15 mL x 3). The organic phase was collected and dried over anhydrous sodium sulfate, filtered, and concentrated under reduced pressure to remove the organic solvent. The resulting product was purified by silica gel column chromatography (PE: DCM from 0 to 70:30) to obtain the title compound as a white solid (360 mg, 99%).

MS *m*/*z* (ESI): 343.0 [M+H]⁺.

### Step 2: preparation of 2-chloro-N-(5-methyl-1H-pyrazol-3-yl)-7-tosyl-7H-pyrrolo[2,3-d]pyrimidin-4-amine

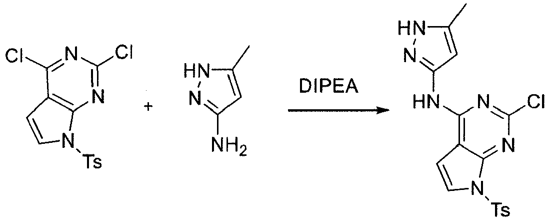

3-Amino-5-methylpyrazol (53 mg, 0.55 mmol) and DIPEA (129 mg, 1 mmol) were added successively into the solution of 2,4-dichloro-5-tosyl-7H-pyrrolo[2,3-d]pyrimidine (171 mg, 0.5 mmol) in MeCN (10 mL), then the mixture was stirred under microwave conditions at 100°C for 2 hours. When the reaction was completed, the reaction mixture was extracted with EA(15 mL x 3) and washed with saturated sodium chloride aqueous solution (15 mL x 3). The organic phase was collected and dried over anhydrous sodium sulfate, filtered and concentrated under reduced pressure. The resulting product was purified by silica gel column chromatography (DCM: MeOH from 0 to 95:5) to obtain the title compound as a pale yellow solid (185 mg, 92%).

MS *m*/*z* (ESI): 402.1 [M+H]⁺.

### Step 3: preparation of tert-butyl-(3-exo)-3-((4-((5-methyl-1H-pyrazol-3-yl)amino)-7-tosyl-7H-pyrrolo[2,3-d]pyrimidin-2-yl)amino)-8-azabicyclo[3.2.1]oct-8-carboxylate

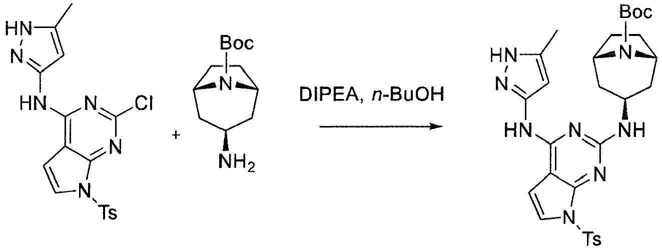

N-Boc-*exo*-3-aminotropane (85 mg, 0.375 mmol) and DIPEA (64.5 mg, 0.5 mmol) were added successively into the solution of 2-chloro-N-(5-methyl-1H-pyrazol-3-yl)-7-tosyl-7H-pyrrolo[2,3-d]pyrimidin-4-amine (100 mg, 0.25 mmol) in n-BuOH (5 mL), then heated to 150°C under microwave condition and stirred for 12 hours. When the reaction was completed, the reaction mixture was extracted with EA (15 mL x 3), and washed with saturated sodium chloride aqueous solution (15 mL x 3). The organic phase was collected and dried over anhydrous sodium sulfate, then filtered, and concentrated under reduced pressure to remove the organic solvent to obtain the title compound as a pale yellow solid (100 mg, 68%).

MS *m*/*z* (ESI): 593.1 [M+H]⁺.

### Step 4: preparation of 3-((3-exo)-3-((4-((5-methyl-1H-pyrazol-3-yl)amino) -7-tosyl-7H-pyrrolo[2,3-d]pyrimidin-2-yl)amino)-8-azabicyclo[3.2.1]oct-8-yl)propionitrile

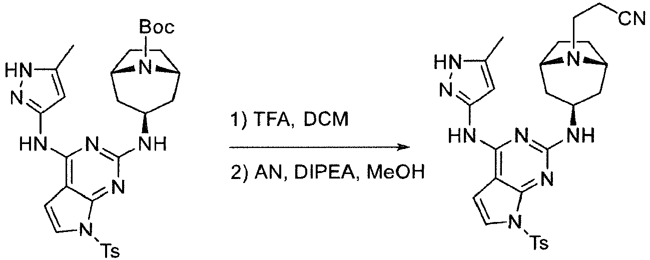

Tert-butyl-(3-exo)-3-((4-((5-methyl-1H-pyrazol-3-yl)amino)-7-tosyl-7H-pyrrolo[2,3-d]pyrimidin-2-yl)amino)-8-azabicyclo[3.2.1]oct-8-carboxylate (100 mg, 0.17 mmol) was dissolved in ethyl acetate hydrochloride (4.0 N, 2 mL). The mixture was stirred at room temperature for 30 minutes, then evaporated to dryness and dissolved with MeOH(10 mL), followed by dropwise addition of DIPEA (88 mg, 0.68 mmol). The mixture was stirred at room temperature for 10 minutes, and acrylonitrile (14 mg, 0.26 mmol) was added and stirred for another 2 hours. The reaction mixture was concentrated under reduced pressure, then purified by silica gel chromatography (DCM:MeOH=10:1) to obtain the title compound as a yellow solid (52.6 mg, 57 %).

MS *m*/*z* (ESI): 546.1 [M+H]⁺.

### Step 5: preparation of 3-((3-exo)-3-((4-((5-methyl-1H-pyrazol-3-yl)amino)-7H-pyrrolo[2,3-d]pyrimidin-2-yl)amino)-8-azabicyclo[3.2.1]oct-8-yl)propionitrile

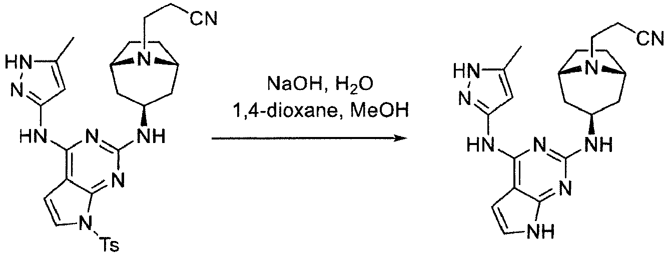

Sodium hydroxide (36 mg, 0.9 mmol) aqueous solution (0.2 mL) was added to the mixed solution of 3-((3-*exo*)-3-((4-((5-methyl-1H-pyrazol-3-yl)amino)-7-tosyl-7H-pyrrolo[2,3-d]pyrimidin-2-yl)amino)-8-azabicyclo[3.2.1]oct-8-yl)propionitrile (50 mg, 0.09 mmol) in 1,4-dioxane (10 mL) and methanol (5 mL). The mixture was then heated to 55°C and stirred overnight. When the reaction was completed, the reaction mixture was concentrated under reduced pressure, then purified by prep-HPLC to obtain the title compound as a white solid (10 mg, 29%).

¹H NMR (400 MHz, DMSO) *δ* 11.85 (s, 1H), 10.72 (s, 1H), 9.38 (s, 1H), 6.67 (s, 3H), 5.92 (s, 1H), 4.11 (s, 1H), 3.29 (s, 2H), 2.62 (s, 4H), 2.20 (s, 3H), 1.90 (s, 2H), 1.82-1.47 (m, 6H).

MS *m*/*z* (ESI): 392.2 [M+H]⁺.

### Embodiment 4

### 3-((3-exo)-3-((4-((5-methyl-1H-pyrazol-3-yl)amino)quinazolin-2-yl)amino)-8-azabicyclo[3.2.1]oct-8-yl)propionitrile

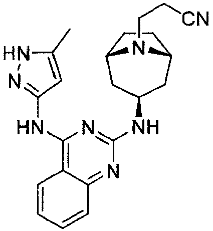

### Step 1: preparation of 2-chloro-N-(5-methyl-1H-pyrazol-3-yl)quinazolin-4-amine

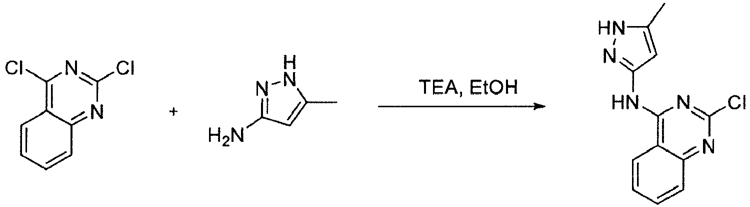

2,4-Dichloroquinazoline (199 mg, 1.0 mmol), 5-methyl-1H-pyrazol-3-amine (99 mg, 1.02 mmol) and triethylamine (213 mg, 2.1 mmol) were added to anhydrous ethanol (5 mL), and the mixture was stirred at room temperature for 18 hours. The reaction mixture was concentrated under reduced pressure, and the resulting solid was suspended in water-ethanol (v\v= 9:1, 20 mL) and then filtered. The resulting solid was washed with petroleum ether, and then dried to obtain the title compound (240 mg, 92%).

MS *m*/*z* (ESI): 260.1, 262.1 [M+H]⁺.

### Step 2: preparation of Tert-butyl (3-exo)-3-((4-((5-methyl-1H-pyrazol-3-yl)amino)quinazolin-2-yl)amino)-8-azabicyclo[3.2.1]oct-8-carboxylate

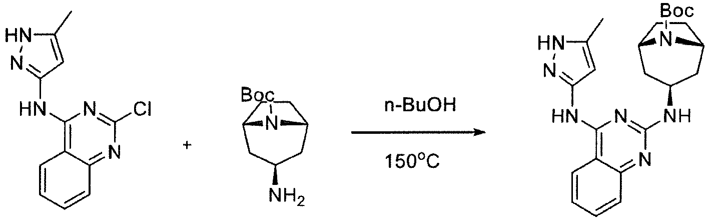

2-Chloro-N-(5-methyl-1H-pyrazol-3-yl)quinazolin-4-amine (40 mg, 0.154 mmol) and tert-butyl (3-*exo*)-3-amino-8-azabicyclo[3.2.1]oct-8-carboxylate (70 mg, 0.308 mmol) were added to n-butanol (3 mL). The mixture was stirred evenly under room temperature, then heated to 150°C with microwave and reacted for 4 hours. The mixture was concentrated under reduced pressure to remove the solvent, and the resulting product was purified by silica gel chromatography to obtain the crude title compound (120 mg), which was used directly in the next step.

MS *m*/*z* (ESI): 450.2 [M+H]⁺.

### Step 3: preparation of 3-((3-exo)-3-((4-((5-methyl-1H-pyrazol-3-yl)amino)quinazolin-2-yl)amino)-8-azabicyclo[3.2.1]oct-8-yl)propionitrile

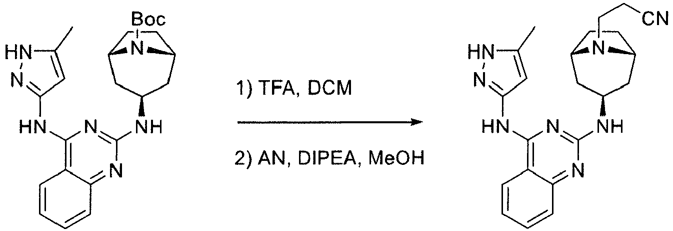

The crude product of Tert-butyl (3-exo)-3-((4-((5-methyl-1H-pyrazol-3-yl)amino)quinazolin-2-yl)amino)-8-azabicyclo[3.2.1]oct-8-carboxylate (120 mg, 0.154 mmol) was dissolved in methanol (3 mL). The mixture was stirred at room temperature and 4M HCl in 1,4-dioxane (10 mL) was added. The resulting mixture was stirred at room temperature for 30 minutes, and concentrated under reduced pressure to remove the solvent. The residue was dissolved in anhydrous methanol (10 mL), stirred at room temperature, followed by successive addition of diisopropylethylamine (0.51 mL, 3.08 mmol) and acrylonitrile (10 mg, 0.154 mmol). The resulting reaction mixture was stirred at room temperature for 2.5 hours, and concentrated under reduced pressure to remove the solvent. The residue was purified successively by silica gel chromatography and reverse phase HPLC to obtain the title compound (6.0 mg, 10%).

¹H NMR (400 MHz, CD₃OD) *δ* 8.04 (d, *J* = 8.1 Hz, 1H), 7.58 (t, *J* = 7.5 Hz, 1H), 7.39 (s, 1H), 7.16 (t, *J* = 7.5 Hz, 1H), 6.62 (s, 1H), 4.35 (s, 1H), 3.37 (s, 2H), 2.76 (t, *J* = 6.9 Hz, 2H), 2.62 (t, *J* = 6.9 Hz, 2H), 2.31 (s, 3H), 2.16-1.74 (m, 6H), 1.67 (t, *J=* 11.7 Hz, 2H).

MS *m*/*z* (ESI): 403.2 [M+H]⁺.

### Embodiment 5

### 3-(cis-5-((7-((5-methyl-1H-pyrazol-3-yl)amino)-1,6-diazanaphthalen-5-yl)amino)hexahydrocyclopentadieno[c]pyrrole-2(1H)-yl)propionitrile

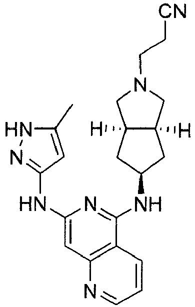

### Step 1: preparation of tert-butyl(3aR,5r,6aS)-5-((7-chloro-1,6-diazanaphthalen-5-yl)amino)hexahydrocyclopentadieno[c]pyrrole-2(1H)-carboxylate

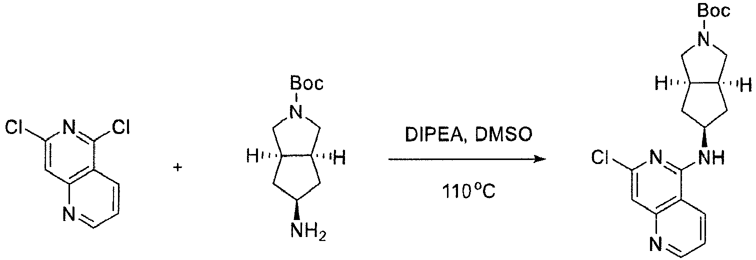

Tert-butyl *cis*-5-aminohexahydrocyclopentadieno[c]pyrrole-2(1H)-carboxylate (100 mg, 0.442 mmol), 5,7-dichloro-1,6-diazanaphthalen(83 mg, 0.42 mmol) and diisopropylethylamine (0.208 mL, 1.26 mmol) were dissolved in DMSO(2 mL). The mixture then heated to 110 °C and stirred for 15 hours. The reaction mixture was then diluted with ethyl acetate. The organic phase was washed with saturated sodium chloride aqueous solution. The organic phase was collected, dried over anhydrous sodium sulfate, filtered, and concentrated under reduced pressure to remove the organic solvent. The resulting product was purified by silica gel chromatography to obtain the title compound (142 mg, 87%).

MS *m*/*z* (ESI): 389.4 [M+H]⁺.

### Step 2: preparation of 3-(cis-5-((7-chloro-1,6-diazanaphthalen-5-yl)amino)hexahydrocyclopentadieno[c]pyrrole-2(1H)-yl)propionitrile

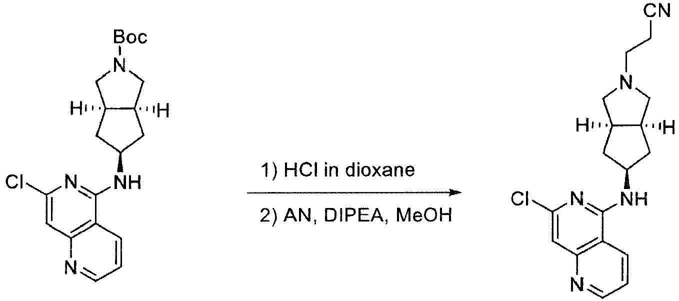

Tert-butyl *cis*-5-((7-chloro-1,6-diazanaphthalen-5-yl)amino)hexahydrocyclopentadieno[c]pyrrole-2(1H)-carboxylate (142 mg, 0.365 mmol) was dissolved in methanol (2 mL). The mixture was stirred at room temperature then 4M HCl in 1,4-dioxane (10 mL) was added. The mixture was stirred at room temperature for 30 minutes, and concentrated under reduced pressure. The residue was dissolved in anhydrous methanol (10 mL), diisopropylethylamine (1.2 mL, 7.3 mmol) and acrylonitrile (0.03 mL, 0.438 mmol) were added successively. The resulting reaction mixture was stirred at room temperature for 2 hours, and concentrated under reduced pressure to remove the solvent. The residue was successively purified by silica gel chromatography to obtain the title compound (123 mg, 98%).

MS *m*/*z* (ESI): 342.1 [M+H]⁺.

### Step 3: preparation of 3-(cis-5-((7-((5-methyl-1H-pyrazol-3-yl)amino)-1,6-diazanaphthalen-5-yl)amino)hexahydrocyclopentadieno[c]pyrrole-2(1H)-yl)propionitrile

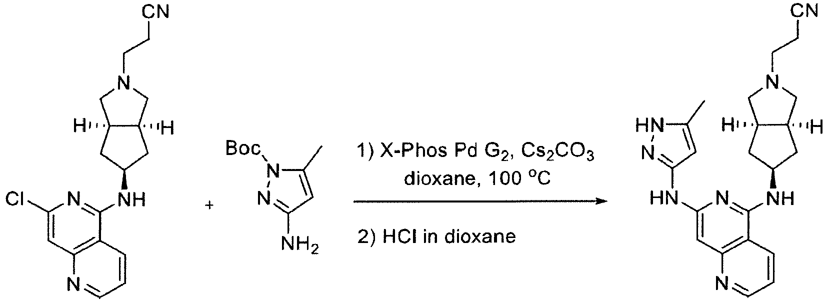

3-(*cis*-5-((7-chloro-1,6-diazanaphthalen-5-yl)amino)hexahydrocyclopentadieno[c]pyrrole-2(1H)-yl)propionitrile (50 mg, 0.146 mmol), tert-butyl 3-amino-5-methyl-1H-pyrazol-1-carboxylate (44 mg, 0.219 mmol), chloro(2-dicyclohexylphosphino-2',4',6'-triisopropyl-1,1'-biphenyl)[2-(2'-amino-1,1'-biphenyl)]palladium(II) (7 mg, 0.009 mmol) and cesium carbonate(86 mg, 0.263 mmol) were added to ultra-dry 1,4-dioxane(5 mL). The reaction mixture was filled with dry nitrogen. The reaction mixture was then sealed, heated to 100°C and stirred for 17 hours, cooled to room temperature, and filtered. The filtrate was concentrated under reduced pressure. The mixture was dissolved in 4M HCl in 1,4-dioxane(10 mL), and stirred at room temperature for 30 minutes, and concentrated under reduced pressure to remove the solvent. The residue was purified by prep-HPLC to obtain the title compound (2.6 mg, 4%).

¹H NMR (400 MHz, CD₃OD) *δ* 8.54 (dd, *J* = 9.0, 5.7 Hz, 1H), 8.43-8.24 (m, 1H), 7.06 (d, *J* = 5.8 Hz, 1H), 6.66 (d, *J* = 5.2 Hz, 1H), 6.12 (s, 1H), 4.63-4.48 (m, 1H), 3.06-2.56 (m, 8H), 2.55-2.34 (m, 4H), 2.28 (s, 3H), 1.57 (s, 2H).

MS *m*/*z* (ESI): 403.2 [M+H]⁺.

### Embodiment 6

### 3-((3-exo)-3-((7-((1H-pyrazol-3-yl)amino)-1,6-diazanaphthalen-5-yl)amino)-8-azabicyclo[3.2.1]oct-8-yl)propionitrile

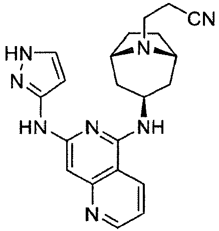

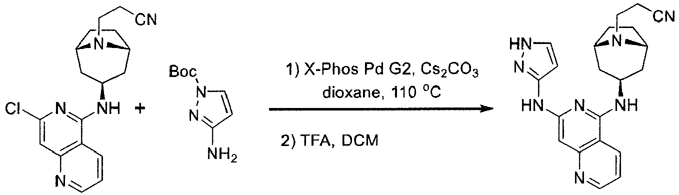

3-((3-*exo*)-3-((7-chloro-1,6-diazanaphthalen-5-yl)amino)-8-azabicyclo[3.2.1]oct-8-yl)propionitrile (50 mg, 0.146 mmol), tert-butyl 3-(12-azaalkyl)-1H-pyrazol-1-carboxylate (40 mg, 0.219 mmol), chloro(2-dicyclohexylphosphino-2,4',6'-triisopropyl-1,1'-biphenyl)[2-(2'-amino-1,1'-biphenyl)]palladium(II)e (7 mg, 0.009 mmol) and cesium carbonate (86 mg, 0.263 mmol) were added to ultra-dry 1,4-dioxane (5 mL), the reaction mixture was filled with dry nitrogen to remove the oxygen. The reaction mixture was then sealed, heated to 110°C and stirred for 16 hours, cooled to room temperature, and filtered. The filtrate was concentrated under reduced pressure. The mixture was dissolved in dichloromethane (2 mL), followed by addition of trifluoroacetate acid (1 mL). The mixture was stirred at room temperature for 30 minutes, and concentrated under reduced pressure. The residue was purified by prep-HPLC to obtain the title compound (17 mg, 31%).

¹H NMR (400 MHz, DMSO-*d*₆) *δ* 12.07 (s, 1H), 8.81 (s, 1H), 8.58 (d, *J* = 3.3 Hz, 1H), 8.42 (d, *J* = 8.6 Hz, 1H), 7.58 (s, 1H), 7.14 (dd, *J* = 6.2, 2.8 Hz, 1H), 6.97 (dd, *J* = 7.9, 4.2 Hz, 1H), 6.75 (s, 1H), 6.34 (s, 1H), 4.59-4.42 (m, 1H), 3.33 (s, 2H), 2.63 (s, 4H), 1.97-1.86 (m, 2H), 1.78 (ddt, *J=* 24.7, 23.4, 7.2 Hz, 6H).

MS *m*/*z* (ESI): 389.2 [M+H]⁺.

### Embodiment 7

### 3-((3-exo)-3-((7-((5-(difluoromethyl)-1H-pyrazol-3-yl)amino)-1,6-diazanaphthalen-5-yl)amino)-8-azabicyclo[3.2.1]oct-8-yl)propionitrile

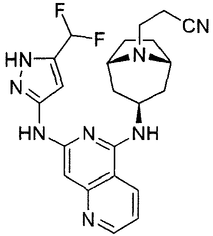

The preparation of 3-((3-*exo*)-3-((7-((5-(difluoromethyl)-1H-pyrazol-3-yl)amino)-1,6-diazanaphthalen-5-yl)amino)-8-azabicyclo[3.2.1]oct-8-yl)propionitrile was carried out with the reference to Embodiment 1.

MS *m*/*z* (ESI): 439.2 [M+H]⁺.

### Embodiment 8

### 3-((3-exo)-3-((7-((5-(trifluoromethyl)-1H-pyrazol-3-yl)amino)-1,6-diazanaphthalen-5-yl)amino)-8-azabicyclo[3.2.1]oct-8-yl)propionitrile

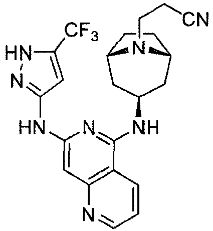

The preparation of 3-((3-*exo*)-3-((7-((5-(trifluoromethyl)-1H-pyrazol-3-yl)amino)-1,6-diazanaphthalen-5-yl)amino)-8-azabicyclo[3.2.1]oct-8-yl)propionitrile was carried out with the reference to Embodiment 1.

MS *m*/*z* (ESI): 457.2 [M+H]⁺.

### Embodiment 9

### 3-((3-exo)-3-((7-((5-fluoro-1H-pyrazol-3-yl)amino)-1,6-diazanaphthalen-5-yl)amino)-8-azabicyclo[3.2.1]oct-8-yl)propionitrile

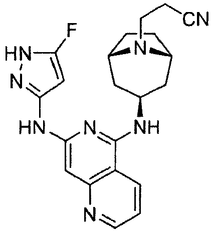

The preparation of 3-((3-*exo*)-3-((7-((5-fluoro-1H-pyrazol-3-yl)amino)-1,6-diazanaphthalen-5-yl)amino)-8-azabicyclo[3.2.1]oct-8-yl)propionitrile was carried out with the reference to Embodiment 1.

¹H NMR (400 MHz, DMSO-*d*₆) *δ* 11.61 (d, *J* = 1.5 Hz, 1H), 9.35 (s, 1H), 8.65 (dd, *J* = 4.3, 1.2 Hz, 1H), 8.49 (d, *J* = 8.8 Hz, 1H), 7.40 (d, *J* = 7.8 Hz, 1H), 7.07 (dd, *J* = 8.4, 4.3 Hz, 1H), 6.25 (s, 1H), 5.77 (dd, *J* = 6.0, 2.1 Hz, 1H), 4.47-4.39 (m, 1H), 3.32 (s, 2H), 2.62 (t, *J* = 4.2 Hz, 4H), 1.95-1.86 (m, 2H), 1.83-1.70 (m, 6H).

MS *m*/*z* (ESI): 407.2 [M+H]⁺.

### Embodiment 10

### 3-((5-(((3-exo)-8-(2-cyanoethyl)-8-azabicyclo[3.2.1]oct-3-yl)amino)-1,6-diazanaphthalen-7-yl)amino)-1H-pyrazol-5-formonitrile

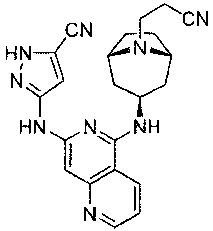

The preparation of 3-((5-(((3-*exo*)-8-(2-cyanoethyl)-8-azabicyclo[3.2.1]oct-3-yl)amino)-1,6-diazanaphthalen-7-yl)amino)-1H-pyrazol-5-formonitrile was carried out with the reference to Embodiment 1.

MS *m*/*z* (ESI): 414.2 [M+H]⁺.

### Embodiment 11

### 3-((3-exo)-3-((7-((5-ethyl-1H-pyrazol-3-yl)amino)-1,6-diazanaphthalen-5-yl)amino)-8-azabicyclo[3 .2.1]oct-8-yl)propionitrile

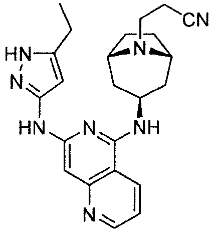

3-((3-*exo*)-3-((7-chloro-1,6-diazanaphthalen-5-yl)amino)-8-azabicyclo[3.2.1]oct-8-yl)propionitrile (100 mg, 0.292 mmol), 5-ethyl-1H-pyrazol-3-amine (65 mg, 0.584 mmol), chloro(2-dicyclohexylphosphino-2',4',6'-triisopropyl-1,1'-biphenyl)[2-(2'-amino-1,1'-biphenyl)]palladium(II) (115 mg, 0.146 mmol) and cesium carbonate (286 mg, 0.876 mmol) were added to ultra-dry 1,4-dioxane (10 mL). The reaction mixture was filled with dry nitrogen, heated to 150 °C with a microwave reactor and reacted for 8 hours. Then the mixture was cooled down to room temperature, and filtered. The filtrate was concentrated under reduced pressure, and successively purified by silica gel chromatography and reverse phase HPLC to obtain the title compound (2.2 mg, 2 %).

¹H NMR (400 MHz, DMSO-*d*₆) *δ* 11.77 (s, 1H), 8.71 (s, 1H), 8.57 (dd, *J* = 4.1, 1.4 Hz, 1H), 8.40 (d, *J* = 7.1 Hz, 1H), 7.12 (d, *J* = 8.4 Hz, 1H), 6.95 (dd, *J* = 8.1, 4.4 Hz, 1H), 6.68 (s, 1H), 6.16 (s, 1H), 4.53 (dd, *J* = 12.8, 5.6 Hz, 1H), 3.33 (s, 2H), 2.63 (ddd, *J* = 24.2, 9.2, 4.8 Hz, 6H), 1.92 (dd, *J* = 8.1, 4.4 Hz, 2H), 1.85-1.66 (m, 6H), 1.20 (t, *J* = 7.6 Hz, 3H).

MS *m*/*z* (ESI): 417.2 [M+H]⁺.

**Embodiment 12**

### 3-((3-exo)-3-((7-((5-cyclopropyl-1H-pyrazol-3-yl)amino)-1,6-diazanaphthalen-5-yl)amino)-8-azabicyclo[3.2.1]oct-8-yl)propionitrile

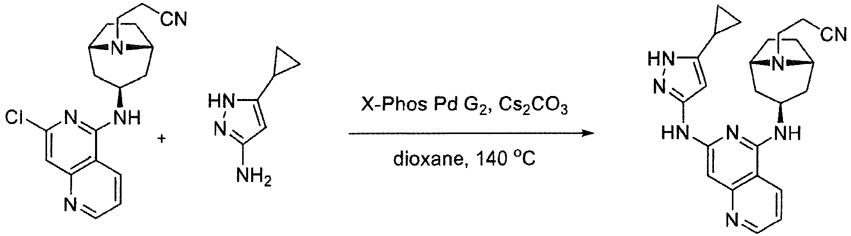

3-((3-*exo*)-3-((7-chloro-1,6-diazanaphthalen-5-yl)amino)-8-azabicyclo[3.2.1]oct-8-yl)propionitrile (100 mg, 0.292 mmol), 5-cyclopropyl-1H-pyrazol-3-amine (72 mg, 0.585 mmol), chloro(2-dicyclohexylphosphino-2',4',6'-triisopropyl-1,1'-biphenyl)[2-(2'-amino-1,1'-biphenyl)]palladium(II) (115 mg, 0.146 mmol) and cesium carbonate (285 mg, 0.876 mmol) were added to ultra-dry 1,4-dioxane (5 mL). The reaction mixture was filled with dry nitrogen, heated to 150°C with microwave, stirred for 3 hours, cooled to room temperature, and filtered. The filtrate was concentrated under reduced pressure, and successively purified by silica gel chromatography and reverse phase HPLC to obtain the title compound (20 mg, 16%).

¹H NMR (400 MHz, DMSO-*d*₆) *δ* 11.75 (s, 1H), 8.67 (s, 1H), 8.57 (d, *J=* 3.2 Hz, 1H), 8.44-8.36 (m, 1H), 7.12 (ddd, *J=* 12.4, 9.6, 3.9 Hz, 1H), 6.96 (dd, *J=* 8.0, 3.9 Hz, 1H), 6.70 (s, 1H), 6.06 (s, 1H), 4.53 (d, *J=* 4.9 Hz, 1H), 3.33 (s, 2H), 2.63 (s, 4H), 2.01-1.63 (m, 9H), 0.91 (d, *J=* 7.5 Hz, 2H), 0.70 (d, *J=* 4.3 Hz, 2H).

MS *m*/*z* (ESI): 429.2 [M+H]⁺.

### Embodiment 13

### 3-((3-exo)-3-((7-((5-(oxetan-3-yl)-1H-pyrazol-3-yl)amino)-1,6-diazanaphthalen-5-yl)amino)-8-azabicyclo[3.2.1]oct-8-yl)propionitrile

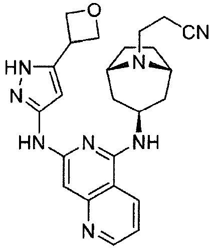

The preparation of 3-((3-*exo*)-3-((7-((5-(oxetan-3-yl)-1H-pyrazol-3-yl)amino)-1,6-diazanaphthalen-5-yl)amino)-8-azabicyclo[3.2.1]oct-8-yl)propionitrile was carried out with the reference to Embodiment 12.

MS *m*/*z* (ESI): 445.2 [M+H]⁺.

### Embodiment 14

### 3-((3-exo)-3-((7-((5-(1-methylazetindin-3-yl)-1H-pyrazol-3-yl)amino)-1,6-diazanaphthalen-5-yl)amino)-8-azabicyclo[3.2.1]oct-8-yl)propionitrile

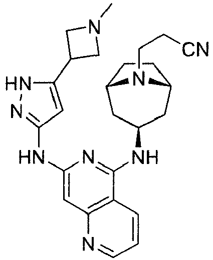

The preparation of 3-((3-*exo*)-3-((7-((5-(1-methylazetindin-3-yl)-1H-pyrazol-3-yl)amino)-1,6-diazanaphthalen-5-yl)amino)-8-azabicyclo[3.2.1]oct-8-yl)propionitrile was carried out with the reference to Embodiment 12.

MS *m*/*z* (ESI): 458.3 [M+H]⁺.

### Embodiment 15

### 3-((3-exo)-3-((7-((4,5-dimethyl-1H-pyrazol-3-yl)amino)-1,6-diazanaphthalen-5-yl)amino)-8-azabicyclo[3.2.1]oct-8-yl)propionitrile

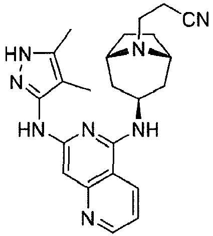

The preparation of 3-((3-*exo*)-3-((7-((4,5-dimethyl-1H-pyrazol-3-yl)amino)-1,6-diazanaphthalen-5-yl)amino)-8-azabicyclo[3.2.1]oct-8-yl)propionitrile was carried out with the reference to Embodiment 12.

¹H NMR (400 MHz, DMSO-*d*₆) *δ* 8.76 (dd, *J=* 4.2, 1.3 Hz, 1H), 8.60 (d, *J=* 8.2 Hz, 1H), 7.44 (d, *J* = 8.4 Hz, 1H), 7.24 (dd, *J* = 8.3, 4.4 Hz, 1H), 7.10 (s, 1H), 4.97 (s, 2H), 4.52 (dd, *J* = 16.9, 8.1 Hz, 1H), 3.33 (s, 2H), 2.66 (dd, *J* = 10.4, 2.9 Hz, 7H), 1.92 (ddd, *J* = 5.9, 3.4, 1.8 Hz, 2H), 1.85 (s, 3H), 1.72 (ddd, *J=* 20.9, 11.4, 4.3 Hz, 6H).

MS *m*/*z* (ESI): 417.1 [M+H]⁺.

### Embodiment 16

### 3-((3-exo)-3-((7-((4-fluoro-5-methyl-1H-pyrazol-3-yl)amino)-1,6-diazanaphthalen-5-yl)amino)-8-azabicyclo[3.2.1]oct-8-yl)propionitrile

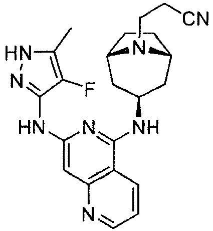

The preparation of 3-((3-*exo*)-3-((7-((4-fluoro-5-methyl-1H-pyrazol-3-yl)amino)-1,6-diazanaphthalen-5-yl)amino)-8-azabicyclo[3.2.1]oct-8-yl)propionitrile was carried out with the reference to Embodiment 12.

MS *m*/*z* (ESI): 421.2 [M+H]⁺.

### Embodiment 17

### 3-((3-exo)-3-((7-((5-methoxyl-1H-pyrazol-3-yl)amino)-1,6-diazanaphthalen-5-yl)amino)-8-azabicyclo[3.2.1]oct-8-yl)propionitrile

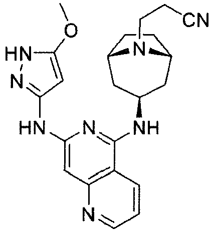

The preparation of 3-((3-*exo*)-3-((7-((5-methoxyl-1H-pyrazol-3-yl)amino)-1,6-diazanaphthalen-5-yl)amino)-8-azabicyclo[3.2.1]oct-8-yl)propionitrile was carried out with the reference to Embodiment 12.

MS *m*/*z* (ESI): 419.2 [M+H]⁺.

### Embodiment 18

### 3-((3-exo)-3-((7-((5-(methoxylmethyl)-1H-pyrazol-3-yl)amino)-1,6-diazanaphthalen-5-yl)amino)-8-azabicyclo[3.2.1]oct-8-yl)propionitrile

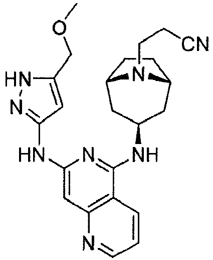

3-((3-*exo*)-3-((7-chloro-1,6-diazanaphthalen-5-yl)amino)-8-azabicyclo[3.2.1]oct-8-yl)propionitrile (72 mg, 0.21 mmol), 2-(2-aminothiazol-5-yl)propan-2-ol (54 mg, 0.42 mmol), chloro(2-dicyclohexylphosphino-2',4',6'-triisopropyl-1,1'-biphenyl)[2-(2'-amino-1,1'-biphenyl)]palladium(II) (83 mg, 0.105 mmol) and cesium carbonate (205 mg, 0.63 mmol) were added to ultra-dry 1,4-dioxane (10 mL). The reaction mixture was filled with dry nitrogen, heated to 150 °C with a microwave reactor and stirred for 7 hours. Then the mixture was cooled to room temperature, and filtered. The filtrate was concentrated under reduced pressure, and purified by prep-HPLC to obtain the title compound (22.4 mg, 25 %).

¹H NMR (400 MHz, DMSO-*d*₆) *δ* 8.57 (d, *J* = 3.5 Hz, 1H), 8.50 (d, *J* = 8.6 Hz, 1H), 7.07 (dd, *J* = 8.0, 4.7 Hz, 1H), 6.74 (s, 1H), 6.35 (s, 1H), 4.63 (td, *J* = 10.8, 6.1 Hz, 1H), 4.43 (s, 2H), 3.71-3.48 (m, 2H), 3.35 (s, 3H), 2.93 (s, 2H), 2.75 (s, 2H), 2.23-1.75 (m, 8H).

MS *m*/*z* (ESI): 433.2 [M+H]⁺.

### Embodiment 19

### 3-((3-exo)-3-((7-((5-(hydroxymethyl)-1H-pyrazol-3-yl)amino)-1,6-diazanaphthalen-5-yl)amino)-8-azabicyclo[3.2.1]oct-8-yl)propionitrile

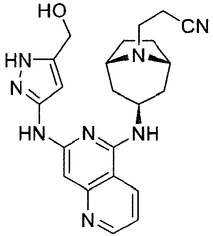

3-((3-*exo*)-3-((7-Chloro-1,6-diazanaphthalen-5-yl)amino)-8-azabicyclo[3.2.1]oct-8-yl)propionitrile (100 mg, 0.292 mmol), (3-amino-1H-pyrazol-5-yl)methanol (66 mg, 0.584 mmol), chloro(2-dicyclohexylphosphino-2',4',6'-triisopropyl-1,1'-biphenyl)[2-(2'-amino-1,1'-biphenyl)]palladium(II) (115 mg, 0.146 mmol) and cesium carbonate (286 mg, 0.877 mmol) were added to ultra-dry 1,4-dioxane (15 mL), the reaction mixture was filled with dry nitrogen, heated to 150 °C with a microwave reactor and stirred for 6 hours. Then the mixture was cooled to room temperature, and filtered. The filtrate was concentrated under reduced pressure, and purified by prep-HPLC to obtain the title compound (15.7 mg, 13 %).

¹H NMR (400 MHz, DMSO-*d*₆) *δ* 11.94 (s, 1H), 8.77 (s, 1H), 8.58 (d, *J* = 3.3 Hz, 1H), 8.41 (d, *J* = 8.1 Hz, 1H), 7.13 (s, 1H), 6.97 (dd, *J* = 7.9, 4.4 Hz, 1H), 6.72 (s, 1H), 6.24 (s, 1H), 5.20 (d, *J* = 1.8 Hz, 1H), 4.53 (dd, *J* = 6.8, 4.2 Hz, 1H), 4.45 (d, *J* = 5.2 Hz, 2H), 3.33 (s, 2H), 2.63 (s, 4H), 1.95-1.87 (m, 2H), 1.80 (dd, *J* = 13.3, 7.7 Hz, 4H), 1.75-1.67 (m, 2H).

MS *m*/*z* (ESI): 419.2 [M+H]⁺.

### Embodiment 20

### 3-((3-exo)-3-((7-((5-(2-hydroxypropan-2-yl)-1H-pyrazol-3-yl)amino)-1,6-diazanaphthalen-5-yl)amino)-8-azabicyclo[3.2.1]oct-8-yl)propionitrile

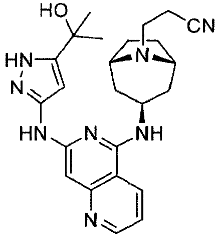

The preparation of 3-((3-*exo*)-3-((7-((5-(2-hydroxypropan-2-yl)-1H-pyrazol-3-yl)amino)-1,6-diazanaphthalen-5-yl)amino)-8-azabicyclo[3.2.1]oct-8-yl)propionitrile was carried out with the reference to Embodiment 2.

MS *m*/*z* (ESI): 447.3 [M+H]⁺.

### Embodiment 21

### 3-((5-(((3-exo)-8-(2-cyanoethyl)-8-azabicyclo[3.2.1]oct-3-yl)amino)-1,6-diazanaphthalen-7-yl)amino)-1H-pyrazol-5-formamide

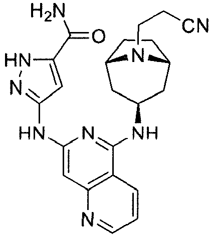

The preparation of 3-((5-(((3-exo)-8-(2-cyanoethyl)-8-azabicyclo[3.2.1]oct-3-yl)amino)-1,6-diazanaphthalen-7-yl)amino)-1H-pyrazol-5-formamide was carried out with the reference to Embodiment 2.

MS *m*/*z* (ESI): 432.2 [M+H]⁺.

### Embodiment 22

### 3-((5-(((3-exo)-8-(2-cyanoethyl)-8-azabicyclo[3.2.1]oct-3-yl)amino)-1,6-diazanaphthalen-7-yl)amino)-N-methyl-1H-pyrazol-5-formamide

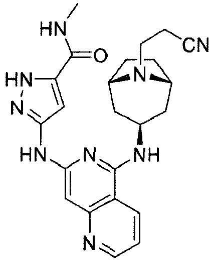

The preparation of 3-((5-(((3-*exo*)-8-(2-cyanoethyl)-8-azabicyclo[3.2.1]oct-3-yl)amino)-1,6-diazanaphthalen-7-yl)amino)-N-methyl-1H-pyrazol-5-formamide was carried out with the reference to Embodiment 2.

MS *m*/*z* (ESI): 446.2 [M+H]⁺.

### Embodiment 23

### 3-((3-exo)-3-((7-((2-methyl-1H-imidazol-4-yl)amino)-1,6-diazanaphthalen-5-yl)amino)-8-azabicyclo[3.2.1]oct-8-yl)propionitrile

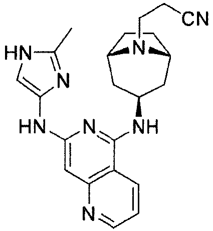

The preparation of 3-((3-*exo*)-3-((7-((2-methyl-1H-imidazol-4-yl)amino)-1,6-diazanaphthalen-5-yl)amino)-8-azabicyclo[3.2. 1]oct-8-yl)propionitrile was carried out with the reference to Embodiment 6.

MS *m*/*z* (ESI): 403.2 [M+H]⁺.

### Embodiment 24

### 3-((3-exo)-3-((7-((5-methyl-1H-1,2,4-triazol-3-yl)amino)-1,6-diazanaphthalen-5-yl)amino)-8-azabicyclo[3.2.1]oct-8-yl)propionitrile

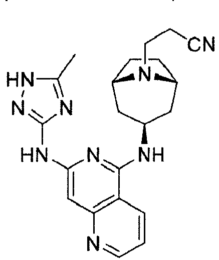

3-((3-*exo*)-3-((7-Chloro-1,6-diazanaphthalen-5-yl)amino)-8-azabicyclo[3.2.1]oct-8-yl)propionitrile (100 mg, 0.292 mmol), 5-methyl-4H-1,2,4-triazol-3-amine hydrochloride (47.2 mg, 0.351 mmol), chloro(2-dicyclohexylphosphino-2',4',6'-triisopropyl-1,1'-biphenyl)[2-(2'-amino-1,1'-biphenyl)]palladium(II) (115 mg, 0.146 mmol) and cesium carbonate (171 mg, 0.526 mmol) were added to ultra-dry 1,4-dioxane (10 mL). The reaction mixture was filled with dry nitrogen, heated to 150 °C with a microwave reactor and stirred for 4 hours. Then the mixture was cooled to room temperature, and filtered. The filtrate was concentrated under reduced pressure, and successively purified by silica gel chromatography and reverse phase HPLC to obtain the title compound (12.4 mg, 10.5%).

¹H NMR (400 MHz, DMSO-*d₆*) *δ* 12.73 (s, 1H), 9.19 (s, 1H), 8.68-8.62 (m, 1H), 8.47 (d, *J* = 8.3 Hz, 1H), 7.23 (dt, *J* = 39.0, 8.1 Hz, 2H), 7.08 (dd, *J* = 8.2, 4.2 Hz, 1H), 4.48 (td, *J* = 12.0, 6.7 Hz, 1H), 3.31-3.30 (m, 2H), 2.63 (t, *J* = 3.0 Hz, 4H), 2.28 (s, 3H), 1.92-1.80 (m, 6H), 1.69 (t, *J* = 11.8 Hz, 2H).

MS *m*/*z* (ESI): 404.2 [M+H]⁺.

### Embodiment 25

### 3-((3-exo)-3-((7-((1-methyl-1H-imidazol-4-yl)amino)-1,6-diazanaphthalen-5-yl)amino)-8-azabicyclo[3.2.1]oct-8-yl)propionitrile

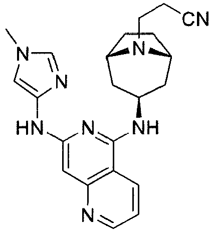

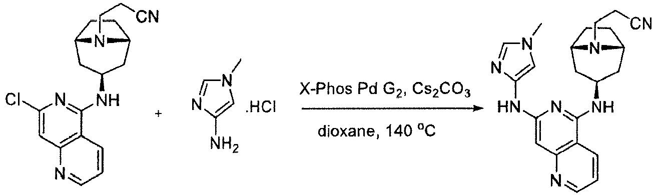

3-((3-*exo*)-3-((7-Chloro-1,6-diazanaphthalen-5-yl)amino)-8-azabicyclo[3.2.1]oct-8-yl)propionitrile(100 mg, 0.292mmol), 1-methyl-1H-imidazol-4-amine hydrochloride (78 mg, 0.585 mmol), chloro(2-dicyclohexylphosphino-2',4',6'-triisopropyl-1,1'-biphenyl)[2-(2'-amino-1,1'-biphenyl)]palladium(II) (115 mg, 0.146 mmol) and cesium carbonate(477 mg, 1.46 mmol) were added to ultra-dry 1,4-dioxane(5 mL). The reaction mixture was filled with dry nitrogen, heated to 140°C with microwave, stirred for 4 hours, cooled to room temperature, and filtered. The filtrate was concentrated under reduced pressure, and successively purified by silica gel chromatography and reverse phase HPLC to obtain the title compound (4.4 mg, 4%).

¹H NMR (400 MHz, DMSO-*d₆*) *δ* 8.77 (s, 1H), 8.55 (dd, *J* = 4.2, 1.3 Hz, 1H), 8.40 (d, *J* = 8.4 Hz, 1H), 7.36 (s, 1H), 7.17 (d, *J* = 7.5 Hz, 2H), 6.92 (dd, *J* = 8.3, 4.3 Hz, 1H), 6.32 (s, 1H), 4.56 (ddd, *J* = 17.5, 12.0, 6.1 Hz, 1H), 3.64 (s, 3H), 3.34 (s, 2H), 2.64 (s, 4H), 2.06-1.65 (m, 8H).

MS *m*/*z* (ESI): 403.2 [M+H]⁺.

### Embodiment 26

### 3-((3-exo)-3-((7-((1-methyl-1H-1,2,4-triazol-3-yl)amino)-1,6-diazanaphthalen-5-yl)amino)-8-azabicyclo[3.2.1]oct-8-yl)propionitrile

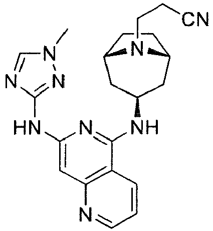

The preparation of 3-((3-*exo*)-3-((7-((1-methyl-1H-1,2,4-triazol-3-yl)amino)-1,6-diazanaphthalen-5-yl)amino)-8-azabicyclo[3.2.1]oct-8-yl)propionitrile was carried out with the reference to Embodiment 25.

MS *m*/*z* (ESI): 404.2 [M+H]⁺.

### Embodiment 27

### 3-((3-exo)-3-((7-((1H-indazol-3-yl)amino)-1,6-diazanaphthalen-5-yl)amino)-8-azabicyclo[3.2.1]oct-8-yl)propionitrile

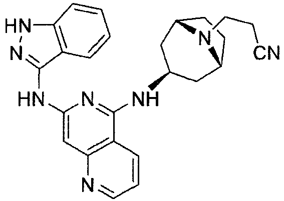

The preparation of 3-((3-*exo*)-3-((7-((1H-indazol-3-yl)amino)-1,6-diazanaphthalen-5-yl)amino)-8-azabicyclo[3.2.1]oct-8-yl)propionitrile was carried out with the reference to Embodiment 6.

MS *m*/*z* (ESI): 439.2 [M+H]⁺.

### Embodiment 28

### 3-((3-exo)-3-((7-((1H-pyrazolo[3,4-c]pyridin-3-yl)amino)-1,6-diazanaphthalen-5-yl)amino)-8-azabicyclo[3.2.1]oct-8-yl)propionitrile

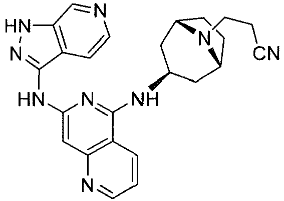

The preparation of 3-((3-*exo*)-3-((7-((1H-pyrazolo[3,4-c]pyridin-3-yl)amino)-1,6-diazanaphthalen-5-yl)amino)-8-azabicyclo[3.2.1]oct-8-yl)propionitrile was carried out with the reference to Embodiment 6.

MS *m*/*z* (ESI): 440.2 [M+H]⁺.

### Embodiment 29

### 3-((3-exo)-3-((7-(pyrazin-2-ylamino)-1,6-diazanaphthalen-5-yl)amino)-8-azabicyclo[3.2.1]oct-8-yl)propionitrile

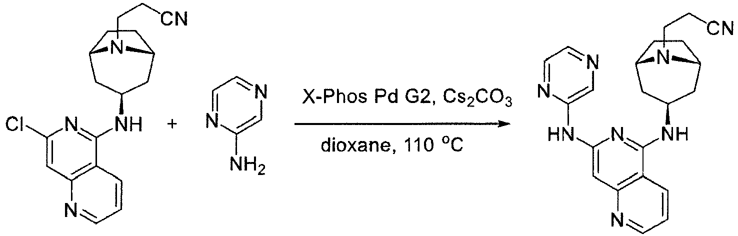

3-((3-*exo*)-3-((7-Chloro-1,6-diazanaphthalen-5-yl)amino)-8-azabicyclo[3.2.1]oct-8-yl)propionitrile (50 mg, 0.146 mmol), pyrazin-2-amine(21 mg, 0.219 mmol), chloro(2-dicyclohexylphosphino-2',4',6'-triisopropyl-1,1'-biphenyl)[2-(2'-amino-1,1'-biphenyl)]palladium(II) (7 mg, 0.009 mmol) and cesium carbonate(86 mg, 0.263 mmol) were added to ultra-dry 1,4-dioxane(5 mL). The reaction mixture was filled with dry nitrogen. The mixture was then sealed, heated to 110°C, stirred for 23 hours, cooled to room temperature, and filtered. The filtrate was concentrated under reduced pressure, and purified by prep-HPLC to obtain the title compound (30.2 mg, 52%).

¹H NMR (400 MHz, DMSO-*d₆*) *δ* 9.65 (s, 1H), 9.28 (d, *J* = 0.4 Hz, 1H), 8.70 (d, *J* = 3.1 Hz, 1H), 8.55 (d, *J* = 8.3 Hz, 1H), 8.33-8.20 (m, 3H), 8.07 (d, *J* = 2.5 Hz, 1H), 7.40 (d, *J* = 8.0 Hz, 1H), 7.15 (dd, *J* = 8.3, 4.3 Hz, 1H), 7.04 (s, 1H), 4.54 (ddd, *J* = 16.1, 11.1, 6.6 Hz, 1H), 3.34 (d, *J* = 0.9 Hz, 2H), 2.64 (s, 4H), 2.00-1.69 (m, 8H).

MS *m*/*z* (ESI): 401.3 [M+H]⁺.

### Embodiment 30

### 3-((3-exo)-3-((7-(pyrimidin-2-ylamino)-1,6-diazanaphthalen-5-yl)amino)-8-azabicyclo[3.2.1]oct-8-yl)propionitrile

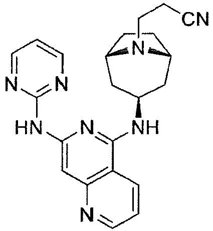

The preparation of 3-((3-*exo*)-3-((7-(pyrimidin-2-ylamino)-1,6-diazanaphthalen-5-yl)amino)-8-azabicyclo[3.2.1]oct-8-yl)propionitrile was carried out with the reference to Embodiment 29.

MS *m*/*z* (ESI): 401.2 [M+H]⁺.

### Embodiment 31

### 3-((3-exo)-3-((7-((5-(e2-hydroxypropan-2-yl)thiazol-2-yl)amino)-1,6-diazanaphthalen-5-yl)amino)-8-azabicyclo[3.2.1]oct-8-yl)propionitrile

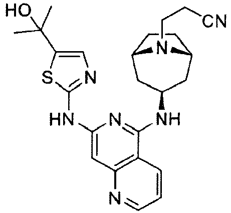

### Step 1: preparation of 2-(2-aminothiazol-5-yl)propan-2-ol

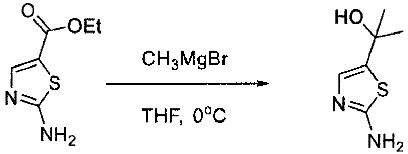

Ethyl 2-aminothiazol-5-carboxylate (345 mg, 2.0 mmol) was dissolved in dry tetrahydrofuran (10 mL), and then cooled to 0 °C. Under nitrogen atmosphere, methyl magnesium bromide (3M ether solution, 4 mL, 12 mmol) was added dropwise thereto, and the reaction mixture was stirred at 0 °C for 17 hours. The reaction was quenched with water, and the reaction mixture was extracted with ethyl acetate. The organic phase was successively washed with saturated sodium bicarbonate aqueous solution and saturated sodium chloride aqueous solution, dried over anhydrous sodium sulfate, and filtered. The filtrate was concentrated under reduced pressure to obtain the title compound (237 mg, 75%).

MS *m*/*z* (ESI): 159.2 [M+H]⁺.

### Step 2: preparation of 3-((3-exo)-3-((7-((5-(2-hydroxypropan-2-yl)thiazol-2-yl)amino)-1,6-diazanaphthalen-5-yl)amino)-8-azabicyclo[3.2.1]oct-8-yl)propionitrile

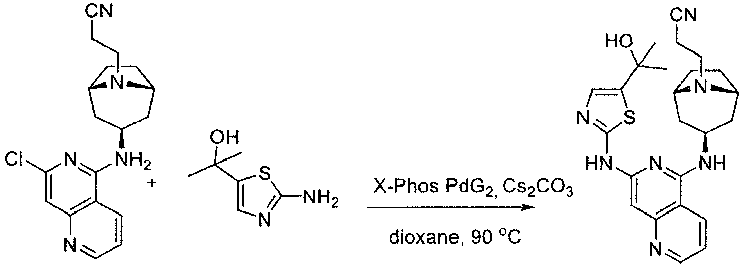

3-((3-*exo*)-3-((7-Chloro-1,6-diazanaphthalen-5-yl)amino)-8-azabicyclo[3.2.1]oct-8-yl)propionitrile (50 mg, 0.146 mmol), 2-(2-aminothiazol-5-yl)propan-2-ol (46 mg, 0.292 mmol), chloro(2-dicyclohexylphosphino-2',4',6'-triisopropyl-1,1'-biphenyl)[2-(2'-amino-1,1'-biphenyl)]palladium(II) (57 mg, 0.073 mmol) and cesium carbonate (143 mg, 0.438 mmol) were added to ultra-dry 1,4-dioxane (5 mL). The reaction mixture was filled with dry nitrogen, heated to 90 °C and stirred for 4 hours. Then the mixture was cooled down to room temperature, and filtered. The filtrate was concentrated under reduced pressure, and purified by prep-HPLC to obtain the title compound (29.8 mg, 44 %).

¹H NMR (400 MHz, DMSO-*d*₆) *δ* 10.71 (s, 1H), 8.67 (dd, *J* = 4.2, 1.2 Hz, 1H), 8.56 (d, *J* = 8.3 Hz, 1H), 7.40 (d, *J* = 8.5 Hz, 1H), 7.13-7.07 (m, 2H), 6.49 (s, 1H), 5.25 (s, 1H), 4.83 (dd, *J* = 17.2, 8.5 Hz, 1H), 3.35 (s, 2H), 2.65 (s, 4H), 1.93 (dt, *J* = 12.9, 9.8 Hz, 4H), 1.80 (dd, *J* = 8.5, 1.5 Hz, 4H), 1.52 (s, 6H).

### MS m/z (ESI): 464.2 [M+H]⁺.

### Embodiment 32

### 3-((3-exo)-3-((7-((5-(1-hydroxylcyclopropyl)thiazol-2-yl)amino)-1,6-diazanaphthalen-5-yl)amino)-8-azabicyclo[3.2.1]oct-8-eyl)propionitrile

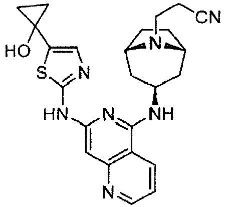

The preparation of 3-((3-*exo*)-3-((7-((5-(1-hydroxylcyclopropyl)thiazol-2-yl)amino)-1,6-diazanaphthalen-5-yl)amino)-8-azabicyclo[3.2.1]oct-8-yl)propionitrile was carried out with the reference to Embodiment 31.

MS *m*/*z* (ESI): 462.2 [M+H]⁺.

### Embodiment 33

### 3-((3-exo)-3-((7-((5-(hydroxymethyl)-4-methylthioazole-2-yl)amino)-1,6-diazanaphthalen-5-yl)amino)-8-azabicyclo[3.2.1]oct-8-yl)propionitrile

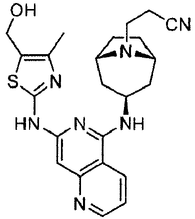

The preparation of 3-((3-exo)-3-((7-((5-(hydroxymethyl)-4-methylthioazole-2-yl)amino)-1,6-diazanaphthalen-5-yl)amino)-8-azabicyclo[3.2.1]oct-8-yl)propionitrile was carried out with the reference to Embodiment 31.

MS *m*/*z* (ESI): 450.2 [M+H]⁺.

### Embodiment 34

### 2-((5-(((3-exo)-8-(2-cyanoethyl)-8-azabicyclo[3.2.1]oct-3-yl)amino)-1,6-diazanaphthalen-7-yl)amino)thiazol-5-sulfonamide

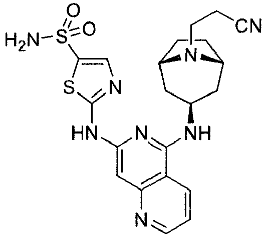

The preparation of 2-((5-(((3-*exo*)-8-(2-cyanoethyl)-8-azabicyclo[3.2.1]oct-3-yl)amino)-1,6-diazanaphthalen-7-yl)amino)thiazol-5-sulfonamide was carried out with the reference to Embodiment 2.

MS *m*/*z* (ESI): 485.1 [M+H]⁺.

### Embodiment 35

### 2-((5-(((3-exo)-8-(2-cyanoethyl)-8-azabicyclo[3.2.1]oct-3-yl)amino)-1,6-diazanaphthalen-7-yl)amino)thiazol-5-formamide

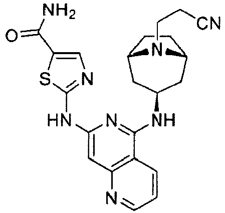

### Step 1: preparation of Tert-butyl (3-exo)-3-((7-((5-carbamidethiazol-2-yl)amino)-1,6-diazanaphthalen-5-yl)amino)-8-azabicyclo[3.2.1]oct-8-carboxylate

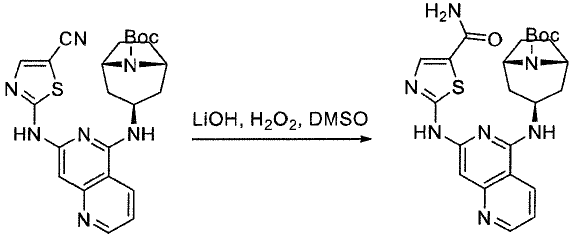

Tert-butyl (3-*exo*)-3-((7-((5-cyanothiazol-2-yl)amino)-1,6-diazanaphthalen-5-yl)amino)-8-azabicyclo[3.2.1]oct-8-carboxylate (72 mg, 0.15 mmol) was dissolved in DMSO (5 mL). The mixture was stirred at room temperature, followed by successive addition of lithium hydroxide monohydrate (19 mg, 0.45 mmol) and 30% hydrogen peroxide aqueous solution (0.18 mL, 0.45 mmol). The mixture was stirred at room temperature for 21 hour, and then diluted with ethyl acetate. The organic phase was washed with saturated sodium chloride aqueous solution, dried over anhydrous sodium sulfate, and filtered. The crude product was used directly in the next step.

MS *m*/*z* (ESI): 496.1 [M+H]⁺.

### Step 2: preparation of 2-((5-(((3-exo)-8-(2-cyanoethyl)-8-azabicyclo[3.2.1]oct-3-yl)amino)-1,6-diazanaphthalen-7-yl)amino)thiazol-5-formamide

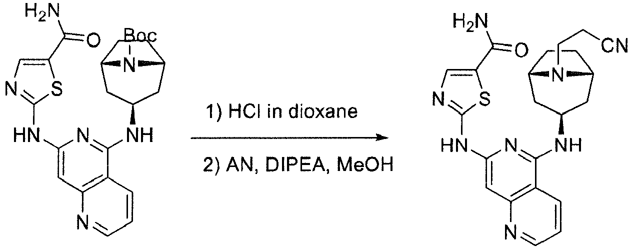

Tert-butyl (3-exo)-3-((7-((5-carbamidethiazol-2-yl)amino)-1,6-diazanaphthalen-5-yl)amino)-8-azabicyclo[3.2.1]oct-8-carboxylate was dissolved in 4M HCl in 1.4-dioxane (10 mL), and the mixture was stirred at room temperature for 15 minutes. Then the mixture was concentrated under reduced pressure, and the residue was dissolved in anhydrous methanol (10 mL), followed by successive addition of DIPEA (0.74 mL, 4.5 mmol) and acrylonitrile (0.2 mL, 3.0 mmol). The resulting reaction mixture was stirred at room temperature for 70minutes, and concentrated under reduced pressure to remove the solvent. The residue was purified by prep-HPLC to obtain the title compound (6.1 mg, 9 %).

¹H NMR (400 MHz, DMSO-*d₆*) *δ* 11.23 (s, 1H), 8.71 (dd, *J* = 4.2, 1.3 Hz, 1H), 8.61 (d, *J* = 7.9 Hz, 1H), 7.95 (s, 1H), 7.70 (s, 1H), 7.49 (d, *J* = 8.6 Hz, 1H), 7.16 (dd, *J* = 8.4, 4.3 Hz, 1H), 7.10 (s, 1H), 6.55 (s, 1H), 4.82 (dd, *J* = 17.6, 9.3 Hz, 1H), 3.32-3.27 (m, 2H), 2.65 (t, *J* = 3.1 Hz, 4H), 2.00 (dd, *J* = 14.0, 6.3 Hz, 2H), 1.91-1.74 (m, 6H).

### Embodiment 36

### 6-((5-(((3-exo)-8-(2-cyanoethyl)-8-azabicyclo[3.2.1]oct-3-yl)amino)-1,6-diazanaphthalen-7-yl)amino)pyridazin-3-formamide

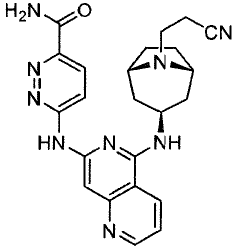

The preparation of 6-((5-(((3-exo)-8-(2-cyanoethyl)-8-azabicyclo[3.2.1]oct-3-yl)amino)-1,6-diazanaphthalen-7-yl)amino)pyridazin-3-formamide was carried out with the reference to Embodiment 2.

### MS m/z (ESI): 444.2 [M+H]⁺.

### Embodiment 37

### 6-((5-(((3-exo)-8-(2-cyanoethyl)-8-azabicyclo[3.2.1]oct-3-yl)amino)-1,6-diazanaphthalen-7-yl)amino)-N-methylpyridazin-3-formamide

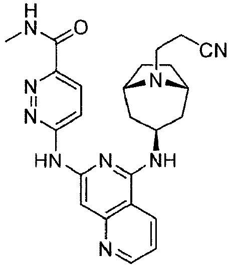

The preparation of 6-((5-(((3-exo)-8-(2-cyanoethyl)-8-azabicyclo[3.2.1]oct-3-yl)amino)-1,6-diazanaphthalen-7-yl)amino)-N-methylpyridazin-3-formamide was carried out with the reference to Embodiment 2.

MS *m*/*z* (ESI): 458.2 [M+H]⁺.

### Embodiment 38

### 5-((5-(((3-exo)-8-(2-cyanoethyl)-8-azabicyclo[3.2.1]oct-3-yl)amino)-1,6-diazanaphthalen-7-yl)amino)pyrazin-2-formamide

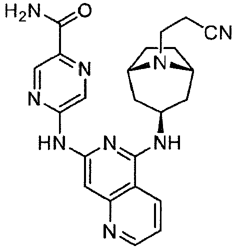

The preparation of 5-((5-(((3-*exo*)-8-(2-cyanoethyl)-8-azabicyclo[3.2.1]oct-3-yl)amino)-1,6-diazanaphthalen-7-yl)amino)pyrazin-2-formamide was carried out with the reference to Embodiment 2.

MS *m*/*z* (ESI): 444.2 [M+H]⁺.

### Embodiment 39

### 3-((3-exo)-3-((2-((5-methyl-1H-pyrazol-3-yl)amino)-7H-pyrrolo[2,3-d]pyrimidin-4-yl)amino)-8-azabicyclo[3.2.1]oct-8-yl)propionitrile

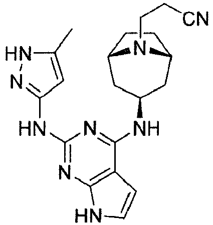

### Step 1: preparation of tert-butyl-(3-exo)-3-((2-chloro-7-tosyl-7H-pyrrolo[2,3-d]pyrimidin-4-yl)amino)-8-azabicyclo[3.2.1]oct-8-carboxylate

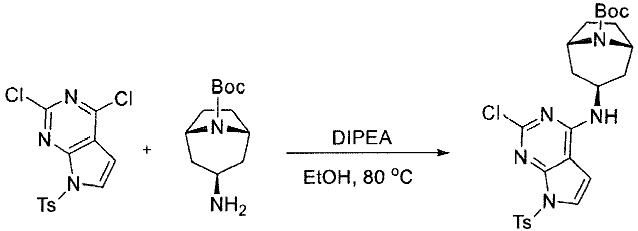

Tert-butyl-(3-*exo*)-3-amino-8-azabicyclo[3.2.1]oct-8-carboxylate (136 mg, 0.6 mmol), DIPEA (129 mg, 1 mmol) were added successively into the solution of 2,4-dichloro-7-tosyl-7H-pyrrolo[2,3-d]pyrimidine (171 mg, 0.5 mmol) in ethanol (10 mL), and stirred at 80°C for 1 hour under reflux condition. When the reaction was completed, the mixture was extracted with dichloromethane (15 mL x 3), and washed with saturated sodium chloride aqueous solution (15 mL x 3). The organic phase was collected, dried over anhydrous sodium sulfate, filtered and concentrated under reduced pressure. The resulting product was purified by silica gel column chromatography (dichloromethane 100%) to obtain the title compound as a white solid (250 mg, 94%).

MS *m*/*z* (ESI): 532.1 [M+H]⁺.

### Step 2: preparation of tert-butyl-(3-exo)-3-((2-((5-methyl-1H-pyrazol-3-yl)amino)-7H-pyrrolo[2,3-d]pyrimidin-4-yl)amino)-8-azabicyclo[3.2.1]oct-8-carboxylate

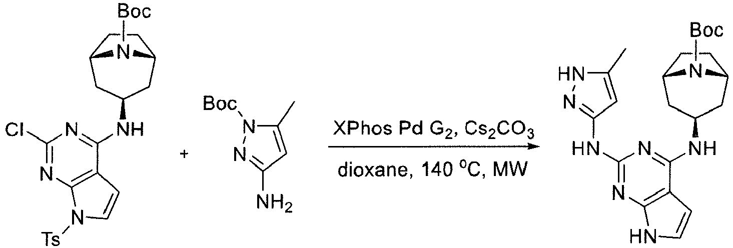

Tert-butyl-(3-*exo*)-3-((2-chloro-7-tosyl-7H-pyrrolo[2,3-d]pyrimidin-4-yl)amino)-8-azabicyclo[3.2.1]oct-8-carboxylate (20 mg, 0.47 mmol), tert-butyl-3amino-5-methyl-1H-pyrazol-1-carboxylate (111 mg, 0.56 mmol), chloro(2-dicyclohexylphosphino-2',4',6'-triisopropyl-1,1'-biphenyl)[2-(2'-amino-1,1'-biphenyl)]palladium(II) (37 mg, 0.047 mmol) and cesium carbonate(306 mg, 0.94 mmol) were suspended in ultra-dry 1,4-dioxane (10 mL). The mixture was filled with nitrogen for 3 times, heated to 140°C with microwave and stirred for 4 hours. When the reaction was completed, the mixture was extracted with dichloromethane (15 mL x 3), and washed with saturated sodium chloride aqueous solution (15 mL x 3). The organic phase was collected, dried over anhydrous sodium sulfate, then filtered and concentrated under reduced pressure. The resulting product was purified by silica gel column chromatography (dichloromethane: methanol=95:5) to obtain the title compound as a yellow solid (100 mg, 48%).

MS *m*/*z* (ESI): 439.2 [M+H]⁺.

### Step 3: preparation of 3-((3-exo)-3-((2-((5-methyl-1H-pyrazol-3-yl)amino)-7H-pyrrolo[2,3-d]pyrimidin-4-yl)amino)-8-azabicyclo[3.2.1]oct-8-yl)propionitrile

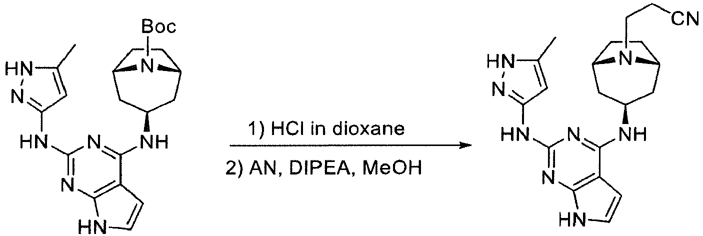

Tert-butyl-(3-*exo*)-3-((2-((5-methyl-1H-pyrazol-3-yl)amino)-7H-pyrrolo[2,3-d]pyrimidin-4-yl)amino)-8-azabicyclo[3.2.1]oct-8-carboxylate (100 mg, 0.23 mmol) was dissolved in hydrochloride 1,4-dioxane solution (4.0 N, 5 mL). The mixture was stirred at room temperature for 30 minutes, concentrated, and then dissolved in methanol (10 mL). DIPEA (118 mg, 0.91 mmol) was slowly added dropwise, and the mixture was stirred at room temperature for 10 minutes. Acrylonitrile (18 mg, 0.35 mmol) was added thereto and the mixture was stirred for another 2 hours. The reaction mixture was concentrated under reduced pressure, and the resulting product was purified by prep-HPLC to obtain the title compound as a yellow solid (15.4 mg, 17 %).

¹H NMR (400 MHz, DMSO) *δ* 11.58 (s, 1H), 10.95 (s, 1H), 8.70 (s, 1H), 7.04 (s, 1H), 6.72 (s, 1H), 6.39 (s, 1H), 6.32-6.17 (m, 1H), 4.46 (s, 1H), 3.30 (s, 2H), 2.62 (dd, *J* = 7.2, 4.0 Hz, 4H), 2.17 (s, 3H), 1.97-1.89 (m, 2H), 1.84-1.59 (m, 6H).

MS *m*/*z* (ESI): 392.2 [M+H]⁺.

### Embodiment 40

### 3-((3-exo)-3-((4-((5-methyl-1H-pyrazol-3-yl)amino)-5H-pyrrolo[3,2-d]pyrimidin-2-yl)amino)-8-azabicyclo[3.2.1]oct-8-yl)propionitrile

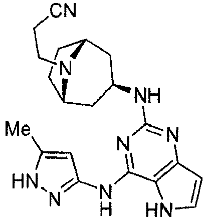

### Step 1: preparation of 2,4-dichloro-5-((2-nitrophenyl)sulfonyl)-5H-pyrrolo[3,2-d]pyrimidine

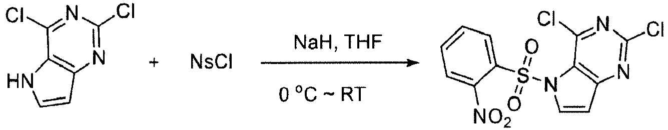

Under ice water bath condition, sodium hydride (120 mg, 5 mmol) was slowly added dropwise in the solution of 2,4-dichloro-5 H-pyrrole[3,2-D]pyrimidine(470 mg, 2.5 mmol) and o-nitrobenzene sulfonyl chloride (600 mg, 2.75 mmol) in tetrahydrofuran (20 mL). The mixture was slowly warmed to room temperature and stirred for 1 hour. When the reaction was completed, under ice water bath condition, the reaction was quenched by slowly adding water (150 mL) dropwise into the reaction system. The mixture was stirred at room temperature for 1 hour and then filtered to obtain the title compound as a pale yellow solid (800 mg, 86%).

MS *m*/*z* (ESI): 372.9 [M+H]⁺.

### Step 2: preparation of 2-chloro-N-(5-methyl-1H-pyrazol-3-yl)-5-((2-nitrophenyl)sulfonyl)-5H-pyrrolo[3,2-d]pyrimidin-4-amine

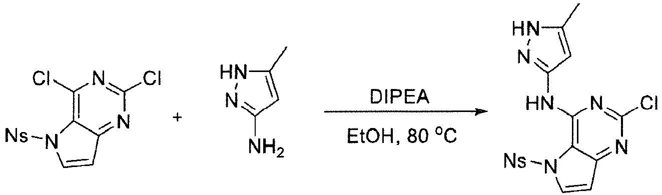

3-Amino-5-methylpyrazol (250 mg, 2.58 mmol), DIPEA (555 mg, 4.3 mmol) were successively added into the solution of 2,4-dichloro-5-((2-nitrophenyl)sulfonyl)-5H-pyrrolo[3,2-d]pyrimidine (800 mg, 2.15 mmol) in ethanol (20 mL). The mixture was then stirred at 80°C for 2 hours under reflux condition. When the reaction was completed, solid was precipitated from the reaction mixture, and the mixture was filtered. The filter cake was washed with ethanol (15 mL x 3), and then dried to obtain the title compound as a yellow solid (370 mg, 40%).

MS *m*/*z* (ESI): 434.0 [M+H]⁺.

### Step 3: preparation of tert-butyl-(3-exo)-3-((4-((5-methyl-1H-pyrazol-3-yl)amino)-5H-pyrrolo[3,2-d]pyrimidin-2-yl)amino)-8-azabicyclo[3.2.1]oct-8-carboxylate

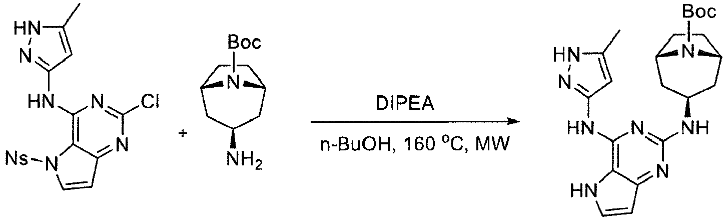

Tert-butyl-(3-*exo*)-3-amino-8-azabicyclo[3.2.1]oct-8-carboxylate (233 mg, 1.03 mmol) and DIPEA (220 mg, 1.71 mmol) were successively added into the solution of 2-chloro-N-(5-methyl-1H-pyrazol-3-yl)-5-((2-nitrophenyl)sulfonyl)-5H-pyrrolo[3,2-d]pyrimidin-4-amine(370 mg, 0.85 mmol) in n-butanol (5 mL), then the mixture was heated to 160°C under microwave condition and stirred for 10 hours. When the reaction was completed, the reaction mixture was extracted with ethyl acetate (15 mL x 3), and washed with saturated sodium chloride aqueous solution (15 mL x 3). The organic phase was collected and dried over anhydrous sodium sulfate, filtered and concentrated under reduced pressure. The resulting product was purified by silica gel column chromatography (dichloromethane: methanol=98:2) to obtain the title compound as a pale yellow solid (40 mg, 11%).

MS *m*/*z* (ESI): 593.1 [M+H]⁺.

### Step 4: 3-((3-exo)-3-((4-((5-methyl-1H-pyrazol-3-yl)amino)-5H-pyrrolo[3,2-d]pyrimidin-2-yl)amino)-8-azabicyclo[3.2.1]oct-8-yl)propionitrile

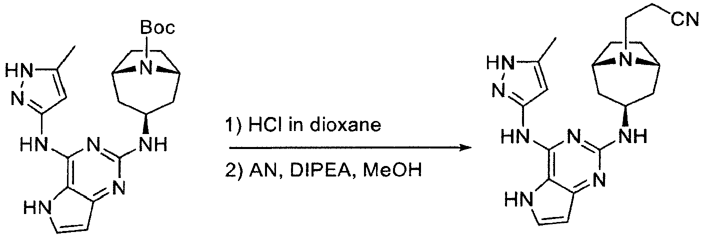

Tert-butyl-(3-*exo*)-3-((4-((5-methyl-1H-pyrazol-3-yl)amino)-5H-pyrrolo[3,2-d]pyrimidin-2-yl)amino)-8-azabicyclo[3.2.1]oct-8-carboxylate (30 mg, 0.07 mmol) was dissolved in hydrochloride 1,4-dioxane solution (4.0 N, 2 mL). The mixture was stirred at room temperature for 30 minutes, concentrated, and dissolved in methanol (5 mL). DIPEA (35 mg, 0.28 mmol) was slowly added dropwise, and the mixture was stirred at room temperature for 10 minutes. Acrylonitrile (5 mg, 0.1 mmol) was added thereto and the mixture was stirred for another 2 hours. The reaction mixture was concentrated under reduced pressure, and the resulting product was purified by prep-HPLC to obtain the title compound as a pale yellow solid (8.2 mg, 31 %).

¹H NMR (400 MHz, DMSO) *δ* 11.93 (s, 1H), 10.57 (s, 1H), 9.43 (s, 1H), 7.29 (s, 1H), 6.77 (s, 1H), 6.02 (s, 1H), 5.77 (s, 1H), 4.12 (s, 1H), 3.28 (s, 2H), 2.62 (s, 4H), 2.22 (s, 3H), 1.90 (s, 2H), 1.81-1.43 (m, 6H).

MS *m*/*z* (ESI): 392.2 [M+H]⁺.

### Embodiment 41

### 3-((3-exo)-3-((5-methyl-4-((5-methyl-1H-pyrazol-3-yl)amino)-7H-pyrrolo[2,3-d]pyrimidin-2-yl)amino)-8-azabicyclo[3.2.1]oct-8-yl)propionitrile

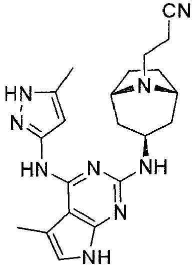

The preparation of 3-((3-*exo*)-3-((5-methyl-4-((5-methyl-1H-pyrazol-3-yl)amino)-7H-pyrrolo[2,3-d]pyrimidin-2-yl)amino)-8-azabicyclo[3.2.1]oct-8-yl)propionitrile was carried out with the reference to Embodiment 3.

MS *m*/*z* (ESI): 406.2 [M+H]⁺.

### Embodiment 42

### 3-((3-exo)-3-((5-methyl-2-((5-methyl-1H-pyrazol-3-yl)amino)-7H-pyrrolo[2,3-d]pyrimidin-4-yl)amino)-8-azabicyclo[3.2.1]oct-8-yl)propionitrile

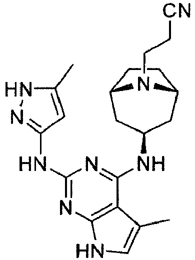

The preparation of 3-((3-*exo*)-3-((5-methyl-2-((5-methyl-1H-pyrazol-3-yl)amino)-7H-pyrrolo[2,3-d]pyrimidin-4-yl)amino)-8-azabicyclo[3.2.1]oct-8-yl)propionitrile was carried out with the reference to Embodiment 39.

MS *m*/*z* (ESI): 406.2 [M+H]⁺.

### Embodiment 43

### 3-((3-exo)-3-((6-methyl-4-((5-methyl-1H-pyrazol-3-yl)amino)-7H-pyrrolo[2,3-d]pyrimidin-2-yl)amino)-8-azabicyclo[3.2.1]oct-8-yl)propionitrile

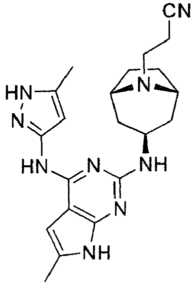

The preparation of 3-((3-*exo*)-3-((6-methyl-4-((5-methyl-1H-pyrazol-3-yl)amino)-7H-pyrrolo[2,3-d]pyrimidin-2-yl)amino)-8-azabicyclo[3.2.1]oct-8-yl)propionitrile was carried out with the reference to Embodiment 3.

MS *m*/*z* (ESI): 406.2 [M+H]⁺.

### Embodiment 44

### 3-((3-exo)-3-((6-methyl-2-((5-methyl-lH-pyrazol-3-yl)amino)-7H-pyrrolo[2,3-d]pyrimidin-4-yl)amino)-8-azabicyclo[3.2.1]oct-8-yl)propionitrile

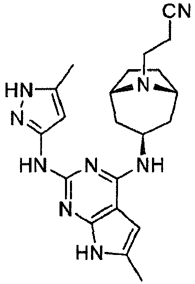

The preparation of 3-((3-*exo*)-3-((6-methyl-2-((5-methyl-1H-pyrazol-3-yl)amino)-7H-pyrrolo[2,3-d]pyrimidin-4-yl)amino)-8-azabicyclo[3.2.1]oct-8-yl)propionitrile was carried out with the reference to Embodiment 39.

MS *m*/*z* (ESI): 406.2 [M+H]⁺.

### Embodiment 45

### 2-(((3-exo)-8-(2-cyanoethyl)-8-azabicyclo[3.2.1]oct-3-yl)amino)-4-((5-methyl-1H-pyrazol-3-yl)amino)-7H-pyrrolo[2,3-d]pyrimidin-5-formonitrile

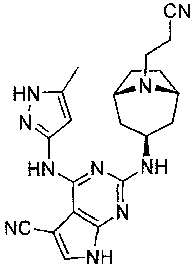

The preparation of 2-(((3-*exo*)-8-(2-cyanoethyl)-8-azabicyclo[3.2.1]oct-3-yl)amino)-4-((5-methyl-1H-pyrazol-3-yl)amino)-7H-pyrrolo[2,3-d]pyrimidin-5-formonitrile was carried out with the reference to Embodiment 3.

MS *m*/*z* (ESI): 417.2 [M+H]⁺.

### Embodiment 46

### 3-((3-exo)-3-((5-fluoro-4-((5-methyl-1H-pyrazol-3-yl)amino)-7H-pyrrolo[2,3-d]pyrimidin-2-yl)amino)-8-azabicyclo[3.2.1]oct-8-yl)propionitrile

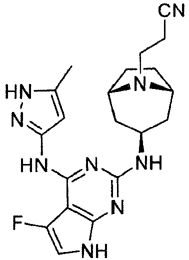

The preparation of 3-((3-*exo*)-3-((5-fluoro-4-((5-methyl-1H-pyrazol-3-yl)amino)-7H-pyrrolo[2,3-d]pyrimidin-2-yl)amino)-8-azabicyclo[3.2.1]oct-8-yl)propionitrile was carried out with the reference to Embodiment 3.

MS *m*/*z* (ESI): 410.2 [M+H]⁺.

### Embodiment 47

### 3-((3-exo)-3-((6-((5-methyl-1H-pyrazol-3-yl)amino)-9H-purin-2-yl)amino)-8-azabicyclo[3.2.1]oct-8-yl)propionitrile

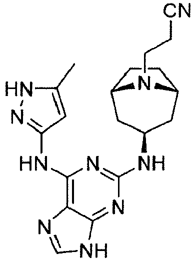

The preparation of 3-((3-*exo*)-3-((6-((5-methyl-1H-pyrazol-3-yl)amino)-9H-purin-2-yl)amino)-8-azabicyclo[3.2.1]oct-8-yl)propionitrile was carried out with the reference to Embodiment 3.

MS *m*/*z* (ESI): 393.2 [M+H]⁺.

### Embodiment 48

### 3-((3-exo)-3-((2-((5-methy]-1H-pyrazol-3-yl)amino)-9H-purin-6-yl)amino)-8-azabicyclo [3.2.1]oct-8-yl)propionitrile

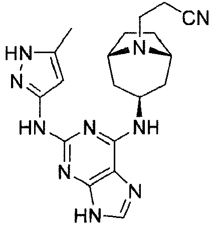

The preparation of 3-((3-*exo*)-3-((2-((5-methyl-1H-pyrazol-3-yl)amino)-9H-purin-6-yl)amino)-8-azabicyclo[3.2.1]oct-8-yl)propionitrile was carried out with the reference to Embodiment 39.

MS *m*/*z* (ESI): 393.2 [M+H]⁺.

### Embodiment 49

### 3-((3-exo)-3-((8-methy|-2-((5-methyl-1H-pyrazol-3-yl)amino)-9H-purin-6-yl)amino)-8-azabicyclo[3.2.1]oct-8-yl)propionitrile

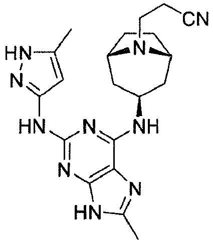

The preparation of 3-((3-*exo*)-3-((8-methyl-2-((5-methyl-1H-pyrazol-3-yl)amino)-9H-purin-6-yl)amino)-8-azabicyclo[3.2.1]oct-8-yl)propionitrile was carried out with the reference to Embodiment 39.

MS *m*/*z* (ESI): 407.2 [M+H]⁺.

### Embodiment 50

### 3-((3-exo)-3-((3-methyl-6-((5-methyl-1H-pyrazol-3-yl)amino)-1H-pyrazolo[3,4-d]pyrimidin-4-yl)amino)-8-azabicyclo[3.2.1]oct-8-yl)propionitrile

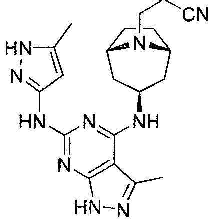

The preparation of 3-((3-*exo*)-3-((3-methyl-6-((5-methyl-1H-pyrazol-3-yl)amino)-1H-pyrazolo[3,4-d]pyrimidin-4-yl)amino)-8-azabicyclo[3.2.1]oct-8-yl)propionitrile was carried out with the reference to Embodiment 39.

MS *m*/*z* (ESI): 407.2 [M+H]⁺.

### Embodiment 51

### 3-((3-exo)-3-((4-((5-methyl-1H-pyrazol-3-yl)amino)-1H-pyrazolo[3,4-d]pyrimidin-6-yl)amino)-8-azabicyclo[3.2.1]oct-8-yl)propionitrile

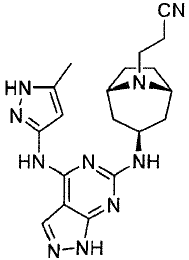

### Step 1: preparation of 4,6-dichloro-1-(4H-2H-pyran-2-yl)-1H-pyrazolo[3,4-d]pyrimidine

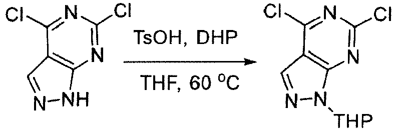

P-toluenesulfonic acid monohydrate(19 mg, 0.1 mmol), 3,4-2H-2H-pyran (133 mg, 1.59 mmol) were successively added to the solution of 4,6-dichloro-1H-pyrazolo[3,4-d]pyrimidine(200 mg, 1.06 mmol) in tetrahydrofuran (5 mL). The mixture was then stirred at 60°C for 2 hours under reflux condition. When the reaction was completed, the mixture was extracted with dichloromethane (15 mL x 3) and washed with saturated sodium chloride aqueous solution (15 mL x 3). The organic phase was collected and dried over anhydrous sodium sulfate, filtered, and concentrated under reduced pressure to remove the organic solvent. The resulting product was purified by silica gel column chromatography (dichloromethane 100%) to obtain the title compound as a white solid (269 mg, 93%).

MS m/z (ESI): 273.0 [M+H]⁺.

### Step 2: preparation of 6-chloro-N-(5-methyl-1H-pyrazol-3-yl)-1-(4H-2H-pyran-2-yl)-1H-pyrazolo[3 ,4-d]pyrimidin-4-amine

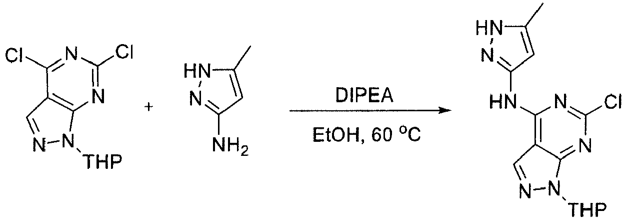

3-Amino-5-methylpyrazol (108 mg, 1.12 mmol) and DIPEA (240 mg, 1.86 mmol) were successively added to the solution of 4,6-dichloro-1-(4H-2H-pyran-2-yl)-1H-pyrazolo[3,4-d]pyrimidine(250 mg, 0.93 mmol) in ethanol (10 mL). The mixture was then stirred at 60°C for 1hour. When the reaction was completed, the reaction mixture was extracted with ethyl acetate (15 mL x 3), and washed with saturated sodium chloride aqueous solution (15 mL x 3). The organic phase was collected, dried over anhydrous sodium sulfate, filtered, and concentrated under reduced pressure. The resulting product was purified by silica gel column chromatography (dichloromethane: methanol=98:2) to obtain the title compound as a white solid (254 mg, 82%).

MS *m*/*z* (ESI): 334.1 [M+H]⁺.

### Step 3: preparation of tert-butyl-(3-exo)-3-((4-((5-methyl-1H-pyrazol-3-yl)amino)-1-(4H-2H-pyran-2-yl)-1H-pyrazolo[3,4-d]pyrimidin-6-yl)amino)-8-azabicyclo[3.2.1]oct-8-carboxylate

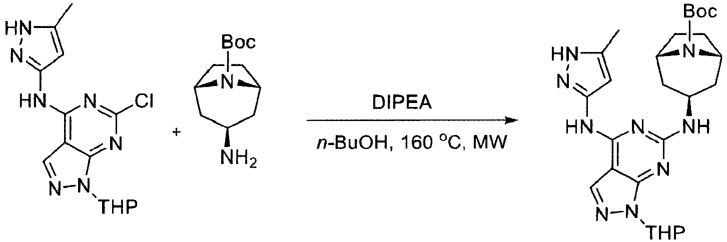

Tert-butyl-(3-*exo*)-3-amino-8-azabicyclo[3.2.1]oct-8-carboxylate (203 mg, 0.9 mmol) and DIPEA (155 mg, 1.2 mmol) were successively added to the solution of 6-chloro-N-(5-methyl-1H-pyrazol-3-yl)-1-(4H-2H-pyran-2-yl)-1H-pyrazolo [3,4-d]pyrimidin-4-amine (200 mg, 0.6 mmol) in n-butanol (5 mL). The mixture was stirred at 160°C for 15 hours under microwave condition. When the reaction was completed, the reaction mixture was extracted with ethyl acetate (15 mL x 3), and washed with saturated sodium chloride aqueous solution (15 mL x 3). The organic phase was collected and dried over anhydrous sodium sulfate, filtered and concentrated under reduced pressure. The resulting product was purified by silica gel column chromatography (dichloromethane: methanol=95:5) to obtain the title compound as a pale yellow solid (144 mg, 46%).

MS *m*/*z* (ESI): 524.2 [M+H]⁺.

### Step 4: preparation of 3-((3-exo)-3-((4-((5-metbyl-1H-pyrazol-3-yl)amino)-1H-pyrazolo[3,4-d]pyrimidin-6-yl)amino)-8-azabicyclo[3.2.1]oct-8-yl)propionitrile

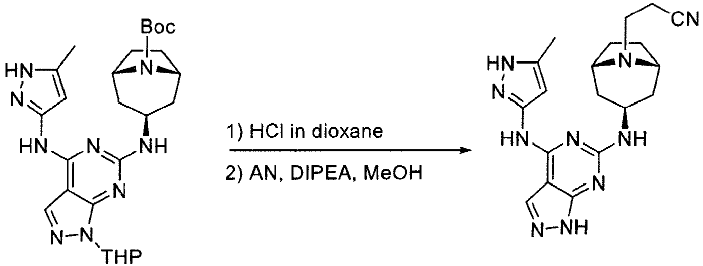

Tert-butyl-(3-*exo*)-3-((4-((5-methyl-1H-pyrazol-3-yl)amino)-1-(4H-2H-pyran-2-yl)-1H-pyrazolo[3,4-d]pyrimidin-6-yl)amino)-8-azabicyclo[3.2.1]oct-8-carboxylate (144 mg, 0.28 mmol) was dissolved in hydrochloride 1,4-dioxane solution (4.0 N, 5 mL). The mixture was stirred at room temperature for 30 minutes, concentrated, and dissolved in methanol (10 mL). DIPEA (142 mg, 1.12 mmol) was slowly added dropwise, and the mixture was stirred at room temperature for 10 minutes. Acrylonitrile (22 mg, 0.41 mmol) was added thereto and the mixture was stirred for another 2 hours. When the reaction was completed, the reaction mixture was concentrated under reduced pressure, and purified by prep-HPLC to obtain the title compound as a white solid (54.6 mg, 51%).

¹H NMR (400 MHz, DMSO-*d*₆) *δ* = 12.49 (s, 1H), 12.03 (s, 1H), 10.02 (s, 1H), 8.05 (s, 1H), 6.81-6.47 (m, 2H), 4.11 (s, 1H), 3.29 (s, 2H), 2.62 (s, 4H), 2.19 (s, 3H), 1.91 (s, 2H), 1.71-1.62 (m, 6H).

MS *m*/*z* (ESI): 393.2 [M+H]⁺.

### Embodiment 52

### 3-((3-exo)-3-((6-((5-methyl-1H-pyrazol-3-yl)amino)-1H-pyrazolo[3,4-d]pyrimidin-4-yl)amino)-8-azabicyclo[3.2.1]oct-8-yl)propionitrile

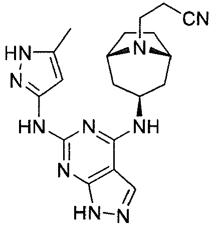

The preparation of 3-((3-*exo*)-3-((6-((5-methyl-1H-pyrazol-3-yl)amino)-1H-pyrazolo[3,4-d]pyrimidin-4-yl)amino)-8-azabicyclo[3.2.1]oct-8-yl)propionitrile was carried out with the reference to Embodiment 39.

MS *m*/*z* (ESI): 393.2 [M+H]⁺.

### Embodiment 53

### 3-((3-exo)-3-((4-((5-methyl-1H-pyrazol-3-yl)amino)-1H-pyrrolo[3,2-c]pyridin-6-yl)amino)-8-azabicyclo[3.2.1]oct-8-yl)propionitrile

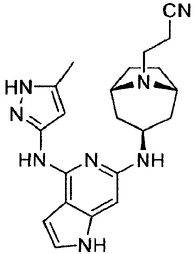

The preparation of 3-((3-*exo*)-3-((4-((5-methyl-1H-pyrazol-3-yl)amino)-1H-pyrrolo[3,2-c]pyridin-6-yl)amino)-8-azabicyclo[3.2.1]oct-8-yl)propionitrile was carried out with the reference to Embodiment 3.

MS *m*/*z* (ESI): 391.2 [M+H]⁺.

### Embodiment 54

### 3-((3-exo)-3-((6-((5-methyl-1H-pyrazol-3-yl)amino)-1H-pyrrolo[3,2-c]pyridin-4-yl)amino)-8-azabicyclo[3.2.1]oct-8-yl)propionitrile

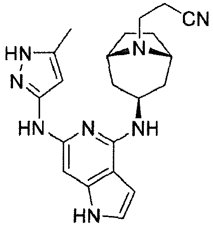

The preparation of 3-((3-*exo*)-3-((6-((5-methyl-1H-pyrazol-3-yl)amino)-1H-pyrrolo[3,2-c]pyridin-4-yl)amino)-8-azabicyclo[3.2.1]oct-8-yl)propionitrile was carried out with the reference to Embodiment 39.

MS *m*/*z* (ESI): 391.2 [M+H]⁺.

### Embodiment 55

### 3-((3-exo)-3-((4-((5-methyl-1H-pyrazol-3-yl)amino)-1H-imidazo[4,5-c]pyridin-6-yl)amino)-8-azabicyclo[3 .2.1]oct-8-yl)propionitrile

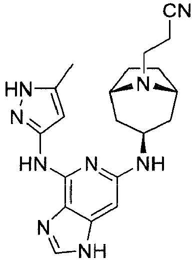

The preparation of 3-((3-*exo*)-3-((4-((5-methyl-1H-pyrazol-3-yl)amino)-1H-imidazo[4,5-c]pyridin-6-yl)amino)-8-azabicyclo[3.2.1]oct-8-yl)propionitrile was carried out with the reference to Embodiment 51.

MS *m*/*z* (ESI): 392.2 [M+H]⁺.

### Embodiment 56

### 3-((3-exo)-3-((6-((5-methyl-1H-pyrazol-3-yl)amino)-1H-imidazo[4,5-c]pyridin-4-yl)amino)-8-azabicyclo[3.2.1]oct-8-yl)propionitrile

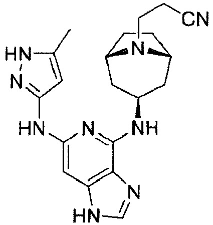

The preparation of 3-((3-*exo*)-3-((6-((5-methyl-1H-pyrazol-3-yl)amino)-1H-imidazo[4,5-c]pyridin-4-yl)amino)-8-azabicyclo[3.2.1]oct-8-yl)propionitrile was carried out with the reference to Embodiment 39.

MS *m*/*z* (ESI): 392.2 [M+H]⁺.

### Embodiment 57

### 3-((3-exo)-3-((7-methyl-6-((5-methyl-1H-pyrazol-3-yl)amino)-7H-purin-2-yl)amino)-8-azabicyclo[3.2.1]oct-8-yl)propionitrile

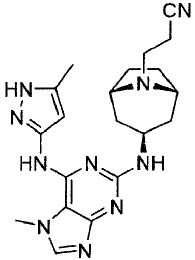

The preparation of 3-((3-exo)-3-((7-methyl-6-((5-methyl-lH-pyrazol-3-yl)amino)-7H-purin-2-yl)amino)-8-azabicyclo[3.2.1]oct-8-yl)propionitrile was carried out with the reference to Embodiment 40.

MS *m*/*z* (ESI): 407.2 [M+H]⁺.

### Embodiment 58

### 3-((3-exo)-3-((7-methyl-2-((5-methyl-1H-pyrazol-3-yl)amino)-7H-purin-6-yl)amino)-8-azabicyclo[3.2.1]oct-8-yl)propionitrile

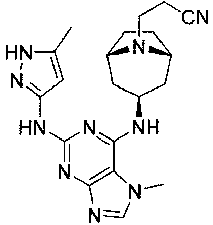

The preparation of 3-((3-*exo*)-3-((7-methyl-2-((5-methyl-1H-pyrazol-3-yl)amino)-7H-purin-6-yl)amino)-8-azabicyclo[3.2.1]oct-8-yl)propionitrile was carried out with the reference to Embodiment 39.

MS *m*/*z* (ESI): 407.2 [M+H]⁺.

### Embodiment 59

### 3-((3-exo)-3-((9-methyl-6-((5-methyl-1H-pyrazol-3-yl)amino)-9H-purin-2-yl)amino)-8-azabicyclo[3.2.1]oct-8-yl)propionitrile

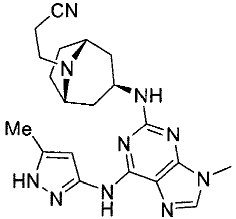

The preparation of 3-((3-*exo*)-3-((9-methyl-6-((5-methyl-1H-pyrazol-3-yl)amino)-9H-purin-2-yl)amino)-8-azabicyclo[3.2.1]oct-8-yl)propionitrile was carried out with the reference to Embodiment 40.

MS m/z (ESI): 407.2 [M+H]⁺.

### Embodiment 60

### 3-((3-exo)-3-((4-((5-methyl-1H-pyrazol-3-yl)amino)thieno[3,2-d]pyrimidin-2-yl)amino)-8-azabicyclo[3.2.1]oct-8-yl)propionitrile

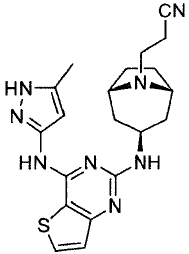

### Step 1: preparation of 2-chloro-N-(5-methyl-1H-pyrazol-3-yl)thieno[3,2-d]pyrimidin-4-amine

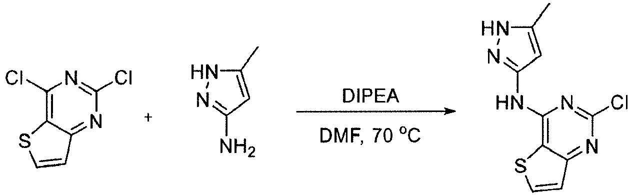

3-Amino-5-methylpyrazol (116 mg, 1.2 mmol) and DIPEA (258 mg, 2 mmol) were successively added to the solution of 2,4-dichlorothieno[3,2-d]pyrimidine (205 mg, 1 mmol) in N-methylpyrrolidone(10 mL). The mixture was stirred at 70°C for 1 hour. When the reaction was completed, water (50 mL) was added to the reaction mixture, the precipitated solid was filtered off and slurried with ethyl acetate to obtain the title compound as a pale yellow solid (135 mg, 51%).

MS *m*/*z* (ESI): 266.0 [M+H]⁺.

### Step 2: preparation of tert-butyl-(3-exo)-3-((4-((5-methyl-1H-pyrazol-3-yl)amino)thieno[3,2-d]pyrimidin-2-yl)amino)-8-azabicyclo[3.2.1]oct-8-carboxylate

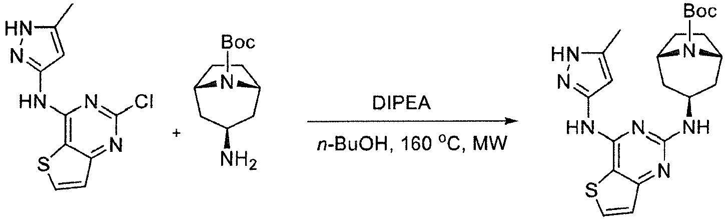

Tert-butyl-(3-*exo*)-3-amino-8-azabicyclo[3.2.1]oct-8-carboxylate (138 mg, 0.61 mmol) and DIPEA (129 mg, 1 mmol) were successively added to 2-chloro-N-(5-methyl-1H-pyrazol-3-yl)thieno[3,2-d]pyrimidin-4-amine(135 mg, 0.51 mmol) in the solution of n-butanol (5 mL). The mixture was stirred at 160°C for 15 hours under microwave condition. When the reaction was completed, the reaction mixture was extracted with ethyl acetate (15 mL x 3), and washed with saturated sodium chloride aqueous solution (15 mL x 3). The organic phase was collected and dried over anhydrous sodium sulfate, filtered, and concentrated under reduced pressure. The resulting product was purified by silica gel column chromatography (dichloromethane: methanol=98:2) to obtain the title compound as a pale yellow solid (146 mg, 63 %).

MS *m*/*z* (ESI): 456.2 [M+H]⁺.

### Step 3: preparation of 3-((3-exo)-3-((4-((5-methyl-lH-pyrazol-3-yl)amino)thieno[3,2-d]pyrimidin-2-yl)amino)-8-azabicyclo[3.2.1]oct-8-yl)propionitrile

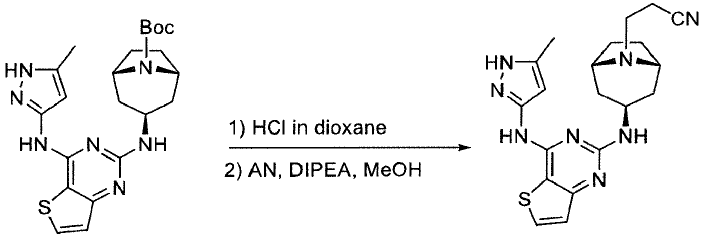

Tert-butyl-(3-*exo*)-3-((4-((5-methyl-1H-pyrazol-3-yl)amino)thieno[3,2-d]pyrimidin-2-yl)amino)-8-azabicyclo[3.2.1]oct-8-carboxylate (146 mg, 0.32 mmol) was dissolved in hydrochloride 1,4-dioxane solution (4.0N, 5 mL). The mixture was stirred at room temperature for 30 minutes, concentrated, and dissolved in methanol (10 mL). DIPEA (166 mg, 1.28 mmol) was slowly added dropwise thereto, and the mixture was stirred at room temperature for 10 minutes. Acrylonitrile (25 mg, 0.48 mmol) was added thereto and the mixture stirred for another 2 hours. The reaction mixture was concentrated under reduced pressure, and the resulting product was purified by prep-HPLC to obtain the title compound as a white solid (14.4 mg, 11 %).

¹H NMR (400 MHz, DMSO) *δ* 12.02 (s, 1H), 9.70 (s, 1H), 7.89 (s, 1H), 6.99 (s, 1H), 6.44 (d, *J* = 59.6 Hz, 2H), 4.14 (s, 1H), 3.29 (s, 2H), 2.62 (s, 4H), 2.22 (s, 3H), 1.89 (s, 2H), 1.64 (dd, *J* = 47.8, 17.6 Hz, 6H).

MS *m*/*z* (ESI): 409.2 [M+H]⁺.

### Embodiment 61

### 3-((3-exo)-3-((7-methyl-4-((5-methyl-1H-pyrazol-3-yl)amino)thieno[3,2-d]pyrimidin-2-yl)amino)-8-azabicyclo[3.2.1]oct-8-yl)propionitrile

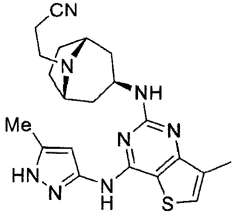

The preparation of 3-((3-*exo*)-3-((7-methyl-4-((5-methyl-1H-pyrazol-3-yl)amino)thieno[3,2-d]pyrimidin-2-yl)amino)-8-azabicyclo[3.2.1]oct-8-yl)propionitrile was carried out with the reference to Embodiment 60.

MS *m*/*z* (ESI): 423.2 [M+H]⁺.

### Embodiment 62

### 3-((3-exo)-3-((4-((5-methyl-1H-pyrazol-3-yl)amino)thieno[2,3-d]pyrimidin-2-yl)amino)-8-azabicyclo[3.2.1]oct-8-yl)propionitrile

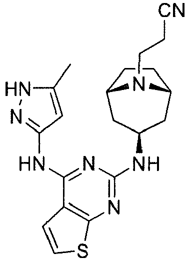

### Step 1: preparation of 2-chloro-N-(5-methyl-1H-pyrazol-3-yl)thieno[2,3-d]pyrimidin-4-amine

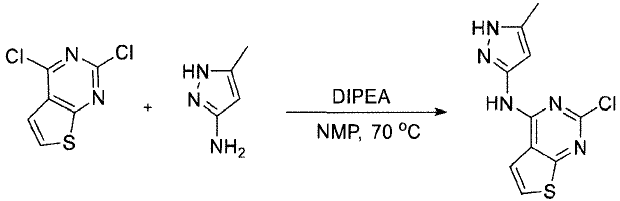

3-Amino-5-methylpyrazol (116 mg, 1.2 mmol) and DIPEA (258 mg, 2 mmol) were successively added to the solution of 2,4-dichlorothieno[2,3-d]pyrimidine (205 mg, 1 mmol) in N-methylpyrrolidone (10 mL). The mixture was stirred at 70°C for 1 hour. When the reaction was completed, water (50 mL) was added to the reaction mixture, and a solid was precipitated, which was then filtered and slurried with ethyl acetate to obtain the title compound as a yellow solid (250 mg, 94%).

### MS m/z (ESI): 266.0 [M+H]⁺.

### Step 2: preparation of tert-butyl-(3-exo)-3-((4-((5-methyl-1H-pyrazol-3-yl)amino)thieno[2,3-d]pyrimidin-2-yl)amino)-8-azabicyclo[3.2. 1]oct-8-carboxylate

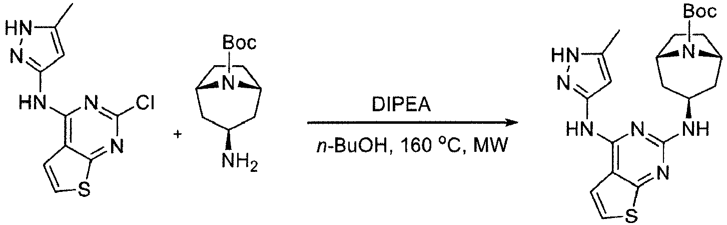

Tert-butyl-(3-*exo*)-3-amino-8-azabicyclo[3.2.1]oct-8-carboxylate (256 mg, 1.13 mmol) and DIPEA (242 mg, 1.88 mmol) were successively added to the solution of 2-chloro-N-(5-methyl-lH-pyrazol-3-yl)thieno[2,3-d]pyrimidin-4-amine(250 mg, 0.94 mmol) in n-butanol (10 mL). The mixture was then stirred at 160°C for 15 hours under microwave condition. When the reaction was completed, the reaction mixture was extracted with ethyl acetate (15 mL x 3), and washed with saturated sodium chloride aqueous solution (15 mL x 3). The organic phase was collected and dried over anhydrous sodium sulfate, filtered and concentrated under reduced pressure. The resulting product was purified by silica gel column chromatography (dichloromethane: methanol=98:2) to obtain the title compound as a pale yellow solid (200 mg, 47%).

MS *m*/*z* (ESI): 456.1 [M+H]⁺.

### Step 3: preparation of 3-((3-exo)-3-((4-((5-methyl-lH-pyrazol-3-yl)amino)thieno[2,3-d]pyrimidin-2-yl)amino)-8-azabicyclo[3.2.1]oct-8-yl)propionitrile

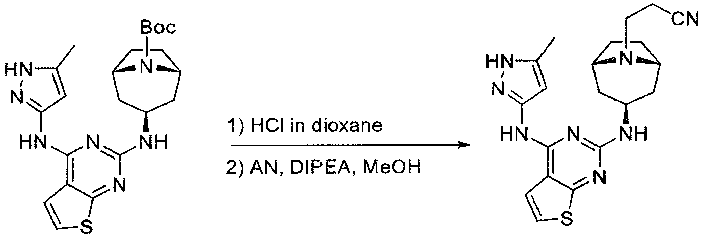

Tert-butyl-(3-*exo*)-3-((4-((5-methyl-1H-pyrazol-3-yl)amino)thieno[2,3-d]pyrimidin-2-yl)amino)-8-azabicyclo[3.2.1]oct-8-carboxylate (200 mg, 0.44 mmol) was dissolved in hydrochloride 1,4-dioxane solution(4.0 N, 5 mL). The mixture was stirred at room temperature for 30 minutes and the mixture was concentrated; then methanol (10 mL) was added to the mixture for dissolution, DIPEA (227 mg, 1.76 mmol) was slowly added dropwise to the mixture, and the mixture was stirred at room temperature for 10 minutes. Acrylonitrile (35 mg, 0.66 mmol) was added thereto and the mixture was stirred for another 2 hours. The reaction mixture was concentrated under reduced pressure, the resulting product was purified by prep-HPLC to obtain the title compound as a white solid (31.6 mg, 18 %).

¹H NMR (400 MHz, DMSO) *δ* 12.13 (s, 1H), 9.93 (s, 1H), 7.73 (s, 1H), 6.88 (d, *J* = 117.2 Hz, 3H), 4.27 (s, 1H), 3.37 (s, 2H), 2.70 (s, 4H), 2.32 (s, 3H), 1.99 (s, 2H), 1.86-1.61 (m, 6H).

MS *m*/*z* (ESI): 409.2 [M+H]⁺.

### Embodiment 63

### 3-((3-exo)-3-((2-((5-methyl-1H-pyrazol-3-yl)amino)thieno[2,3-d]pyrimidin-4-yl)amino)-8-azabicyclo[3.2.1]oct-8-yl)propionitrile

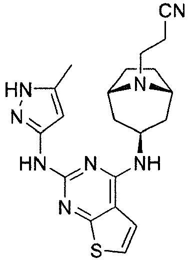

The preparation of 3-((3-*exo*)-3-((2-((5-methyl-1H-pyrazol-3-yl)amino)thieno[2,3-d]pyrimidin-4-yl)amino)-8-azabicyclo[3.2.1]oct-8-yl)propionitrile was carried out with the reference to Embodiment 39.

MS *m*/*z* (ESI): 409.2 [M+H]⁺.

### Embodiment 64

### 3-((3-exo)-3-((7-((5-methyl-1H-pyrazol-3-yl)amino)thiazolo[4,5-d]pyrimidin-5-yl)amino)-8-azabicyclo[3.2.1]oct-8-yl)propionitrile

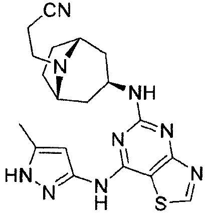

The preparation of 3-((3-*exo*)-3-((7-((5-methyl-1H-pyrazol-3-yl)amino)thiazolo[4,5-d]pyrimidin-5-yl)amino)-8-azabicyclo[3.2.1]oct-8-yl)propionitrile was carried out with the reference to Embodiment 60.

MS *m*/*z* (ESI): 410.2 [M+H]⁺.

### Embodiment 65

### 3-((3-exo)-3-((5-((5-methyl-1H-pyrazol-3-yl)amino)thiazolo[4,5-d]pyrimidin-7-yl)amino)-8-azabicyclo[3.2.1]oct-8-yl)propionitrile

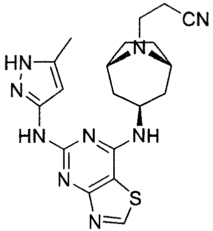

The preparation of 3-((3-*exo*)-3-((5-((5-methyl-1H-pyrazol-3-yl)amino)thiazolo[4,5-d]pyrimidin-7-yl)amino)-8-azabicyclo[3.2.1]oct-8-yl)propionitrile was carried out with the reference to Embodiment 39.

MS *m*/*z* (ESI): 410.2 [M+H]⁺.

### Embodiment 66

### 3-((3-exo)-3-((7-((5-methyl-1H-pyrazol-3-yl)amino)thiazolo[5,4-d]pyrimidin-5-yl)amino)-8-azabicyclo[3.2.1]oct-8-yl)propionitrile

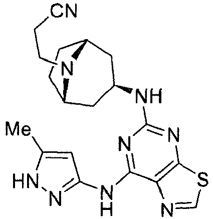

### Step 1: preparation of 5-chloro-N-(5-methyl-1H-pyrazol-3-yl)thiazolo[5,4-d]pyrimidin-7-amine

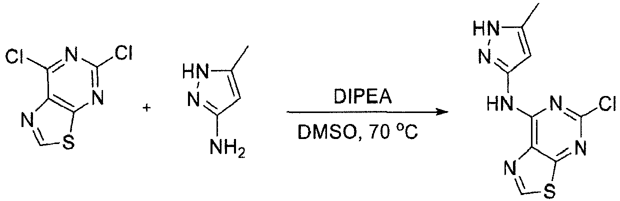

3-Amino-5-methylpyrazol (116 mg, 1.2 mmol) and DIPEA (258 mg, 2 mmol) were successively added to the solution of 5,7-dichlorothiazolo[5,4-d]pyrimidine(206 mg, 1 mmol) in dimethyl sulfoxide (10 mL), and the mixture was then stirred at 70°C for 1 hour. When the reaction was completed, water (50 mL) was added to the reaction mixture, and a solid was precipitated, which was then filtered and slurried with ethyl acetate to obtain the title compound as a yellow solid (200 mg, 75%).

MS *m*/*z* (ESI): 267.0 [M+H]⁺.

### Step 2: preparation of tert-butyl-(3-exo)-3-((7-((5-methyl-1H-pyrazol-3-yl)amino)thiazolo[5,4-d]pyrimidin-5-yl)amino)-8-azabicyclo[3.2.1]oct-8-carboxylate

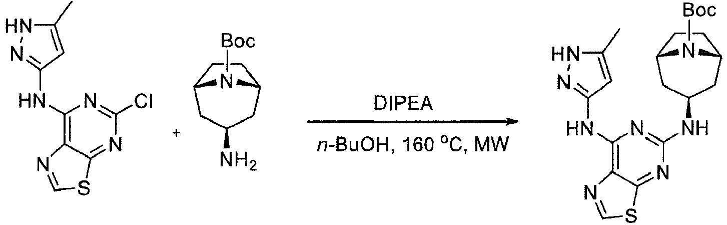

Tert-butyl-(3-*exo*)-3-amino-8-azabicyclo[3.2.1]oct-8-carboxylate (204 mg, 0.9 mmol) and DIPEA (193 mg, 1.5 mmol) were successively added to the solution of 5-chloro-N-(5-methyl-1H-pyrazol-3-yl)thiazolo[5,4-d]pyrimidin-7-amine(200 mg, 0.75 mmol) in n-butanol (10 mL). The mixture was stirred at 160°C for 15 hours under microwave condition. When the reaction was completed, the reaction mixture was extracted with ethyl acetate (15 mL x 3), and washed with saturated sodium chloride aqueous solution (15 mL x 3). The organic phase was collected, dried over anhydrous sodium sulfate, filtered and concentrated under reduced pressure. The resulting product was purified by silica gel column chromatography (dichloromethane: methanol=95:5) to obtain the title compound as a pale yellow solid (74 mg, 22%).

MS m/z (ESI): 457.1 [M+H]⁺.

### Step 3: preparation of 3-((3-exo)-3-((7-((5-methyl-1H-pyrazol-3-yl)amino)thiazolo[5,4-d]pyrimidin-5-yl)amino)-8-azabicyclo[3.2.1]oct-8-yl)propionitrile

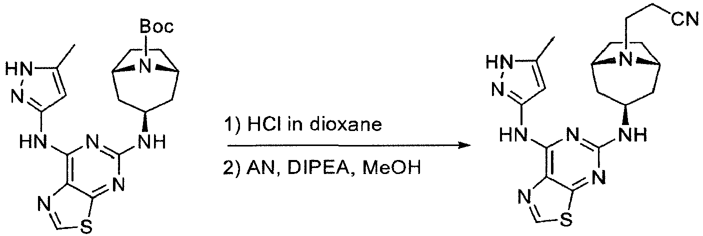

Tert-butyl-(3-*exo*)-3-((7-((5-methyl-1H-pyrazol-3-yl)amino)thiazolo[5,4-d]pyrimidin-5-yl)amino)-8-azabicyclo[3.2.1]oct-8-carboxylate (74 mg, 0.16 mmol) was dissolved in hydrochloride 1,4-dioxane solution(4.0 N, 2 mL). The mixture was stirred at room temperature for 30 minutes, concentrated, and dissolved in methanol (10 mL). DIPEA (83 mg, 0.64 mmol) was slowly added dropwise thereto, and the mixture was stirred at room temperature for 10 minutes. Acrylonitrile (9 mg, 0.24 mmol) was added thereto and the mixture stirred for another 2 hour. The reaction mixture was concentrated under reduced pressure, and the resulting product was purified by prep-HPLC to obtain the title compound as a white solid (16.3 mg, 25 %).

¹H NMR (400 MHz, DMSO) *δ* 12.07 (s, 1H), 9.33 (s, 1H), 8.76 (d, *J* = 20.4 Hz, 1H), 6.96 (s, 1H), 6.55 (d, *J* = 12.0 Hz, 1H), 4.14 (s, 1H), 3.31 (s, 2H), 2.61 (s, 4H), 2.21 (s, 3H), 1.91 (s, 2H), 1.78-1.54 (m, 6H).

### Embodiment 67

### 3-((3-exo)-3-((5-((5-methyl-1H-pyrazol-3-yl)amino)thiazolo[5,4-d]pyrimidin-7-yl)amino)-8-azabicyclo[3.2.1]oct-8-yl)propionitrile

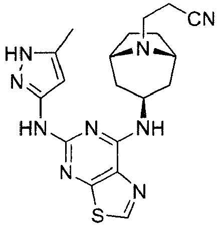

The preparation of 3-((3-*exo*)-3-((5-((5-methyl-1H-pyrazol-3-yl)amino)thiazolo[5,4-d]pyrimidin-7-yl)amino)-8-azabicyclo[3.2.1]oct-8-yl)propionitrile was carried out with the reference to Embodiment 39.

MS *m*/*z* (ESI): 410.2 [M+H]⁺.

### Embodiment 68

### 3-((3-exo)-3-((8-((5-methyl-1H-pyrazol-3-yl)amino)imidazo[1,2-a]pyrazin-6-yl)amino)-8-azabicyclo[3 .2.1]oct-8-yl)propionitrile

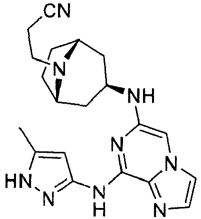

The preparation of 3-((3-*exo*)-3-((8-((5-methyl-1H-pyrazol-3-yl)amino)imidazo[1,2-a]pyrazin-6-yl)amino)-8-azabicyclo[3.2.1]oct-8-yl)propionitrile was carried out with the reference to Embodiment 40.

MS *m*/*z* (ESI): 392.2 [M+H]⁺.

### Embodiment 69

### 3-((3-exo)-3-((6-((5-methyl-1H-pyrazol-3-yl)amino)imidazo[1,2-a]pyrazin-8-yl)amino)-8-azabicyclo[3.2.1]oct-8-yl)propionitrile

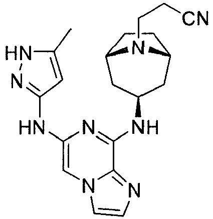

The preparation of 3-((3-*exo*)-3-((6-((5-methyl-1H-pyrazol-3-yl)amino)imidazo[1,2-a]pyrazin-8-yl)amino)-8-azabicyclo[3.2.1]oct-8-yl)propionitrile was carried out with the reference to Embodiment 39.

MS m/z (ESI): 392.2 [M+H]⁺.

### Embodiment 70

### 3-((3-exo)-3-((6-((5-methyl-1H-pyrazol-3-yl)amino)pyrazolo[1,5-a]pyrazin-4-yl)amino)-8-azabicyclo[3.2.1]oct-8-yl)propionitrile

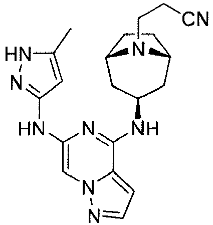

The preparation of 3-((3-*exo*)-3-((6-((5-methyl-1H-pyrazol-3-yl)amino)pyrazolo[1,5-a]pyrazin-4-yl)amino)-8-azabicyclo[3.2.1]oct-8-yl)propionitrile was carried out with the reference to Embodiment 39.

MS *m*/*z* (ESI): 392.2 [M+H]⁺.

### Embodiment 71

### 3-((3-exo)-3-((4-((5-(hydroxymethyl)thiazol-2-yl)amino)pyrrolo[2,1-f][1,2,4]triazin-2-yl)amino)-8-azabicyclo[3.2.1]oct-8-yl)propionitrile

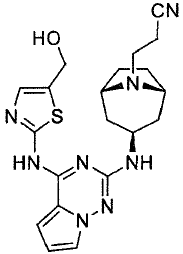

The preparation of 3-((3-exo)-3-((4-((5-(hydroxymethyl)thiazol-2-yl)amino)pyrrolo[2,1-f][1,2,4]triazin-2-yl)amino)-8-azabicyclo[3.2.1]oct-8-yl)propionitrile was carried out with the reference to Embodiment 2.

MS *m*/*z* (ESI): 425.2 [M+H]⁺.

### Embodiment 72

### 3-((3-exo)-3-((2-((5-methyl-1H-pyrazol-3-yl)amino)pyrrolo[2,1-f][1,2,4]triazin-4-yl)amino)-8-azabicyclo[3 .2.1]oct-8-yl)propionitrile

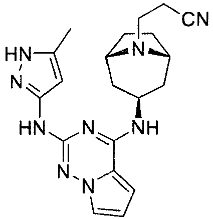

### Step 1: preparation of 3-((3-exo)-3-((2-chloropyrrolo[2,1-f][1,2,4]triazin-4-yl)amino)-8-azabicyclo[3.2.1]oct-8-carboxylate

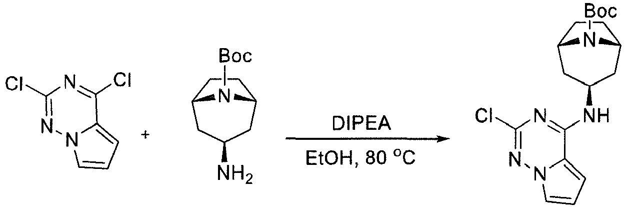

Tert-butyl-(3-*exo*)-3-amino-8-azabicyclo[3.2.1]oct-8-carboxylate (271 mg, 1.2 mmol) and DIPEA (258 mg, 2 mmol) were successively added to the solution of 5,7-dichloropyrrolo[2,1-f][1,2,4]triazin(188 mg, 1 mmol) in ethanol (10 mL). The mixture was then stirred at 80°C for 2 hours under reflux. When the reaction was completed, the mixture was extracted with dichloromethane (15 mL x 3), and washed with saturated sodium chloride aqueous solution (15 mL x 3). The organic phase was collected, dried over anhydrous sodium sulfate, filtered and concentrated under reduced pressure. The resulting product was purified by silica gel column chromatography (dichloromethane 100%) to obtain the title compound as a pale yellow solid (350 mg, 93%).

MS *m*/*z* (ESI): 378.1 [M+H]⁺.

### Step 2: preparation of tert-butyl-(3-exo)-3-((2-((5-methyl-lH-pyrazol-3-yl)amino)pyrrolo[2,1-f][1,2,4]triazin-4-yl)amino)-8-azabicyclo[3.2.1]oct-8-carboxylate

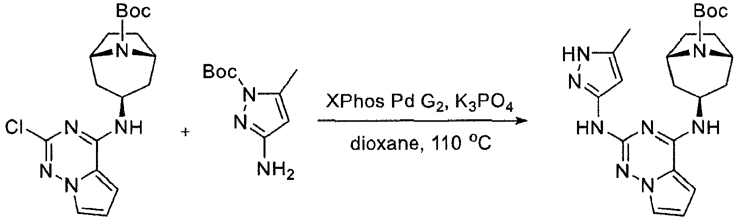

3-((3-*exo*)-3-((2-chloropyrrolo[2,1-f][1,2,4]triazin-4-yl)amino)-8-azabicyclo[3.2.1]oct-8-carboxylate (350 mg, 0.93 mmol), tert-butyl-3amino-5-methyl-1 H-pyrazol-1-carboxylic acid(219 mg, 1.11 mmol), chloro(2-dicyclohexylphosphino-2',4',6'-triisopropyl-1,1'-biphenyl)[2-(2'-amino-1,1'-biphenyl)]palladium(II) (73 mg, 0.093 mmol) and potassium phosphate (591 mg, 2.79 mmol) were suspended in ultra-dry 1,4-dioxane (20 mL), and the mixture was filled with nitrogen for 3 times. The reaction mixture was then sealed, and stirred at 110°Covernight. When the reaction was completed, the mixture was extracted with dichloromethane (15 mL x 3), and washed with saturated sodium chloride aqueous solution (15 mL x 3). The organic phase was collected, dried over anhydrous sodium sulfate, filtered and concentrated under reduced pressure. The resulting product was purified by silica gel column chromatography (dichloromethane: methanol=95:5) to obtain the title compound as a yellow solid (100 mg, 24%).

MS *m*/*z* (ESI): 439.2 [M+H]⁺.

### Step 3: preparation of 3-((3-exo)-3-((2-((5-methyl-1H-pyrazol-3-yl)amino)pyrrolo[2,1-f][1,2,4]triazin-4-yl)amino)-8-azabicyclo[3.2.1]oct-8-yl)propionitrile

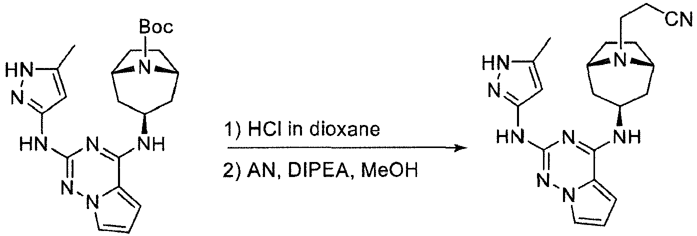

Tert-butyl-(3-exo)-3-((2-((5-methyl-1H-pyrazol-3-yl)amino)pyrrolo[2,1-f][1,2,4] triazin-4-yl)amino)-8-azabicyclo[3.2.1]oct-8-carboxylate (100 mg, 0.23 mmol) was dissolved in hydrochloride 1,4-dioxane solution(4.0 N, 5 mL). The mixture was stirred at room temperature for 30 minutes, concentrated, and then dissolved in methanol (10 mL). DIPEA (119 mg, 0.92 mmol) was slowly added dropwise into the mixture, and the mixture was stirred at room temperature for 10minutes. Acrylonitrile (18 mg, 0.35 mmol) was added thereto and the mixture was stirred for another 2 hours. The reaction mixture was concentrated under reduced pressure, and the resulting product was purified by prep-HPLC to obtain the title compound as a white solid (31.8 mg, 35 %).

¹H NMR (400 MHz, DMSO) *δ* 11.67 (s, 1H), 8.48 (s, 1H), 7.74 (s, 1H), 7.35 (s, 1H), 6.73 (s, 1H), 6.36 (s, 2H), 4.49 (s, 1H), 3.30 (s, 2H), 2.72-2.59 (m, 4H), 2.18 (s, 3H), 1.91 (s, 2H), 1.83-1.64 (m, 6H).

MS *m*/*z* (ESI): 392.2 [M+H]⁺.

### Embodiment 73

### 3-((3-exo)-3-((8-((5-methyl-1H-pyrazol-3-yl)amino)-[1,2,4]triazolo[1,5-a]pyrazin-6-yl)amino)-8-azabicyclo[3.2.1]oct-8-yl)propionitrile

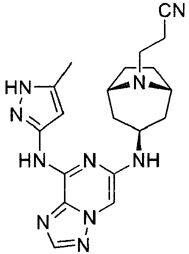

The preparation of 3-((3-*exo*)-3-((8-((5-methyl-1H-pyrazol-3-yl)amino)-[1,2,4]triazolo[1,5-a]pyrazin-6-yl)amino)-8-azabicyclo[3.2.1]oct-8-yl)propionitrile was carried out with the reference to Embodiment 6.

MS *m*/*z* (ESI): 393.2 [M+H]⁺.

### Embodiment 74

### 3-((3-exo)-3-((6-((5-methyl-1H-pyrazol-3-yl)amino)-[1,2,4]triazolo[1,5-a]pyrazin-8-yl)amino)-8-azabicyclo[3.2.1]oct-8-yl)propionitrile

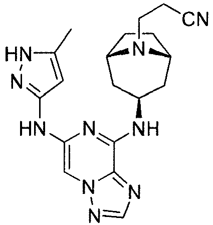

The preparation of 3-((3-*exo*)-3-((6-((5-methyl-1H-pyrazol-3-yl)amino)-[1,2,4]triazolo[1,5-a]pyrazin-8-yl)amino)-8-azabicyclo[3.2.1]oct-8-yl)propionitrile was carried out with the reference to Embodiment 72.

MS *m*/*z* (ESI): 393.2 [M+H]⁺.

### Embodiment 75

### 3-((3-exo)-3-((2-methyl-6-((5-methyl-1H-pyrazol-3-yl)amino)-[1,2,4]triazolo[1,5-a]pyrazin-8-yl)amino)-8-azabicyclo[3.2.1]oct-8-yl)propionitrile

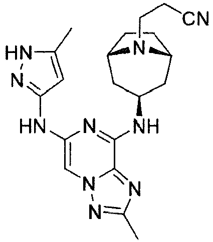

The preparation of 3-((3-*exo*)-3-((2-methyl-6-((5-methyl-1H-pyrazol-3-yl)amino)-[1,2,4]triazolo[1,5-a]pyrazin-8-yl)amino)-8-azabicyclo[3.2.1]oct-8-yl)propionitrile was carried out with the reference to Embodiment 72.

MS *m*/*z* (ESI): 407.2 [M+H]⁺.

### Embodiment 76

### 3-((3-exo)-3-((8-((5-methyl-1H-pyrazol-3-yl)amino)-[1,2,4]triazolo[4,3-a]pyrazin-6-yl)amino)-8-azabicyclo[3.2.1]oct-8-yl)propionitrile

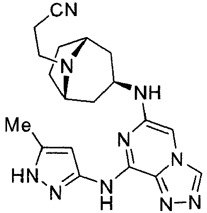

The preparation of 3-((3-*exo*)-3-((8-((5-methyl-1H-pyrazol-3-yl)amino)-[1,2,4]triazolo[4,3-a]pyrazin-6-yl)amino)-8-azabicyclo[3.2.1]oct-8-yl)propionitrile was carried out with the reference to Embodiment 66.

MS *m*/*z* (ESI): 393.2 [M+H]⁺.

### Embodiment 77

### 3-((3-exo)-3-((6-((5-methyl-1H-pyrazol-3-yl)amino)-[1,2,4]triazolo[4,3-a]pyrazin-8-yl)amino)-8-azabicyclo[3.2.1]oct-8-yl)propionitrile

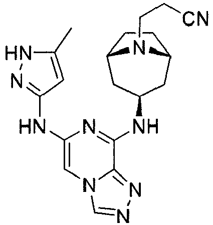

The preparation of 3-((3-*exo*)-3-((6-((5-methyl-1H-pyrazol-3-yl)amino)-[1,2,4]triazolo[4,3-a]pyrazin-8-yl)amino)-8-azabicyclo[3.2.1]oct-8-yl)propionitrile was carried out with the reference to Embodiment 72.

MS *m*/*z* (ESI): 393.2 [M+H]⁺.

### Embodiment 78

### 3-((3-exo)-3-((5-((5-(hydroxymethyl)thiazol-2-yl)amino)imidazo[1,2-c]pyrimidin-7-yl)amino)-8-azabicyclo[3.2.1]oct-8-yl)propionitrile

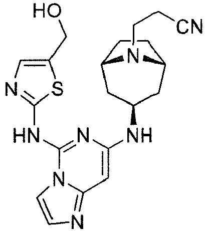

The preparation of 3-((3-*exo*)-3-((5-((5-(hydroxymethyl)thiazol-2-yl)imidazo[1,2-c]pyrimidin-7-yl)amino)-8-azabicyclo[3.2.1]oct-8-yl)propionitrile was carried out with the reference to Embodiment 2.

MS m/z (ESI): 425.2 [M+H]⁺.

### Embodiment 79

### 3-((3-exo)-3-((7-((5-methyl-1H-pyrazol-3-yl)amino)imidazo[1,2-c]pyrimidin-5-yl)amino)-8-azabicyclo[3.2.1]oct-8-yl)propionitrile

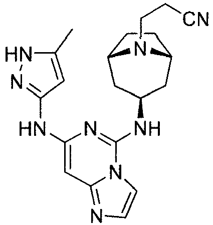

### Step 1: preparation of 3-((3-exo)-3-((7-chloroimidazo[1,2-c]pyrimidin-5-yl)amino)-8-azabicyclo[3.2.1]oct-8-carboxylate

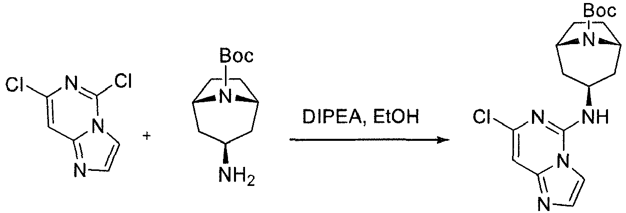

Tert-butyl-(3-*exo*)-3-amino-8-azabicyclo[3.2.1]oct-8-carboxylate (271 mg, 1.2 mmol), DIPEA (258 mg, 2 mmol) were successively added to the solution of 5,7-dichloroimidazo[1,2-c]pyrimidine(188 mg, 1 mmol) in ethanol (10 mL), and the mixture was stirred at room temperature overnight. When the reaction was completed, the mixture was extracted with dichloromethane (15 mL x 3), and washed with saturated sodium chloride aqueous solution (15 mL x 3). The organic phase was collected, dried over anhydrous sodium sulfate, filtered and concentrated under reduced pressure. The resulting product was purified by silica gel column chromatography (dichloromethane: methanol=99:1) to obtain the title compound as a pale yellow solid (374 mg, 99%).

MS *m*/*z* (ESI): 378.1 [M+H]⁺.

### Step 2: preparation of tert-butyl-(3-exo)-3-((7-((5-methyl-1H-pyrazol-3-yl)amino)imidazo[1,2-c]pyrimidin-5-yl)amino)-8-azabicyclo[3.2.1]oct-8-carboxylate

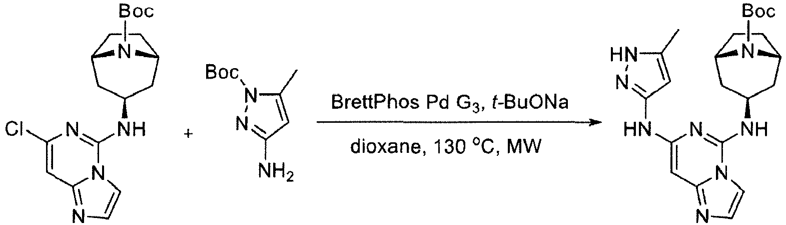

3-((3-*exo*)-3-((7-chloroimidazo[1,2-c]pyrimidin-5-yl)amino)-8-azabicyclo[3.2.1]oct-8-carboxylate (330 mg, 0.87 mmol), tert-butyl-3amino-5-methyl-1H-pyrazol-1-carboxylate (259 mg, 1.31 mmol), methanesulfonato(2-dicyclohexylphosphine-3,6-dimethoxyl-2',4',6'-triisopropyl-1,1'-biphenyl)(2'-amino-1,1'-biphenyl-2-yl)palladium(II) (82 mg, 0.09 mmol) and sodium tert-butoxide (252 mg, 2.62 mmol) were suspended in ultra-dry 1,4-dioxane (20 mL). The mixture was filled with nitrogen for 3 times, then heated to 130°C with microwave and reacted for 4 hours. When the reaction was completed, the mixture was extracted with dichloromethane (15 mL x 3), and washed with saturated sodium chloride aqueous solution (15 mL x 3). The organic phase was collected, dried over anhydrous sodium sulfate, filtered and concentrated under reduced pressure. The resulting product was purified by silica gel column chromatography (dichloromethane: methanol=95:5) to obtain the title compound as a yellow solid (30 mg, 8%).

MS *m*/*z* (ESI): 439.2 [M+H]⁺.

### Step 3: preparation of 3-((3-exo)-3-((7-((5-methyl-1H-pyrazol-3-yl)amino)imidazo[1,2-c]pyrimidin-5-yl)amino)-8-azabicyclo[3.2.1]oct-8-yl)propionitrile

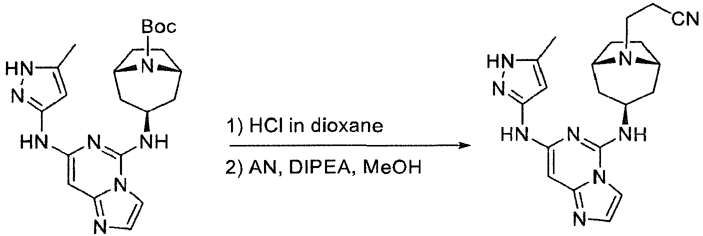

Tert-butyl-(3-*exo*)-3-((7-((5-methyl-1H-pyrazol-3-yl)amino)imidazo[1,2-c]pyrimidin-5-yl)amino)-8-azabicyclo[3.2.1]oct-8-carboxylate (30 mg, 0.07 mmol) was dissolved in hydrochloride 1,4-dioxane solution(4.0 N, 2 mL). The mixture was stirred at room temperature for 30 minutes, concentrated, and then dissolved in methanol (5 mL). DIPEA (36 mg, 0.28 mmol) was slowly added dropwise into the mixture, and the mixture was stirred at room temperature for 10 minutes. Acrylonitrile (5 mg, 0.1 mmol) was added thereto and the mixture stirred for another 2 hours. The reaction mixture was concentrated under reduced pressure, and the resulting product was purified by prep-HPLC to obtain the title compound as a yellow solid (8.9 mg, 32 %).

¹H NMR (400 MHz, DMSO) *δ* = 11.73 (s, 1H), 8.65 (s, 1H), 7.71 (s, 1H), 7.23 (d, *J*= 8.4, 1H), 7.19 (d, *J* = 1.2, 1H), 6.55 (s, 1H), 5.99 (s, 1H), 4.48-4.21 (m, 1H), 3.30 (s, 2H), 2.62 (m, 4H), 2.20 (s, 3H), 1.97-1.90 (m, 2H), 1.87-1.79 (m, 2H), 1.76-1.56 (m, 4H).

MS *m*/*z* (ESI): 392.2 [M+H]⁺.

### Embodiment 80

### 3-((3-exo)-3-((7-((5-methyl-1H-pyrazol-3-yl)amino)pyrazolo[1,5-c]pyrimidin-5-yl)amino)-8-azabicyclo[3.2.1]oct-8-yl)propionitrile

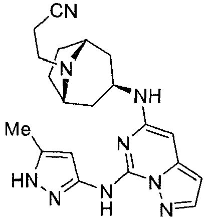

The preparation of 3-((3-*exo*)-3-((7-((5-methyl-1H-pyrazol-3-yl)amino)pyrazolo[1,5-c]pyrimidin-5-yl)amino)-8-azabicyclo[3.2.1]oct-8-yl)propionitrile was carried out with the reference to Embodiment 66.

MS *m*/*z* (ESI): 392.2 [M+H]⁺.

### Embodiment 81

### 3-((3-exo)-3-((7-((5-methyl-1H-pyrazol-3-yl)amino)-[1,2,4]triazolo[1,5-c]pyrimidin-5-yl)amino)-8-azabicyclo[3.2.1]oct-8-yl)propionitrile

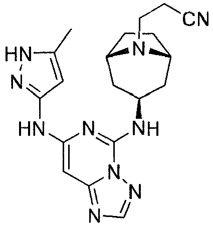

The preparation of 3-((3-*exo*)-3-((7-((5-methyl-1H-pyrazol-3-yl)amino)-[1,2,4]triazolo[1,5-c]pyrimidin-5-yl)amino)-8-azabicyclo[3.2.1]oct-8-yl)propionitrile was carried out with the reference to Embodiment 79.

MS *m*/*z* (ESI): 393.2 [M+H]⁺.

### Embodiment 82

### 3-((3-exo)-3-((7-((5-methyl-1H-pyrazol-3-yl)amino)-[1,2,4]triazolo[4,3-c]pyrimidin-5-yl)amino)-8-azabicyclo[3.2.1]oct-8-yl)propionitrile

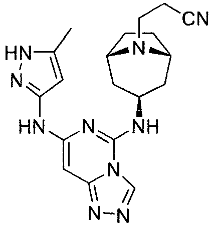

The preparation of 3-((3-*exo*)-3-((7-((5-methyl-1H-pyrazol-3-yl)amino)-[1,2,4]triazolo[4,3-c]pyrimidin-5-yl)amino)-8-azabicyclo[3.2.1]oct-8-yl)propionitrile was carried out with the reference to Embodiment 79.

MS *m*/*z* (ESI): 393.2 [M+H]⁺.

### Embodiment 83

### 3-((3-exo)-3-((7-((5-methyl-1H-pyrazol-3-yl)amino)imidazo[1,5-b]pyridazin-5-yl)amino)-8-azabicyclo[3.2.1]oct-8-yl)propionitrile

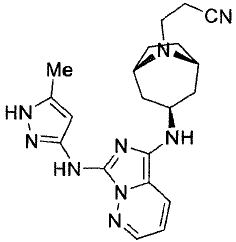

The preparation of 3-((3-*exo*)-3-((7-((5-methyl-1H-pyrazol-3-yl)amino)imidazo[1,5-b]pyridazin-5-yl)amino)-8-azabicyclo[3.2.1]oct-8-yl)propionitrile was carried out with the reference to Embodiment 6.

MS m/z (ESI): 392.2 [M+H]⁺.

### Embodiment 84

### 3-((3-exo)-3-((8-((5-methyl-1H-pyrazol-3-yl)amino)imidazo[1,5-a]pyrimidin-6-yl)amino)-8-azabicyclo[3.2.1]oct-8-yl)propionitrile

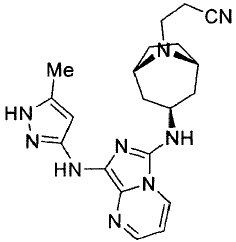

The preparation of 3-((3-*exo*)-3-((8-((5-methyl-1H-pyrazol-3-yl)amino)imidazo[1,5-a]pyrimidin-6-yl)amino)-8-azabicyclo[3.2.1]oct-8-yl)propionitrile was carried out with the reference to Embodiment 66.

MS *m*/*z* (ESI): 392.2 [M+H]⁺.

### Embodiment 85

### 3-((3-exo)-3-((1-((5-methyl-1H-pyrazol-3-yl)amino)imidazo[1,5-a]pyrazin-3-yl)amino)-8-azabicyclo[3 .2.1]oct-8-yl)propionitrile

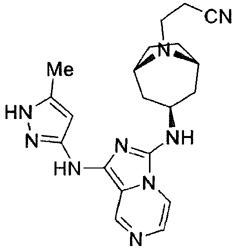

The preparation of 3-((3-*exo*)-3-((1-((5-methyl-1H-pyrazol-3-yl)amino)imidazo[1,5-a]pyrazin-3-yl)amino)-8-azabicyclo[3.2.1]oct-8-yl)propionitrile was carried out with the reference to Embodiment 66.

MS *m*/*z* (ESI): 392.2 [M+H]⁺.

### Embodiment 86

### 3-((3-exo)-3-((5-((5-methyl-1H-pyrazol-3-yl)amino)-1,6-diazanaphthalen-7-yl)amino)-8-azabicyclo[3.2.1]oct-8-yl)propionitrile

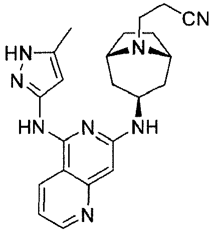

The preparation of 3-((3-*exo*)-3-((5-((5-methyl-1H-pyrazol-3-yl)amino)-1,6-diazanaphthalen-7-yl)amino)-8-azabicyclo[3.2.1]oct-8-yl)propionitrile was carried out with the reference to Embodiment 4.

MS *m*/*z* (ESI): 403.2 [M+H]⁺.

### Embodiment 87

### 3-((3-exo)-3-((2-((5-methyl-1H-pyrazol-3-yl)amino)quinazolin-4-yl)amino)-8-azabicyclo[3.2.1]oct-8-yl)propionitrile

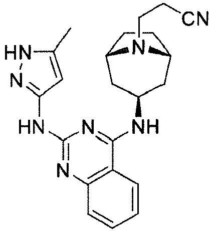

The preparation of 3-((3-*exo*)-3-((2-((5-methyl-1H-pyrazol-3-yl)amino)quinazolin-4-yl)amino)-8-azabicyclo[3.2.1]oct-8-yl)propionitrile was carried out with the reference to Embodiment 39.

¹H NMR (400 MHz, DMSO) *δ* = 11.76 (s, 1H), 8.90 (s, 1H), 8.08 (d, *J* = 8.0, 1H), 7.72 (s, 1H), 7.53 (t,*J*= 7.4, 1H), 7.36 (s, 1H), 7.10 (s, 1H), 6.55 (s, 1H), 4.72-4.58 (m, 1H), 3.30 (s, 2H), 2.64 (s, 4H), 2.20 (s, 3H), 1.92 (s, 2H), 1.86-1.62 (m, 6H).

MS m/z (ESI): 403.2 [M+H]⁺.

### Embodiment 88

### 3-((3-exo)-3-((7-((5-methyl-1H-pyrazol-3-yl)amino)pyrido[3,4-b]pyrazin-5-yl)amino)-8-azabicyclo[3.2.1]oct-8-yl)propionitrile

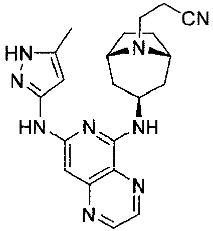

### Step 1: preparation of 3-((3-exo)-3-((7-chloropyrido[3,4-b]pyrazin-5-yl)amino)-8-azabicyclo[3.2.1]oct-8-carboxylate

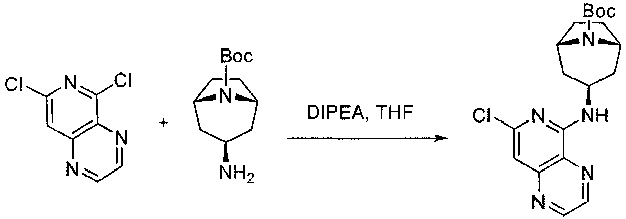

Tert-butyl-(3-exo)-3-amino-8-azabicyclo[3.2.1]oct-8-carboxylate (271 mg, 1.2 mmol) and DIPEA (258 mg, 2 mmol) were successively added to the solution of 5,7-dichloropyrido[3,4-b]pyrazin (200 mg, 1 mmol) in tetrahydrofuran (10 mL), and the mixture was stirred at room temperature for 2 hours. When the reaction was completed, the mixture was extracted with dichloromethane (15 mL x 3), and washed with saturated sodium chloride aqueous solution (15 mL x 3). The organic phase was collected, dried over anhydrous sodium sulfate, filtered and concentrated under reduced pressure. The resulting product was purified by silica gel column chromatography (dichloromethane: methanol=99:1) to obtain the title compound as a pale yellow solid (350 mg, 90%).

MS *m*/*z* (ESI): 390.1 [M+H]⁺.

### Step 2: preparation of tert-butyl-(3-exo)-3-((7-((5-methyl-1H-pyrazol-3-yl)amino)pyrido[3,4-b]pyrazin-5-yl)amino)-8-azabicyclo[3.2.1]oct-8-carboxylate

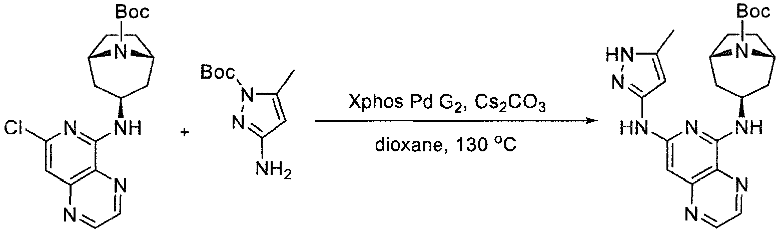

3-((3-*exo*)-3-((7-chloropyrido[3,4-b]pyrazin-5-yl)amino)-8-azabicyclo[3.2.1]oct-8-carboxylate (350 mg, 0.9 mmol), tert-butyl-3amino-5-methyl-1H-pyrazol-1-carboxylate (266 mg, 1.35 mmol), chloro(2-dicyclohexylphosphino-2',4',6'-triisopropyl-1,1'-biphenyl)[2-(2'-amino-1,1'-biphenyl)]palladium(II) (71 mg, 0.09 mmol) and cesium carbonate (880 mg, 2.7 mmol) were suspended in ultra-dry 1,4-dioxane (20 mL), and the mixture was filled with nitrogen for 3 times. The reaction mixture was then sealed, heated to 130°C and reacted overnight. When the reaction was completed, the mixture was extracted with dichloromethane (15 mL x 3), and washed with saturated sodium chloride aqueous solution (15 mL x 3). The organic phase was collected, dried over anhydrous sodium sulfate, filtered and concentrated under reduced pressure. The resulting product was purified by silica gel column chromatography (dichloromethane: methanol=98:2) to obtain the title compound as a yellow solid (32.4 mg, 8%).

MS *m*/*z* (ESI): 451.2 [M+H]⁺.

### Step 3: preparation of 3-((3-exo)-3-((7-((5-methyl-1H-pyrazol-3-yl)amino)pyrido[3,4-b]pyrazin-5-yl)amino)-8-azabicyclo[3.2.1]oct-8-yl)propionitrile

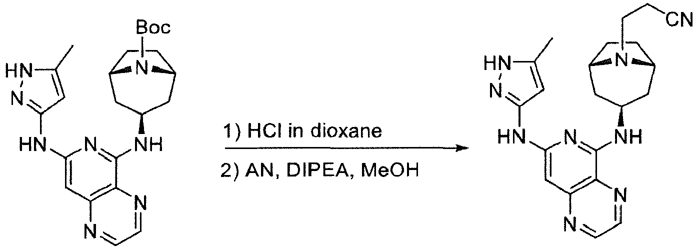

3-((3-exo)-3-((7-chloropyrido[3,4-b]pyrazin-5-yl)amino)-8-azabicyclo[3.2.1]oct-8-carboxylate (32.4 mg, 0.07 mmol) was dissolved in hydrochloride 1,4-dioxane solution(4.0 N, 2 mL). The mixture was stirred at room temperature for 30 minutes, concentrated, and then dissolved in methanol (5 mL). DIPEA (36 mg, 0.28 mmol) was slowly added dropwise into the mixture, and the mixture was stirred at room temperature for 10 minutes. Acrylonitrile (5 mg, 0.1 mmol) was added thereto and the mixture was stirred for another 2 hours. The reaction mixture was concentrated under reduced pressure, and the resulting product was purified by prep-HPLC to obtain the title compound as a yellow solid (8.2 mg, 29 %).

¹H NMR (400 MHz, DMSO) *δ* = 11.80 (s, 1H), 9.16 (s, 1H), 8.71 (d, *J* = 2.0, 1H), 8.31 (d, *J* = 1.6, 1H), 7.48 (d, *J* = 8.4,1H), 6.69 (s, 1H), 6.29 (s, 1H), 4.66-4.51 (m, 1H), 3.30 (s, 2H), 2.76 (dt, J= 12.4, 6.8, 4H), 2.30 (s, 3H), 2.08-1.93 (m, 2H), 1.85-1.83 (m, 6H).

MS *m*/*z* (ESI): 404.2 [M+H]⁺.

### Embodiment 89

### 3-((3-exo)-3-((4-((5-methyl-1H-pyrazol-3-yl)amino)pyrido[2,3-d]pyrimidin-2-yl)amino)-8-azabicyclo[3.2.1]oct-8-yl)propionitrile

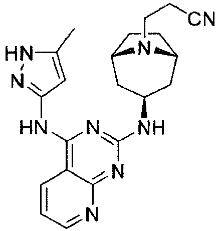

### Step 1: preparation of 2-chloro-N-(5-methyl-1H-pyrazol-3-yl)pyrido[2,3-d]pyrimidin-4-amine

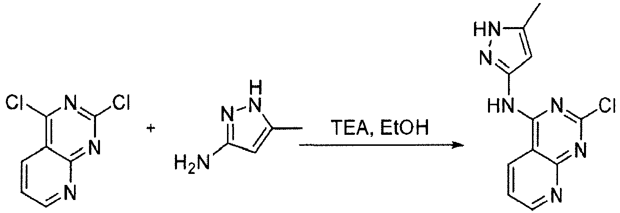

2,4-Dichloropyrido[2,3-d]pyrimidine (200 mg, 1.0 mmol), 5-methyl-1H-pyrazol-3-amine (107 mg, 1.1 mmol) and DIPEA (0.5 mL, 3.0 mmol) were added to anhydrous ethanol (5 mL), and the mixture was stirred at room temperature for 13.5 hours. Then the mixture was concentrated under reduced pressure, and the residue solid was washed with water-ethanol (v\v= 9:1, 20 mL), followed by filtration. The filter residue was then dried under reduced pressure to obtain the title compound (185 mg, 71%).

MS m/z (ESI): 261.1 [M+H]⁺.

### Step 2: preparation of Tert-butyl (3-exo)-3-((4-((5-methyl-1H-pyrazol-3-yl)amino)pyrido[2,3-d]pyrimidin-2-yl)amino)-8-azabicyclo[3.2.1]oct-8-carboxylate

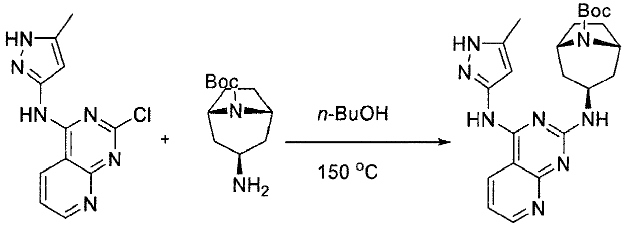

2-Chloro-N-(5-methyl-1H-pyrazol-3-yl)pyrido[2,3-d]pyrimidin-4-amine (50 mg, 0.192 mmol) and tert-butyl (3-*exo*)-3-amino-8-azabicyclo[3.2.1]oct-8-carboxylate (87 mg, 0.383 mmol) were added to n-butanol (3 mL). The mixture was heated to 150 °C with a microwave reactor and reacted for 4 hour. The mixture was concentrated under reduced pressure to remove the solvent, and the residue was purified by reverse phase chromatography to obtain the title compound (39.5 mg, 46%).

MS *m*/*z* (ESI): 451.2 [M+H]⁺.

### Step 3: preparation of 3-((3-exo)-3-((4-((5-methyl-1H-pyrazol-3-yl)amino)quinazolin-2-yl)amino)-8-azabicyclo[3.2.1]oct-8-yl)propionitrile

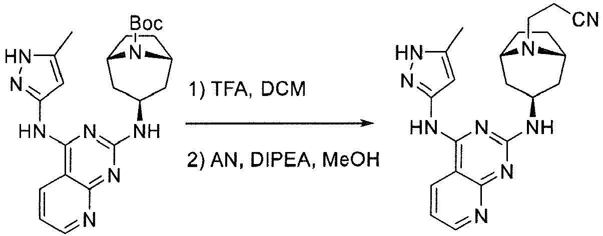

Tert-butyl (3-*exo*)-3-((4-((5-methyl-1H-pyrazol-3-yl)amino)pyrido[2,3-d]pyrimidin-2-yl)amino)-8-azabicyclo[3.2.1]oct-8-carboxylate (39.5 mg, 0.088 mmol) was dissolved in 4M HCl in 1,4-dioxane solution (10 mL). The mixture was stirred at room temperature for 30 minutes, and concentrated under reduced pressure to remove the solvent. The residue was dissolved in anhydrous methanol (10 mL), followed by successive addition of DIPEA (0.72 mL, 4.38 mmol) and acrylonitrile (0.29 mL, 4.38 mmol). The reaction mixture was stirred at room temperature for 75 minutes, and concentrated under reduced pressure to remove the solvent. The residue was purified by reverse phase HPLC to obtain the title compound (10.6 mg, 30%).

¹H NMR (400 MHz, DMSO-*d*₆) *δ* 12.07 (s, 1H), 10.11 (s, 1H), 8.71 (dd, *J* = 9.4,1.8 Hz, 1H), 8.63 (dd, *J* = 3.7, 1.1 Hz, 1H), 7.02 (ddd, *J* = 11.4, 10.6, 6.7 Hz, 2H), 6.76 (d, *J* = 84.4 Hz, 1H), 4.26 (s, 1H), 3.31-3.25 (m, 2H), 2.64 (t, *J* = 11.0 Hz, 4H), 2.25 (d, *J*= 13.7 Hz, 3H), 1.92 (d, *J=* 8.5 Hz, 2H), 1.64 (dt, *J=* 25.4, 10.4 Hz, 6H).

MS *m*/*z* (ESI): 404.3 [M+H]⁺.

### Embodiment 90

### 3-((3-exo)-3-((2-((5-methyl-1H-pyrazol-3-yl)amino)pyrido[2,3-d]pyrimidin-4-yl)amino)-8-azabicyclo[3 .2.1]oct-8-yl)propionitrile

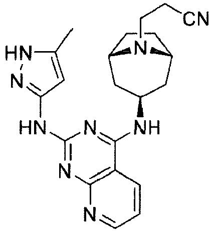

3-((3-*exo*)-3-((2-((5-methyl-1H-pyrazol-3-yl)amino)pyrido[2,3-d]pyrimidin-4-yl)amino)-8-azabicyclo[3.2.1]oct-8-yl)propionitrile was carried out with the reference to Embodiment 6.

¹H NMR (400 MHz, DMSO) *δ* = 11.83 (s, 1H), 9.19 (s, 1H), 8.67 (s, 1H), 8.48 (s, 1H), 7.93 (s, 1H), 7.09 (s, 1H), 6.54 (s, 1H), 4.58 (s, 1H), 3.30 (s, 2H), 2.68-2.59 (m, 4H), 2.22 (s, 3H), 1.93 (s, 2H), 1.86-1.67 (m, 6H).

MS *m*/*z* (ESI): 404.2 [M+H]⁺.

### Embodiment 91

### 3-((3-exo)-3-((7-((5-methyl-1H-pyrazol-3-yl)amino)pyrido[4,3-d]pyrimidin-5-yl)amino)-8-azabicyclo[3.2.1]oct-8-yl)propionitrile

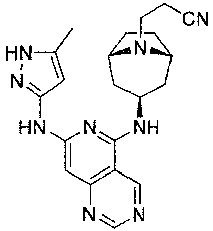

3-((3-*exo*)-3-((7-((5-methyl-1H-pyrazol-3-yl)amino)pyrido[4,3-d]pyrimidin-5-yl)amino)-8-azabicyclo[3.2.1]oct-8-yl)propionitrile was carried out with the reference to Embodiment 88.

MS *m*/*z* (ESI): 404.2 [M+H]⁺.

### Embodiment 92

### 3-((3-exo)-3-((2-((5-methyl-1H-pyrazol-3-yl)amino)pteridin-4-yl)amino)-8-azabicyclo[3.2.1]oct-8-yl)propionitrile

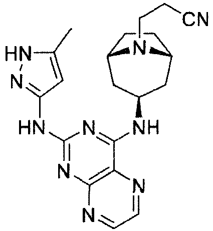

3-((3-*exo*)-3-((2-((5-methyl-1H-pyrazol-3-yl)amino)pteridin-4-yl)amino)-8-azabicyclo[3.2.1]oct-8-yl)propionitrile was carried out with the reference to Embodiment 88.

MS *m*/*z* (ESI): 405.2 [M+H]⁺.

### Embodiment 93

### 3-((3-exo)-3-((7-((5-methyl-1H-pyrazol-3-yl)amino)-2-carbonyl-1,2-2H-1,6-diazanaphthalen-5-yl)amino)-8-azabicyclo[3.2.1]oct-8-yl)propionitrile

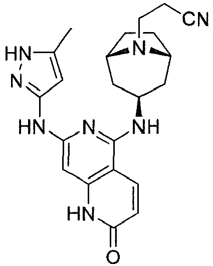

3-((3-*exo*)-3-((7-((5-methyl-1H-pyrazol-3-yl)amino)-2-carbonyl-1,2-2H-1,6-diazanaphthalen-5-yl)amino)-8-azabicyclo[3.2.1]oct-8-yl)propionitrile was carried out with the reference to Embodiment 88.

MS *m*/*z* (ESI): 419.2 [M+H]⁺.

### Embodiment 94

### 3-((3-exo)-3-((1-methyl-7-((5-methyl-1H-pyrazol-3-yl)amino)-2-carbonyl-1,2-2H-1,6-diazanaphthalen-5-yl)amino)-8-azabicyclo[3.2.1]oct-8-yl)propionitrile

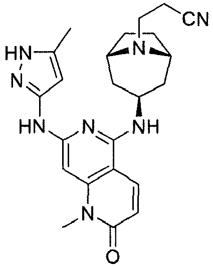

3-((3-*exo*)-3-((1-methyl-7-((5-methyl-1H-pyrazol-3-yl)amino)-2-carbonyl-1,2-2H-1,6-diazanaphthalen-5-yl)amino)-8-azabicyclo[3.2.1]oct-8-yl)propionitrile was carried out with the reference to Embodiment 88.

MS *m*/*z* (ESI): 433.2 [M+H]⁺.

### Embodiment 95

### 3-((3-exo)-3-((6-methyl-3-((5-methyl-1H-pyrazol-3-yl)amino)-5-carbonyl-5,6-2H-2,6-diazanaphthalen-1-yl)amino)-8-azabicyclo[3.2.1]oct-8-yl)propionitrile

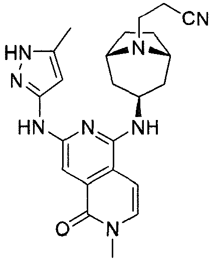

3-((3-*exo*)-3-((6-methyl-3-((5-methyl-1H-pyrazol-3-yl)amino)-5-carbonyl-5,6-2H-2,6-diazanaphthalen-1-yl)amino)-8-azabicyclo[3.2.1]oct-8-y])propionitrile was carried out with the reference to Embodiment 88.

MS *m*/*z* (ESI): 433.2 [M+H]⁺.

### Embodiment 96

### 5-(((3-exo)-8-(2-cyanoethyl)-8-azabicyclo[3.2.1]oct-3-yl)amino)-7-((5-methyl-1H-pyrazol-3-yl)amino)-1,6-diazanaphthalen-2-formonitrile

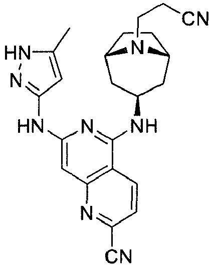

5-(((3-*exo*)-8-(2-cyanoethyl)-8-azabicyclo[3.2.1]oct-3-yl)amino)-7-((5-methyl-1H-pyrazol-3-yl)amino)-1,6-diazanaphthalen-2-formonitrile was carried out with the reference to Embodiment 88.

MS *m*/*z* (ESI): 428.2 [M+H]⁺.

### Embodiment 97

### 3-((3-exo)-3-((3-fluoro-7-((5-methyl-1H-pyrazol-3-yl)amino)-1,6-diazanaphthalen-5-yl)amino)-8-azabicyclo[3.2.1]oct-8-yl)propionitrile

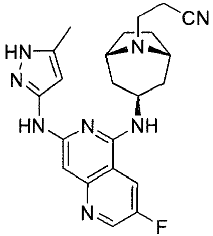

3-((3-*exo*)-3-((3-fluoro-7-((5-methyl-1H-pyrazol-3-yl)amino)-1,6-diazanaphthalen-5-yl)amino)-8-azabicyclo[3.2.1]oct-8-yl)propionitrile was carried out with the reference to Embodiment 88.

MS *m*/*z* (ESI): 421.2 [M+H]⁺.

### Embodiment 98

### 3-((3-exo)-3-((4-hydroxy-7-((5-methyl-1H-pyrazol-3-yl)amino)-1,6-diazanaphthalen-5-yl)amino)-8-azabicyclo[3.2.1]oct-8-yl)propionitrile

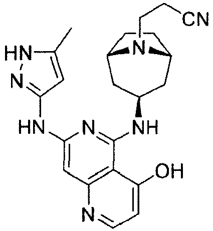

3-((3-*exo*)-3-((4-hydroxy-7-((5-methyl-1H-pyrazol-3-yl)amino)-1,6-diazanaphthalen-5-yl)amino)-8-azabicyclo[3.2.1]oct-8-yl)propionitrile was carried out with the reference to Embodiment 88.

MS *m*/*z* (ESI): 419.2 [M+H]⁺.

### Embodiment 99

### 3-((3-exo)-3-((8-methyl-7-((5-methyl-1H-pyrazol-3-yl)amino)-1,6-diazanaphthalen-5-yl)amino)-8-azabicyclo[3.2.1]oct-8-yl)propionitrile

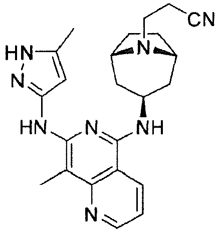

3-((3-*exo*)-3-((8-methyl-7-((5-methyl-1H-pyrazol-3-yl)amino)-1,6-diazanaphthalen-5-yl)amino)-8-azabicyclo[3.2.1]oct-8-yl)propionitrile was carried out with the reference to Embodiment 88.

MS *m*/*z* (ESI): 417.2 [M+H]⁺.

### Embodiment 100

### 3-((3-exo)-3-((8-methoxyl-7-((5-methyl-1H-pyrazol-3-yl)amino)-1,6-diazanaphthalen-5-yl)amino)-8-azabicyclo[3.2.1]oct-8-yl)propionitrile

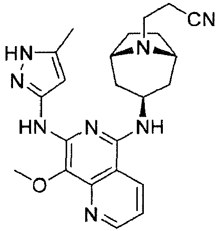

3-((3-*exo*)-3-((8-methoxyl-7-((5-methyl-1H-pyrazol-3-yl)amino)-1,6-diazanaphthalen-5-yl)amino)-8-azabicyclo[3.2.1]oct-8-yl)propionitrile was carried out with the reference to Embodiment 88.

MS *m*/*z* (ESI): 433.2 [M+H]⁺.

### Embodiment 101

### 5-(((3-exo)-8-(2-cyanoethyl)-8-azabicyclo[3.2.1]oct-3-yl)amino)-7-((5-methyl-1H-pyrazol-3-yl)amino)-1,6-diazanaphthalen-8-formonitrile

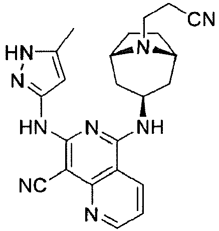

5-(((3-*exo*)-8-(2-cyanoethyl)-8-azabicyclo[3.2.1]oct-3-yl)amino)-7-((5-methyl-1H-pyrazol-3-yl)amino)-1,6-diazanaphthalen-8-formonitrile was carried out with the reference to Embodiment 88.

MS *m*/*z* (ESI): 428.2 [M+H]⁺.

### Embodiment 102

### 3-((3-exo)-3-((4-((5-methyl-1H-pyrazol-3-yl)amino)-6,7-2H-5H-pyrano[2,3-d]pyrimidin-2-yl)amino)-8-azabicyclo[3.2.1]oct-8-yl)propionitrile

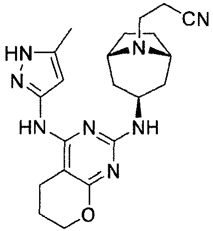

3-((3-*exo*)-3-((4-((5-methyl-1H-pyrazol-3-yl)amino)-6,7-2H-5H-pyrano[2,3-d]pyrimidin-2-yl)amino)-8-azabicyclo[3.2.1]oct-8-yl)propionitrile was carried out with the reference to Embodiment 4.

MS *m*/*z* (ESI): 409.2 [M+H]⁺.

### Embodiment 103

### 3-((3-exo)-3-((2-((5-methyl-1H-pyrazol-3-yl)amino)-6,7-2H-5H-pyrano[2,3-d]pyrimidin-4-yl)amino)-8-azabicyclo[3.2.1]oct-8-yl)propionitrile

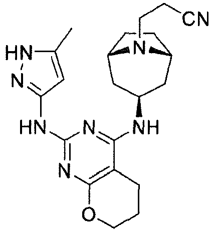

### 3-((3-exo)-3-((2-((5-methyl-1H-pyrazol-3-yl)amino)-6,7-2H-5H-pyrano[2,3-d]pyrimidin-4-yl)amino)-8-azabicyclo[3.2.1]oct-8-yl)propionitrile was carried out with the reference to Embodiment 39.

MS *m*/*z* (ESI): 409.2 [M+H]⁺.

### Embodiment 104

### 3-((3-exo)-3-((2-((5-methyl-1H-pyrazol-3-y|)amino)-6,7-2H-[1,4]dioxino[2,3-d]pyrimidin-4-yl)amino)-8-azabicyclo[3.2.1]oct-8-yl)propionitrile

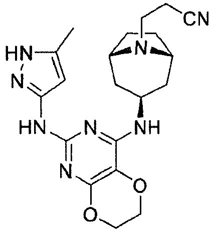

3-((3-*exo*)-3-((2-((5-methyl-1H-pyrazol-3-yl)amino)-6,7-2H-[1,4]dioxino[2,3-d]pyrimidin-4-yl)amino)-8-azabicyclo[3.2.1]oct-8-yl)propionitrile was carried out with the reference to Embodiment 39.

MS *m*/*z* (ESI): 411.2 [M+H]⁺.

### Embodiment 105

### 3-(5-((7-((5-methyl-1H-pyrazol-3-yl)amino)-1,6-diazanaphthalen-5-yl)amino)-2-azatricyclic[3.3.1.13,7]dec-2-yl)propionitrile

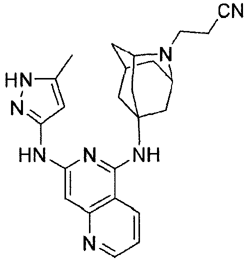

3-(5-((7-((5-methyl-1H-pyrazol-3-yl)amino)-1,6-diazanaphthalen-5-yl)amino)-2-azatricyclic[3.3.1.13,7]dec-2-yl)propionitrile was carried out with the reference to Embodiment 5.

MS m/z (ESI): 429.2 [M+H]⁺.

### Embodiment 106

### 3-((3-exo)-3-((7-((5-methyl-1H-pyrazol-3-yl)amino)-1,6-diazanaphthalen-5-yl)amino)-6-azabicyclo[3.1.1]hept-6-yl)propionitrile

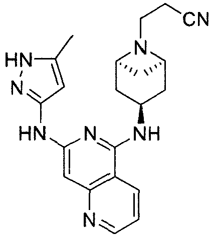

3-((3-exo)-3-((7-((5-methyl-1H-pyrazol-3-yl)amino)-1,6-diazanaphthalen-5-yl)amino)-6-azabicyclo[3.1.1]hept-6-yl)propionitrile was carried out with the reference to Embodiment 5.

MS *m*/*z* (ESI): 389.2 [M+H]⁺.

### Embodiment 107

### 3-((1S,4S,SS)-5-((7-((5-methyl-1H-pyrazol-3-yl)amino)-1,6-diazanaphthalen-5-yl)amino)-2-azabicyclo[2.2.2]oct-2-yl)propionitrile

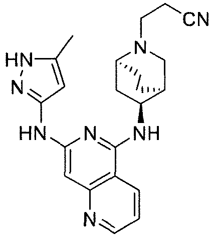

3-((1S,4S,SS)-5-((7-((5-methyl-1H-pyrazol-3-yl)amino)-1,6-diazanaphthalen-5-yl)amino)-2-azabicyclo[2.2.2]oct-2-yl)propionitrile was carried out with the reference to Embodiment 5.

MS *m*/*z* (ESI): 403.2 [M+H]⁺.

### Embodiment 108

### 3-((1S,4S,5S)-5-((7-((5-methyl-1H-pyrazol-3-yl)amino)-1,6-diazanaphthalen-5-yl)amino)-2-azabicyclo[2.2.1]hept-2-yl)propionitrile

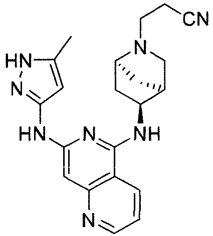

3-((1S,4S,5S)-5-((7-((5-methyl-1H-pyrazol-3-yl)amino)-1,6-diazanaphthalen-5-yl)amino)-2-azabicyclo[2.2.1]hept-2-yl)propionitrile was carried out with the reference to Embodiment 5.

MS *m*/*z* (ESI): 389.2 [M+H]⁺.

### Embodiment 109

### 3-(3-(7-((5-methyl-1H-pyrazol-3-yl)amino)-1,6-diazanaphthalen-5-yl)-3,8-diazabicyclo[3.2.1]oct-8-yl)propionitrile

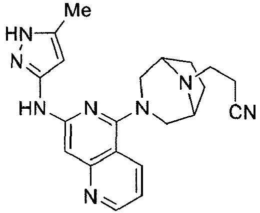

### Step 1: preparation of tert-butyl-3-(7-chloro-1,6-diazanaphthalen-5-yl)-3,8-diazabicyclo[3.2.1]oct-8-carboxylate

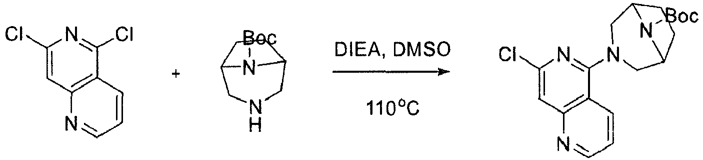

Tert-butyl-3,8-diazabicyclo[3.2.1]oct-8-carboxylate (119 mg, 0.53 mmol), 5,7-dichloro-1,6-diazanaphthalen(100 mg, 0.5 mmol) and diisopropylethylamine(95 mg, 1.5 mmol) were dissolved in DMSO(4 mL). The mixture was stirred at room temperature for dissolution, then heated to 110°C and stirred for 22 hours. The reaction mixture was diluted with ethyl acetate. The organic phase was washed with saturated sodium chloride aqueous solution, dried over anhydrous sodium sulfate, filtered, and then concentrated under reduced pressure to remove the organic solvent. The resulting product was purified by silica gel column chromatography to obtain the title compound (173 mg, 92%).

¹H NMR (400 MHz, DMSO-*d*₆) *δ* 8.97 (dd, *J* = 4.1, 1.1 Hz, 1H), 8.47 (d, *J*= 8.4 Hz, 1H), 7.53 (dd, *J* = 8.5, 4.2 Hz, 1H), 7.37 (s, 1H), 4.22 (s, 2H), 3.86 (d, *J* = 12.2 Hz, 2H), 3.34 (s, 1H), 3.31-3.29 (m, 1H), 1.96-1.80 (m, 4H), 1.44 (s, 9H).

MS *m*/*z* (ESI): 375.2 [M+H]⁺.

### Step 2: preparation of 3-(3-(7-chloro-1,6-diazanaphthalen-5-yl)-3,8-diazabicyclo[3.2.1]oct-8-yl)propionitrile

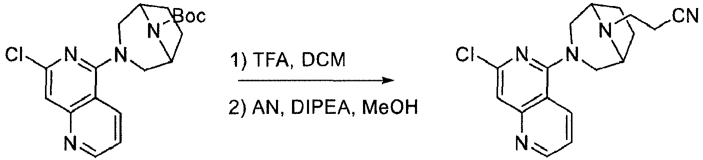

Tert-butyl-3-(7-chloro-1,6-diazanaphthalen-5-yl)-3,8-diazabicyclo[3.2.1]oct-8-carboxylate (169 mg, 0.45 mmol) was dissolved in dichloromethane (2 mL), trifluoroacetate acid (1 mL) was added thereto with stirring. The resulting mixture was stirred at room temperature for 30 minutes, and then concentrated under reduced pressure to obtain brown oil. The obtained brown oil was dissolved in anhydrous methanol (2 mL), followed by successive addition of diisopropylethylamine (0.745 mL, 4.5 mmol) and acrylonitrile (0.036 mL, 0.54 mmol) at room temperature with stirring. The mixture was stirred at room temperature for 12 hours, and then concentrated under reduced pressure to obtain the crude product, which was purified by silica gel chromatography to obtain the title compound (135 mg, 91%).

¹H NMR (400 MHz, DMSO-*d*₆) *δ* 8.94 (dd, *J* = 4.1, 1.1 Hz, 1H), 8.43 (d, *J*= 8.3 Hz, 1H), 7.50 (dd, *J* = 8.5, 4.2 Hz, 1H), 7.29 (s, 1H), 3.81 (d, *J=* 10.2 Hz, 2H), 3.36 (d, *J* = 11.6 Hz, 4H), 2.67 (t, *J=* 6.4 Hz, 2H), 2.58 (t, *J=* 6.4 Hz, 2H), 1.85 (dd, *J=* 12.4, 6.7 Hz, 2H), 1.80-1.72 (m, 2H).

MS *m*/*z* (ESI): 328.2 [M+H]⁺.

### Step 3: preparation of 3-(3-(7-((5-methyl-1H-pyrazol-3-yl)amino)-1,6-diazanaphthalen-5-yl)-3,8-diazabicyclo[3.2.1]oct-8-yl)propionitrile

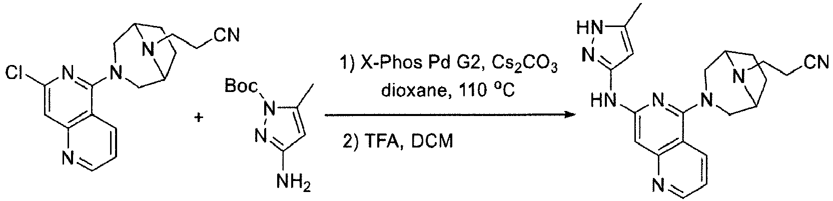

3-(3-(7-Chloro-1,6-diazanaphthalen-5-yl)-3,8-diazabicyclo[3.2.1]oct-8-yl)propionitrile (50 mg, 0.153 mmol), tert-butyl 3-amino-5-methyl-1H-pyrazol-1-carboxylate (36 mg, 0.183 mmol), chloro(2-dicyclohexylphosphino-2',4',6'-triisopropyl-1,1'-biphenyl)[2-(2'-amino-1,1'-biphenyl)]palladium(II) (7 mg, 0.009 mmol) and cesium carbonate(65 mg, 0.20 mmol) were added to ultra-dry 1,4-dioxane (5 mL), and the reaction mixture was filled with dry nitrogen. The reaction mixture was then sealed, heated to 110°C, stirred for 36 hours, cooled to room temperature, and filtered. The filtrate was concentrated under reduced pressure. The mixture was dissolved in dichloromethane (2 mL), followed by addition of trifluoroacetate acid (1 mL). The mixture was stirred at room temperature for 30 minutes. The mixture was concentrated under reduced pressure, and the residue was purified by prep-HPLC to obtain the title compound (2.7 mg, 5%).

¹H NMR (400 MHz, CD₃OD) *δ* 8.61 (d, *J*= 2.8 Hz, 1H), 8.32 (dd, *J* = 8.3, 2.6 Hz, 1H), 7.22-6.99 (m, 2H), 6.07 (s, 1H), 3.73 (dd, *J*= 11.7, 1.5 Hz, 2H), 3.51-3.36 (m, 4H), 2.69 (qd, *J* = 8.2, 1.7 Hz, 4H), 2.29 (s, 3H), 2.04 (s, 4H).

MS *m*/*z* (ESI): 389.3 [M+H]⁺.

### Embodiment 110

### 3-((3-(7-((5-methyl-1H-pyrazol-3-yl)amino)-1,6-diazanaphthalen-5-yl)-3-azabicyclo[3.2.1]oct-8-yl)amino)propionitrile

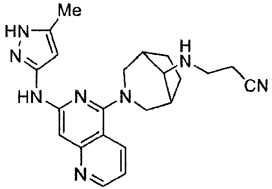

The preparation of 3-((3-(7-((5-methyl-1H-pyrazol-3-yl)amino)-1,6-diazanaphthalen-5-yl)-3-azabicyclo[3.2.1]oct-8-yl)amino)propionitrile was carried out with the reference to Embodiment 109.

MS *m*/*z* (ESI): 403.2 [M+H]⁺.

### Embodiment 111

### 3-((3-exo)-3-(methyl(7-((5-methyl-1H-pyrazol-3-yl)amino)-1,6-diazanaphthalen-5-yl)amino)-8-azabicyclo[3.2.1]oct-8-yl)propionitrile

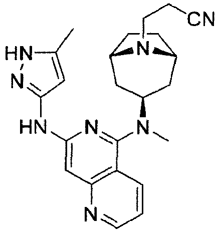

### Step 1: preparation of tert-butyl (3-exo)-3-((7-chloro-1,6-diazanaphthalen-5-yl)(methyl)amino)-8-azabicyclo[3.2.1]oct-8-carboxylate

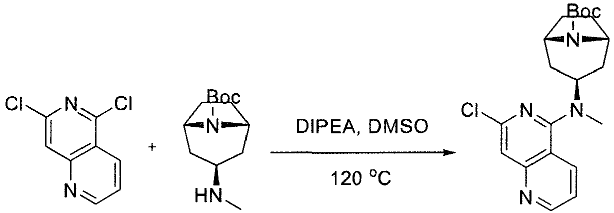

5,7-Dichloro-1,6-diazanaphthalen (200 mg, 1.0 mmol), tert-butyl (3-*exo*)-3-(methylamino)-8-azabicyclo[3.2.1]oct-8-carboxylate (264 mg, 1.1 mmol) and DIPEA (0.5 mL, 3.0 mmol) were dissolved in dimethyl sulfoxide (3 mL). The reaction mixture was then heated to 120 °C and stirred for 30 hours. Then the mixture was cooled to room temperature, and diluted with ethyl acetate. The organic phase was washed with saturated sodium chloride aqueous solution, dried over anhydrous sodium sulfate, filtered, concentrated, and the residue was used directly in the next step.

MS *m*/*z* (ESI): 403.1 [M+H]⁺.

### Step 2: preparation of 3-((3-exo)-3-((7-chloro-1,6-diazanaphthalen-5-yl)(methyl)amino)-8-azabicyclo[3.2.1]oct-8-yl)propionitrile

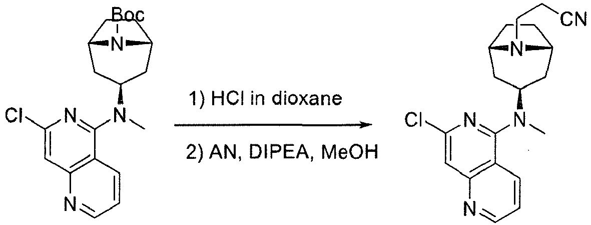

Tert-butyl (3-exo)-3-((7-chloro-1,6-diazanaphthalen-5-yl)(methyl)amino)-8-azabicyclo[3.2.1]oct-8-carboxylate was dissolved in 4M HCl in 1,4-dioxane (10 mL). The resulting reaction mixture was stirred at room temperature for 30 minutes, and concentrated under reduced pressure. The oily residue was dissolved in anhydrous methanol (15 mL), followed by successive addition of DIPEA (8.24 mL, 50 mmol) and acrylonitrile (1.32 mL, 20 mmol). The resulting reaction mixture was stirred at room temperature for 1 hour, and concentrated under reduced pressure to remove the solvent. The residue was purified by silica gel chromatography to obtain the title compound (242 mg, 68%).

MS *m*/*z* (ESI): 356.1 [M+H]⁺.

### Step 3: preparation of 3-((3-exo)-3-(methyl(7-((5-methyl-1H-pyrazol-3-yl)amino)-1,6-diazanaphthalen-5-yl)amino)-8-azabicyclo[3.2.1]oct-8-yl)propionitrile

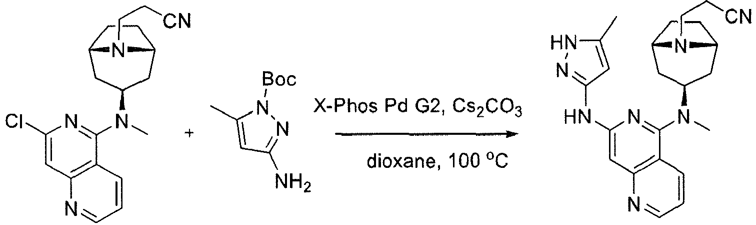

3-((3-*exo*)-3-((7-chloro-1,6-diazanaphthalen-5-yl)(methyl)amino)-8-azabicyclo[3.2.1]oct-8-yl)propionitrile (50 mg, 0.14 mmol), tert-butyl3-amino-5-methyl-1H-pyrazol-1-carboxylate (42 mg, 0.21 mmol), chloro(2-dicyclohexylphosphino-2',4',6'-triisopropyl-1,1'-biphenyl)[2-(2'-amino-1,1'-biphenyl)]palladium(II) (24 mg, 0.03 mmol) and cesium carbonate (138 mg, 0.42 mmol) were added to ultra-dry 1, 4-dioxane (5 mL), and the reaction mixture was filled with dry nitrogen. The reaction mixture was then sealed, heated to 100 °C, stirred for 26 hours, cooled to room temperature, and filtered. The filtrate was concentrated under reduced pressure, and then purified by prep-HPLC to obtain the title compound (23.7 mg, 41%).

¹H NMR (400 MHz, DMSO-*d*₆) *δ* 11.76 (s, 1H), 8.82 (s, 1H), 8.65 (dd, *J* = 4.1, 1.3 Hz, 1H), 8.13 (d, *J* = 8.1 Hz, 1H), 7.19 (s, 1H), 7.06 (dd, *J* = 8.3, 4.2 Hz, 1H), 5.96 (s, 1H), 4.27-4.14 (m, 1H), 3.33-3.31 (m, 2H), 2.93 (s, 3H), 2.61 (dd, *J* = 11.0, 5.0 Hz, 4H), 2.20 (s, 3H), 1.94 (t, *J* = 11.1 Hz, 2H), 1.87-1.79 (m, 2H), 1.68-1.60 (m, 2H), 1.52 (d, *J* = 7.7 Hz, 2H).

MS *m*/*z* (ESI): 417.3 [M+H]⁺.

### Embodiment 112

### 3-((3-exo)-3-((7-((5-methyl-1H-pyrazol-3-yl)amino)-1,6-diazanaphthalen-5-yl)oxo)-8-azabicyclo[3.2.1]oct-8-yl)propionitrile

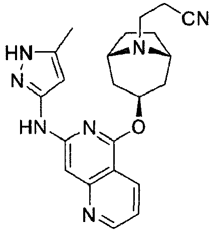

The preparation of 3-((3-*exo*)-3-((7-((5-methyl-1H-pyrazol-3-yl)amino)-1,6-diazanaphthalen-5-yl)oxo)-8-azabicyclo[3.2.1]oct-8-yl)propionitrile was carried out with the reference to Embodiment 5.

MS *m*/*z* (ESI): 404.2 [M+H]⁺.

### Embodiment 113

### 3-((3-exo)-3-((7-((5-methyl-1H-pyrazol-3-yl)amino)-1,6-diazanaphthalen-5-yl)methyl)-8-azabicyclo[3.2.1]oct-8-yl)propionitrile

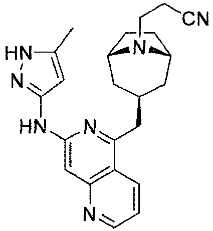

The preparation of 3-((3-*exo*)-3-((7-((5-methyl-1H-pyrazol-3-yl)amino)-1,6-diazanaphthalen-5-yl)methyl)-8-azabicyclo[3.2.1]oct-8-yl)propionitrile was carried out with the reference to Embodiment 111.

MS *m*/*z* (ESI): 402.2 [M+H]⁺.

### Embodiment 114

### 3-(3-((7-((5-methyl-1H-pyrazol-3-yl)amino)-1,6-diazanaphthalen-5-yl)methyl)-3,8-diazabicyclo[3.2.1]oct-8-yl)propionitrile

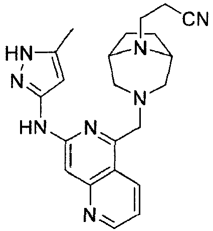

The preparation of 3-(3-((7-((5-methyl-1H-pyrazol-3-yl)amino)-1,6-diazanaphthalen-5-yl)methyl)-3,8-diazabicyclo[3.2.1]oct-8-yl)propionitrile was carried out with the reference to Embodiment 111.

MS m/z (ESI): 403.2 [M+H]⁺.

### Embodiment 115

### 3-(3-((7-((5-methyl-1H-pyrazol-3-yl)amino)-1,6-diazanaphthalen-5-yl)sulfonyl)-3,8-diazabicyclo[3.2.1]oct-8-yl)propionitrile

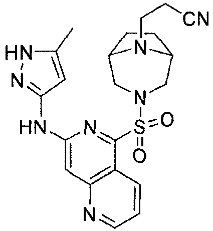

The preparation of 3-(3-((7-((5-methyl-1H-pyrazol-3-yl)amino)-1,6-diazanaphthalen-5-yl)sulfonyl)-3,8-diazabicyclo[3.2.1]oct-8-yl)propionitrile was carried out with the reference to Embodiment 111.

MS *m*/*z* (ESI): 453.2 [M+H]⁺.

### Embodiment 116

### 3-((3-exo)-3-(7-((5-methyl-1H-pyrazol-3-yl)amino)-1,6-diazanaphthalen-5-carbonyl)-8-azabicyclo[3.2.1]oct-8-yl)propionitrile

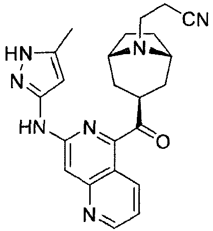

The preparation of 3-((3-*exo*)-3-(7-((5-methyl-1H-pyrazol-3-yl)amino)-1,6-diazanaphthalen-5-carbonyl)-8-azabicyclo[3.2.1]oct-8-yl)propionitrile was carried out with the reference to Embodiment 111.

MS *m*/*z* (ESI): 416.2 [M+H]⁺.

### Embodiment 117

### 3-((3-exo)-3-((S)-hydroxyl(7-((5-methyl-1H-pyrazol-3-yl)amino)-1,6-diazanaphthalen-5-yl)methyl)-8-azabicyclo[3.2.1]oct-8-yl)propionitrile

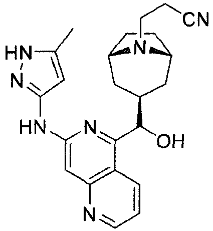

The preparation of 3-((3-*exo*)-3-((S)-hydroxyl(7-((5-methyl-1H-pyrazol-3-yl)amino)-1,6-diazanaphthalen-5-yl)methyl)-8-azabicyclo[3.2.1]oct-8-yl)propionitrile was carried out with the reference to Embodiment 111.

MS *m*/*z* (ESI): 418.2 [M+H]⁺.

### Embodiment 118

### N7-(5-methyl-1H-pyrazol-3-yl)-N5-((3-exo)-8-(oxetan-3-ylmethyl)-8-azabicyclo[3.2.1]oct-3-yl)-1,6-diazanaphthalen-5,7-diamine

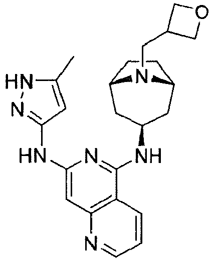

The preparation of N7-(5-methyl-1H-pyrazol-3-yl)-N5-((3-*exo*)-8-(oxetan-3-ylmethyl)-8-azabicyclo[3.2.1]oct-3-yl)-1,6-diazanaphthalen-5,7-diamine was carried out with the reference to Embodiment 111.

MS *m*/*z* (ESI): 420.2 [M+H]⁺.

### Embodiment 119

### (cis)-3-((3-exo)-3-((7-((5-methyl-1H-pyrazol-3-yl)amino)-1,6-diazanaphthalen-5-yl)amino)-8-azabicyclo[3.2.1]oct-8-yl)cyclobutane-1-formonitrile

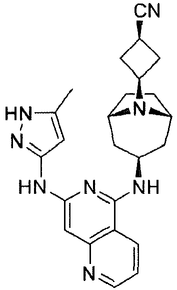

The preparation of (*cis*)-3-((3-*exo*)-3-((7-((5-methyl-1H-pyrazol-3-yl)amino)-1,6-diazanaphthalen-5-yl)amino)-8-azabicyclo[3.2.1]oct-8-yl)cyclobutane-1-formonitrile was carried out with the reference to Embodiment 111.

MS *m*/*z* (ESI): 429.2 [M+H]⁺.

### Embodiment 120

### (trans)-3-((3-exo)-3-((7-((5-methyl-1H-pyrazol-3-yl)amino)-1,6-diazanaphthalen-5-yl)amino)-8-azabicyclo[3.2.1]oct-8-yl)cyclobutane-1-formonitrile

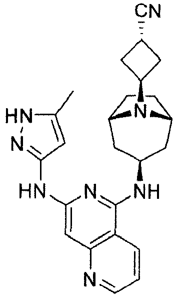

The preparation of (*trans*)-3-((3-*exo*)-3-((7-((5-methyl-1H-pyrazol-3-yl)amino)-1,6-diazanaphthalen-5-yl)amino)-8-azabicyclo[3.2.1]oct-8-yl)cyclobutane-1-formonitrile was carried out with the reference to Embodiment 111.

MS *m*/*z* (ESI): 429.2 [M+H]⁺.

### Embodiment 121

### (cis)-3-(((3-exo)-3-((7-((5-methyl-1H-pyrazol-3-yl)amino)-1,6-diazanaphthalen-5-yl)amino)-8-azabicyclo[3.2.1]oct-8-yl)methyl)cyclobutane-1-formonitrile

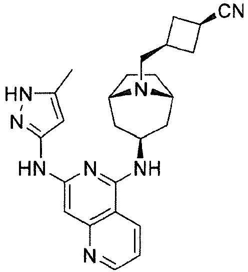

The preparation of (*cis*)-3-(((3-*exo*)-3-((7-((5-methyl-1H-pyrazol-3-yl)amino)-1,6-diazanaphthalen-5-yl)amino)-8-azabicyclo[3.2.1]oct-8-yl)methyl)cyclobutane-1-formonitrile was carried out with the reference to Embodiment 111.

MS *m*/*z* (ESI): 443.3 [M+H]⁺.

### Embodiment 122

### (trans)-3-(((3-exo)-3-((7-((5-methyl-1H-pyrazol-3-yl)amino)-1,6-diazanaphthalen-5-yl)amino)-8-azabicyclo[3.2.1]oct-8-yl)methyl)cyclobutane-1-formonitrile

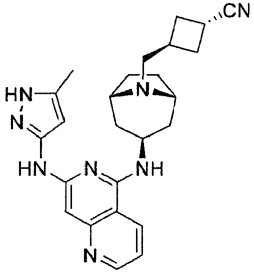

The preparation of (*trans*)-3-(((3-*exo*)-3-((7-((5-methyl-1H-pyrazol-3-yl)amino)-1,6-diazanaphthalen-5-yl)amino)-8-azabicyclo[3.2.1]oct-8-yl)methyl)cyclobutane-1-formonitrile was carried out with the reference to Embodiment 111.

MS *m*/*z* (ESI): 443.3 [M+H]⁺.

### Embodiment 123

### N5-((3-exo)-8-(azetindin-3-ylmethyl)-8-azabicyclo[3.2.1]oct-3-yl)-N7-(5-methyl-1H-pyrazol-3-yl)-1,6-diazanaphthalen-5,7-diamine

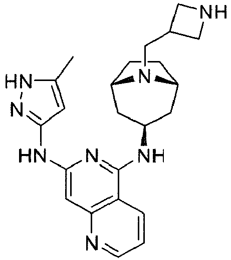

The preparation of N5-((3-*exo*)-8-(azetindin-3-ylmethyl)-8-azabicyclo[3.2.1]oct-3-yl)-N7-(5-methyl-1H-pyrazol-3-yl)-1,6-diazanaphthalen-5,7-diamine was carried out with the reference to Embodiment 111.

MS *m*/*z* (ESI): 419.3 [M+H]⁺.

### Embodiment 124

### N7-(5-methyl-1H-pyrazol-3-yl)-N5-((3-exo)-8-(piperidin-4-ylmethyl)-8-azabicyclo[3.2.1]oct-3-yl)-1,6-diazanaphthalen-5,7-diamine

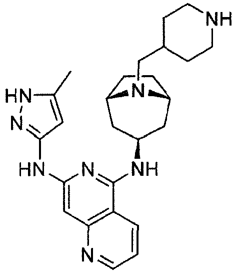

The preparation of N7-(5-methyl-1H-pyrazol-3-yl)-N5-((3-*exo*)-8-(piperidin-4-ylmethyl)-8-azabicyclo[3.2.1]oct-3-yl)-1,6-diazanaphthalen-5,7-diamine was carried out with the reference to Embodiment 111.

MS *m*/*z* (ESI): 447.3 [M+H]⁺.

### Embodiment 125

### 3-(3-((3-exo)-3-((7-((5-methyl-1H-pyrazol-3-yl)amino)-1,6-diazanaphthalen-5-yl)amino)-8-azabicyclo[3.2.1]oct-8-yl)azetindin-1-yl)propionitrile

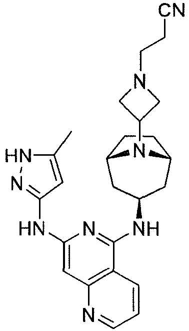

The preparation of 3-(3-((3-*exo*)-3-((7-((5-methyl-1H-pyrazol-3-yl)amino)-1,6-diazanaphthalen-5-yl)amino)-8-azabicyclo[3.2.1]oct-8-yl)azetindin-1-yl)propionitrile was carried out with the reference to Embodiment 111.

MS *m*/*z* (ESI): 458.3 [M+H]⁺.

### Embodiment 126

### 1-(((3-exo)-3-(methyl(7-((5-methyl-1H-pyrazol-3-yl)amino)-1,6-diazanaphthalen-5-yl)amino)-9-azabicyclo[3.3.1]non-9-yl)sulfonyl)azetindin-3-formonitrile

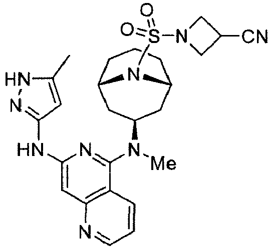

The preparation of 1-(((3-*exo*)-3-(methyl(7-((5-methyl-1H-pyrazol-3-yl)amino)-1,6-diazanaphthalen-5-yl)amino)-9-azabicyclo[3.3.1]non-9-yl)sulfonyl)azetindin-3-formonitrile was carried out with the reference to Embodiment 111.

MS *m*/*z* (ESI): 522.2 [M+H]⁺.

### Embodiment 127

### (3-exo)-N-(2-cyanoethyl)-3-((7-((5-methyl-1H-pyrazol-3-yl)amino)-1,6-diazanaphthalen-5-yl)amino)-9-azabicyclo[3.3.1]non-9-sulfonamide

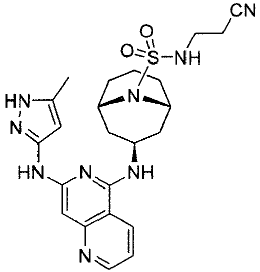

The preparation of (3-*exo*)-N-(2-cyanoethyl)-3-((7-((5-methyl-1H-pyrazol-3-yl)amino)-1,6-diazanaphthalen-5-yl)amino)-9-azabicyclo[3.3.1]non-9-sulfonamide was carried out with the reference to Embodiment 111.

MS *m*/*z* (ESI): 496.2 [M+H]⁺.

### Embodiment 128

### 1-(((3-exo)-3-((7-((5-(hydroxymethyl)-1H-pyrazol-3-yl)amino)-1,6-diazanaphthalen-5-yl)(methyl)amino)-9-azabicyclo[3.3.1]non-9-yl)sulfonyl)azetindin-3-formonitrile

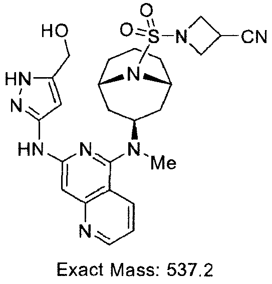

The preparation of 1-(((3-*exo*)-3-((7-((5-(hydroxymethyl)-1H-pyrazol-3-yl)amino)-1,6-diazanaphthalen-5-yl)(methyl)amino)-9-azabicyclo[3.3.1]non-9-yl)sulfonyl)azetindin-3-formonitrile was carried out with the reference to Embodiment 2.

MS *m*/*z* (ESI): 538.2 [M+H]⁺.

### Embodiment 129

### 1-(((3-exo)-3-((7-((5-(hydroxymethyl)-1H-pyrazol-3-yl)amino)-1,6-diazanaphthalen-5-yl)amino)-9-azabicyclo[3.3.1]non-9-yl)sulfonyl)azetindin-3-formonitrile

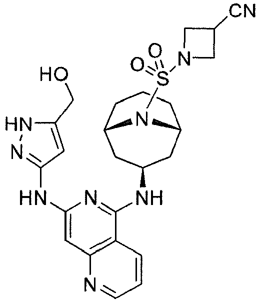

The preparation of 1-(((3-*exo*)-3-((7-((5-(hydroxymethyl)-1H-pyrazol-3-yl)amino)-1,6-diazanaphthalen-5-yl)amino)-9-azabicyclo[3.3.1]non-9-yl)sulfonyl)azetindin-3-formonitrile was carried out with the reference to Embodiment 2.

MS *m*/*z* (ESI): 524.2 [M+H]⁺.

### Embodiment 130

### 1-(((3-exo)-3-((7-((5-(hydroxymethyl)thiazol-2-yl)amino)-1,6-diazanaphthalen-5-yl)amino)-9-azabicyclo[3.3.1]non-9-yl)sulfonyl)azetindin-3-formonitrile

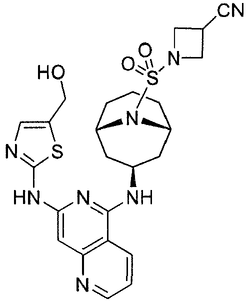

The preparation of 1-(((3-*exo*)-3-((7-((5-(hydroxymethyl)thiazol-2-yl)amino)-1,6-diazanaphthalen-5-yl)amino)-9-azabicyclo [3.3.1]non-9-yl)sulfonyl)azetindin-3-formonitrile was carried out with the reference to Embodiment 2.

MS *m*/*z* (ESI): 541.2 [M+H]⁺.

### Embodiment 131

### 1-(((3-exo)-3-((4-((5-(hydroxymethyl)-1H-pyrazol-3-yl)amino)-7H-pyrrolo[2,3-d]pyrimidin-2-yl)(methyl)amino)-9-azabicyclo[3.3.1]non-9-yl)sulfonyl)azetindin-3-formonitrile

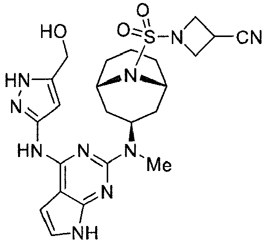

The preparation of 1-(((3-*exo*)-3-((4-((5-(hydroxymethyl)-1H-pyrazol-3-yl)amino)-7H-pyrrolo[2,3-d]pyrimidin-2-yl)(methyl)amino)-9-azabicyclo[3.3.1]non-9-yl)sulfonyl)azetindin-3-formonitrile was carried out with the reference to Embodiment 3.

MS *m*/*z* (ESI): 527.2 [M+H]⁺.

### Embodiment 132

### 1-(((3-exo)-3-((4-((5-(hydroxymethyl)-1H-pyrazol-3-yl)amino)thieno[2,3-d]pyrimidin-2-yl)(methyl)amino)-9-azabicyclo[3.3.1]non-9-yl)sulfonyl)azetindin-3-formonitrile

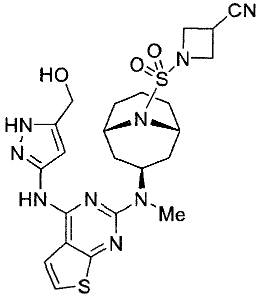

### Step 1: preparation of (3-((2-chlorothieno[2,3-d]pyrimidin-4-yl)amino)-1H-pyrazol-5-yl)methanol

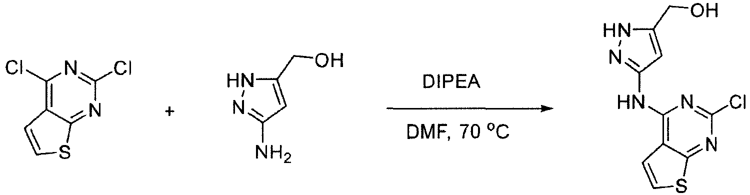

2,4-Dichlorothieno[2,3-d]pyrimidine(100 mg, 0.49 mmol), (3-amino-1H-pyrazol-5-yl)methanol (55 mg, 0.49 mmol) and DIPEA (190 mg, 1.47 mmol) were added to N'N-dimethylformamide(2 mL), and the reaction mixture was stirred at 70 °C overnight. Then the mixture was concentrated under reduced pressure, and the crude product obtained was purified by flash silica gel chromatography to obtain the title compound as a yellow solid (100 mg, 73%).

MS *m*/*z* (ESI):282.0 [M+H]⁺.

### Step 2: preparation of tert-butyl (3-exo)-3-((4-((5-(hydroxymethyl)-1H-pyrazol-3-yl)amino)thieno[2,3-d]pyrimidin-2-yl)(methyl)amino)-9-azabicyclo[3.3.1]non-9-carboxylate

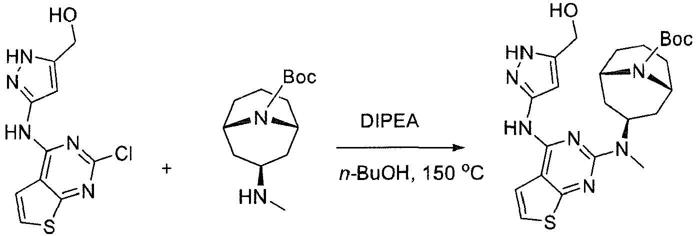

(3-((2-Chlorothieno[2,3-d]pyrimidin-4-yl)amino)-1H-pyrazol-5-yl)methanol (100 mg, 0.36 mmol), tert-butyl (3-*exo*)-3-(methylamino)-9-azabicyclo[3.3.1]non-9-carboxylate (135 mg, 0.53 mmol) and DIPEA (140 mg, 1.08 mmol) were added to n-butanol (2.5 mL). The mixture was mixed evenly, then heated to 150 °C by microwave and reacted for 10 hours. The mixture was then cooled to room temperature, and concentrated under reduced pressure. The crude product obtained was purified by flash silica gel chromatography to obtain the target product as a white solid (70 mg, 39 %).

MS *m*/*z* (ESI): 500.1 [M+H]⁺.

### Step 3: preparation of 1-(((3-exo)-3-((4-((5-(hydroxymethyl)-1H-pyrazol-3-yl)amino)thieno[2,3-d]pyrimidin-2-yl)(methyl)amino)-9-azabicyclo[3.3.1]non-9-yl)sulfonyl)azetindin-3-formonitrile

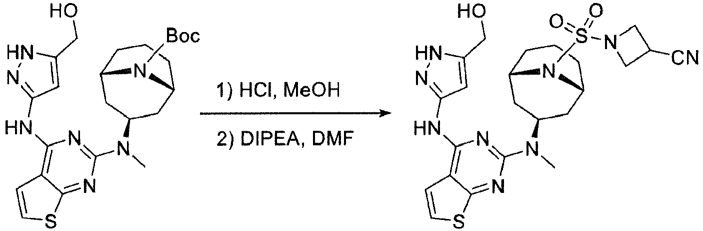

Hydrochloride dioxane (4N, 2.5 mL) was slowly added dropwise to the solution of tert-butyl (3-*exo*)-3-((4-((5-(hydroxymethyl)-1H-pyrazol-3-yl)amino)thieno[2,3-d]pyrimidin-2-yl)(methyl)amino)-9-azabicyclo[3.3.1]non-9-carboxylate (70 mg, 0.14 mmol) in methanol (10 mL). The reaction was carried out at room temperature for 2 hours, and then concentrated under reduced pressure. The crude product was dissolved in DMF(5 mL), followed by successive addition of DIPEA (0.3 mL) and 3-cyanoazacyclobutane-1-sulfonylchlorine (22 mg, 0.12 mmol) at 0 °C under ice water bath condition. The reaction was carried out at room temperature overnight. The reaction mixture was concentrated under reduced pressure, and purified by prep-HPLC to obtain the target compound as a white solid (9.7 mg, 13%).

¹H NMR (400 MHz, DMSO-*d₆*) *δ* 9.81 (s, 1H), 7.68 (d, *J* = 4.4 Hz, 1H),7.04 (d, *J* = 6.0 Hz, 1H), 6.52-6.54 (m, 1H), 5.53-5.55 (m, 1H), 5.33-5.35 (m, 1H), 4.44 (d, *J* = 5.2 Hz, 2H), 4.05-4.01 (m, 4H), 3.94-3.90 (m, 2H), 382-3.79 (m,1 H), 2.89 (d, *J* = 8.4 Hz, 3H), 2.08-1.68 (m, 11H).

MS *m*/*z* (ESI): 544.1 [M+H]⁺.

### Embodiment 133

### 1-(((3-exo)-3-((7-((5-(hydroxymethyl)-1H-pyrazol-3-yl)amino)thiazolo[5,4-d]pyrimidin-5-yl)(methyl)amino)-9-azabicyclo[3.3.1]non-9-yl)sulfonyl)azetindin-3-formonitrile

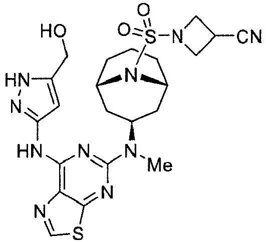

The preparation of 1-(((3-*exo*)-3-((7-((5-(hydroxymethyl)-1H-pyrazol-3-yl)amino)thiazolo[5,4-d]pyrimidin-5-yl)(methyl)amino)-9-azabicyclo[3.3.1]non-9-yl)sulfonyl)azetindin-3-formonitrile was carried out with the reference to Embodiment 2.

MS *m*/*z* (ESI): 545.2 [M+H]⁺.

### Embodiment 134

### 1-(((3-exo)-3-((5-((5-(hydroxymethyl)-1H-pyrazol-3-yl)amino)-1,6-diazanaphthalen-7-yl)(methyl)amino)-9-azabicyclo[3.3.1]non-9-yl)sulfonyl)azetindin-3-formonitrile

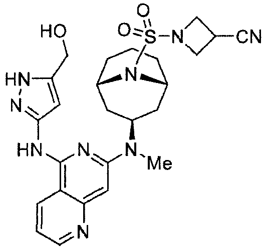

The preparation of 1-(((3-*exo*)-3-((5-((5-(hydroxymethyl)-1H-pyrazol-3-yl)amino)-1,6-diazanaphthalen-7-yl)(methyl)amino)-9-azabicyclo[3.3.1]non-9-yl)sulfonyl)azetindin-3-formonitrile was carried out with the reference to Embodiment 132.

MS *m*/*z* (ESI): 538.2 [M+H]⁺.

### Embodiment 135

### 1-(((3-exo)-3-((7-((5-(hydroxymethyl)-1H-pyrazol-3-yl)amino)pyrido[3,4-b]pyrazin-5-yl)(methyl)amino)-9-azabicyclo[3.3.1]non-9-yl)sulfonyl)azetindin-3-formonitrile

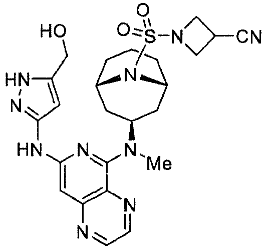

The preparation of 1-(((3-*exo*)-3-((7-((5-(hydroxymethyl)-1H-pyrazol-3-yl)amino)pyrido[3,4-b]pyrazin-5-yl)(methyl)amino)-9-azabicyclo[3.3.1]non-9-yl)sulfonyl)azetindin-3-formonitrile was carried out with the reference to Embodiment 2.

MS *m*/*z* (ESI): 539.2 [M+H]⁺.

### Embodiment 136

### 1-(((3-exo)-3-((4-((5-(hydroxymethyl)thiazol-2-yl)amino)-7H-pyrrolo[2,3-d]pyrimidin-2-yl)(methyl)amino)-9-azabicyclo[3.3.1]non-9-yl)sulfonyl)azetindin-3-formonitrile

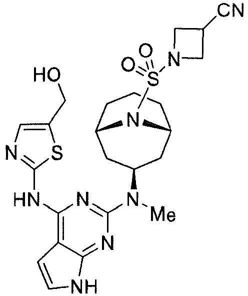

The preparation of 1-(((3-*exo*)-3-((4-((5-(hydroxymethyl)thiazol-2-yl)amino)-7H-pyrrolo[2,3-d]pyrimidin-2-yl)(methyl)amino)-9-azabicyclo[3.3.1]non-9-yl)sulfonyl)azetindin-3-formonitrile was carried out with the reference to Embodiment 3.

MS *m*/*z* (ESI): 544.2 [M+H]⁺.

### Embodiment 137

### 1-(((3-exo)-3-((4-((5-(hydroxymethyl)thiazol-2-yl)amino)thieno[2,3-d]pyrimidin-2-yl)(methyl)amino)-9-azabicyclo [3.3.1]non-9-yl)sulfonyl)azetindin-3 -formonitrile

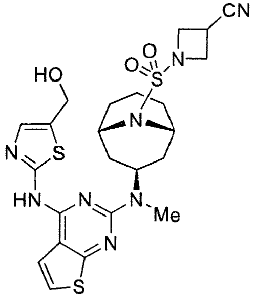

The preparation of 1-(((3-*exo*)-3-((4-((5-(hydroxymethyl)thiazol-2-yl)amino)thieno[2,3-d]pyrimidin-2-yl)(methyl)amino)-9-azabicyclo[3.3.1]non-9-yl)sulfonyl)azetindin-3-formonitrile was carried out with the reference to Embodiment 132.

MS *m*/*z* (ESI): 561.1 [M+H]⁺.

### Embodiment 138

### 1-(((3-exo)-3-((4-((5-(hydroxymethyl)-1,3,4-thiadiazol-2-yl)amino)quinazolin-2-yl)(methyl)amino)-9-azabicyclo[3.3.1]non-9-yl)sulfonyl)azetindin-3-formonitrile

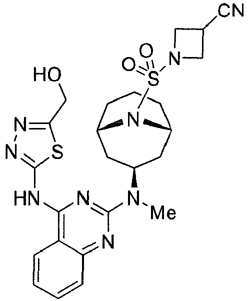

The preparation of 1-(((3-*exo*)-3-((4-((5-(hydroxymethyl)-1,3,4-thiadiazol-2-yl)amino)quinazolin-2-yl)(methyl)amino)-9-azabicyclo[3.3.1]non-9-yl)sulfonyl)azetindin-3-formonitrile was carried out with the reference to Embodiment 132.

MS *m*/*z* (ESI): 556.2 [M+H]⁺.

### Embodiment 139

### 1-(((3-exo)-3-((6-((5-(hydroxymethyl)thiazol-2-yl)amino)-1H-pyrrolo[3,2-c]pyridin-4-yl)amino)-9-azabicyclo[3.3.1]non-9-yl)sulfonyl)azetindin-3-formonitrile

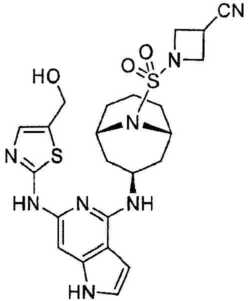

The preparation of 1-(((3-*exo*)-3-((6-((5-(hydroxymethyl)thiazol-2-yl)amino)-1H-pyrrolo[3,2-c]pyridin-4-yl)amino)-9-azabicyclo[3.3.1]non-9-yl)sulfonyl)azetindin-3-formonitrile was carried out with the reference to Embodiment 2.

MS *m*/*z* (ESI): 529.2 [M+H]⁺.

### Embodiment 140

### 1-(((3-exo)-3-((7-((5-(hydroxymethyl)thiazol-2-yl)amino)imidazo[1,2-c]pyrimidin-5-yl)amino)-9-azabicyclo[3.3.1]non-9-yl)sulfonyl)azetindin-3-formonitrile

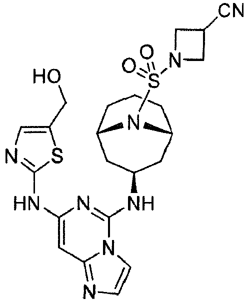

The preparation of 1-(((3-*exo*)-3-((7-((5-(hydroxymethyl)thiazol-2-yl)amino)imidazo[1 ,2-c]pyrimidin-5-yl)amino)-9-azabicyclo[3.3.1]non-9-yl)sulfonyl)azetindin-3-formonitrile was carried out with the reference to Embodiment 2.

MS *m*/*z* (ESI): 530.2 [M+H]⁺.

### Embodiment 141

### 3-((3-exo)-3-((4-((5-(hydroxymethyl)thiazol-2-yl)amino)-7H-pyrrolo[2,3-d]pyrimidin-2-yl)amino)-8-azabicyclo[3.2.1]oct-8-yl)propionitrile

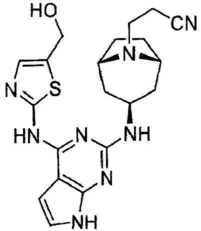

The preparation of 3-((3-*exo*)-3-((4-((5-(hydroxymethyl)thiazol-2-yl)amino)-7H-pyrrolo[2,3-d]pyrimidin-2-yl)amino)-8-azabicyclo[3.2.1]oct-8-yl)propionitrile was carried out with the reference to Embodiment 132.

MS *m*/*z* (ESI): 425.2 [M+H]⁺.

### Embodiment 142

### 3-((3-exo)-3-((4-((5-(hydroxymethyl)thiazol-2-yl)amino)thieno[2,3-d]pyrimidin-2-yl)amino)-8-azabicyclo[3.2.1]oct-8-yl)propionitrile

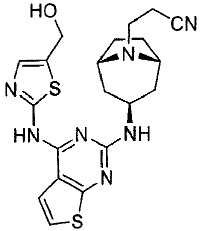

The preparation of 3-((3-exo)-3-((4-((5-(hydroxymethyl)thiazol-2-yl)amino)thieno[2,3-d]pyrimidin-2-yl)amino)-8-azabicyclo[3.2.1]oct-8-yl)propionitrile was carried out with the reference to Embodiment 132.

MS *m*/*z* (ESI): 442.1 [M+H]⁺.

### Embodiment 143

### 3-((3-exo)-3-((4-((5-(hydroxymethyl)thiazol-2-yl)amino)quinazolin-2-yl)amino)-8-azabicyclo[3.2.1]oct-8-yl)propionitrile

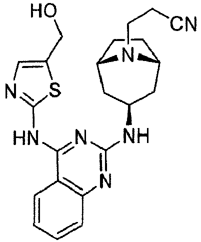

The preparation of 3-((3-*exo*)-3-((4-((5-(hydroxymethyl)thiazol-2-yl)amino)quinazolin-2-yl)amino)-8-azabicyclo[3.2.1]oct-8-yl)propionitrile was carried out with the reference to Embodiment 132.

MS *m*/*z* (ESI): 436.2 [M+H]⁺.

### Embodiment 144

### 3-((3-exo)-3-((6-((5-(hydroxymethyl)thiazol-2-yl)amino)-1H-pyrrolo[3,2-c]pyridin-4-yl)amino)-8-azabicyclo[3.2.1]oct-8-yl)propionitrile

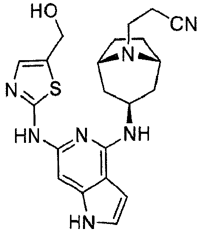

The preparation of 3-((3-*exo*)-3-((6-((5-(hydroxymethyl)thiazol-2-yl)amino)-1H-pyrrolo[3,2-c]pyridin-4-yl)amino)-8-azabicyclo[3.2.1]oct-8-yl)propionitrile was carried out with the reference to Embodiment 2.

MS *m*/*z* (ESI): 424.2 [M+H]⁺.

### Embodiment 145

### 3-((3-exo)-3-((7-((5-(hydroxymethyl)thiazol-2-yl)amino)imidazo[1,2-c]pyrimidin-5-yl)amino)-8-azabicyclo[3.2.1]oct-8-yl)propionitrile

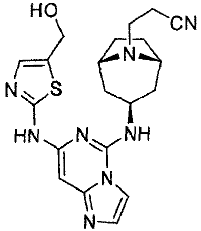

The preparation of 3-((3-*exo*)-3-((7-((5-(hydroxymethyl)thiazol-2-yl)amino)imidazo[1,2-c]pyrimidin-5-yl)amino)-8-azabicyclo[3.2.1]oct-8-yl)propionitrile was carried out with the reference to Embodiment 2.

MS *m*/*z* (ESI): 425.2 [M+H]⁺.

### Embodiment 146

### N-(5-(((3-exo)-8-(2-cyanoethyl)-8-azabicyclo[3.2.1]oct-3-yl)amino)-1,6-diazanaphthalen-7-yl)-5-methyl-1H-pyrazol-3-formamide

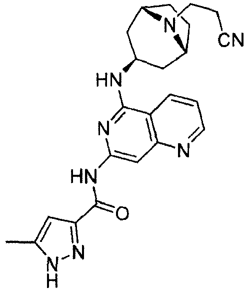

The preparation of N-(5-(((3-*exo*)-8-(2-cyanoethyl)-8-azabicyclo[3.2. 1]oct-3-yl)amino)-1,6-diazanaphthalen-7-yl)-5-methyl-1H-pyrazol-3-formamide was carried out with the reference to Embodiment 1.

MS *m*/*z* (ESI): 431.2 [M+H]⁺.

### Embodiment 147

### 3-((3-exo)-3-((2-(5-methyl-1H-pyrazol-3-yl)-1H-benzo[d]imidazol-4-yl)amino)-8-azabicyclo[3.2.1]oct-8-yl)propionitrile

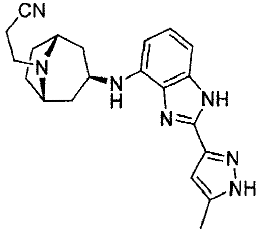

The preparation of 3-((3-*exo*)-3-((2-(5-methyl-1H-pyrazol-3-yl)-1H-benzo[d]imidazol-4-yl)amino)-8-azabicyclo[3.2.1]oct-8-yl)propionitrile was carried out with the reference to Embodiment 3.

MS *m*/*z* (ESI): 376.2 [M+H]⁺.

### Embodiment 148

### 3-((3-exo)-3-(3-((5-methyl-1H-pyrazol-3-yl)amino)-5H-pyrrolo[2,3-b]pyrazin-5-yl)-8-azabicyclo[3.2.1]oct-8-yl)propionitrile

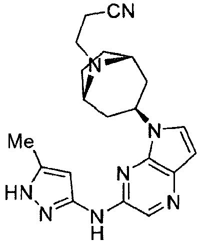

The preparation of 3-((3-*exo*)-3-(3-((5-methyl-1H-pyrazol-3-yl)amino)-5H-pyrrolo[2,3-b]pyrazin-5-yl)-8-azabicyclo[3.2.1]oct-8-yl)propionitrile was carried out with the reference to Embodiment 111.

MS *m*/*z* (ESI): 377.2 [M+H]⁺.

### Embodiment 149

### 3-((3-exo)-3-(6-((5-methyl-1H-pyrazol-3-yl)amino)-1H-pyrazolo[3,4-b]pyrazin-1-yl)-8-azabicyclo[3.2.1]oct-8-yl)propionitrile

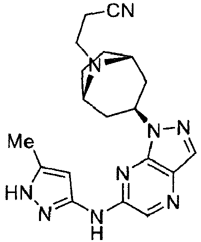

The preparation of 3-((3-*exo*)-3-(6-((5-methyl-1H-pyrazol-3-yl)amino)-1H-pyrazolo[3,4-b]pyrazin-1-yl)-8-azabicyclo[3.2.1]oct-8-yl)propionitrile was carried out with the reference to Embodiment 111.

MS *m*/*z* (ESI): 378.2 [M+H]⁺.

### Embodiment 150

### 3-((3-exo)-3-(6-((5-methyl-1H-pyrazol-3-yl)amino)-2-carbonyl-2,3-2H-1H-imidazo[4,5-b]pyrazin-1-yl)-8-azabicyclo[3.2.1]oct-8-yl)propionitrile

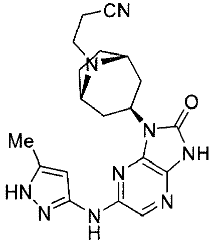

The preparation of 3-((3-*exo*)-3-(6-((5-methyl-1H-pyrazol-3-yl)amino)-2-carbonyl-2,3-2H-1H-imidazo[4,5-b]pyrazin-1-yl)-8-azabicyclo[3.2.1]oct-8-yl)propionitrile was carried out with the reference to Embodiment 111.

MS *m*/*z* (ESI): 394.2 [M+H]⁺.

### Embodiment 151

### 3-((3-exo)-3-(6-((5-methyl-1H-pyrazol-3-yl)amino)-1H-imidazo[4,5-b]pyrazin-1-yl)-8-azabicyclo[3.2.1]oct-8-yl)propionitrile

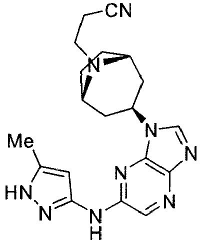

The preparation of 3-((3-*exo*)-3-(6-((5-methyl-1H-pyrazol-3-yl)amino)-1H-imidazo[4,5-b]pyrazin-1-yl)-8-azabicyclo[3.2.1]oct-8-yl)propionitrile was carried out with the reference to Embodiment 111.

MS *m*/*z* (ESI): 378.2 [M+H]⁺.

### Embodiment 152

### 3-((3-exo)-3-(2-methyl-6-((5-methyl-1H-pyrazol-3-yl)amino)-1H-imidazo[4,5-b]pyrazin-1-yl)-8-azabicyclo[3.2.1]oct-8-yl)propionitrile

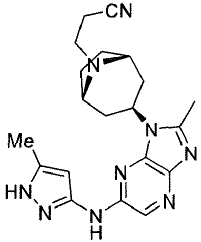

The preparation of 3-((3-*exo*)-3-(2-methyl-6-((5-methyl-1H-pyrazol-3-yl)amino)-1H-imidazo[4,5-b]pyrazin-1-yl)-8-azabicyclo[3.2.1]oct-8-yl)propionitrile was carried out with the reference to Embodiment 111.

MS *m*/*z* (ESI): 392.2 [M+H]⁺.

### Embodiment 153

### 3-((3-exo)-3-(7-methoxyl-5-((5-methyl-1H-pyrazol-3-yl)amino)-3H-imidazo[4,5-b]pyridin-3-yl)-8-azabicyclo[3.2.1]oct-8-yl)propionitrile

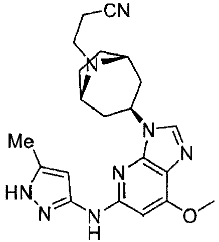

The preparation of 3-((3-*exo*)-3-(7-methoxyl-5-((5-methyl-1H-pyrazol-3-yl)amino)-3H-imidazo[4,5-b]pyridin-3-yl)-8-azabicyclo[3.2.1]oct-8-yl)propionitrile was carried out with the reference to Embodiment 111.

MS *m*/*z* (ESI): 407.2 [M+H]⁺.

### Embodiment 154

### 3-((3-exo)-3-(6-methoxyl-8-methyl-2-((5-methyl-1H-pyrazol-3-yl)amino)-9H-purin-9-yl)-8-azabicyclo[3.2.1]oct-8-yl)propionitrile

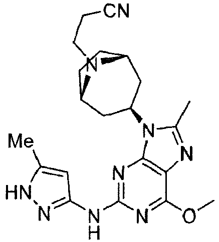

The preparation of 3-((3-*exo*)-3-(6-methoxyl-8-methyl-2-((5-methyl-1H-pyrazol-3-yl)amino)-9H-purin-9-yl)-8-azabicyclo[3.2.1]oct-8-yl)propionitrile was carried out with the reference to Embodiment 111.

MS *m*/*z* (ESI): 422.2 [M+H]⁺.

### Embodiment 155

### 3-((3-exo)-3-(4-methoxyl-2-((5-methyl-1H-pyrazol-3-yl)amino)-7H-pyrrolo[2,3-d]pyrimidin-7-yl)-8-azabicyclo[3.2.1]oct-8-yl)propionitrile

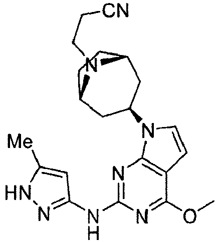

The preparation of 3-((3-*exo*)-3-(4-methoxyl-2-((5-methyl-1H-pyrazol-3-yl)amino)-7H-pyrrolo[2,3-d]pyrimidin-7-yl)-8-azabicyclo[3.2.1]oct-8-yl)propionitrile was carried out with the reference to Embodiment 111.

MS *m*/*z* (ESI): 407.2 [M+H]⁺.

### Embodiment 156

### 3-((3-exo)-3-(2-((5-methyl-1H-pyrazol-3-yl)amino)-6-morpholino-9H-purin-9-yl)-8-azabicyclo[3.2.1]oct-8-yl)propionitrile

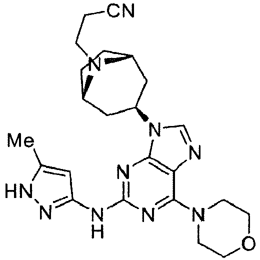

The preparation of 3-((3-*exo*)-3-(2-((5-methyl-1H-pyrazol-3-yl)amino)-6-morpholino-9H-purin-9-yl)-8-azabicyclo[3.2.1]oct-8-yl)propionitrile was carried out with the reference to Embodiment 111.

MS *m*/*z* (ESI): 463.3 [M+H]⁺.

### Embodiment 157

### 3-((1R,5S)-3-(2-((5-methyl-1H-pyrazol-3-yl)amino)imidazo[1,2-b][1,2,4]triazin-7-yl)-3,8-diazabicyclo[3.2.1]oct-8-yl)propionitrile

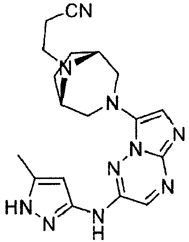

The preparation of 3-((1R,5S)-3-(2-((5-methyl-1H-pyrazol-3-yl)amino)imidazo[1,2-b][1,2,4]triazin-7-yl)-3,8-diazabicyclo[3.2.1]oct-8-yl)propionitrile was carried out with the reference to Embodiment 109.

MS *m*/*z* (ESI): 379.2 [M+H]⁺.

### Embodiment 158

### 3-((1R,5S)-3-(5-((5-methyl-1H-pyrazol-3-yl)amino)-[1,2,4]triazolo[1,5-a]pyridin-2-yl)-3,8-diazabicyclo[3.2.1]oct-8-yl)propionitrile

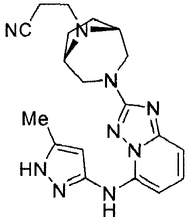

The preparation of 3-((1R,5S)-3-(5-((5-methyl-1H-pyrazol-3-yl)amino)-[1,2,4]triazolo[1,5-a]pyridin-2-yl)-3,8-diazabicyclo[3.2.1]oct-8-yl)propionitrile was carried out with the reference to Embodiment 109.

MS *m*/*z* (ESI): 378.2 [M+H]⁺.

### Embodiment 159

### 3-((1R,5S)-3-(3-((5-methyl-1H-pyrazol-3-yl)amino)quinoxalin-5-yl)-3,8-diazabicyclo[3.2.1]oct-8-yl)propionitrile

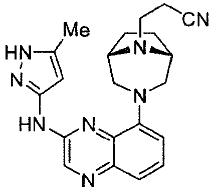

The preparation of 3-((1R,5S)-3-(3-((5-methyl-1H-pyrazol-3-yl)amino)quinoxalin-5-yl)-3,8-diazabicyclo[3.2.1]oct-8-yl)propionitrile was carried out with the reference to Embodiment 109.

MS *m*/*z* (ESI): 389.2 [M+H]⁺.

### Embodiment 160

### 2-((5-(((3-exo)-8-(2-cyanoethyl)-8-azabicyclo[3.2.1]oct-3-yl)amino)-1,6-diazanaphthalen-7-yl)amino)thiazol-5-formonitrile

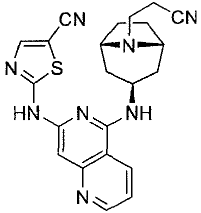

### Step 1: preparation of (3-exo)-3-((7-((5-cyanothiazol-2-yl)amino)-1,6-diazanaphthalen-5-yl)amino)-8-azabicyclo[3.2.1]oct-8-carboxylate

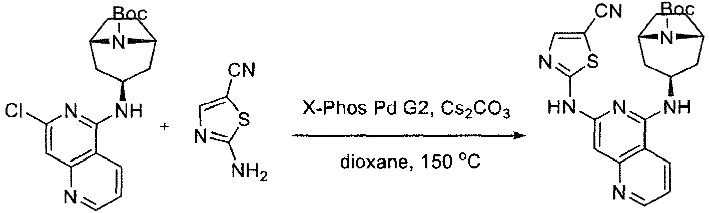

(3-*exo*)-3-((7-Chloro-1,6-diazanaphthalen-5-yl)amino)-8-azabicyclo[3.2.1]oct-8-carboxylate (194 mg, 0.5 mmol), 2-aminothiazol-5-formonitrile (125 mg, 1.0 mmol), chloro(2-dicyclohexylphosphino-2',4',6'-triisopropyl-1,1'-biphenyl)[2-(2'-amino-1,1'-biphenyl)]palladium(II) (196 mg, 0.25 mmol) and cesium carbonate (489 mg, 1.5 mmol) were added to 10 mL ultra-dry 1,4-dioxane. The reaction mixture was filled with dry nitrogen, heated to 150 °C with microwave and stirred for 9 hours. Then the mixture was cooled to room temperature, and filtered. The filtrate was concentrated under reduced pressure, and the residue was purified by silica gel chromatography to obtain the title compound (151.6 mg, 63 %).

MS *m*/*z* (ESI): 478.1 [M+H]⁺.

### Step 2: preparation of 2-((5-(((3-exo)-8-(2-cyanoethyl)-8-azabicyclo[3.2.1]oct-3-yl)amino)-1,6-diazanaphthalen-7-yl)amino)thiazol-5-formonitrile

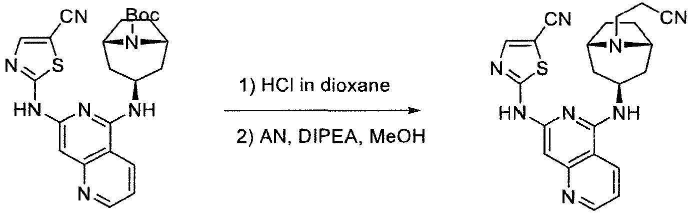

Tert-butyl (3-*exo*)-3-((7-((5-cyanothiazol-2-yl)amino)-1,6-diazanaphthalen-5-yl)amino)-8-azabicyclo[3.2.1]oct-8-carboxylate (180 mg, 0.377 mmol) was dissolved in 4M HCl in 1,4-dioxane (20 mL), and the mixture was stirred at room temperature for 30 minutes. Then the mixture was concentrated under reduced pressure, and the residue was dissolved in anhydrous methanol (10 mL), followed by successive addition of DIPEA (1.86 mL, 11.3 mmol) and acrylonitrile (0.25 mL, 3.77 mmol). The resulting reaction mixture was stirred at room temperature for 105 minutes, and concentrated under reduced pressure to remove the solvent. The residue was successively purified by silica gel chromatography and prep-HPLC to obtain the title compound (8.6 mg, 2.0%).

¹H NMR (400 MHz, DMSO-*d₆*) *δ* 11.91 (s, 1H), 8.76 (d, *J* = 3.2 Hz, 1H), 8.65 (d, *J* = 8.4 Hz, 1H), 8.23 (s, 1H), 7.68 (d, *J* = 8.5 Hz, 1H), 7.23 (dd, *J* = 8.3, 4.3 Hz, 1H), 6.59 (s, 1H), 4.76 (td, *J* = 17.4, 8.7 Hz, 1H), 3.38 (s, 2H), 2.65 (s, 4H), 2.07-1.72 (m, 8H).

MS *m*/*z* (ESI): 431.1 [M+H]⁺.

### Embodiment 161

### 3-((3-exo)-3-((7-((5-(2-hydroxylethyl)thiazol-2-yl)amino)-1,6-diazanaphthalen-5-yl)amino)-8-azabicyclo[3.2.1]oct-8-yl)propionitrile

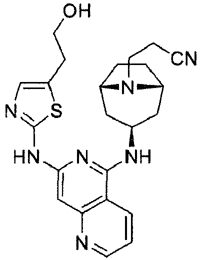

The preparation of 3-((3-*exo*)-3-((7-((5-(2-hydroxylethyl)thiazol-2-yl)amino)-1,6-diazanaphthalen-5-yl)amino)-8-azabicyclo[3.2.1]oct-8-yl)propionitrile was carried out with the reference to Embodiment 2.

¹H NMR (400 MHz, DMSO-*d₆*) *δ* 10.78 (s, 1H), 8.67 (d, *J* = 4.8 Hz, 1H), 8.55 (d, *J* = 7.1 Hz, 1H), 7.45 (d, *J* = 7.8 Hz, 1H), 7.12-7.04 (m, 2H), 6.47 (s, 1H), 4.87-4.77 (m, 2H), 3.61 (dd, *J* = 12.7, 7.0 Hz, 2H), 3.34 (s, 2H), 2.85-2.79 (m, 2H), 2.65 (s, 4H), 1.88 (ddd, *J* = 29.8, 26.6, 7.6 Hz, 8H).

MS *m*/*z* (ESI): 450.2 [M+H]⁺.

### Embodiment 162

### 3-((3-exo)-3-((7-((5-(1-hydroxylethyl)thiazol-2-yl)amino)-1,6-diazanaphthalen-5-yl)amino)-8-azabicyclo[3.2.1]oct-8-yl)propionitrile

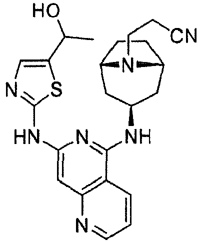

### Step 1: preparation of 1-(2-aminothiazol-5-yl)ethane-1-ol

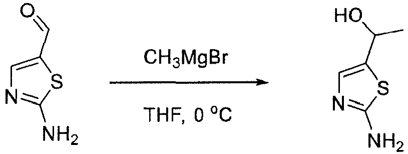

2-Aminothiazol-5-formaldehyde (256 mg, 2.0 mmol) was dissolved in dry tetrahydrofuran (10 mL). The reaction mixture was then cooled to 0 °C, followed by addition of methyl magnesium bromide (3M ether solution, 3.5 mL, 10 mmol) under nitrogen atmosphere. The reaction mixture was stirred at 0 °C for 70 minutes. The reaction mixture was then quenched with water, and extracted with ethyl acetate. The organic phase was successively washed with saturated sodium bicarbonate aqueous solution and saturated sodium chloride aqueous solution, dried over anhydrous sodium sulfate, filtered, and concentrated under reduced pressure to obtain the title compound (150 mg, 52%).

MS *m*/*z* (ESI): 145.2 [M+H]⁺.

### Step 2: preparation of 3-((3-exo)-3-((7-((5-(1-hydroxylethyl)thiazol-2-yl)amino)-1,6-diazanaphthalen-5-yl)amino)-8-azabicyclo[3.2.1]oct-8-yl)propionitrile

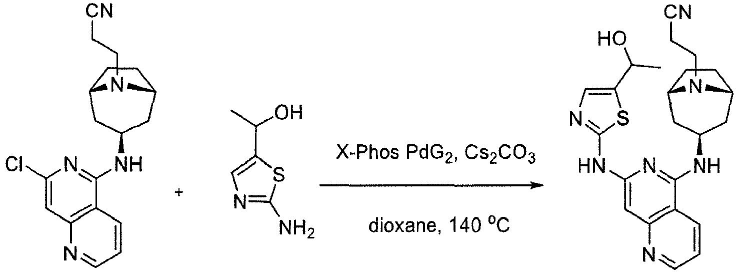

3-((3-*exo*)-3-((7-Chloro-1,6-diazanaphthalen-5-yl)amino)-8-azabicyclo[3.2.1]oct-8-yl)propionitrile (50 mg, 0.146 mmol), 1-(2-aminothiazol-5-yl)ethane-1-ol (42 mg, 0.292 mmol), chloro(2-dicyclohexylphosphino-2',4',6'-triisopropyl-1,1'-biphenyl)[2-(2'-amino-1,1'-biphenyl)]palladium(II) (57 mg, 0.073 mmol) and cesium carbonate (143 mg, 0.438 mmol) were added to ultra-dry 1,4-dioxane (5 mL). The reaction mixture was filled with dry nitrogen then heated to 140 °C with microwave and stirred for 4 hours. Then the mixture was cooled to room temperature, and filtered. The filtrate was concentrated under reduced pressure, and the residue was purified by prep-HPLC to obtain the title compound (1.9 mg, 3 %).

¹H NMR (400 MHz, DMSO-*d₆*) *δ* 10.79 (s, 1H), 8.67 (d, *J* = 3.0 Hz, 1H), 8.56 (d, *J* = 8.0 Hz, 1H), 7.44 (d, *J* = 8.4 Hz, 1H), 7.16-7.07 (m, 2H), 6.49 (s, 1H), 5.29 (d, *J* = 4.2 Hz, 1H), 4.92-4.81 (m, 2H), 3.34 (s, 2H), 2.65 (s, 4H), 1.93 (s, 4H), 1.81 (d, *J* = 9.9 Hz, 4H), 1.44 (d, *J* = 6.4 Hz, 3H).

MS *m*/*z* (ESI): 450.2, 452.0 [M+H]⁺.

### Embodiment 163

### 3-((3-exo)-3-((7-((5-(hydroxymethyl)thiazol-2-yl)amino)-1,6-diazanaphthalen-5-yl)amino)-9-azabicyclo[3.3.1]non-9-yl)propionitrile

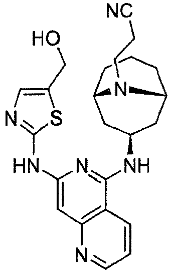

### Step 1: preparation of tert-butyl (3-exo)-3-((7-chloro-1,6-diazanaphthalen-5-yl)amino)-9-azabicyclo[3.3.1]non-9-carboxylate

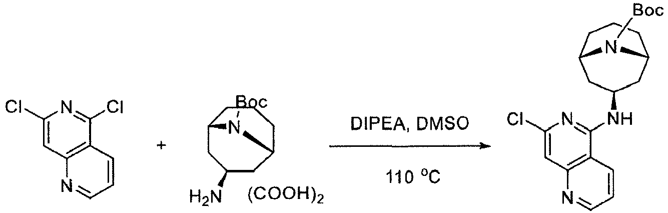

5,7-Dichloro-1,6-diazanaphthalen (300 mg, 1.51 mmol), tert-butyl-(3-exo)-3-amino-9-azabicyclo[3.3.1]non-9-carboxylate oxalate (750 mg, 2.26 mmol) and DIPEA (580 mg, 4.53 mmol) were added to DMSO(5 mL). The reaction mixture was mixed evenly, reacted at 110 °C overnight. The mixture was then cooled to room temperature, and concentrated under reduced pressure. The crude product obtained was purified by flash silica gel chromatography to obtain the title compound as a yellow solid (500 mg, 82 %).

MS *m*/*z* (ESI): 403.1 [M+H]⁺.

### Step 2: preparation of tert-butyl-(3-exo)-3-((7-((5-formylthiazol-2-yl)amino)-1,6-diazanaphthalen-5-yl)amino)-9-azabicyclo[3.3.1]non-9-carboxylate

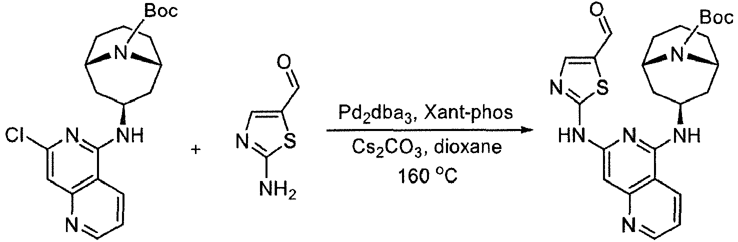

(3-*exo*)-3-((7-Chloro-1,6-diazanaphthalen-5-yl)amino)-9-azabicyclo[3.3.1]non-9-carboxylate (200 mg, 0.5 mmol), 2-aminothiazol-5-formaldehyde (96 mg, 0.75 mmol), tris(dibenzylideneacetone)dipalladium (46 mg, 0.05 mmol), 4,5-bis(diphenylphosphine)-9,9-dimethylxanthene (60 mg, 0.1 mmol) and cesium carbonate(490 mg, 1.5 mmol) were added to 1,4-dioxane (5 mL). Under nitrogen atmosphere, the mixture was heated to 160 °C under microwave and reacted for 3 hours. Then the mixture was cooled to room temperature, and concentrated under reduced pressure. The crude product obtained was purified by flash silica gel chromatography to obtain the title compound as a yellow solid (150 mg, 61 %).

MS *m*/*z* (ESI): 495.1 [M+H]⁺.

### Step 3: preparation of (3-exo)-3-((7-((5-(hydroxymethyl)thiazol-2-yl)amino)-1,6-diazanaphthalen-5-yl)amino)-9-azabicyclo[3.3.1]non-9-carboxylate

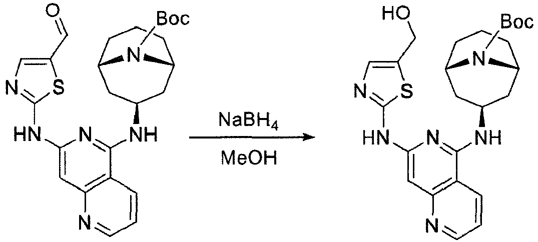

(3-*exo*)-3-((7-((5-formylthiazol-2-yl)amino)-1,6-diazanaphthalen-5-yl)amino)-9-azabicyclo[3.3.1]non-9-carboxylate (150 mg, 0.3 mmol) was added to methanol (10 mL) at 0 °C, and sodium borohydride (45 mg, 1.2 mmol) was slowly added to the reaction mixture. After 0.5 hour, the reaction mixture was quenched with water (0.2 mL), and concentrated under reduced pressure. The crude product obtained was purified by flash silica gel chromatography to obtain the title compound as a yellow solid (100 mg, 67 %).

MS *m*/*z* (ESI): 497.2 [M+H]⁺.

### Step 4: preparation of 3-((3-exo)-3-((7-((5-(hydroxymethyl)thiazol-2-yl)amino)-1,6-diazanaphthalen-5-yl)amino)-9-azabicyclo[3.3.1]non-9-yl)propionitrile

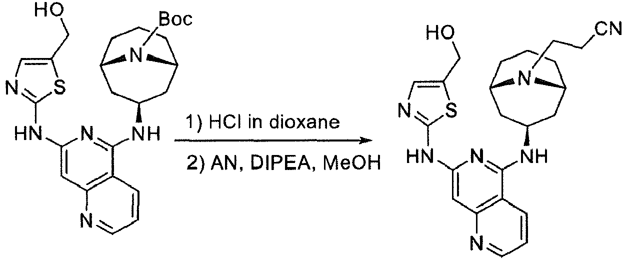

(3-*exo*)-3-((7-((5-(hydroxymethyl)thiazol-2-yl)amino)-1,6-diazanaphthalen-5-yl)amino)-9-azabicyclo[3.3.1]non-9-carboxylate (100 mg, 0.2 mmol) was added to methanol (10 mL), and hydrochloride dioxanesolution (4N, 2 mL) was slowly added to the reaction mixture. The reaction was carried out at room temperature for 1 hour, and then the mixture was concentrated under reduced pressure. A mixed solution of acrylonitrile (0.2 mL), DIPEA (0.2 mL) and methanol (10 mL) was added to the crude product obtained (100 mg), and the reaction was carried out at room temperature for 1 hour. The reaction mixture was concentrated under reduced pressure, and the crude product obtained was purified by prep-HPLC to obtain the title compound as a white solid (19.2 mg, 15%).

¹H NMR (400 MHz, DMSO-*d₆*) *δ* 10.88 (s, 1H), 8.69 (t, *J* = 2.8 Hz, 1H), 8.58 (d, *J* = 8.0 Hz, 1H), 7.32 (d, *J* = 8.0 Hz, 1H), 7.18-7.12 (m, 2H), 6.50 (s, 1H), 5.18-5.09 (m, 2H), 4.50 (d, *J* = 5.2 Hz, 2H), 3.03-2.92 (m, 4H), 2.63 (t, *J=* 6.4 Hz, 2H), 2.24-2.16 (m, 1H), 1.98-1.87 (m, 6H), 1.73-1.67 (m, 3H).

MS *m*/*z* (ESI): 450.1 [M+H]⁺.

### Embodiment 164

### 1-((3-exo)-3-((7-((5-(hydroxymethyl)thiazol-2-yl)amino)-1,6-diazanaphthalen-5-yl)amino)-9-azabicyclo[3.3.1]non-9-yl)-2-(pyrrolidin-1-yl)ethane-1-one

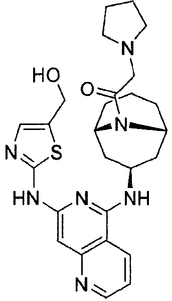

The preparation of 1-((3-*exo*)-3-((7-((5-(hydroxymethyl)thiazol-2-yl)amino)-1,6-diazanaphthalen-5-yl)amino)-9-azabicyclo[3.3.1]non-9-yl)-2-(pyrrolidin-1-yl)ethane-1-one was carried out with the reference to Embodiment 163.

MS *m*/*z* (ESI): 508.2 [M+H]⁺.

### Embodiment 165

### 1-((3-exo)-3-((7-((5-(hydroxymethyl)thiazol-2-yl)amino)-1,6-diazanaphthalen-5-yl)amino)-8-azabicyclo[3.2.1]oct-8-yl)-2-(piperidin-1-yl)ethane-1-one

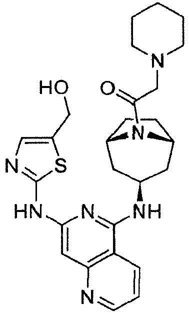

The preparation of 1-((3-*exo*)-3-((7-((5-(hydroxymethyl)thiazol-2-yl)amino)-1,6-diazanaphthalen-5-yl)amino)-8-azabicyclo[3.2.1]oct-8-yl)-2-(piperidin-1-yl)ethane-1-one was carried out with the reference to Embodiment 163.

¹H NMR (400 MHz, DMSO-*d₆*) *δ* 10.89 (s, 1H), 8.68 (dd, *J* = 4.0, 1.2 Hz, 1H), 8.52 (d, *J* = 8.4 Hz, 1H), 7.46 (d, *J* = 8.4 Hz, 1H), 7.19-7.11 (m, 2H), 6.52 (s, 1H), 5.18-5.09 (m, 2H), 4.56-4.54 (m, 4H), 3.16-3.13(m, 1H), 3.03-3.00 (m, 1H), 2.51-2.39 (m, 4H), 2.09-1.76 (m, 8H), 1.52-1.50 (m, 4H), 1.38-1.35 (m, 2H).

MS *m*/*z* (ESI): 508.1 [M+H]⁺.

### Embodiment 166

### 2,2-difluoro-1-((3-exo)-3-((7-((5-(hydroxymethyl)thiazol-2-yl)amino)-1,6-diazanaphthalen-5-yl)amino)-8-azabicyclo[3.2.1]oct-8-yl)ethane-1-one

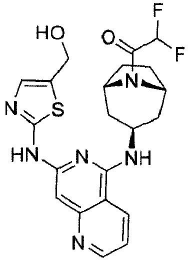

### Step 1: preparation of 1-((3-exo)-3-((7-chloro-1,6-diazanaphthalen-5-yl)amino)-8-azabicyclo[3.2.1]oct-8-yl)-2,2-difluoroethane-1-one

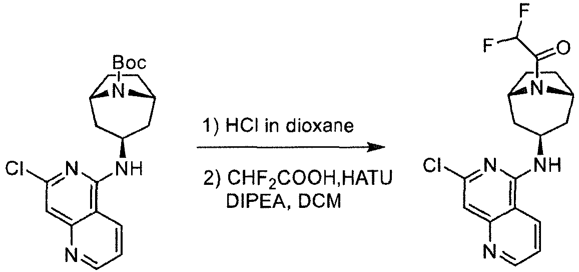

The compound (3-*exo*)-3-((7-chloro-1,6-diazanaphthalen-5-yl)amino)-8-azabicyclo[3.2.1]oct-8-carboxylate (300 mg, 0.771 mmol) was added to methanol (2 mL), and the solution of HCl in methanol (2 mL, 4 M) was added to the mixture at room temperature. Then the mixture was stirred at room temperature for 1 hour. The reaction mixture was concentrated to obtain the orange solid.

The orange solid (100 mg, 0.251 mmol), 2,2-difluoroacetic acid(29 mg, 0.301 mmol) and DIPEA (143 mg, 0.377 mmol) were separately added to DCM (2 mL), and HATU(143 mg, 113 mmol) was added to the mixture in batches at room temperature. The mixture was stirred at room temperature for 30 minutes. The reaction mixture was concentrated under reduced pressure, and the residue was slurried in the mixed solution of methanol and water (each 2 mL) to obtain the title compound as a white solid (33 mg, 36%).

MS *m*/*z* (ESI): 367.1 [M+H]⁺.

### Step 2: preparation of 2,2-difluoro-1-((3-exo)-3-((7-((5-(hydroxymethyl)thiazol-2-yl)amino)-1,6-diazanaphthalen-5-yl)amino)-8-azabicyclo[3.2.1]oct-8-yl)ethane-1-one

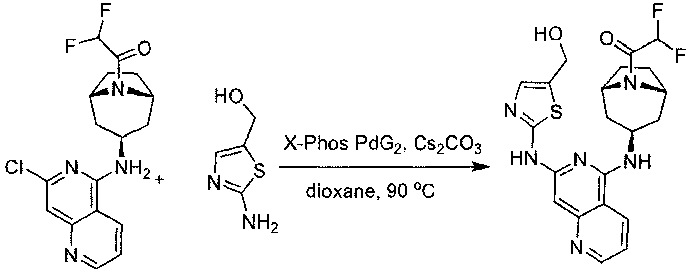

1-((3-*exo*)-3-((7-Chloro-1,6-diazanaphthalen-5-yl)amino)-8-azabicyclo[3.2.1]oct-8-yl)-2,2-difluoroethane-1-one (72 mg, 0.197 mmol), (2-aminothiazol-5-yl)methanol (38 mg, 0.295 mmol), cesium carbonate (128 mg, 0.394 mmol), chloro(2-dicyclohexylphosphino-2,4,6-triisopropyl-1,1-biphenyl)[2-(2-amino-1,1-biphenyl)]palladium(II) (X-Phos Pd G2) (31 mg, 0.0394 mmol) and XPhos(19 mg, 0.0394 mmol) were separately added to dioxane (3.5 mL). The mixture was filled with nitrogen for 3 times, then heated to 90 °C and stirred for 16 hours. The reaction mixture was concentrated under reduced pressure, and the residue was successively purified by silica gel chromatography and prep-HPLC to obtain the title compound as a yellow solid (7.4 mg, 8%).

¹H NMR (400 MHz, DMSO-*d₆*) *δ* 10.91 (s, 1H), 8.69 (dd, *J* = 4.3, 1.6 Hz, 1H), 8.50 (d, *J* = 8.4 Hz, 1H), 7.52 (d, *J* = 8.6 Hz, 1H), 7.19 (s, 1H), 7.13 (dd, *J* = 8.4, 4.3 Hz, 1H), 6.69 (t, *J* = 53.0 Hz, 1H), 6.54 (s, 1H), 5.31-5.04 (m, 2H), 4.63-4.49 (m, 4H), 2.20-1.72 (m, 8H).

MS *m*/*z* (ESI): 461.1 [M+H]⁺.

### Embodiment 167

### (2-((5-(((3-exo)-8-(2-fluoroethyl)-8-azabicyclo[3.2.1]oct-3-yl)amino)-1,6-diazanaphthalen-7-yl)amino)thiazol-5-yl)methanol

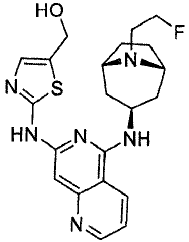

### Step 1: preparation of 7-chloro-N-((3-exo)-8-(2-fluoroethyl)-8-azabicyclo[3.2.1]oct-3-yl)-1,6-diazanaphthalen-5-amine.

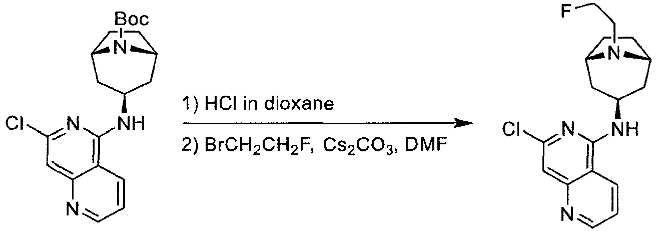

(3-*exo*)-3-((7-Chloro-1,6-diazanaphthalen-5-yl)amino)-8-azabicyclo[3.2.1]oct-8-carboxylate (200 mg, 0.514 mmol) was dissolved in methanol (1.6 mL), and the solution of HCl in dioxane (1.2 mL, 4 M) was added thereto at room temperature. The mixture was stirred at room temperature for 1 hour. The reaction mixture was concentrated under reduced pressure, and the residue was diluted with N,N-dimethylformamide(2 mL), followed by addition of cesium carbonate (670 mg, 2.06 mmol) and 1-bromine-2-fluoroethane (131 mg, 1.03 mmol). The mixture was stirred at 100 °C for 4 hours. The reaction mixture was cooled to room temperature, then diluted with ethyl acetate, and washed with saturated sodium chloride aqueous solution. The organic phase was collected and dried over anhydrous sodium sulfate, filtered, then concentrated under reduced pressure to remove the organic solvent, and the resulting product obtained was purified by silica gel chromatography to obtain the title compound as a brown solid (108 mg, 63%).

MS *m*/*z* (ESI): 335.1 [M+H]⁺.

### Step 2: preparation of (2-((5-(((3-exo)-8-(2-fluoroethyl)-8-azabicyclo[3.2.1]oct-3-yl)amino)-1,6-diazanaphthalen-7-yl)amino)thiazol-5-yl)methanol

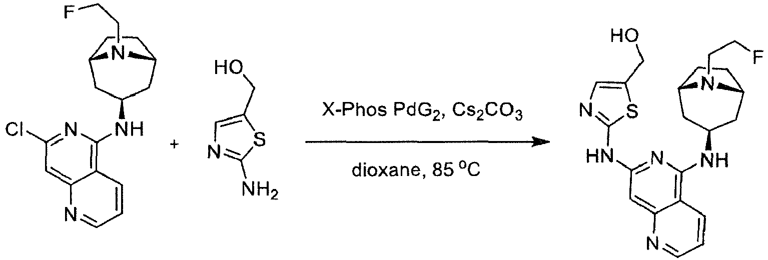

7-Chloro-N-((3-*exo*)-8-(2-fluoroethyl)-8-azabicyclo[3.2.1]oct-3-yl)-1,6-diazanaphthalen-5-amine (60 mg, 0.179 mmol), (2-aminothiazol-5-yl)methanol (47 mg, 0.358 mmol), cesium carbonate (117 mg, 0.358 mmol), chloro(2-dicyclohexylphosphino-2,4,6-triisopropyl-1,1-biphenyl)[2-(2-amino-1,1-biphenyl)]palladium(II) (X-Phos Pd G2) (28 mg, 0.0358 mmol) and XPhos(17 mg, 0.0358 mmol) were separately added to dioxane (2 mL). The mixture was filled with nitrogen for 3 times, then stirred at 85 °C for 16 hours. The reaction mixture was cooled down, diluted with methanol, and filtered. The filtrate was concentrated under reduced pressure, and the residue was purified by silica gel chromatography to obtain the title compound as a yellow solid (6.0 mg, 8%).

¹H NMR (400 MHz, DMSO-*d₆*) *δ* 10.86 (s, 1H), 8.68 (dd, *J* = 4.3, 1.6 Hz, 1H), 8.54 (d, *J* = 8.4 Hz, 1H), 7.45 (d, *J* = 8.6 Hz, 1H), 7.18 (s, 1H), 7.11 (dd, *J* = 8.4, 4.3 Hz, 1H), 6.49 (s, 1H), 5.17 (t, *J* = 5.3 Hz, 1H), 4.95-4.79 (m, 1H), 4.64-4.53 (m, 3H), 4.46 (t, *J* = 5.2 Hz, 1H), 2.77 (t, *J* = 5.2 Hz, 1H), 2.71 (t, *J* = 5.3 Hz, 1H), 2.00-1.74 (m, 8H).

MS *m*/*z* (ESI): 429.1 [M+H]⁺.

### Embodiment 168

### 3-((3-exo)-3-((7-((5-(hydroxymethyl)thiazol-2-yl)amino)-1,6-diazanaphthalen-5-yl)oxo)-8-azabicyclo[3.2.1]oct-8-yl)propionitrile

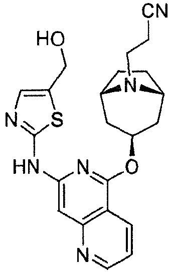

The preparation of 3-((3-*ex*)-3-((7-((5-(hydroxymethyl)thiazol-2-yl)amino)-1,6-diazanaphthalen-5-yl)oxo)-8-azabicyclo[3.2.1]oct-8-yl)propionitrile was carried out with the reference to Embodiment 2.

MS *m*/*z* (ESI): 437.2 [M+H]⁺.

### Embodiment 169

### 3-((3-exo)-3-((7-methoxyl-4-((5-methyl-1H-pyrazol-3-yl)amino)quinazolin-2-yl)amino)-8-azabicyclo[3.2.1]oct-8-yl)propionitrile

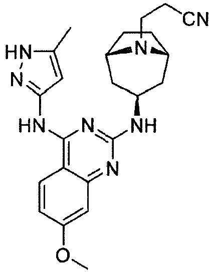

### Step 1: preparation of 2-chloro-7-methoxyl-N-(5-methyl-1H-pyrazol-3-yl)quinazolin-4-amine

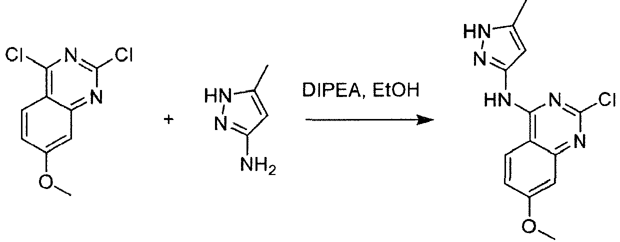

2,4-Dichloro-7-methoxylquinazoline (500 mg, 2.18 mmol), 5-methyl-1H-pyrazol-3-amine(223 mg, 2.29 mmol) and DIPEA (592 mg, 4.58 mmol) were separately added to anhydrous ethanol (10 mL), and the mixture was stirred at room temperature for 3 days. The reaction mixture was filtered, and the filter cake was washed with acetonitrile (5 mL), and dried under vacuum to obtain the title compound as a white solid (355 mg, 56%).

MS *m*/*z* (ESI): 290.1 [M+H]⁺.

### Step 2: preparation of (3-exo)-3-((7-methoxyl-4-((5-methyl-1H-pyrazol-3-yl)amino)quinazolin-2-yl)amino)-8-azabicyclo[3.2.1]oct-8-carboxylate

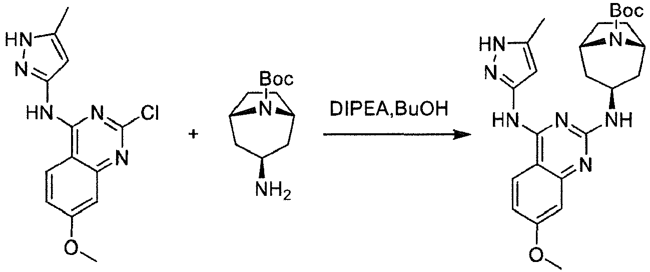

2-Chloro-7-methoxyl-N-(5-methyl-1H-pyrazol-3-yl)quinazolin-4-amine (355 mg, 1.23 mmol), Tert-butyl (3-exo)-3-amino-8-azabicyclo[3.2.1]oct-8-carboxylate acetate (421 mg, 1.47 mmol) and DIPEA (475 mg, 3.68 mmol) were mixed in n-butanol (7 mL). The reaction mixture was then heated to 150 °C with microwave and stirred for 4 hours. The reaction mixture was then cooled down to room temperature, and then concentrated under reduced pressure. The resulting product was purified by silica gel chromatography to obtain the title compound as a white solid (259 mg, 44%).

MS *m*/*z* (ESI): 480.2 [M+H]⁺.

### Step 3: preparation of 3-((3-exo)-3-((7-methoxyl-4-((5-methyl-1H-pyrazol-3-yl)amino)quinazolin-2-yl)amino)-8-azabicyclo[3.2.1]oct-8-yl)propionitrile

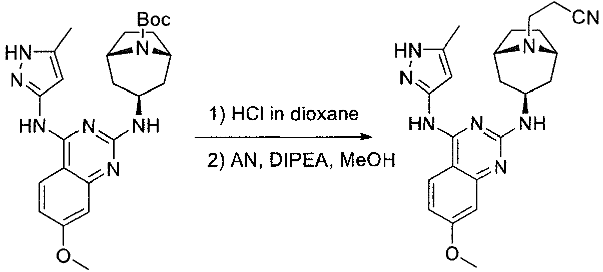

Tert-butyl (3-exo)-3-((7-methoxyl-4-((5-methyl-1H-pyrazol-3-yl)amino)quinazolin-2-yl)amino)-8-azabicyclo[3.2.1]oct-8-carboxylate (259 mg, 0.540 mmol) was dissolved in methanol (3 mL), and the mixture was stirred at room temperature, followed by addition of 4M HCl in 1,4-dioxane(4 mL). The reaction mixture was stirred at room temperature for 1 hour, and then concentrated under reduced pressure. The residue was dissolved in anhydrous methanol (3 mL), followed by successive addition of DIPEA (349 mg, 2.70 mmol) and acrylonitrile (43 mg, 0.810 mmol). The resulting reaction mixture was stirred at room temperature for 0.5 hour, and filtered. The filtrate was concentrated under reduced pressure, and the resulting product was purified by silica gel chromatography to obtain the title compound as a white solid (76.8 mg, 33%).

¹H NMR (400 MHz, Methanol-*d*₄) *δ* 8.12 (s, 1H), 7.14-6.75 (m, 2H), 6.50 (s, 1H), 4.50-4.21 (m, 1H), 3.92 (s, 3H), 3.41 (s, 2H), 2.91-2.55 (m, 4H), 2.34 (s, 3H), 2.14-1.50 (m, 7H), 1.40-1.23 (m, 1H).

MS *m*/*z* (ESI): 433.2[M+H]⁺.

### Embodiment 170

### 3-((3-exo)-3-((7-bromine-4-((5-methyl-1H-pyrazol-3-yl)amino)quinazolin-2-yl)amino)-8-azabicyclo[3.2.1]oct-8-yl)propionitrile

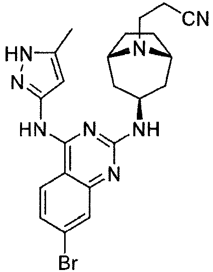

### Step 1: preparation of 7-bromine-2-chloro-N-(5-methyl-1H-pyrazol-3-yl)quinazolin-4-amine

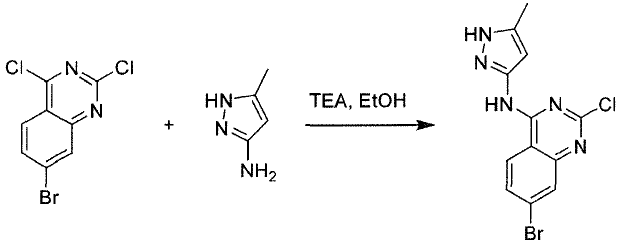

7-Bromine-2,4-dichloroquinazoline (3.36 g, 12.1 mmol), 5-methyl-1H-pyrazol-3-amine(1.29 g, 13.3 mmol) and TEA (2.57 g, 25.4 mmol) were separately added to anhydrous ethanol (67 mL), and the mixture was stirred at room temperature for 16 hours. The reaction mixture was filtered, and the filter cake was washed with anhydrous ethanol (20 mL), dried under vacuum to obtain the title compound as a white solid (4.17 g, 100%).

### Step 2: preparation of tert-butyl (3-exo)-3-((7-bromine-4-((5-methyl-1H-pyrazol-3-yl)amino)quinazolin-2-yl)amino)-8-azabicyclo[3.2.1]oct-8-carboxylate

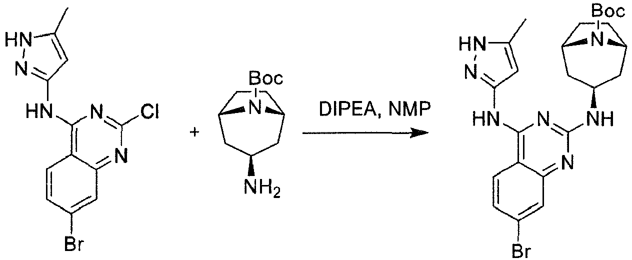

7-Bromine-2-chloro-N-(5-methyl-1H-pyrazol-3-yl)quinazolin-4-amine (500 mg, 1.48 mmol), tert-butyl (3-exo)-3-amino-8-azabicyclo[3.2.1]oct-8-carboxylate acetate (465 mg, 1.62 mmol) and DIPEA (591 mg, 4.58 mmol) were mixed in NMP(5 mL), and the mixture was then heated to 130 °C with microwave and stirred for 4 hours. The reaction mixture was cooled to room temperature, followed by addition of 25 mL ice water, and stirred for 30 minutes. The mixture was filtered, and the filter cake was washed with acetonitrile (2 mL), dried under reduced pressure to obtain the title compound as a gray solid (877 mg, 100%).

### Step 3: preparation of 3-((3-exo)-3-((7-bromine-4-((5-methyl-1H-pyrazol-3-yl)amino)quinazolin-2-yl)amino)-8-azabicyclo[3.2.1]oct-8-yl)propionitrile

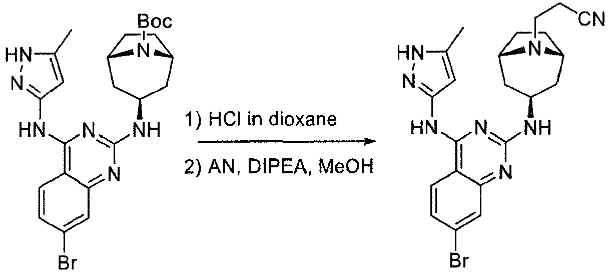

Tert-butyl (3-*exo*)-3-((7-bromine-4-((5-methyl-1H-pyrazol-3-yl)amino)quinazolin-2-yl)amino)-8-azabicyclo[3.2.1]oct-8-carboxylate (220 mg, 0.416 mmol) was dissolved in methanol (2 mL), followed by addition of 4M HCl in 1,4-dioxane (2 mL) with stirring at room temperature. The reaction mixture was stirred at room temperature for 2 hours, and then concentrated under reduced pressure. The residue was dissolved in anhydrous methanol (2 mL), and DIPEA (269 mg, 2.08 mmol) and acrylonitrile (66 mg, 1.25 mmol) were successively added thereto. The resulting reaction mixture was stirred at room temperature for 1 hour. The reaction mixture was filtered, and the filtrate was concentrated under reduced pressure. The resulting product was purified by silica gel chromatography to obtain the title compound as a white solid (17.4 mg, 9%).

¹H NMR (400 MHz, CD₃OD) *δ* 7.94 (d, *J* = 8.8 Hz, 1H), 7.74-7.37 (m, 1H), 7.24 (dd, *J* = 8.9, 2.0 Hz, 1H), 6.59 (s, 0.8H), 5.92 (s, 0.2H), 4.51-4.12 (m, 1H), 3.42-3.35 (m, 2H), 2.75 (t, *J* = 6.9 Hz, 2H), 2.62 (t, *J=* 6.9 Hz, 2H), 2.31 (s, 3H), 2.09-1.61 (m, 8H).

MS *m*/*z* (ESI): 481.1 [M+H]⁺.

### Embodiment 171

### 3-((3-exo)-3-((7-chloro-4-((5-methyl-1H-pyrazol-3-yl)amino)quinazolin-2-yl)amino)-8-azabiclo[3.2.1oct-8-yl)propionitrile

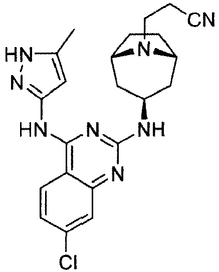

### Step 1: preparation of 2,7-dichloro-N-(5-methyl-1H-pyrazol-3-yl)quinazolin-4-amine

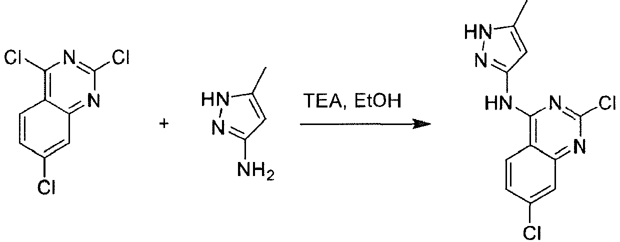

2,4,7-Trifluoroquinazoline(2.0 g, 8.58 mmol), 5-methyl-1H-pyrazol-3-amine(915 mg, 9.42 mmol) and TEA (1.82 g, 18.0 mmol) were separately added to anhydrous ethanol (40 mL), and the mixture was stirred at room temperature for 16 hours. The reaction mixture was filtered, and the filter cake was washed with anhydrous ethanol (5 mL), and dried under vacuum to obtain the title compound as a white solid (2.5 g, 99%).

MS *m*/*z* (ESI): 294.0 [M+H]⁺.

### Step 2: preparation of Tert-butyl (3-exo)-3-((7-chloro-4-((5-methyl-1H-pyrazol-3-yl)amino)quinazolin-2-yl)amino)-8-azabicyclo[3.2.1]oct-8-carboxylate

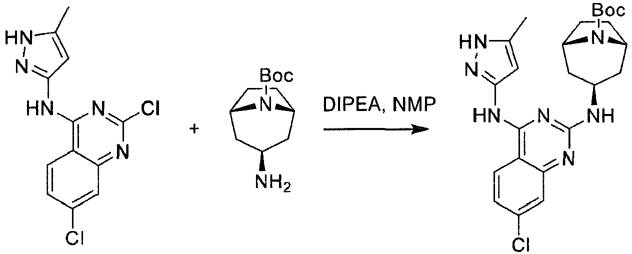

2,7-Dichloro-N-(5-methyl-1H-pyrazol-3-yl)quinazolin-4-amine (500 mg, 1.70 mmol), Tert-butyl (3-*exo*)-3-amino-8-azabicyclo[3.2.1]oct-8-carboxylate acetate (535 mg, 1.87 mmol) and DIPEA (681 mg, 5.27 mmol) were mixed in NMP (7 mL). The reaction mixture was then heated to 180 °C with microwave and stirred for 2 hours. The reaction mixture was then cooled to room temperature, followed by addition of ice water with stirring. A solid was precipitated out and filtered. The filter cake was washed with water, dried under vacuum, and then purified by silica gel chromatography to obtain the title compound as a white solid (405 mg, 49%).

MS *m*/*z* (ESI): 484.2 [M+H]⁺.

### Step 3: preparation of 3-((3-exo)-3-((7-chloro-4-((5-methyl-1H-pyrazol-3-yl)amino)quinazolin-2-yl)amino)-8-azabicyclo[3.2.1]oct-8-yl)propionitrile

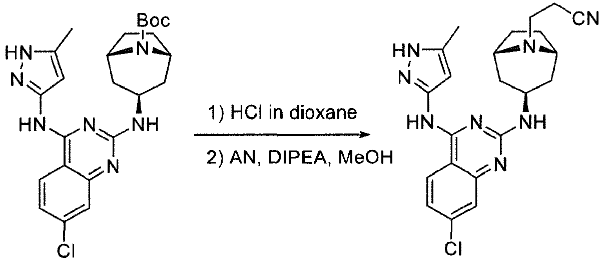

Tert-butyl (3-*exo*)-3-((7-chloro-4-((5-methyl-1H-pyrazol-3-yl)amino)quinazolin-2-yl)amino)-8-azabicyclo[3.2.1]oct-8-carboxylate (405 mg, 0.837 mmol) was dissolved in methanol (4 mL), and the mixture was stirred at room temperature, followed by addition of 4M HCl in 1,4-dioxane (2.5 mL). The reaction mixture was stirred at room temperature for 1 hour, and concentrated under reduced pressure. The residue was dissolved in anhydrous methanol (4 mL), and DIPEA (486 mg, 3.77 mmol) and acrylonitrile (53 mg, 1.00 mmol) were successively added thereto. The resulting reaction mixture was stirred at room temperature for 16 hours. The reaction mixture was filtered, and the filtrate was concentrated under reduced pressure. The residue was successively purified by silica gel chromatography and prep-HPLC to obtain the title compound as a white solid (40 mg, 11%).

¹H NMR (400 MHz, Methanol-*d*₄) *δ* 8.02 (d, *J* = 8.8 Hz, 1H), 7.54-7.22 (m, 1H), 7.14-7.07 (m, 1H), 6.71-6.49 (m, 0.6H), 6.05-5.76 (m, 0.4H), 4.44-4.17 (m, 1H), 3.40-3.35 (m, 2H), 2.75 (t, *J* = 7.0 Hz, 2H), 2.62 (t, *J* = 6.9 Hz, 2H), 2.46-2.12 (m, 3H), 2.07-2.00 (m, 2H), 1.96-1.75 (m, 4H), 1.71-1.61 (m, 2H).

MS *m*/*z* (ESI): 437.2[M+H]⁺.

### Embodiment 172

### 3-((3-exo)-3-((7-fluoro-4-((5-methyl-1H-pyrazol-3-yl)amino)quinazolin-2-yl)amino)-8-azabicyclo[3.2.1]oct-8-yl)propionitrile

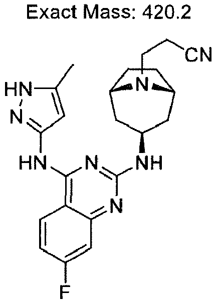

The preparation of 3-((3-*exo*)-3-((7-fluoro-4-((5-methyl-1H-pyrazol-3-yl)amino)quinazolin-2-yl)amino)-8-azabicyclo[3.2.1]oct-8-yl)propionitrile was carried out with the reference to Embodiment 171.

MS *m*/*z* (ESI): 421.2 [M+H]⁺.

### Embodiment 173

### 3-((3-exo)-3-((4-((5-methyl-1H-pyrazol-3-yl)amino)pyrido[3,4-d]pyrimidin-2-yl)amino)-8-azabicyclo[3.2.1]oct-8-yl)propionitrile

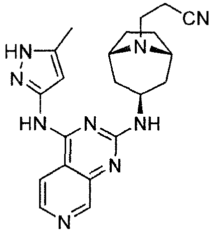

### Step 1: preparation of 2-chloro-N-(5-methyl-1H-pyrazol-3-yl)pyrido[3,4-d]pyrimidin-4-amine

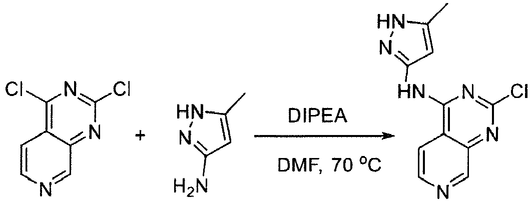

2,4-Dichloropyrido[3,4-d]pyrimidine(120 mg, 0.6 mmol), 5-methyl-1H-pyrazol-3-amine (64 mg, 0.66 mmol) and DIPEA (150 mg, 1.2 mmol) were added to N,N-dimethylformamide(4 mL). The reaction mixture was mixed evenly, heated at 70 °C in oil bath and stirred overnight. Then the mixture was concentrated under reduced pressure, and the crude product obtained was added to methanol (5 mL). A solid was precipitated, filtered, and dried to obtain the title compound as a yellow solid (140 mg, 89 %).

MS *m*/*z* (ESI): 261.1 [M+H]⁺.

### Step 2: preparation of tert-butyl-(3-exo)-3-((4-((5-methyl-1H-pyrazol-3-yl)amino)pyrido[3,4-d]pyrimidin-2-yl)amino)-8-azabicyclo[3.2.1]oct-8-carboxylate

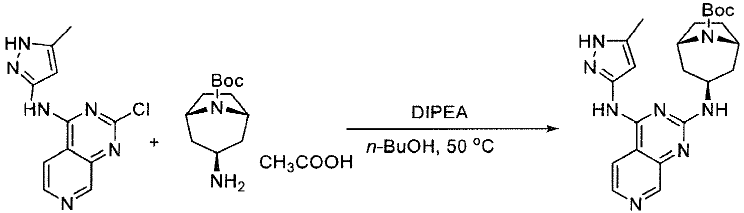

2,4-Dichloropyrido[3,4-d]pyrimidine(100 mg, 0.22 mmol), Tert-butyl (3-exo)-3-amino-8-azabicyclo[3.2.1]oct-8-carboxylate acetate(95 mg, 0.33 mmol) and DIPEA (85 mg, 0.66 mmol) were added to n-butanol (2 mL). T he reaction mixture was mixed evenly, then heated to 150 °C with microwave and reacted for 16 hours. The mixture was then cooled down to room temperature, and concentrated under reduced pressure. The crude product obtained was purified by flash silica gel chromatography to obtain the title compound as a pale yellow solid (40 mg, 40 %).

MS *m*/*z* (ESI):451.2 [M+H]⁺.

### Step 3: preparation of 3-((3-exo)-3-((4-((5-methyl-1H-pyrazol-3-yl)amino)pyrido[3,4-d]pyrimidin-2-yl)amino)-8-azabicyclo[3.2.1]oct-8-yl)propionitrile

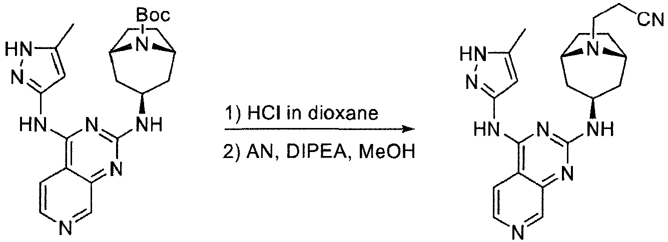

Methanol (10 mL) was added to tert-butyl-(3-*exo*)-3-((4-((5-methyl-1H-pyrazol-3-yl)amino)pyrido[3,4-d]pyrimidin-2-yl)amino)-8-azabicyclo[3.2.1]oct-8-carboxylate (40 mg, 0.09 mmol). The reaction mixture was mixed evenly, and hydrochloride dioxanesolution (4 N, 2 mL) was slowly added dropwise thereto. The reaction was carried out at room temperature for 2 hours, and then the mixture was concentrated under reduced pressure. The crude product obtained was added to the mixed solution of methanol (5 mL), DIPEA (0.5 mL) and acrylonitrile (1 mL), and then the reaction was carried out at room temperature for 2 hours. The mixture was concentrated under reduced pressure, and purified by prep-HPLC to obtain the title compound as a yellow solid (5 mg, 14 %).

¹H NMR (400 MHz, CD₃OD) *δ* 9.50-9.38 (m, 1H), 8.96-8.91 (m, 2 H),7.47-7.45 (m, 1H), 5.05-5.02 (m, 2H), 4.10 (s, 2H), 3.94 (s, 1H), 3.53-3.48 (s, 2H), 3.14-3.08 (m, 3 H), 2.84-2.40 (m, 8 H).

MS *m*/*z* (ESI):404.2 [M+H]⁺.

### Embodiment 174

### 3-((3-exo)-3-((4-((5-methyl-1H-pyrazol-3-yl)amino)pyrido[3,2-d]pyrimidin-2-yl)amino)-8-azabicyclo[3.2.1]oct-8-yl)propionitrile

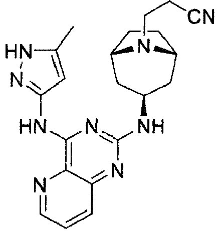

The preparation of 3-((3-*exo*)-3-((4-((5-methyl-1H-pyrazol-3-yl)amino)pyrido[3,2-d]pyrimidin-2-yl)amino)-8-azabicyclo[3.2.1]oct-8-yl)propionitrile was carried out with the reference to Embodiment 169.

¹H NMR (400 MHz, CD₃OD) *δ* 8.46-8.36 (m, 1H), 7.86-7.60 (m, 1H), 7.55 (dd, *J* = 8.5, 4.2 Hz, 1H), 6.81 (s, 0.8H), 5.99 (s, 0.2H), 4.54-4.17 (m, 1H), 3.47-3.35 (m, 2H), 2.76 (t, *J* = 7.0 Hz, 2H), 2.62 (t, *J* = 7.0 Hz, 2H), 2.41-2.21 (m, 3H), 2.13-2.01 (m, 2H), 1.97-1.81 (m, 4H), 1.75-1.64 (m, 2H).

MS *m*/*z* (ESI): 404.2 [M+H]⁺.

### Embodiment 175

### 3-((3-exo)-3-((5-chloro-4-((5-methyl-1H-pyrazol-3-yl)amino)quinazolin-2-yl)amino)-8-azabicyclo[3.2.1]oct-8-yl)propionitrile

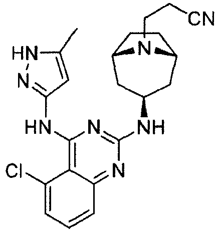

The preparation of 3-((3-*exo*)-3-((5-chloro-4-((5-methyl-1H-pyrazol-3-yl)amino)quinazolin-2-yl)amino)-8-azabicyclo[3.2.1]oct-8-yl)propionitrile was carried out with the reference to Embodiment 169.

¹H NMR (400 MHz, DMSO-*d₆*) *δ* 12.24 (s, 1H), 9.62 (s, 1H), 7.45 (t, *J* = 8.0 Hz, 1H), 7.33-6.92 (m, 3H), 6.92-6.55 (m, 1H), 4.32-4.15 (m, 1H), 3.33-3.25 (m, 2H), 2.68-2.56 (m, 4H), 2.26 (s, 3H), 2.00-1.85 (m, 2H), 1.83-1.54 (m, 6H).

MS *m*/*z* (ESI): 437.2 [M+H]⁺.

### Embodiment 176

### 3-((3-exo)-3-((8-methyl-4-((5-methyl-1H-pyrazol-3-yl)amino)quinazolin-2-yl)amino)-8-azabicyclo[3.2.1]oct-8-yl)propionitrile

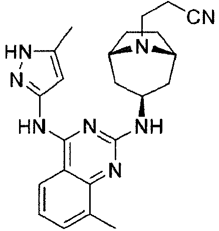

The preparation of 3-((3-*exo*)-3-((8-methyl-4-((5-methyl-1H-pyrazol-3-yl)amino)quinazolin-2-yl)amino)-8-azabicyclo[3.2.1]oct-8-yl)propionitrile was carried out with the reference to Embodiment 169.

¹H NMR (400 MHz, Methanol-*d*₄) *δ* 7.86 (d, 1H), 7.44 (d, *J* = 7.1 Hz, 1H), 7.03 (t, *J* = 7.7 Hz, 1H), 6.70-6.54 (m, 0.6H), 5.96-5.84 (m, 0.4H), 4.49-4.32 (m, 1H), 3.46-3.36 (m, 2H), 2.75 (t, *J* = 6.9 Hz, 2H), 2.62 (t, *J* = 6.9 Hz, 2H), 2.48 (s, 3H), 2.38-2.17 (m, 3H), 2.10-1.89 (m, 4H), 1.88-1.77 (m, 2H), 1.64 (t, *J* = 12.0 Hz, 2H).

MS *m*/*z* (ESI): 417.2 [M+H]⁺.

### Embodiment 177

### 3-((3-exo)-3-((8-chloro-4-((5-methyl-1H-pyrazol-3-yl)amino)quinazolin-2-yl)amino)-8-azabicyclo[3.2.1]oct-8-yl)propionitrile

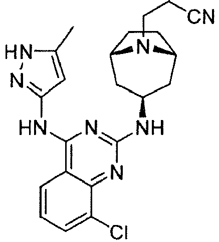

The preparation of 3-((3-*exo*)-3-((8-chloro-4-((5-methyl-1H-pyrazol-3-yl)amino)quinazolin-2-yl)amino)-8-azabicyclo[3.2.1]oct-8-yl)propionitrile was carried out with the reference to Embodiment 169.

¹H NMR (400 MHz, Methanol-*d*₄) *δ* 8.01 (s, 1H), 7.69 (d, *J* = 7.6 Hz, 1H), 7.09 (s, 1H), 6.83-5.77 (m, 1H), 4.52-4.26 (m, 1H), 3.57-3.36 (m, 2H), 2.94-2.71 (m, 2H), 2.71-2.53 (m, 2H), 2.32 (s, 3H), 2.19-1.49 (m, 8H).

MS *m*/*z* (ESI): 437.2 [M+H]⁺.

### Embodiment 178

### 3-((3-exo)-3-((6-chloro-4-((5-methyl-1H-pyrazol-3-yl)amino)quinazolin-2-yl)amino)-8-azabicyclo[3.2.1]oct-8-yl)propionitrile

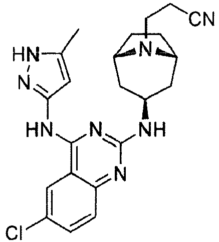

The preparation of 3-((3-*exo*)-3-((6-chloro-4-((5-methyl-1H-pyrazol-3-yl)amino)quinazolin-2-yl)amino)-8-azabicyclo[3.2.1]oct-8-yl)propionitrile was carried out with the reference to Embodiment 169.

¹H NMR (400 MHz, Methanol-*d*₄) *δ* 8.15 (d, *J* = 2.3 Hz, 1H), 7.54 (d, *J* = 8.8 Hz, 1H), 7.49-7.21 (m, 1H), 6.72-6.46 (m, 0.6H), 6.08-5.75 (m, 0.4H), 4.46-4.20 (m, 1H), 3.41-3.36 (m, 2H), 2.76 (t, *J* = 7.0 Hz, 2H), 2.62 (t, *J* = 7.0 Hz, 2H), 2.42-2.22 (m, 3H), 2.08-2.01 (m, 2H), 1.97-1.77 (m, 4H), 1.72-1.61 (m, 2H).

MS *m*/*z* (ESI): 437.2 [M+H]⁺.

### Embodiment 179

### 3-((3-exo)-3-((4-((5-methyl-1H-pyrazol-3-yl)amino)-7-(pyridin-3-yl)quinazolin-2-yl)amino)-8-azabicyclo[3.2.1]oct-8-yl)propionitrile

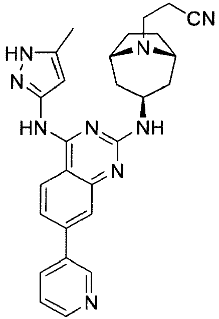

### Step 1: preparation of Tert-butyl (3-exo)-3-((4-((5-methyl-1H-pyrazol-3-yl)amino)-7-(pyridin-3-yl)quinazolin-2-yl)amino)-8-azabicyclo[3.2.1]oct-8-carboxylate

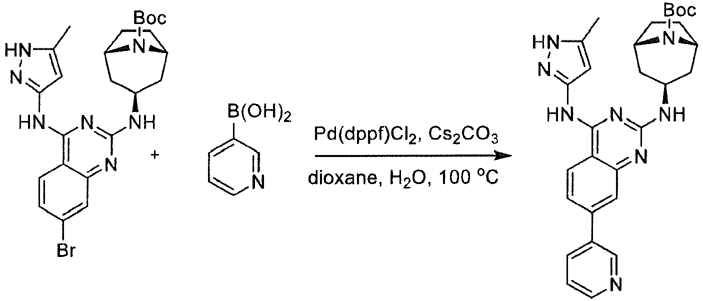

Tert-butyl (3-exo)-3-((7-bromine-4-((5-methyl-1H-pyrazol-3-yl)amino)quinazolin-2-yl)amino)-8-azabicyclo[3.2.1]oct-8-carboxylate (400 mg, 0.758 mmol), 3-eboronic acid(187 mg, 1.52 mmol), Pd(dppf)Cl₂ (110 mg, 0.152 mmol), and cesium carbonate (740 g, 2.27 mmol) were separately added to the mixed solvent of dioxane(8 mL) and water (0.8 mL). Under nitrogen atmosphere, the mixture was stirred at 100 °C for 1 hour. The reaction mixture was concentrated, and the residue was purified by silica gel chromatography to obtain the title compound as pale yellow colloid (160 mg, 40%).

### Step 2: preparation of 3-((3-exo)-3-((4-((5-methyl-1H-pyrazol-3-yl)amino)-7-(pyridin-3-yl)quinazolin-2-yl)amino)-8-azabicyclo[3.2.1]oct-8-yl)propionitrile

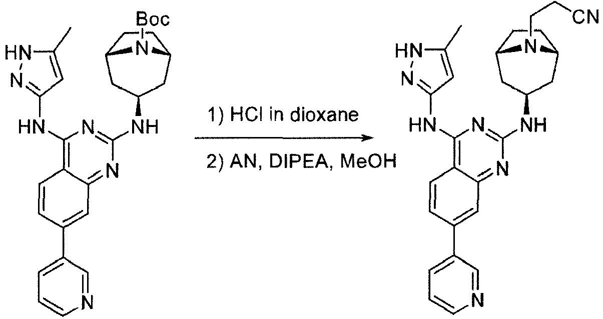

Tert-butyl (3-exo)-3-((4-((5-methyl-1H-pyrazol-3-yl)amino)-7-(pyridin-3-yl)quinazolin-2-yl)amino)-8-azabicyclo[3.2.1]oct-8-carboxylate (160 mg, 0.302 mmol) was dissolved in methanol (4 mL). The mixture was stirred at room temperature, and 4M HCl in 1,4-dioxane (4 mL) was added thereto. The reaction mixture was then stirred at room temperature for 1 hour, and concentrated under reduced pressure. The residue was dissolved in anhydrous methanol (2 mL), and DIPEA (195 mg, 1.51 mmol) and acrylonitrile (48 mg, 0.906 mmol) were successively added thereto. The resulting reaction mixture was stirred at room temperature for 2 hours. When the reaction was completed, the mixture was concentrated under reduced pressure, and the residue was preliminarily purified by silica gel chromatography to obtain a gray solid, which was slurried with N,N-dimethylformamide/acetonitrile (2 mL/4 mL), followed by filtration. The filtered solid was slurried with N,N-dimethylformamide/acetonitrile (1.1 mL/2.2 mL), filtered, and dried under vacuum to obtain the title compound as a white solid (49 mg, 34%).

¹H NMR (400 MHz, CD₃OD) *δ* 8.90 (d, *J* = 2.3 Hz, 1H), 8.59 (dd, *J* = 4.9, 1.6 Hz, 1H), 8.31-8.08 (m, 2H), 7.84-7.40 (m, 3H), 6.63 (s, 0.8H), 5.94 (s, 0.2H), 4.49-4.26 (m, 1H), 3.45-3.37 (m, 2H), 2.77 (t, *J* = 6.9 Hz, 2H), 2.63 (t, *J* = 6.9 Hz, 2H), 2.34 (s, 3H), 2.13-1.63 (m, 8H).

MS *m*/*z* (ESI): 480.2[M+H]⁺.

### Embodiment 180

### 3-((3-exo)-3-((4-((5-methy]-1H-pyrazol-3-yl)amino)-7-(pyridin-4-yl)quinazolin-2-yl)amino)-8-azabicyclo[3.2.1]oct-8-yl)propionitrile

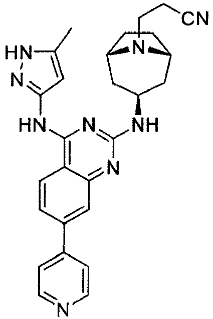

The preparation of 3-((3-*exo*)-3-((4-((5-methyl-1H-pyrazol-3-yl)amino)-7-(pyridin-4-yl)quinazolin-2-yl)amino)-8-azabicyclo[3.2.1]oct-8-yl)propionitrile was carried out with the reference to Embodiment 179.

¹H NMR (400 MHz, CD₃OD) *δ* 8.73-8.58 (m, 2H), 8.21 (d, *J=* 8.5 Hz, 1H), 7.95-7.64 (m, 3H), 7.59-7.49 (m, 1H), 6.64 (s, 1H), 4.49-4.22 (m, 1H), 3.45-3.35 (m, 2H), 2.77 (t, *J* = 7.0 Hz, 2H), 2.63 (t, *J=* 7.0 Hz, 2H), 2.33 (s, 3H), 2.16-1.58 (m, 8H).

MS *m*/*z* (ESI): 480.2[M+H]⁺.

### Embodiment 181

### 3-((3-exo)-3-((4-((5-methyl-1H-pyrazol-3-yl)amino)-7-(pyridin-2-yl)quinazolin-2-yl)amino)-8-azabicyclo[3.2.1]oct-8-yl)propionitrile

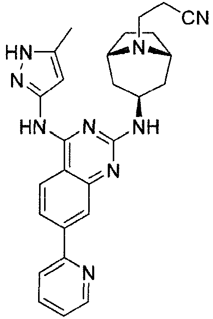

The preparation of 3-((3-*exo*)-3-((7-fluoro-4-((5-methyl-1H-pyrazol-3-yl)amino)quinazolin-2-yl)amino)-8-azabicyclo[3.2.1]oct-8-yl)propionitrile was carried out with the reference to Embodiment 179.

MS *m*/*z* (ESI): 480.3 [M+H]⁺.

### Embodiment 182

### 3-((3-exo)-3-((7-(5-methoxylpyridin-3-yl)-4-((5-methyl-1H-pyrazol-3-yl)amino)quinazolin-2-yl)amino)-8-azabicyclo[3.2.1]oct-8-yl)propionitrile

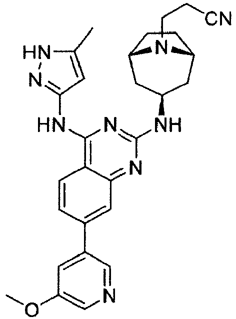

The preparation of 3-((3-*exo*)-3-((7-(5-methoxylpyridin-3-yl)-4-((5-methyl-1H-pyrazol-3-yl)amino)quinazolin-2-yl)amino)-8-azabicyclo[3.2.1]oct-8-yl)propionitrile was carried out with the reference to Embodiment 179.

¹H NMR (400 MHz, CD₃OD) *δ* 8.54-8.41 (m, 1H), 8.27 (d, *J* = 2.7 Hz, 1H), 8.17 (d, *J* = 8.5 Hz, 1H), 7.88-7.56 (m, 2H), 7.47 (dd, *J* = 8.5, 1.8 Hz, 1H), 6.62 (s, 1H), 4.49-4.25 (m, 1H), 3.97 (s, 3H), 3.44-3.37 (m, 2H), 2.76 (t, *J* = 7.0 Hz, 2H), 2.63 (t, *J* = 7.0 Hz, 2H), 2.32 (s, 3H), 2.10-1.64 (m, 8H).

MS *m*/*z* (ESI): 510.2[M+H]⁺.

### Embodiment 183

### 3-((3-exo)-3-((7-(6-methoxylpyridin-3-yl)-4-((5-methyl-1H-pyrazol-3-yl)amino)quinazolin-2-yl)amino)-8-azabicyclo[3.2.1]oct-8-yl)propionitrile

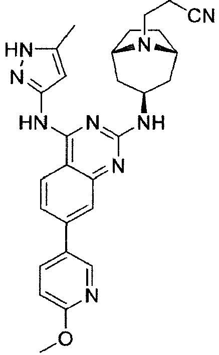

The preparation of 3-((3-*exo*)-3-((7-(6-methoxylpyridin-3-yl)-4-((5-methyl-1H-pyrazol-3-yl)amino)quinazolin-2-yl)amino)-8-azabicyclo[3.2.1]oct-8-yl)propionitrile was carried out with the reference to Embodiment 179.

MS *m*/*z* (ESI): 510.3 [M+H]⁺.

### Embodiment 184

### 3-((3-exo)-3-((4-((5-methyl-1H-pyrazol-3-yl)amino)-7-phenylquinazolin-2-yl)amino)-8-azabicyclo[3.2.1]oct-8-yl)propionitrile

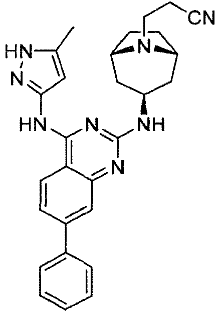

The preparation of 3-((3-*exo*)-3-((4-((5-methyl-1H-pyrazol-3-yl)amino)-7-phenylquinazolin-2-yl)amino)-8-azabicyclo[3.2.1]oct-8-yl)propionitrile was carried out with the reference to Embodiment 179.

¹H NMR (400 MHz, DMSO-*d*₆, a small amount of CD₃OD) *δ* 8.47-8.23 (m, 1H), 7.88-7.69 (m, 2H), 7.67-7.25 (m, 5H), 6.92-6.62 (m, 0.8H), 5.88 (s, 0.2H), 4.41-4.20 (m, 1H), 3.58 (s, 2H), 2.76-2.57 (m, 4H), 2.38-2.11 (m, 3H), 2.06-1.47 (m, 8H).

MS *m*/*z* (ESI): 479.3 [M+H]⁺.

### Embodiment 185

### 3-((3-exo)-3-((7-(1-cyclopropyl-1H-pyrazol-4-yl)-4-((5-methyl-1H-pyrazol-3-yl)amino)quinazolin-2-yl)amino)-8-azabicyclo[3.2.1]oct-8-yl)propionitrile

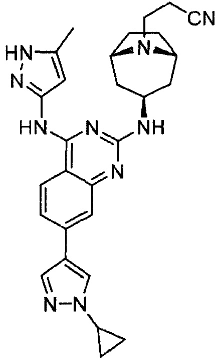

### Step 1: preparation ofTert-butyl (3-exo)-3-((7-(1-cyclopropyl-1H-pyrazol-4-yl)-4-((5-methyl-1H-pyrazol-3-yl)amino)quinazolin-2-yl)amino)-8-azabicyclo[3.2.1]oct-8-carboxylate

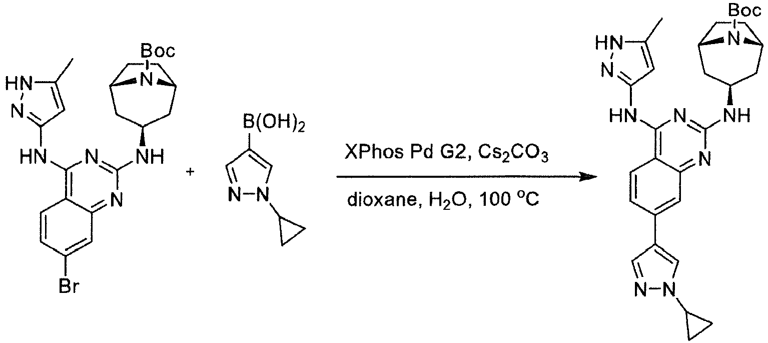

Tert-butyl (3-exo)-3-((7-bromine-4-((5-methyl-1H-pyrazol-3-yl)amino)quinazolin-2-yl)amino)-8-azabicyclo[3.2.1]oct-8-carboxylate (100 mg, 0.189 mmol), (1-cyclopropyl-1H-pyrazol-4-yl) boric acid (35 mg, 0.227 mmol), cesium carbonate (185 mg, 0.567 mmol), and chloro(2-dicyclohexylphosphino-2,4,6-triisopropyl-1,1-biphenyl)[2-(2-amino-1,1 - biphenyl)]palladium(II) (X-Phos Pd G2) (15 mg, 0.0189 mmol) were separately added to the mixed solvent of dioxane (2 mL) and water (0.4 mL). The mixture was filled with nitrogen for 3 times, and then stirred at 100 °C for 2 hours. The reaction mixture was cooled down and concentrated under reduced pressure. The residue was purified by silica gel chromatography to obtain the title compound as brown oil (60 mg, 57%).

### Step 2: preparation of 3-((3-exo)-3-((7-(1-cyclopropyl-1H-pyrazol-4-yl)-4-((5-methyl-1H-pyrazol-3-yl)amino)quinazolin-2-yl)amino)-8-azabicyclo[3.2.1]oct-8-yl)propionitrile

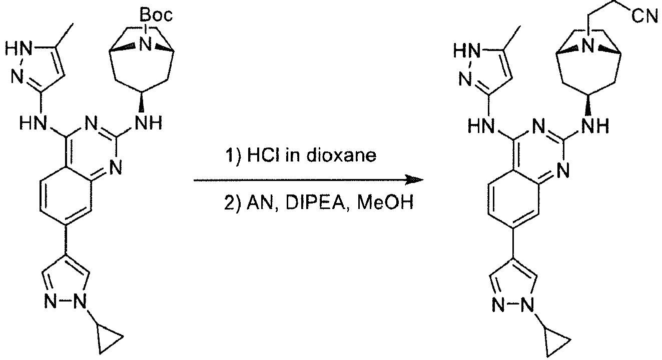

Tert-butyl (3-exo)-3-((7-(1-cyclopropyl-1H-pyrazol-4-yl)-4-((5-methyl-1H-pyrazol-3-yl)amino)quinazolin-2-yl)amino)-8-azabicyclo[3.2.1]oct-8-carboxylate (60 mg, 0.108 mmol) was dissolved in methanol (2 mL). The mixture was stirred at room temperature, and 4M HCl in 1,4-dioxane (2 mL) was added thereto. The reaction mixture was then stirred at room temperature for 1 hour, and concentrated under reduced pressure. The residue was dissolved in anhydrous methanol (1 mL), and DIPEA (70 mg, 0.542 mmol) and acrylonitrile (17 mg, 0.324 mmol) were successively added to the mixture. The resulting reaction mixture was stirred at room temperature for 16 hours. The reaction mixture was diluted with DCM (30 mL), washed with water (10 mL), and concentrated under reduced pressure. The residue was purified by silica gel chromatography to obtain the title compound as a gray solid (20 mg, 36%).

¹H NMR (400 MHz, CD₃OD) *δ* 8.20 (s, 1H), 8.06 (d, *J* = 8.3 Hz, 1H), 7.93 (s, 1H), 7.72-7.34 (m, 2H), 6.60 (s, 1H), 4.45-4.23 (m, 1H), 3.78-3.67 (m, 1H), 3.43-3.36 (m, 2H), 2.76 (t, *J=* 6.8 Hz, 2H), 2.63 (t, *J* = 6.8 Hz, 2H), 2.32 (s, 3H), 2.09-1.64 (m, 8H), 1.24-1.00 (m, 4H).

MS *m*/*z* (ESI): 509.2 [M+H]⁺.

### Embodiment 186

### 3-((3-exo)-3-((4-((5-methyl-1H-pyrazol-3-yl)amino)-7-(1-methyl-1H-pyrazol-4-yl)quinazolin-2-yl)amino)-8-azabicyclo[3.2.1]oct-8-yl)propionitrile

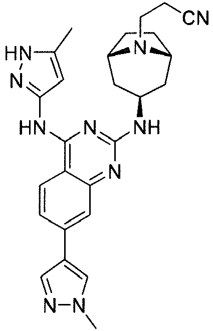

The preparation of 3-((3-*exo*)-3-((4-((5-methyl-1H-pyrazol-3-yl)amino)-7-(1-methyl-1H-pyrazol-4-yl)quinazolin-2-yl)amino)-8-azabicyclo[3.2.1]oct-8-yl)propionitrile was carried out with the reference to Embodiment 185.

¹H NMR (400 MHz, CD₃OD) *δ* 8.09 (s, 1H), 8.03 (d, *J* = 8.5 Hz, 1H), 7.93 (s, 1H), 7.64-7.43 (m, 1H), 7.38 (dd, *J* = 8.6, 1.7 Hz, 1H), 6.62 (s, 0.8H), 5.92 (s, 0.2H), 4.42-4.28 (m, 1H), 3.95 (s, 3H), 3.42-3.36 (m, 2H), 2.76 (t, *J* = 7.0 Hz, 2H), 2.63 (t, *J* = 7.0 Hz, 2H), 2.32 (s, 3H), 2.08-2.01 (m, 2H), 2.00-1.80 (m, 4H), 1.77-1.62 (m, 2H).

MS *m*/*z* (ESI): 483.2 [M+H]⁺.

### Embodiment 187

### 3-((3-exo)-3-((7-(1-(2-fluoroethyl)-1H-pyrazol-4-yl)-4-((5-methyl-1H-pyrazol-3-yl)amino)quinazolin-2-yl)amino)-8-azabicyclo[3.2.1]oct-8-yl)propionitrile

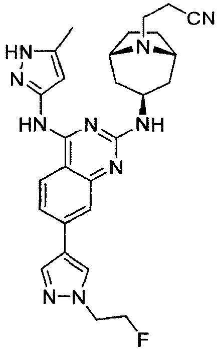

The preparation of 3-((3-*exo*)-3-((7-(1-(2-fluoroethyl)-1H-pyrazol-4-yl)-4-((5-methyl-1H-pyrazol-3-yl)amino)quinazolin-2-yl)amino)-8-azabicyclo[3.2.1]oct-8-yl)propionitrile was carried out with the reference to Embodiment 185.

MS *m*/*z* (ESI): 515.3 [M+H]⁺.

### Embodiment 188

### 3-((3-exo)-3-((4-((5-methyl-1H-pyrazol-3-yl)amino)-7-(thiazol-4-yl)quinazolin-2-yl)amino)-8-azabicyclo[3.2.1]oct-8-yl)propionitrile

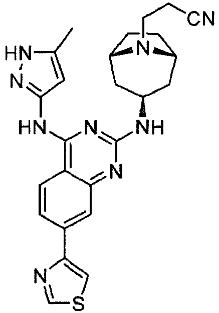

The preparation of 3-((3-*exo*)-3-((4-((5-methyl-1H-pyrazol-3-yl)amino)-7-(thiazol-4-yl)quinazolin-2-yl)amino)-8-azabicyclo[3.2.1]oct-8-yl)propionitrile was carried out with the reference to Embodiment 185.

MS *m*/*z* (ESI): 486.2 [M+H]⁺.

### Embodiment 189

### 3-((3-exo)-3-((4-((5-methyl-1H-pyrazol-3-yl)amino)-7-(pyridin-3-yl)quinazolin-2-yl)amino)-9-azabicyclo[3.3.1]non-9-yl)propionitrile

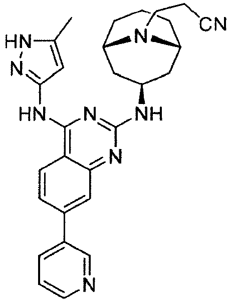

The preparation of 3-((3-*exo*)-3-((4-((5-methyl-1H-pyrazol-3-yl)amino)-7-(pyridin-3-yl)quinazolin-2-yl)amino)-9-azabicyclo[3.3.1]non-9-yl)propionitrile was carried out with the reference to Embodiment 179.

MS *m*/*z* (ESI): 494.3 [M+H]⁺.

### Embodiment 190

### 3-((3-exo)-3-((4-((5-methyl-1H-pyrazol-3-yl)amino)-7-(1-methyl-1H-pyrazol-4-yl)quinazolin-2-yl)amino)-9-azabicyclo[3.3.1]non-9-yl)propionitrile

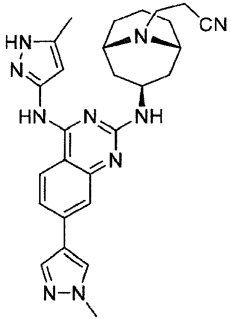

The preparation of 3-((3-*exo*)-3-((4-((5-methyl-1H-pyrazol-3-yl)amino)-7-(1-methyl-1H-pyrazol-4-yl)quinazolin-2-yl)amino)-9-azabicyclo[3.3.1]non-9-yl)propionitrile was carried out with the reference to Embodiment 179.

MS *m*/*z* (ESI):497.3 [M+H]⁺.

### Embodiment 191

### 3-((3-exo)-3-((4-((5-methyl-1H-pyrazol-3-yl)amino)-7-morpholinoquinazolin-2-yl)amino)-8-azabicyclo[3.2.1]oct-8-yl)propionitrile

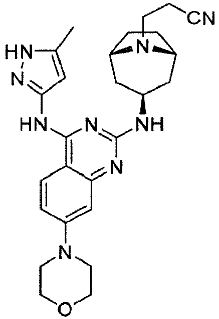

### Step 1: preparation of Tert-butyl (3-exo)-3-((4-((5-methyl-1H-pyrazol-3-yl)amino)-7-morpholinoquinazolin-2-yl)amino)-8-azabicyclo[3.2.1]oct-8-carboxylate

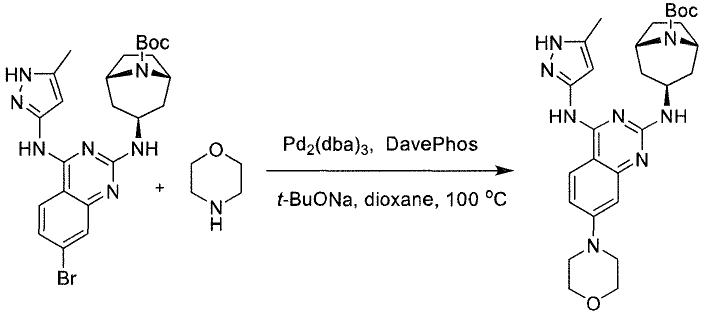

Tert-butyl (3-exo)-3-((7-bromine-4-((5-methyl-1H-pyrazol-3-yl)amino)quinazolin-2-yl)amino)-8-azabicyclo[3.2.1]oct-8-carboxylate (300 mg, 0.568 mmol), morpholine (494 mg, 5.68 mmol), Pd₂(dba)₃ (104 mg, 0.114 mmol), DavePhos(90 mg, 0.227 mmol) and t-BuONa (109 mg, 1.14 mmol) were separately added to dioxane(6 mL). Under nitrogen atmosphere, the mixture was stirred at 100 °C for 4 hours. The reaction mixture was cooled to room temperature, diluted with ethyl acetate (20 mL), and washed with water (20 mL) and saturated sodium chloride aqueous solution (10 mL). The organic phase was collected, dried over anhydrous sodium sulfate, filtered, and concentrated under reduced pressure. The resulting product obtained was purified by silica gel chromatography to obtain the title compound as pale yellow oil (66 mg, 22%).

### Step 2: preparation of 3-((3-exo)-3-((4-((5-methyl-1H-pyrazol-3-yl)amino)-7-morpholinoquinazolin-2-yl)amino)-8-azabicyclo[3.2.1]oct-8-yl)propionitrile

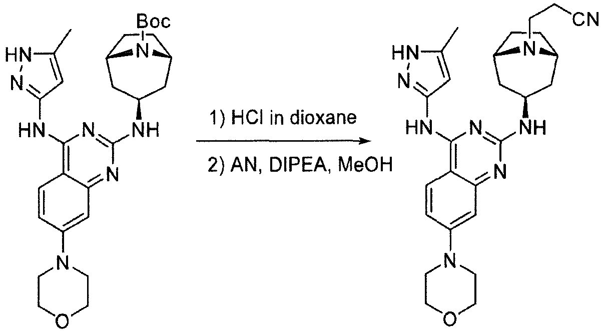

Tert-butyl (3-exo)-3-((4-((5-methyl-1H-pyrazol-3-yl)amino)-7-morpholinoquinazolin-2-yl)amino)-8-azabicyclo[3.2.1]oct-8-carboxylate (66 mg, 0.123 mmol) was dissolved in methanol (2 mL). The mixture was stirred at room temperature, and 4M HCl in 1,4-dioxane (2 mL) was added thereto. The reaction mixture was then stirred at room temperature for 1 hour, and concentrated under reduced pressure. The residue was dissolved in anhydrous methanol (1 mL), and DIPEA (80 mg, 0.617 mmol) and acrylonitrile (20 mg, 0.369 mmol) were successively added thereto. The resulting reaction mixture was stirred at room temperature for 2 hours. The reaction mixture was concentrated under reduced pressure, and the residue was successively purified by silica gel chromatography and preparative TLC, to obtain the title compound as a gray solid (12 mg, 20%).

¹H NMR (400 MHz, CD₃OD) *δ* 8.06 (d, *J=* 9.3 Hz, 1H), 7.08 (d, *J* = 9.4 Hz, 1H), 6.68 (s, 1H), 6.49 (s, 1H), 4.46-4.28 (m, 1H), 3.97-3.73 (m, 4H), 3.52-3.36 (m, 6H), 2.73 (t, *J* = 6.7 Hz, 2H), 2.62 (t, *J* = 6.7 Hz, 2H), 2.34 (s, 3H), 2.11-1.61 (m, 8H).

MS *m*/*z* (ESI): 488.2[M+H]⁺.

### Embodiment 192

### 3-((3-exo)-3-((7-(3-methoxylazetindin-1-yl)-4-((5-methyl-1H-pyrazol-3-yl)amino)quinazolin-2-yl)amino)-8-azabicyclo[3.2.1]oct-8-yl)propionitrile

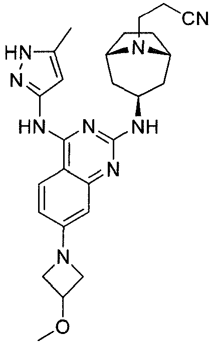

The preparation of 3-((3-*exo*)-3-((7-(3-methoxylazetindin-1-yl)-4-((5-methyl-1H-pyrazol-3-yl)amino)quinazolin-2-yl)amino)-8-azabicyclo[3.2.1]oct-8-yl)propionitrile was carried out with the reference to Embodiment 191.

MS *m*/*z* (ESI): 488.3 [M+H]⁺.

### Embodiment 193

### 3-((3-exo)-3-((7-(4-methoxylpiperidin-1-yl)-4-((5-methyl-1H-pyrazol-3-yl)amino)quinazolin-2-yl)amino)-8-azabicyclo[3.2.1]oct-8-yl)propionitrile

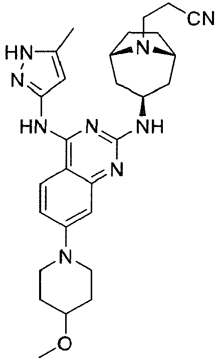

The preparation of 3-((3-*exo*)-3-((7-(4-methoxylpiperidin-1-yl)-4-((5-methyl-1H-pyrazol-3-yl)amino)quinazolin-2-yl)amino)-8-azabicyclo[3.2.1]oct-8-yl)propionitrile was carried out with the reference to Embodiment 191.

MS *m*/*z* (ESI): 516.3 [M+H]⁺.

### Embodiment 194

### 3-((3-exo)-3-((7-(4-(dimethylamine)piperidin-1-yl)-4-((5-methyl-1H-pyrazol-3-yl)amino)quinazolin-2-yl)amino)-8-azabicyclo[3.2.1]oct-8-yl)propionitrile

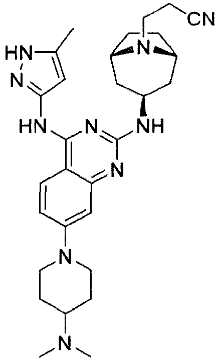

The preparation of 3-((3-*exo*)-3-((7-(4-(dimethylamine)piperidin-1-yl)-4-((5-methyl-1H-pyrazol-3-yl)amino)quinazolin-2-yl)amino)-8-azabicyclo[3.2.1]oct-8-yl)propionitrile was carried out with the reference to Embodiment 191.

MS *m*/*z* (ESI): 529.3 [M+H]⁺.

### Embodiment 195

### 3-((3-exo)-3-((4-((5-methyl-1H-pyrazol-3-yl)amino)-7-(pyrrolidin-1-yl)quinazolin-2-yl)amino)-8-azabicyclo[3.2.1]oct-8-yl)propionitrile

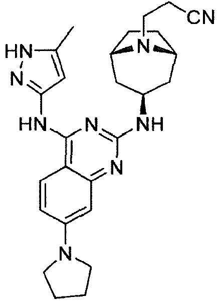

The preparation of 3-((3-*exo*)-3-((4-((5-methyl-1H-pyrazol-3-yl)amino)-7-(pyrrolidin-1-yl)quinazolin-2-yl)amino)-8-azabicyclo[3.2.1]oct-8-yl)propionitrile was carried out with the reference to Embodiment 191.

MS *m*/*z* (ESI): 472.3 [M+H]⁺.

### Embodiment 196

### 3-((3-exo)-3-((4-((5-methyl-1H-pyrazol-3-yl)amino)-7-(methylamino)quinazolin-2-yl)amino)-8-azabicyclo[3.2.1]oct-8-yl)propionitrile

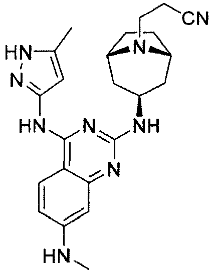

The preparation of 3-((3-*exo*)-3-((4-((5-methyl-1H-pyrazol-3-yl)amino)-7-(methylamino)quinazolin-2-yl)amino)-8-azabicyclo[3.2.1]oct-8-yl)propionitrile was carried out with the reference to Embodiment 191.

MS *m*/*z* (ESI): 432.3 [M+H]⁺.

### Embodiment 197

### 3-((3-exo)-3-((7-(methyl(oxetan-3-ylmethyl)amino)-4-((5-methyl-1H-pyrazol-3-yl)amino)quinazolin-2-yl)amino)-8-azabicyclo[3.2.1]oct-8-yl)propionitrile

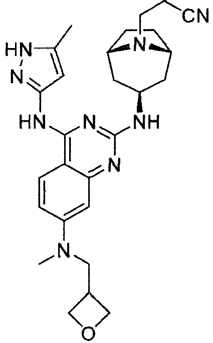

The preparation of 3-((3-*exo*)-3-((7-(methyl(oxetan-3-ylmethyl)amino)-4-((5-methyl-1H-pyrazol-3-yl)amino)quinazolin-2-yl)amino)-8-azabicyclo[3.2.1]oct-8-yl)propionitrile was carried out with the reference to Embodiment 191.

MS *m*/*z* (ESI): 502.3 [M+H]⁺.

### Embodiment 198

### 3-((3-exo)-3-((4-((5-methyl-1H-pyrazol-3-yl)amino)-7-(((1-methylazetindin-3-yl)methyl)amino)quinazolin-2-yl)amino)-8-azabicyclo[3.2.1]oct-8-yl)propionitrile

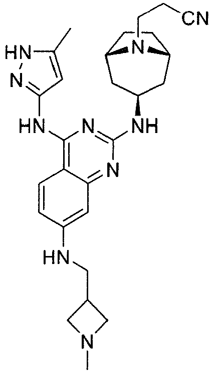

The preparation of 3-((3-*exo*)-3-((4-((5-methyl-1H-pyrazol-3-yl)amino)-7-(((1-methylazetindin-3-yl)methyl)amino)quinazolin-2-yl)amino)-8-azabicyclo[3.2.1]oct-8-yl)propionitrile was carried out with the reference to Embodiment 191.

MS *m*/*z* (ESI): 501.3 [M+H]⁺.

### Embodiment 199

### 3-((3-exo)-3-((4-((5-methyl-1H-pyrazol-3-yl)amino)-7-(((4H-2H-pyran-4-yl)methyl)amino)quinazolin-2-yl)amino)-8-azabicyclo[3.2.1]oct-8-yl)propionitrile

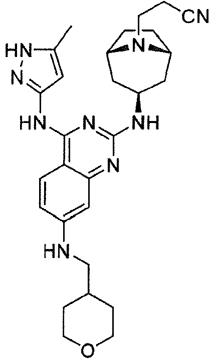

The preparation of 3-((3-*exo*)-3-((4-((5-methyl-1H-pyrazol-3-yl)amino)-7-(((4H-2H-pyran-4-yl)methyl)amino)quinazolin-2-yl)amino)-8-azabicyclo[3.2.1]oct-8-yl)propionitrile was carried out with the reference to Embodiment 191.

MS *m*/*z* (ESI): 516.3 [M+H]⁺.

### Embodiment 200

### 3-((3-exo)-3-((4-((5-methyl-1H-pyrazol-3-yl)amino)-7-(((1-methylpiperidin-4-yl)methyl)amino)quinazolin-2-yl)amino)-8-azabicyclo[3.2.1]oct-8-yl)propionitrile

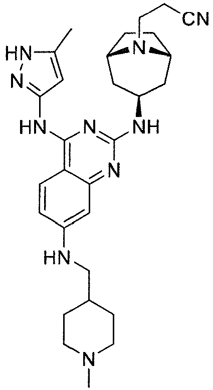

The preparation of 3-((3-*exo*)-3-((4-((5-methyl-1H-pyrazol-3-yl)amino)-7-(((1-methylpiperidin-4-yl)methyl)amino)quinazolin-2-yl)amino)-8-azabicyclo[3.2.1]oct-8-yl)propionitrile was carried out with the reference to Embodiment 191.

MS *m*/*z* (ESI): 529.3 [M+H]⁺.

### Embodiment 201

### 3-((3-exo)-3-((7-(methyl(pyridin-3-ylmethyl)amino)-4-((5-methyl-1H-pyrazol-3-yl)amino)quinazolin-2-yl)amino)-8-azabicyclo[3.2.1]oct-8-yl)propionitrile

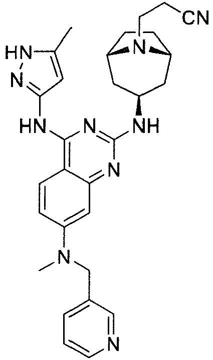

The preparation of 3-((3-*exo*)-3-((7-(methyl(pyridin-3-ylmethyl)amino)-4-((5-methyl-1H-pyrazol-3-yl)amino)quinazolin-2-yl)amino)-8-azabicyclo[3.2.1]oct-8-yl)propionitrile was carried out with the reference to Embodiment 191.

MS *m*/*z* (ESI): 523.3 [M+H]⁺.

### Embodiment 202

### 3-((3-exo)-3-((4-((5-methyl-1H-pyrazol-3-yl)amino)-7-morpholinoquinazolin-2-yl)amino)-9-azabicyclo[3.3.1]non-9-yl)propionitrile

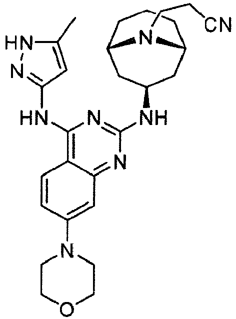

The preparation of 3-((3-*exo*)-3-((4-((5-methyl-1H-pyrazol-3-yl)amino)-7-morpholinoquinazolin-2-yl)amino)-9-azabicyclo[3.3.1]non-9-yl)propionitrile was carried out with the reference to Embodiment 191.

MS *m*/*z* (ESI): 502.3 [M+H]⁺.

### Embodiment 203

### 3-((3-exo)-3-((7-(1H-imidazol-1-yl)-4-((5-methyl-1 H-pyrazol-3 -yl)amino)quinazolin-2-yl)amino)-8-azabicyclo[3.2.1]oct-8-yl)propionitrile

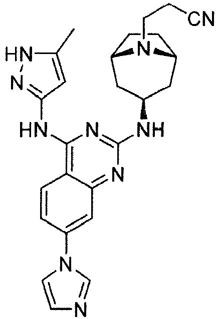

The preparation of 3-((3-*exo*)-3-((7-(1H-imidazol-1-yl)-4-((5-methyl-1H-pyrazol-3-yl)amino)quinazolin-2-yl)amino)-8-azabicyclo[3.2.1]oct-8-yl)propionitrile was carried out with the reference to Embodiment 191.

¹H NMR (400 MHz, CD₃OD) *δ* 8.29 (s, 1H), 8.21 (d, *J* = 8.9 Hz, 1H), 7.70 (s, 1H), 7.64-7.26 (m, 2H), 7.19 (s, 1H), 6.62 (s, 0.8H), 5.93 (s, 0.2H), 4.47-4.22 (m, 1H), 3.41-3.36 (m, 2H), 2.76 (t, *J* = 6.9 Hz, 2H), 2.63 (t, *J* = 6.9 Hz, 2H), 2.33 (s, 3H), 2.08-2.01 (m, 2H), 2.00-1.79 (m, 4H), 1.74-1.63 (m, 2H).

MS *m*/*z* (ESI): 469.2 [M+H]⁺.

### Embodiment 204

### 3-((3-exo)-3-((7-(2-methoxylethoxyl)-4-((5-methyl-1H-pyrazol-3-yl)amino)quinazolin-2-yl)amino)-8-azabicyclo[3.2.1]oct-8-yl)propionitrile

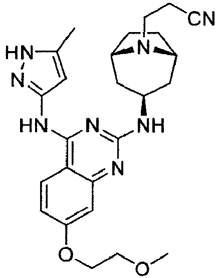

The preparation of 3-((3-*exo*)-3-((7-(2-methoxylethoxyl)-4-((5-methyl-1H-pyrazol-3-yl)amino)quinazolin-2-yl)amino)-8-azabicyclo[3.2.1]oct-8-yl)propionitrile was carried out with the reference to Embodiment 169.

MS *m*/*z* (ESI): 477.3 [M+H]⁺.

### Embodiment 205

### 3-((3-exo)-3-((4-((5-methyl-1H-pyrazol-3-yl)amino)-7-(oxetan-3-ylmethoxyl)quinazolin-2-yl)amino)-9-azabicyclo[3.3.1]non-9-yl)propionitrile

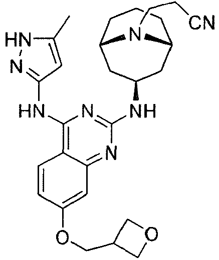

The preparation of 3-((3-*exo*)-3-((4-((5-methyl-1H-pyrazol-3-yl)amino)-7-(oxetan-3-ylmethoxyl)quinazolin-2-yl)amino)-9-azabicyclo[3.3.1]non-9-yl)propionitrile was carried out with the reference to Embodiment 169.

MS *m*/*z* (ESI): 503.3 [M+H]⁺.

### Embodiment 206

### 3-((3-exo)-3-((4-((5-methyl-1H-pyrazol-3-yl)amino)-7-((1-methylazetindin-3-yl)methoxyl)quinazolin-2-yl)amino)-8-azabicyclo[3.2.1]oct-8-yl)propionitrile

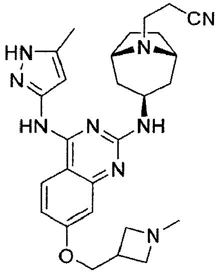

The preparation of 3-((3-*exo*)-3-((4-((5-methyl-1H-pyrazol-3-yl)amino)-7-((1-methylazetindin-3-yl)methoxyl)quinazolin-2-yl)amino)-8-azabicyclo[3.2.1]oct-8-yl)propionitrile was carried out with the reference to Embodiment 169.

MS *m*/*z* (ESI): 502.3 [M+H]⁺.

### Embodiment 207

### 3-((3-exo)-3-((4-((5-methyl-1H-pyrazol-3-yl)amino)-7-(pyridin-3-ylmethoxyl)quinazolin-2-yl)amino)-8-azabicyclo[3.2.1]oct-8-yl)propionitrile

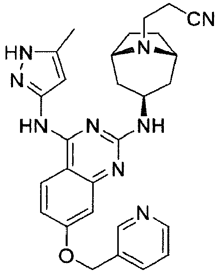

The preparation of 3-((3-*exo*)-3-((4-((5-methyl-1H-pyrazol-3-yl)amino)-7-(pyridin-3-ylmethoxyl)quinazolin-2-yl)amino)-8-azabicyclo[3.2.1]oct-8-yl)propionitrile was carried out with the reference to Embodiment 169.

MS *m*/*z* (ESI): 510.3 [M+H]⁺.

### Embodiment 208

### 3-((3-exo)-3-((4-((5-methyl-1H-pyrazol-3-yl)amino)-7-((1-methylazetindin-3-yl)oxo)quinazolin-2-yl)amino)-8-azabicyclo[3.2.1]oct-8-yl)propionitrile

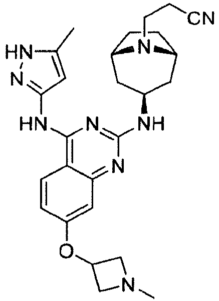

The preparation of 3-((3-*exo*)-3-((4-((5-methyl-1H-pyrazol-3-yl)amino)-7-((1-methylazetindin-3-yl)oxo)quinazolin-2-yl)amino)-8-azabicyclo[3.2.1]oct-8-yl)propionitrile was carried out with the reference to Embodiment 169.

MS *m*/*z* (ESI): 488.3 [M+H]⁺.

### Embodiment 209

### 3-((3-exo)-3-((4-((5-methyl-1H-pyrazol-3-yl)amino)-7-((1-methylpiperidin-4-yl)oxouinazolin-2-yl)amino)-8-azabicyclo[3.2.1]oct-8-yl)propionitrile

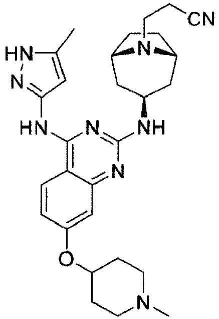

The preparation of 3-((3-*exo*)-3-((4-((5-methyl-1H-pyrazol-3-yl)amino)-7-((1-methylpiperidin-4-yl)oxo)quinazolin-2-yl)amino)-8-azabicyclo[3.2.1]oct-8-yl)propionitrile was carried out with the reference to Embodiment 169.

MS *m*/*z* (ESI): 516.3 [M+H]⁺.

### Embodiment 210

### 3-((3-exo)-3-((4-((5-methyl-1H-pyrazol-3-yl)amino)-7-(morpholinomethyl)quinazolin-2-yl)amino)-9-azabicyclo[3.3.1]non-9-yl)propionitrile

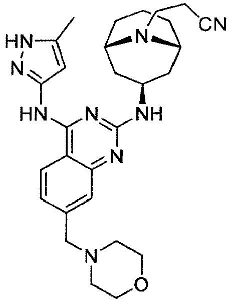

The preparation of 3-((3-*exo*)-3-((4-((5-methyl-1H-pyrazol-3-yl)amino)-7-(morpholinomethyl)quinazolin-2-yl)amino)-9-azabicyclo[3.3.1]non-9-yl)propionitrile was carried out with the reference to Embodiment 169.

MS *m*/*z* (ESI): 516.3 [M+H]⁺.

### Embodiment 211

### 3-((3-exo)-3-((4-((5-methyl-1H-pyrazol-3-yl)amino)-7-(1-methylazetindin-3-yl)quinazolin-2-yl)amino)-9-azabicyclo[3.3.1]non-9-yl)propionitrile

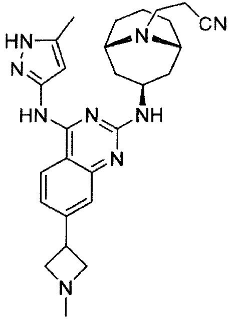

The preparation of 3-((3-*exo*)-3-((4-((5-methyl-1H-pyrazol-3-yl)amino)-7-(1-methylazetindin-3-yl)quinazolin-2-yl)amino)-9-azabicyclo[3.3.1]non-9-yl)propionitrile was carried out with the reference to Embodiment 169.

MS *m*/*z* (ESI):486.3 [M+H]⁺.

### Embodiment 212

### 3-((3-exo)-3-((4-((5-methyl-1H-pyrazol-3-yl)amino)-7-(oxetan-3-yl)quinazolin-2-yl)amino)-9-azabicyclo[3.3.1]non-9-yl)propionitrile

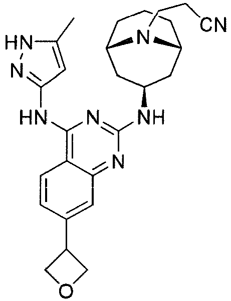

The preparation of 3-((3-*exo*)-3-((4-((5-methyl-1H-pyrazol-3-yl)amino)-7-(oxetan-3-yl)quinazolin-2-yl)amino)-9-azabicyclo[3.3.1]non-9-yl)propionitrile was carried out with the reference to Embodiment 169.

MS *m*/*z* (ESI):473.3 [M+H]⁺.

### Embodiment 213

### 1-(((3-exo)-3-((4-((5-methyl-1H-pyrazol-3-yl)amino)quinazolin-2-yl)amino)-8-azabicyclo[3.2.1]oct-8-yl)sulfonyl)azetindin-3-formonitrile

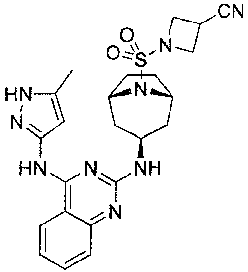

Tert-butyl (3-exo)-3-((4-((5-methyl-1H-pyrazol-3-yl)amino)quinazolin-2-yl)amino)-8-azabicyclo[3.2.1]oct-8-carboxylate (100 mg, 0.222 mmol) was dissolved in 4M HCl in 1,4-dioxane solution (10 mL). The mixture was stirred at room temperature for 30 minutes, and concentrated under reduced pressure to remove the solvent. The residue was dissolved in anhydrous N,N-dimethylformamide (10 mL), then cooled to 0 °C, and DIPEA (0.73 mL, 4.44 mmol) and 3-cyanoazetindin-1-sulfonyl chloride (44 mg, 0.244 mmol) were successively added thereto. The reaction mixture was stirred at 0 °C for 5 hours, and concentrated under reduced pressure to remove the solvent. The residue was purified by reverse phase HPLC to obtain the title compound (59.3 mg, 54%).

¹H NMR (400 MHz, CD₃OD) *δ* 8.12 (s, 1H), 7.50 (s, 1H), 7.29 (d, *J* = 44.1 Hz, 1H), 7.06 (s, 1H), 6.58 (s, 1H), 4.36 (s, 1H), 4.12 (s, 2H), 4.03 (t, *J* = 8.4 Hz, 2H), 3.90 (t, *J* = 7.0 Hz, 2H), 3.69-3.57 (m, 1H), 2.20 (s, 3H), 2.10- 1.51 (m, 8H).

MS *m*/*z* (ESI): 494.2 [M+H]⁺.

### Embodiment 214

### 1-(((3-exo)-3-((7-chloro-4-((5-methyl-1H-pyrazol-3-yl)amino)quinazolin-2-yl)amino)-8-azabicyclo[3.2.1]oct-8-yl)sulfonyl)azetindin-3-formonitrile

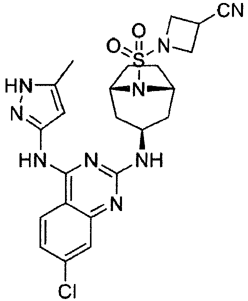

Tert-butyl (3-exo)-3-((7-chloro-4-((5-methyl-1H-pyrazol-3-yl)amino)quinazolin-2-yl)amino)-8-azabicyclo[3.2.1]oct-8-carboxylate (174 mg, 0.36 mmol) was dissolved in 4M HCl in 1,4-dioxane solution (20 mL). The mixture was stirred at room temperature for 30 minutes, and concentrated under reduced pressure to remove the solvent. The residue was dissolved in anhydrous N,N-dimethylformamide (10 mL), then cooled to 0 °C, and DIPEA (1.19 mL, 7.2 mmol) and 3-cyanoazetindin-1-sulfonyl chloride (78 mg, 0.432 mmol) were successively added thereto. The reaction mixture was stirred at 0 °C for 16.5 hour, and concentrated under reduced pressure to remove the solvent. The residue was purified by reverse phase HPLC to obtain the title compound (17.7 mg, 9 %).

¹H NMR (400 MHz, MeOD-*d₄*) *δ* 8.02 (s, 1H), 7.42 (s, 1H), 7.20 (s, 1H), 6.57 (s, 1H), 4.51-4.40 (m, 1H), 4.27 (s, 2H), 4.17 (t, *J* = 8.5 Hz, 2H), 4.13-4.05 (m, 2H), 3.64-3.53 (m, 1H), 2.34 (s, 3H), 2.16 (s, 4H), 1.98 (d, *J* = 42.2 Hz, 2H), 1.76 (t, *J=* 11.9 Hz, 2H).

MS *m*/*z* (ESI): 528.2 [M+H]⁺.

### Embodiment 215

### 1-(((3-exo)-3-((7-fluoro-4-((5-methyl-1H-pyrazol-3-yl)amino)quinazolin-2-yl)amino)-8-azabicyclo[3.2.1]oct-8-yl)sulfonyl)azetindin-3-formonitrile

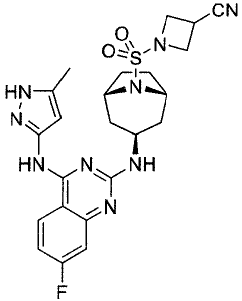

The preparation of 1-(((3-*exo*)-3-((7-fluoro-4-((5-methyl-1H-pyrazol-3-yl)amino)quinazolin-2-yl)amino)-8-azabicyclo[3.2.1]oct-8-yl)sulfonyl)azetindin-3-formonitrile was carried out with the reference to Embodiment 213.

¹H NMR (400 MHz, CD₃OD) *δ* 8.11 (dd, *J* = 9.1, 6.1 Hz, 1H), 7.21-6.82 (m, 2H), 6.56 (s, 0.8H), 5.88 (s, 0.2H), 4.58-4.34 (m, 1H), 4.29-4.19 (m, 2H), 4.17-4.08 (m, 2H), 4.06-3.96 (m, 2H), 3.72-3.58 (m, 1H), 2.31 (s, 3H), 2.18-1.85 (m, 6H), 1.82-1.66 (m, 2H).

MS *m*/*z* (ESI): 512.1[M+H]⁺.

### Embodiment 216

### 1-(((3-exo)-3-((7-cyclopropyl-4-((5-methyl-1H-pyrazol-3-yl)amino)quinazolin-2-yl)amino)-8-azabicyclo[3.2.1]oct-8-yl)sulfonyl)azetindin-3-formonitrile

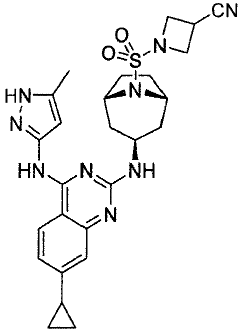

The preparation of 1-(((3-*exo*)-3-((7-cyclopropyl-4-((5-methyl-1H-pyrazol-3-yl)amino)quinazolin-2-yl)amino)-8-azabicyclo[3.2.1]oct-8-yl)sulfonyl)azetindin-3-formonitrile was carried out with the reference to Embodiment 213.

¹H NMR (400 MHz, CD₃OD) *δ* 8.04 (s, 1H), 7.26-6.93 (m, 2H), 6.50 (s, 1H), 4.58-4.37 (m, 1H), 4.31-4.18 (m, 2H), 4.18-4.07 (m, 2H), 4.06-3.95 (m, 2H), 3.71-3.58 (m, 1H), 2.32 (s, 3H), 2.17-1.71 (m, 8H), 1.34-1.25 (m, 1H), 1.19-1.03 (m, 2H), 0.94-0.75 (m, 2H).

MS *m*/*z* (ESI): 534.1[M+H]⁺.

### Embodiment 217

### 3-((3-exo)-3-(((4-((5-methyl-1H-pyrazol-3-yl)amino)quinazolin-2-yl)amino)-9-azabicyclo[3.3.1]non-9-yl)sulfonyl)azacyclobutane-3-nitrile

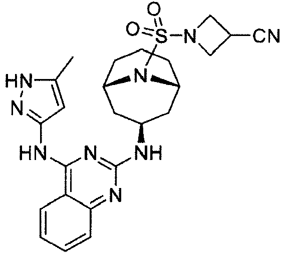

### Step 1: preparation of tert-butyl-(3-exo)-3-((4-((5-methyl-1H-pyrazol-3-yl)amino)quinazolin-2-yl)amino)-9-azabicyclo[3.3.1]non-9-carboxylate

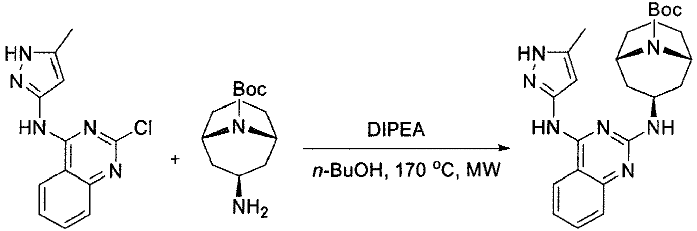

Tert-butyl-(3-*exo*)-3-amino-9-azabicyclo[3.3.1]non-9-carboxylate (222 mg, 0.92 mmol) and DIPEA (199 mg, 1.54 mmol) were successively added to the solution of 2-chloro-N-(5-methyl-1H-pyrazol-3-yl)quinazolin-4-amine(200 mg, 0.77 mmol) in n-butanol (10 mL). The mixture was then stirred at 170°C under microwave condition for 4 hours. When the reaction was completed, the reaction mixture was extracted with ethyl acetate (15 mL x 3), and washed with saturated sodium chloride aqueous solution (15 mL x 3). The organic phase was collected, dried over anhydrous sodium sulfate, filtered, and concentrated under reduced pressure. The resulting product was purified by silica gel column chromatography (dichloromethane: methanol=95:5) to obtain the title compound as a white solid (275 mg, 77%).

MS *m*/*z* (ESI): 464.2 [M+H]⁺.

### Step 3: preparation of 3-((3-exo)-3-(((4-((5-methyl-1H-pyrazol-3-yl)amino)quinazolin-2-yl)amino)-9-azabicyclo[3.3.1]non-9-yl)sulfonyl)azacyclobutane-3-nitrile

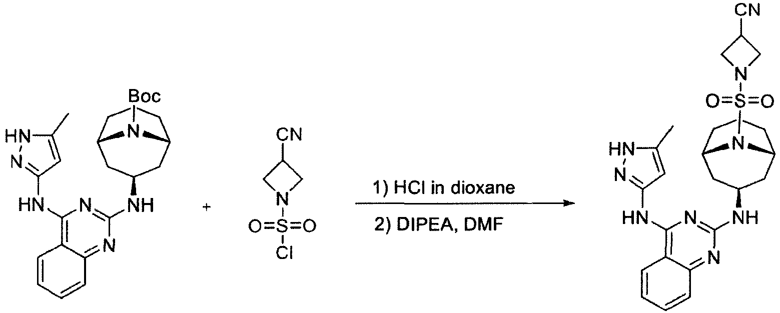

Tert-butyl-(3-*exo*)-3-((4-((5-methyl-1H-pyrazol-3-yl)amino)quinazolin-2-yl)amino)-9-azabicyclo[3.3.1]non-9-carboxylate (100 mg, 0.21 mmol) was dissolved in hydrochloride 1,4-dioxane solution(4.0 N, 5 mL). The mixture was stirred at room temperature for 30 minutes, concentrated, and then dissolved in N,N-dimethylformamide(10 mL). DIPEA (108 mg, 0.84 mmol) was slowly added dropwise into the mixture, and the mixture was stirred at room temperature for 10 minutes. 3-Nitrile azacyclobutane-1-sulfonylchlorine (45 mg, 0.25 mmol) was added to the mixture, and the mixture was stirred at room temperature overnight. The reaction mixture was concentrated under reduced pressure, and the resulting product was purified by prep-HPLC to obtain the title compound as a white solid (30.5 mg, 29%).

¹H NMR (400 MHz, DMSO) *δ* = 12.09 (s, 1H), 10.04 (s, 1H), 8.24 (s, 1H), 7.45 (s, 1H), 7.33-6.42 (m, 4H), 4.79 (s, 1H), 4.01-3.79 (m, 6H), 3.74-3.67 (m, 1H), 2.15 (s, 3H), 2.09-1.57 (m, 10H).

MS *m*/*z* (ESI): 508.2 [M+H]⁺.

### Embodiment 218

### 1-(((3-exo)-3-((7-methoxyl-4-((5-methyl-1H-pyrazol-3-yl)amino)quinazolin-2-yl)(methyl)amino)-8-azabicyclo[3.2.1]oct-8-yl)sulfonyl)azetindin-3-formonitrile

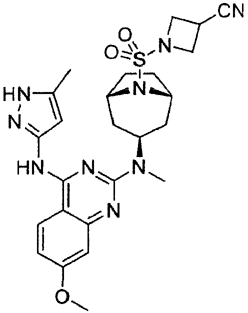

### Step 1: preparation of 2-chloro-7-methoxyl-N-(5-methyl-1H-pyrazol-3-yl)quinazolin-4-amine

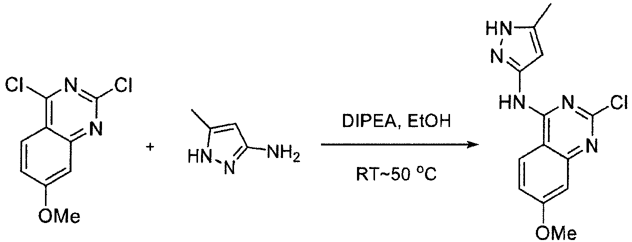

2,4-Dichloro-7-methoxylquinazoline (497 mg, 2.17 mmol), 5-methyl-1H-pyrazol-3-amine (221 mg, 2.28 mmol) and DIPEA (0.75 mL, 4.56 mmol) were added to anhydrous ethanol (10 mL). The mixture was stirred at room temperature for 24 hours, heated to 50 °C, reacted for 5 hour and then concentrated under reduced pressure to remove the solvent. The residue was washed with the mixed solvent of ethanol- water (v/v = 1: 9, 20 mL), and filtered. The residue was dried under reduced pressure to obtain the title compound (509 mg, 81%).

MS *m*/*z* (ESI): 290.0 [M+H]⁺.

### Step 2: preparation of Tert-butyl (3-exo)-3-((7-methoxyl-4-((5-methyl-1H-pyrazol-3-yl)amino)quinazolin-2-yl)(methyl)amino)-8-azabicyclo[3.2.1]oct-8-carboxylate

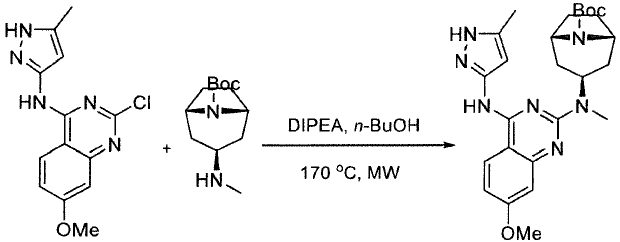

2-Chloro-7-methoxyl-N-(5-methyl-1H-pyrazol-3-yl)quinazolin-4-amine (150 mg, 0.518 mmol), Tert-butyl (3-exo)-3-(methylamino)-8-azabicyclo[3.2.1]oct-8-carboxylate (249 mg, 1.036 mmol) and DIPEA (0.43 mL, 2.59 mmol) were added to n-butanol (3 mL). The mixture was heated to 170 °C with a microwave reactor, reacted for 6 hours, and concentrated under reduced pressure to remove the solvent. The residue was purified by reverse phase chromatography to obtain the title compound (193 mg, 75%).

MS *m*/*z* (ESI): 494.2 [M+H]⁺.

### Step 3: preparation of 1-(((3-exo)-3-((7-methoxyl-4-((5-methyl-1H-pyrazol-3-yl)amino)quinazolin-2-yl)(methyl)amino)-8-azabicyclo[3.2.1]oct-8-yl)sulfonyl)azetindin-3-formonitrile

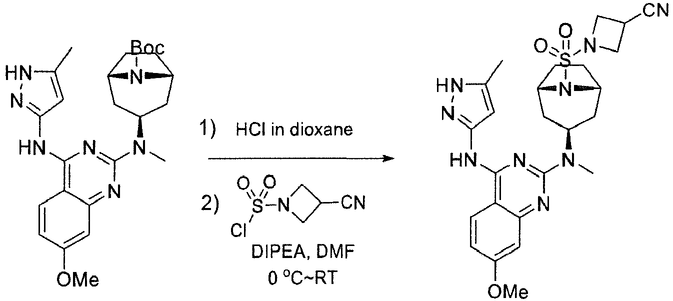

Tert-butyl (3-exo)-3-((7-methoxyl-4-((5-methyl-1H-pyrazol-3-yl)amino)quinazolin-2-yl)(methyl)amino)-8-azabicyclo[3.2.1]oct-8-carboxylate (193 mg, 0.39 mmol) was dissolved in 4 M HCl in 1,4-dioxane (20 mL). The mixture was stirred at room temperature for 60 minutes, and concentrated under reduced pressure to remove the solvent. The residue solid was dissolved in anhydrous N,N-dimethylformamide (10 mL), then cooled to 0 °C, and DIPEA (1.93 mL, 11.7 mmol) and 3-cyanoazetindin-1-sulfonyl chloride (71 mg, 0.39 mmol) were successively added thereto. The reaction mixture stirred at 0 °C for 4 hours, and concentrated under reduced pressure to remove the solvent. The residue was purified by prep-HPLC to obtain the title compound (97 mg, 46%).

¹H NMR (400 MHz, DMSO-*d₆*) *δ* 12.11 (s, 1H), 9.77 (s, 1H), 8.23 (d, *J* = 8.9 Hz, 1H), 6.72 (s, 1H), 6.67 (d, *J* = 8.8 Hz, 1H), 6.48 (s, 1H), 5.40-5.25 (m, 1H), 4.18 (s, 2H), 4.06 (t, *J* = 8.6 Hz, 2H), 4.01- 3.92 (m, 2H), 3.87-3.74 (m, 4H), 2.96 (s, 3H), 2.23 (s, 3H), 2.06-1.89 (m, 4H), 1.83 (d, *J* = 5.8 Hz, 2H), 1.61 (d, *J* = 11.2 Hz, 2H).

MS *m*/*z* (ESI): 538.2 [M+H]⁺.

### Embodiment 219

### 1-(((3-exo)-3-((7-methoxyl-4-((5-methyl-1H-pyrazol-3-yl)amino)quinazolin-2-yl)amino)-9-azabicyclo[3.3.1]non-9-yl)sulfonyl)azetindin-3-formonitrile

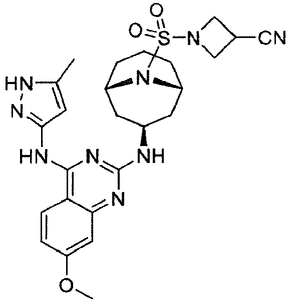

### Step 1: preparation of tert-butyl (3-exo)-3-((7-methoxyl-4-((5-methyl-1H-pyrazol-3-yl)amino)quinazolin-2-yl)amino)-9-azabicyclo[3.3.1]non-9-carboxylate

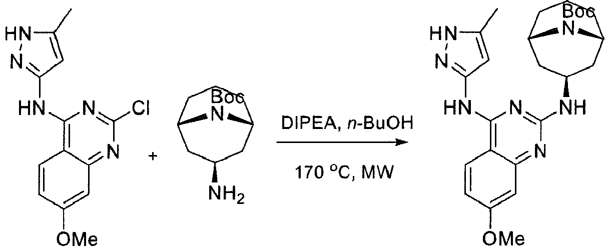

2-Chloro-7-methoxyl-N-(5-methyl-1H-pyrazol-3-yl)quinazolin-4-amine (50 mg, 0.173 mmol) and Tert-butyl (3-exo)-3-amino-9-azabicyclo[3.3.1]non-9-carboxylate oxalate (171 mg, 0.518 mmol) were added to n-butanol (10 mL). The mixture was heated to 170 °C with a microwave reactor, reacted for 8 hours, and concentrated under reduced pressure to remove the solvent. The residue was purified by silica gel chromatography to obtain the title compound (68 mg, 80%).

MS *m*/*z* (ESI): 494.2 [M+H]⁺.

### Step 2: preparation of 1-(((3-exo)-3-((7-methoxyl-4-((5-methyl-1H-pyrazol-3-yl)amino)quinazolin-2-yl)amino)-9-azabicyclo[3.3.1]non-9-yl)sulfonyl)azetindin-3-formonitrile

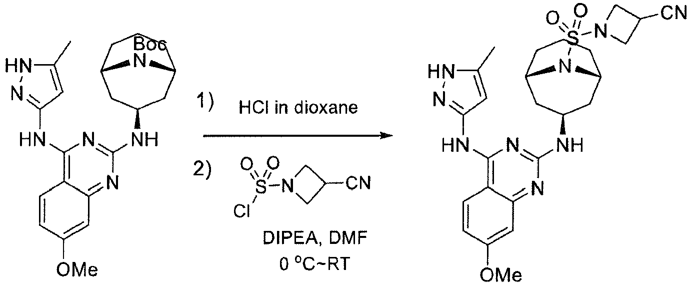

Tert-butyl (3-exo)-3-((7-methoxyl-4-((5-methyl-1H-pyrazol-3-yl)amino)quinazolin-2-yl)amino)-9-azabicyclo[3.3.1]non-9-carboxylate (68 mg, 0.138 mmol) was dissolved in 4 M HCl in 1,4-dioxane (15 mL). The mixture was stirred at room temperature for 60 minutes, and concentrated under reduced pressure to remove the solvent. The residue solid was dissolved in anhydrous N,N-dimethylformamide (10 mL), then cooled to 0 °C, and DIPEA (0.68 mL, 4.14 mmol) and 3-cyanoazetindin-1-sulfonyl chloride (25 mg, 0.138 mmol) were successively added thereto. The reaction mixture was stirred at 0 °C for 8 hours, and concentrated under reduced pressure to remove the solvent. The residue was purified by prep-HPLC to obtain the title compound (6.9 mg, 9%).

¹H NMR (400 MHz, DMSO-*d₆*) *δ* 12.10 (s, 1H), 9.83 (s, 1H), 8.24 (d, *J* = 7.3 Hz, 1H), 6.65 (dd, *J* = 29.7, 20.4 Hz, 4H), 4.83 (s, 1H), 4.02 (t, *J* = 8.5 Hz, 2H), 3.92 (dd, *J* = 14.9, 8.4 Hz, 4H), 3.87-3.73 (m, 4H), 2.21 (s, 3H), 2.04 (d, *J=* 4.3 Hz, 3H), 1.92-1.68 (m, 7H).

MS *m*/*z* (ESI): 538.2 [M+H]⁺.

### Embodiment 220

### 1-(((3-exo)-3-((4-((5-methyl-1H-pyrazol-3-yl)amino)pyrido[3,2-d]pyrimidin-2-yl)amino)-8-azabicyclo[3.2.1]oct-8-yl)sulfonyl)azetindin-3-formonitrile

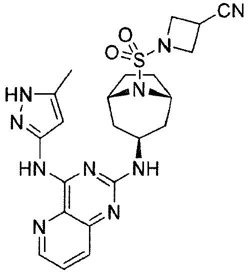

The preparation of 1-(((3-*exo*)-3-((4-((5-methyl-1H-pyrazol-3-yl)amino)pyrido[3,2-d]pyrimidin-2-yl)amino)-8-azabicyclo[3.2.1]oct-8-yl)sulfonyl)azetindin-3-formonitrile was carried out with the reference to Embodiment 213.

¹H NMR (400 MHz, DMSO-*d₆*) *δ* 12.61-12.05 (m, 1H), 10.86 (s, 0.2H), 9.45-9.08 (m, 0.8H), 8.40 (s, 1H), 7.82-7.49 (m, 2H), 7.20-6.96 (m, 1H), 6.93-6.59 (m, 0.8H), 6.08 (s, 0.2H), 4.47-4.27 (m, 1H), 4.22-4.11 (m, 2H), 4.10-4.01 (m, 2H), 3.99-3.88 (m, 2H), 3.87-3.74 (m, 1H), 2.26 (s, 3H), 2.14-1.79 (m, 6H), 1.76-1.57 (m, 2H).

MS *m*/*z* (ESI): 495.1[M+H]⁺.

### Embodiment 221

### 1-(((3-exo)-3-((4-((5-methylthioazole-2-yl)amino)quinazolin-2-yl)amino)-8-azabicyclo[3.2.1]oct-8-yl)sulfonyl)azetindin-3-formonitrile

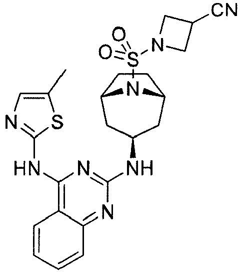

### Step 1: preparation of Tert-butyl (3-exo)-3-((4-((5-methylthioazole-2-yl)amino)quinazolin-2-yl)amino)-8-azabicyclo[3.2.1]oct-8-carboxylate

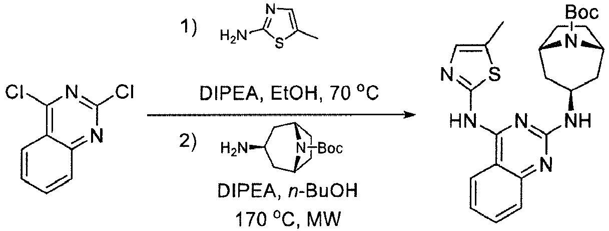

2,4-Dichloroquinazoline (398 mg, 2.0 mmol), 5-methylthioazole-2-amine(251 mg, 2.2 mmol) and DIPEA (1 mL, 6.0 mmol) were dispersed in anhydrous ethanol (10 mL). The resulting reaction mixture was heated to 70 °C and stirred for 24 hours. Then mixture was then cooled to room temperature, and filtered. The yellow solid obtained was washed with a small amount of anhydrous ethanol and dried. The crude product was dispersed in n-butanol (10 mL), and Tert-butyl (3-exo)-3-amino-8-azabicyclo[3.2.1]oct-8-carboxylate (452 mg, 2.0 mmol) and DIPEA (0.33 mL, 2.0 mmol) were added thereto. The mixture was stirred at room temperature and mixed evenly. The mixture was then transferred to a microwave reactor, heated to 170 °C, and reacted for 3 hour. The mixture was concentrated under reduced pressure to remove the solvent, and the crude product was purified by silica gel chromatography to obtain the title compound as a pale yellow solid (45 mg, 5%).

MS *m*/*z* (ESI): 467.1 [M+H]⁺.

### Step 2: preparation of 1-(((3-exo)-3-((4-((5-methylthioazole-2-yl)amino)quinazolin-2-yl)amino)-8-azabicyclo[3 .2.1]oct-8-yl)sulfonyl)azetindin-3-formonitrile

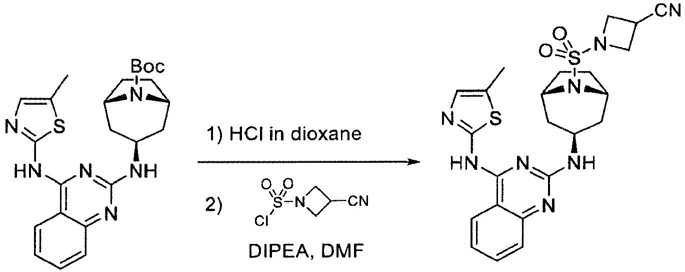

Tert-butyl (3-exo)-3-((4-((5-methylthioazole-2-yl)amino)quinazolin-2-yl)amino)-8-azabicyclo[3.2.1]oct-8-carboxylate (45 mg, 0.097 mmol) was dissolved in 4 M HCl in 1,4-dioxane (15 mL). The mixture was stirred at room temperature for 60 minutes, and concentrated under reduced pressure to remove the solvent. The residue solid was dissolved in anhydrous N,N-dimethylformamide (5 mL), then cooled to 0 °C, and DIPEA (0.32 mL, 1.94 mmol) and 3-cyanoazetindin-1-sulfonyl chloride (19 mg, 0.106 mmol) were successively added thereto. The reaction mixture was stirred at 0 °C for 5 hours, and concentrated under reduced pressure to remove the solvent. The residue was purified by prep-HPLC to obtain the title compound as a pale yellow solid compound (12.4 mg, 25%).

¹H NMR (400 MHz, CD₃OD: CDCl₃, v/v= 1:2) *δ* 8.30 (d, *J* = 4.5 Hz, 1H), 7.71-7.65 (m, 1H), 7.39-7.27 (m, 2H), 7.17 (s, 1H), 4.86-4.79 (m, 1H), 4.32 (s, 2H), 4.19 (t, *J=* 8.5 Hz, 2H), 4.11-4.04 (m, 2H), 3.62 (ddd, *J* = 15.1, 8.5, 6.1 Hz, 1H), 2.45 (s, 3H), 2.32-2.16 (m, 4H), 2.09 (d, *J* = 7.6 Hz, 2H), 1.93-1.84 (m, 2H).

MS *m*/*z* (ESI): 511.1 [M+H]⁺.

### Embodiment 222

### 1-(((3-exo)-3-((7-chloro-4-((5-methylthioazole-2-yl)amino)quinazolin-2-yl)amino)-8-azabicyclo[3.2.1]oct-8-yl)sulfonyl)azetindin-3-formonitrile

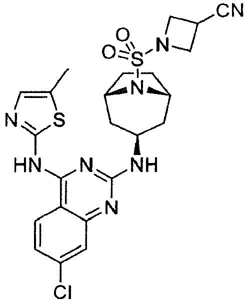

The preparation of 1-(((3-*exo*)-3-((7-chloro-4-((5-methylthioazole-2-yl)amino)quinazolin-2-yl)amino)-8-azabicyclo[3.2.1]oct-8-yl)sulfonyl)azetindin-3-formonitrile was carried out with the reference to Embodiment 221.

MS *m*/*z* (ESI): 545.1 [M+H]⁺.

### Embodiment 223

### 1-(((3-exo)-3-((7-methoxyl-4-((5-methylthioazole-2-yl)amino)quinazolin-2-yl)amino)-8-azabicyclo[3.2.1]oct-8-yl)sulfonyl)azetindin-3-formonitrile

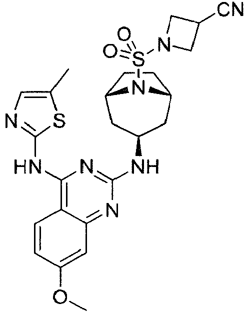

The preparation of 1-(((3-*exo*)-3-((7-methoxyl-4-((5-methylthioazole-2-yl)amino)quinazolin-2-yl)amino)-8-azabicyclo[3.2.1]oct-8-yl)sulfonyl)azetindin-3-formonitrile was carried out with the reference to Embodiment 221.

MS *m*/*z* (ESI): 541.2 [M+H]⁺.

### Embodiment 224

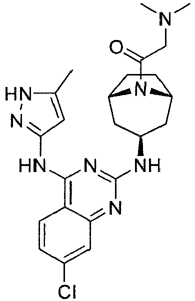

Tert-butyl (3-exo)-3-((7-chloro-4-((5-methyl-1H-pyrazol-3-yl)amino)quinazolin-2-yl)amino)-8-azabicyclo[3.2.1]oct-8-carboxylate was dispersed in dichloromethane (2 mL), and 4 M HCl in 1,4-dioxane solution (20 mL) was added thereto. The mixture was stirred at room temperature for 1.5 hours, and concentrated under reduced pressure to remove the solvent. The residue was dried with an oil pump under reduced pressure for 10 minutes. The crude product obtained was dissolved in anhydrous N,N-dimethylformamide(8 mL), then cooled in ice water bath to 0 °C, and DIPEA (1.2 mL, 7.1 mmol), dimethyl glycine (0.31 mL, 4.72 mmol) and HATU (118 mg, 0.31 mmol) were successively added thereto with stirring. The resulting reaction mixture was stirred at 0 °C for 60 minutes, and concentrated under reduced pressure to remove the solvent. Tthe crude product was purified by prep-HPLC to obtain the title compound as a white solid (20.7 mg, 21%).

¹H NMR (400 MHz, DMSO-*d₆*) *δ* 10.12 (s, 1H), 8.33 (s, 1H), 7.31 (s, 1H), 7.17 (s, 1H), 7.13-6.86 (m, 2H), 6.60 (s, 1H), 4.52 (s, 1H), 4.42 (d, *J* = 3.4 Hz, 2H), 3.16 (s, 2H), 2.38-2.12 (m, 9H), 2.05-1.94 (m, 2H), 1.93-1.73 (m, 4H), 1.63-1.46 (m, 2H).

### MS m/z (ESI): 469.1 [M+H]⁺.

### Embodiment 225

### 2-(dimethylamine)-1-((3-exo)-3-((4-((5-methyl-1H-pyrazol-3-yl)amino)quinazolin-2-yl)amino)-8-azabicyclo[3.2.1]oct-8-yl)ethane-1-one

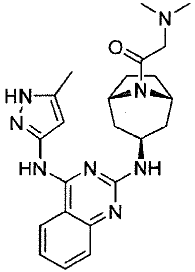

The preparation of 2-(dimethylamine)-1-((3-*exo*)-3-((4-((5-methyl-1H-pyrazol-3-yl)amino)quinazolin-2-yl)amino)-8-azabicyclo[3.2.1]oct-8-yl)ethane-1-one was carried out with the reference to Embodiment 224.

¹H NMR (400 MHz, DMSO-*d₆*) *δ* 12.21 (s, 1H), 10.13 (s, 1H), 8.33 (s, 1H), 7.54 (t, *J* = 7.5 Hz, 1H), 7.27 (d, *J* = 30.8 Hz, 1H), 7.08 (s, 1H), 6.78 (s, 1H), 6.61 (s, 1H), 4.56 (d, *J* = 6.1 Hz, 1H), 4.48 (s, 1H), 4.32 (d, *J* = 5.3 Hz, 1H), 3.65 (dd, *J* = 32.9, 14.8 Hz, 2H), 2.53 (s, 6H), 2.25 (s, 3H), 1.94 (ddd, *J* = 36.8, 20.0, 10.6 Hz, 6H), 1.56 (dd, *J* = 19.2, 9.5 Hz, 2H).

MS *m*/*z* (ESI): 435.2 [M+H]⁺.

### Embodiment 226

### ((3-exo)-3-((4-((5-methyl-1H-pyrazol-3-yl)amino)quinazolin-2-yl)amino)-8-azabicyclo[3.2.1]oct-8-yl)(pyridin-2-yl)methanone

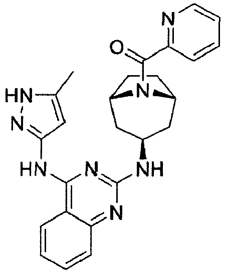

The preparation of ((3-*exo*)-3-((4-((5-methyl-1H-pyrazol-3-yl)amino)quinazolin-2-yl)amino)-8-azabicyclo[3.2.1]oct-8-yl)(pyridin-2-yl)methanone was carried out with the reference to Embodiment 224.

¹H NMR (400 MHz, DMSO-*d₆*) *δ* 12.29 (s, 1H), 10.80 (s, 1H), 10.33 (s, 1H), 8.61 (d, *J* = 4.3 Hz, 1H), 8.52-8.24 (m, 1H), 7.96 (td, *J* = 7.8, 1.7 Hz, 1H), 7.73 (d, *J* = 7.8 Hz, 1H), 7.71-7.57 (m, 1H), 7.52 (ddd, *J* = 7.5, 4.9, 1.0 Hz, 1H), 7.39 (d, *J* = 18.4 Hz, 1H), 7.21 (d, *J* = 39.2 Hz, 1H), 6.60 (s, 1H), 4.74 (s, 1H), 4.69-4.44 (m, 2H), 2.27 (s, 3H), 2.12-1.69 (m, 8H).

MS *m*/*z* (ESI): 455.2 [M+H]⁺.

### Embodiment 227

### ((3-exo)-3-((4-((5-methyl-1H-pyrazol-3-yl)amino)quinazolin-2-yl)amino)-8-azabicyclo[3.2.1]oct-8-yl)(pyridin-3-yl)methanone

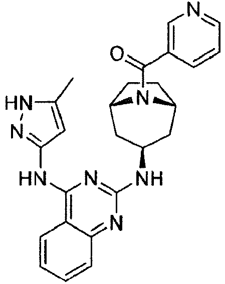

The preparation of ((3-*exo*)-3-((4-((5-methyl-1H-pyrazol-3-yl)amino)quinazolin-2-yl)amino)-8-azabicyclo[3.2.1]oct-8-yl)(pyridin-3-yl)methanone was carried out with the reference to Embodiment 224.

¹H NMR (400 MHz, DMSO-*d₆*) *δ* 12.25 (s, 1H), 10.36 (s, 1H), 10.05 (s, 1H), 8.78-8.62 (m, 2H), 8.34 (d, *J* = 29.6 Hz, 1H), 7.90 (d, *J* = 7.0 Hz, 1H), 7.62-7.48 (m, 2H), 7.31 (dd, *J* = 19.3, 8.1 Hz, 1H), 7.12 (s, 1H), 6.60 (s, 1H), 4.68 (d, *J* = 4.8 Hz, 1H), 4.53 (d, *J* = 9.2 Hz, 1H), 4.02 (*d, J* = 3.1 Hz, 1H), 2.25 (s, 3H), 2.16-1.48 (m, 8H).

MS *m*/*z* (ESI): 455.2 [M+H]⁺.

### Embodiment 228

### ((3-exo)-3-((4-((5-methyl-1H-pyrazol-3-yl)amino)quinazolin-2-yl)amino)-8-azabicyclo[3.2.1]oct-8-yl)(pyridin-4-yl)methanone

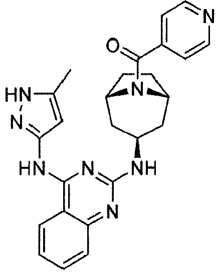

The preparation of ((3-*exo*)-3-((4-((5-methyl-1H-pyrazol-3-yl)amino)quinazolin-2-yl)amino)-8-azabicyclo[3.2.1]oct-8-yl)(pyridin-4-yl)methanone was carried out with the reference to Embodiment 224.

¹H NMR (400 MHz, DMSO-*d₆*) *δ* 12.23 (s, 1H), 10.21 (s, 1H), 8.70 (d, *J* = 5.5 Hz, 2H), 8.32 (dd, *J* = 28.3, 8.1 Hz, 1H), 7.59-7.51 (m, 1H), 7.45 (d, *J=* 1.8 Hz, 2H), 7.36-7.22 (m, 1H), 7.09 (t, *J* = 7.4 Hz, 1H), 6.86 (s, 1H), 6.59 (s, 1H), 4.67 (d, *J* = 4.9 Hz, 1H), 4.61-4.44 (m, 1H), 3.94 (d, *J* = 1.9 Hz, 1H), 2.24 (s, 3H), 2.09-1.53 (m, 8H).

MS *m*/*z* (ESI): 455.2 [M+H]⁺.

### Embodiment 229

### 1-((3-exo)-3-((4-((5-methylthioazole-2-yl)amino)quinazolin-2-yl)amino)-8-azabicyclo[3.2.1]oct-8-yl)-2-morpholinoethane-1-one

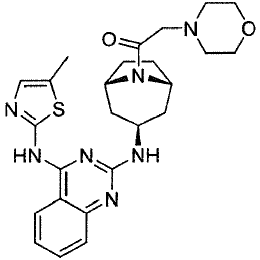

The preparation of 1-((3-*exo*)-3-((4-((5-methylthioazole-2-yl)amino)quinazolin-2-yl)amino)-8-azabicyclo[3.2.1]oct-8-yl)-2-morpholinoethane-1-one was carried out with the reference to Embodiment 224.

MS *m*/*z* (ESI): 494.2 [M+H]⁺.

### Embodiment 230

### 2-(methylamino)-1-((3-exo)-3-((4-((5-methylthioazole-2-yl)amino)quinazolin-2-yl)amino)-9-azabicyclo[3.3.1]non-9-yl)ethane-1-one

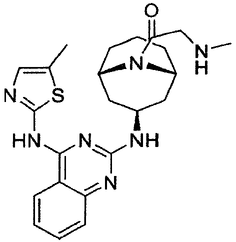

The preparation of 2-(methylamino)-1-((3-*exo*)-3-((4-((5-methylthioazole-2-yl)amino)quinazolin-2-yl)amino)-9-azabicyclo[3.3.1]non-9-yl)ethane-1-one was carried out with the reference to Embodiment 224.

MS *m*/*z* (ESI): 452.2 [M+H]⁺.

### Embodiment 231

### 2,2-difluoro-1-((3-exo)-3-((4-((5-methyl-1H-pyrazol-3-yl)amino)quinazolin-2-yl)amino)-8-azabicyclo[3.2.1]oct-8-yl)ethane-1-one

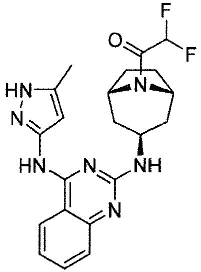

Tert-butyl (3-exo)-3-((4-((5-methyl-1H-pyrazol-3-yl)amino)quinazolin-2-yl)amino)-8-azabicyclo[3.2.1]oct-8-carboxylate (100 mg, 0.222 mmol) was dissolved in 4M HCl in 1,4-dioxane solution (10 mL). The mixture was stirred at room temperature for 30 minutes and concentrated under reduced pressure to remove the solvent. The residue was dissolved in anhydrous N,N-dimethylformamide (10 mL), then cooled to 0 °C, and DIPEA (0.73 mL, 4.44 mmol) was added thereto. The mixture was mixed evenly and the mixture (pre-dissolved in 1 mL dry N,N-dimethylformamide for 10 minutes reaction) of difluoroacetic acid (0.023 mL, 0.233 mmol) and HATU (169 mg, 4.44 mmol) were added thereto. The reaction mixture was stirred at 0 °C for 1 hour, and concentrated under reduced pressure to remove the solvent. The residue was purified by reverse phase HPLC to obtain the title compound (48.9 mg, 52%).

¹H NMR (400 MHz, MeOD-*d₄*) *δ* 7.99 (d, *J* = 7.7 Hz, 1H), 7.56-7.44 (m, 1H), 7.40-7.19 (m, 1H), 7.13-7.03 (m, 1H), 6.55 (d, *J=* 4.7 Hz, 1H), 6.33 (t, *J* = 53.6 Hz, 1H), 4.57 (s, 2H), 4.46-4.40 (m, 1H), 2.18 (d, *J* = 33.6 Hz, 3H), 2.09-1.75 (m, 6H), 1.56 (t, *J* = 12.1 Hz, 2H).

MS *m*/*z* (ESI): 428.1 [M+H]⁺.

### Embodiment 232

### N4-(5-methyl-1H-pyrazol-3-yl)-N2-((3-exo)-8-(pyridin-3-ylsulfonyl)-8-azabicyclo[3.2.1]oct-3-yl)quinazolin-2,4-diamine

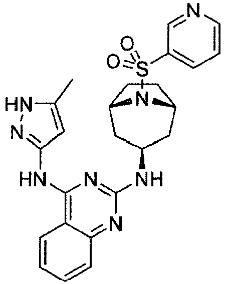

Tert-butyl (3-exo)-3-((4-((5-methyl-1H-pyrazol-3-yl)amino)quinazolin-2-yl)amino)-8-azabicyclo[3.2.1]oct-8-carboxylate (100 mg, 0.222 mmol) was dissolved in 4M HCl in 1,4-dioxane solution (10 mL). The mixture was stirred at room temperature for 30 minutes, and concentrated under reduced pressure to remove the solvent. The residue was dissolved in anhydrous N,N-dimethylformamide (10 mL), then cooled to 0 °C, and DIPEA (0.73 mL, 4.44 mmol) and 3-pyridinsulfonyl chloride hydrochloride (50 mg, 0.233 mmol) were successively added thereto. The reaction mixture was stirred at 0 °C for 0.5 hour, and concentrated under reduced pressure to remove the solvent. The residue was purified by reverse phase HPLC to obtain the title compound (20.5 mg, 19%).

¹H NMR (400 MHz, DMSO-*d₆*) *δ* 8.98 (d, *J* = 2.0 Hz, 1H), 8.76 (dd, *J* = 4.8, 1.4 Hz, 1H), 8.27-8.19 (m, 1H), 8.14-8.01 (m, 1H), 7.60-7.44 (m, 2H), 7.29 (ddd, *J* = 15.0, 9.9, 4.2 Hz, 1H), 7.07 (t, *J* = 7.4 Hz, 1H), 6.58-6.39 (m, 1H), 4.30 (dd, *J* = 6.0, 2.6 Hz, 3H), 2.17 (s, 3H), 2.08-1.95 (m, 2H), 1.74 (dd, *J* = 16.7, 6.2 Hz, 2H), 1.64 (dd, *J* = 17.3, 6.7 Hz, 2H), 1.43-1.32 (m, 2H).

MS *m*/*z* (ESI): 491.1 [M+H]⁺.

### Embodiment 233

### N2-((3-exo)-8-((2-methoxylethyl)sulfonyl)-8-azabicyclo[3.2.1]oct-3-yl)-N4-(5-methyl-1H-yrazol-3-yl)quinazolin-2,4-diamine

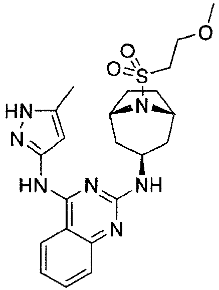

Tert-butyl (3-exo)-3-((4-((5-methyl-1H-pyrazol-3-yl)amino)quinazolin-2-yl)amino)-8-azabicyclo[3.2.1]oct-8-carboxylate (100 mg, 0.222 mmol) was dissolved in 4M HCl in 1,4-dioxane solution (10 mL). The mixture was stirred at room temperature for 30 minutes, and concentrated under reduced pressure to remove the solvent. The residue was dissolved in anhydrous N,N-dimethylformamide (10 mL), then cooled to 0 °C, and DIPEA (0.73 mL, 4.44 mmol) and 2-methoxylethane-1-sulfonyl chloride (37 mg, 0.233 mmol) were successively added thereto. The reaction mixture was stirred at 0 °C for 2 hours, and concentrated under reduced pressure to remove the solvent. The residue was purified by reverse phase HPLC to obtain the title compound (25.1 mg, 43%).

¹H NMR (400 MHz, DMSO-*d₆*) *δ* 8.31 (s, 1H), 7.64 (s, 1H), 7.41 (s, 1H), 7.24 (dd, *J=* 23.8, 8.5 Hz, 1H), 6.61 (s, 1H), 4.49-4.43 (m, 1H), 4.26 (s, 2H), 3.76 (t, *J* = 6.2 Hz, 2H), 3.43-3.29 (m, 5H), 2.32 (s, 3H), 2.11-1.86 (m, 6H), 1.74 (t, *J* = 13.5 Hz, 2H).

MS *m*/*z* (ESI): 472.2 [M+H]⁺.

### Embodiment 234

### N2-((3-exo)-8-(2-fluoroethyl)-8-azabicyclo[3.2.1]oct-3-yl)-N4-(5-methyl-1H-pyrazol-3-yl)quinazolin-2,4-diamine

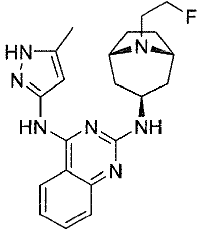

Tert-butyl (3-exo)-3-((4-((5-methyl-1H-pyrazol-3-yl)amino)quinazolin-2-yl)amino)-8-azabicyclo[3.2.1]oct-8-carboxylate (100 mg, 0.222 mmol) was dissolved in 4M HCl in 1,4-dioxane solution (10 mL). The mixture was stirred at room temperature for 30 minutes, and concentrated under reduced pressure to remove the solvent. The residue was dissolved in anhydrous N,N-dimethylformamide (5 mL), and anhydrous potassium carbonate (184 mg, 1.33 mmol) and 1-bromine-2-fluoroethane (50 mg, 0.233 mmol) were successively added thereto. The reaction mixture was stirred at 40 °C for 19 hours, and concentrated under reduced pressure to remove the solvent. The residue was purified by reverse phase HPLC to obtain the title compound (27.3 mg, 31%).

¹H NMR (400 MHz, DMSO-*d₆*) *δ* 8.15 (d, *J* = 17.8 Hz, 1H), 7.56 (s, 1H), 7.35 (d, *J* = 44.5 Hz, 1H), 7.11 (s, 1H), 6.71 (s, 1H), 4.64-4.45 (m, 2H), 4.33-4.19 (m, 1H), 3.35 (s, 2H), 2.91-2.68 (m, 2H), 2.32 (s, 3H), 2.11-1.56 (m, 8H).

MS *m*/*z* (ESI): 396.2 [M+H]⁺.

### Embodiment 235

### 3-((3-exo)-3-((7-chloro-4-((5-(hydroxymethyl)-1H-pyrazol-3-yl)amino)quinazolin-2-yl)(methyl)amino)-9-azabicyclo[3.3.1]non-9-yl)propionitrile

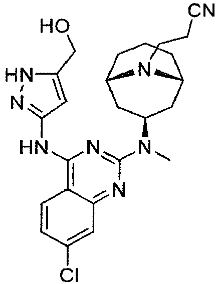

### Step 1: preparation of (3-((2,7-dichloroquinazolin-4-yl)amino)-1H-pyrazol-5-yl)methanol

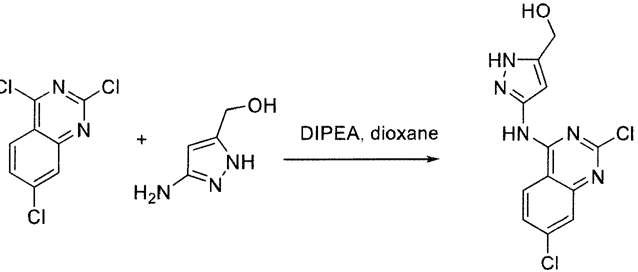

2,4,7-Trifluoroquinazoline(300 mg, 1.29 mmol), (3-amino-1H-pyrazol-5-yl)methanol (180 mg, 1.55 mmol) nand DIPEA (500 mg, 3.87 mmol) were added to 1,4-dioxane (5 mL). The mixture was mixed evenly and the reaction was carried out at room temperature overnight. Then the mixture was concentrated under reduced pressure, and the crude product obtained was added to methanol (5 mL), and filtered. The solid was dried to obtain the title compound as a white solid (350 mg, 87 %)

MS *m*/*z* (ESI): 310.0 [M+H]⁺.

### Step 2: preparation of tert-butyl (3-exo)-3-((7-chloro-4-((5-(hydroxymethyl)-1H-pyrazol-3-yl)amino)quinazolin-2-yl)(methyl)amino)-8-azabicyclo[3.2.1]oct-8-carboxylate

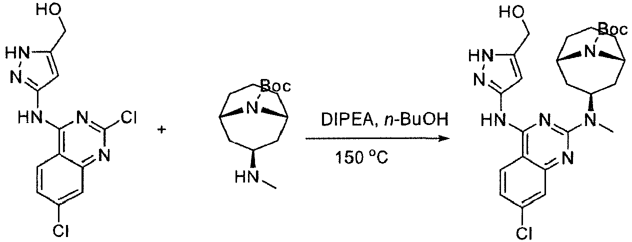

(3-((2,7-Dichloroquinazolin-4-yl)amino)-1H-pyrazol-5-yl)methanol (150 mg, 0.49 mmol), Tert-butyl (3-exo)-3-(methylamino)-9-azabicyclo[3.3.1]non-9-carboxylate (150 mg, 0.58 mmol) and DIPEA (190 mg, 1.47 mmol) were added to n-butanol (2 mL). The mixture was mixed evenly, heated to 150 °C under microwave condition, and reacted for 10 hour. The mixture was then cooled down to room temperature,and concentrated under reduced pressure. The crude product obtained was purified by flash silica gel chromatography to obtain the target product as a white solid (140 mg, 55 %).

MS *m*/*z* (ESI): 528.2 [M+H]⁺.

### Step 3: preparation of 3-((3-exo)-3-((7-chloro-4-((5-(hydroxymethyl)-1H-pyrazol-3-yl)amino)quinazolin-2-yl)(methyl)amino)-9-azabicyclo[3.3.1]non-9-yl)propionitrile

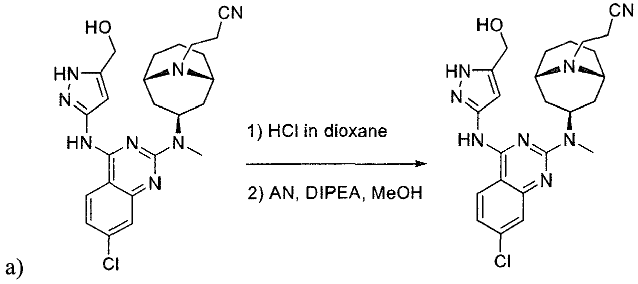

Hydrochloride dioxane(4N, 2 mL) was slowly added dropwise into the solution of tert-butyl (3-*exo*)-3-((7-chloro-4-((5-(hydroxymethyl)-1H-pyrazol-3-yl)amino)quinazolin-2-yl)(methyl)amino)-8-azabicyclo[3.2.1]oct-8-carboxylate (140 mg, 0.27 mmol) in methanol (10 mL). The reaction was carried out at room temperature for 2 hours, and the reaction mixture was concentrated under reduced pressure. The crude product obtained was dissolved in methanol (15 mL), and DIPEA (0.5 mL) and acrylonitrile (25 mg, 0.46 mmol) were separately added thereto at room temperature. The reaction was carried out at room temperature for 1 hour. The reaction mixture was concentrated under reduced pressure, and purified by prep-HPLC to obtain the title compound as a white solid (22 mg, 20 %).

¹H NMR (400 MHz, DMSO-*d₆*) *δ* 12.41 (s, 1H), 10.02 (s, 1H), 8.35 (d, *J* = 8.4 Hz, 1H), 7.28 (s, 1H), 7.06 (d, *J=* 8.4 Hz, 1H), 6.52-6.54 (m, 1H), 5.53-5.55 (m, 1H), 5.25 (s, 1H), 4.46 (t, *J* = 5.2 Hz, 2H), 3.31-2.87 (m, 7H), 2.66-2.59 (m, 2H), 2.08-1.87 (m, 5 H), 1.60-1.41 (m, 5H).

MS *m*/*z* (ESI): 481.2 [M+H]⁺.

### Embodiment 236

### 3-((3-exo)-3-((7-chloro-4-((5-(hydroxymethyl)-1H-pyrazol-3-yl)amino)quinazolin-2-yl)(methyl)amino)-8-azabicyclo[3.2.1]oct-8-yl)propionitrile

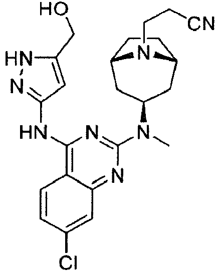

The preparation of 3-((3-*exo*)-3-((7-chloro-4-((5-(hydroxymethyl)-1H-pyrazol-3-yl)amino)quinazolin-2-yl)(methyl)amino)-8-azabicyclo[3.2.1]oct-8-yl)propionitrile was carried out with the reference to Embodiment 235.

¹H NMR (400 MHz, DMSO-*d₆*) *δ* 12.45 (s, 1H), 10.08 (d, *J*= 8.8 Hz, 1H), 8.36 (d, *J* = 8.8 Hz, 1H), 7.29 (s, 1H), 7.07 (d, *J* = 8.8 Hz, 1H), 6.62-6.54 (m, 1H), 5.27-5.11 (m, 2H), 4.50 (d, *J* = 5.6 Hz, 2H), 3.31-2.27 (m, 2H), 2.94 (d, *J* = 16.0 Hz, 3H), 2.67-2.58 (m, 4H), 1.92-1.81 (m, 4H), 1.71-1.62 (m, 2H), 1.39-1.23 (m, 2H).

MS *m*/*z* (ESI): 467.2 [M+H]⁺.

### Embodiment 237

### 1-(((3-exo)-3-((4-((5-methyl-1H-pyrazol-3-yl)amino)thieno[2,3-d]pyrimidin-2-yl)amino)-8-azabicyclo[3.2.1]oct-8-yl)sulfonyl)azetindin-3-formonitrile

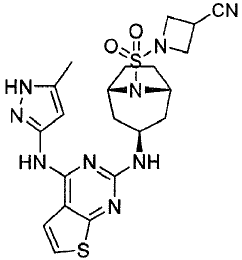

Tert-butyl (3-*exo*)-3-((4-((5-methyl-1H-pyrazol-3-yl)amino)thieno[2,3-d]pyrimidin-2-yl)amino)-8-azabicyclo[3.2.1]oct-8-carboxylate (200 mg, 0.439 mmol) was dissolved in 4M HCl in 1,4-dioxane solution (20 mL). The mixture was stirred at room temperature for 30 minutes, and concentrated under reduced pressure to remove the solvent. The residue was dissolved in anhydrous N,N-dimethylformamide (10 mL), then cooled to 0 °C, and DIPEA (1.45 mL, 8.78 mmol) and 3-cyanoazetindin-1-sulfonyl chloride (95 mg, 0.527 mmol) were successively added thereto. The reaction mixture was stirred at 0 °C for 16.5 hours, and concentrated under reduced pressure to remove the solvent. The residue was purified by reverse phase HPLC to obtain the title compound (70 mg, 32%).

¹H NMR (400 MHz, MeOD-*d₄*) *δ* 7.37 (d, *J* = 6.0 Hz, 1H), 6.94 (d, *J* = 6.0 Hz, 1H), 6.25 (s, 1H), 4.44-4.34 (m, 1H), 4.26 (s, 2H), 4.16 (t, *J* = 8.5 Hz, 2H), 4.12-4.05 (m, 2H), 3.57 (ddd, *J =* 15.3, 8.7, 6.5 Hz, 1H), 2.31 (s, 3H), 2.23-2.10 (m, 4H), 2.01 (d, *J =* 7.4 Hz, 2H), 1.73 (dd, *J* = 18.2, 7.1 Hz, 2H).

MS *m*/*z* (ESI): 500.1 [M+H]⁺.

### Embodiment 238

### 3-((3-exo)-3-(((4-((5-methyl-1H-pyrazol-3-yl)amino)thieno[2,3-d]pyrimidin-2-yl)amino))-9-azabicyclo[3.3.1]non-9-yl)sulfonyl)azacyclobutane-3-nitrile

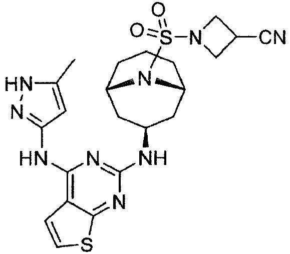

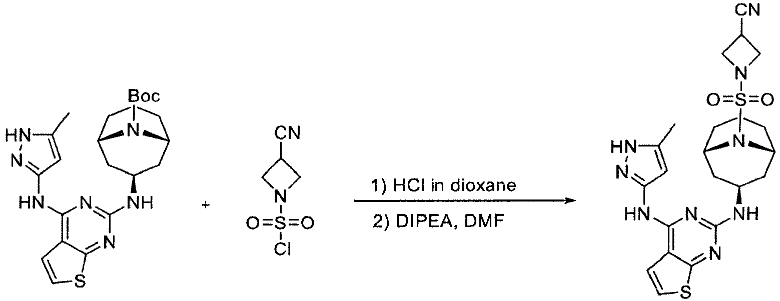

Tert-butyl-(3-*exo*)-3-((7-((5-methyl-1H-pyrazol-3-yl)amino)thieno[2,3-d]pyrimidin-2-yl)amino)-9-azabicyclo[3.3.1]non-9-carboxylate (100 mg, 0.21 mmol) was dissolved in hydrochloride 1,4-dioxane solution (4.0 N, 5 mL). The mixture was stirred at room temperature for 30 minutes, concentrated, and then dissolved in N,N-dimethylformamide (10 mL). DIPEA (108 mg, 0.84 mmol) was slowly added dropwise into the mixture, and the mixture was stirred at room temperature for 10 minutes. 3-Nitrile azacyclobutane-1-sulfonylchlorine (45 mg, 0.25 mmol) was added to the mixture, and the mixture was stirred at room temperature overnight. The reaction mixture was concentrated under reduced pressure, and the resulting product was purified by prep-HPLC to obtain the title compound as a white solid (14.4 mg, 13%).

¹H NMR (400 MHz, DMSO) *δ* = 12.02 (s, 1H), 9.81 (s, 1H), 7.61 (s, 1H), 6.90 (s, 1H), 6.59 (d, *J =* 57.6 Hz, 2H), 4.74 (s, 1H), 3.96 (t, *J* = 8.4 Hz, 2H), 3.85 (dd, *J* = 16.8 Hz, 6.4, 4H), 3.75-3.67 (m, 1H), 2.14 (s, 3H), 2.00 (d, *J* = 8.4 Hz, 2H), 1.87-1.60 (m, 8H).

MS *m*/*z* (ESI): 514.1 [M+H]⁺.

### Embodiment 239

### 1-(((3-exo)-3-(methyl(4-((5-methyl-1H-pyrazol-3-yl)amino)thieno[2,3-d]pyrimidin-2-yl)amino)-9-azabicyclo[3.3.1]non-9-yl)sulfonyl)azacyclobutane-3-nitrile

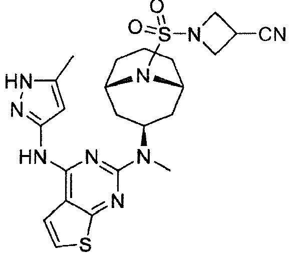

### Step 1: preparation of tert-butyl-(3-exo)-3-(methyl(4-((5-methyl-1H-pyrazol-3-yl)amino)thieno[2,3-d]pyrimidin-2-yl)amino)-9-azabicyclo[3.3.1]non-9-carboxylate

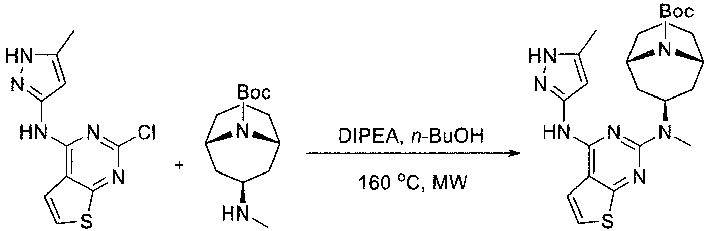

Tert-butyl-(3-*exo*)-3-(methylamino)-9-azabicyclo[3.3.1]non-9-carboxylate (287 mg, 1.13 mmol) and DIPEA (242 mg, 1.88 mmol) were successively added to the solution of 2-chloro-N-(5-methyl-1H-pyrazol-3-yl)thieno[2,3-d]pyrimidin-4-amine(250 mg, 0.94 mmol) in n-butanol (10 mL). The mixture was stirred at 160°C under microwave condition for 15 hours. When the reaction was completed, the reaction mixture was extracted with ethyl acetate (15 mL x 3), and washed with saturated sodium chloride aqueous solution (15 mL x 3). The organic phase was collected, dried over anhydrous sodium sulfate, filtered and concentrated under reduced pressure. The resulting product was purified by silica gel column chromatography (dichloromethane: methanol=98:2) to obtain the title compound as pale white solid (228 mg, 50%).

MS *m*/*z* (ESI): 484.2 [M+H]⁺.

### Step 2: preparation of 1-(((3-exo)-3-(methyl(4-((5-methyl-1H-pyrazol-3-yl)amino)thieno[2,3-d]pyrimidin-2-yl)amino)-9-azabicyclo[3.3.1]non-9-yl)sulfonyl)azacyclobutane-3-nitrile

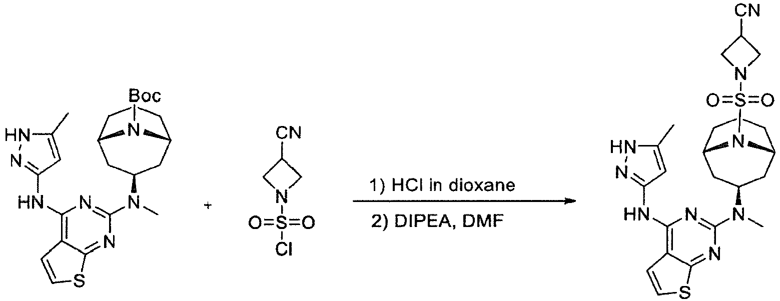

Tert-butyl-(3-*exo*)-3-(methyl(4-((5-methyl-1H-pyrazol-3-yl)amino)thieno[2,3-d]pyrimidin-2-yl)amino)-9-azabicyclo[3.3.1]non-9-carboxylate (100 mg, 0.21 mmol) was dissolved in hydrochloride 1,4-dioxane solution (4.0 N, 5 mL). The mixture was stirred at room temperature for 30 minutes, concentrated, and then dissolved in N,N-dimethylformamide(10 mL). DIPEA (108 mg, 0.84 mmol) was slowly added dropwise into the mixture, and the mixture was stirred at room temperature for 10 minutes. 3-Nitrile azacyclobutane-1-sulfonylchlorine (45 mg, 0.25 mmol) was added to the mixture, and the mixture was stirred at room temperature overnight. The reaction mixture was concentrated under reduced pressure, and the resulting product was purified by prep-HPLC to obtain the title compound as a white solid (46.0 mg, 42%).

¹H NMR (400 MHz, DMSO) *δ* = 12.09 (s, 1H), 9.79 (s, 1H), 7.68 (d, *J*= 6.0 Hz, 1H), 7.02 (d, *J* = 6.0 Hz, 1H), 6.43 (s, 1H), 5.77 (s, 1H), 3.98 (dt, *J* = 14.4, 8.4 Hz, 6H), 3.84-3.74 (m, 1H), 2.90 (s, 3H), 2.22 (s, 3H), 2.13-1.61 (m, 10H).

MS *m*/*z* (ESI): 528.2 [M+H]⁺.

### Embodiment 240

### 1-(((3-exo)-3-(methyl(6-methyl-4-((5-methyl-1H-pyrazol-3-yl)amino)thieno[2,3-d]pyrimidin-2-yl)amino)-8-azabicyclo[3.2.1]oct-8-yl)sulfonyl)azetindin-3-formonitrile

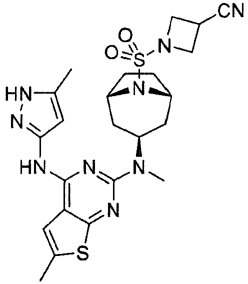

### Step 1: preparation of tert-butyl (3-exo)-3-(methyl(6-methyl-4-((5-methyl-1H-pyrazol-3-yl)amino)thieno[2,3-d]pyrimidin-2-yl)amino)-8-azabicyclo[3.2.1]oct-8-carboxylate

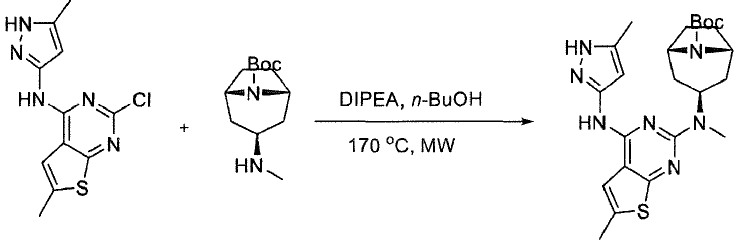

2-Chloro-6-methyl-N-(5-methyl-1H-pyrazol-3-yl)thieno[2,3-d]pyrimidin-4-amine (150 mg, 0.536 mmol) and Tert-butyl (3-exo)-3-(methylamino)-8-azabicyclo[3.2.1]oct-8-carboxylate (257 mg, 1.072 mmol) were added to n-butanol (10 mL). The mixture was heated to 170 °C with a microwave reactor, reacted for 8 hours, and concentrated under reduced pressure to remove the solvent. The residue was dissolved in dichloromethane. The mixture was successively washed with saturated sodium bicarbonate aqueous solution and saturated sodium chloride aqueous solution. The organic phase was dried over anhydrous sodium sulfate, filtered, concentrated, and purified by silica gel chromatography to obtain the title compound (73 mg, 28%).

MS *m*/*z* (ESI): 484.2 [M+H]⁺.

### Step 2: preparation of 1-(((3-exo)-3-(methyl(6-methyl-4-((5-methyl-1H-pyrazol-3-yl)amino)thieno[2,3-d]pyrimidin-2-yl)amino)-8-azabicyclo[3.2.1]oct-8-yl)sulfonyl)azetindin-3-formonitrile

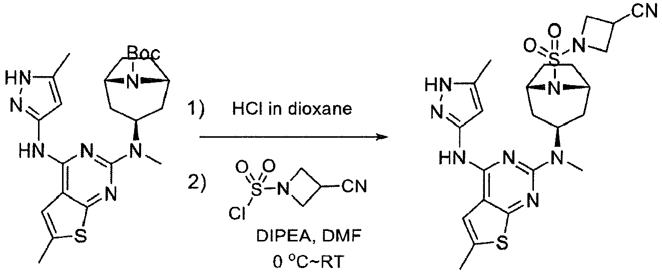

Tert-butyl (3-exo)-3-(methyl(6-methyl-4-((5-methyl-1H-pyrazol-3-yl)amino)thieno[2,3-d]pyrimidin-2-yl)amino)-8-azabicyclo[3.2.1]oct-8-carboxylate (73 mg, 0.151 mmol) was dissolved in 4 M HCl in 1,4-dioxane (20 mL). The mixture was stirred at room temperature for 30 minutes, and concentrated under reduced pressure to remove the solvent. The residue solid was dissolved in anhydrous N,N-dimethylformamide (10 mL), then cooled to 0 °C, and DIPEA (0.75 mL, 4.53 mmol) and 3-cyanoazetindin-1-sulfonyl chloride (30 mg, 0.166 mmol) were successively added thereto. The reaction mixture was stirred at 0 °C for 4.5 hour, and concentrated under reduced pressure to remove the solvent. The residue was purified by prep-HPLC to obtain the title compound (31.5 mg, 40%).

¹H NMR (400 MHz, DMSO-*d₆*) *δ* 12.06 (s, 1H), 9.66 (s, 1H), 7.35 (s, 1H), 6.48 (s, 1H), 5.31-5.15 (m, 1H), 4.18 (d, *J* = 1.0 Hz, 2H), 4.06 (t, *J* = 8.6 Hz, 2H), 4.00-3.91 (m, 2H), 3.80 (ddd, *J* = 12.8, 8.9, 6.5 Hz, 1H), 2.90 (s, 3H), 2.40 (s, 3H), 2.22 (s, 3H), 2.07-1.99 (m, 2H), 1.95 (dd, *J=* 18.2, 7.0 Hz, 2H), 1.88-1.79 (m, 2H), 1.62 (dd, *J =* 11.8, 4.1 Hz, 2H).

MS *m*/*z* (ESI): 528.2 [M+H]⁺.

### Embodiment 241

### 1-(((3-exo)-3-(methyl(4-((5-methyl-1H-pyrazol-3-yl)amino)thieno[2,3-d]pyrimidin-2-yl)amino)-8-azabicyclo[3.2.1]oct-8-yl)sulfonyl)azetindin-3-formonitrile

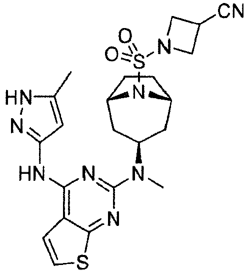

### Step 1: preparation of Tert-butyl (3-exo)-3-(methyl(4-((5-methyl-1H-pyrazol-3-yl)amino)thieno[2,3-d]pyrimidin-2-yl)amino)-8-azabicyclo[3.2.1]oct-8-carboxylate

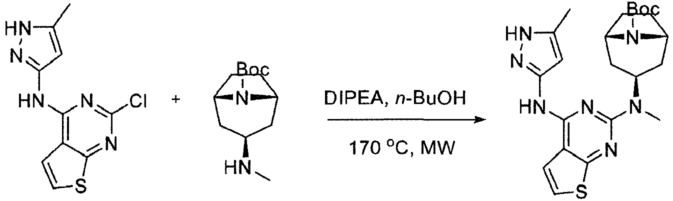

2-Chloro-N-(5-methyl-1H-pyrazol-3-yl)thieno[2,3-d]pyrimidin-4-amine (100 mg, 0.376 mmol) and tert-butyl (3-*exo*)-3-(methylamino)-8-azabicyclo[3.2.1]oct-8-carboxylate (181 mg, 0.752 mmol) were added to n-butanol (3 mL). The mixture was heated by a microwave reactor to 170 °C, reacted for 18 hours, and concentrated under reduced pressure to remove the solvent. The residue was used directly in the next step.

MS *m*/*z* (ESI): 470.2 [M+H]⁺.

### Step 2: preparation of 1-(((3-exo)-3-(methyl(4-((5-methyl-1H-pyrazol-3-yl)amino)thieno[2,3-d]pyrimidin-2-yl)amino)-8-azabicyclo[3.2.1]oct-8-yl)sulfonyl)azetindin-3-formonitrile

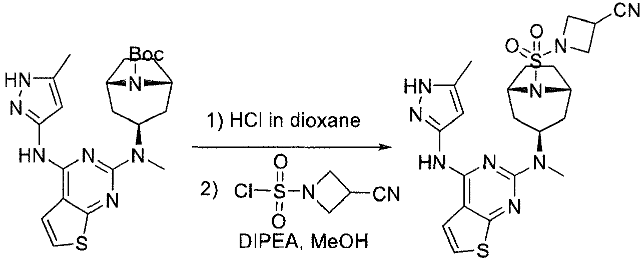

Tert-butyl (3-exo)-3-(methyl(4-((5-methyl-1H-pyrazol-3-yl)amino)thieno[2,3-d]pyrimidin-2-yl)amino)-8-azabicyclo[3.2.1]oct-8-carboxylate was dissolved in 4 M HCl in 1,4-dioxane (20 mL). The mixture was stirred at room temperature for 30 minutes, and concentrated under reduced pressure to remove the solvent. The residue was purified by reverse phase chromatography to obtain 117 mg white solid.

The white solid was dissolved in anhydrous N,N-dimethylformamide (10 mL), then cooled to 0 °C, and DIPEA (0.14 mL, 0.632 mmol) and 3-cyanoazetindin-1-sulfonyl chloride (57 mg, 0.316 mmol) were successively added thereto. The reaction mixture was stirred at 0 °C for 17 hours, and concentrated under reduced pressure to remove the solvent. The residue was purified by prep-HPLC to obtain the title compound (16.4 mg, 10%).

¹H NMR (400 MHz, MeOD-*d₄*) *δ* 7.37 (d, *J* = 5.9 Hz, 1H), 6.98 (d, *J* = 5.7 Hz, 1H), 6.40 (s, 1H), 5.40-5.28 (m, 1H), 4.31-4.24 (m, 2H), 4.17 (t, *J* = 8.5 Hz, 2H), 4.11-4.04 (m, 2H), 3.57 (ddd, *J* = 15.4, 8.9, 6.7 Hz, 1H), 3.04 (s, 3H), 2.31 (s, 3H), 2.17 (dd, *J* = 8.6, 3.3 Hz, 2H), 2.11-2.01 (m, 2H), 2.00-1.92 (m, 2H), 1.75 (ddd, *J* = 10.8, 4.3, 2.7 Hz, 2H).

MS *m*/*z* (ESI): 514.1 [M+H]⁺.

### Embodiment 242

### 2-(dimethylamine)-1-((3-exo)-3-((4-((5-methyl-1H-pyrazol-3-yl)amino)thieno[2,3-d]pyrimidin-2-yl)amino)-9-azabicyclo[3.3.1]non-9-yl)ethane-1-one

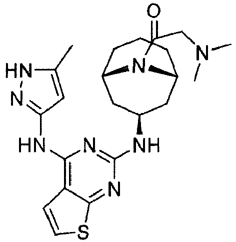

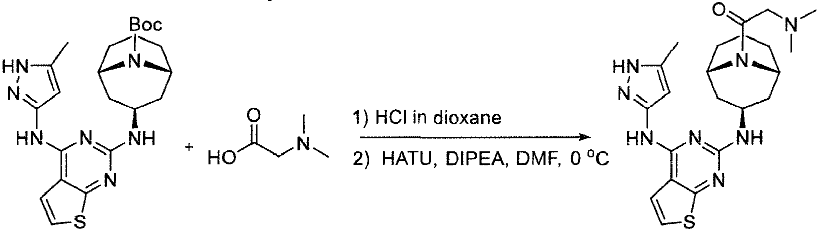

Tert-butyl-(3-*exo*)-3-((7-((5-methyl-1H-pyrazol-3-yl)amino)thieno[2,3-d]pyrimidin-2-yl)amino)-9-azabicyclo[3.3.1]non-9-carboxylate (100 mg, 0.21 mmol) was dissolved in hydrochloride 1,4-dioxane solution(4.0 N, 5 mL). The mixture was stirred at room temperature for 30 minutes, concentrated, and then 2-(7-oxide benzotriazole)-N,N,N',N'-tetramethylurea hexafluorophosphate (120 mg, 0.32 mmol), was added to the mixture. The mixture was then dissolved in N,N-dimethylformamide (5 mL), and DIPEA (108 mg, 0.84 mmol) was slowly added dropwise thereto. The mixture was then stirred under ice water bath for 10 minutes, dimethyl glycine (24 mg, 0.23 mmol) was added thereto and the mixture was stirred for another 1 hour. The reaction mixture was concentrated under reduced pressure, and the resulting product was purified by prep-HPLC to obtain the title compound as a white solid (17.1 mg, 18 %).

¹H NMR (400 MHz, DMSO) *δ* = 12.00 (s, 1H), 9.80 (s, 1H), 7.61 (s, 1H), 6.98-6.45 (m, 3H), 4.76 (s, 1H), 4.59 (s, 1H), 4.27 (s, 1H), 3.30 (s, 6H), 3.05 (s, 2H), 2.16 (s, 3H), 2.14 (s, 2H), 2.07-1.92 (m, 2H), 1.86-1.40 (m, 6H).

MS *m*/*z* (ESI): 455.2 [M+H]⁺.

### Embodiment 243

### 2-(dimetbylamine)-1-((3-exo)-3-((4-((5-methyl-1H-pyrazol-3-yl)amino)thieno[2,3-d]pyrimidin-2-yl)amino)-8-azabicyclo[3.2.1]oct-8-yl)ethane-1-one

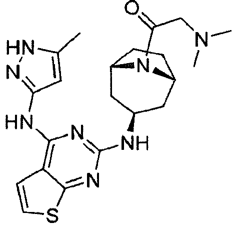

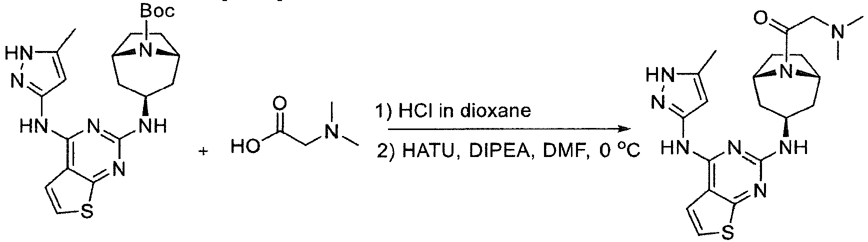

Tert-butyl-(3-*exo*)-3-((4-((5-methyl-1H-pyrazol-3-yl)amino)thieno[2,3-d]pyrimidin-2-yl)amino)-8-azabicyclo[3.2.1]oct-8-carboxylate (40 mg, 0.09 mmol) was dissolved in hydrochloride 1,4-dioxane solution(4.0 N, 2 mL). The mixture was stirred at room temperature for 30 minutes, concentrated, and then 2-(7-oxide benzotriazole)-N,N,N',N'-tetramethylurea hexafluorophosphate (51 mg, 0.13 mmol) was added to the mixture. The mixture was then dissolved in N,N-dimethylformamide (5 mL), and DIPEA (46 mg, 0.36 mmol) was slowly added dropwise thereto. The mixture was then stirred under ice water bath for 10 minutes. Dimethyl glycine (10 mg, 0.1 mmol) was added thereto and the mixture was stirred for another 1 hour. The reaction mixture was concentrated under reduced pressure, and the resulting product was purified by prep-HPLC to obtain the title compound as a white solid (4.4 mg, 11 %).

¹H NMR (400 MHz, DMSO) *δ* = 12.08 (s, 1H), 9.88 (s, 1H), 7.65 (s, 1H), 7.11-6.46 (m, 3H), 4.47 (d, *J=* 30.0 Hz, 3H), 3.06 (s, 2H), 2.21 (s, 9H), 2.04-1.66 (m, 6H), 1.62-1.44 (m, 2H).

MS *m*/*z* (ESI): 441.2 [M+H]⁺.

### Embodiment 244

### 1-((3-exo)-3-((4-((5-methyl-1H-pyrazol-3-yl)amino)thieno[2,3-d]pyrimidin-2-yl)amino)-8-azabicyclo[3.2.1]oct-8-yl)-2-morpholinoethane-1-one

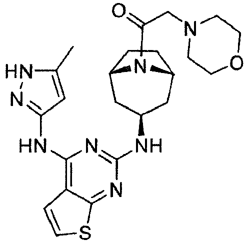

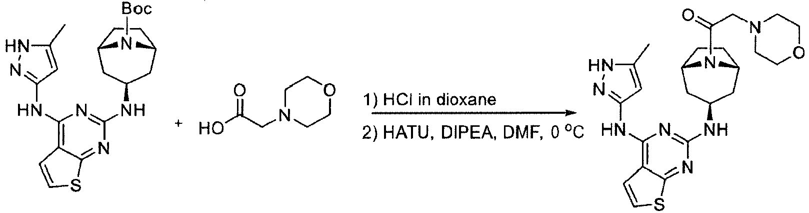

Tert-butyl-(3-*exo*)-3-((4-((5-methyl-1H-pyrazol-3-yl)amino)thieno[2,3-d]pyrimidin-2-yl)amino)-8-azabicyclo[3.2.1]oct-8-carboxylate (40 mg, 0.09 mmol) was dissolved in hydrochloride 1,4-dioxane solution (4.0 N, 2 mL). The mixture was stirred at room temperature for 30 minutes, concentrated, and then 2-(7-oxide benzotriazole)-N,N,N',N'-tetramethylurea hexafluorophosphate (51 mg, 0.13 mmol) was added to the mixture. The mixture was dissolved in N,N-dimethylformamide (5 mL). DIPEA (46 mg, 0.36 mmol) was slowly added dropwise into the mixture, and the mixture was then stirred under ice water bath for 10 minutes. 2-morpholino acetate (14.5 mg, 0.1 mmol) was added thereto and the mixture was stirred for another 1 hour. The reaction mixture was concentrated under reduced pressure, and the resulting product was purified by prep-HPLC to obtain the title compound as a white solid (7.8 mg, 18 %).

¹H NMR (400 MHz, DMSO) *δ* = 12.07 (s, 1H), 9.88 (s, 1H), 7.66 (s, 1H), 7.11-6.49 (m, 3H), 4.48 (d, *J* = 26.4 Hz, 3H), 3.60 (s, 4H), 3.17 (s, 2H), 2.46 (s, 4H), 2.23 (s, 3H), 1.98 (s, 2H), 1.90-1.45 (m, 6H).

MS *m*/*z* (ESI): 483.2 [M+H]⁺.

### Embodiment 245

### 1-((3-exo)-3-(methyl(4-((5-methyl-1H-pyrazol-3-yl)amino)thieno[2,3-d]pyrimidin-2-yl)amino)-8-azabicyclo[3.2.1]oct-8-yl)-2-morpholinoethane-1-one

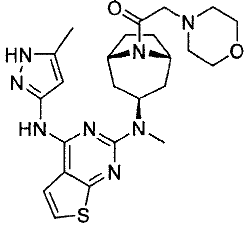

The preparation of 1-((3-*exo*)-3-(methyl(4-((5-methyl-1H-pyrazol-3-yl)amino)thieno[2,3-d]pyrimidin-2-yl)amino)-8-azabicyclo[3.2.1]oct-8-yl)-2-morpholinoethane-1-one was carried out with the reference to Embodiment 244.

¹H NMR (400 MHz, DMSO-*d₆*) *δ* = 9.85 (s, 1H), 8.22 (s, 1H), 7.68 (d, *J* = 6.0 Hz, 1H), 7.02 (d, *J* = 6.0 Hz, 1H), 6.53 (s, 1H), 5.37 (s, 1H), 4.54 (d, *J* = 16.4 Hz, 2H), 3.58 (d, *J* = 4.0 Hz, 4H), 3.04 (d, *J* = 13.2 Hz, 2H), 2.85 (s, 3H), 2.45 (s, 4H), 2.23 (s, 3H), 2.03-1.97 (m, 2H), 1.87-1.59 (m, 6H).

MS *m*/*z* (ESI): 497.2 [M+H]⁺.

### Embodiment 246

### 1-((3-exo)-3-((4-((5-methyl-1H-pyrazol-3-yl)amino)thieno[2,3-d]pyrimidin-2-yl)amino)-9-azabicyclo[3.3.1]non-9-yl)-2-morpholinoethane-1-one

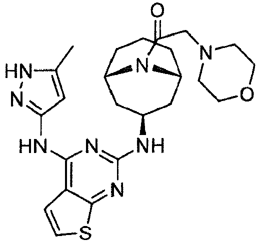

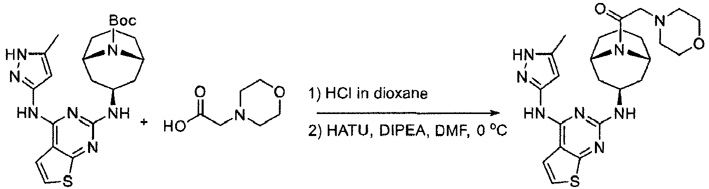

Tert-butyl-(3-*exo*)-3-((7-((5-methyl-1H-pyrazol-3-yl)amino)thieno[2,3-d]pyrimidin-2-yl)amino)-9-azabicyclo[3.3.1]non-9-carboxylate (100 mg, 0.21 mmol) was dissolved in hydrochloride 1,4-dioxane solution(4.0 N, 5 mL). The mixture was stirred at room temperature for 30 minutes, concentrated, and then 2-(7-oxide benzotriazole)-N,N,N',N'-tetramethylurea hexafluorophosphate (120 mg, 0.31 mmol) was added to the mixture. The mixture was then dissolved in N,N-dimethylformamide(5 mL). DIPEA (108 mg, 0.84 mmol) was slowly added dropwise into the mixture, and the mixture was then stirred under ice water bath for 10 minutes. 2-Morpholino acetate (33 mg, 0.23 mmol) was added thereto and the mixture was stirred for another 1 hour. The reaction mixture was concentrated under reduced pressure, the resulting product was purified by prep-HPLC to obtain the title compound as a white solid (18.0 mg, 17 %).

¹H NMR (400 MHz, DMSO) *δ* = 12.08 (s, 1H), 9.87 (s, 1H), 7.68 (s, 1H), 7.07-6.53 (m, 3H), 4.83 (s, 1H), 4.65 (s, 1H), 4.37 (s, 1H), 3.59 (d, *J* = 4.0 Hz, 4H), 3.12 (dd, *J* = 25.2, 12.4 Hz, 2H), 2.39 (s, 4H), 2.21 (s, 3H), 2.11-1.51 (m, 10H).

MS *m*/*z* (ESI): 497.2 [M+H]⁺.

### Embodiment 247

### 1-((3-exo)-3-((4-((5-methyl-1H-pyrazol-3-yl)amino)thieno[2,3-d]pyrimidin-2-yl)amino)-9-azabicyclo[3.3.1]non-9-yl)-2-(methylamino)-ethane-1-one

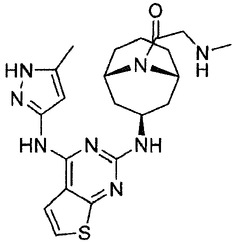

### Step 1: preparation of tert-butylmethyl(2-((3-exo)-3-((4-((5-methyl-1H-pyrazol-3-yl)amino)thieno[2,3-d]pyrimidin-2-yl)amino)-9-azabicyclo[3.3.1]non-9-yl)-2carbonylethyl)amino formate

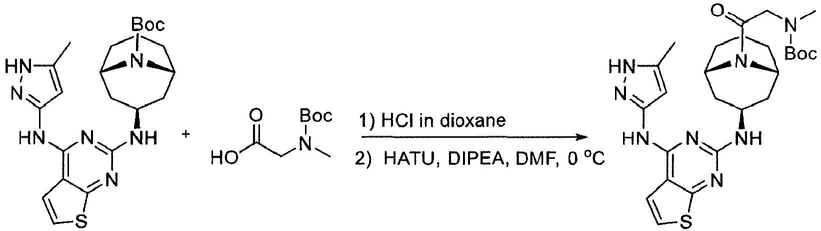

Tert-butyl-(3-*exo*)-3-((7-((5-methyl-1H-pyrazol-3-yl)amino)thieno[2,3-d]pyrimidin-2-yl)amino)-9-azabicyclo[3.3.1]non-9-carboxylate (200 mg, 0.42 mmol) was dissolved in hydrochloride 1,4-dioxane solution (4.0 N, 10 mL). The mixture was stirred at room temperature for 30 minutes, concentrated, and then 2-(7-oxide benzotriazole)-N,N,N',N'-tetramethylurea hexafluorophosphate (240 mg, 0.64 mmol) was added to the mixture, and the mixture was dissolved in N,N-dimethylformamide(5 mL). DIPEA (216 mg, 1.68 mmol) was slowly added dropwise into the mixture, and the mixture was then stirred under ice water bath for 10 minutes. N-(tert-butoxycarbonyl)-N-methyl glycine (87 mg, 0.46 mmol) was added thereto and the mixture was stirred for another 1 hour. When the reaction was completed, the mixture was extracted with dichloromethane (15 mL x 3), and washed with saturated sodium chloride aqueous solution (15 mL x 3). The organic phase was collected, dried over anhydrous sodium sulfate, filtered, and concentrated under reduced pressure. The resulting product was purified by silica gel column chromatography (dichloromethane: methanol=95:5) to obtain the title compound as a white solid (205 mg, 90%).

MS *m*/*z* (ESI): 541.2 [M+H]⁺.

### Step 2: preparation of 1-((3-exo)-3-((4-((5-methyl-1H-pyrazol-3-yl)amino)thieno[2,3-d]pyrimidin-2-yl)amino)-9-azabicyclo[3.3.1]non-9-yl)-2-(methylamino)-ethane-1-one

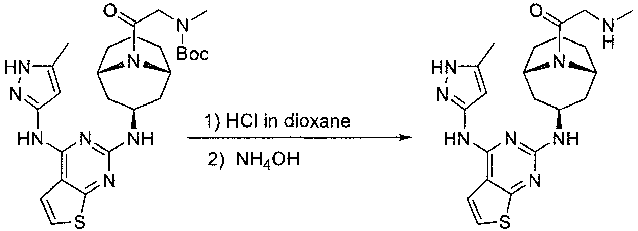

Tert-butylmethyl(2-((3-*exo*)-3-((4-((5-methyl-1H-pyrazol-3-yl)amino)thieno[2,3-d]pyrimidin-2-yl)amino)-9-azabicyclo[3.3.1]non-9-yl)-2carbonylethyl)amino formate (205 mg, 0.38 mmol) was dissolved in hydrochloride 1,4-dioxane solution (4.0 N, 10 mL). The mixture was stirred at room temperature for 30 minutes, and ammonia (10 mL) was added dropwise to the reaction mixture in an ice water bath. The reaction mixture was concentrated under reduced pressure, and the resulting product was purified by prep-HPLC to obtain the title compound as a white solid (37.6 mg, 22 %).

¹H NMR (400 MHz, DMSO) *δ* = 12.10 (s, 1H), 9.88 (s, 1H), 7.68 (s, 1H), 6.96 (s, 1H), 6.61 (s, 2H), 4.84 (s, 1H), 4.69 (s, 1H), 4.12 (s, 1H), 2.29 (s, 3H), 2.20 (s, 3H), 2.15-1.96 (m, 3H), 1.87-1.47 (m, 10H).

MS *m*/*z* (ESI): 441.2 [M+H]⁺.

### Embodiment 248

### ((3-exo)-3-((4-((5-methyl-1H-pyrazol-3-yl)amino)thieno[2,3-d]pyrimidin-2-yl)amino)-9-azabicyclo[3.3.1]non-9-yl) ((R)-pyrrolidin-2-yl)methanone

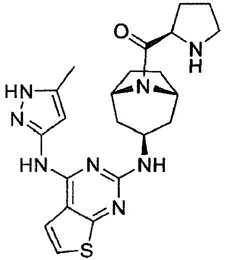

The preparation of ((3-*exo*)-3-((4-((5-methyl-1H-pyrazol-3-yl)amino)thieno[2,3-d]pyrimidin-2-yl)amino)-9-azabicyclo[3.3.1]non-9-yl) ((R)-pyrrolidin-2-yl)methanone was carried out with the reference to Embodiment 247.

¹H NMR (400 MHz, DMSO-*d₆*) *δ* = 12.08 (s, 1H), 9.89 (s, 1H), 7.66 (s, 1H), 7.06-6.51 (m, 3H), 4.55-4.35 (m, 3H), 3.73 (s, 1H), 3.01 (s, 1H), 2.64 (d, *J* = 6.8 Hz, 2H), 2.23 (s, 3H), 2.10-1.43 (m, 12H).

MS *m*/*z* (ESI): 453.1 [M+H]⁺.

### Embodiment 249

### ((3-exo)-3-((4-((5-methyl-1H-pyrazol-3-yl)amino)thieno[2,3-d]pyrimidin-2-yl)amino)-9-azabicyclo[3.3.1]non-9-yl) ((S)-pyrrolidin-2-yl)methanone

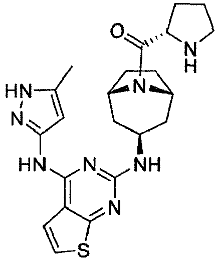

The preparation of ((3-*exo*)-3-((4-((5-methyl-1H-pyrazol-3-yl)amino)thieno[2,3-d]pyrimidin-2-yl)amino)-9-azabicyclo[3.3.1]non-9-yl) ((S)-pyrrolidin-2-yl)methanone was carried out with the reference to Embodiment 247.

¹H NMR (400 MHz, DMSO-*d₆*) *δ* = 12.11 (s, 1H), 9.90 (s, 1H), 7.67 (s, 1H), 5.98-6.54 (m, 3H), 4.58-4.35 (m, 3H), 4.09-4.02 (m, 1H), 3.11 (s, 1H), 2.97-2.64 (m, 2H), 2.23 (s, 3H), 2.10-1.37 (m, 10H).

MS *m*/*z* (ESI): 453.1 [M+H]⁺.

### Embodiment 250

### ((3-exo)-3-((4-((5-methyl-1H-pyrazol-3-yl)amino)thieno[2,3-d]pyrimidin-2-yl)amino)-9-azabicyclo[3.3.1]non-9-yl)((R)-morpholine-3-yl)methanone

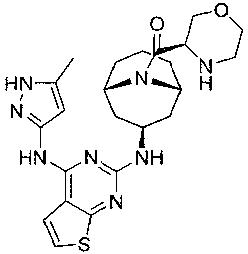

The preparation of ((3-*exo*)-3-((4-((5-methyl-1H-pyrazol-3-yl)amino)thieno[2,3-d]pyrimidin-2-yl)amino)-9-azabicyclo[3.3.1]non-9-yl)((R)-morpholine-3-yl)methanone was carried out with the reference to Embodiment 247.

MS *m*/*z* (ESI): 483.2 [M+H]⁺.

### Embodiment 251

### ((3-exo)-3-((4-((5-methyl-1H-pyrazol-3-yl)amino)thieno[2,3-d]pyrimidin-2-yl)amino)-9-azabicyclo[3.3.1]non-9-yl)((R)-pyrrolidin-2-yl)methanone

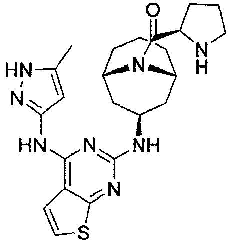

The preparation of ((3-*exo*)-3-((4-((5-methyl-1H-pyrazol-3-yl)amino)thieno[2,3-d]pyrimidin-2-yl)amino)-9-azabicyclo[3.3.1]non-9-yl)((R)-pyrrolidin-2-yl)methanone was carried out with the reference to Embodiment 247.

MS *m*/*z* (ESI): 467.2 [M+H]⁺.

### Embodiment 252

### 2-((2-methoxylethyl)amino)-1-((3-exo)-3-((4-((5-methyl-1H-pyrazol-3-yl)amino)thieno[2,3-d]pyrimidin-2-yl)amino)-9-azabicyclo[3.3.1]non-9-yl)ethane-1-one

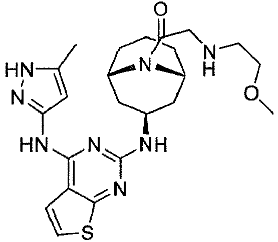

The preparation of 2-((2-methoxylethyl)amino)-1-((3-*exo*)-3-((4-((5-methyl-1H-pyrazol-3-yl)amino)thieno[2,3-d]pyrimidin-2-yl)amino)-9-azabicyclo[3.3.1]non-9-yl)ethane-1-one was carried out with the reference to Embodiment 247.

MS *m*/*z* (ESI): 485.2 [M+H]⁺.

### Embodiment 253

### 1-((3-exo)-3-((4-((5-methyl-1H-pyrazol-3-yl)amino)thieno[2,3-d]pyrimidin-2-yl)amino)-9-azabicyclo[3.3.1]non-9-yl)-2-((pyridin-3-ylmethyl)amino)ethane-1-one

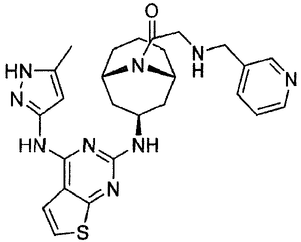

The preparation of 1-((3-*exo*)-3-((4-((5-methyl-1H-pyrazol-3-yl)amino)thieno[2,3-d]pyrimidin-2-yl)amino)-9-azabicyclo[3.3.1]non-9-yl)-2-((pyridin-3-ylmethyl)amino)ethane-1-one was carried out with the reference to Embodiment 247.

MS *m*/*z* (ESI): 518.2 [M+H]⁺.

### Embodiment 254

### 2-((4-methoxybenzyl)amino)-1-((3-exo)-3-((4-((5-methyl-1H-pyrazol-3-yl)amino)thieno[2,3-d]pyrimidin-2-yl)amino)-8-azabicyclo[3.2.1]oct-8-yl)ethane-1-one

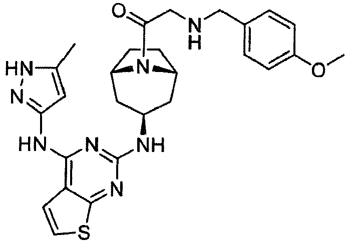

The preparation of 2-((4-methoxybenzyl)amino)-1-((3-*exo*)-3-((4-((5-methyl-1H-pyrazol-3-yl)amino)thieno[2,3-d]pyrimidin-2-yl)amino)-8-azabicyclo[3.2.1]oct-8-yl)ethane-1-one was carried out with the reference to Embodiment 247.

MS *m*/*z* (ESI): 533.2 [M+H]⁺.

### Embodiment 255

### 2-(ethylamino)-1-((3-exo)-3-((4-((5-methyl-1H-pyrazol-3-yl)amino)thieno[2,3-d]pyrimidin-2-yl)amino)-8-azabicyclo[3.2.1]oct-8-yl)ethane-1-one

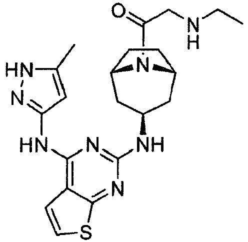

The preparation of 2-(ethylamino)-1-((3-*exo*)-3-((4-((5-methyl-1H-pyrazol-3-yl)amino)thieno[2,3-d]pyrimidin-2-yl)amino)-8-azabicyclo[3.2.1]oct-8-yl)ethane-1-one was carried out with the reference to Embodiment 247.

MS *m*/*z* (ESI): 441.2 [M+H]⁺.

### Embodiment 256

### 2-(cyclopropylamino)-1-((3-exo)-3-((4-((5-methyl-1H-pyrazol-3-yl)amino)thieno[2,3-d]pyrimidin-2-yl)amino)-8-azabicyclo[3.2.1]oct-8-yl)ethane-1-one

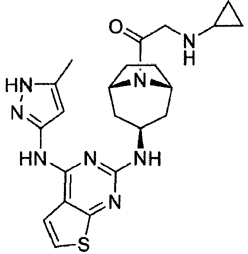

The preparation of 2-(cyclopropylamino)-]-((3-*exo*)-3-((4-((5-methyl-1H-pyrazol-3-yl)amino)thieno[2,3-d]pyrimidin-2-yl)amino)-8-azabicyclo[3.2.1]oct-8-yl)ethane-1-one was carried out with the reference to Embodiment 247.

MS *m*/*z* (ESI): 453.2 [M+H]⁺.

### Embodiment 257

### 1-((3-exo)-3-(methyl(4-((5-methyl-1H-pyrazol-3-yl)amino)thieno[2,3-d]pyrimidin-2-yl)amino)-9-azabicyclo[3.3.1]non-9-yl)-2-(4-methylpiperazin-1-yl)ethane-1-one

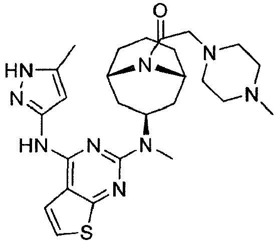

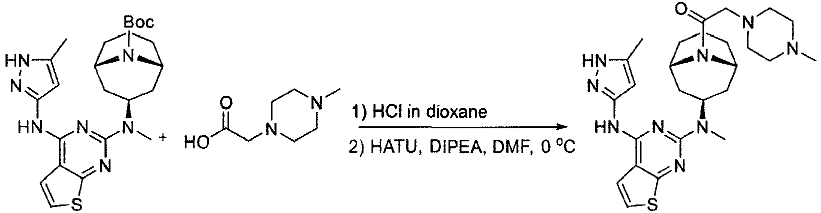

Tert-butyl-(3-exo)-3-(methyl(4-((5-methyl-1H-pyrazol-3-yl)amino)thieno[2,3-d]pyrimidin-2-yl)amino)-9-azabicyclo[3.3.1]non-9-carboxylate (100 mg, 0.21 mmol) was dissolved in hydrochloride 1,4-dioxane solution(4.0 N, 5 mL). The mixture was stirred at room temperature for 30 minutes, concentrated, and then 2-(7-oxide benzotriazole)-N,N,N',N'-tetramethylurea hexafluorophosphate (120 mg, 0.31 mmol) was added to the mixture. The mixture was dissolved in N,N-dimethylformamide(5 mL). DIPEA (108 mg, 0.84 mmol) was slowly added dropwise into the mixture, and the mixture was then stirred under ice water bath for 10 minutes. 2-(4-Methylpiperazin-1-yl) acetic acid (36 mg, 0.23 mmol) was added thereto, and the mixture was stirred for another 1 hour. The reaction mixture was concentrated under reduced pressure, and the resulting product was purified by prep-HPLC to obtain the title compound as a white solid (43.8 mg, 40 %).

¹H NMR (400 MHz, DMSO) *δ* = 12.07 (s, 1H), 9.79 (s, 1H), 7.68 (d, *J* = 6.0 Hz, 1H), 7.02 (d, *J* = 6.0 Hz, 1H), 6.46 (s, 1H), 5.81 (s, 1H), 4.71 (s, 1H), 4.39 (s, 1H), 3.22 (d, *J* = 12.8 Hz, 1H), 3.06 (d, *J* = 12.8 Hz, 1H), 2.85 (s, 3H), 2.40 (s, 8H), 2.22 (s, 3H), 2.17 (s, 3H), 2.12-2.02 (m 2H), 1.90-1.61 (m, 8H).

MS *m*/*z* (ESI): 424.2 [M+H]⁺.

### Embodiment 258

### 1-((3-exo)-3-((4-((5-methyl-1H-pyrazol-3-yl)amino)thieno[2,3-d]pyrimidin-2-yl)amino)-9-azabicyclo[3.3.1]non-9-yl)-2-(4-methylpiperazin-1-yl)ethane-1-one

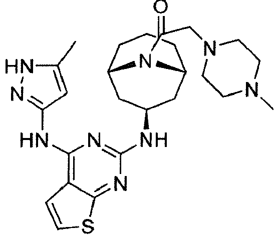

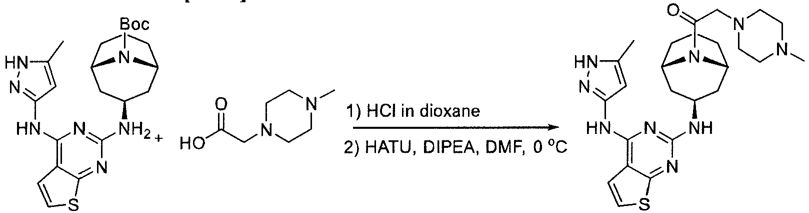

Tert-butyl-(3-*exo*)-3-((4-((5-methyl-1H-pyrazol-3-yl)amino)thieno[2,3-d]pyrimidin-2-yl)amino)-9-azabicyclo[3.3.1]non-9-carboxylate (100 mg, 0.21 mmol) was dissolved in hydrochloride 1,4-dioxane solution(4.0 N, 5 mL). The mixture was stirred at room temperature for 30 minutes, concentrated, and then 2-(7-oxide benzotriazole)-N,N,N',N'-tetramethylurea hexafluorophosphate (120 mg, 0.31 mmol) was added to the mixture. The mixture was dissolved in N,N-dimethylformamide(5 mL). DIPEA (108 mg, 0.84 mmol) was slowly added dropwise into the mixture, and the mixture was then stirred under ice water bath for 10 minutes. 2-(4-Methylpiperazin-1-yl) acetic acid (36 mg, 0.23 mmol) was added thereto and the mixture was stirred for another 1 hour. The reaction mixture was concentrated under reduced pressure, the resulting product was purified by prep-HPLC to obtain the title compound as a white solid (25.2 mg, 24 %).

¹H NMR (400 MHz, DMSO-*d₆*) *δ* = 12.07 (s, 1H), 9.92 (s, 1H), 7.68 (s, 1H), 6.96-6.61 (m, 3H), 4.85 (s, 1H), 4.65 (s, 1H), 4.37 (s, 1H), 3.10 (s, 2H), 2.37 (s, 8H), 2.21 (s, 3H), 2.14 (s, 3H), 2.09-1.99 (m, 2H), 1.97-1.46 (m, 8H).

MS *m*/*z* (ESI): 510.2 [M+H]⁺.

### Embodiment 259

### 1-((3-exo)-3-((4-((5-methyl-1H-pyrazol-3-yl)amino)thieno[2,3-d]pyrimidin-2-yl)amino)-9-azabicyclo[3.3.1]non-9-yl)-2-(piperazin-1-yl)ethane-1-one

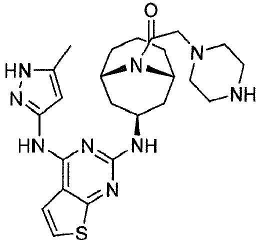

The preparation of 1-((3-*exo*)-3-((4-((5-methyl-1H-pyrazol-3-yl)amino)thieno[2,3-d]pyrimidin-2-yl)amino)-9-azabicyclo[3.3.1]non-9-yl)-2-(piperazin-1-yl)ethane-1-one was carried out with the reference to Embodiment 247.

MS *m*/*z* (ESI): 496.2 [M+H]⁺.

### Embodiment 260

### ((3-exo)-3-((4-((5-methyl-1H-pyrazol-3-yl)amino)thieno[2,3-d]pyrimidin-2-yl)amino)-9-azabicyclo[3.3.1]non-9-yl)(pyridin-2-yl)methanone

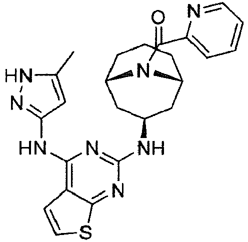

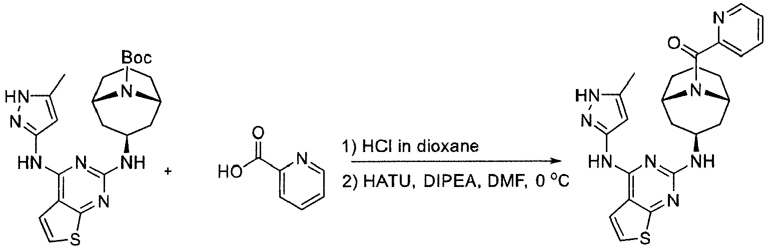

Tert-butyl-(3-*exo*)-3-((7-((5-methyl-1H-pyrazol-3-yl)amino)thieno[2,3-d]pyrimidin-2-yl)amino)-9-azabicyclo[3.3.1]non-9-carboxylate (100 mg, 0.21 mmol) was dissolved in hydrochloride 1,4-dioxane solution (4.0 N, 5 mL). The mixture was stirred at room temperature for 30 minutes, concentrated, and then 2-(7-oxide benzotriazole)-N,N,N',N'-tetramethylurea hexafluorophosphate (120 mg, 0.31 mmol) was added to the mixture. The mixture was dissolved in N,N-dimethylformamide(5 mL). DIPEA (108 mg, 0.84 mmol) was slowly added dropwise into the mixture, and the mixture was then stirred under ice water bath for 10 minutes. Pyridin-2-formic acid (28 mg, 0.23 mmol) was added thereto and the mixture was stirred for another 1 hour. The reaction mixture was concentrated under reduced pressure, and the resulting product was purified by prep-HPLC to obtain the title compound as a white solid (27.3 mg, 21 %).

¹H NMR (400 MHz, DMSO) *δ* = 12.07 (s, 1H), 9.86 (s, 1H), 8.59 (d, *J* = 4.4 Hz, 1H), 7.94 (td, *J* = 7.7, 1.6 Hz, 1H), 7.73-7.44 (m, 3H), 7.05-6.50 (m, 3H), 4.84 (d, *J* = 28.0 Hz, 2H), 3.94 (s, 1H), 2.21 (s, 3H), 2.18-1.59 (m, 10H).

MS *m*/*z* (ESI): 475.1 [M+H]⁺.

### Embodiment 261

### ((3-exo)-3-((4-((5-methyl-1H-pyrazol-3-yl)amino)thieno[2,3-d]pyrimidin-2-yl)amino)-9-azabicyclo[3.3.1]non-9-yl)(pyridin-3-yl)methanone

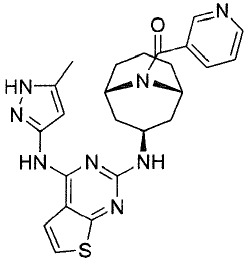

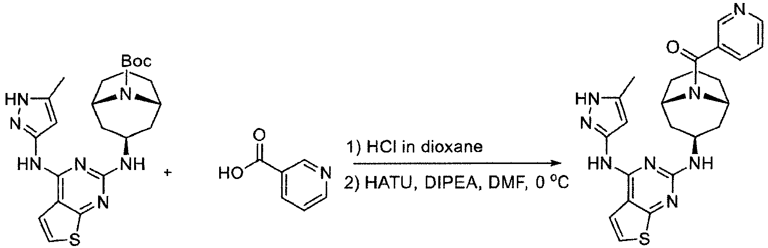

Tert-butyl-(3-*exo*)-3-((7-((5-methyl-1H-pyrazol-3-yl)amino)thieno[2,3-d]pyrimidin-2-yl)amino)-9-azabicyclo[3.3.1]non-9-carboxylate (100 mg, 0.21 mmol) was dissolved in hydrochloride 1,4-dioxane solution(4.0 N, 5 mL). The mixture was stirred at room temperature for 30 minutes, concentrated, and then 2-(7-oxide benzotriazole)-N,N,N',N'-tetramethylurea hexafluorophosphate (120 mg, 0.31 mmol) was added to the mixture. The mixutre was dissolved in N,N-dimethylformamide(5 mL). DIPEA (108 mg, 0.84 mmol) was slowly added dropwise into the mixture, and the mixture was then stirred under ice water bath for 10 minutes. Pyridin-3-formic acid (28 mg, 0.23 mmol) was added thereto and the mixture was stirred for another 1 hour. The reaction mixture was concentrated under reduced pressure, and the resulting product was purified by prep-HPLC to obtain the title compound as a white solid (28.6 mg, 22 %).

¹H NMR (400 MHz, DMSO-*d₆*) *δ* = 12.07 (s, 1H), 9.87 (s, 1H), 8.71-8.58 (m, 2H), 7.84 (d, *J* = 7.6 Hz, 1H), 7.69 (d, *J* = 6.0 Hz, 1H), 7.51 (dd, *J* = 7.6, 4.8 Hz, 1H), 6.96 (d, *J* = 5.2 Hz, 1H), 6.68-6.50 (m, 2H), 4.83 (d, *J* = 39.2 Hz, 2H), 3.78 (s, 1H), 2.21 (s, 3H), 2.13-1.61 (m, 10H).

MS *m*/*z* (ESI): 475.1 [M+H]⁺.

### Embodiment 262

### ((3-exo)-3-((4-((5-methyl-1H-pyrazol-3-yl)amino)thieno[2,3-d]pyrimidin-2-yl)amino)-9-azabicyclo[3.3.1]non-9-yl)(pyridin-4-yl)methanone

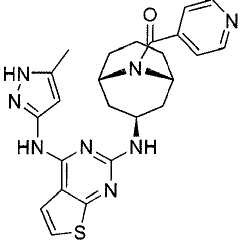

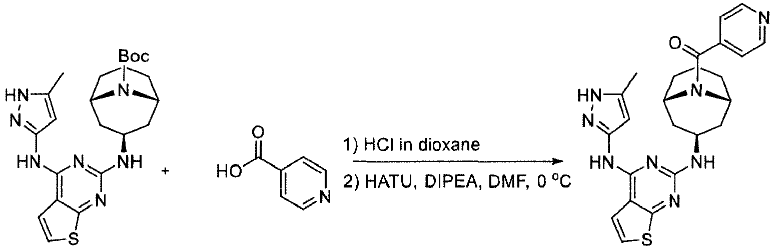

Tert-butyl-(3-*exo*)-3-((7-((5-methyl-1H-pyrazol-3-yl)amino)thieno[2,3-d]pyrimidin-2-yl)amino)-9-azabicyclo[3.3.1]non-9-carboxylate (100 mg, 0.21 mmol) was dissolved in hydrochloride 1,4-dioxane solution(4.0 N, 5 mL). The mixture was stirred at room temperature for 30 minutes, concentrated, and then 2-(7-oxide benzotriazole)-N,N,N',N'-tetramethylurea hexafluorophosphate (120 mg, 0.31 mmol) was added to the mixture. The mixture was dissolved in N,N-dimethylformamide(5 mL). DIPEA (108 mg, 0.84 mmol) was slowly added dropwise into the mixture, and the mixture was then stirred under ice water bath for 10 minutes. Pyridin-4-formic acid (28 mg, 0.23 mmol) was added thereto and the mixture was stirred for another 1 hour. The reaction mixture was concentrated under reduced pressure, and the resulting product was purified by prep-HPLC to obtain the title compound as a white solid (34.5 mg, 27 %).

¹H NMR (400 MHz, DMSO-*d₆*) *δ* = 12.07 (s, 1H), 9.87 (s, 1H), 8.69 (d, *J* = 6.0 Hz, 2H), 7.69 (d, *J* = 5.6 Hz, 1H), 7.39 (d, *J* = 5.6 Hz, 2H), 6.97 (d, *J* = 6.0 Hz, 1H), 6.57 (d, *J* = 7.6 Hz, 2H), 4.82 (d, *J=* 41.6 Hz, 2H), 3.68 (s, 1H), 2.21 (s, 3H), 2.14-1.59 (m, 10H).

MS *m*/*z* (ESI): 475.1 [M+H]⁺.

### Embodiment 263

### (1-methyl-1H-imidazol-2-yl) ((3-exo)-3-((4-((5-methyl-1H-pyrazol-3-yl)amino)thieno[2,3-d]pyrimidin-2-yl)amino)-9-azabicyclo[3.3.1]non-9-yl)methanone

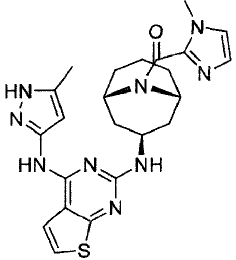

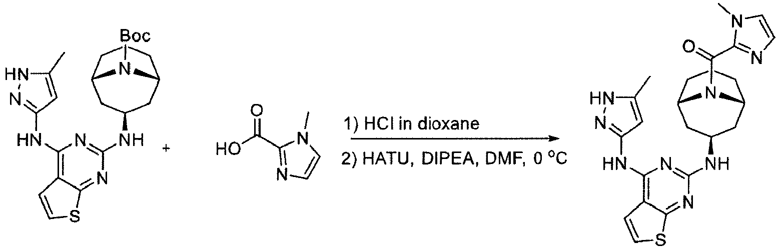

Tert-butyl-(3-*exo*)-3-((7-((5-methyl-1H-pyrazol-3-yl)amino)thieno[2,3-d]pyrimidin-2-yl)amino)-9-azabicyclo[3.3.1]non-9-carboxylate (100 mg, 0.21 mmol) was dissolved in hydrochloride 1,4-dioxane solution(4.0 N, 5 mL). The mixture was stirred at room temperature for 30 minutes, concentrated, and then 2-(7-oxide benzotriazole)-N,N,N',N'-tetramethylurea hexafluorophosphate (120 mg, 0.31 mmol) was added to the mixture, which was dissolved in N,N-dimethylformamide(5 mL). DIPEA (108 mg, 0.84 mmol) was slowly added dropwise into the mixture, and the mixture was then stirred under ice water bath for 10 minutes. 1-Methyl-1H-imidazol-2-carboxylic acid (29 mg, 0.23 mmol) was added thereto and the mixture was stirred for another 1 hour. The reaction mixture was concentrated under reduced pressure, and the resulting product was purified by prep-HPLC to obtain the title compound as a white solid (15.0 mg, 12 %).

¹H NMR (400 MHz, DMSO-*d₆*) *δ* = 12.08 (s, 1H), 9.87 (s, 1H), 7.68 (s, 1H), 7.29 (s, 1H), 6.97 (s, 2H), 6.65 (s, 2H), 4.91-4.80 (m, 3H), 3.77 (s, 3H), 2.22 (s, 3H), 2.14-1.60 (m, 10H).

MS *m*/*z* (ESI): 478.2 [M+H]⁺.

### Embodiment 264

### (1-methyl-1H-imidazol-4-yl) ((3-exo)-3-((4-((5-methyl-1H-pyrazol-3-yl)amino)thieno[2,3-d]pyrimidin-2-yl)amino)-9-azabicyclo[3.3.1]non-9-yl)methanone

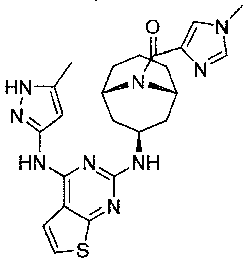

The preparation of (1-methyl-1H-imidazol-4-yl) ((3-*exo*)-3-((4-((5-methyl-1H-pyrazol-3-yl)amino)thieno[2,3-d]pyrimidin-2-yl)amino)-9-azabicyclo[3.3.1]non-9-yl)methanone was carried out with the reference to Embodiment 250.

¹H NMR (400 MHz, DMSO-*d₆*) *δ* = 12.08 (s, 1H), 9.86 (s, 1H), 7.70-7.60 (m, 3H), 6.95 (s, 1H), 6.65 (s, 1H), 5.57 (s, 1H), 4.82 (d, *J=* 65.2 Hz, 3H), 3.68 (s, 3H), 2.22 (s, 3H), 2.13-1.58 (s, 10H).

MS *m*/*z* (ESI): 478.1 [M+H]⁺.

### Embodiment 265

### N⁴-(5-methyl-1H-pyrazol-3-yl)-N²-((3-exo)-8(pyridin-3yl sulfonyl)-8-azabicyclo[3.2.1]oct-3-yl)thieno[2,3-d]pyrimidin-2,4-diamine

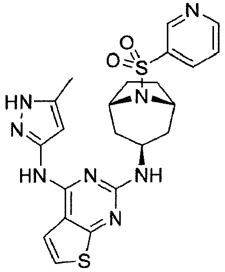

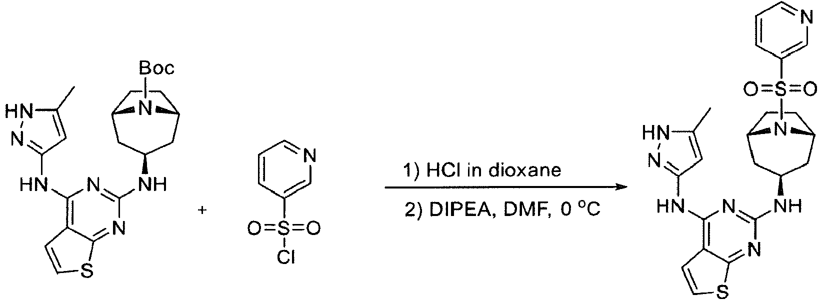

Tert-butyl-(3-*exo*)-3-((4-((5-methyl-1H-pyrazol-3-yl)amino)thieno[2,3-d]pyrimidin-2-yl)amino)-8-azabicyclo[3.2.1]oct-8-carboxylate (40 mg, 0.09 mmol) was dissolved in hydrochloride 1,4-dioxane solution(4.0 N, 2 mL). The mixture was stirred at room temperature for 30 minutes, concentrated, and then N,N-dimethylformamide (5 mL) was added to the mixture for dissolution. DIPEA (46 mg, 0.36 mmol) was slowly added dropwise into the mixture, and the mixture was then stirred under ice water bath for 10 minutes. Pyridin-3-sulfonylchlorine (18 mg, 0.1 mmol) was added thereto and the mixture was stirred for another 1 hour. The reaction mixture was concentrated under reduced pressure, and the resulting product was purified by prep-HPLC to obtain the title compound as a white solid (5.6 mg, 13 %).

¹H NMR (400 MHz, DMSO) *δ* = 12.01 (s, 1H), 9.86 (s, 1H), 9.05 (s, 1H), 8.87 (d, *J=* 4.4 Hz, 1H), 8.31 (d, *J* = 8.0 Hz, 1H), 7.65 (dd, *J =* 7.8 Hz, 5.0, 2H), 6.97 (s, 1H), 6.78 (s, 1H), 6.54 (s, 1H), 4.33 (s, 2H), 3.17 (d, *J* = 5.2 Hz, 1H), 2.14 (s, 3H), 1.99 (s, 2H), 1.76-1. 56 (m, 4H), 1.35-1.26 (m, 2H).

MS *m*/*z* (ESI): 497.1 [M+H]⁺.

### Embodiment 266

### N4-(5-methyl-1H-pyrazol-3-yl)-N2-((3-exo)-8-(pyridin-2-ylsulfonyl)-8-azabicyclo[3.2.1]oct-3-yl)thieno[2,3-d]pyrimidin-2,4-diamine

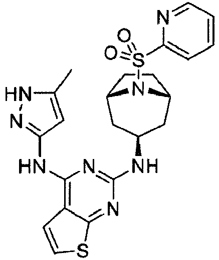

The preparation of N4-(5-methyl-1H-pyrazol-3-yl)-N2-((3-*exo*)-8-(pyridin-2-ylsulfonyl)-8-azabicyclo[3.2.1]oct-3-yl)thieno[2,3-d]pyrimidin-2,4-diamine was carried out with the reference to Embodiment 252.

¹H NMR (400 MHz, CD₃OD: CDCl₃, v/v= 1:2) *δ* 8.70 (d, *J* = 4.6 Hz, 1H), 8.00 (dt, *J* = 8.0, 4.6 Hz, 2H), 7.60 (ddd, *J* = 6.8, 4.8, 1.8 Hz, 1H), 7.35 (d, *J* = 6.0 Hz, 1H), 6.92 (d, *J* = 6.0 Hz, 1H), 6.19 (s, 1H), 4.43 (s, 2H), 4.40-4.32 (m, 1H), 2.27 (s, 3H), 2.15 (ddd, *J* = 12.7, 5.3, 2.6 Hz, 2H), 1.88-1.81 (m, 2H), 1.80-1.70 (m, 2H), 1.62 (dd, *J* = 8.6, 4.7 Hz, 2H).

MS *m*/*z* (ESI): 497.1 [M+H]⁺.

### Embodiment 267

### N4-(5-methyl-1H-pyrazol-3-yl)-N2-((3-exo)-9-(pyridin-2-ylsulfonyl)-9-azabicyclo[3.3.1]non-3-yl)thieno[2,3-d]pyrimidin-2,4-diamine

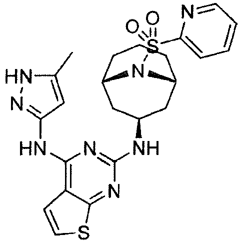

Tert-butyl (3-exo)-3-((4-((5-methyl-1H-pyrazol-3-yl)amino)thieno[2,3-d]pyrimidin-2-yl)amino)-9-azabicyclo[3.3.1]non-9-carboxylate (100 mg, 0.213 mmol) was dispersed in 4 M HCl in 1,4-dioxane (15 mL). The mixture was stirred at room temperature for 60 minutes, and concentrated under reduced pressure to remove the solvent. The residue solid was dissolved in anhydrous N,N-dimethylformamide (10 mL), then cooled to 0 °C, and DIPEA (1.05 mL, 6.39 mmol) and pyridin-2-sulfonyl chloride (40 mg, 0.224 mmol) successively added thereto. The reaction mixture was stirred at 0 °C for 2.5 hours, and concentrated under reduced pressure to remove the solvent. The residue was purified by prep-HPLC to obtain the title compound as a white solid (12.4 mg, 25%).

¹H NMR (400 MHz, DMSO-*d₆*) *δ* 12.07 (s, 1H), 9.85 (s, 1H), 8.78 (d, *J* = 4.0 Hz, 1H), 8.08 (td, *J* = 7.7, 1.4 Hz, 1H), 7.96 (d, *J* = 7.8 Hz, 1H), 7.67 (dd, *J* = 6.7, 4.7 Hz, 2H), 6.95 (s, 1H), 6.59 (d, *J* = 30.3 Hz, 2H), 4.85-4.71 (m, 1H), 4.18 (s, 2H), 2.17 (s, 3H), 2.05 (dd, *J* = 12.8, 4.9 Hz, 3H), 1.68 (d, *J* = 2.6 Hz, 7H).

MS *m*/*z* (ESI): 511.1 [M+H]⁺.

### Embodiment 268

### N4-(5-methyl-1H-pyrazol-3-yl)-N2-((3-exo)-9-(pyridin-3-ylsulfonyl)-9-azabicyclo[3.3.1]non-3-yl)thieno[2,3-d]pyrimidin-2,4-diamine

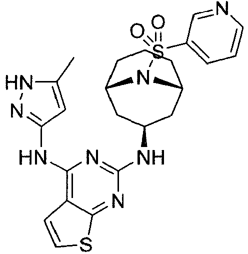

The preparation of N4-(5-methyl-1H-pyrazol-3-yl)-N2-((3-*exo*)-9-(pyridin-3-ylsulfonyl)-9-azabicyclo[3.3.1]non-3-yl)thieno[2,3-d]pyrimidin-2,4-diamine was carried out with the reference to Embodiment 267.

¹H NMR (400 MHz, CD₃OD:CDCl₃, v/v=1:1) *δ* 9.11 (s, 1H), 8.84 (d, *J =* 3.9 Hz, 1H), 8.29 (d, *J* = 8.2 Hz, 1H), 7.70-7.62 (m, 1H), 7.39 (d, *J* = 5.9 Hz, 1H), 6.96 (d, *J* = 5.8 Hz, 1H), 6.62 (s, 1H), 5.05-4.90 (m, 1H), 4.34 (d, *J=* 2.8 Hz, 2H), 2.55-2.19 (m, 5H), 2.19-1.61 (m, 8H).

MS *m*/*z* (ESI): 511.1 [M+H]⁺.

### Embodiment 269

### N2-((3-exo)-9-((1-methyl-1H-imidazol-2-yl)sulfonyl)-9-azabicyclo[3.3.1]non-3-yl)-N4-(5-methyl-1H-pyrazol-3-yl)thieno[2,3-d]pyrimidin-2,4-diamine

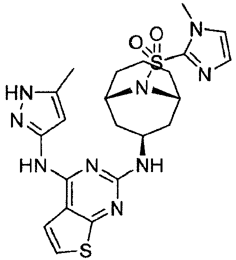

The preparation of N2-((3-*exo*)-9-((1-methyl-1H-imidazol-2-yl)sulfonyl)-9-azabicyclo[3.3.1]non-3-yl)-N4-(5-methyl-1H-pyrazol-3-yl)thieno[2,3-d]pyrimidin-2,4-diamine was carried out with the reference to Embodiment 267.

¹H NMR (400 MHz, DMSO-*d₆*) *δ* 12.05 (s, 1H), 9.86 (s, 1H), 7.67 (d, *J* = 2.9 Hz, 1H), 7.45 (s, 1H), 7.08 (s, 1H), 6.96 (d, *J* = 4.9 Hz, 1H), 6.73-6.47 (m, 2H), 4.88-4.74 (m, 1H), 4.12 (s, 2H), 3.87 (s, 3H), 2.19 (s, 3H), 2.09 (ddd, *J* = 5.5, 5.1, 1.0 Hz, 3H), 1.91-1.58 (m, 7H).

MS *m*/*z* (ESI): 514.1 [M+H]⁺.

### Embodiment 270

### N,N-dimethyl-2-((3-exo)-3-((4-((5-methyl-1H-pyrazol-3-yl)amino)thieno[2,3-d]pyrimidin-2-yl)amino)-9-azabicyclo[3.3.1]non-9-yl)acetamide

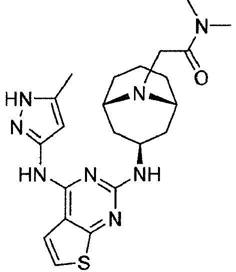

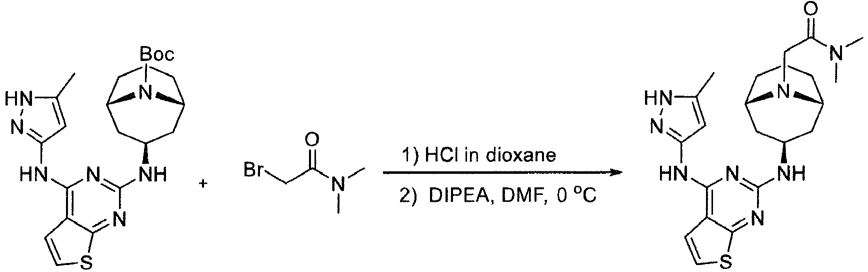

Tert-butyl-(3-*exo*)-3-((7-((5-methyl-1H-pyrazol-3-yl)amino)thieno[2,3-d]pyrimidin-2-yl)amino)-9-azabicyclo[3.3.1]non-9-carboxylate (100 mg, 0.21 mmol) was dissolved in hydrochloride 1,4-dioxane solution (4.0 N, 5 mL). The mixture was stirred at room temperature for 30 minutes, concentrated, and then N,N-dimethylformamide (5 mL) was added to the mixture for dissolution. DIPEA (108 mg, 0.84 mmol) was slowly added dropwise into the mixture, and the mixture was then stirred under ice water bath for 10 minutes. 2-Bromine-N,N-dimethylacetamide (38 mg, 0.23 mmol) were added thereto and the mixture was stirred for another 1 hour. The reaction mixture was concentrated under reduced pressure, and the resulting product was purified by prep-HPLC to obtain the title compound as a white solid (16.6 mg, 17 %).

¹H NMR (400 MHz, DMSO) *δ* = 12.00 (s, 1H), 9.80 (s, 1H), 7.63 (s, 1H), 6.74 (d, *J* = 128.0 Hz, 3H), 4.62 (s, 1H), 3.42 (s, 2H), 3.04 (s, 3H), 2.86 (s, 2H), 2.77 (s, 3H), 2.16 (s, 3H), 1.96-1.47 (m, 10H).

MS *m*/*z* (ESI): 455.2 [M+H]⁺.

### Embodiment 271

### N⁴-(5-methyl-1H-pyrazol-3-yl)-N²-((3-exo)-9-(pyridin-2-ylmethyl)-9-azabicyclo[3.3.1]non-3-yl)thieno[2,3-d]pyrimidin-2,4-diamine

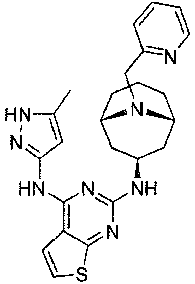

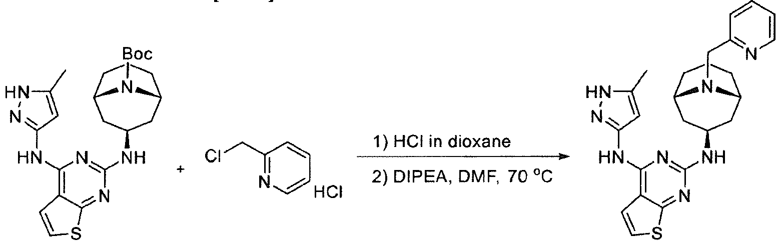

Tert-butyl-(3-*exo*)-3-((7-((5-methyl-1H-pyrazol-3-yl)amino)thieno[2,3-d)pyrimidin-2-yl)amino)-9-azabicyclo[3.3.1]non-9-carboxylate (100 mg, 0.21 mmol) was dissolved in hydrochloride 1,4-dioxane solution (4.0 N, 5 mL). The mixture was stirred at room temperature for 30 minutes, concentrated, and then the mixture was dissolved in N,N-dimethylformamide(5 mL). DIPEA (108 mg, 0.84 mmol) was slowly added dropwise into the mixture, and the mixture was then stirred under ice water bath for 10 minutes. 2-(Chloromethyl)pyridinyl hydrochloride (38 mg, 0.23 mmol) was added to the mixture, and the mixture was then stirred at 70°C overnight. The reaction mixture was concentrated under reduced pressure, and the resulting product was purified by prep-HPLC to obtain the title compound as a white solid (20.8 mg, 22 %).

¹H NMR (400 MHz, DMSO-*d₆*) *δ* = 12.06 (s, 1H), 9.85 (s, 1H), 8.47 (d, *J* = 4.0 Hz, 1H), 7.81-7.48 (m, 3H), 7.33-6.52 (m, 4H), 4.74 (s, 1H), 3.92 (s, 2H), 2.89 (s, 2H), 2.23 (d, *J* = 13.6 Hz, 3H), 2.08-1.50 (m, 10H).

MS *m*/*z* (ESI): 461.1 [M+H]⁺.

### Embodiment 272

### N²-((3-exo)-9-((1-methyl-1H-imidazol-2-yl)methyl)-9-azabicyclo[3.3.1]non-3-yl)-N⁴-(5-methyl-1H-pyrazol-3-yl)thieno[2,3-d]pyrimidin-2,4-diamine

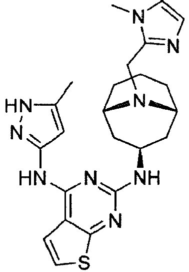

The preparation of N²-((3-exo)-9-((1-methyl-1H-imidazol-2-yl)methyl)-9-azabicyclo[3.3.1]non-3-yl)-N⁴-(5-methyl-1H-pyrazol-3-yl)thieno[2,3-d]pyrimidin-2,4-diamine was carried out with the reference to Embodiment 271.

¹H NMR (400 MHz, DMSO-*d₆*) *δ* = 12.06 (s, 1H), 9.84 (s, 1H), 7.67 (s, 1H), 7.08 (s, 1H), 6.95 (s, 1H), 6.74-6.55 (m, 3H), 4.69 (s, 1H), 3.91 (s, 2H), 3.69 (s, 3H), 2.84 (s, 2H), 2.20 (s, 3H), 2.01-1.66 (m, 10H).

MS *m*/*z* (ESI): 464.2 [M+H]⁺.

### Embodiment 273

### 3-((3-exo))-3-((4-((5-(hydroxymethyl)-1H-pyrazol-3-yl)amino)thieno[2,3-d]pyrimidin-2-yl)amino)-8-azabicyclo[3.2.1]oct-8-yl)propionitrile

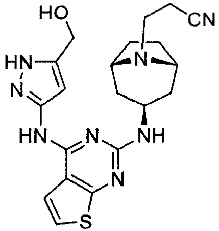

### Step 1: preparation of (3-((2-chlorothieno[2,3-d]pyrimidin-4-yl)amino)-1H-pyrazol-5-yl)methanol

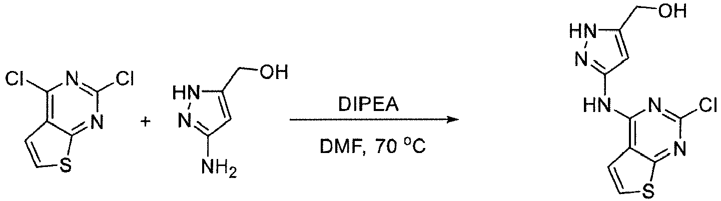

2,4-Dichlorothieno[2,3-d]pyrimidine (100 mg, 0.49 mmol), (3-amino-1H-pyrazol-5-yl)methanol (55 mg, 0.49 mmol) and DIPEA (190 mg, 1.47 mmol) were added to N'N-dimethylformamide(2 mL). The reaction mixture was stirred at 70 °C overnight. Then the mixture was concentrated under reduced pressure, and the crude product obtained was purified by flash silica gel chromatography to obtain the title compound as a yellow solid (100 mg, 73%).

MS *m*/*z* (ESI):282.0 [M+H]⁺.

### Step 2: preparation of Tert-butyl (3-exo)-3-((4-((5-(hydroxymethyl)-1H-pyrazol-3-yl)amino)thieno[2,3-d]pyrimidin-2-yl)amino)-8-azabicyclo[3.2.1]oct-8-carboxylate

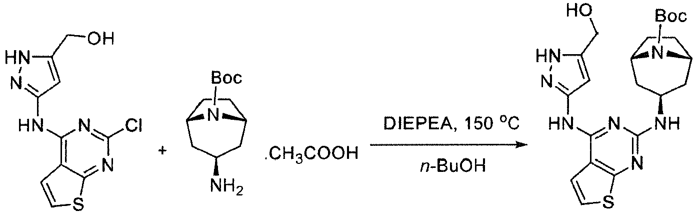

(3-((2-Chlorothieno[2,3-d]pyrimidin-4-yl)amino)-1H-pyrazol-5-yl)methanol (100 mg, 0.36 mmol), N-Boc-exo-3-aminotropane acetate (113 mg, 0.40 mmol) and DIPEA (140 mg, 1.08 mmol) were added to n-butanol (2.5 mL). The reaction mixture was mixed evenly, then heated to 150 °C under microwave condition, reacted for 10 hours, then cooled down to room temperature, and concentrated under reduced pressure. The crude product obtained was purified by flash silica gel chromatography to obtain the title compound as a pale yellow solid (60 mg, 35%).

MS *m*/*z* (ESI):472.0 [M+H]⁺.

### Step 3: preparation of 3-((3-exo)-3-((4-((5-(hydroxymethyl)-1H-pyrazol-3-yl)amino)thieno[2,3-d]pyrimidin-2-yl)amino)-8-azabicyclo[3.2.1]oct-8-yl)propionitrile

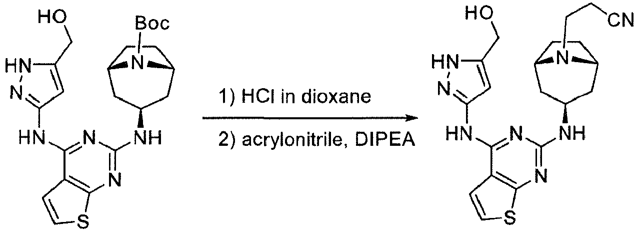

Tert-butyl (3-exo)-3-((4-((5-(hydroxymethyl)-1H-pyrazol-3-yl)amino)thieno[2,3-d]pyrimidin-2-yl)amino)-8-azabicyclo[3.2.1]oct-8-carboxylate (60 mg, 0.13 mmol) was dissolved in methanol (10 mL), and then hydrochloride dioxane (4N, 2.5 mL) was slowly added dropwise into the reaction mixture. The reaction was carried out at room temperature for 2 hours, and then the mixture was concentrated under reduced pressure. The crude product obtained were added to a mixed solution of methanol (15 mL), DIPEA (0.5 mL) and acrylonitrile (1 mL). The reaction was carried out at room temperature for 2 hour, and the mixture was concentrated under reduced pressure, and then purified by prep-HPLC to obtain the title compound as a white solid (11.6 mg, 21%).

¹H NMR (400 MHz, CD₃OD) *δ* 7.39 (dd, *J* = 6.0 Hz, 1H), 6.99 (dd, *J* = 5.6 Hz, 1H),6.02-6.04(m,1H), 4.60 (s,2 H), 4.21-4.24 (m, 1 H),3.45-3.42 (m,2H), 2.83 (s, 2 H), 2.69-2.65 (m, 2 H),2.08-1.91 (m, 6 H), 1.69(t, *J* = 12.4 Hz, 2 H).

MS *m*/*z* (ESI):425.1[M+H]⁺.

### Embodiment 274

### 3-((3-exo)-3-((4-((5-(hydroxymethyl)-1H-pyrazol-3-yl)amino)thieno[2,3-d]pyrimidin-2-yl)amino)-9-azabicyclo[3.3.1]non-9-yl)propionitrile

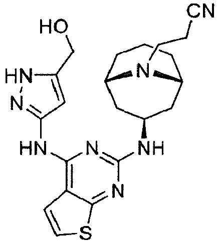

The preparation of 3-((3-*exo*)-3-((4-((5-(hydroxymethyl)-1H-pyrazol-3-yl)amino)thieno[2,3-d]pyrimidin-2-yl)amino)-9-azabicyclo[3.3.1]non-9-yl)propionitrile was carried out with the reference to Embodiment 273.

MS *m*/*z* (ESI): 439.2 [M+H]⁺.

### Embodiment 275

### 3-((3-exo)-3-((4-((5-(hydroxymethyl)-1H-pyrazol-3-yl)amino)thieno[2,3-d]pyrimidin-2-yl)(methyl)amino)-9-azabicyclo[3.3.1]non-9-yl)propionitrile

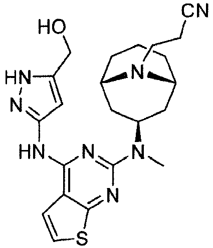

The preparation of 3-((3-*exo*)-3-((4-((5-(hydroxymethyl)-1H-pyrazol-3-yl)amino)thieno[2,3-d]pyrimidin-2-yl)(methyl)amino)-9-azabicyclo[3.3.1]non-9-yl)propionitrile was carried out with the reference to Embodiment 273.

MS *m*/*z* (ESI): 453.2 [M+H]⁺.

### Embodiment 276

### 1-(((3-exo)-3-((4-((5-(hydroxymethyl)-1H-pyrazol-3-yl)amino)thieno[2,3-d]pyrimidin-2-yl)amino)-9-azabicyclo[3.3.1]non-9-yl)sulfonyl)azetindin-3-formonitrile

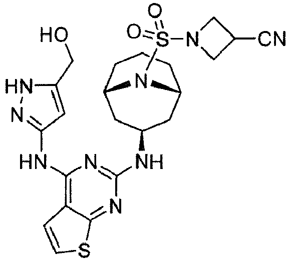

The preparation of 1-(((3-*exo*)-3-((4-((5-(hydroxymethyl)-1H-pyrazol-3-yl)amino)thieno[2,3-d]pyrimidin-2-yl)amino)-9-azabicyclo[3.3.1]non-9-yl)sulfonyl)azetindin-3-formonitrile was carried out with the reference to Embodiment 273.

MS *m*/*z* (ESI): 530.2 [M+H]⁺.

### Embodiment 277

### 3-((3-exo)-3-((6-methyl-4-((5-methyl-1H-pyrazol-3-yl)amino)thieno[2,3-d]pyrimidin-2-yl)amino)-8-azabicyclo[3.2.1]oct-8-yl)propionitrile

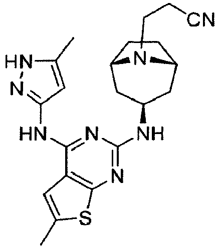

### Step 1: preparation of tert-butyl-(3-exo)-3-((6-methyl-4-((5-methyl-1H-pyrazol-3-yl)amino)thieno[2,3-d]pyrimidin-2-yl)amino)-8-azabicyclo[3.2.1]oct-8-carboxylate

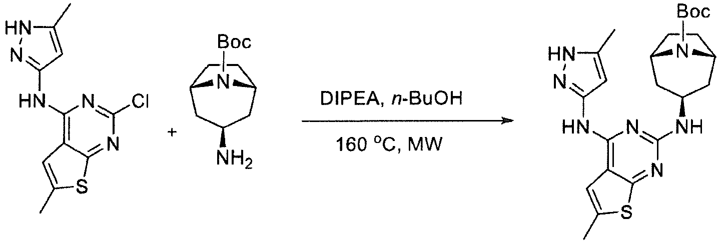

Tert-butyl-(3-*exo*)-3-amino-8-azabicyclo[3.2.1]oct-8-carboxylate (194 mg, 0.86 mmol) and DIPEA (186 mg, 1.44 mmol) were successively added the solution of 2-chloro-6-methyl-N-(5-methyl-1H-pyrazol-3-yl)thieno[2,3-d]pyrimidin-4-amine (200 mg, 0.72 mmol) in n-butanol (10 mL), and then the mixture was stirred at 160°C for 15 hours. When the reaction was completed, the reaction mixture was extracted with ethyl acetate (15 mL x 3), and washed with saturated sodium chloride aqueous solution (15 mL x 3). The organic phase was collected and dried over anhydrous sodium sulfate, filtered and concentrated under reduced pressure. The resulting product was purified by silica gel column chromatography (dichloromethane: methanol=98:2) to obtain the title compound as a pale yellow solid (124 mg, 37%).

MS *m*/*z* (ESI): 470.2 [M+H]⁺.

### Step 2: preparation of 3-((3-exo)-3-((6-methyl-4-((5-methyl-1H-pyrazol-3-yl)amino)thieno[2,3-d]pyrimidin-2-yl)amino)-8-azabicyclo[3.2.1]oct-8-yl)propionitrile

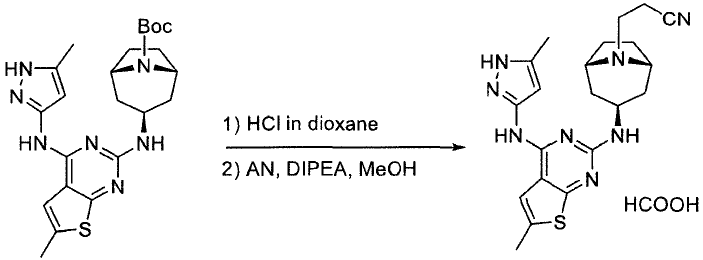

Tert-butyl-(3-*exo*)-3-((6-methyl-4-((5-methyl-1H-pyrazol-3-yl)amino)thieno[2,3-d]pyrimidin-2-yl)amino)-8-azabicyclo[3.2.1]oct-8-carboxylate (124 mg, 0.26 mmol) was dissolved in hydrochloride 1,4-dioxane solution (4.0 N, 5 mL). The mixture was stirred at room temperature for 30 minutes, concentrated, and then methanol (10 mL) was added to the mixture for dissolution. DIPEA (137 mg, 1.06 mmol) was slowly added dropwise into the mixture, and the mixture was stirred at room temperature for 10 minutes. Acrylonitrile (21 mg, 0.39 mmol) was added thereto and the mixture was stirred for another 2 hours. The reaction mixture was concentrated under reduced pressure, and the resulting product was purified by prep-HPLC to obtain the title compound as a white solid (12.7 mg, 12 %).

¹H NMR (400 MHz, DMSO) *δ* = 9.70 (s, 1H), 7.30 (s, 1H), 6.59 (s, 3H), 4.15 (s, 1H), 3.29 (s, 2H), 2.61 (s, 4H), 2.39 (s, 3H), 2.22 (s, 3H), 1.90 (s, 2H), 1.78-1.50 (m, 6H).

MS *m*/*z* (ESI): 423.2 [M+H]⁺.

### Embodiment 278

### 3-((3-exo)-3-((7-((5-methyl-1H-pyrazol-3-yl)amino)thiazolo[5,4-d]pyrimidin-5-yl)amino)-9-azabicyclo[3.3.1]non-9-yl)propionitrile

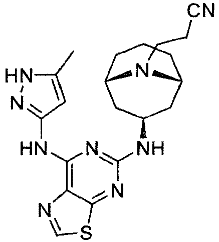

### Step 1: preparation of 5-chloro-N-(5-methyl-1H-pyrazol-3-yl)thiazolo[5,4-d]pyrimidin-7-amine

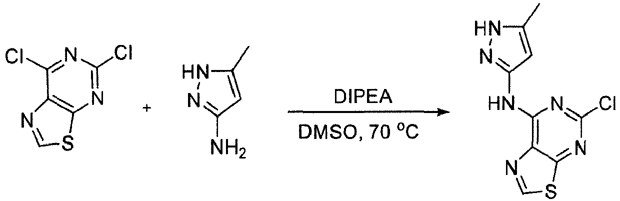

3-Amino-5-methylpyrazol (116 mg, 1.2 mmol) and DIPEA (258 mg, 2 mmol) were successively added to the solution of 5,7-dichlorothiazolo[5,4-d]pyrimidine (206 mg, 1 mmol) in dimethyl sulfoxide (10 mL), and then the mixture was stirred at 70°C for 1 hour. When the reaction was completed, water (50 mL) was added to the reaction mixture, and a solid was precipitated, which was then filtered and slurried with ethyl acetate to obtain the title compound as a yellow solid (200 mg, 75%).

MS *m*/*z* (ESI): 267.0 [M+H]⁺.

### Step 2: preparation of tert-butyl-(3-exo)-3-((7-((5-methyl-1H-pyrazol-3-yl)amino)thiazolo[5,4-d]pyrimidin-5-yl)amino)-9-azabicyclo[3.3.1]non-9-carboxylate

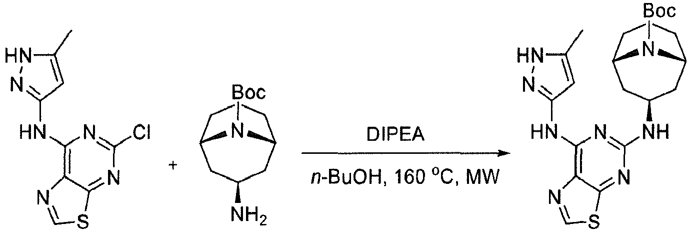

Tert-butyl-(3-*exo*)-3-amino-9-azabicyclo[3.3.1]non-9-carboxylate (216 mg, 0.9 mmol), DIPEA (193 mg, 1.5 mmol) were successively added to the solution of 5-chloro-N-(5-methyl-1H-pyrazol-3-yl)thiazolo[5,4-d]pyrimidin-7-amine (200 mg, 0.75 mmol) in n-butanol (10 mL), and the mixture was stirred at 160°C under microwave condition for 15 hours. When the reaction was completed, the reaction mixture was extracted with ethyl acetate (15 mL x 3), and washed with saturated sodium chloride aqueous solution (15 mL x 3). The organic phase was collected and dried over anhydrous sodium sulfate, filtered and concentrated under reduced pressure. The resulting product was purified by silica gel column chromatography (dichloromethane: methanol=95:5) to obtain the title compound as a pale yellow solid (232 mg, 66%).

MS *m*/*z* (ESI): 471.2 [M+H]⁺.

### Step 3: preparation of 3-((3-exo)-3-((7-((5-methyl-1H-pyrazol-3-yl)amino)thiazolo[5,4-d]pyrimidin-5-yl)amino)-9-azabicyclo[3.3.1]non-9-yl)propionitrile

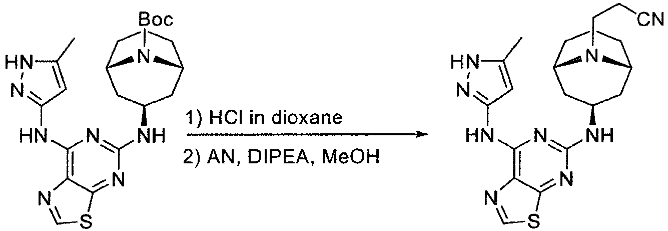

Tert-butyl-(3-*exo*)-3-((7-((5-methyl-1H-pyrazol-3-yl)amino)thiazolo[5,4-d]pyrimidin-5-yl)amino)-9-azabicyclo[3.3.1]non-9-carboxylate (232 mg, 0.49 mmol) was dissolved in hydrochloride 1,4-dioxane solution (4.0 N, 5 mL). The mixture was stirred at room temperature for 30 minutes and the mixture was concentrated, and then methanol (10 mL) was added to the mixture for dissolution. DIPEA (127 mg, 0.98 mmol) was slowly added dropwise into the mixture, and the mixture was stirred at room temperature for 10 minutes. Acrylonitrile (39 mg, 0.74 mmol) was added thereto and the mixture was stirred for another 2 hours. The reaction mixture was concentrated under reduced pressure, and the resulting product was purified by prep-HPLC to obtain the title compound as a pale yellow solid (63 mg, 30 %).

¹H NMR (400 MHz, DMSO) *δ* = 12.06 (s, 1H), 9.29 (s, 1H), 8.76 (d, *J* = 18.8 Hz, 1H), 6.92 (d, *J* = 7.2 Hz, 1H), 6.57 (s, 1H), 4.67 (s, 1H), 3.31 (s, 2H), 2.58 (t, *J* = 6.2 Hz, 4H), 2.19 (s, 3H), 2.00-1.65 (m, 10H).

MS *m*/*z* (ESI): 424.2 [M+H]⁺.

### Embodiment 279

### 3-((3-exo)-3-((4-((5-methyl-1H-pyrazol-3-yl)amino)thieno[2,3-d]pyrimidin-2-yl)oxo)-8-azabicyclo[3.2.1]oct-8-yl)propionitrile

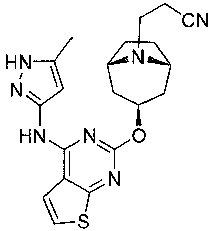

### Step 1: preparation of Tert-butyl (3-exo)-3-((4-((5-methyl-1H-pyrazol-3-yl)amino)thieno[2,3-d]pyrimidin-2-yl)oxo)-8-azabicyclo[3.2.1]oct-8-carboxylate

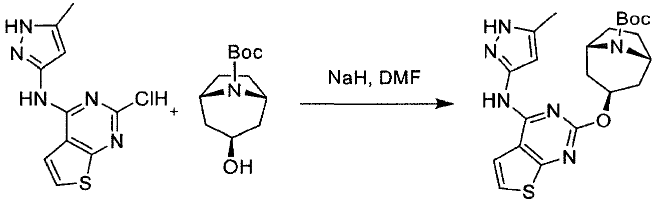

NaH (120 mg, 3.01 mmol, 60%) were added in batches to the solution of Tert-butyl (3-exo)-3-hydroxy-8-azabicyclo[3.2.1]oct-8-carboxylate (427 mg, 1.88 mmol) in N,N-dimethylformamide (2 mL) at room temperature. The mixture was stirred at room temperature for 5 minutes, and then the solution of 2-chloro-N-(5-methyl-1H-pyrazol-3-yl)thieno[2,3-d]pyrimidin-4-amine (100 mg, 0.376 mmol) in N,N-dimethylformamide (1 mL) was added dropwise. Under nitrogen atmosphere, the mixture was stirred at 120 °C for 2 hours, and cooled to room temperature. The mixture was then poured into ice water (10 mL), stirred for 10 minutes, and filtered. The filtrate was extracted with ethyl acetate. The organic phases were combined and washed with saturated sodium chloride aqueous solution. The organic phase was collected and dried over anhydrous sodium sulfate, filtered, and then concentrated under reduced pressure to remove the organic solvent. The resulting product obtained was purified by silica gel chromatography to obtain the title compound as a yellow oil (149 mg, 87%).

### Step 2: preparation of 3-((3-exo)-3-((4-((5-methyl-1H-pyrazol-3-yl)amino)thieno[2,3-d]pyrimidin-2-yl)oxo)-8-azabicyclo[3.2.1]oct-8-yl)propionitrile

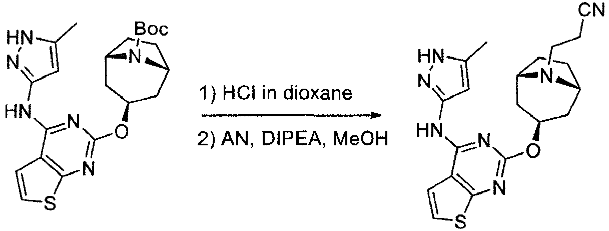

Tert-butyl (3-exo)-3-((4-((5-methyl-1H-pyrazol-3-yl)amino)thieno[2,3-d]pyrimidin-2-yl)oxo)-8-azabicyclo[3.2.1]oct-8-carboxylate (77 mg, 0.169 mmol) was dissolved in methanol (2 mL). The mixture was stirred at room temperature and 4M HCl in 1,4-dioxane (2 mL) was added thereto. The mixture was stirred at room temperature for 1 hour, and concentrated under reduced pressure. The residue was dissolved in anhydrous methanol (1 mL), and DIPEA (109 mg, 0.844 mmol) and acrylonitrile (45 mg, 0.844 mmol) were successively added thereto. The resulting reaction mixture was stirred at room temperature for another 1 hour. The reaction mixture was concentrated under reduced pressure, the residue was successively purified by silica gel chromatography and preparative TLC, to obtain the title compound gray solid (7 mg, 10%).

¹H NMR (400 MHz, CD₃OD) *δ* 7.50 (d, *J*= 6.1 Hz, 1H), 7.22 (d, *J*= 5.9 Hz, 1H), 6.51 (s, 1H), 5.43-5.26 (m, 1H), 3.44-3.37 (m, 2H), 2.78 (t, *J*= 6.9 Hz, 2H), 2.62 (t, *J*= 6.9 Hz, 2H), 2.33 (s, 3H), 2.12-2.00 (m, 4H), 1.86-1.74 (m, 4H).

MS *m*/*z* (ESI): 410.1[M+H]⁺.

### Embodiment 280

### 3-((3-exo)-3-((6-(methoxylmethyl)-4-((5-methyl-1H-pyrazol-3-yl)amino)thieno[2,3-d]pyrimidin-2-yl)amino)-8-azabicyclo[3.2.1]oct-8-yl)propionitrile

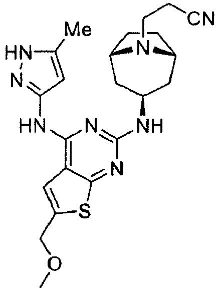

The preparation of 3-((3-*exo*)-3-((6-(methoxylmethyl)-4-((5-methyl-1H-pyrazol-3-yl)amino)thieno[2,3-d]pyrimidin-2-yl)amino)-8-azabicyclo[3.2.1]oct-8-yl)propionitrile was carried out with the reference to Embodiment 277.

MS *m*/*z* (ESI): 453.2 [M+H]⁺.

### Embodiment 281

### 3-((3-exo)-3-((4-((5-methyl-1H-pyrazol-3-yl)amino)-6-morpholinothieno[2,3-d]pyrimidin-2-yl)amino)-8-azabicyclo[3.2.1]oct-8-yl)propionitrile

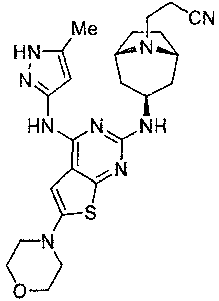

The preparation of 3-((3-*exo*)-3-((4-((5-methyl-1H-pyrazol-3-yl)amino)-6-morpholinothieno[2,3-d]pyrimidin-2-yl)amino)-8-azabicyclo[3.2.1]oct-8-yl)propionitrile was carried out with the reference to Embodiment 277.

MS *m*/*z* (ESI):494.2 [M+H]⁺.

### Embodiment 282

### 3-((3-exo)-3-((4-((5-methyl-1H-pyrazol-3-yl)amino)-6-(morpholinomethyl)thieno[2,3-d]pyrimidin-2-yl)amino)-8-azabicyclo[3.2.1]oct-8-yl)propionitrile

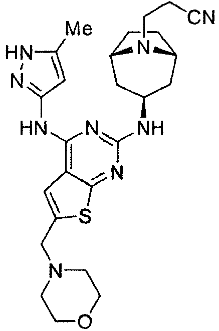

The preparation of 3-((3-*exo*)-3-((4-((5-methyl-1H-pyrazol-3-yl)amino)-6-(morpholinomethyl)thieno[2,3-d]pyrimidin-2-yl)amino)-8-azabicyclo[3.2.1]oct-8-yl)propionitrile was carried out with the reference to Embodiment 277.

MS *m*/*z* (ESI): 508.3 [M+H]⁺.

### Embodiment 283

### 3-((3-exo)-3-((4-((5-methyl-1H-pyrazol-3-yl)amino)-6-((4-methylpiperazin-1-yl)methyl)thieno[2,3-d]pyrimidin-2-yl)amino)-9-azabicyclo[3.3.1]non-9-yl)propionitrile

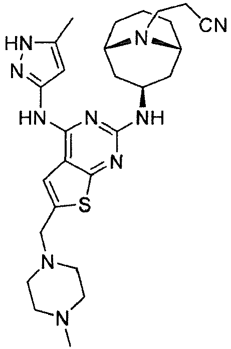

The preparation of 3-((3-*exo*)-3-((4-((5-methyl-1H-pyrazol-3-yl)amino)-6-((4-methylpiperazin-1-yl)methyl)thieno[2,3-d]pyrimidin-2-yl)amino)-9-azabicyclo[3.3.1]non-9-yl)propionitrile was carried out with the reference to Embodiment 277.

MS *m*/*z* (ESI): 535.3 [M+H]⁺.

### Embodiment 284

### 3-((3-exo)-3-((4-((5-methyl-1H-pyrazol-3-yl)amino)-6-(pyridin-3-yl thio)thieno[2,3-d]pyrimidin-2-yl)amino)-9-azabicyclo[3.3.1]non-9-yl)propionitrile

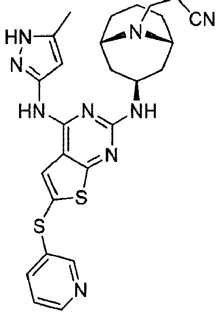

The preparation of 3-((3-*exo*)-3-((4-((5-methyl-1H-pyrazol-3-yl)amino)-6-(pyridin-3-yl thio)thieno[2,3-d]pyrimidin-2-yl)amino)-9-azabicyclo[3.3.1]non-9-yl)propionitrile was carried out with the reference to Embodiment 277.

MS *m*/*z* (ESI): 532.2 [M+H]⁺.

### Embodiment 285

### 3-((3-exo)-3-((4-((5-methyl-1H-pyrazol-3-yl)amino)thieno[3,2-d]pyrimidin-2-yl)amino)-8-azabicyclo[3.2.1]oct-8-yl)sulfonyl)azacyclobutane-3-nitrile

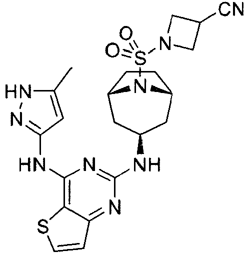

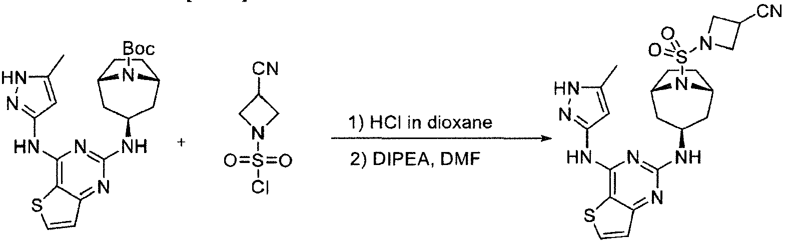

Tert-butyl-(3-*exo*)-3-((4-((5-methyl-1H-pyrazol-3-yl)amino)thieno[3,2-d]pyrimidin-2-yl)amino)-8-azabicyclo[3.2.1]oct-8-carboxylate (100 mg, 0.22 mmol) was dissolved in hydrochloride 1,4-dioxane solution (4.0 N, 5 mL). The mixture was stirred at room temperature for 30 minutes, concentrated, and then the mixture was dissolved in N,N-dimethylformamide (10 mL). DIPEA (108 mg, 0.84 mmol) was slowly added dropwise into the mixture, and the mixture was stirred at room temperature for 10 minutes. 3-Nitrile azacyclobutane-1-sulfonylchlorine (45 mg, 0.25 mmol) were added thereto the mixture, and the mixture was stirred at room temperature overnight. The reaction mixture was concentrated under reduced pressure, and the resulting product was purified by prep-HPLC to obtain the title compound as a white solid (23.2 mg, 21%).

¹H NMR (400 MHz, DMSO-*d₆*) *δ* = 12.07 (s, 1H), 9.74 (s, 1H), 7.90 (s, 1H), 7.00 (s, 1H), 6.54 (s, 2H), 4.27 (s, 1H), 4.13 (s, 2H), 4.04 (t, *J* = 8.4 Hz, 2H), 3.98-3.89 (m, 2H), 3.80 (dd, *J* = 15.2, 6.0 Hz, 1H), 2.23 (s, 3H), 1.99 (s, 4H), 1.84 (d, *J* = 7.2 Hz, 2H), 1.63 (s, 2H).

MS *m*/*z* (ESI): 500.1 [M+H]⁺.

### Embodiment 286

### 1-(((3-exo)-3-((6-methyl-4-((5-methyl-1H-pyrazol-3-yl)amino)thieno[3,2-d]pyrimidin-2-yl)amino)-8-azabicyclo[3.2.1]oct-8-yl)sulfonyl)azetindin-3-formonitrile

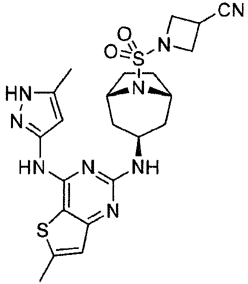

The preparation of 1-(((3-*exo*)-3-((6-methyl-4-((5-methyl-1H-pyrazol-3-yl)amino)thieno[3,2-d]pyrimidin-2-yl)amino)-8-azabicyclo[3.2.1]oct-8-yl)sulfonyl)azetindin-3-formonitrile was carried out with the reference to Embodiment 267.

¹H NMR (400 MHz, DMSO-*d₆*) *δ* 12.02 (s, 1H), 9.64 (s, 1H), 6.77-6.45 (m, 3H), 4.25-4.23 (m, 1H), 4.12 (s, 2H), 4.06-4.02 (m, 2H), 3.95-3.88 (m, 2H), 3.83-3.77 (m, 1H), 2.24-2.21 (m, 4H), 1.99-1.98 (m, 5H), 1.84-1.81 (m, 2H), 1.64-1.59 (m, 3H).

MS *m*/*z* (ESI): 513.1 [M+H]⁺.

### Embodiment 287

### 2-(dimethylamine)-1-((3-exo)-3-((4-((5-methyl-1H-pyrazol-3-yl)amino)thieno[3,2-d]pyrimidin-2-yl)amino)-8-azabicyclo[3.2.1]oct-8-yl)ethane-1-one

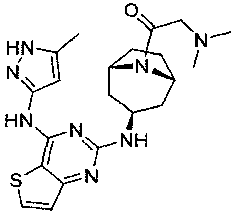

The preparation of 2-(dimethylamine)-1-((3-exo)-3-((4-((5-methyl-1H-pyrazol-3-yl)amino)thieno[3,2-d]pyrimidin-2-yl)amino)-8-azabicyclo[3.2.1]oct-8-yl)ethane-1-one was carried out with the reference to Embodiment 285.

¹H NMR (400 MHz, DMSO-*d₆*) *δ* = 12.06 (s, 1H), 9.72 (s, 1H), 7.89 (s, 1H), 6.99 (s, 1H), 6.49 (d, J= 58.8 Hz, 2H), 4.59-4.28 (m, 3H), 3.04 (s, 2H), 2.15 (s, 9H), 1.98-1.80 (m, 6H), 1.59-1.45 (m, 2H).

MS *m*/*z* (ESI): 441.1 [M+H]⁺.

### Embodiment 288

### 1-((3-exo)-3-((4-((5-methyl-1H-pyrazol-3-yl)amino)thieno[3,2-d]pyrimidin-2-yl)amino)-9-azabicyclo[3.3.1]non-9-yl)-2-morpholinoethane-1-one

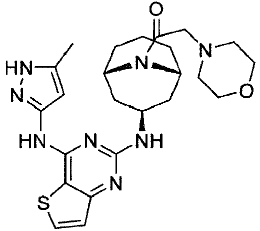

### Step 1: preparation of tert-butyl-(3-exo)-3-((4-((5-methyl-1H-pyrazol-3-yl)amino)thieno[3,2-d]pyrimidin-2-yl)amino)-9-azabicyclo[3.3.1]non-9-carboxylate

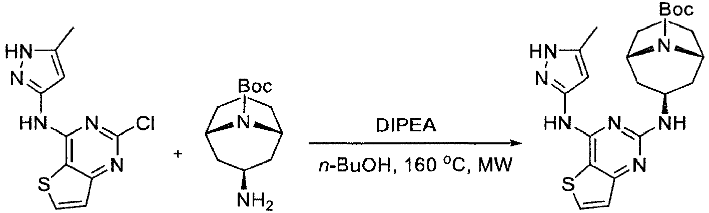

Tert-butyl-(3-*exo*)-3-amino-9-azabicyclo[3.3.1]non-9-carboxylate (271 mg, 1.13 mmol) was added to 2-chloro-N-(5-methyl-1H-pyrazol-3-yl)thieno[3,2-d]pyrimidin-4-amine(250 mg, 0.94 mmol) in n-butanol (10 mL), and DIPEA (242 mg, 1.88 mmol) were successively added thereto. The mixture was then stirred at 160°C under microwave condition for 15 hours. When the reaction was completed, the reaction mixture was extracted with ethyl acetate (15 mL x 3), and washed with saturated sodium chloride aqueous solution (15 mL x 3). The organic phase was collected and dried over anhydrous sodium sulfate, filtered and concentrated under reduced pressure. The resulting product was purified by silica gel column chromatography (dichloromethane: methanol=98:2) to obtain the title compound as pale white solid (150 mg, 34%).

MS *m*/*z* (ESI): 470.1 [M+H]⁺.

### Step 2: preparation of tert-butyl-(3-exo)-3-((4-((5-methyl-1H-pyrazol-3-yl)amino)thieno[3,2-d]pyrimidin-2-yl)amino)-9-azabicyclo[3.3.1]non-9-carboxylate

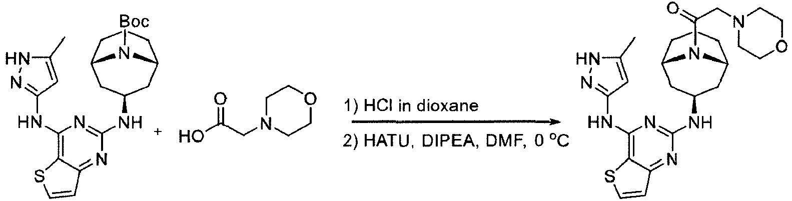

Tert-butyl-(3-*exo*)-3-((4-((5-methyl-1H-pyrazol-3-yl)amino)thieno[3,2-d]pyrimidin-2-yl)amino)-9-azabicyclo[3.3.1]non-9-carboxylate (100 mg, 0.21 mmol) was dissolved in hydrochloride 1,4-dioxane solution(4.0 N, 5 mL). The mixture was stirred at room temperature for 30 minutes, concentrated, and then 2-(7-oxide benzotriazole)-N,N,N',N'-tetramethylurea hexafluorophosphate (120 mg, 0.31 mmol) was added to the mixture. The mixture was dissolved in N,N-dimethylformamide (5 mL). DIPEA (108 mg, 0.84 mmol) was slowly added dropwise into the mixture, and the mixture was then stirred under ice water bath for 10 minutes. 2-Morpholino acetic acid (33 mg, 0.23 mmol) was added thereto and the mixture was stirred for another 1 hour. The reaction mixture was concentrated under reduced pressure, and the resulting product was purified by prep-HPLC to obtain the title compound as a white solid (17.8 mg, 17 %).

¹H NMR (400 MHz, DMSO-*d₆*) *δ* = 11.99 (s, 1H), 9.69 (s, 1H), 7.84 (s, 1H), 7.07-6.23 (m, 3H), 4.77 (s, 1H), 4.58 (s, 1H), 4.30 (s, 1H), 3.52 (d, *J* = 4.0 Hz, 4H), 3.10-3.01 (m, 2H), 2.32 (s, 3H), 2.14 (s, 2H), 2.09-1.39 (m, 10H).

MS *m*/*z* (ESI): 497.1 [M+H]⁺.

### Embodiment 289

### 1-((3-exo)-3-((4-((5-methyl-1H-pyrazol-3-yl)amino)thieno[3,2-d]pyrimidin-2-yl)amino)-9-azabicyclo[3.3.1]non-9-yl)-2-(methylamino)ethane-1-one

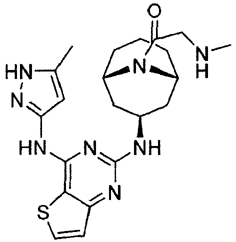

The preparation of 1-((3-*exo*)-3-((4-((5-methyl-1H-pyrazol-3-yl)amino)thieno[3,2-d]pyrimidin-2-yl)amino)-9-azabicyclo[3.3.1]non-9-yl)-2-(methylamino)ethane-1-one was carried out with the reference to Embodiment 247.

MS *m*/*z* (ESI): 441.2 [M+H]⁺.

### Embodiment 290

### ((3-exo)-3-((4-((5-methyl-1H-pyrazol-3-yl)amino)thieno[3,2-d]pyrimidin-2-yl)amino)-9-azabicyclo[3.3.1]non-9-yl)(pyridin-2-yl)methanone

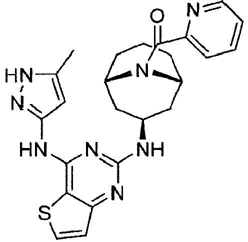

The preparation of ((3-*exo*)-3-((4-((5-methyl-1H-pyrazol-3-yl)amino)thieno[3,2-d]pyrimidin-2-yl)amino)-9-azabicyclo[3.3.1]non-9-yl)(pyridin-2-yl)methanone was carried out with the reference to Embodiment 288.

MS *m*/*z* (ESI): 475.2 [M+H]⁺.

### Embodiment 291

### (1-methyl-1H-imidazol-2-yl)((3-exo)-3-((4-((5-methyl-1H-pyrazol-3-yl)amino)thieno[3,2-d]pyrimidin-2-yl)amino)-9-azabicyclo[3.3.1]non-9-yl)methanone

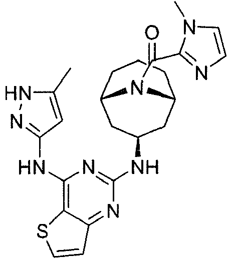

The preparation of (1-methyl-1H-imidazol-2-yl)((3-*exo*)-3-((4-((5-methyl-1H-pyrazol-3-yl)amino)thieno[3,2-d]pyrimidin-2-yl)amino)-9-azabicyclo[3.3.1]non-9-yl)methanone was carried out with the reference to Embodiment 288.

MS *m*/*z* (ESI): 478.2 [M+H]⁺.

### Embodiment 292

### 2-(dimethylamine)-1-((1R,3r,5S)-3-((4-((5-methyl-1H-pyrazol-3-yl)amino)thieno[3,2-d]pyrimidin-2-yl)amino)-8-azabicyclo[3.2.1]oct-8-yl)ethane-1-one

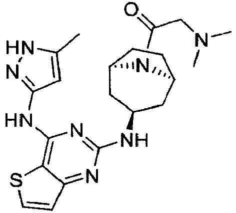

The preparation of 2-(dimethylamine)-1-((1R,3r,5S)-3-((4-((5-methyl-1H-pyrazol-3-yl)amino)thieno[3,2-d]pyrimidin-2-yl)amino)-8-azabicyclo[3.2.1]oct-8-yl)ethane-1-one was carried out with the reference to Embodiment 242.

MS *m*/*z* (ESI): 441.2 [M+H]⁺.

### Embodiment 293

### N,N-dimethyl-2-((3-exo)-3-((4-((5-methyl-1H-pyrazol-3-yl)amino)thieno[3,2-d]pyrimidin-2-yl)amino)-8-azabicyclo[3.2.1]oct-8-yl)acetamide

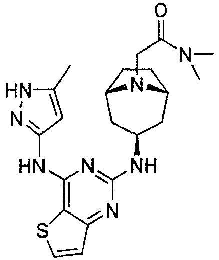

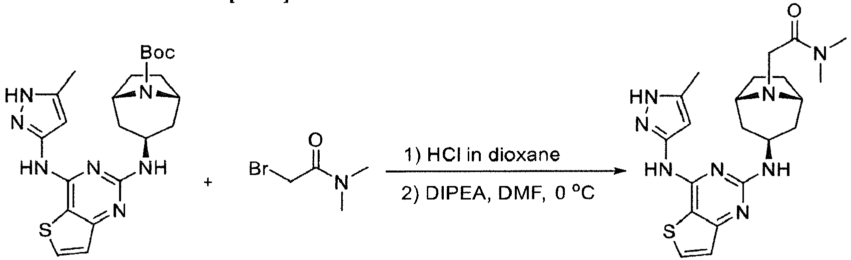

Tert-butyl-(3-*exo*)-3-((4-((5-methyl-1H-pyrazol-3-yl)amino)thieno[3,2-d]pyrimidin-2-yl)amino)-8-azabicyclo[3.2.1]oct-8-carboxylate (100 mg, 0.21 mmol) was dissolved in hydrochloride 1,4-dioxane solution(4.0 N, 5 mL). The mixture was stirred at room temperature for 30 minutes, concentrated, and then N,N-dimethylformamide (5 mL) was added to the mixture for dissolution. DIPEA (108 mg, 0.84 mmol) was slowly added dropwise into the mixture, and the mixture was then stirred under ice water bath for 10 minutes. 2-Bromine-N,N-dimethylacetamide(38 mg, 0.23 mmol) were added thereto and the mixture was stirred for another 1 hour. The reaction mixture was concentrated under reduced pressure, and the resulting product was purified by prep-HPLC to obtain the title compound as a white solid (16.4 mg, 17 %).

¹H NMR (400 MHz, DMSO-*d₆*) *δ* = 12.05 (s, 1H), 9.74 (s, 1H), 7.90 (d, *J* = 4.0 Hz, 1H), 7.14-6.30 (m, 3H), 4.15 (s, 1H), 3.32-3.23 (m, 4H), 3.08 (s, 3H), 2.83 (s, 3H), 2.22 (s, 3H), 1.97 (s, 2H), 1.82-1.55 (m, 6H).

MS *m*/*z* (ESI): 441.1 [M+H]⁺.

### Embodiment 294

### 3-((3-exo)-3-((4-((5-(hydroxymethyl)-1H-pyrazol-3-yl)amino)-6-methylthieno[3,2-d pyrimidin-2-yl)amino)-9-azabicyclo[3.3.1]non-9-yl)propionitrile

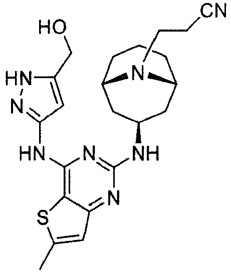

### Step 1: preparation of (3-((2-chloro-6-methylthieno[3,2-d]pyrimidin-4-yl)amino)-1H-pyrazol-5-yl)methanol

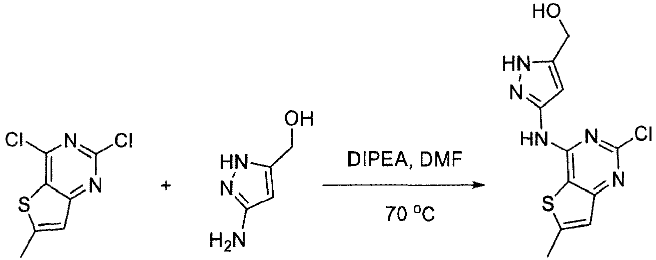

2,4-Dichloro-6-methylthieno[3,2-d]pyrimidine (200 mg, 0.91 mmol), (3-amino-1H-pyrazol-5-yl)methanol (120 mg, 1.09 mmol) and DIPEA (350 mg, 2.73 mmol) was dissolved in N,N-dimethylformamide (10 mL). The mixture was mixed evenly and the reaction was carried out at 70 °C overnight. Then the mixture was cooled to room temperature, and successively extracted with water (30 mL) and ethyl acetate (20 mL *3). The organic phases were combined, and concentrated under reduced pressure. The crude product obtained was purified by flash silica gel chromatography to obtain the title compound as a white solid (200 mg, 75 %).

MS *m*/*z* (ESI): 296.0 [M+H]⁺.

### Step 2: preparation of Tert-butyl (3-exo)-3-((4-((5-(hydroxymethyl)-1H-pyrazol-3-yl)amino)-6-methylthieno[3,2-d]pyrimidin-2-yl)amino)-9-azabicyclo[3.3.1]non-9-carboxylate

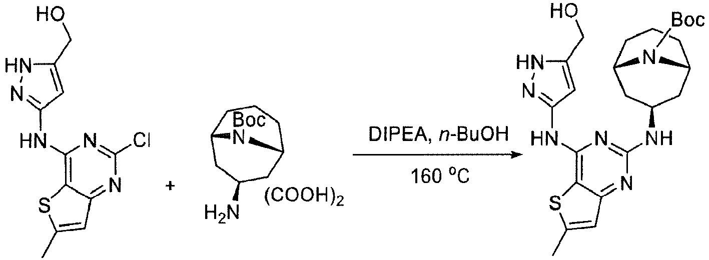

(3-((2-Chloro-6-methylthieno[3,2-d]pyrimidin-4-yl)amino)-1H-pyrazol-5-yl)methanol (150 mg, 0.51 mmol), Tert-butyl (3-exo)-3-amino-9-azabicyclo[3.3.1]non-9-carboxylate oxalate(200 mg, 0.61 mmol) and DIPEA (200 mg, 1.53 mmol) were added to n-butanol (3 mL). The mixture was mixed evenly, and the reaction was carried out at 165 °C for 8 hour. The mixture was then cooled to room temperature, and concentrated under reduced pressure. The crude product obtained (200 mg) was used directly in the next step without purification.

MS *m*/*z* (ESI): 500.1 [M+H]⁺.

### Step 3: preparation of (3-((2-(((3-exo)-9-azabicyclo[3.3.1]non-3-yl)amino)-6-methylthieno [3,2-d]pyrimidin-4-yl)amino)-1H-pyrazol-5-yl)methanol

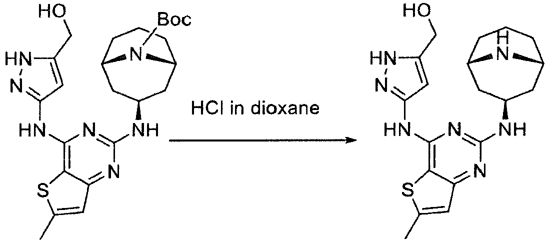

Hydrochloride dioxane(4N, 5 mL) was slowly added dropwise to the solution of Tert-butyl (3-exo)-3-((4-((5-(hydroxymethyl)-1H-pyrazol-3-yl)amino)-6-methylthieno[3,2-d]pyrimidin-2-yl)amino)-9-azabicyclo[3.3.1]non-9-carboxylate (200 mg, 0.40 mmol) in methanol (10 mL). The reaction was carried out at room temperature for 3 hours, and then the mixture was concentrated under reduced pressure. The crude product obtained was purified by prep-HPLC to obtain the title compound as a yellow solid (100 mg, 63 %).

MS *m*/*z* (ESI): 400.1 [M+H]⁺.

### Step 4: 3-((3-exo)-3-((4-((5-(hydroxymethyl)-1H-pyrazol-3-yl)amino)-6-methylthieno[3,2-d pyrimidin-2-yl)amino)-9-azabicyclo[3.3.1]non-9-yl)propionitrile

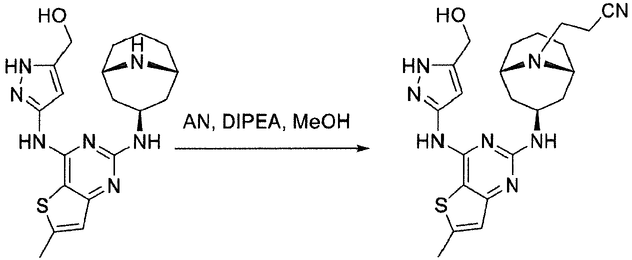

(3-((2-(((3-exo)-9-Azabicyclo[3.3.1]non-3-yl)amino)-6-methylthieno[3,2-d]pyrimidin-4-yl)amino)-1H-pyrazol-5-yl)methanol (100 mg, 0.25 mmol), acrylonitrile (0.2 mL) and DIPEA (0.1 mL) were added to methanol (10 mL). The mixture was mixed evenly, and the reaction was carried out at room temperature for 1 hour. The mixture was then concentrated under reduced pressure, and the crude product obtained was purified by prep-HPLC to obtain the title compound as a white solid (11.7 mg, 10 %).

¹H NMR (400 MHz, DMSO-*d₆*) *δ* 12.28 (s, 1H), 10.12 (s, 1H), 7.09-6.64 (m, 2H), 6.29-6.23 (s, 1H), 5.22-4.94 (m, 1H), 4.67-4.37 (m, 3H), 2.95 (s, 2H), 2.85-2.81 (m, 2H), 2.70-2.57 (m, 5H), 1.95-1.49 (m, 10H).

MS *m*/*z* (ESI): 453.2 [M+H]⁺.

### Embodiment 295

### 3-((3-exo)-3-((1-methyl-4-((5-methyl-1H-pyrazol-3-yl)amino)-1H-pyrazolo[3,4-d]pyrimidin-6-yl)amino)-8-azabicyclo[3.2.1]oct-8-yl)propionitrile

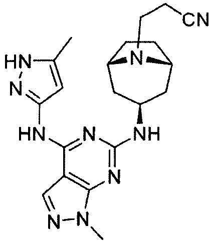

### Step 1: preparation of 6-chloro-1-methyl-N-(5-methyl-1H-pyrazol-3-yl)-1H-pyrazolo[3,4-d]pyrimidin-4-amine

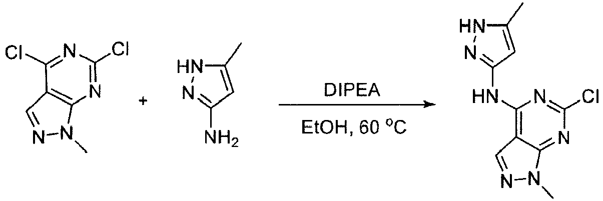

3-Amino-5-methylpyrazol (117 mg, 1.2 mmol) and DIPEA (258 mg, 2 mmol) were successively added to the solution of 4,6-dichloro-1-methyl-1H-pyrazolo[3,4-d]pyrimidine (203 mg, 1 mmol) in ethanol (10 mL). The mixture was then stirred at 60°C for 1 hour. When the reaction was completed, the reaction mixture was extracted with ethyl acetate (15 mL x 3), and washed with saturated sodium chloride aqueous solution (15 mL x 3). The organic phase was collected, dried over anhydrous sodium sulfate, filtered and concentrated under reduced pressure. The resulting product was purified by silica gel column chromatography (dichloromethane: methanol=98:2) to obtain the title compound as a yellow solid (250 mg, 95%).

MS *m*/*z* (ESI): 264.0 [M+H]⁺.

### Step 2: preparation of tert-butyl-(3-exo)-3-((1-methyl-4-((5-methyl-1H-pyrazol-3-yl)amino)-1H-pyrazolo[3,4-d]pyrimidin-6-yl)amino)-8-azabicyclo[3.2.1]oct-8-carboxylate

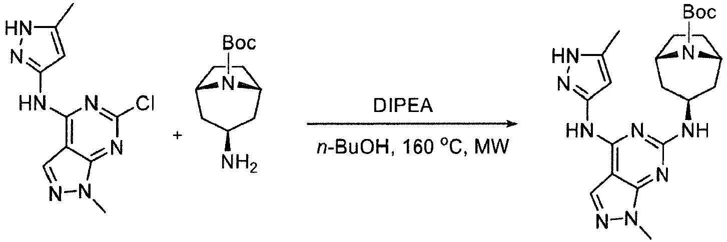

Tert-butyl-(3-*exo*)-3-amino-8-azabicyclo[3.2.1]oct-8-carboxylate (170 mg, 0.75 mmol) and DIPEA (129 mg, 1 mmol) were successively added to the solution of 6-chloro-1-methyl-N-(5-methyl-1H-pyrazol-3-yl)-1H-pyrazolo[3,4-d]pyrimidin-4-amine (132 mg, 0.5 mmol) in n-butanol (5 mL), then the mixture was stirred at 160°Cunder microwave condition for 15 hours. When the reaction was completed, the reaction mixture was extracted with ethyl acetate (15 mL x 3), and washed with saturated sodium chloride aqueous solution (15 mL x 3). The organic phase was collected, dried over anhydrous sodium sulfate, filtered and concentrated under reduced pressure. The resulting product was purified by silica gel column chromatography (dichloromethane: methanol=95:5) to obtain the title compound as a white solid (165 mg, 73%).

MS *m*/*z* (ESI): 454.2 [M+H]⁺.

### Step 3: preparation of 3-((3-exo)-3-((1-methyl-4-((5-methyl-1H-pyrazol-3-yl)amino)-1H-pyrazolo[3,4-d]pyrimidin-6-yl)amino)-8-azabicyclo[3.2.1]oct-8-yl)propionitrile

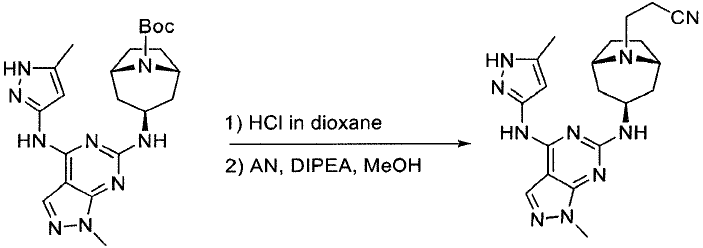

Tert-butyl-(3-exo)-3-((1-methyl-4-((5-methyl-1H-pyrazol-3-yl)amino)-1H-pyrazolo[3,4-d]pyrimidin-6-yl)amino)-8-azabicyclo[3.2.1]oct-8-carboxylate (165 mg, 0.36 mmol) was dissolved in hydrochloride 1,4-dioxane solution(4.0 N, 5 mL). The mixture was stirred at room temperature for 30 minutes, concentrated, and then methanol (10 mL) was added to the mixture for dissolution. DIPEA (186 mg, 1.44 mmol) was slowly added dropwise into the mixture, and the mixture was stirred at room temperature for 10 minutes. Acrylonitrile (29 mg, 0.55 mmol) was added thereto and the mixture was stirred for another 2 hours. When the reaction was completed, the reaction mixture was concentrated under reduced pressure, then purified by prep-HPLC to obtain the title compound as a white solid (60.4 mg, 41%).

¹H NMR (400 MHz, DMSO-*d₆*) *δ* = 11.96 (s, 1H), 10.04 (s, 1H), 7.99 (s, 1H), 6.64 (s, 2H), 4.18 (s, 1H), 3.63 (d, *J* = 20.4 Hz, 3H), 3.22 (s, 2H), 2.55 (s, 4H), 2.17 (s, 3H), 1.84 (s, 2H), 1.73-1.48 (m, 6H).

MS *m*/*z* (ESI): 407.2 [M+H]⁺.

### Embodiment 296

### 1-((3-exo)-3-((1-methyl-4-((5-methyl-1H-pyrazol-3-yl)amino)-1H-pyrazolo[3,4-d]pyrimidin-6-yl)amino)-9-azabicyclo[3.3.1]non-9-yl)-2-morpholinoethane-1-one

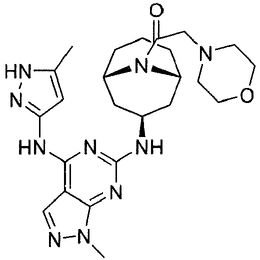

The preparation of 1-((3-*exo*)-3-((1-methyl-4-((5-methyl-1H-pyrazol-3-yl)amino)-1H-pyrazolo[3,4-d]pyrimidin-6-yl)amino)-9-azabicyclo[3.3.1]non-9-yl)-2-morpholinoethane-1-one was carried out with the reference to Embodiment 246.

MS *m*/*z* (ESI): 495.2 [M+H]⁺.

### Embodiment 297

### ((3-exo)-3-((1-methyl-4-((5-methyl-1H-pyrazol-3-yl)amino)-1H-pyrazolo[3,4-d]pyrimidin-6-yl)amino)-9-azabicyclo[3.3.1]non-9-yl)(pyridin-2-yl)methanone

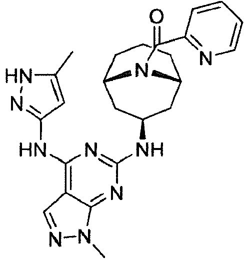

The preparation of ((3-*exo*)-3-((1-methyl-4-((5-methyl-1H-pyrazol-3-yl)amino)-1H-pyrazolo[3,4-d]pyrimidin-6-yl)amino)-9-azabicyclo[3.3.1]non-9-yl)(pyridin-2-yl)methanone was carried out with the reference to Embodiment 260.

MS *m*/*z* (ESI): 473.2 [M+H]⁺.

### Embodiment 298

### 3-(cis-5-((4-((5-methyl-1H-pyrazol-3-yl)amino)thieno[2,3-d]pyrimidin-2-yl)amino)hexahydrocyclopentadieno[c]pyrole-2(1H)-yl)propionitrile

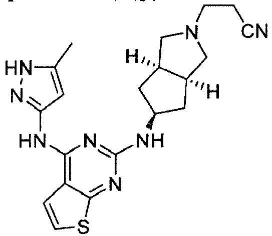

### Step 1: preparation of tert-butyl cis-5-((4-((5-methyl-1H-pyrazol-3-yl)amino)thieno[2,3-d]pyrimidin-2-yl)amino)hexahydrocyclopentadieno[c]pyrrole-2(1H)-carboxylate

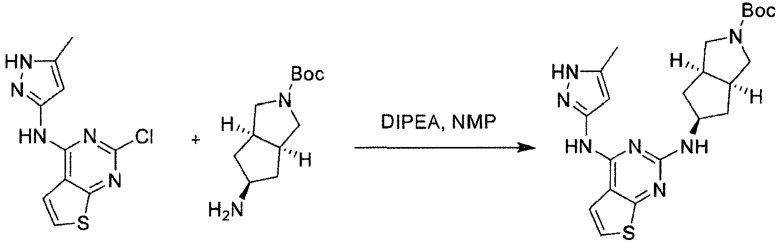

2-Chloro-N-(5-methyl-1H-pyrazol-3-yl)thieno[2,3-d]pyrimidin-4-amine(100 mg, 0.376 mmol), tert-butyl *cis*-5-aminohexahydrocyclopentadieno[c]pyrrole-2(1H)-carboxylate (102 mg, 0.452 mmol) and DIPEA (146 mg, 1.13 mmol) were successively added to NMP(1 mL). Under nitrogen atmosphere, the mixture was heated to 160 °C by microwave and reacted for 8 hours. The reaction mixture was cooled to room temperature, then poured into ice water (10 mL), stirred for 10 minutes, and filtered. The filter cake was washed with water (15 mL) and dried under vacuum to obtain the title compound as a yellow solid (171 mg, the crude product).

### Step 2: preparation of 3-(cis-5-((4-((5-methyl-1H-pyrazol-3-yl)amino)thieno[2,3-d]pyrimidin-2-yl)amino)hexahydrocyclopentadieno[c]pyrrole-2(1H)-yl)propionitrile

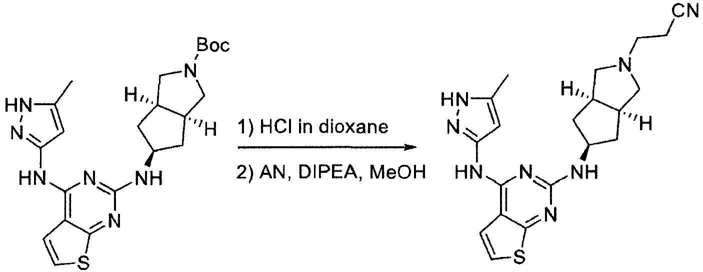

Tert-butyl*cis*-5-((4-((5-methyl-1H-pyrazol-3-yl)amino)thieno[2,3-d]pyrimidin-2-yl)amino)hexahydrocyclopentadieno[c]pyrrole-2(1H)-carboxylate (86 mg, 0.188 mmol) was dissolved in methanol (2 mL). The mixture was stirred at room temperature and 4M HCl in 1,4-dioxane (2 mL) was added thereto. The reaction mixture was stirred at room temperature for 1 hour, and concentrated under reduced pressure. The residue was dissolved in anhydrous methanol (2 mL), and DIPEA(121 mg, 0.938 mmol) and acrylonitrile (15 mg, 0.282 mmol) were successively added thereto. The resulting reaction mixture was stirred at room temperature for 16 hours. The reaction mixture was diluted with DCM (20 mL), and washed with water (5mL). The organic phase was dried over anhydrous sodium sulfate, filtered, and concentrated under reduced pressure. The residue was purified by silica gel chromatography to obtain the title compound as a white solid (17 mg, 22%).

¹H NMR (400 MHz, CD₃OD) *δ* 7.36 (d, *J* = 6.0 Hz, 1H), 7.02-6.86 (m, 1H), 6.54 (s, 0.6H), 5.80 (s, 0.4H), 4.34-4.09 (m, 1H), 2.86-2.72 (m, 4H), 2.73-2.57 (m, 4H), 2.40-2.19 (m, 7H), 1.57-1.37 (m, 2H).

MS *m*/*z* (ESI): 409.1 [M+H]⁺.

### Embodiment 299

### 3-(cis-5-((4-((5-methyl-1H-pyrazol-3-yl)amino)thieno[3,2-d]pyrimidin-2-yl)amino)hexahydrocyclopentadieno[c]pyrrole-2(1H)-yl)propionitrile

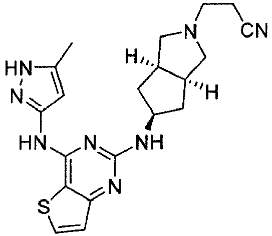

### Step 1: preparation of tert-butyl-cis-5-((4-((5-methyl-1H-pyrazol-3-yl)amino)thieno[3,2-d]pyrimidin-2-yl)amino)hexahydrocyclopentadieno[c]pyrrole-2(1H)-carboxylate

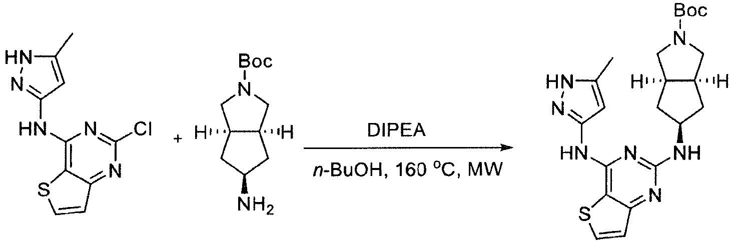

Tert-butyl-*cis*-5-aminohexahydrocyclopentadieno[c]pyrrole-2(1H)-carboxylate (102 mg, 0.45 mmol) and DIPEA (98 mg, 0.76 mmol) were successively added to the solution of 2-chloro-N-(5-methyl-1H-pyrazol-3-yl)thieno[3,2-d]pyrimidin-4-amine(100 mg, 0.38 mmol) in n-butanol (5 mL). The mixture was then stirred at 160°C under microwave condition for 15 hours. When the reaction was completed, the reaction mixture was extracted with ethyl acetate (15 mL x 3), and washed with saturated sodium chloride aqueous solution (15 mL x 3). The organic phase was collected, dried over anhydrous sodium sulfate, filtered and concentrated under reduced pressure. The resulting product was purified by silica gel column chromatography (dichloromethane: methanol=95:5) to obtain the title compound as a pale yellow solid (80 mg, 46 %).

MS *m*/*z* (ESI): 456.2 [M+H]⁺.

### Step 2: preparation of 3-(cis-5-((4-((5-methyl-1H-pyrazol-3-yl)amino)thieno[3,2-d]pyrimidin-2-yl)amino)hexahydrocyclopentadieno[c]pyrrole-2(1H)-yl)propionitrile

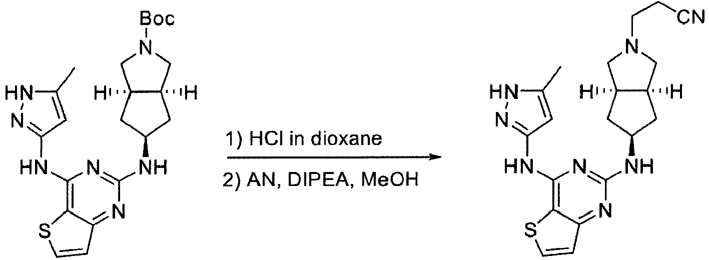

Tert-butyl-*cis*-5-((4-((5-methyl-1H-pyrazol-3-yl)amino)thieno[3,2-d]pyrimidin-2-yl)amino)hexahydrocyclopentadieno[c]pyrrole-2(1H)-carboxylate (80 mg, 0.18 mmol) was dissolved in hydrochloride 1,4-dioxane solution (4.0 N, 2 mL). The mixture was stirred at room temperature for 30 minutes, concentrated, and then methanol (5 mL) was added to the mixture for dissolution. DIPEA (93 mg, 0.72 mmol) was slowly added dropwise into the mixture, and the mixture was stirred at room temperature for 10 minutes. Acrylonitrile (14 mg, 0.27 mmol) was added thereto and the mixture was stirred for another 2 hours. The reaction mixture was concentrated under reduced pressure, and the resulting product was purified by prep-HPLC to obtain the title compound as a white solid (26.3 mg, 37 %).

¹H NMR (400 MHz, DMSO-*d₆*) *δ* = 12.30 (s, 1H), 9.92 (s, 1H), 7.90 (s, 1H), 7.51-6.25 (m, 3H), 4.11 (s, 1H), 2.66 (dd, *J* = 13.6, 7.2 Hz, 6H), 2.22 (s, 8H), 1.31 (s, 3H).

MS *m*/*z* (ESI): 409.1 [M+H]⁺.

### Embodiment 300

### 3-(cis-5-((4-((5-methyl-1H-pyrazol-3-yl)amino)quinazolin-2-yl)amino)hexahydrocyclopentadieno[c]pyrrole-2(1H)-yl)propionitrile

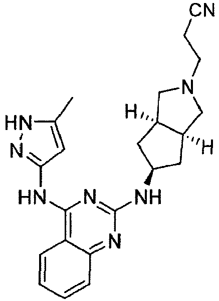

The preparation of 3-(*cis*-5-((4-((5-methyl-1H-pyrazol-3-yl)amino)quinazolin-2-yl)amino)hexahydrocyclopentadieno[c]pyrrole-2(1H)-yl)propionitrile was carried out with the reference to Embodiment 281.

¹H NMR (400 MHz, DMSO-*d₆*) *δ* 12.14 (s, 1H), 10.16 (s, 1H), 8.29 (s, 1H), 7.54 (t, *J=* 7.5 Hz, 1H), 7.32 (d, *J* = 8.2 Hz, 2H), 7.09 (t, *J* = 7.2 Hz, 1H), 6.63 (s, 1H), 4.26-4.09 (m, 1H), 2.63 (dd, *J* = 25.8, 13.5 Hz, 8H), 2.42-1.98 (m, 7H), 1.34 (dd, *J* = 15.0, 9.5 Hz, 2H).

MS *m*/*z* (ESI): 403.2 [M+H]⁺.

### Embodiment 301

### 3-(4-((4-((5-methyl-1H-pyrazol-3-yl)amino)thieno[2,3-d]pyrimidin-2-yl)amino)piperidin-1-yl)propionitrile

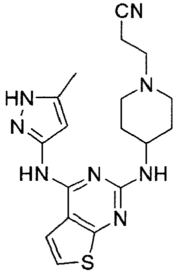

### Step 1: preparation of tert-butyl4-((4-((5-methyl-1H-pyrazol-3-yl)amino)thieno[2,3-d]pyrimidin-2-yl)amino)piperidin-1-carboxylate

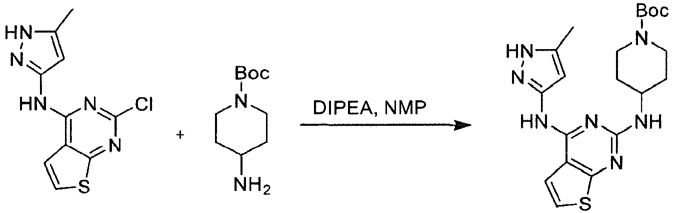

2-Chloro-N-(5-methyl-1H-pyrazol-3-yl)thieno[2,3-d]pyrimidin-4-amine(100 mg, 0.376 mmol), 1-BOC-4-aminopiperidin(108 mg, 0.539 mmol) and DIPEA (146 mg, 1.13 mmol) were successively added to NMP(1 mL). Under nitrogen atmosphere, the mixture was then heated to 130 °C by microwave, and reacted for 16 hours. The reaction mixture was cooled to room temperature, then poured into ice water (10 mL), stirred for 10 minutes, and filtered. The filter cake was washed with water (5 mL), dried under vacuum to obtain the title compound as a yellow solid (100 mg, the crude product).

### Step 2: preparation of 3-(4-((4-((5-methyl-1H-pyrazol-3-yl)amino)thieno[2,3-d]pyrimidin-2-yl)amino)piperidin-1-yl)propionitrile

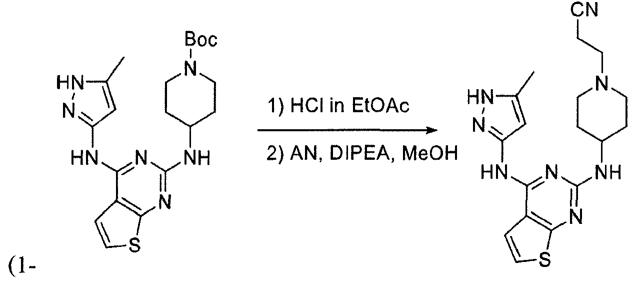

Tert-butyl 4-((4-((5-methyl-1H-pyrazol-3-yl)amino)thieno[2,3-d]pyrimidin-2-yl)amino)piperidin-1-carboxylate (100 mg, 0.233 mmol) was dissolved in methanol (2 mL). The mixture was stirred at room temperature for 4M HCl in ethyl acetate (2 mL) was added thereto. The mixture was stirred at room temperature for 2 hours, and concentrated under reduced pressure. The residue was dissolved in anhydrous methanol (2 mL), and DIPEA (150 mg, 1.17 mmol) and acrylonitrile (62 mg, 1.17 mmol) were successively added thereto. The resulting reaction mixture was stirred at room temperature for 1 hour. The reaction mixture was diluted with DCM(20 mL), and washed with water (5mL). The organic phase was dried over anhydrous sodium sulfate, filtered, and concentrated under reduced pressure. The residue was purified by silica gel chromatography to obtain the title compound as a white solid (18 mg, 20%).

¹H NMR (400 MHz, CD₃OD) *δ* 7.37 (d, *J* = 6.0 Hz, 1H), 6.97 (d, *J* = 6.1 Hz, 1H), 6.55 (s, 0.5H), 5.81 (s, 0.5H), 3.92-3.74 (m, 1H), 3.04-2.88 (m, 2H), 2.81-2.57 (m, 4H), 2.44-2.15 (m, 5H), 2.14-1.97 (m, 2H), 1.73-1.52 (m, 2H).

MS *m*/*z* (ESI): 383.1[M+H]⁺.

### Embodiment 302

### 1-((4-((4-((5-methyl-1H-pyrazol-3-yl)amino)thieno[2,3-d]pyrimidin-2-yl)amino)piperidin-1-yl)sulfonyl)azetindin-3-formonitrile

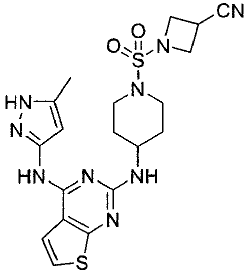

The preparation of 1-((4-((4-((5-methyl-1H-pyrazol-3-yl)amino)thieno[2,3-d]pyrimidin-2-yl)amino)piperidin-1-yl)sulfonyl)azetindin-3-formonitrile was carried out with the reference to Embodiment 237.

¹H NMR (400 MHz, CD₃OD) *δ* 7.38 (d, *J* = 6.0 Hz, 1H), 6.99 (d, *J* = 6.0 Hz, 1H), 6.49 (s, 0.5H), 5.83 (s, 0.5H), 4.20-4.10 (m, 2H), 4.07-3.99 (m, 2H), 3.99-3.89 (m, 1H), 3.77-3.61 (m, 3H), 3.09-2.99 (m, 2H), 2.28 (s, 3H), 2.17-2.06 (m, 2H), 1.67-1.51 (m, 2H).

MS *m*/*z* (ESI): 474.0[M+H]⁺.

### Embodiment 303

### 1-(4-((4-((5-methyl-1H-pyrazol-3-yl)amino)thieno[2,3-d]pyrimidin-2-yl)amino)piperidin-1-yl)-2-(methylamino)ethane-1-one

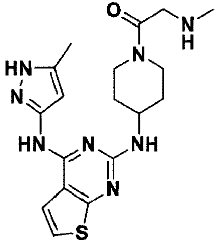

The preparation of 1-(4-((4-((5-methyl-1H-pyrazol-3-yl)amino)thieno[2,3-d]pyrimidin-2-yl)amino)piperidin-1-yl)-2-(methylamino)ethane-1-one was carried out with the reference to Embodiment 247.

MS *m*/*z* (ESI): 401.2 [M+H]⁺.

### Embodiment 304

### 1-(4-((4-((5-methyl-1H-pyrazol-3-yl)amino)thieno[2,3-d]pyrimidin-2-yl)amino)piperidin-1-yl)-2-morpholinoethane-1-one

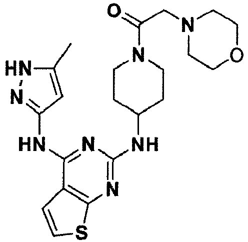

The preparation of 1-(4-((4-((5-methyl-1H-pyrazol-3-yl)amino)thieno[2,3-d]pyrimidin-2-yl)amino)piperidin-1-yl)-2-morpholinoethane-1-one was carried out with the reference to Embodiment 246.

MS *m*/*z* (ESI): 457.2 [M+H]⁺.

### Embodiment 305

### (4-((4-((5-methyl-1H-pyrazol-3-yl)amino)thieno[2,3-d]pyrimidin-2-yl)amino)piperidin-1-yl)(pyridin-2-yl)methanone

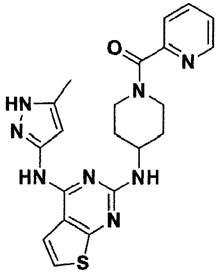

The preparation of (4-((4-((5-methyl-1H-pyrazol-3-yl)amino)thieno[2,3-d]pyrimidin-2-yl)amino)piperidin-1-yl)(pyridin-2-yl)methanone was carried out with the reference to Embodiment 260.

MS *m*/*z* (ESI): 435.2 [M+H]⁺.

### Embodiment 306

### 3-(4-((4-((5-methyl-1H-pyrazol-3-yl)amino)thieno[3,2-d]pyrimidin-2-yl)amino)piperidin-1-yl)propionitrile

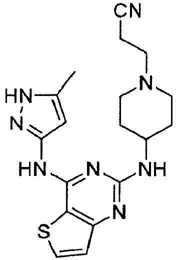

The preparation of 3-(4-((4-((5-methyl-1H-pyrazol-3-yl)amino)thieno[3,2-d]pyrimidin-2-yl)amino)piperidin-1-yl)propionitrile was carried out with the reference to Embodiment 60.

MS *m*/*z* (ESI): 383.2 [M+H]⁺.

### Embodiment 307

### 1-((4-((4-((5-methyl-1H-pyrazol-3-yl)amino)thieno[3,2-d]pyrimidin-2-yl)amino)piperidin-1-yl)sulfonyl)azetindin-3-formonitrile

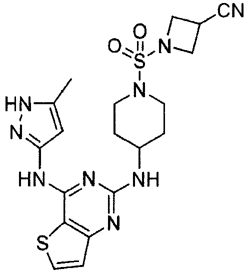

The preparation of 1-((4-((4-((5-methyl-1H-pyrazol-3-yl)amino)thieno[3,2-d]pyrimidin-2-yl)amino)piperidin-1-yl)sulfonyl)azetindin-3-formonitrile was carried out with the reference to Embodiment 60.

MS *m*/*z* (ESI): 474.1 [M+H]⁺.

### Embodiment 308

### 1-(4-((4-((5-methyl-1H-pyrazol-3-yl)amino)thieno[3,2-d]pyrimidin-2-yl)amino)piperidin-1-yl)-2-morpholinoethane-1-one

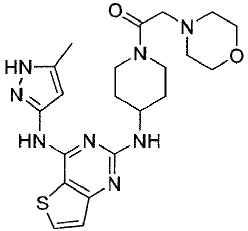

The preparation of 1-(4-((4-((5-methyl-1H-pyrazol-3-yl)amino)thieno[3,2-d]pyrimidin-2-yl)amino)piperidin-1-yl)-2-morpholinoethane-1-one was carried out with the reference to Embodiment 246.

MS *m*/*z* (ESI): 457.2 [M+H]⁺.

### Embodiment 309

### (4-((4-((5-methyl-1H-pyrazol-3-yl)amino)thieno[3,2-d]pyrimidin-2-yl)amino)piperidin-1-yl)(pyridin-2-yl)methanone

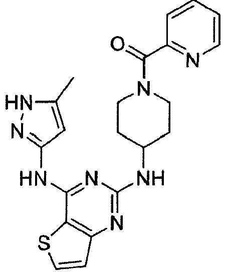

The preparation of (4-((4-((5-methyl-1H-pyrazol-3-yl)amino)thieno[3,2-d]pyrimidin-2-yl)amino)piperidin-1-yl)(pyridin-2-yl)methanone was carried out with the reference to Embodiment 260.

MS *m*/*z* (ESI): 435.2 [M+H]⁺.

### Embodiment 310

### 3-(4-((4-((5-methyl-1H-pyrazol-3-yl)amino)quinazolin-2-yl)amino)piperidin-1-yl)propionitrile

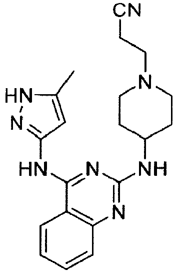

The preparation of 3-(4-((4-((5-methyl-1H-pyrazol-3-yl)amino)quinazolin-2-yl)amino)piperidin-1-yl)propionitrile was carried out with the reference to Embodiment 301.

¹H NMR (400 MHz, CD₃OD: CDCl₃, v/v= 1:1) *δ* 8.03 (d, *J* = 8.1 Hz, 1H), 7.59 (d, J= 7.9 Hz, 1H), 7.44 (s, 1H), 7.20 (t, *J* = 7.4 Hz, 1H), 6.63 (s, 1H), 5.92 (s, 1H), 4.01-3.87 (m, 1H), 2.98 (d, *J* = 11.6 Hz, 2H), 2.77 (t, *J* = 6.9 Hz, 2H), 2.64 (t, *J* = 6.9 Hz, 2H), 2.45-2.22 (m, 5H), 2.19-2.07 (m, 2H), 1.65 (td, *J* = 14.0, 3.4 Hz, 2H).

MS *m*/*z* (ESI): 377.1 [M+H]⁺.

### Embodiment 311

### 1-((4-((4-((5-methyl-1H-pyrazol-3-yl)amino)quinazolin-2-yl)amino)piperidin-1-yl)sulfonyl)azetindin-3-formonitrile

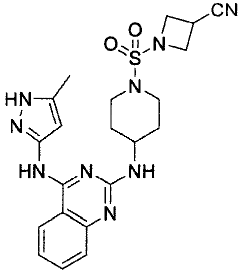

The preparation of 1-((4-((4-((5-methyl-1H-pyrazol-3-yl)amino)quinazolin-2-yl)amino)piperidin-1-yl)sulfonyl)azetindin-3-formonitrile was carried out with the reference to Embodiment 213.

¹H NMR (400 MHz, CD₃OD: CDCl₃, v/v= 1:1) *δ* 8.04 (d, *J* = 8.1 Hz, 1H), 7.65-7.59 (m, 1H), 7.45 (d, *J* = 8.2 Hz, 1H), 7.22 (t, *J* = 7.5 Hz, 1H), 6.31 (s, 1H), 4.17 (t, *J* = 8.3 Hz, 2H), 4.12-4.01 (m, 3H), 3.74 (d, *J* = 12.7 Hz, 2H), 3.61 (ddd, *J* = 15.1, 8.7, 6.4 Hz, 1H), 3.06 (t, *J*= 11.3 Hz, 2H), 2.32 (s, 3H), 2.21-2.11 (m, 2H), 1.64 (td, *J* = 13.6, 3.3 Hz, 2H).

MS *m*/*z* (ESI): 468.1 [M+H]⁺.

### Embodiment 312

### 3-(4-((7-((5-methyl-1H-pyrazol-3-yl)amino)-1,6-diazanaphthalen-5-yl)amino)piperidin-1-yl)propionitrile

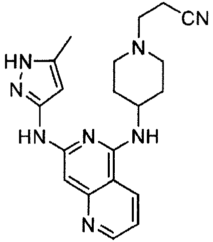

The preparation of 3-(4-((7-((5-methyl-1H-pyrazol-3-yl)amino)-1,6-diazanaphthalen-5-yl)amino)piperidin-1-yl)propionitrile was carried out with the reference to Embodiment 111.

MS *m*/*z* (ESI): 377.2 [M+H]⁺.

### Embodiment 313

### 1-((4-((7-((5-methyl-1H-pyrazol-3-yl)amino)-1,6-diazanaphthalen-5-yl)amino)piperidin-1-yl)sulfonyl)azetindin-3-formonitrile

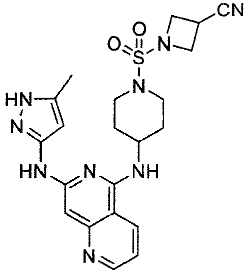

The preparation of 1-((4-((7-((5-methyl-1H-pyrazol-3-yl)amino)-1,6-diazanaphthalen-5-yl)amino)piperidin-1-yl)sulfonyl)azetindin-3-formonitrile was carried out with the reference to Embodiment 111.

MS *m*/*z* (ESI): 468.2 [M+H]⁺.

### Embodiment 314

### 3-(6-((7-((5-methyl-1H-pyrazol-3-yl)amino)-1,6-diazanaphthalen-5-yl)amino)-2-azaspiro[3.3]hept-2-yl)propionitrile

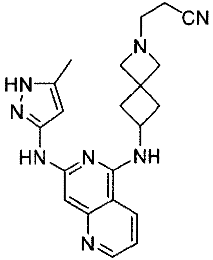

### Step 1: preparation of tert-butyl6-((7-chloro-1,6-diazanaphthalen-5-yl)amino)-2-azaspiro[3.3]hept-2-carboxylate

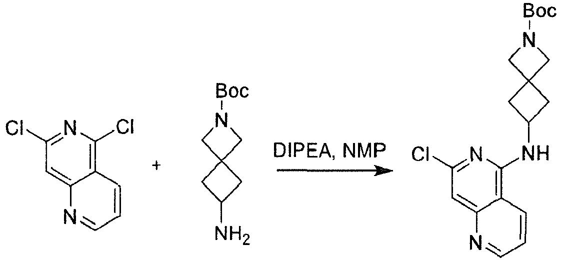

5,7-Dichloro-1,6-diazanaphthalen(200 mg, 1.00 mmol), tert-butyl6-amino-2-azaspiro[3.3]hept-2-formate(256 mg, 1.21 mmol) and DIPEA (324 mg, 2.51 mmol) were successively added to NMP (2 mL), and the mixture was stirred at 120 °C for 3 hours. The reaction mixture was cooled to room temperature, poured into ice water (20 mL), stirred for 10 minutes, and then extracted with ethyl acetate. The organic phases were combined and washed with saturated sodium chloride aqueous solution. The organic phase was collected, dried over anhydrous sodium sulfate, filtered, and concentrated under reduced pressure to remove the organic solvent. The resulting product obtained was purified by silica gel chromatography to obtain the title compound as brown oil (340 mg, 91%).

### Step 2: preparation of tert-butyl 6-((7-((1-(tert-butyl carbonyl)-5-methyl-1H-pyrazol-3-yl)amino)-1,6-diazanaphthalen-5-yl)amino)-2-azaspiro[3,3]hept-2-carboxylate

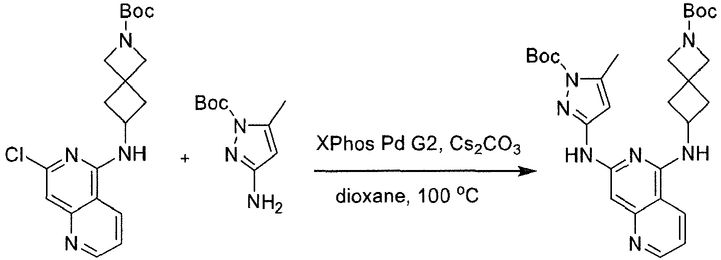

Tert-butyl 6-((7-chloro-1,6-diazanaphthalen-5-yl)amino)-2-azaspiro[3.3]hept-2-carboxylate (100 mg, 0.267 mmol), 1-Boc-3-amino-5-methylpyrazol (79 mg, 0.400 mmol), cesium carbonate (174 mg, 0.534 mmol), XPhos Pd G2 (105 mg, 0.134 mmol) and XPhos(127 mg, 0.267 mmol) were successively added to dioxane (5 mL). The mixture was filled with nitrogen for 3 times, and then stirred at 100 °C for 4 hours. The reaction mixture was diluted with ethyl acetate (20 mL), and filtered with diatomaceous earth. The filtrate was successively washed with water and saturated sodium chloride aqueous solution, dried over anhydrous sodium sulfate, filtered, and concentrated under reduced pressure. The resulting product obtained was purified by silica gel chromatography to obtain the title compound as a yellow oil (44 mg, the crude product).

### Step 3: preparation of 3-(6-((7-((5-methyl-1H-pyrazol-3-yl)amino)-1,6-diazanaphthalen-5-yl)amino)-2-azaspiro[3.3]hept-2-yl)propionitrile

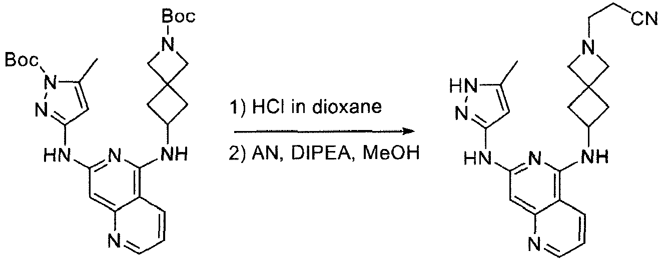

Tert-butyl 6-((7-((1-(tert-butyl carbonyl)-5-methyl-1H-pyrazol-3-yl)amino)-1,6-diazanaphthalen-5-yl)amino)-2-azaspiro[3.3]hept-2-carboxylate (66 mg, the crude product) was dissolved in methanol (2 mL). The mixture was stirred at room temperature and 4M HCl in 1,4-dioxane (2 mL) was added thereto. The mixture was stirred at room temperature for 2 hours, and concentrated under reduced pressure. The residue was dissolved in anhydrous methanol (1 mL), and DIPEA (48 mg, 0.370 mmol) and acrylonitrile (10 mg, 0.185 mmol) were successively added thereto. The resulting reaction mixture was stirred at room temperature for 1 hour. The reaction mixture was concentrated under reduced pressure, and the residue was successively purified by silica gel chromatography and prep-HPLC to obtain the title compound as a yellow solid (6 mg, 12%).

¹H NMR (400 MHz, CD₃OD) *δ* 8.55 (s, 1H), 8.38 (d, J= 8.2 Hz, 1H), 7.16-6.98 (m, 1H), 6.64 (s, 0.4H), 6.11 (s, 0.6H), 4.61-4.37 (m, 1H), 3.56-3.46 (m, 2H), 3.37-3.32 (m, 2H), 2.83-2.63 (m, 4H), 2.55-2.44 (m, 2H), 2.39-2.16 (m, 5H).

MS *m*/*z* (ESI): 389.2 [M+H]⁺.

### Embodiment 315

### N5-((3-exo)-8-((1H-pyrazol-4-yl)sulfonyl)-8-azabicyclo[3.2.1]oct-3-yl)-N7-(5-methyl-1H-pyrazol-3-yl)-1,6-diazanaphthalen-5,7-diamine

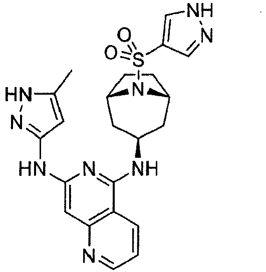

The preparation of N5-((3-*exo*)-8-((1H-pyrazol-4-yl)sulfonyl)-8-azabicyclo[3.2.1]oct-3-yl)-N7-(5-methyl-1H-pyrazol-3-yl)-1,6-diazanaphthalen-5,7-diamine was carried out with the reference to Embodiment 111.

¹H NMR (400 MHz, DMSO-*d₆*) *δ* = 9.03 (s, 1H), 8.78 (s, 1H), 8.59 (d, *J* = 2.8 Hz, 1H), 8.49 (s, 2H), 8.37 (d, *J* = 8.4 Hz, 1H), 8.32 (s, 1H), 7.21 (d, *J* = 8.0 Hz, 1H), 6.98 (dd, *J* = 8.4, 4.0 Hz, 1H), 6.69 (s, 1H), 6.05 (s, 1H), 4.62 (s, 1H), 4.35 (s, 2H), 2.16 (s, 3H), 2.10-2.02 (m, 2H), 1.78-1.73 (m, 4H), 1.41-1.32 (m, 2H).

MS *m*/*z* (ESI): 480.2 [M+H]⁺.

### Embodiment 316

### 3-(endo-6-((7-((5-methyl-1H-pyrazol-3-yl)amino)-1,6-diazanaphthalen-5-yl)amino)-3-azabicyclo[3.1.0]hex-3-yl)propionitrile

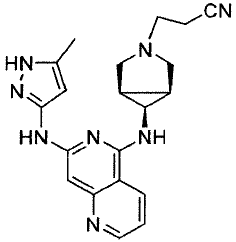

The preparation of 3-(*endo*-6-((7-((5-methyl-1H-pyrazol-3-yl)amino)-1,6-diazanaphthalen-5-yl)amino)-3-azabicyclo[3.1.0]hex-3-yl)propionitrile was carried out with the reference to Embodiment 111.

MS *m*/*z* (ESI): 375.2 [M+H]⁺.

### Embodiment 317

### 3-(endo-6-((4-((5-methyl-1H-pyrazol-3-yl)amino)thieno[2,3-d]pyrimidin-2-yl)amino)-3-azabicyclo[3.1.0]hex-3-yl)propionitrile

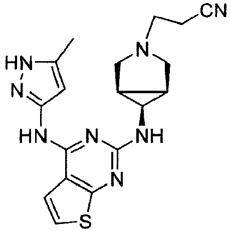

The preparation of 3-(*endo*-6-((4-((5-methyl-1H-pyrazol-3-yl)amino)thieno[2,3-d]pyrimidin-2-yl)amino)-3-azabicyclo[3.1.0]hex-3-yl)propionitrile was carried out with the reference to Embodiment 62.

MS *m*/*z* (ESI): 381.2 [M+H]⁺.

### Embodiment 318

### 3-(endo-6-((4-((5-methyl-1H-pyrazol-3-yl)amino)thieno[3,2-d]pyrimidin-2-yl)amino)-3-azabicyclo[3.1.0]hex-3-yl)propionitrile

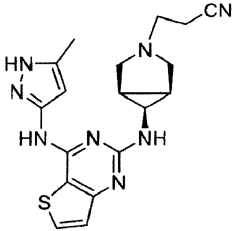

The preparation of 3-(*endo*-6-((4-((5-methyl-1H-pyrazol-3-yl)amino)thieno[3,2-d]pyrimidin-2-yl)amino)-3-azabicyclo[3.1.0]hex-3-yl)propionitrile was carried out with the reference to Embodiment 60.

MS *m*/*z* (ESI): 381.2 [M+H]⁺.

### Embodiment 319

### 3-(endo-6-((4-((5-methyl-1H-pyrazol-3-yl)amino)quinazolin-2-yl)amino)-3-azabicyclo[3.1.0]hex-3-yl)propionitrile

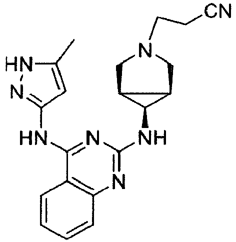

The preparation of 3-(*endo*-6-((4-((5-methyl-1H-pyrazol-3-yl)amino)quinazolin-2-yl)amino)-3-azabicyclo[3.1.0]hex-3-yl)propionitrile was carried out with the reference to Embodiment 4.

MS *m*/*z* (ESI): 375.2 [M+H]⁺.

### Biological test evaluation

The present disclosure is further described and explained in combination with the following test embodiments, but these embodiments are not meant to limit the scope of the present disclosure.

### Test Embodiment 1. Determination of the inhibitory effect of the compound of the present disclosure on JAK kinase activity

Experimental objective: The objective of this test is to test the inhibitory effect of the compound on JAK kinase activity.

Experimental instrument: centrifuge (5702R) purchased from Eppendorf, pipette purchased from Eppendorf or Rainin, microplate reader purchased from American BioTek, the model of which was SynergyH1 full-function microplate reader.

Experimental method: the fluorescence resonance energy transfer (TR-FRET) method was used to test the inhibitory effect of the compound on JAK kinase activity, and the half inhibitory concentration IC₅₀ of the compound on JAK kinase activity was obtained.

### The specific experimental procedures are as follows:

Kinase reaction was carried out in a white 384-well plate (PerkinElmer). 1-5µL of different concentrations of the compound diluted with DMSO and ddH₂O was added into each well, and 1-5µL of the corresponding solvent was added to the positive control wells, followed by addition of 0.1-20nM JAK kinase solution diluted with 1-5µL of kinase buffer (HEPES 50-250mM, MgCl₂ 5-20 mM, etc.) was added to each well. 1-5µL of kinase buffer was added to the negative control well. 1-5µL of substrate mixture containing peptide substrate and ATP was added. The plate was incubated at room temperature for 0.5-5 hours. 10µL EDTA and detection solution containing labeled antibody was added, and then the plate was incubated at room temperature for another 1-24 hours. The fluorescence signal value at about 620nm and 665nm of each well was measured by BioTek Synergy H1 plate reader, and the inhibition rate was calculated by the fluorescence signal value. According to the inhibition rate of different concentrations, the IC₅₀ of the compound was obtained by curve fitting.

### Experimental data processing method:

The percentage inhibition data of the wells treated with the compound was calculated with the positive control wells (DMSO control wells) and negative control wells (no kinase added) on the plate {% inhibition rate=100-[(test compound value-negative control value)] / (Positive control value-negative control value)×100}. IC₅₀ value was calculated by using GraphPad prism to fit the data of different concentrations and corresponding percentage inhibition rates to a 4-parameter non-linear logic formula.

### Experimental results:

According to the above scheme, the biological activities of the embodiment compounds of the present disclosure showed in the JAK1/2/3/TYK2 kinase activity test are as shown in Table 1.

**Table 1**

| Embodiment No. | JAK1, IC₅₀ (nM) | JAK2, IC₅₀ (nM) | JAK3, IC₅₀ (nM) | TYK2, IC₅₀ (nM) |
|---|---|---|---|---|
| **1** | 0.34 | 0.69 | 2.68 | 0.55 |
| **2** | 0.11 | 0.53 | 8.95 | 2.30 |
| **3** | 0.11 | 0.29 | 0.81 | 0.17 |
| **4** | 0.86 | 0.55 | 5.72 | 0.68 |
| **5** | 0.85 | 1.39 | 26.46 | 4.44 |
| **6** | 4.03 | NA | NA | 10.71 |
| **9** | 30.65 | NA | NA | >100 |
| **11** | 0.30 | NA | NA | 9.13 |
| **12** | 2.71 | NA | NA | 10.09 |
| **19** | 2.23 | NA | NA | 40.70 |
| **25** | 1.63 | NA | NA | 4.57 |
| **29** | 44.20 | >100 | >100 | 47.30 |
| **31** | 48.60 | NA | NA | >100 |
| **35** | 5.60 | NA | NA | 22.22 |
| **39** | 2.94 | NA | NA | 9.49 |
| **40** | 0.83 | 0.77 | 8.78 | 1.01 |
| **51** | 0.24 | NA | NA | 0.24 |
| **60** | 0.12 | NA | NA | 0.46 |
| **62** | 0.08 | NA | NA | 0.17 |
| **66** | 3.02 | NA | NA | 1.49 |
| **72** | 4.32 | NA | NA | 15.95 |
| **79** | 2.95 | NA | NA | 5.77 |
| **87** | 35.10 | NA | NA | >100 |
| **88** | 1.75 | NA | NA | 3.53 |
| **89** | 0.65 | NA | NA | 0.15 |
| **90** | 7.23 | NA | NA | 21.61 |
| **109** | 7.85 | 6.27 | 25.85 | 33.89 |
| **111** | 5.69 | NA | NA | 1.63 |
| **132** | 0.10 | 0.77 | 3.43 | 0.42 |
| **160** | 11.68 | NA | NA | 6.15 |
| **161** | 20.81 | NA | NA | 35.04 |
| **163** | 0.45 | NA | NA | 24.41 |
| **164** | 0.85 | NA | NA | 16.57 |
| **165** | 4.92 | NA | NA | >100 |
| **166** | 0.19 | NA | NA | 9.31 |
| **167** | 2.55 | NA | NA | 7.28 |
| **169** | 0.63 | 1.07 | 28.84 | 0.50 |
| **170** | 0.66 | NA | NA | 0.225 |
| **171** | 0.85 | 0.67 | 23.32 | 0.60 |
| **173** | 1.07 | 1.15 | 26.26 | 0.84 |
| **174** | 2.73 | NA | NA | 1.88 |
| **175** | >100 | NA | NA | 42.82 |
| **176** | 4.18 | NA | NA | 2.36 |
| **177** | 3.36 | NA | NA | 1.58 |
| **178** | 10.60 | NA | NA | >100 |
| **179** | 0.44 | NA | NA | 0.47 |
| **180** | 0.35 | NA | NA | 0.56 |
| **182** | 0.37 | NA | NA | 0.25 |
| **183** | 0.18 | NA | NA | 0.1 |
| **184** | 0.14 | NA | NA | 0.48 |
| **185** | 0.29 | NA | NA | 0.6 |
| **186** | 0.12 | 0.73 | 14.72 | 0.2 |
| **191** | 0.41 | NA | NA | 0.94 |
| **203** | 0.23 | NA | NA | 0.19 |
| **213** | 0.20 | 0.30 | 12.64 | 0.46 |
| **214** | 0.35 | 0.82 | 133.20 | 0.455 |
| **215** | 0.12 | NA | NA | 0.27 |
| **216** | 0.04 | NA | NA | 0.48 |
| **217** | 0.04 | 1.61 | 1.92 | 0.1 |
| **218** | 0.27 | 1.05 | 19.58 | 0.4 |
| **219** | 0.09 | 0.76 | 2.61 | 0.13 |
| **220** | 0.67 | NA | NA | 4.27 |
| **221** | 0.79 | NA | NA | 1.26 |
| **224** | 1.76 | NA | NA | >100 |
| **225** | 9.16 | NA | NA | 0.94 |
| **226** | 13.12 | NA | NA | 0.3 |
| **227** | 5.17 | NA | NA | 4.77 |
| **228** | 2.63 | NA | NA | 9.99 |
| **231** | 11.33 | NA | NA | 3.78 |
| **232** | 7.20 | NA | NA | 2.00 |
| **233** | 3.55 | NA | NA | 2.09 |
| **234** | 111.90 | NA | NA | 7.11 |
| **235** | 3.33 | NA | NA | 2.71 |
| **236** | 8.81 | NA | NA | 4.77 |
| **237** | 0.07 | 0.79 | 3.62 | 0.32 |
| **238** | 0.03 | 0.44 | 0.71 | 0.12 |
| **239** | 0.13 | 1.25 | 3.11 | 0.22 |
| **240** | 0.49 | NA | NA | 0.68 |
| **241** | 0.15 | 1.39 | 7.35 | 0.22 |
| **242** | 0.21 | 0.85 | 6.58 | 0.89 |
| **243** | 0.53 | 2.42 | 147.80 | 1.48 |
| **244** | 1.15 | NA | NA | 2.45 |
| **245** | 2.77 | NA | NA | 2.76 |
| **246** | 0.22 | 0.71 | 6.87 | 1.88 |
| **247** | 0.10 | 0.73 | 2.40 | 0.12 |
| **248** | 0.44 | NA | NA | 0.36 |
| **249** | 1.88 | NA | NA | 0.28 |
| **250** | 0.04 | NA | NA | 0.07 |
| **255** | 1.17 | NA | NA | 2.38 |
| **257** | 0.07 | 0.83 | 19.95 | 0.2 |
| **258** | 0.27 | NA | NA | 0.61 |
| **260** | 0.19 | 0.36 | 3.83 | 0.225 |
| **261** | 0.04 | NA | NA | 5.58 |
| **262** | 0.07 | NA | NA | 1.35 |
| **263** | 0.11 | NA | NA | 0.22 |
| **264** | 0.19 | NA | NA | 0.48 |
| **265** | 1.29 | 1.81 | 170.10 | 1.00 |
| **266** | 10.51 | NA | NA | 10.24 |
| **267** | 0.13 | NA | NA | 0.39 |
| **268** | 0.21 | NA | NA | 0.19 |
| **269** | 3.22 | NA | NA | 3.08 |
| **270** | 1.22 | NA | NA | 5.78 |
| **271** | 0.05 | NA | NA | 4.91 |
| **272** | 0.60 | NA | NA | 24.73 |
| **273** | 1.28 | 2.96 | >200 | 2.19 |
| **277** | 0.61 | 1.01 | 8.26 | 0.58 |
| **278** | 0.71 | 1.34 | 7.05 | 0.98 |
| **279** | 0.24 | NA | NA | 0.66 |
| **285** | 0.17 | NA | NA | 0.51 |
| **286** | 0.06 | NA | NA | 14.02 |
| **287** | 0.58 | NA | NA | 4.21 |
| **288** | 0.27 | NA | NA | 8.09 |
| **293** | 3.97 | NA | NA | 10.83 |
| **294** | 0.64 | NA | NA | 0.23 |
| **295** | 0.30 | NA | NA | 10.83 |
| **298** | 0.67 | NA | NA | 22.81 |
| **299** | 0.28 | NA | NA | 0.56 |
| **300** | 5.11 | NA | NA | 14.73 |
| **301** | 1.64 | NA | NA | 0.45 |
| **302** | 1.89 | NA | NA | 0.34 |
| **310** | 8.67 | NA | NA | 0.26 |
| **311** | 7.50 | NA | NA | 1.44 |
| **315** | 13.10 | NA | NA | >100 |

It can be seen from the above table that the compounds of the above embodiments can significantly inhibit the enzymatic activity of JAK1/2/3/TYK2 kinase, and some compounds have a strong inhibitory effect on JAK1/2/3/TYK2 kinase (NA refers to not being tested).

### Test Embodiment 2. Determination of the inhibitory effect of the compound of the present disclosure on the cellular JAK-STAT signal pathway

### Experimental objective:

The objective of the test is to test the inhibitory activity of the compound on the cellular JAK-STAT signaling pathway.

### Experimental instrument:

Microplate shaker (88880024) purchased from Thermo Scientific™
Centrifuge (5702R) purchased from Eppendorf
Pipette purchased from Eppendorf
Microplate reader purchased from BioTek, the model of which was SynergyHl full-function microplate reader.

### Experimental method:

U266 cell line was used in this experiment, by activating the JAK-STAT signaling pathway through INF-α stimulation, the inhibitory activity of the compound on its downstream STAT3 phosphorylation was detected, and the half inhibitory concentration IC₅₀ of the compound on the JAK-STAT signaling pathway activity was obtained.

### The specific experimental procedures are as follows:

3-12 µL of U266 cell line was spread into the 384-well detection plate, and the number of cells in each well was 100-300 K, followed by addition of 2 µL of the compound solution serially diluted. The plate was incubated for 2 hours with shaking at 350 rpm at room temperature. After 2 hours, 2 µL of INF-α with a final concentration of 1000 U/mL was added. The plate was shaken at room temperature for 15 minutes. 2-5 µL (5X) LANCE Ultra Lysis Buffer 2 solution was added and the plate was shaken at room temperature for 2 hours. After 2 hours, 5 µL of LANCE Ultra Eu-labeled Anti-STAT3 Antibody (PerkinElmer) with a final concentration of 0.5 nM and LANCE Ultra ULight-labeled Anti-STAT3 Antibody (PerkinElmer) with a final concentration of 5 nM were added, and the plate was incubated overnight at room temperature. The fluorescence signal value at 665 nm of each well was measured with the microplate reader, and the inhibition rate was calculated from the fluorescence signal value. According to the inhibition rate of different concentrations, the IC₅₀ of the compound was obtained by curve fitting.

### Experimental data processing method:

The percentage inhibition data of the wells treated with the compound was calculated with the positive control wells (DMSO control wells) and negative control wells (no kinase added) on the plate {% inhibition rate=100-[(test compound value-negative control value)] / (Positive control value-negative control value)×100}. IC₅₀ value was calculated by using GraphPad prism to fit the data of different concentrations and corresponding percentage inhibition rates to a 4-parameter non-linear logic formula.

### Experimental results:

According to the above scheme, the biological activities of the embodiment compounds of the present disclosure showed in JAK-STAT signal pathway of U266 cells are as shown in Table 2.

**Table 2**

| **Embodiment** | **U266 IC₅₀ (nM)** | **Embodiment** | **U266 IC₅₀ (nM)** | **Embodiment** | **U266 IC₅₀ (nM)** |
|---|---|---|---|---|---|
| Embodiment 1 | 0.15 | Embodiment 217 | 36.70 | Embodiment 264 | 33.47 |
| Embodiment 3 | 4.50 | Embodiment 218 | 61.50 | Embodiment 265 | 20.4 |
| Embodiment 4 | 2.60 | Embodiment 219 | 117.00 | Embodiment 267 | 18.00 |
| Embodiment 5 | 4.12 | Embodiment 220 | 10.17 | Embodiment 268 | 42.90 |
| Embodiment 11 | 4.77 | Embodiment 235 | 44.4 | Embodiment 270 | 14.30 |
| Embodiment 40 | 17.44 | Embodiment 237 | 0.56 | Embodiment 271 | 1.49 |
| Embodiment 60 | 2.90 | Embodiment 238 | 1.15 | Embodiment 272 | 15.30 |
| Embodiment 62 | 1.90 | Embodiment 239 | 39.00 | Embodiment 273 | 41.90 |
| Embodiment 66 | 4.06 | Embodiment 240 | 14.4 | Embodiment 277 | 0.49 |
| Embodiment 72 | 5.39 | Embodiment 241 | 115.00 | Embodiment 278 | 0.29 |
| Embodiment 88 | 3.10 | Embodiment 242 | 0.24 | Embodiment 279 | 2.20 |
| Embodiment 111 | 2.93 | Embodiment 243 | 0.24 | Embodiment 285 | 42.03 |
| Embodiment 132 | 23.8 | Embodiment 244 | 3.79 | Embodiment 287 | 10.50 |
| Embodiment 163 | 52.6 | Embodiment 245 | 16.7 | Embodiment 288 | 4.03 |
| Embodiment 166 | 26.7 | Embodiment 246 | 1.00 | Embodiment 293 | 16.50 |
| Embodiment 167 | 14.80 | Embodiment 247 | 2.27 | Embodiment 294 | 15.30 |
| Embodiment 169 | 9.32 | Embodiment 248 | 43.50 | Embodiment 295 | 3.63 |
| Embodiment 170 | 3.35 | Embodiment 250 | 7.30 | Embodiment 298 | 1.86 |
| Embodiment 171 | 0.85 | Embodiment 255 | 8.67 | Embodiment 299 | 2.46 |
| Embodiment 174 | 0.42 | Embodiment 257 | 1.45 | Embodiment 301 | 1.26 |
| Embodiment 179 | 9.84 | Embodiment 258 | 32.70 | Embodiment 310 | 5.41 |
| Embodiment 191 | 2.80 | Embodiment 260 | 1.10 | | |
| Embodiment 213 | 11.05 | Embodiment 261 | 10.64 | | |
| Embodiment 214 | 17.70 | Embodiment 262 | 9.47 | | |
| Embodiment 215 | 24.10 | Embodiment 263 | 4.07 | | |

It can be seen from the above table that the compounds of the above embodiments have a significant inhibitory effect on the JAK-STAT signal pathway activity of human myeloma cell U266.

### Test Embodiment 3. Determination of Balb/C mouse pharmacokinetics

### 1. Research purpose:

Balb/C mouse was used as the test animal, the pharmacokinetic behavior in mice (plasma and colon, ileum tissue) was studied after oral administering the embodiment 1, embodiment 60, embodiment 169, embodiment 170, embodiment 171, embodiment 179, embodiment 191, embodiment 213, embodiment 214, embodiment 237, embodiment 238, embodiment 244, embodiment 246, embodiment 247, embodiment 257, embodiment 260, embodiment 262, embodiment 263, embodiment 267, embodiment 277, embodiment 278, embodiment 279, embodiment 288, embodiment 295, embodiment 299 and embodiment 301 at a dose of 5 mg/kg. After analyzing the drug concentration in the colon and ileum, the ratio of the drug concentration in the colon to that in ileum, and the ratio of the drug concentration in the colon to that in the plasma, the compounds with excellent PK were selected for further research.

### 2. Test scheme

### 2.1 Test drugs:

Embodiment 1, embodiment 60, embodiment 169, embodiment 170, embodiment 171, embodiment 179, embodiment 191, embodiment 213, embodiment 214, embodiment 237, embodiment 238, embodiment 244, embodiment 246, embodiment 247, embodiment 257, embodiment 260, embodiment 262, embodiment 263, embodiment 267, embodiment 277, embodiment 278, embodiment 279, embodiment 288, embodiment 295, embodiment 299 and embodiment 301 prepared in the present disclosure.

### 2.2 Test animals:

12 Balb/C mice in each group, male, Shanghai Jiesjie Experimental Animal Co., Ltd., animal production license number (SCXK (Shanghai) 2013-0006 N0.311620400001794).

### 2.3 Administration:

12 Balb/C mice in each group, male; p.o. after one night fast; the dose was 5 mg/kg; the administration volume was 10 mL/kg.

### 2.4 Sample collection:

At 0, 0.5, 1, 2, 3, 5 and 7 hours before and after the administration, the mice were executed by CO₂ and 0.2 mL of blood was collected from the heart, placed in an EDTA-K₂ test tube, and centrifuged at 6000 rpm at 4 °C for 6 minutes to separate the plasma, which was then stored at -80°C. The ileum was taken close to the cecum end with a length of about 4-5cm, and the colon was also taken close to the cecum end with a length of about 2-3cm, weighed and placed in a 2 mL centrifuge tube, then stored at -80 °C.

### 2.5 Sample processing:

1) 40 uL of plasma sample was precipitated by adding 160 uL of acetonitrile, mixed and centrifuged at 3500 × g for 5-20 minutes.
2) 30 µL of plasma and intestinal homogenate sample were precipitated by adding 90 µL of acetonitrile containing internal standard (100ng/mL), mixed and centrifuged at 13000 rpm for 8 minutes.
3) 70 µL of the supernatant solution after treatment was collected and 70 µL of water was added thereto, vortexed and mixed for 10 minutes, and then 20µL was collected to analyze the concentration of the test compound by LC/MS/MS. LC/MS/MS analysis instrument: AB Sciex API 4000 Qtrap.

### 2.6 Liquid phase analysis

Liquid phase conditions: Shimadzu LC-20AD Pump
Chromatographic column: Agilent ZORBAX XDB-C18 (50×2.1 mm, 3.5 µm) mobile phase: 0.1% formic acid aqueous solution as A solution, acetonitrile as B solution
Flow rate: 0.4 mL/min
Elution time: 0-4.0 minutes, the eluent is as follows:

| Time/min | A solution | B solution |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

### 3. Test results and analysis

The main pharmacokinetic parameters were calculated with WinNonlin 6.1, and the results of mouse pharmacokinetic experiments are shown in Table 3:

**Table 3**

| Embodiment No. | Pharmacokinetic experiment (5 mg/kg) | | | | | | |
|---|---|---|---|---|---|---|---|
| | | Time to peak | Blood concentration | Curve area | Curve area | Half life period | Mean detention time |
| | | tₘₐₓ (h) | Cₘₐₓ (ng/mL) | AUC₀₋ₜ (ng/mL×h) | AUC_{0-∞} (ng/mL×h) | t_{1/2} (h) | MRT (h) |
| 1 | blood | 0.50 | 25.57 | 58.10 | 61.50 | 1.75 | 2.03 |
| 1 | colon | 3.00 | 2717.33 | 8322.33 | NA | NA | 3.68 |
| 1 | ileum | 3.00 | 27066.67 | 90470.00 | 93698.05 | 1.08 | 2.84 |
| 60 | blood | 2.00 | 10.32 | 31.14 | 32.85 | 1.41 | 2.58 |
| 60 | colon | 5.00 | 2313.33 | 9131.17 | NA | NA | 3.83 |
| 60 | ileum | 1.00 | 26133.33 | 97959.67 | 102735.06 | 1.26 | 2.49 |
| 169 | blood | 0.50 | 2.21 | 3.04 | NA | NA | 1.08 |
| 169 | colon | 5.00 | 9010.00 | 23168.33 | NA | NA | 2.29 |
| 169 | ileum | 1.00 | 23306.67 | 68927.27 | NA | NA | 4.64 |
| 170 | blood | 7.00 | 15.98 | 36.70 | NA | NA | 4.13 |
| 170 | colon | 3.00 | 4323.33 | 12952.50 | NA | NA | 3.36 |
| 170 | ileum | 1.00 | 49935.33 | 118039.13 | 172293.32 | 5.48 | 2.19 |
| 171 | blood | 2.00 | 8.91 | 23.40 | 26.70 | 1.93 | 3.05 |
| 171 | colon | 3.00 | 438.00 | 2007.00 | NA | NA | 4.24 |
| 171 | ileum | 3.00 | 17037.00 | 79777.00 | NA | NA | 3.35 |
| 179 | blood | 0.50 | 3.80 | 6.76 | NA | NA | 1.66 |
| 179 | colon | 1.00 | 484.97 | 2044.08 | 2182.71 | 1.49 | 2.83 |
| 179 | ileum | 3.00 | 2332.67 | 10335.83 | NA | NA | 2.88 |
| 191 | blood | 1.00 | 0.81 | NA | NA | NA | NA |
| 191 | colon | 5.00 | 3350.00 | 10573.00 | NA | NA | 4.59 |
| 191 | ileum | 1.00 | 32600.00 | 51491.00 | 51747.00 | 1.39 | 1.13 |
| 213 | blood | 3.00 | 9.69 | 7.37 | NA | NA | 2.66 |
| 213 | colon | 5.00 | 1752.00 | 4933.58 | NA | NA | 4.92 |
| 213 | ileum | 5.00 | 321.43 | 1235.32 | NA | NA | 4.26 |
| 214 | blood | 3.00 | 1.60 | 3.59 | NA | NA | 1.82 |
| 214 | colon | 5.00 | 5593.33 | 19456.33 | NA | NA | 4.4 |
| 214 | ileum | 1.00 | 24816.67 | 49326.83 | 49523.32 | 0.79 | 1.52 |
| 237 | blood | 2.00 | 7.12 | 11.03 | NA | NA | 1.73 |
| 237 | colon | 3.00 | 2205.33 | 6459.08 | 7244.45 | 1.52 | 3.43 |
| 237 | ileum | 3.00 | 4136.67 | 15340.80 | 15361.95 | 0.55 | 2.43 |
| 238 | blood | 1.00 | 12.67 | 19.65 | NA | NA | 1.47 |
| 238 | colon | 3.00 | 1315.00 | 5270.95 | 7376.51 | 2.89 | 4.16 |
| 238 | ileum | 1.00 | 20960.00 | 36588.13 | 36609.48 | 0.58 | 1.29 |
| 244 | blood | 3.00 | 12.00 | 18.01 | NA | NA | 2.12 |
| 244 | colon | 7.00 | 1190.00 | 4902.83 | NA | NA | 4.38 |
| 244 | ileum | 1.00 | 9012.85 | 22556.48 | 22618.33 | 0.66 | 1.83 |
| 246 | blood | 2.00 | 5.56 | 4.76 | NA | NA | 1.46 |
| 246 | colon | 7.00 | 3256.67 | 13768.65 | NA | NA | 4.79 |
| 246 | ileum | 1.00 | 16766.67 | 27918.73 | 28435.59 | 1.81 | 1.28 |
| 247 | blood | NA | NA | NA | NA | NA | NA |
| 247 | colon | 3.00 | 2274.33 | 10215.63 | NA | NA | 4.05 |
| 247 | ileum | 1.00 | 13766.67 | 24740.60 | 24856.41 | 0.92 | 1.39 |
| 257 | blood | 1.00 | 33.92 | 64.70 | 64.80 | 1.22 | 2.18 |
| 257 | colon | 5.00 | 3993.33 | 12295.93 | NA | NA | 4.24 |
| 257 | ileum | 3.00 | 25833.33 | 104414.82 | 122516.16 | 1.98 | 3.07 |
| 260 | blood | 1.00 | 24.00 | 27.26 | 29.38 | 1.57 | 1.37 |
| 260 | colon | 3.00 | 8876.67 | 24424.50 | NA | NA | 3.39 |
| 260 | ileum | 1.00 | 9083.33 | 18455.27 | 18667.76 | 1.01 | 1.57 |
| 262 | blood | 2.00 | 8.01 | 13.42 | NA | NA | 1.77 |
| 262 | colon | 3.00 | 1236.67 | 3942.77 | 4522.81 | 1.69 | 3.72 |
| 262 | ileum | 1.00 | 22335.00 | 40246.99 | 40255.23 | 0.49 | 1.34 |
| 263 | blood | 3.00 | 12.74 | 57.62 | NA | NA | 3.51 |
| 263 | colon | 3.00 | 5540.00 | 15486.72 | NA | NA | 3.58 |
| 263 | ileum | 1.00 | 5490.00 | 9908.63 | 9939.86 | 0.82 | 1.36 |
| 267 | blood | NA | NA | NA | NA | NA | NA |
| 267 | colon | 5.00 | 1267.00 | 5609.00 | NA | NA | 4.40 |
| 267 | ileum | 1.00 | 12170.00 | 20694.00 | 20698.00 | 0.53 | 1.24 |
| 277 | blood | 2.00 | 11.96 | 29.12 | 31.05 | 0.96 | 2.53 |
| 277 | colon | 7.00 | 649.00 | 2891.73 | NA | NA | 3.89 |
| 277 | ileum | 1.00 | 18736.67 | 42963.67 | 43348.57 | 0.92 | 1.77 |
| 278 | blood | 0.50 | 1.79 | 3.62 | NA | NA | 1.50 |
| 278 | colon | 5.00 | 1297.67 | 4641.18 | NA | NA | 4.26 |
| 278 | ileum | 1.00 | 3696.00 | 6243.00 | 6263.27 | 0.95 | 1.28 |
| 288 | blood | 2.00 | 11.03 | 21.50 | 24.50 | 2.60 | 2.5 |
| 288 | colon | 3.00 | 676.33 | 2899.00 | NA | NA | 3.91 |
| 288 | ileum | 3.00 | 7376.67 | 17648.00 | NA | NA | 2.89 |
| 295 | blood | 2.00 | 6.54 | 10.36 | NA | NA | 1.77 |
| 295 | colon | 3.00 | 1028.00 | 3394.72 | NA | NA | 3.82 |
| 295 | ileum | 3.00 | 4302.33 | 10688.77 | NA | NA | 2.69 |
| 299 | blood | 0.50 | 6.01 | 10.59 | 17.81 | 3.76 | 1.92 |
| 299 | colon | 7.00 | 295.00 | 948.28 | NA | NA | 4.94 |
| 299 | ileum | 3.00 | 530.67 | 1323.01 | 1353.39 | 0.89 | 3.37 |
| 301 | blood | 1.00 | 69.43 | 142.73 | 145.22 | 1.07 | 1.65 |
| 301 | colon | 5.00 | 1497.00 | 4242.63 | NA | NA | 4.74 |
| 301 | ileum | 1.00 | 21093.33 | 35291.01 | 35304.02 | 0.60 | 1.23 |

| | | | | | | | |
|---|---|---|---|---|---|---|---|
| **[3254]** NA refers to none detected or not being tested (the detection limit of blood concentration was 1ng/mL, when the Cₘₐₓ in the blood test is NA, then NA in the blood test index refers to none detected; when the Cₘₐₓ in the blood test is higher than the quantitative limit 1ng/mL, then NA in the blood test index refers to not being tested; NA in the tissues (colon and ileum) refers to not being tested). | | | | | | | |

### Experimental results:

From the mouse pharmacokinetics (PK) experimental results in the table, it can be seen that the embodiment compounds of the present disclosure show good exposure levels in the colon and ileum. The area under the plasma concentration-time curve (AUC) and the maximum plasma concentration (Cₘₐₓ) all meet the screening criteria. The ratio of the drug concentration in colon to that in ileum and the ratio of the drug concentration in colon to that in plasma of the compounds are high, which shows good selectivity.

### Test 4. In vivo efficacy test procedures and results

### 4.1 Experimental objective:

To evaluate the drug efficacy of the embodiment compound on the C57BL/6 mouse colitis model induced by DSS (dextran sulfate sodium).

### 4.2. Main experimental materials

### 4.2.1 Instruments

1. balance Mettler toledo AL104
2. balance TP-602

### 4.2.2 Reagents

1. Dextran Sodium Sulfate (DSS): MP Biomedicals, LLC, Solon, Ohio, Item No.:160110
2. Cyclosporine (CsA): Novartis, Switzerland, batch number: S0033A
3. Sodium carboxymethyl cellulose: Sinopharm Chemical Reagent Co., Ltd.
4. Twain 80: Sigma, Item No.: 8CBM 513V

### 4.2.3 Experimental animals

| Animal species and strains: | C57BL/6 |
|---|---|
| Gender, age/weight: | Female, 6-8 weeks old / 18-20 grams |
| supplier: | Shanghai Slac Laboratory Animal CO.LTD |

### 4.3. Experimental procedures

### 4.3.1 Grouping

According to the weight of the animal, BioBook software was used to randomly group the animals on day -1 to ensure that the weight values of each group of animals were similar to reduce deviation, and the grouping and dosing regimen are shown in the following Table.

**Grouping and dosing schedule**

| Group | Test compound | Number of animals | Administration route | Dosage mg/kg | Dosing regimen |
|---|---|---|---|---|---|
| 1 | Blank ^{a} | 10 | p.o. | N/A | BID ^{b}, Day |
| | (Blank control group) | | | | 0-9 |
| 2 | Vehicle ^{a} (Vehicle control group) | 10 | p.o. | N/A | BID, Day 0-9 |
| 3 | Embodiment compound ^{a} | 10 | p.o. | 10 | BID, Day 0-9 |

| | | | | | |
|---|---|---|---|---|---|
| [3274] a: the vehicle was 0.5% CMC-Na+1% Tween 80 [3275] b: interval of 8 hours | | | | | |

### 4.3.2 Experiment process

### 1. Formulation of the reagent

Drinking water containing DSS: an appropriate amount of DSS powder was dissolved in the autoclaved drinking water to prepare a 2% DSS solution.

### 2. Induction of enteritis

On day -1, the animals were equally divided into 12 groups with 10 animals in each group. (Refer to Table 1 for specific grouping scheme)

From 9:00 on day 0 to 9:00 on day 6, the mice in groups 2 to 9 were given 2% DSS aqueous solution for 6 days (from day 0 to day 6), after which the mice were allowed to freely drink normal water for 3 days (from 9:00 on day 6 to before necropsy on day 9). The day of modeling was counted as day 0. The DSS aqueous solution was wrapped in tin foil to protect it from light. The DSS aqueous solution was replaced every 2 days.

The mice in group 1 are allowed to drink normal water freely for 9 days (from 9:00 on day 0 to before necropsy on day 9).

### 3. Dosing

Refer to the above Table for specific dosage, route and time of administration.

### 4.4 Measurement

### 1) Body weight

The recording frequency was once a day.

### 2) Daily Disease Index (DAI)

The recording frequency was once a day, and 4 levels were rated according to the following standards:
Weight change (0: ≤1%; 1: 1-5%; 2: 5-10%; 3: 10-15%; 4: >15%);
Bloody stool (0: negative; 4: positive);
Stool score (0: normal; 2: loose stool; 4: diarrhea)
Daily Disease Index (DAI) was obtained by dividing the sum the scores of the above 3 parts by 3. DAI-time (day) curve based on the daily DAI score was plotted, and the peak area (AUC) under the curve was calculated. The reduction ratio of DAI AUC was calculated by comparing the administration group with the vehicle group, with a calculation formula of (DAI AUC_{administration group}-DAI AUC_{vehicle})/DAI AUCVehicle×100%

### [3292 4.5. Experimental results:

| Embodiment No. | dosage (mg/kg) | reduction ratio of DAI AUC (%) |
|---|---|---|
| Blank (Blank control group) | / | / |
| Vehicle (Vehicle control group) | / | / |
| 60 | 10 | -20.75 |
| 62 | 10 | -32.25 |
| 169 | 10 | -34.31 |
| 170 | 10 | -27.94 |
| 213 | 10 | -24.45 |
| 214 | 10 | -28.91 |
| 246 | 10 | -38.64 |
| 247 | 10 | -22.11 |
| 260 | 10 | -23.38 |
| 278 | 10 | -34.88 |

### 4.6. Experimental conclusion

In the C57BL/6 mouse colitis model induced by DSS, the above embodiment compounds can significantly reduce the daily disease index (DAI) and have obvious pharmacological effects.

## Claims

1. A compound of general formula (I), a stereoisomer thereof or a pharmaceutically acceptable salt thereof: 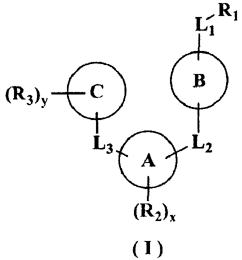 wherein:
L₁ is selected from bond, alkylene, alkenylene, alkynyl, cycloalkyl, heterocyclyl, - (CH₂)ₙ₁C(O)(CH₂)ₘ₁-, -(CH₂)ₙ₁C(O)(CH₂)ₘ₁NRₐₐ(CH₂)ₘ₂-, -NRₐₐ(CH₂)ₙ₁-, -(CH₂)ₙ₁S(O)ₘ₁-, or -(CH₂)ₙ₁S(O)ₘ₁NRₐₐ-;
L₂ is selected from bond, oxygen atom, sulfur atom, -CRₐₐR_{bb}-, -(CH₂)ₙ₁C(O)-, -NR₄-, or - (CH₂)ₙ₁S(O)ₘ₁-;
L₃ is selected from bond, -NRₐₐ-, or -C(O)NRₐₐ-;
ring A is 6-14 membered heteroaryl, wherein the 6-14 membered heteroaryl is selected from 6-14 membered fused heteroaryl; preferably 5,5 fused heteroaryl, 5,6 fused heteroaryl or 6,6 fused heteroaryl;
ring B is selected from 3-10 membered monocyclic heterocyclyl, 6-14 membered bridged heterocyclyl, 6-14 membered fused heterocyclyl or 6-14 membered spiro heterocyclyl;
ring C is heteroaryl;
R₁ is selected from hydrogen atom, deuterium atom, oxo, alkyl, deuterated alkyl, haloalkyl, alkoxy, haloalkoxy, hydroxyalkyl, halogen, amino, nitro, hydroxyl, cyano, alkenyl, alkynyl, cycloalkyl, heterocyclyl, oxoheterocyclyl, thioxoheterocyclyl, aryl, heteroaryl, -(CH₂)ₙ₁Rₐₐ, - (CH₂)ₙ₁ORₐₐ, -NRₐₐC(O)(CH₂)ₙ₁ORₐₐ, -NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, -(CH₂)ₙ₁C(O)NRₐₐR_{bb}, - (CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, alkyl, haloalkyl, halogen, amino, oxo, nitro, cyano, hydroxyl, alkenyl, alkynyl, alkoxy, haloalkoxy, hydroxyalkyl, cycloalkyl, heterocyclyl, aryl, heteroaryl, -(CH₂)ₙ₁-, - (CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc}, - (CH₂)ₙ₁S(O)ₘ₁R_{cc}, -(CH₂)ₙ₁NR_{cc}R_{dd}, -(CH₂)ₙ₁C(O)NR_{cc}R_{dd}, -(CH₂)ₙ₁NR_{cc}C(O)R_{dd} or - (CH₂)ₙ₁NR_{cc}S(O)ₘ₁R_{dd};
R₂ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl, heteroaryl, -(CH₂)ₙ₁Rₐₐ, - (CH₂)ₙ₁O(CH₂)ₘ₁Rₐₐ, -(CH₂)ₙ₁ORₐₐ, -(CH₂)ₙ₁NRₐₐ(CH₂)ₘ₁Rₐₐ, -NRₐₐC(O)(CH₂)ₙ₁ORₐₐ, - NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁S(O)ₘ₁NRₐₐR_{bb}, -(CH₂)ₙ₁P(O)ₘ₂RₐₐR_{bb}, -(CH₂)ₙ₁NRₐₐR_{bb}, -(CH₂)ₙ₁S-, - (CH₂)ₙ₁C(O)NRₐₐR_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted or unsubstituted heteroaryl, - (CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc}, - (CH₂)ₙ₁S(O)ₘ₁R_{cc}, -(CH₂)ₙ₁S(O)ₘ₁NR_{cc}R_{dd}, -(CH₂)ₙ₁NR_{cc}R_{dd}, -(CH₂)ₙ₁C(O)NR_{cc}R_{dd}, - (CH₂)ₙ₁C(O)NR_{cc}S(O)ₘ₁R_{dd}, -(CH₂)ₙ₁NR_{cc}S(O)ₘ₁R_{dd} or -(CH₂)ₙ₁NR_{cc}S(O)ₘ₁R_{dd};
R₃ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl, heteroaryl, -(CH₂)ₙ₁Rₐₐ, - (CH₂)ₙ₁ORₐₐ, -NRₐₐC(O)(CH₂)ₙ₁ORₐₐ, -NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁S(O)ₘ₁NRₐₐR_{bb}, -(CH₂)ₙ₁P(O)ₘ₂RₐₐR_{bb}, - (CH₂)ₙ₁NRₐₐR_{bb}, -(CH₂)ₙ₁C(O)NRₐₐR_{bb}, -(CH₂)ₙ₁NRₐₐC(O)R_{bb} or -(CH₂)ₙ₁N_{Raa}S(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl, substituted or unsubstituted heteroaryl, -(CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, - (CH₂)ₙ₁C(O)OR_{cc}, -(CH₂)ₙ₁S(O)ₘ₁R_{cc}, -(CH₂)ₙ₁S(O)ₘ₁NR_{cc}R_{dd}, -(CH₂)ₙ₁NR_{cc}R_{dd}, - (CH₂)ₙ₁C(O)NR_{cc}R_{dd}, -(CH₂)ₙ₁C(O)NR_{cc}S(O)ₘ₁R_{dd}, -(CH₂)ₙ₁NR_{cc}S(O)ₘ₁R_{dd}, or - (CH₂)ₙ₁NR_{cc}S(O)ₘ₁R_{dd};
R₄ is selected from hydrogen atom, deuterium atom, alkyl, deuterated alkyl, haloalkyl, alkoxy, hydroxyalkyl, haloalkoxy, halogen, amino, nitro, hydroxyl, cyano, alkenyl, or alkynyl;
Rₐₐ, R_{bb}, R_{cc} and R_{dd} are identical or different and are each independently selected from hydrogen atom, deuterium atom, alkyl, deuterated alkyl, haloalkyl, alkoxy, hydroxyalkyl, haloalkoxy, halogen, cyano, nitro, hydroxyl, amino, alkenyl, alkynyl, cycloalkyl, heterocyclyl, aryl, or heteroaryl, wherein the alkyl, deuterated alkyl, haloalkyl, alkoxy, hydroxyalkyl, haloalkoxy, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl, or substituted or unsubstituted heteroaryl;
x is an integer of 0, 1, 2 or 3;
y is an integer of 0, 1, 2, 3, 4 or 5;
m₁ is an integer of 0, 1 or 2;
m₂ is an integer of 0, 1 or 2; and
n₁ is an integer of 0, 1, 2, 3, 4 or 5.

2. The compound of general formula (I), the stereoisomer thereof or the pharmaceutically acceptable salt thereof according to claim 1, wherein,
L₁ is selected from bond, alkylene, -(CH₂)ₙ₁C(O)(CH₂)ₘ₁-, - (CH₂)ₙ₁C(O)(CH₂)ₘ₁NRₐₐ(CH₂)ₘ₂-, or -(CH₂)ₙ₁S(O)ₘ₁-;
and/or, L₂ is selected from bond, oxygen atom, or -NR₄-;
and/or, R₄ is selected from hydrogen atom, or alkyl;
and/or, L₃ is -NRₐₐ-;
and/or, ring A is 6-14 membered fused heteroaryl;
and/or, ring B is selected from 3-10 membered monocyclic heterocyclyl, 6-14 membered bridged heterocyclyl, or 6-14 membered fused heterocyclyl;
and/or, ring C is heteroaryl;
and/or, R₁ is selected from hydrogen atom, alkyl, haloalkyl, amino, heterocyclyl, or heteroaryl; wherein the alkyl, amino, heterocyclyl, or heteroaryl is optionally further substituted with one or more substituents selected from halogen, cyano, alkyl, or alkoxy;
and/or, R₂ is selected from hydrogen atom, alkyl, alkoxy, halogen, cycloalkyl, heterocyclyl, or heteroaryl; wherein the heteroaryl is optionally further substituted with one or more substituents selected from unsubstituted alkyl, unsubstituted alkoxy, or unsubstituted cycloalkyl;
and/or, R₃ is selected from hydrogen atom, alkyl, hydroxyalkyl, halogen, cyano, cycloalkyl, -(CH₂)ₙ₁C(O)Rₐₐ, or -(CH₂)ₙ₁C(O)NRₐₐR_{bb}; wherein the alkyl is optionally further substituted with hydroxyl;
and/or, Rₐₐ and R_{bb} are identical or different, and are each independently selected from hydrogen atom, alkyl, or amino;
and/or, x is 0, 1, 2 or 3;
and/or, y is 0, 1, 2 or 3;
and/or, m₁ is 0, 1 or 2;
and/or, m₂ is 0, 1 or 2;
and/or, n₁ is 0, 1 or 2.

3. The compound of general formula (I), the stereoisomer thereof or the pharmaceutically acceptable salt thereof according to claim 1, wherein,
the compound of formula (I) is a compound of formula (1-1), 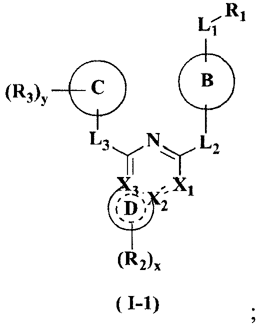
wherein, ring D is heterocycloalkenyl, aryl, or heteroaryl;
and/or, the compound of formula (I) is a compound of formula (I-1), 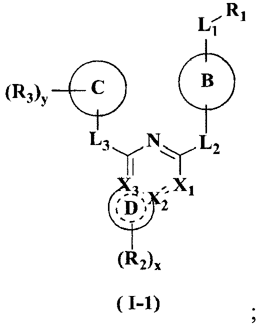
wherein, X₁, X₂ and X₃ are independently CH or N;
and/or, L₁ is selected from alkylene, -(CH₂)ₙ₁C(O)(CH₂)ₘ₁-, - (CH₂)ₙ₁C(O)(CH₂)ₘ₁NRₐₐ(CH₂)ₘ₂-, or -(CH₂)ₙ₁S(O)ₘ₁-;
and/or, R₁ is selected from halogen, cyano, alkyl, heterocyclyl, or heteroaryl; wherein the alkyl is optionally further substituted with halogen or alkoxy; wherein the heterocyclyl is optionally further substituted with cyano; wherein the heteroaryl is optionally further substituted with alkyl;
and/or, when L₁ is -(CH₂)ₙ₁C(O)(CH₂)ₘ₁-, R₁ is alkyl, heterocyclyl, or heteroaryl; wherein the alkyl is optionally further substituted with halogen; wherein the heterocyclyl or heteroaryl is optionally further substituted with alkyl;
and/or, L₂ is selected from oxygen atom, or -NR₄-;
and/or, R₄ is selected from hydrogen atom, or alkyl;
and/or, L₃ is -NH-;
and/or, ring B is selected from 3-10 membered monocyclic heterocyclyl, 6-14 membered fused heterocyclyl, or 6-14 membered bridged heterocyclyl;
and/or, ring C is heteroaryl;
and/or, R₂ is selected from hydrogen atom, alkyl, alkoxy, halogen, cycloalkyl, heterocyclyl, aryl, or heteroaryl; wherein the heteroaryl is optionally further substituted with unsubstituted alkyl, unsubstituted alkoxy, or unsubstituted cycloalkyl;
and/or, Rₐₐ is selected from hydrogen atom, or alkyl;
and/or, R₃ is selected from hydrogen atom, alkyl, or hydroxyalkyl.

4. The compound of general formula (I), the stereoisomer thereof or the pharmaceutically acceptable salt thereof according to claim 1, wherein, the compound of formula (I) is as defined in any one of the following schemes: 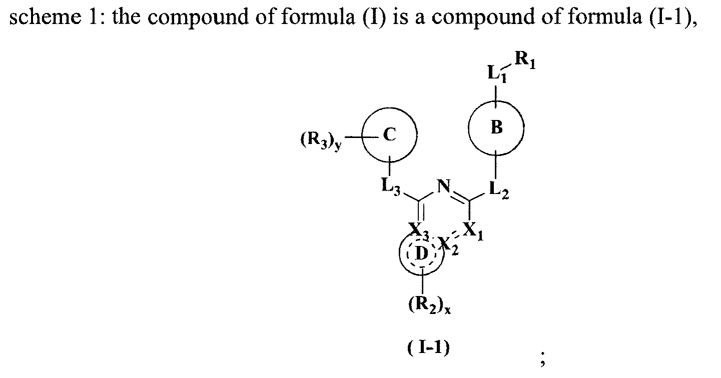
wherein, ring D is heterocycloalkenyl, aryl, or heteroaryl;
X₁, X₂ and X₃ are independently CH or N;
L₁ is selected from alkylene, -(CH₂)ₙ₁C(O)(CH₂)ₘ₁-, -(CH₂)ₙ₁C(O)(CH₂)ₘ₁NRₐₐ(CH₂)ₘ₂-, or -(CH₂)ₙ₁S(O)ₘ₁-;
R₁ is selected from halogen, cyano, alkyl, alkoxy, cycloalkyl, heterocyclyl, or heteroaryl; wherein the alkyl is optionally further substituted with halogen or alkoxy; wherein the heterocyclyl is optionally further substituted with cyano; wherein the heteroaryl is optionally further substituted with alkyl;
when L₁ is -(CH₂)ₙ₁C(O)(CH₂)ₘ₁-, R₁ is alkyl, heterocyclyl, or heteroaryl; wherein the alkyl is optionally further substituted with halogen; wherein the heterocyclyl or heteroaryl is optionally further substituted with alkyl;
scheme 2:
the compound of formula (I) is a compound of formula (I-2), 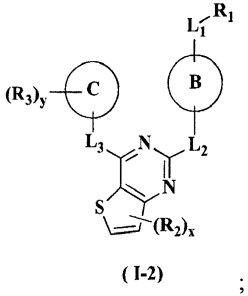 wherein, x is 0, 1 or 2;
scheme 3:
the compound of formula (I) is a compound of formula (I-3), 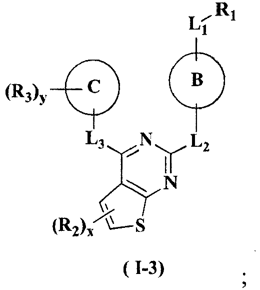 wherein, x is 0, 1 or 2;
scheme 4:
the compound of formula (I) is a compound of formula (I-4), 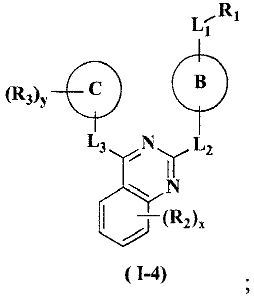
wherein, L₁ is selected from alkylene, -(CH₂)ₙ₁C(O)(CH₂)ₘ₁-, - (CH₂)ₙ₁C(O)(CH₂)ₘ₁NRₐₐ(CH₂)ₘ₂-, or -(CH₂)ₙ₁S(O)ₘ₁-;
R₁ is selected from halogen, cyano, alkyl, alkoxy, heterocyclyl, or heteroaryl; wherein the alkyl is optionally further substituted with halogen or alkoxy; wherein the heterocyclyl is optionally further substituted with cyano;
when L₁ is -(CH₂)ₙ₁C(O)(CH₂)ₘ₁-, R₁ is alkyl, heterocyclyl, or heteroaryl; wherein the alkyl is optionally further substituted with halogen; wherein the heterocyclyl or heteroaryl is optionally further substituted with alkyl.

5. The compound of general formula (I), the stereoisomer thereof or the pharmaceutically acceptable salt thereof according to claim 1, wherein, the compound of formula (I) is as defined in any one of the following schemes:
scheme 1:
L₁ is selected from bond, alkylene, cycloalkyl, heterocyclyl, -(CH₂)ₙ₁C(O)(CH₂)ₘ₁-, - (CH₂)ₙ₁C(O)(CH₂)ₘ₁NRₐₐ(CH₂)ₘ₂-, -NRₐₐ(CH₂)ₙ₁-, -(CH₂)ₙS(O)ₘ₁-, or -(CH₂)ₙ₁S(O)ₘ₁NRₐₐ-;
L₂ is selected from bond, oxygen atom, -CRₐₐR_{bb}-, -(CH₂)ₙ₁C(O)-, -NR₄-, or - (CH₂)ₙ₁S(O)ₘ₁-;
R₄ is selected from hydrogen atom, or alkyl;
L₃ is selected from bond, -NRₐₐ-, or -C(O)NRₐₐ-;
ring A is 6-14 membered fused heteroaryl;
ring B is selected from 3-10 membered monocyclic heterocyclyl, 6-14 membered bridged heterocyclyl, 6-14 membered fused heterocyclyl, or 6-14 membered spiro heterocyclyl;
ring C is heteroaryl;
R₁ is selected from hydrogen atom, alkyl, haloalkyl, alkoxy, halogen, amino, hydroxyl, cyano, cycloalkyl, heterocyclyl, aryl, or heteroaryl; wherein the alkyl, amino, heterocyclyl, aryl or heteroaryl is optionally further substituted with one or more substituents selected from halogen, cyano, alkyl, or alkoxy;
R₂ is selected from hydrogen atom, alkyl, alkoxy, halogen, amino, hydroxyl, cyano, cycloalkyl, heterocyclyl, aryl, heteroaryl, -(CH₂)ₙ₁C(O)R_{cc}, -(CH₂)ₙ₁SRₐₐ, or -(CH₂)ₙ₁NRₐₐR_{bb}; wherein the alkyl, alkoxy, amino, cycloalkyl, heterocyclyl, aryl, or heteroaryl is optionally further substituted with one or more substituents selected from substituted or unsubstituted alkyl, unsubstituted alkoxy, unsubstituted cycloalkyl, substituted or unsubstituted amino, substituted or unsubstituted heterocyclyl, or unsubstituted heteroaryl; wherein "substituted" refers to be substituted with alkyl, or halogen;
R_{cc} is heterocyclyl; wherein the heterocyclyl is optionally substituted with unsubstituted alkyl;
R₃ is selected from hydrogen atom, alkyl, haloalkyl, hydroxyalkyl, alkoxy, halogen, hydroxyl, cyano, cycloalkyl, heterocyclyl, -(CH₂)ₙ₁C(O)Rₐₐ, -(CH₂)ₙ₁C(O)NRₐₐR_{bb}, - (CH₂)ₙ₁S(O)ₘ₁Rₐₐ, or -(CH₂)ₙ₁S(O)ₘ₁NRₐₐR_{bb}; wherein the alkyl, alkoxy, cycloalkyl, or heterocyclyl is optionally further substituted with one or more substituents selected from halogen, hydroxyl, unsubstituted alkyl, or unsubstituted alkoxy;
Rₐₐ and R_{bb} are identical or different and are each independently selected from hydrogen atom, alkyl, amino, or heteroaryl; wherein the alkyl and amino are optionally further substituted with one or more substituents selected from unsubstituted alkyl, substituted or unsubstituted heterocyclyl, unsubstituted heteroaryl; wherein "substituted" refers to be substituted with alkyl, or halogen;
x is 0, 1, 2 or 3;
y is 0, 1, 2 or 3;
m₁ is 0, 1 or 2;
m₂ is 0, 1 or 2; and
n₁ is 0, 1 or 2;
scheme 2:
L₁ is selected from bond, alkylene, -(CH₂)ₙ₁C(O)(CH₂)ₘ₁-, - (CH₂)ₙ₁C(O)(CH₂)ₘ₁NRₐₐ(CH₂)ₘ₂-, or -(CH₂)ₙ₁S(O)ₘ₁-;
L₂ is selected from bond, oxygen atom, or -NR₄-;
R₄ is selected from hydrogen atom, or alkyl;
L₃ is -NRₐₐ-;
ring A is 6-14 membered fused heteroaryl;
ring B is selected from 3-10 membered monocyclic heterocyclyl, 6-14 membered bridged heterocyclyl, or 6-14 membered fused heterocyclyl;
ring C is heteroaryl;
R₁ is selected from hydrogen atom, alkyl, haloalkyl, amino, heterocyclyl, or heteroaryl; wherein the alkyl, amino, heterocyclyl, or heteroaryl is optionally further substituted with one or more substituents selected from halogen, cyano, alkyl, or alkoxy;
R₂ is selected from hydrogen atom, alkyl, alkoxy, halogen, cycloalkyl, heterocyclyl, or heteroaryl; wherein the heteroaryl is optionally further substituted with one or more substituents selected from unsubstituted alkyl, unsubstituted alkoxy, or unsubstituted cycloalkyl;
R₃ is selected from hydrogen atom, alkyl, hydroxyalkyl, halogen, cyano, cycloalkyl, - (CH₂)ₙ₁C(O)Rₐₐ, or -(CH₂)ₙ₁C(O)NRₐₐR_{bb}; wherein the alkyl is optionally further substituted with hydroxyl;
Rₐₐ and R_{bb} are identical or different and are each independently selected from hydrogen atom, alkyl, or amino;
x is 0, 1, 2 or 3;
y is 0, 1, 2 or 3;
m₁ is 0, 1 or 2;
m₂ is 0, 1 or 2; and
n₁ is 0, 1 or 2.

6. The compound of general formula (I), the stereoisomer thereof or the pharmaceutically acceptable salt thereof according to claim 1, wherein, the compound of formula (I) is as defined in any one of the following schemes: 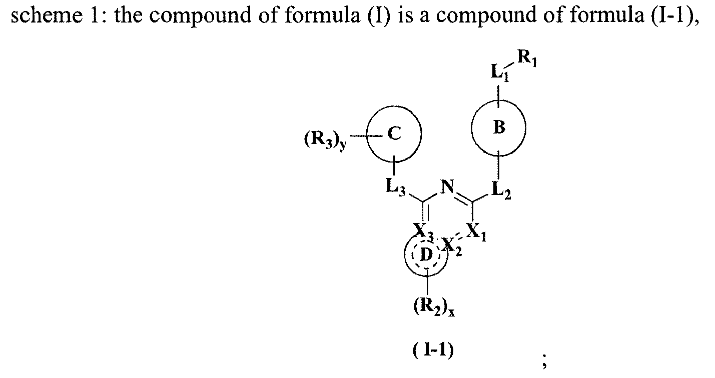
wherein, ring D is heterocycloalkenyl, aryl, or heteroaryl;
X₁, X₂ and X₃ are independently CH or N;
L₁ is selected from alkylene, -(CH₂)ₙ₁C(O)(CH₂)ₘ₁-, -(CH₂)ₙ₁C(O)(CH₂)ₘ₁NRₐₐ(CH₂)ₘ₂-, or -(CH₂)ₙ₁S(O)ₘ₁-;
R₁ is selected from halogen, cyano, alkyl, alkoxy, cycloalkyl, heterocyclyl, or heteroaryl; wherein the alkyl is optionally further substituted with halogen or alkoxy; wherein the heterocyclyl is optionally further substituted with cyano; wherein the heteroaryl is optionally further substituted with alkyl;
when L₁ is -(CH₂)ₙ₁C(O)(CH₂)ₘ₁-, R₁ is alkyl, heterocyclyl, or heteroaryl; wherein the alkyl is optionally further substituted with halogen; wherein the heterocyclyl or heteroaryl is optionally further substituted with alkyl;
L₂ is selected from oxygen atom, or -NR₄-;
R₄ is selected from hydrogen atom, or alkyl;
L₃ is -NH-;
ring B is selected from 3-10 membered monocyclic heterocyclyl, 6-14 membered fused heterocyclyl, or 6-14 membered bridged heterocyclyl;
ring C is heteroaryl;
R₂ is selected from hydrogen atom, alkyl, alkoxy, halogen, cycloalkyl, heterocyclyl, aryl, heteroaryl, -(CH₂)ₙ₁OR_{cc}, or -(CH₂)ₙ₁NRₐₐR_{bb}; wherein the alkyl is optionally further substituted with heterocyclyl unsubstituted or substituted with alkyl; wherein the alkoxy is optionally further substituted with unsubstituted alkoxy, heterocyclyl unsubstituted or substituted with alkyl, or unsubstituted heteroaryl; wherein the heterocyclyl is optionally further substituted with unsubstituted alkyl, unsubstituted alkoxy, or amino substituted with alkyl; wherein the heteroaryl is optionally further substituted with alkyl unsubstituted or substituted with halogen, unsubstituted alkoxy, or unsubstituted cycloalkyl;
Rₐₐ and R_{bb} are identical or different and are each independently selected from hydrogen atom, or alkyl; wherein the alkyl is optionally further substituted with heterocyclyl unsubstituted or substituted with alkyl, or unsubstituted heteroaryl;
R_{cc} is heterocyclyl; wherein the heterocyclyl is optionally further substituted with unsubstituted alkyl;
R₃ is selected from hydrogen atom, alkyl, or hydroxyalkyl; 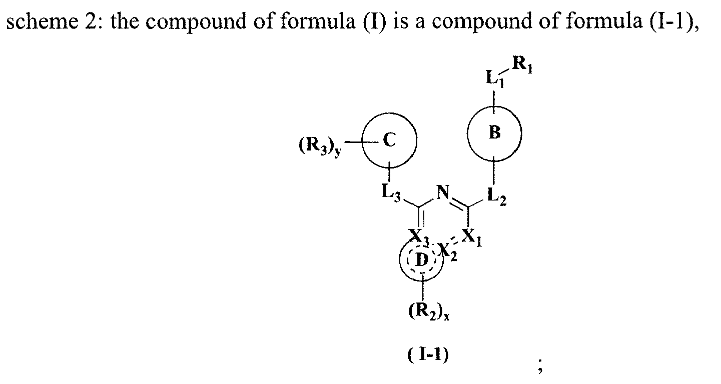 wherein, ring D is heterocycloalkenyl, aryl, or heteroaryl;
X₁, X₂ and X₃ are independently CH or N;
L₁ is selected from alkylene, -(CH₂)ₙ₁C(O)(CH₂)ₘ₁-, -(CH₂)ₙ₁C(O)(CH₂)ₘ₁NRₐₐ(CH₂)m₂-, or -(CH₂)ₙ₁S(O)ₘ₁-;
R₁ is selected from halogen, cyano, alkyl, heterocyclyl, or heteroaryl; wherein the alkyl is optionally further substituted with halogen or alkoxy; wherein the heterocyclyl is optionally further substituted with cyano; wherein the heteroaryl is optionally further substituted with alkyl;
when L₁ is -(CH₂)ₙ₁C(O)(CH₂)ₘ₁-, R₁ is alkyl, heterocyclyl, or heteroaryl; wherein the alkyl is optionally further substituted with halogen; wherein the heterocyclyl or heteroaryl is optionally further substituted with alkyl;
L₂ is selected from oxygen atom, or -NR₄-;
R₄ is selected from hydrogen atom, or alkyl;
L₃ is -NH-;
ring B is selected from 3-10 membered monocyclic heterocyclyl, 6-14 membered fused heterocyclyl, or 6-14 membered bridged heterocyclyl;
ring C is heteroaryl;
R₂ is selected from hydrogen atom, alkyl, alkoxy, halogen, cycloalkyl, heterocyclyl, aryl, or heteroaryl; wherein the heteroaryl is optionally further substituted with unsubstituted alkyl, unsubstituted alkoxy, or unsubstituted cycloalkyl;
Rₐₐ is selected from hydrogen atom, or alkyl;
R₃ is selected from hydrogen atom, alkyl, or hydroxyalkyl;
or, 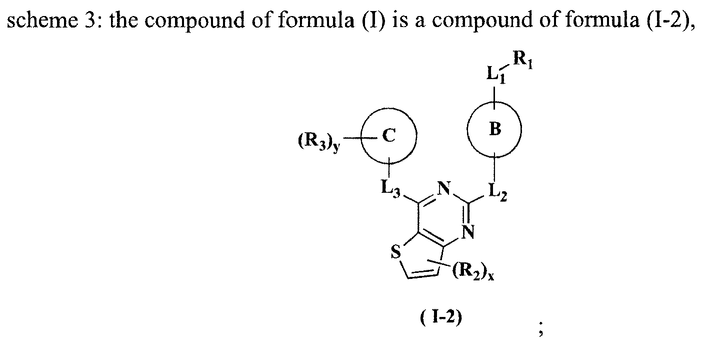
wherein, L₁ is alkylene, -(CH₂)ₙ₁C(O)(CH₂)ₘ₁-, -(CH₂)ₙ₁C(O)(CH₂)ₘ₁NRₐₐ(CH₂)ₘ₂-, or - (CH₂)ₙ₁ S(O)ₘ₁-;
Rₐₐ is selected from hydrogen atom, or alkyl;
L₂ is -NH-;
L₃ is -NH-;
ring B is selected from 3-10 membered monocyclic heterocyclyl, 6-14 membered bridged heterocyclyl, or 6-14 membered fused heterocyclyl;
ring C is heteroaryl;
R₁ is alkyl, cyano, heterocyclyl, or heteroaryl; wherein the heterocyclyl is optionally further substituted with cyano;
R₂ is selected from hydrogen atom, or alkyl;
R₃ is selected from hydrogen atom, alkyl, or hydroxyalkyl;
x is 0, 1 or 2;
scheme 4: the compound of formula (I) is a compound of formula (I-2), 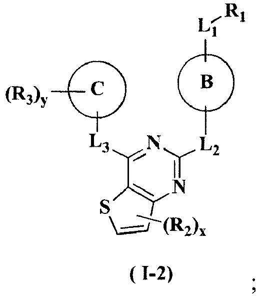
wherein, L₁ is alkylene, -(CH₂)ₙ₁C(O)(CH₂)ₘ₁-, -(CH₂)ₙ₁C(O)(CH₂)ₘ₁NRₐₐ(CH₂)ₘ₂-, - (CH₂)ₙ₁S(O)ₘ₁-;
Rₐₐ is selected from hydrogen atom, or alkyl;
L₂ is -NH-;
L₃ is -NH-;
ring B is selected from 6-14 membered bridged heterocyclyl, or 6-14 membered fused heterocyclyl;
ring C is heteroaryl;
R₁ is alkyl, cyano, or heterocyclyl;
R₂ is selected from hydrogen atom, or alkyl;
R₃ is selected from hydrogen atom, alkyl, or hydroxyalkyl;
x is 0, 1 or 2; 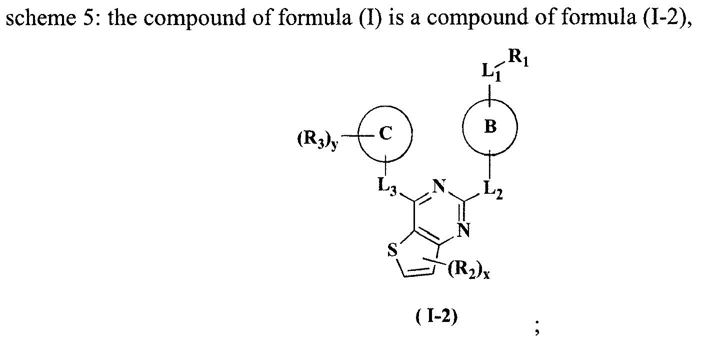
wherein, L₁ is C₁-C₄ alkylene, -(CH₂)ₙ₁C(O)(CH₂)ₘ₁-, -(CH₂)ₙ₁C(O)(CH₂)ₘ₁NRₐₐ(CH₂)ₘ₂-, -(CH₂)ₙ₁S(O)ₘ₁-;
Rₐₐ is selected from hydrogen atom, or C₁-C₃ alkyl;
L₂ is -NH-;
L₃ is -NH-;
ring B is selected from 7-10 membered bridged heterocyclyl with "heteroatom selected from one or more of N, O or S, and the number of the heteroatom being 1-3", or 7-10 membered fused heterocyclyl with "heteroatom selected from one or more of N, O or S, and the number of the heteroatom being 1-3";
ring C is 5-6 membered monocyclic heteroaryl with "heteroatom selected from one or more of N, O or S, and the number of the heteroatom being 1-3";
R₁ is C₁-C₄ alkyl, cyano, or 4-6 membered monocyclic heterocyclyl with "heteroatom selected from one or more of N, O or S, and the number of the heteroatom being 1-3";
R₂ is selected from hydrogen atom, or C₁-C₃ alkyl;
R₃ is selected from hydrogen atom, C₁-C₃ alkyl, or C₁-C₃ hydroxyalkyl;
x is 0, 1 or 2;
or, 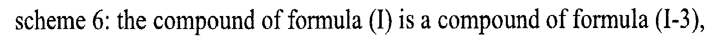 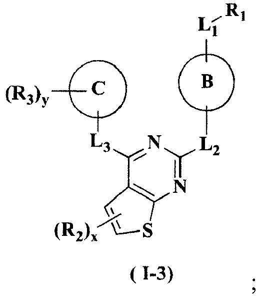
wherein, L₁ is alkylene, -(CH₂)ₙ₁C(O)(CH₂)ₘ₁-, -(CH₂)ₙ₁C(O)(CH₂)ₘ₁NRₐₐ(CH₂)ₘ₂-, or - (CH₂)ₙ₁S(O)ₘ₁-;
L₂ is oxygen atom, or -NR₄-;
R₄ is selected from hydrogen atom, or alkyl;
L₃ is -NH-;
ring B is selected from 3-10 membered monocyclic heterocyclyl, 6-14 membered bridged heterocyclyl, or 6-14 membered fused heterocyclyl;
ring C is heteroaryl;
R₁ is hydrogen atom, alkyl, alkoxy, cyano, cycloalkyl, heterocyclyl, aryl, or heteroaryl; wherein the heterocyclyl, or heteroaryl is optionally further substituted with unsubstituted alkyl, or cyano; wherein the aryl is optionally further substituted with unsubstituted alkoxy;
R₂ is selected from hydrogen atom, alkyl, heterocyclyl, or -(CH₂)ₙ₁SRₐₐ; wherein the alkyl is optionally further substituted with unsubstituted alkoxy, or heterocyclyl unsubstituted or substituted with alkyl; wherein the heterocyclyl is optionally further substituted with alkyl;
R₃ is selected from hydrogen atom, alkyl, or hydroxyalkyl;
Rₐₐ is selected from hydrogen atom, or alkyl;
x is 0, 1 or 2; 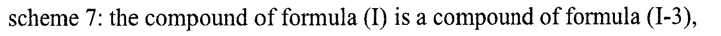 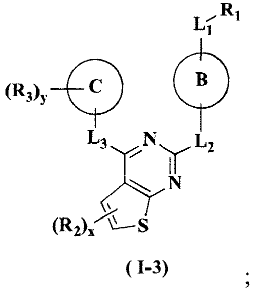
wherein, L₁ is alkylene, -(CH₂)ₙ₁C(O)(CH₂)ₘ₁-, -(CH₂)ₙ₁C(O)(CH₂)ₘ₁NRₐₐ(CH₂)ₘ₂-, or - (CH₂)ₙ₁S(O)ₘ₁-;
L₂ is oxygen atom, or -NR₄-;
R₄ is selected from hydrogen atom, or alkyl;
L₃ is -NH-;
ring B is selected from 3-10 membered monocyclic heterocyclyl, 6-14 membered bridged heterocyclyl, or 6-14 membered fused heterocyclyl;
ring C is heteroaryl;
R₁ is hydrogen atom, alkyl, cyano, heterocyclyl, or heteroaryl; wherein the heterocyclyl, or heteroaryl is optionally further substituted with unsubstituted alkyl, or cyano;
R₂ is selected from hydrogen atom, or alkyl;
R₃ is selected from hydrogen atom, alkyl, or hydroxyalkyl;
Rₐₐ is selected from hydrogen atom, or alkyl;
x is 0, 1 or 2; 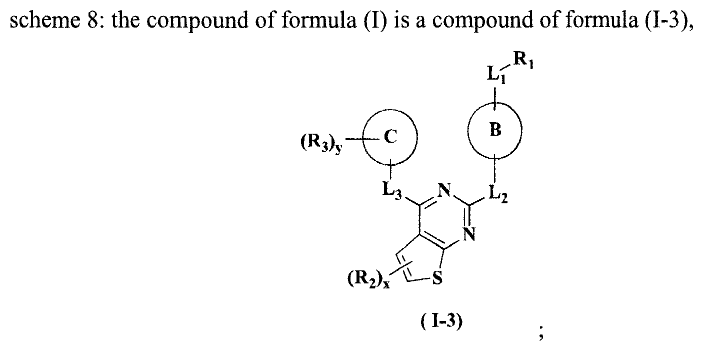
wherein, L₁ is C₁-C₄ alkylene, -(CH₂)ₙ₁C(O)(CH₂)ₘ₁-, -(CH₂)ₙ₁C(O)(CH₂)ₘ₁NRₐₐ(CH₂)ₘ₂-, or -(CH₂)ₙ₁S(O)ₘ₁-;
L₂ is oxygen atom, or -NR₄-;
R₄ is selected from hydrogen atom, or C₁-C₃ alkyl;
L₃ is -NH-;
ring B is selected from 4-6 membered monocyclic heterocyclyl with "heteroatom selected from one or more of N, O or S, and the number of the heteroatom being 1-3", 7-10 membered bridged heterocyclyl with "heteroatom selected from one or more of N, O or S, and the number of the heteroatom being 1-3", or 7-10 membered fused heterocyclyl with "heteroatom selected from one or more of N, O or S, and the number of the heteroatom being 1-3";
ring C is 5-6 membered monocyclic heteroaryl with "heteroatom selected from one or more of N, O or S, and the number of the heteroatom being 1-3";
R₁ is hydrogen atom, C₁-C₃ alkyl, cyano, 4-6 membered monocyclic heterocyclyl with "heteroatom selected from one or more of N, O or S, and the number of the heteroatom being 1-3", or 5-6 membered heteroaryl with "heteroatom selected from one or more of N, O or S, and the number of the heteroatom being 1-3"; wherein the monocyclic heterocyclyl, or heteroaryl is optionally further substituted with C₁-C₃ alkyl, or cyano;
R₂ is selected from hydrogen atom, or C₁-C₃ alkyl;
R3 is selected from hydrogen atom, C₁-C₃ alkyl, or C₁-C₃ hydroxyalkyl;
Rₐₐ is selected from hydrogen atom, or C₁-C₃ alkyl;
x is 0, 1 or 2; 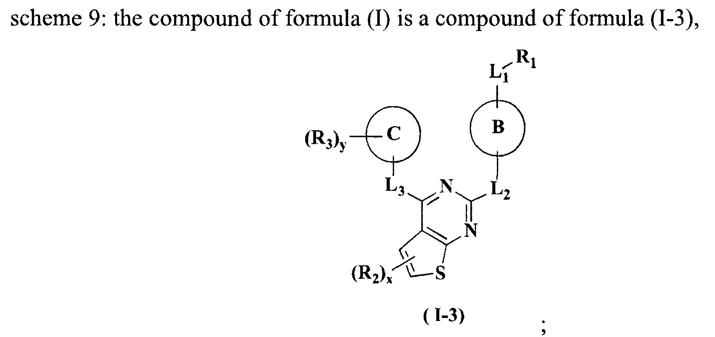
wherein, L₁ is alkylene, -(CH₂)ₙ₁C(O)(CH₂)ₘ-, or -(CH₂)ₙ₁C(O)(CH₂)ₘ₁NRₐₐ(CH₂)ₘ₂-;
R₁ is alkyl, cyano, heterocyclyl or heteroaryl; wherein the heterocyclyl is optionally further substituted with alkyl;
L₂ is oxygen atom, or -NR₄-;
R₄ is selected from hydrogen atom, or alkyl;
L₃ is -NH-;
ring B is 6-14 membered bridged heterocyclyl;
ring C is heteroaryl;
R₂ is selected from hydrogen atom, or alkyl;
R₃ is selected from hydrogen atom, or alkyl;
Rₐₐ is selected from hydrogen atom, or alkyl;
or, 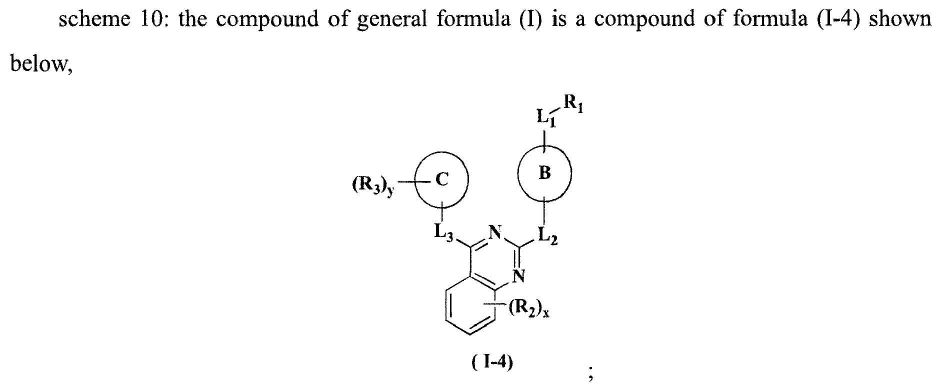
wherein, L₁ is selected from alkylene, -(CH₂)ₙ₁C(O)(CH₂)ₘ₁-, - (CH₂)ₙ₁C(O)(CH₂)ₘ₁NRₐₐ(CH₂)m₂-, or -(CH₂)ₙ₁S(O)ₘ₁-;
R₁ is selected from halogen, cyano, alkyl, alkoxy, heterocyclyl, or heteroaryl; wherein the alkyl is optionally further substituted with halogen or alkoxy; wherein the heterocyclyl is optionally further substituted with cyano;
when L₁ is -(CH₂)ₙ₁C(O)(CH₂)ₘ₁-, R₁ is alkyl, heterocyclyl, or heteroaryl; wherein the alkyl is optionally further substituted with halogen; wherein the heterocyclyl or heteroaryl is optionally further substituted with alkyl;
L₂ is -NR₄-;
R4 is selected from hydrogen atom, or alkyl;
L₃ is -NH-;
ring B is selected from 3-10 membered monocyclic heterocyclyl, 6-14 membered bridged heterocyclyl, or 6-14 membered fused heterocyclyl;
ring C is heteroaryl;
R₂ is selected from hydrogen atom, alkyl, alkoxy, halogen, cycloalkyl, heterocyclyl, aryl, heteroaryl, -(CH₂)ₙ₁OR_{cc}, or -(CH₂)ₙ₁NRₐₐR_{bb}; wherein the alkyl is optionally further substituted with heterocyclyl unsubstituted or substituted with alkyl; wherein the alkoxy is optionally further substituted with unsubstituted alkoxy, heterocyclyl unsubstituted or substituted with alkyl, or unsubstituted heteroaryl; wherein the heterocyclyl is optionally further substituted with unsubstituted alkyl, unsubstituted alkoxy, or amino substituted with alkyl; wherein the heteroaryl is optionally further substituted with alkyl unsubstituted or substituted with halogen, unsubstituted alkoxy, or unsubstituted cycloalkyl;
Rₐₐ and R_{bb} are identical or different and are each independently selected from hydrogen atom, or alkyl; wherein the alkyl is optionally further substituted with heterocyclyl unsubstituted or substituted with alkyl, or unsubstituted heteroaryl;
R_{cc} is heterocyclyl; wherein the heterocyclyl is optionally further substituted with unsubstituted alkyl;
R₃ is selected from hydrogen atom, alkyl, or hydroxyalkyl; 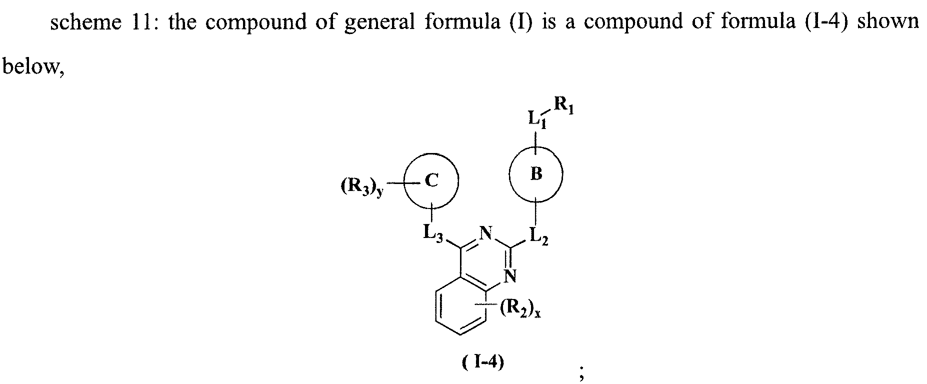
wherein, L₁ is selected from alkylene, -(CH₂)ₙ₁C(O)(CH₂)ₘ₁-, - (CH₂)ₙ₁C(O)(CH₂)ₘ₁NRₐₐ(CH₂)ₘ₂-, or -(CH₂)ₙ₁S(O)ₘ₁-;
R₁ is selected from halogen, cyano, alkyl, heterocyclyl, or heteroaryl; wherein the alkyl is optionally further substituted with halogen or alkoxy; wherein the heterocyclyl is optionally further substituted with cyano; wherein the heteroaryl is optionally further substituted with alkyl;
when L₁ is -(CH₂)ₙ₁C(O)(CH₂)ₘ₁-, R₁ is alkyl, heterocyclyl, or heteroaryl; wherein the alkyl is optionally further substituted with halogen; wherein the heterocyclyl or heteroaryl is optionally further substituted with alkyl;
L₂ is -NR₄-;
R₄ is selected from hydrogen atom, or alkyl;
L₃ is -NH-;
ring B is selected from 3-10 membered monocyclic heterocyclyl, 6-14 membered bridged heterocyclyl, or 6-14 membered fused heterocyclyl;
ring C is heteroaryl;
R₂ is selected from hydrogen atom, alkyl, alkoxy, halogen, cycloalkyl, heterocyclyl, aryl, or heteroaryl; wherein the heteroaryl is optionally further substituted with unsubstituted alkyl, unsubstituted alkoxy, or unsubstituted cycloalkyl;
R₃ is selected from hydrogen atom, alkyl, or hydroxyalkyl; 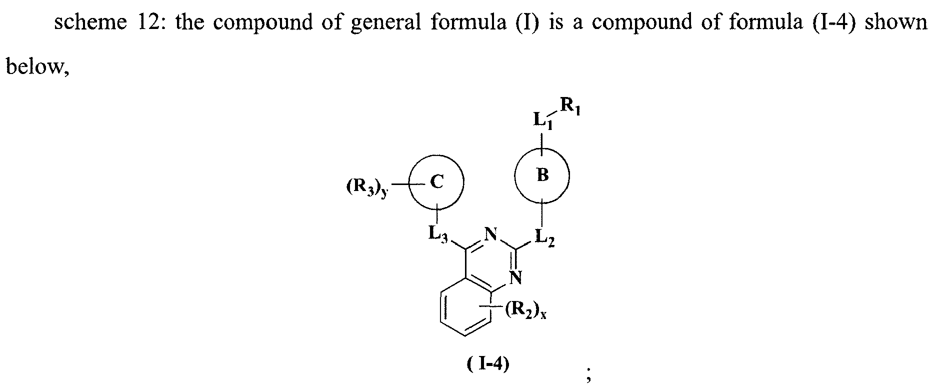
wherein, L₁ is selected from C₁-C₄ alkylene, -(CH₂)ₙ₁C(O)(CH₂)ₘ₁-, - (CH₂)ₙ₁C(O)(CH₂)ₘ₁NRₐₐ(CH₂)ₘ₂-, or -(CH₂)ₙ₁S(O)ₘ₁-;
R₁ is selected from halogen, cyano, C₁-C₃ alkyl, 4-6 membered monocyclic heterocyclyl with "heteroatom selected from one or more of N, O or S, and the number of the heteroatom being 1-3", or 5-6 membered monocyclic heteroaryl with "heteroatom selected from one or more of N, O or S, and the number of the heteroatom being 1-3"; the C₁-C₃ alkyl is optionally further substituted with halogen or C₁-C₃ alkoxy; wherein the heterocyclyl is optionally further substituted with cyano; wherein the heteroaryl is optionally further substituted with C₁-C₃ alkyl;
when L₁ is -(CH₂)ₙ₁C(O)(CH₂)ₘ₁-, then R₁ is C₁-C₃ alkyl, 4-6 membered monocyclic heterocyclyl with "heteroatom selected from one or more of N, O or S, and the number of the heteroatom being 1-3", or 5-6 membered monocyclic heteroaryl with "heteroatom selected from one or more of N, O or S, and the number of the heteroatom being 1-3"; wherein the C₁-C₃ alkyl is optionally further substituted with halogen; wherein the heterocyclyl or heteroaryl is optionally further substituted with C₁-C₃ alkyl;
L₂ is -NR₄-;
R₄ is selected from hydrogen atom, or C₁-C₃ alkyl;
L₃ is -NH-;
ring B is selected from 4-6 membered monocyclic heterocyclyl with "heteroatom selected from one or more of N, O or S, and the number of the heteroatom being 1-3", 7-10 membered bridged heterocyclyl with "heteroatom selected from one or more of N, O or S, and the number of the heteroatom being 1-3", or 7-10 membered fused heterocyclyl with "heteroatom selected from one or more of N, O or S, and the number of the heteroatom being 1-3";
ring C is 5-6 membered monocyclic heteroaryl with "heteroatom selected from one or more of N, O or S, and the number of the heteroatom being 1-3";
R₂ is selected from hydrogen atom, C₁-C₃ alkyl, C₁-C₃ alkoxy, halogen, cycloalkyl, 4-6 membered monocyclic heterocyclyl with "heteroatom selected from one or more of N, O or S, and the number of the heteroatom being 1-3", 6-10 membered aryl, or 5-6 membered heteroaryl with "heteroatom selected from one or more of N, O or S, and the number of the heteroatom being 1-3"; wherein the heteroaryl is optionally further substituted with unsubstituted C₁-C₃ alkyl, unsubstituted C₁-C₃ alkoxy, or unsubstituted 4-6 membered monocyclic heterocyclyl with "heteroatom selected from one or more of N, O or S, and the number of the heteroatom being 1-3";
R₃ is selected from hydrogen atom, C₁-C₃ alkyl, or C₁-C₃ hydroxyalkyl; 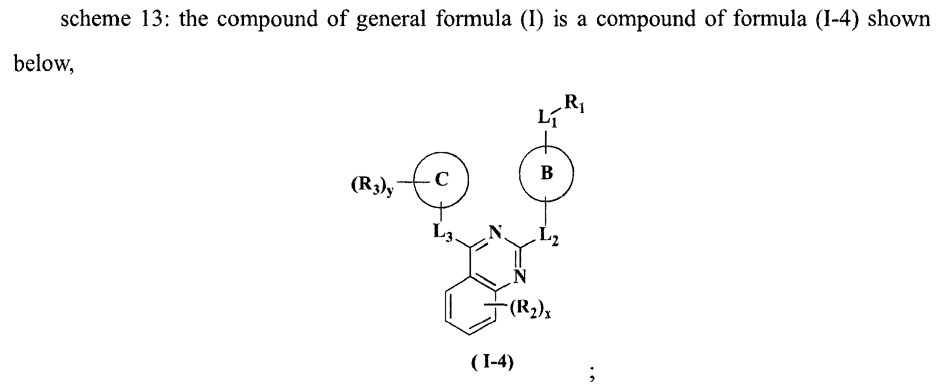
wherein, L₁ is alkylene, or -(CH₂)ₙ₁S(O)ₘ₁-;
R₁ is cyano, or heterocyclyl; wherein the heterocyclyl is optionally further substituted with cyano;
L₂ is -NH-;
L₃ is -NH-;
R₂ is selected from hydrogen atom, halogen, or alkoxy;
R₃ is selected from hydrogen atom, or alkyl.

7. The compound of general formula (I), the stereoisomer thereof or the pharmaceutically acceptable salt thereof according to any one of claims 1-6, wherein,
when L₁ is alkylene, the alkylene is preferably C₁-C₄ alkylene, more preferably -CH₂-, - (CH₂)₂-, -(CH₂)₃- or -(CH₂)₄-, more preferably -CH₂-, or -(CH₂)₂-;
and/or, when L₁ is cycloalkyl, the cycloalkyl is C₃-C₈ cycloalkyl, preferably C₃-C₆ cycloalkyl, more preferably 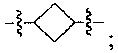
and/or, when L₁ is heterocyclyl, the heterocyclyl is 3-8 membered monocyclic heterocyclyl with "heteroatom selected from one or more of N, O or S, and the number of the heteroatom being 1-4", preferably 4-6 membered monocyclic heterocyclyl with "heteroatom selected from one or more of N, O or S, and the number of the heteroatom being 1-3", more preferably 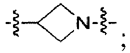
and/or, when L₁ is -(CH₂)ₙ₁C(O)(CH₂)ₘ₁-, the -(CH₂)ₙ₁C(O)(CH₂)ₘ₁- is -C(O)-, -C(O)CH₂-, or -CH₂C(O)-;
and/or, when L₁ is -(CH₂)ₙ₁C(O)(CH₂)ₘ₁NRₐₐ(CH₂)ₘ₂-, the - (CH₂)ₙ₁C(O)(CH₂)ₘ₁NRₐₐ(CH₂)ₘ₂- is -C(O)CH₂NH-, -C(O)CH₂N(CH₃)-, -C(O)CH₂NHCH₂-, - C(O)CH₂NH(CH₂)₂-, or -CH₂C(O)N(CH₃)-;
and/or, when L₁ is -NRₐₐ(CH₂)ₙ₁-, the -NRₐₐ(CH₂)ₙ₁- is -NH(CH₂)₂-;
and/or, when L₁ is -(CH₂)ₙ₁S(O)ₘ₁-, the -(CH₂)ₙ₁S(O)ₘ₁- is -S(O)₂-;
and/or, when L₁ is -(CH₂)ₙ₁S(O)ₘ₁NRₐₐ-, the -(CH₂)ₙ₁S(O)ₘ₁NRₐₐ- is -S(O)₂NH-;
and/or, the left end of L₁ is connected with ring B, and the right end of L₁ is connected with R₁;
and/or, when L₂ is -CRₐₐR_{bb}-, the -CRₐₐR_{bb}- is -CH₂- or -CH(OH)-;
and/or, when L₂ is -(CH₂)ₙ₁C(O)-, the -(CH₂)ₙ₁C(O)- is -C(O)-;
and/or, when L₂ is -NR₄-, the -NR₄- is preferably -NH- or -N(CH₃)-;
and/or, when L₂ is -(CH₂)ₙ₁S(O)ₘ₁-, the -(CH₂)ₙ₁S(O)ₘ₁- is -S(O)₂-;
and/or, the left end of L₂ is connected with ring A, and the right end of L₂ is connected with ring B;
and/or, when R₄ is alkyl, the alkyl is C₁-C₈ alkyl, preferably C₁-C₆ alkyl, more preferably C₁-C₃ alkyl, more preferably methyl, ethyl, propyl or isopropyl, further preferably methyl;
and/or, when L₃ is -NRₐₐ-, the -NRₐₐ- is -NH-;
and/or, when L₃ is -C(O)NRₐₐ-, the -C(O)NRₐₐ- is -C(O)NH-;
and/or, the left end of L₃ is connected with ring C, and the right end of L₃ is connected with ring B;
and/or, when the ring A is 6-14 membered fused heteroaryl, the 6-14 membered fused heteroaryl is 6-14 membered fused heteroaryl with "heteroatom selected from one or more of N, O or S, and the number of the heteroatom being 1-4", preferably 5,6 fused heteroaryl with "heteroatom selected from one or more of N, O or S, and the number of the heteroatom being 1 - 4", or 6,6 fused heteroaryl with "heteroatom selected from one or more of N, O or S, and the number of the heteroatom being 1-4"; wherein the 5,6 fused heteroaryl is preferably thienopyrimidinyl or pyrazolopyrimidinyl, benzopyrrolyl, pyrrolopyridyl, imidazolopyridyl, triazolopyridyl, imidazolopyridazinyl, pyrrolopyrimidinyl, pyrazolopyrimidinyl, imidazolopyrimidinyl, triazolopyrimidinyl, thienopyrimidinyl, thiazolopyrimidinyl, pyrrolopyrazinyl, pyrazolopyrazinyl, imidazolopyrazinyl, triazolopyrazinyl, pyrrolotriazinyl, imidazolotriazinyl or imidazolopyrazinonyl, more preferably 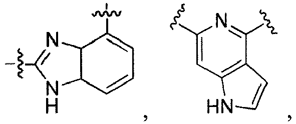 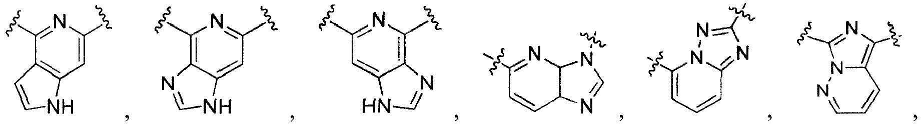 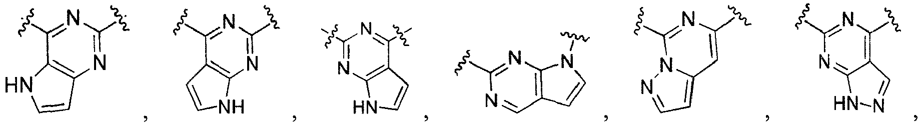 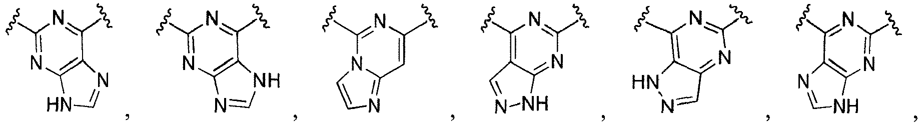 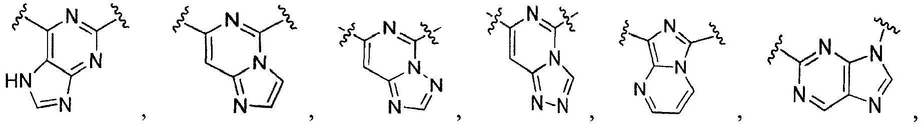 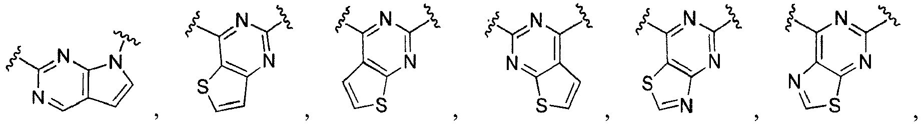 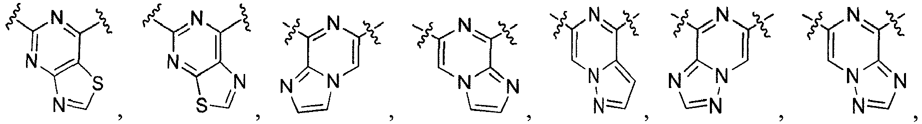 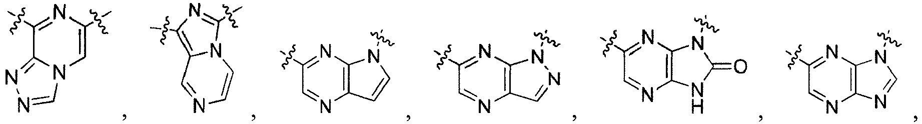 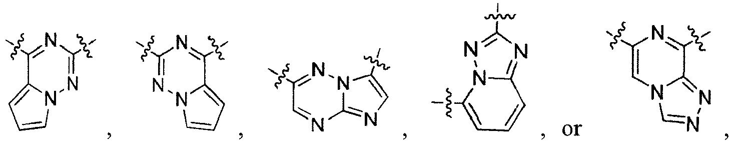 further preferably 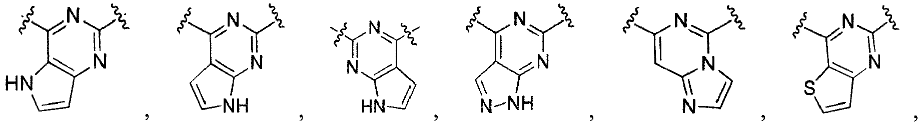 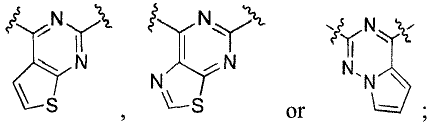 wherein the 6,6 fused heteroaryl is preferably isoquinolinyl, naphthyridinyl, pyridopyrazinyl, pyridopyrimidinyl, quinazolinyl, pteridinyl, quinoxalinyl, dihydropyranopyrimidinyl, dihydrodioxinopyrimidinyl, 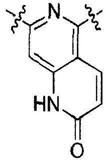 or 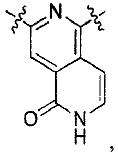 more preferably 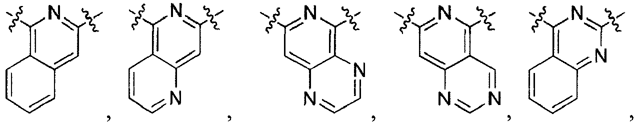 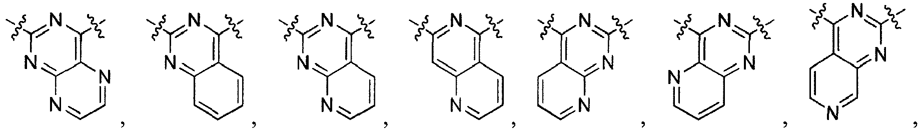 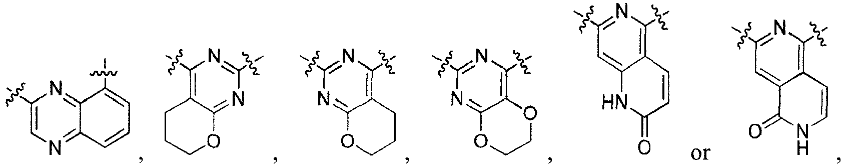 further preferably 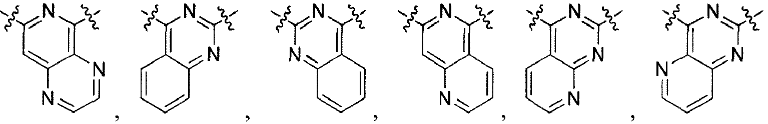 or 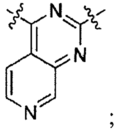 the left end of ring A is connected with L₃, and the right end of ring A is connected with L₂;
and/or, when the ring B is 3-10 membered monocyclic heterocyclyl, the 3-10 membered monocyclic heterocyclyl is 3-8 membered monocyclic heterocyclyl with "heteroatom selected from one or more of N, O or S, and the number of the heteroatom being 1-4", preferably 4-6 membered monocyclic heterocyclyl with "heteroatom selected from one or more of N, O or S, and the number of the heteroatom being 1-3", more preferably 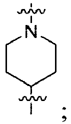
and/or, when the ring B is 6-14 membered bridged heterocyclyl, the 6-14 membered bridged heterocyclyl is 6-14 membered bridged heterocyclyl with "heteroatom selected from one or more of N, O or S, and the number of the heteroatom being 1-4", preferably 7-10 membered bridged heterocyclyl with "heteroatom selected from one or more of N, O or S, and the number of the heteroatom being 1-3", more preferably 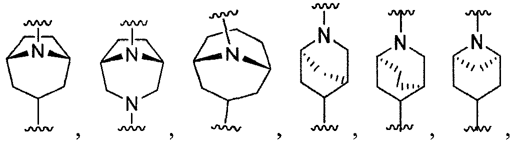 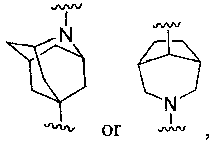 further preferably 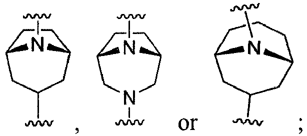
and/or, when the ring B is 6-14 membered fused heterocyclyl, the 6-14 membered fused heterocyclyl is 6-14 membered fused heterocyclyl with "heteroatom selected from one or more of N, O or S, and the number of the heteroatom being 1-4", preferably 7-10 membered fused heterocyclyl with "heteroatom selected from one or more of N, O or S, and the number of the heteroatom being 1-3", more preferably 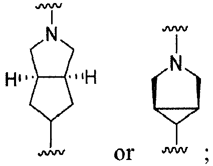
and/or, when the ring B is 6-14 membered spiro heterocyclyl, the 6-14 membered spiro heterocyclyl is 6-14 membered spiro heterocyclyl with "heteroatom selected from one or more of N, O or S, and the number of the heteroatom being 1-4", preferably 7-10 membered spiro heterocyclyl with "heteroatom selected from one or more of N, O or S, and the number of the heteroatom being 1-3", more preferably 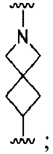
and/or, the upper end of ring B is connected with L₁, and the lower end of ring B is connected with L₂;
and/or, when the ring C is heteroaryl, the heteroaryl is 6-14 membered heteroaryl with "heteroatom selected from one or more of N, O or S, and the number of the heteroatom being 1-4", preferably 5-6 membered monocyclic heteroaryl with "heteroatom selected from one or more of N, O or S, and the number of the heteroatom being 1-3", or 8-10 membered fused heteroaryl with "heteroatom selected from one or more of N, O or S, and the number of the heteroatom being 1-3"; wherein the 5-6 membered monocyclic heteroaryl is preferably pyrazolyl, imidazolyl, thiazolyl, triazolyl, pyridazinyl, pyrimidinyl or pyrazinyl,, more preferably 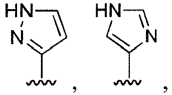 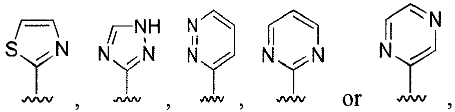 further preferably 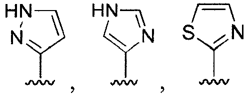 or 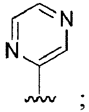 wherein the 8-10 membered fused heteroaryl is preferably indazolyl, or pyrazolopyridyl, more preferably 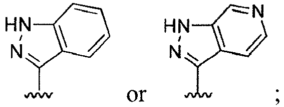
and/or, when R₁ is alkyl, the alkyl is C₁-C₈ alkyl, preferably C₁-C₆ alkyl, further preferably C₁-C₃ alkyl, further preferably methyl, ethyl, propyl or isopropyl, further preferably methyl or ethyl;
and/or, when R₁ is haloalkyl, the haloalkyl is C₁-C₈ haloalkyl, more preferably C₁-C₆ haloalkyl, further preferably C₁-C₃ haloalkyl, further preferably -CHF₂ or CF₃;
and/or, when R₁ is alkoxy, the alkoxy is C₁-C₈ alkoxy, more preferably C₁-C₆ alkoxy, further preferably C₁-C₃ alkoxy, further preferably methoxy, ethoxy, propoxy or isopropoxy, further preferably methoxy or ethoxy;
and/or, when R₁ is halogen, the halogen is fluorine;
and/or, when R₁ is cycloalkyl, the cycloalkyl is C₃-C₈ cycloalkyl, preferably C₃-C₆ cycloalkyl, more preferably 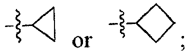
and/or, when R₁ is heterocyclyl, the heterocyclyl is 3-8 membered monocyclic heterocyclyl with "heteroatom selected from one or more of N, O or S, and the number of the heteroatom being 1-4", preferably 4-6 membered monocyclic heterocyclyl with "heteroatom selected from one or more of N, O or S, and the number of the heteroatom being 1-3", more preferably 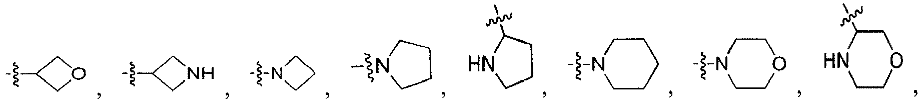 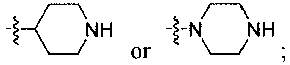
and/or, when R₁ is aryl, the aryl is 6-10 membered aryl, preferably phenyl;
and/or, when R₁ is heteroaryl, the heteroaryl is 5-10 membered heteroaryl with "heteroatom selected from one or more of N, O or S, and the number of the heteroatom being 1-4", preferably 5-6 membered heteroaryl with "heteroatom selected from one or more of N, O or S, and the number of the heteroatom being 1-3", more preferably 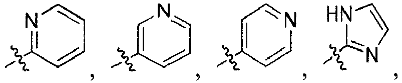 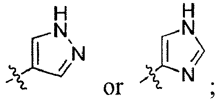
and/or, when R₁ is alkyl, amino, heterocyclyl, aryl or heteroaryl, wherein the alkyl, amino, heterocyclyl, aryl or heteroaryl is optionally further substituted with halogen, then the halogen is fluorine;
and/or, when R₁ is amino, heterocyclyl, aryl or heteroaryl, wherein the amino, heterocyclyl, aryl or heteroaryl is optionally further substituted with alkyl, then the alkyl is C₁-C₈ alkyl, preferably C₁-C₆ alkyl, further preferably C₁-C₃ alkyl, further preferably methyl, ethyl, propyl or isopropyl, further preferably methyl;
and/or, when R₁ is alkyl, amino, heterocyclyl, aryl or heteroaryl, wherein the alkyl, amino, heterocyclyl, aryl or heteroaryl is optionally further substituted with alkoxy, then the alkoxy is C₁-C₈ alkoxy, preferably C₁-C₆ alkoxy, further preferably C₁-C₃ alkoxy, further preferably methoxy, ethoxy, propoxy or isopropoxy, further preferably methoxy;
and/or, when R₂ is alkyl, the alkyl is C₁-C₈ alkyl, preferably C₁-C₆ alkyl, further preferably C₁-C₃ alkyl, further preferably methyl, ethyl, propyl or isopropyl, further preferably methyl or ethyl;
and/or, when R₂ is alkoxy, the alkoxy is C₁-C₈ alkoxy, preferably C₁-C₆ alkoxy, further preferably C₁-C₃ alkoxy, further preferably methoxy, ethoxy, propoxy or isopropoxy, further preferably methoxy or ethoxy;
and/or, when R₂ is halogen, the halogen is fluorine, chlorine or bromine;
and/or, when R₂ is cycloalkyl, the cycloalkyl is C₃-C₈ cycloalkyl, preferably C₃-C₆ cycloalkyl, more preferably 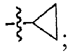
and/or, when R₂ is heterocyclyl, the heterocyclyl is 3-8 membered monocyclic heterocyclyl with "heteroatom selected from one or more of N, O or S, and the number of the heteroatom being 1-4", preferably 4-6 membered monocyclic heterocyclyl with "heteroatom selected from one or more of N, O or S, and the number of the heteroatom being 1-3", more preferably 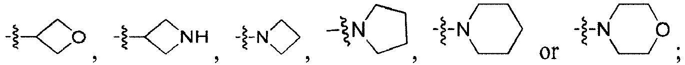
and/or, when R₂ is aryl, the aryl is 6-10 membered aryl, preferably phenyl;
and/or, when R₂ is heteroaryl, the heteroaryl is 5-10 membered heteroaryl with "heteroatom selected from one or more of N, O or S, and the number of the heteroatom being 1-4", preferably 5-6 membered heteroaryl with "heteroatom selected from one or more of N, O or S, and the number of the heteroatom being 1-3", more preferably 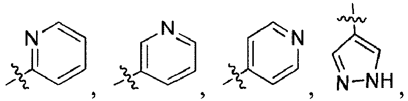 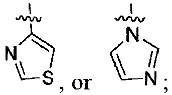
and/or, when R₂ is -(CH₂)ₙ₁SRₐₐ, the -(CH₂)ₙ₁SRₐₐ is 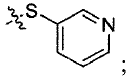 and/or, when R₂ is -(CH₂)ₙ₁NRₐₐR_{bb}, the -(CH₂)ₙ₁NRₐₐR_{bb} is 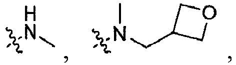 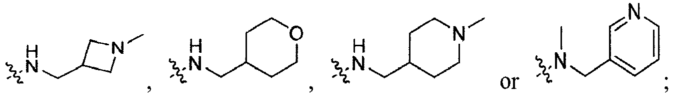
and/or, when R₂ is alkyl, and the alkyl is optionally further substituted with unsubstituted alkoxy, then the alkoxy is C₁-C₈ alkoxy, preferably C₁-C₆ alkoxy, further preferably C₁-C₃ alkoxy, further preferably methoxy, ethoxy, propoxy or isopropoxy, further preferably methoxy;
and/or, when R₂ is alkyl, and the alkyl is optionally further substituted with substituted or unsubstituted heterocyclyl, then the heterocyclyl is 3-8 membered monocyclic heterocyclyl with "heteroatom selected from one or more of N, O or S, and the number of the heteroatom being 1-4", preferably 4-6 membered monocyclic heterocyclyl with "heteroatom selected from one or more of N, O or S, and the number of the heteroatom being 1-3", more preferably 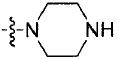 or 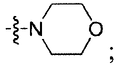
and/or, when R₂ is alkyl, and the alkyl is optionally further substituted with substituted or unsubstituted heterocyclyl, then the heterocyclyl is substituted with alkyl, wherein the alkyl is C₁-C₈ alkyl, preferably C₁-C₆ alkyl, further preferably C₁-C₃ alkyl, further preferably methyl, ethyl, propyl or isopropyl, further preferably methyl;
and/or, when R₂ is alkoxy, and the alkoxy is optionally further substituted with unsubstituted alkoxy, then the unsubstituted alkoxy is C₁-C₈ alkoxy, preferably C₁-C₆ alkoxy, further preferably C₁-C₃ alkoxy, further preferably methoxy, ethoxy, propoxy or isopropoxy, further preferably methoxy;
and/or, when R₂ is alkoxy, and the alkoxy is optionally further substituted with substituted or unsubstituted heterocyclyl, then the heterocyclyl is 3-8 membered monocyclic heterocyclyl with "heteroatom selected from one or more of N, O or S, and the number of the heteroatom being 1-4", preferably 4-6 membered monocyclic heterocyclyl with "heteroatom selected from one or more of N, O or S, and the number of the heteroatom being 1-3", more preferably 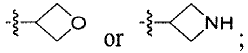
and/or, when R₂ is alkoxy, and the alkoxy is optionally further substituted with substituted or unsubstituted heterocyclyl, then the heterocyclyl is substituted with alkyl, wherein the alkyl is C₁-C₈ alkyl, preferably C₁-C₆ alkyl, further preferably C₁-C₃ alkyl, further preferably methyl, ethyl, propyl or isopropyl, further preferably methyl;
and/or, when R₂ is alkoxy, and the alkoxy is optionally further substituted with unsubstituted heteroaryl, then the heteroaryl is 5-10 membered heteroaryl with "heteroatom selected from one or more of N, O or S, and the number of the heteroatom being 1-4", preferably 5-6 membered heteroaryl with "heteroatom selected from one or more of N, O or S, and the number of the heteroatom being 1-3", more preferably 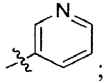
and/or, when R₂ is amino, and the amino is optionally further substituted with unsubstituted alkyl, then the alkyl is C₁-C₈ alkyl, preferably C₁-C₆ alkyl, further preferably C₁-C₃ alkyl, further preferably methyl, ethyl, propyl or isopropyl, further preferably methyl;
and/or, when R₂ is heterocyclyl, and the heterocyclyl is optionally further substituted with unsubstituted alkyl, then the alkyl is C₁-C₈ alkyl, preferably C₁-C₆ alkyl, further preferably C₁-C₃ alkyl, further preferably methyl, ethyl, propyl or isopropyl, further preferably methyl;
and/or, when R₂ is heterocyclyl, and the heterocyclyl is optionally further substituted with unsubstituted alkoxy, then the alkoxy is C₁-C₈ alkoxy, preferably C₁-C₆ alkoxy, further preferably C₁-C₃ alkoxy, further preferably methoxy, ethoxy, propoxy or isopropoxy, further preferably methoxy;
and/or, when R₂ is heterocyclyl, and the heterocyclyl is optionally further substituted with substituted or unsubstituted amino, then the amino is substituted with alkyl, wherein the alkyl is C₁-C₈ alkyl, preferably C₁-C₆ alkyl, further preferably C₁-C₃ alkyl, further preferably methyl, ethyl, propyl or isopropyl, further preferably methyl;
and/or, when R₂ is heteroaryl, and the heteroaryl is optionally further substituted with substituted or unsubstituted alkyl, then the alkyl is C₁-C₈ alkyl, preferably C₁-C₆ alkyl, further preferably C₁-C₃ alkyl, further preferably methyl, ethyl, propyl or isopropyl, further preferably methyl;
and/or, when R₂ is heteroaryl, and the heteroaryl is optionally further substituted with substituted or unsubstituted alkyl, then the alkyl is substituted with halogen, wherein the halogen is fluorine;
and/or, when R₂ is heteroaryl, and the heteroaryl is optionally further substituted with unsubstituted alkoxy, then the alkoxy is C₁-C₈ alkoxy, preferably C₁-C₆ alkoxy, further preferably C₁-C₃ alkoxy, further preferably methoxy, ethoxy, propoxy or isopropoxy, further preferably methoxy;
and/or, when R₂ is heteroaryl, and the heteroaryl is optionally further substituted with unsubstituted cycloalkyl, then the cycloalkyl is C₃-C₈ cycloalkyl, preferably C₃-C₆ cycloalkyl, more preferably 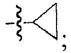
and/or, when R_{cc} is heterocyclyl, the heterocyclyl is preferably 3-8 membered monocyclic heterocyclyl with "heteroatom selected from one or more of N, O or S, and the number of the heteroatom being 1-4", more preferably 4-6 membered monocyclic heterocyclyl with "heteroatom selected from one or more of N, O or S, and the number of the heteroatom being 1-*3", e.g.,* 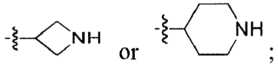
and/or, when R_{cc} is heterocyclyl, and the heterocyclyl is optionally substituted with unsubstituted alkyl, then the alkyl is preferably C₁-C₈ alkyl, more preferably C₁-C₆ alkyl, further preferably C₁-C₃ alkyl, e.g., methyl, ethyl, propyl or isopropyl, further preferably methyl;
and/or, when R₃ is alkyl, the alkyl is C₁-C₈ alkyl, preferably C₁-C₆ alkyl, further preferably C₁-C₃ alkyl, further preferably methyl, ethyl, propyl or isopropyl, further preferably methyl or ethyl;
and/or, when R₃ is hydroxyalkyl, the alkyl is C₁-C₈ hydroxyalkyl, preferably C₁-C₆ hydroxyalkyl, further preferably C₁-C₃ hydroxyalkyl, further preferably hydroxymethyl, hydroxyethyl, hydroxypropyl or hydroxyisopropyl, further preferably hydroxymethyl;
and/or, when R₃ is haloalkyl, the haloalkyl is C₁-C₈ haloalkyl, preferably C₁-C₆ haloalkyl, further preferably C₁-C₃ haloalkyl, further preferably -CHF₂ or CF₃;
and/or, when R₃ is alkoxy, the alkoxy is C₁-C₈ alkoxy, preferably C₁-C₆ alkoxy, further preferably C₁-C₃ alkoxy, further preferably methoxy, ethoxy, propoxy or isopropoxy, further preferably methoxy;
and/or, when R₃ is halogen, the halogen is fluorine;
and/or, when R₃ is cycloalkyl, the cycloalkyl is C₃-C₈ cycloalkyl, preferably C₃-C₆ cycloalkyl, more preferably 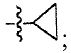
and/or, when R₃ is heterocyclyl, the heterocyclyl is 3-8 membered monocyclic heterocyclyl with "heteroatom selected from one or more of N, O or S, and the number of the heteroatom being 1-4", preferably 4-6 membered monocyclic heterocyclyl with "heteroatom selected from one or more of N, O or S, and the number of the heteroatom being 1-3", more preferably 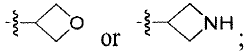
and/or, when R₃ is alkyl, and the alkyl is optionally further substituted with halogen, then the halogen is fluorine;
and/or, when R₃ is alkyl, and the alkyl is optionally further substituted with unsubstituted alkoxy, then the alkoxy is C₁-C₈ alkoxy, preferably C₁-C₆ alkoxy, further preferably C₁-C₃ alkoxy, further preferably methoxy, ethoxy, propoxy or isopropoxy, further preferably methoxy;
and/or, when R₃ is alkoxy, and the alkoxy is optionally further substituted with unsubstituted alkyl, then the alkyl is C₁-C₈ alkyl, preferably C₁-C₆ alkyl, further preferably C₁-C₃ alkyl, further preferably methyl, ethyl, propyl or isopropyl, further preferably methyl;
and/or, when R₃ is cycloalkyl, the cycloalkyl is substituted with hydroxyl;
and/or, when R₃ is heterocyclyl, and the heterocyclyl is optionally further substituted with unsubstituted alkyl, then the alkyl is C₁-C₈ alkyl, preferably C₁-C₆ alkyl, further preferably Ci-C₃ alkyl, further preferably methyl, ethyl, propyl or isopropyl, further preferably methyl;
and/or, when R₃ is -(CH₂)ₙ₁C(O)Rₐₐ, the -(CH₂)ₙ₁(O)Rₐₐ is 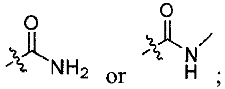
and/or, when R₃ is -(CH₂)ₙC(O)NRₐₐR_{bb}, the -(CH₂)ₙ₁C(O)NRₐₐR_{bb} is 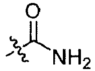 or 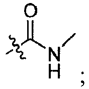
and/or, when R₃ is -(CH₂)ₙ₁S(O)ₘ₁Rₐₐ, the -(CH₂)ₙ₁S(O)ₘ₁Rₐₐ is 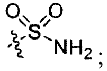
and/or, when R₃ is -(CH₂)ₙ₁S(O)ₘ₁NRₐₐR_{bb}, the -(CH₂)ₙ₁S(O)ₘ₁NRₐₐR_{bb} is 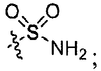
and/or, when Rₐₐ and R_{bb} are independently alkyl, the alkyl is C₁-C₈ alkyl, preferably C₁-C₆ alkyl, further preferably C₁-C₃ alkyl, further preferably methyl, ethyl, propyl or isopropyl, further preferably methyl;
and/or, when Rₐₐ and R_{bb} are independently heteroaryl, the heteroaryl is 5-10 membered heteroaryl with "heteroatom selected from one or more of N, O or S, and the number of the heteroatom being 1-4", preferably 5-6 membered heteroaryl with "heteroatom selected from one or more of N, O or S, and the number of the heteroatom being 1-3", more preferably 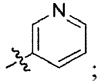
and/or, when Rₐₐ and R_{bb} are independently alkyl, and the alkyl is optionally further substituted with substituted or unsubstituted heterocyclyl, then the heterocyclyl is 3-8 membered monocyclic heterocyclyl with "heteroatom selected from one or more of N, O or S, and the number of the heteroatom being 1-4", preferably 4-6 membered monocyclic heterocyclyl with "heteroatom selected from one or more of N, O or S, and the number of the heteroatom being 1-3", more preferably 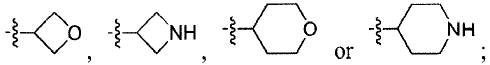
and/or, when Rₐₐ and R_{bb} are independently alkyl, and the alkyl is optionally further substituted with substituted or unsubstituted heterocyclyl, then the heterocyclyl is substituted with alkyl, the alkyl is C₁-C₈ alkyl, preferably C₁-C₆ alkyl, further preferably C₁-C₃ alkyl, further preferably methyl, ethyl, propyl or isopropyl, further preferably methyl;
and/or, when Rₐₐ and R_{bb} are independently alkyl, and the alkyl is optionally further substituted with unsubstituted heteroaryl, then the heteroaryl is 5-10 membered heteroaryl with "heteroatom selected from one or more of N, O or S, and the number of the heteroatom being 1-4", preferably 5-6 membered heteroaryl with "heteroatom selected from one or more of N, O or S, and the number of the heteroatom being 1-3", more preferably 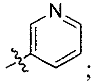
and/or, when Rₐₐ and R_{bb} are independently amino, and the amino is optionally further substituted with unsubstituted alkyl, then the alkyl is C₁-C₈ alkyl, preferably C₁-C₆ alkyl, further preferably C₁-C₃ alkyl, further preferably methyl, ethyl, propyl or isopropyl, further preferably methyl.

8. The compound of general formula (I), the stereoisomer thereof or the pharmaceutically acceptable salt thereof according to claim 7, wherein, -L₁- is bond, -CH₂-, -(CH₂)₂-, 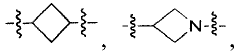 -C(O)-, -C(O)CH₂-, -CH₂C(O)-, - C(O)CH₂NH-, -C(O)CH₂N(CH₃)-, -C(O)CH₂NHCH₂-, -C(O)CH₂NH(CH₂)₂-, - CH₂C(O)N(CH₃)-, -NH(CH₂)₂-, -S(O)₂-, or -S(O)₂NH-, preferably bond, -CH₂-, -(CH₂)₂-, -C(O)-, -C(O)CH₂-, -C(O)CH₂NH-, -C(O)CH₂N(CH₃)-, -C(O)CH₂NH(CH₂)₂-, -CH₂C(O)N(CH₃)-, or - S(O)₂-; the left end of L₁ is connected with ring B, and the right end of L₁ is connected with R₁;
and/or, L₂ is bond, oxygen atom, -CH₂-, -CH(OH)-, -C(O)-, -NH-, -N(CH₃)- or -S(O)₂-, preferably bond, -NH-, or -N(CH₃)-; the left end of L₂ is connected with ring A, and the right end of L₂ is connected with ring B;
and/or, L₃ is bond, -NH- or -C(O)NH-, preferably -NH-; the left end of L₃ is connected with ring C, and the right end of L₃ is connected with ring B;
and/or, the ring A is the following group, 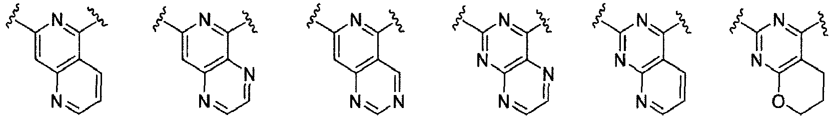 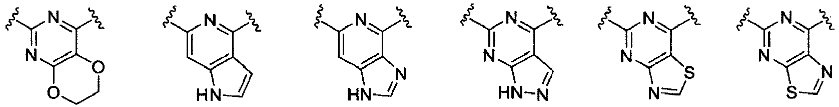 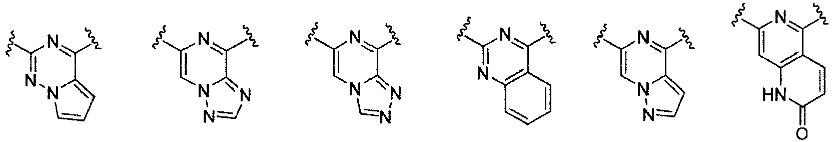 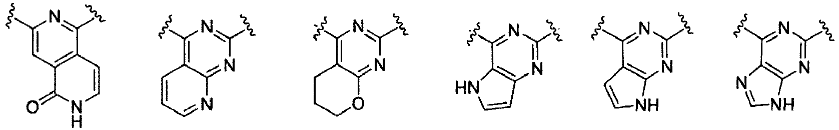 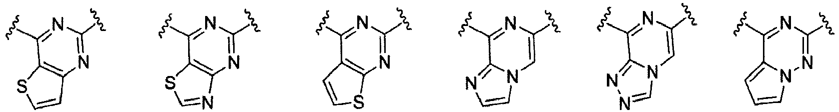 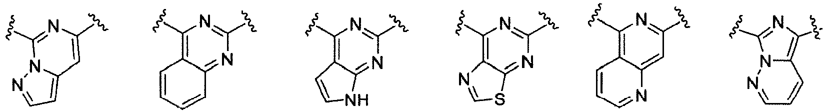 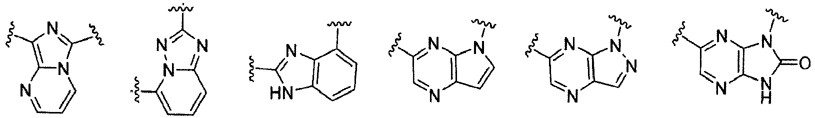 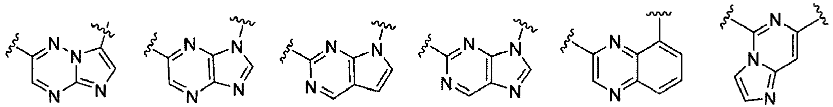 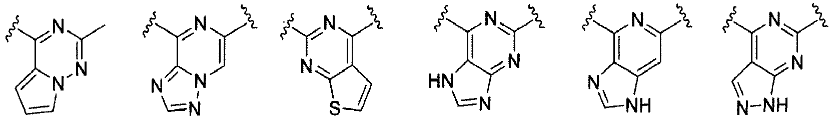 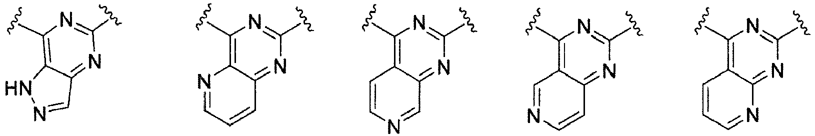 or 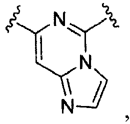 preferably 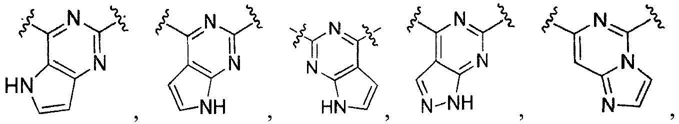 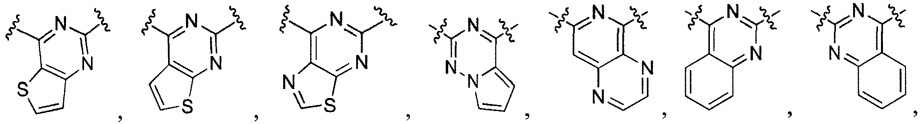 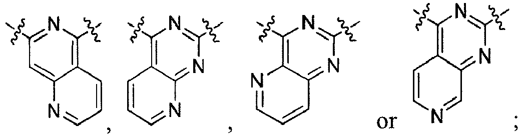 the left end of ring A is connected with L₃, and the right end of ring A is connected with L₂;
and/or, the ring B is the following group, 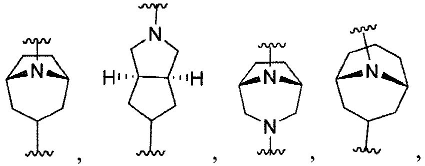 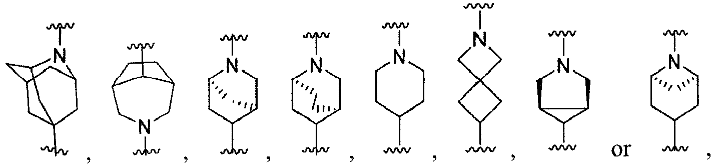 preferably 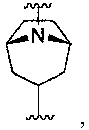 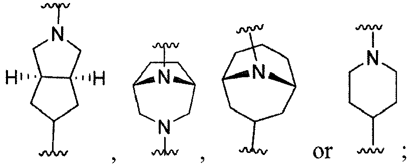 the upper end of ring B is connected with L₁, and the lower end of ring B is connected with L₂
and/or, the ring C is the following group, 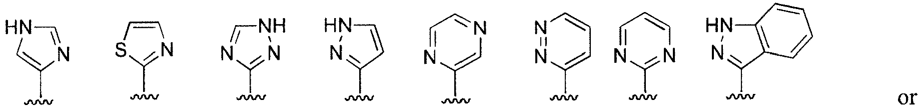 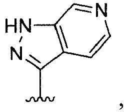 preferably 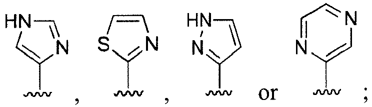 and/or, R₁ is hydrogen atom, methyl, ethyl, fluorine, methoxy, ethoxy, phenyl, cyano, -CHF₂, -CH₂CH₂CN, -CH₂CH₂F, -NHCH₂CH₂CN, 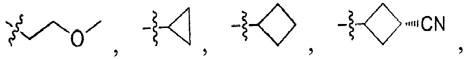 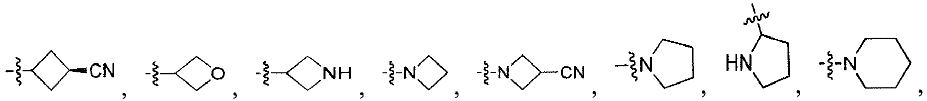 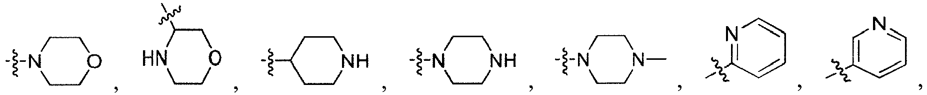 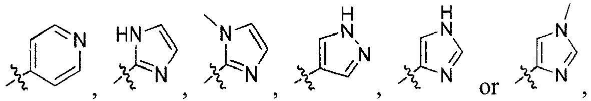 preferably hydrogen atom, methyl, ethyl, fluorine, cyano, -CHF₂, -CH₂CH₂CN, -CH₂CH₂F, 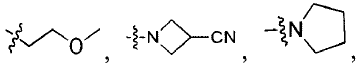 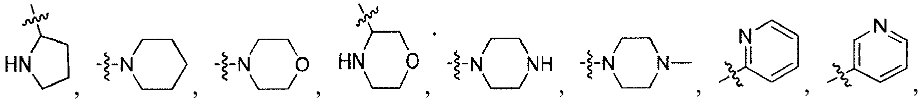 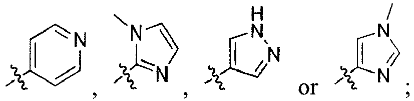
and/or, R₂ is hydrogen atom, fluorine, chlorine, bromine, amino, hydroxyl, cyano, methyl, methoxy, 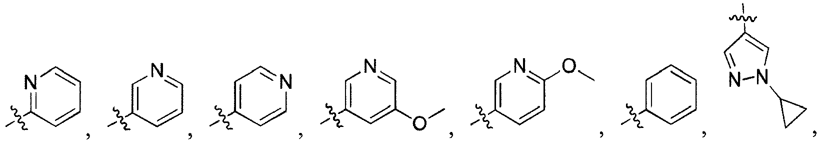 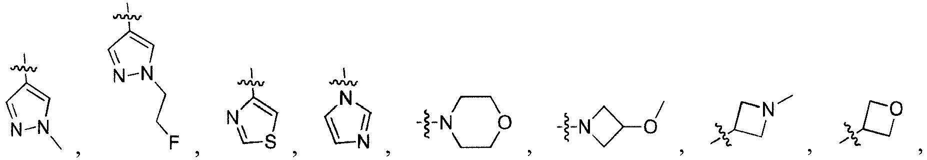 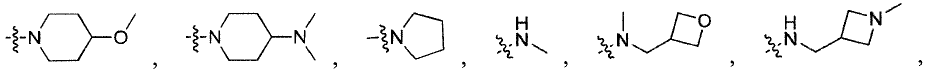 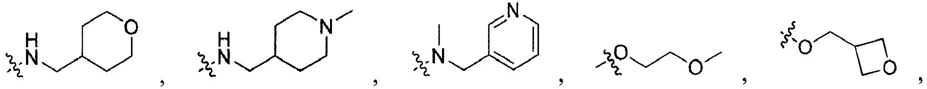 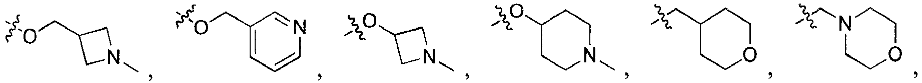 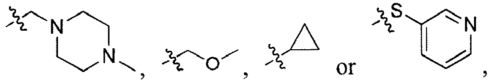 preferably hydrogen atom, fluorine, chlorine, bromine, methyl, methoxy, 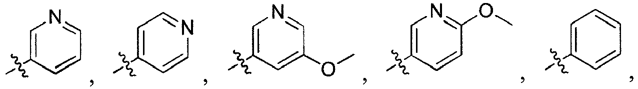 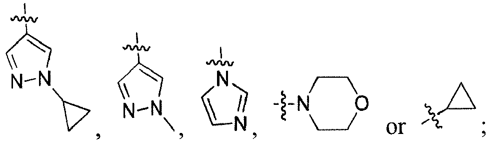
and/or, R₃ is hydrogen atom, methyl, ethyl, fluorine, cyano, -CHF₂, CF₃, 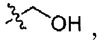 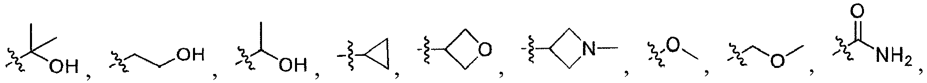 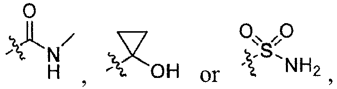 preferably hydrogen atom, methyl, ethyl, fluorine, cyano, 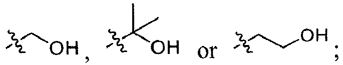
and/or, structure -L₁-R₁ is preferably 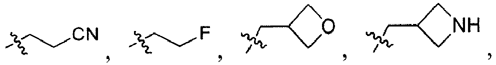 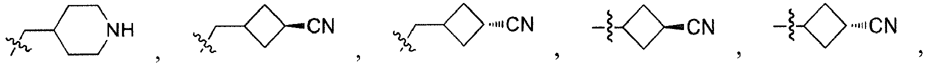 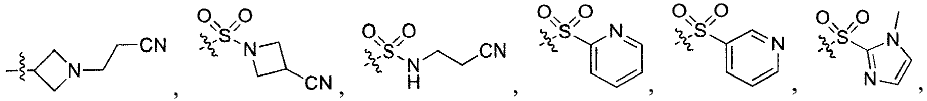 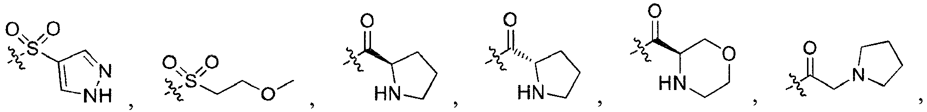 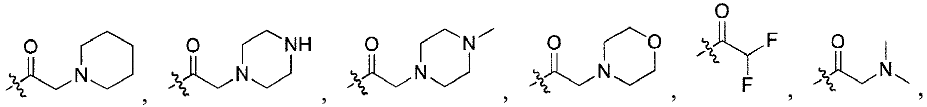 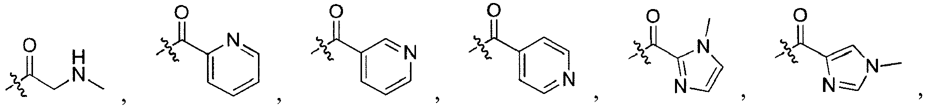 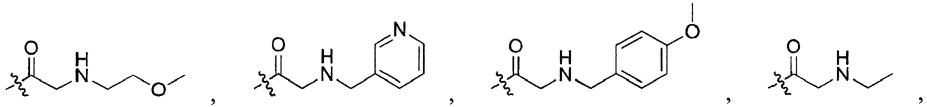 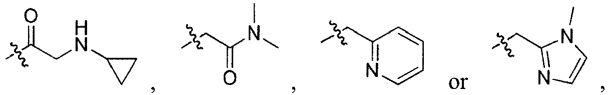 more preferably

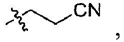

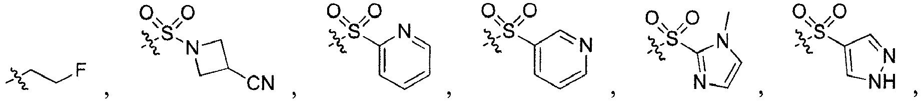

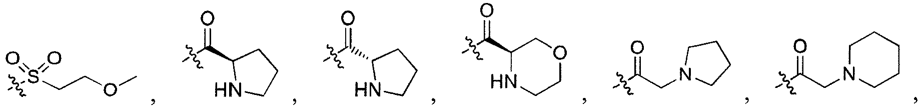

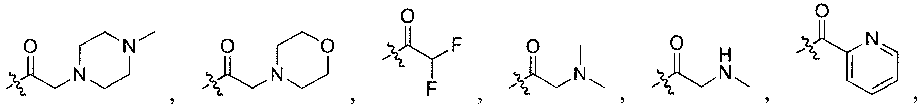

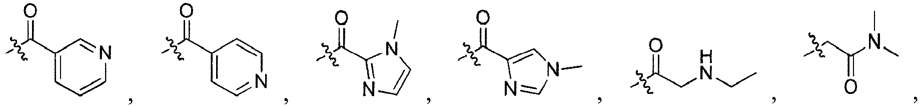

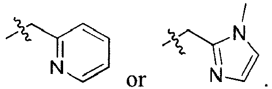

9. The compound of general formula (I), the stereoisomer thereof or the pharmaceutically acceptable salt thereof according to claim 1, wherein,
L₁ is selected from bond, alkylene, alkenylene, alkynyl, cycloalkyl, heterocyclyl, - (CH₂)ₙ₁C(O)(CH₂)ₘ₁-, -(CH₂)ₙ₁C(O)(CH₂)ₘ₁NRₐₐ(CH₂)ₘ₂-, -NRₐₐ(CH₂)ₙ₁-, -(CH₂)ₙ₁S(O)ₘ₁- or - (CH₂)ₙ₁S(O)ₘ₁NRₐₐ-;
L₂ is selected from bond, oxygen atom, sulfur atom, -CRₐₐR_{bb}-, -(CH₂)ₙ₁C(O)-, -NR₄- or - (CH₂)ₙ₁S(O)ₘ₁-;
L₃ is selected from bond, -NRₐₐ- or -C(O)NRₐₐ-;
ring A is selected from 6-14 membered heteroaryl, wherein the 6-14 membered heteroaryl is selected from 6-14 membered fused heteroaryl; preferably 5,5 fused heteroaryl, 5,6 fused heteroaryl or 6,6 fused heteroaryl;
ring B is selected from 3-10 membered monocyclic heterocyclyl, 6-14 membered bridged heterocyclyl or 6-14 membered fused heterocyclyl;
ring C is selected from heteroaryl;
R₁ is selected from hydrogen atom, deuterium atom, oxo, alkyl, deuterated alkyl, haloalkyl, alkoxy, haloalkoxy, hydroxyalkyl, halogen, amino, nitro, hydroxyl, cyano, alkenyl, alkynyl, cycloalkyl, heterocyclyl, oxoheterocyclyl, thioxoheterocyclyl, aryl, heteroaryl, -(CH₂)ₙ₁Rₐₐ, - (CH₂)ₙ₁ORₐₐ, -NRₐₐC(O)(CH₂)ₙ₁ORₐₐ, -NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, -(CH₂)ₙ₁C(O)NRₐₐR_{bb}, - (CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, alkyl, haloalkyl, halogen, amino, oxo, nitro, cyano, hydroxyl, alkenyl, alkynyl, alkoxy, haloalkoxy, hydroxyalkyl, cycloalkyl, heterocyclyl, aryl, heteroaryl, -(CH₂)ₙ₁-, - (CH₂)ₙ₁R_{cc}, -(CH₂)ₙ₁OR_{cc}, -(CH₂)ₙ₁SR_{cc}, -(CH₂)ₙ₁(O)R_{cc}, -(CH₂)ₙ₁C(O)OR_{cc}, - (CH₂)ₙ₁S(O)ₘ₁R_{cc}, -(CH₂)ₙ₁NR_{cc}R_{dd}, -(CH₂)ₙ₁C(O)NR_{cc}R_{dd}, -(CH₂)ₙ₁NR_{cc}C(O)R_{dd} or - (CH₂)ₙ₁NR_{cc}S(O)ₘ₁R_{dd};
R₂ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl, heteroaryl, -(CH₂)ₙ₁Rₐₐ, - (CH₂)ₙ₁O(CH₂)ₘ₁Rₐₐ, -(CH₂)ₙ₁ORₐₐ, -(CH₂)ₙ₁NRₐₐ(CH₂)ₘ₁Rₐₐ, -NRₐₐC(O)(CH₂)ₙ₁ORₐₐ, - NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁S(O)ₘ₁NRₐₐR_{bb}, -(CH₂)ₙ₁P(O)ₘ₂RₐₐR_{bb}, -(CH₂)ₙ₁NRₐₐR_{bb}, -(CH₂)ₙ₁S-, - (CH₂)ₙ₁C(O)NRₐₐR_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl and substituted, unsubstituted heteroaryl, - (CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc}, - (CH₂)ₙ₁S(O)ₘ₁R_{cc}, -(CH₂)ₙ₁S(O)ₘ₁NR_{cc}R_{dd}, -(CH₂)ₙ₁NR_{cc}R_{dd}, -(CH₂)ₙC(O)NR_{cc}R_{dd}, - (CH₂)ₙ₁C(O)NR_{cc}S(O)ₘ₁R_{dd}, -(CH₂)ₙ₁NR_{cc}S(O)ₘ₁R_{dd} or -(CH₂)ₙ₁NR_{cc}S(O)ₘ₁R_{dd};
R₃ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl, heteroaryl, -(CH₂)ₙ₁Rₐₐ, - (CH₂)ₙ₁ORₐₐ, -NRₐₐC(O)(CH₂)ₙ₁ORₐₐ, -NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁S(O)ₘ₁NRₐₐR_{bb}, -(CH₂)ₙ₁P(O)ₘ₂RₐₐR_{bb}, - (CH₂)ₙ₁NRₐₐR_{bb}, -(CH₂)ₙ₁C(O)NRₐₐR_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl, substituted or unsubstituted heteroaryl, -(CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, - (CH₂)ₙ₁C(O)OR_{cc}, -(CH₂)ₙ₁S(O)ₘ₁R_{cc}, -(CH₂)ₙ₁S(O)ₘ₁NR_{cc}R_{dd}, -(CH₂)ₙ₁NR_{cc}R_{dd}, - (CH₂)ₙ₁C(O)NR_{cc}R_{dd}, -(CH₂)ₙ₁C(O)NR_{cc}S(O)ₘ₁R_{dd}, -(CH₂)ₙ₁NR_{cc}S(O)ₘ₁R_{dd} or - (CH₂)ₙ₁NR_{cc}S(O)ₘ₁R_{dd};
R₄ is selected from hydrogen atom, deuterium atom, alkyl, deuterated alkyl, haloalkyl, alkoxy, hydroxyalkyl, haloalkoxy, halogen, amino, nitro, hydroxyl, cyano, alkenyl, or alkynyl;
Rₐₐ, R_{bb}, R_{cc} and R_{dd} are identical or different, and are each independently selected from hydrogen atom, deuterium atom, alkyl, deuterated alkyl, haloalkyl, alkoxy, hydroxyalkyl, haloalkoxy, halogen, cyano, nitro, hydroxyl, amino, alkenyl, alkynyl, cycloalkyl, heterocyclyl, aryl, or heteroaryl, wherein the alkyl, deuterated alkyl, haloalkyl, alkoxy, hydroxyalkyl, haloalkoxy, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl, or substituted or unsubstituted heteroaryl;
x is an integer of 0, 1, 2 or 3;
y is an integer of 0, 1, 2, 3, 4 or 5;
m₁ is an integer of 0, 1 or 2;
m₂ is an integer of 0, 1 or 2; and
n₁ is an integer of 0, 1, 2, 3, 4 or 5.

10. The compound of general formula (I), the stereoisomer thereof or the pharmaceutically acceptable salt thereof according to claim 9, wherein,
L₁ is selected from bond, alkylene, alkenylene, alkynyl, cycloalkyl, heterocyclyl, - NRₐₐ(CH₂)ₙ₁-, -(CH₂)ₙ₁S(O)ₘ₁- or -(CH₂)ₙ₁S(O)ₘ₁NRₐₐ-;
L2 is selected from bond, oxygen atom, sulfur atom, -CRₐₐR_{bb}-, -(CH₂)ₙ₁C(O)-, -NR₄- or - (CH₂)ₙ₁S(O)ₘ₁-;
L₃ is selected from bond, -NRₐₐ- or -C(O)NRₐₐ-;
ring A is selected from 6-14 membered heteroaryl, wherein the 6-14 membered heteroaryl is selected from 6-14 membered fused heteroaryl; preferably 5,5 fused heteroaryl, 5,6 fused heteroaryl or 6,6 fused heteroaryl;
ring B is selected from 6-14 membered bridged heterocyclyl or 6-14 membered fused heterocyclyl;
ring C is selected from heteroaryl;
R₁ is selected from hydrogen atom, deuterium atom, oxo, alkyl, deuterated alkyl, haloalkyl, alkoxy, haloalkoxy, hydroxyalkyl, halogen, amino, nitro, hydroxyl, cyano, alkenyl, alkynyl, cycloalkyl, heterocyclyl, oxoheterocyclyl, thioxoheterocyclyl, aryl, heteroaryl, -(CH₂)ₙ₁Rₐₐ, - (CH₂)ₙ₁ORₐₐ, -NRₐₐC(O)(CH₂)ₙ₁ORₐₐ, -NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, -(CH₂)ₙ₁C(O)NRₐₐR_{bb}, - (CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, alkyl, haloalkyl, halogen, amino, oxo, nitro, cyano, hydroxyl, alkenyl, alkynyl, alkoxy, haloalkoxy, hydroxyalkyl, cycloalkyl, heterocyclyl, aryl, heteroaryl, -(CH₂)ₙ₁-, - (CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc}, - (CH₂)ₙ₁S(O)ₘ₁R_{cc}, -(CH₂)ₙ₁NR_{cc}R_{dd}, -(CH₂)ₙ₁C(O)NR_{cc}R_{dd}, -(CH₂)ₙ₁NR_{cc}C(O)R_{dd} or - (CH₂)ₙ₁NR_{cc}S(O)ₘ₁R_{dd};
R₂ is selected from hydrogen atom, deuterium atom, alkyl, deuterated alkyl, oxo, haloalkyl, alkoxy, hydroxyalkyl, haloalkoxy, halogen, amino, nitro, hydroxyl, cyano, alkenyl or alkynyl;
R3 is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl, heteroaryl, -(CH₂)ₙ₁Rₐₐ, - (CH₂)ₙ₁ORₐₐ, -NRₐₐC(O)(CH₂)ₙ₁ORₐₐ, -NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁S(O)ₘ₁NRₐₐR_{bb}, -(CH₂)ₙ₁P(O)ₘ₂RₐₐR_{bb}, - (CH₂)ₙ₁NRₐₐR_{bb}, -(CH₂)ₙ₁C(O)NRₐₐR_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl, substituted or unsubstituted heteroaryl, -(CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, - (CH₂)ₙ₁C(O)OR_{cc}, -(CH₂)ₙ₁(O)ₘ₁R_{cc}, -(CH₂)ₙ₁S(O)ₘ₁NR_{cc}R_{dd}, -(CH₂)ₙ₁NR_{cc}R_{dd}, - (CH₂)ₙ₁C(O)NR_{cc}R_{dd}, -(CH₂)ₙ₁C(O)NR_{cc}S(O)ₘ₁R_{dd}, -(CH₂)ₙ₁NR_{cc}S(O)ₘ₁R_{dd} or - (CH₂)ₙ₁NR_{cc}S(O)ₘ₁R_{dd};
R₄ is selected from hydrogen atom, deuterium atom, alkyl, deuterated alkyl, haloalkyl, alkoxy, hydroxyalkyl, haloalkoxy, halogen, amino, nitro, hydroxyl, cyano, alkenyl or alkynyl;
Rₐₐ, R_{bb}, R_{cc} and R_{dd} are identical or different and are each independently selected from hydrogen atom, deuterium atom, alkyl, deuterated alkyl, haloalkyl, alkoxy, hydroxyalkyl, haloalkoxy, halogen, cyano, nitro, hydroxyl, amino, alkenyl, alkynyl, cycloalkyl, heterocyclyl, aryl or heteroaryl, wherein the alkyl, deuterated alkyl, haloalkyl, alkoxy, hydroxyalkyl, haloalkoxy, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl, or substituted or unsubstituted heteroaryl;
x is an integer of 0, 1, 2 or 3;
y is an integer of 0, 1, 2, 3, 4 or 5;
m₁ is an integer of 0, 1 or 2;
m₂ is an integer of 0, 1 or 2; and
n₁ is an integer of 0, 1, 2, 3, 4 or 5.

11. The compound of general formula (I), the stereoisomer thereof or the pharmaceutically acceptable salt thereof according to claim 9, which is a compound of general formula (II) and a compound of general formula (IIA), a stereoisomer thereof or a pharmaceutically acceptable salt thereof: 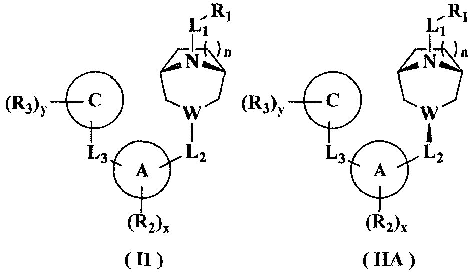 wherein:
W is selected from nitrogen atom or alkylene; and
n is an integer of 0, 1, 2 or 3;
L₁, L₂, L₃, ring A, ring C, R₁-R₃, x and y are as defined in claim 9.

12. The compound of general formula (I), the stereoisomer thereof or the pharmaceutically acceptable salt thereof according to claim 9, which is a compound of general formula (IIIA), a stereoisomer thereof or a pharmaceutically acceptable salt thereof: 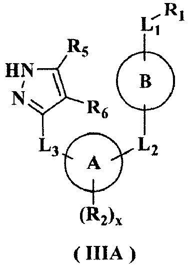 wherein:
R₅ is selected from hydrogen atom, halogen, cyano, alkyl, alkoxy, hydroxyalkyl, haloalkyl, -(CH₂)ₙ₁ORₐₐ, -(CH₂)ₙ₁C(O)NRₐₐR_{bb}, cycloalkyl, heterocyclyl, aryl or heteroaryl, wherein the alkyl, alkoxy, hydroxyalkyl, haloalkyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl, substituted or unsubstituted heteroaryl, -(CH₂)ₙ₁-, -(CH₂)ₙ₁R_{cc}, -(CH₂)ₙ₁OR_{cc}, -(CH₂)ₙ₁SR_{cc}, -(CH₂)ₙC(O)R_{cc}, -(CH₂)ₙ₁C(O)OR_{cc}, -(CH₂)ₙS(O)ₘ₁R_{cc}, - (CH₂)ₙ₁S(O)ₘ₁NR_{cc}R_{dd}, -(CH₂)ₙ₁NR_{cc}R_{dd}, -(CH₂)ₙ₁C(O)NR_{cc}R_{dd}, -(CH₂)ₙ₁C(O)NR_{cc}S(O)ₘ₁R_{dd}, -(CH₂)ₙ₁NR_{cc}S(O)ₘ₁R_{dd} or -(CH₂)ₙ₁NR_{cc}S(O)ₘ₁R_{dd};
R₆ is selected from hydrogen atom, halogen, cyano, alkyl, alkoxy, hydroxyalkyl, haloalkyl, cycloalkyl, heterocyclyl or -(CH₂)ₙ₁C(O)NRₐₐR_{bb};
L₁, L₂, L₃, ring A, ring B, R₁, R₂ and x are as defined in claim 9.

13. The compound of general formula (I), the stereoisomer thereof or the pharmaceutically acceptable salt thereof according to claim 9, which is a compound of general formula (III), a stereoisomer thereof or a pharmaceutically acceptable salt thereof: 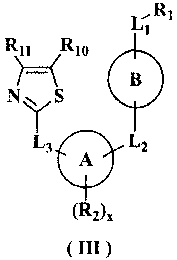 wherein:
R₁₀ and R₁₁, are identical or different and are each independently selected from hydrogen atom, halogen, cyano, alkyl, alkoxy, hydroxyalkyl, haloalkyl, -(CH₂)ₙ₁ORₐₐ, - (CH₂)ₙ₁C(O)NRₐₐR_{bb}, -(CH₂)ₙ₁S(O)ₘ₁NRₐₐR_{bb}-, cycloalkyl, heterocyclyl, aryl or heteroaryl, wherein the alkyl, alkoxy, hydroxyalkyl, haloalkyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl, substituted or unsubstituted heteroaryl, -(CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc}, -(CH₂)ₙ₁S(O)ₘ₁R_{cc}, - (CH₂)ₙ₁S(O)ₘ₁NR_{cc}R_{dd}, -(CH₂)ₙ₁NR_{cc}R_{dd}, -(CH₂)ₙ₁C(O)NR_{cc}R_{dd}, -(CH₂)ₙ₁C(O)NR_{cc}S(O)ₘ₁R_{dd}, -(CH₂)ₙ₁NR_{cc}S(O)ₘ₁R_{dd} or -(CH₂)ₙ₁NR_{cc}S(O)ₘ₁R_{dd};
L₁, L₂, L₃, ring A, ring B, R₁, R₂ and x are as defined in claim 11.

14. The compound of general formula (I), the stereoisomer thereof or the pharmaceutically acceptable salt thereof according to claim 9, which is a compound of general formula (IV), a stereoisomer thereof or a pharmaceutically acceptable salt thereof: 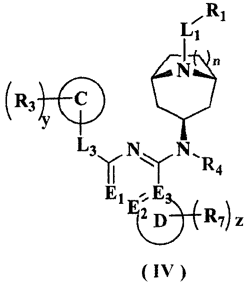 wherein:
ring D is selected from heterocyclyl, aryl or heteroaryl; preferably 5-6 membered heterocyclyl, 6-10 membered aryl or 5-6 membered heteroaryl;
E₁, E₂ and E₃ are identical or different and are each independently selected from nitrogen atom or -CRₐₐ-;
R₄ is selected from hydrogen atom, halogen, cyano, alkyl, alkenyl, alkynyl or alkoxy;
R₇ is selected from hydrogen atom, halogen, cyano, alkyl, alkenyl, alkynyl, alkoxy, oxo or thioxo; and
z is an integer of 0, 1, 2 or 3;
L₁, L₃, ring C, R₁, R₃, Rₐₐ, y and n are as defined in claim 9.

15. The compound of general formula (I), the stereoisomer thereof or the pharmaceutically acceptable salt thereof according to claim 9, which is a compound of general formula (V), a stereoisomer thereof or a pharmaceutically acceptable salt thereof: 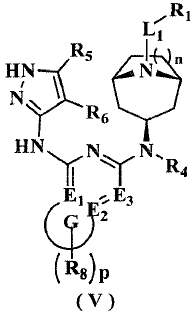 wherein:
ring G is selected from heterocyclyl, aryl or heteroaryl; preferably 5-6 membered heterocyclyl, 6-10 membered aryl or 5-6 membered heteroaryl;
R₈ is selected from hydrogen atom, halogen, cyano, alkyl, alkenyl, alkynyl, alkoxy, oxo or thioxo; and
p is an integer 0, 1, 2 or 3;
R₅ and R₆ are as defined in claim 11;
L₁, E₁, E₂, E₃, R₄ and n are as defined in claim 13.

16. The compound of general formula (I), the stereoisomer thereof or the pharmaceutically acceptable salt thereof according to claim 9, which is a compound of general formula (VI), a stereoisomer thereof or a pharmaceutically acceptable salt thereof: 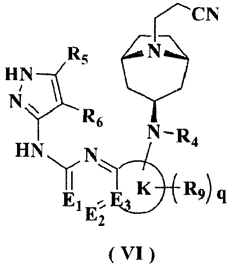 wherein:
ring K is selected from heterocyclyl, aryl or heteroaryl; preferably 5-6 membered heterocyclyl, 6-10 membered aryl, or 5-6 membered heteroaryl;
R₉ is selected from hydrogen atom, halogen, cyano, alkyl, alkenyl, alkynyl, alkoxy, oxo or thioxo; and
q is an integer of 0, 1, 2 or 3;
R₅ and F₁₆ are as defined in claim 11;
E₁, E₂, E₃ and R₄ are as defined in claim 13.

17. The compound of general formula (I), the stereoisomer thereof or the pharmaceutically acceptable salt thereof according to claim 9, which is a compound of general formula (VII), a stereoisomer thereof or a pharmaceutically acceptable salt thereof: 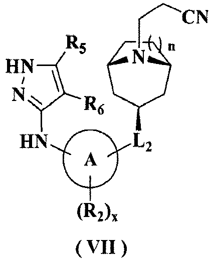 wherein:
n is an integer of 0, 1 or 2;
L₂, ring A, R₂, R₅, R₆ and x are as defined in claim 11.

18. The compound of general formula (III), the stereoisomer thereof or the pharmaceutically acceptable salt thereof according to claim 12, which is a compound of general formula (VIII), a stereoisomer thereof or a pharmaceutically acceptable salt thereof: 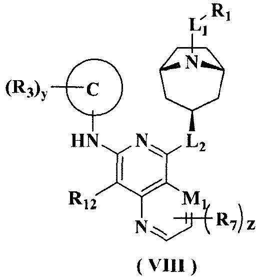 wherein:
M₁ is selected from nitrogen atom or -CRₐₐ-;
R₁₂ is selected from hydrogen atom, cyano, halogen, alkyl or alkoxy; and
z is an integer of 0, 1 or 2;
L₁, L₂, ring C, R₁, R₃, R₇, Rₐₐ and y are as defined in claim 13.

19. The compound of general formula (I), the stereoisomer thereof or the pharmaceutically acceptable salt thereof according to claim 9, which is a compound of general formula (IX), a stereoisomer thereof or a pharmaceutically acceptable salt thereof: 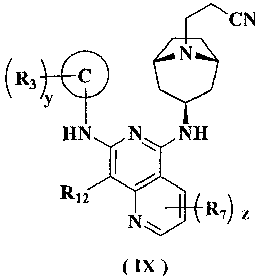 wherein:
ring C, R₃, R₁₂, R₇, y and z are as defined in claim 16.

20. The compound of general formula (I), the stereoisomer thereof or the pharmaceutically acceptable salt thereof according to claim 9, which is a compound of general formula (X), a stereoisomer thereof or a pharmaceutically acceptable salt thereof: 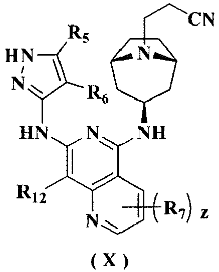 wherein:
R₅-R₆ are as defined in claim 11;
R₁₂, R₇ and z are as defined in claim 16.

21. The compound of general formula (I), the stereoisomer thereof or the pharmaceutically acceptable salt thereof according to claim 9, which is a compound of general formula (XI), a stereoisomer thereof or a pharmaceutically acceptable salt thereof: 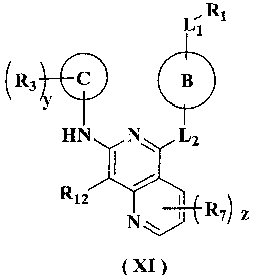 wherein:
L₁, L₂, ring B, ring C, R₃ and R₁₂ are as defined in claim 9;
R₇, y and z are as defined in claim 18.

22. The compound of general formula (I), the stereoisomer thereof or the pharmaceutically acceptable salt thereof according to claim 9, which is a compound of general formula (XII), a stereoisomer thereof or a pharmaceutically acceptable salt thereof: 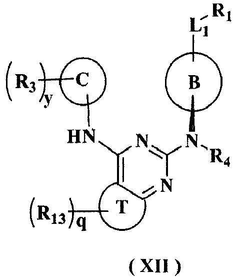 wherein:
L₁ is selected from bond, alkylene, alkenylene, alkynyl, cycloalkyl, heterocyclyl, - (CH₂)ₙ₁C(O)(CH₂)ₘ₁-, -(CH₂)ₙ₁C(O)(CH₂)ₘ₁NRₐₐ(CH₂)ₘ₂-, -(CH₂)ₙ₁S(O)ₘ₁- or - (CH₂)ₙ₁S(O)ₘ₁NRₐₐ-;
ring B is selected from 3-10 membered monocyclic heterocyclyl, 6-14 membered spiro heterocyclyl, 6-14 membered bridged heterocyclyl or 6-14 membered fused heterocyclyl;
ring T is selected from aryl or heteroaryl;
R₁ is selected from hydrogen atom, deuterium atom, oxo, alkyl, deuterated alkyl, haloalkyl, alkoxy, haloalkoxy, hydroxyalkyl, halogen, amino, nitro, hydroxyl, cyano, alkenyl, alkynyl, cycloalkyl, heterocyclyl, oxoheterocyclyl, thioxoheterocyclyl, aryl, heteroaryl, -(CH₂)ₙ₁Rₐₐ, - (CH₂)ₙ₁ORₐₐ, -NRₐₐC(O)(CH₂)ₙ₁ORₐₐ, -NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, -(CH₂)ₙ₁C(O)NRₐₐR_{bb}, - (CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, alkyl, haloalkyl, halogen, amino, oxo, nitro, cyano, hydroxyl, alkenyl, alkynyl, alkoxy, haloalkoxy, hydroxyalkyl, cycloalkyl, heterocyclyl, aryl, heteroaryl, -(CH₂)ₙ₁-, - (CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc}, - (CH₂)ₙ₁S(O)ₘ₁R_{cc}, -(CH₂)ₙ₁NR_{cc}R_{dd}, -(CH₂)ₙ₁C(O)NR_{cc}R_{dd}, -(CH₂)ₙ₁NR_{cc}C(O)R_{dd} or - (CH₂)ₙ₁NR_{cc}S(O)ₘ₁R_{dd};
R₃ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl, heteroaryl, -(CH₂)ₙ₁Rₐₐ, - (CH₂)ₙ₁ORₐₐ, -NRₐₐC(O)(CH₂)ₙ₁ORₐₐ, -NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁S(O)ₘ₁NRₐₐR_{bb}, -(CH₂)ₙ₁P(O)ₘ₂RₐₐR_{bb}, - (CH₂)ₙ)NRₐₐR_{bb}, -(CH₂)ₙ₁C(O)NRₐₐR_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl, substituted or unsubstituted heteroaryl, -(CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, - (CH₂)ₙ₁C(O)OR_{cc}, -(CH₂)ₙ₁S(O)ₘ₁R_{cc}, -(CH₂)ₙ₁S(O)ₘ₁NR_{cc}R_{dd}, -(CH₂)ₙ₁NR_{cc}R_{dd}, - (CH₂)ₙ₁C(O)NR_{cc}R_{dd}, -(CH₂)ₙ₁C(O)NR_{cc}S(O)ₘ₁R_{dd}, -(CH₂)ₙ₁NR_{cc}S(O)ₘ₁R_{dd} or - (CH₂)ₙ₁NR_{cc}S(O)ₘ₁R_{dd};
R₄ is selected from hydrogen atom, deuterium atom, alkyl, deuterated alkyl, haloalkyl, alkoxy, hydroxyalkyl, haloalkoxy, halogen, amino, nitro, hydroxyl, cyano, alkenyl or alkynyl;
R₁₃ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl, heteroaryl, -(CH₂)ₙ₁Rₐₐ, - (CH₂)ₙ₁O(CH₂)ₘ₁Rₐₐ, -(CH₂)ₙ₁ORₐₐ, -(CH₂)ₙ₁NRₐₐ(CH₂)ₘ₁Rₐₐ, -NRₐₐC(O)(CH₂)ₙ₁Oₐₐ, - NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁S(O)ₘ₁NRₐₐR_{bb}, -(CH₂)ₙ₁P(O)ₘ₂RₐₐR_{bb}, -(CH₂)ₙ₁NRₐₐR_{bb}, -(CH₂)ₙ₁S-, - (CH₂)ₙ₁C(O)NRₐₐR_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl, substituted or unsubstituted heteroaryl, -(CH₂)ₙ₁-, -(CH₂)ₙ₁R_{cc}, -(CH₂)ₙ₁OR_{cc}, -(CH₂)ₙ₁SR_{cc}, -(CH₂)ₙC(O)R_{cc}, -(CH₂)ₙ₁C(O)OR_{cc}, - (CH₂)ₙ₁S(O)ₘ₁R_{cc}, -(CH₂)ₙ₁S(O)ₘ₁NR_{cc}R_{dd}, -(CH₂)ₙ₁NR_{cc}R_{dd}, -(CH₂)ₙ₁C(O)NR_{cc}R_{dd}, - (CH₂)ₙ₁C(O)NR_{cc}S(O)ₘ₁R_{dd}, -(CH₂)ₙ₁NR_{cc}S(O)ₘ₁R_{dd} or -(CH₂)ₙ₁NR_{cc}S(O)ₘ₁R_{dd};
Rₐₐ, R_{bb}, R_{cc} and R_{dd} are identical or different and are each independently selected from hydrogen atom, deuterium atom, alkyl, deuterated alkyl, haloalkyl, alkoxy, hydroxyalkyl, haloalkoxy, halogen, cyano, nitro, hydroxyl, amino, alkenyl, alkynyl, cycloalkyl, heterocyclyl, aryl or heteroaryl, wherein the alkyl, deuterated alkyl, haloalkyl, alkoxy, hydroxyalkyl, haloalkoxy, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl, or substituted or unsubstituted heteroaryl;
y is an integer of 0, 1, 2, 3, 4 or 5;
q is an integer of 0, 1, 2, 3, 4 or 5;
m₁ is an integer of 0, 1 or 2;
m₂ is an integer of 0, 1 or 2; and
n₁ is an integer of 0, 1, 2, 3, 4 or 5.

23. The compound of general formula (I), the stereoisomer thereof or the pharmaceutically acceptable salt thereof according to claim 9, which is a compound of general formula (XIII), a stereoisomer thereof or a pharmaceutically acceptable salt thereof: 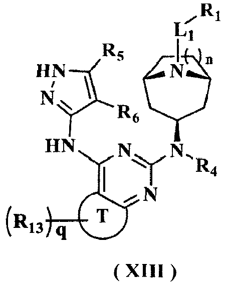 wherein:
R₅ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl or heteroaryl;
R₆ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl or heteroaryl;
n is an integer of 0, 1 or 2;
L₁, ring T, R₁, R₃, R₄, R₁₃ and q are as defined in claim 22.

24. The compound of general formula (I), the stereoisomer thereof or the pharmaceutically acceptable salt thereof according to claim 9, which is a compound of the general formula (XIV), a stereoisomer thereof or a pharmaceutically acceptable salt thereof: 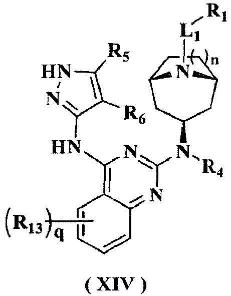 wherein:
L₁, R₁, R₄-R₆, R₁₃, q and n are as defined in claim 23.

25. The compound of general formula (I), the stereoisomer thereof or the pharmaceutically acceptable salt thereof according to claim 9, which is a compound of the general formula (XV), a stereoisomer thereof or a pharmaceutically acceptable salt thereof: 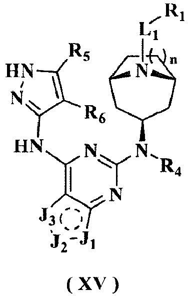 wherein:
L₁ is selected from bond, alkylene, alkenylene, alkynyl, cycloalkyl, heterocyclyl, - (CH₂)ₙ₁C(O)(CH₂)ₘ₁-, -(CH₂)ₙ₁C(O)(CH₂)ₘ₁NRₐₐ(CH₂)ₘ₂-, -(CH₂)ₙ₁S(O)ₘ₁- or - (CH₂)ₙ₁S(O)ₘ₁NRₐₐ-;
J₁, J₂ and J₃ are identical or different and are each independently selected from nitrogen atom, sulfur atom, oxygen atom, NRₐₐ or CR₁₄;
R₁ is selected from hydrogen atom, deuterium atom, oxo, alkyl, deuterated alkyl, haloalkyl, alkoxy, haloalkoxy, hydroxyalkyl, halogen, amino, nitro, hydroxyl, cyano, alkenyl, alkynyl, cycloalkyl, heterocyclyl, oxoheterocyclyl, thioxoheterocyclyl, aryl, heteroaryl, -(CH₂)ₙ₁Rₐₐ, - (CH₂)ₙ₁ORₐₐ, -NRₐₐC(O)(CH₂)ₙ₁ORₐₐ, -NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁NRₐₐR_{bb}, -(CH₂)ₙ₁C(O)NRₐₐR_{bb}, - (CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, alkyl, haloalkyl, halogen, amino, oxo, nitro, cyano, hydroxyl, alkenyl, alkynyl, alkoxy, haloalkoxy, hydroxyalkyl, cycloalkyl, heterocyclyl, aryl, heteroaryl, -(CH₂)ₙ₁-, - (CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙC(O)OR_{cc}, - (CH₂)ₙ₁S(O)ₘ₁R_{cc}, -(CH₂)ₙ₁NR_{cc}R_{dd}, -(CH₂)ₙ₁C(O)NR_{cc}R_{dd}, -(CH₂)ₙ₁NR_{cc}C(O)R_{dd} or - (CH₂)ₙ₁NR_{cc}S(O)ₘ₁R_{dd};
R₄ is selected from hydrogen atom, deuterium atom, alkyl, deuterated alkyl, haloalkyl, alkoxy, hydroxyalkyl, haloalkoxy, halogen, amino, nitro, hydroxyl, cyano, alkenyl or alkynyl;
R₅ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl or heteroaryl;
R₆ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl or heteroaryl;
R₁₄ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl, heteroaryl, -(CH₂)ₙ₁Rₐₐ, - (CH₂)ₙ₁O(CH₂)ₘ₁Rₐₐ, -(CH₂)ₙ₁ORₐₐ, -(CH₂)ₙ₁NRₐₐ(CH₂)ₘ₁Rₐₐ, -NRₐₐC(O)(CH₂)ₙ₁ORₐₐ, - NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁S(O)ₘ₁NRₐₐR_{bb}, -(CH₂)ₙ₁P(O)ₘ₂RₐₐR_{bb}, -(CH₂)ₙ₁NRₐₐR_{bb}, -(CH₂)ₙ₁S-, - (CH₂)ₙ₁C(O)NRₐₐR_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl, substituted or unsubstituted heteroaryl, -(CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc}, - (CH₂)ₙ₁S(O)ₘ₁R_{cc}, -(CH₂)ₙ₁S(O)ₘ₁NR_{cc}R_{dd}, -(CH₂)ₙ₁NR_{cc}R_{dd}, -(CH₂)ₙ₁C(O)NR_{cc}R_{dd}, - (CH₂)ₙ₁C(O)NR_{cc}S(O)ₘ₁R_{dd}, -(CH₂)ₙ₁NR_{cc}S(O)ₘ₁R_{dd} or -(CH₂)ₙ₁NR_{cc}S(O)ₘ₁R_{dd};
Rₐₐ, R_{bb}, R_{cc} and R_{dd} are identical or different and are each independently selected from hydrogen atom, deuterium atom, alkyl, deuterated alkyl, haloalkyl, alkoxy, hydroxyalkyl, haloalkoxy, halogen, cyano, nitro, hydroxyl, amino, alkenyl, alkynyl, cycloalkyl, heterocyclyl, aryl or heteroaryl, wherein the alkyl, deuterated alkyl, haloalkyl, alkoxy, hydroxyalkyl, haloalkoxy, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl, or substituted or unsubstituted heteroaryl;
n is an integer of 0, 1 or 2;
m₁ is an integer of 0, 1 or 2;
m₂ is an integer of 0, 1 or 2; and
n₁ is an integer of 0, 1, 2, 3, 4 or 5.

26. The compound of general formula (I), the stereoisomer thereof or the pharmaceutically acceptable salt thereof according to claim 9, which is a compound of the general formula (XVI), a stereoisomer thereof or a pharmaceutically acceptable salt thereof: 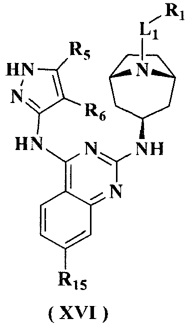 wherein:
R₁₅ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl, heteroaryl, -(CH₂)ₙ₁Rₐₐ, - (CH₂)ₙ₁O(CH₂)ₘ₁Rₐₐ, -(CH₂)ₙ₁ORₐₐ, -(CH₂)ₙ₁NRₐₐ(CH₂)ₘ₁Rₐₐ, -NRₐₐC(O)(CH₂)ₙ₁ORₐₐ, - NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁S(O)ₘ₁NRₐₐR_{bb}, -(CH₂)ₙ₁P(O)ₘ₂RₐₐR_{bb}, -(CH₂)ₙ₁NRₐₐR_{bb}, -(CH₂)ₙ₁S-, - (CH₂)ₙ₁C(O)NRₐₐR_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl, or substituted or unsubstituted heteroaryl, - (CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc}, - (CH₂)ₙ₁S(O)ₘ₁R_{cc}, -(CH₂)ₙ₁S(O)ₘ₁NR_{cc}R_{dd}, -(CH₂)ₙ₁NR_{cc}R_{dd}, -(CH₂)ₙ₁C(O)NR_{cc}R_{dd}, - (CH₂)ₙ₁C(O)NR_{cc}S(O)ₘ₁R_{dd}, -(CH₂)ₙ₁NR_{cc}S(O)ₘ₁R_{dd} or -(CH₂)ₙ₁NR_{cc}S(O)ₘ₁R_{dd};
L₁, R₁, R₅-R₆, Rₐₐ-R_{dd} and n are as defined in claim 22.

27. The compound of general formula (I), the stereoisomer thereof or the pharmaceutically acceptable salt thereof according to claim 9, which is a compound of general formula (XVII), a stereoisomer thereof or a pharmaceutically acceptable salt thereof: 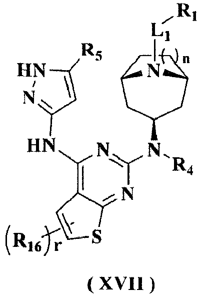 wherein:
R₁₆ is selected from hydrogen atom, deuterium atom, alkyl, deuterated alkyl, haloalkyl, alkoxy, haloalkoxy, halogen, amino, nitro, hydroxyl, cyano, alkenyl, alkynyl, cycloalkyl, hydroxyalkyl, heterocyclyl, oxoheterocyclyl, thioxoheterocyclyl, aryl, heteroaryl, -(CH₂)ₙ₁Rₐₐ, - (CH₂)ₙ₁O(CH₂)ₘ₁Rₐₐ, -(CH₂)ₙ₁ORₐₐ, -(CH₂)ₙ₁NRₐₐ(CH₂)ₘ₁Rₐₐ, -NRₐₐC(O)(CH₂)ₙ₁ORₐₐ, - NRₐₐC(=S)(CH₂)ₙ₁OR_{bb}, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘ₁Rₐₐ, -(CH₂)ₙ₁S(O)ₘ₁NRₐₐR_{bb}, -(CH₂)ₙ₁P(O)ₘ₂RₐₐR_{bb}, -(CH₂)ₙ₁NRₐₐR_{bb}, -(CH₂)ₙ₁S-, - (CH₂)ₙ₁C(O)NRₐₐR_{bb}, -(CH₂)ₙ₁NRₐₐC(O)R_{bb} or -(CH₂)ₙ₁NRₐₐS(O)ₘ₁R_{bb}, wherein the alkyl, haloalkyl, alkoxy, haloalkoxy, hydroxyalkyl, amino, alkenyl, alkynyl, cycloalkyl, heterocyclyl, aryl and heteroaryl are optionally further substituted with one or more substituents selected from hydrogen atom, deuterium atom, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl, substituted or unsubstituted heteroaryl, -(CH₂)ₙ₁-, -(CH₂)ₙ₁R_{cc}, -(CH₂)ₙ₁OR_{cc}, -(CH₂)ₙ₁SR_{cc}, -(CH₂)ₙ₁C(O)R_{cc}, -(CH₂)ₙ₁C(O)OR_{cc}, - (CH₂)ₙ₁S(O)ₘ₁R_{cc}, -(CH₂)ₙ₁S(O)ₘ₁NR_{cc}R_{dd}, -(CH₂)ₙ₁NR_{cc}R_{dd}, -(CH₂)ₙ₁C(O)NR_{cc}R_{dd}, - (CH₂)ₙ₁C(O)NR_{cc}S(O)ₘ₁R_{dd}, -(CH₂)ₙ₁NR_{cc}S(O)ₘ₁R_{dd} or -(CH₂)ₙ₁NR_{cc}S(O)ₘ₁R_{dd};
r is an integer of 0, 1 or 2; and
L₁, R₁, R₄-R₆, Rₐₐ-R_{dd}, n, n₁, m₁ and m₂ are as defined in claim 25.

28. The compound of general formula (I), the stereoisomer thereof or the pharmaceutically acceptable salt thereof according to any one of claims 9 to 27, wherein:
ring A is selected from the following group: 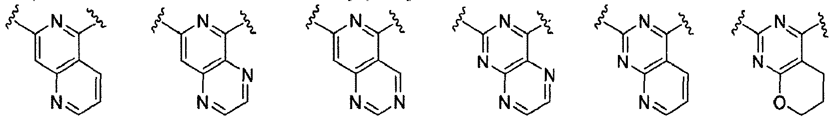 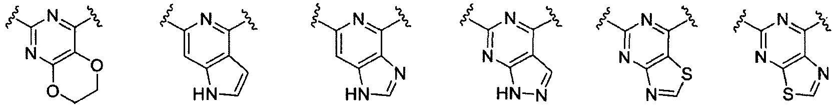 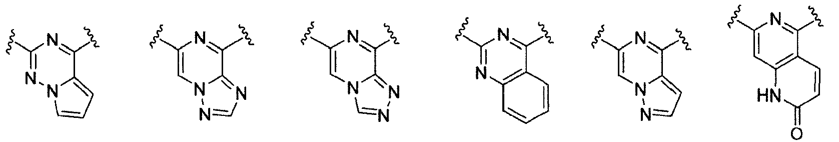 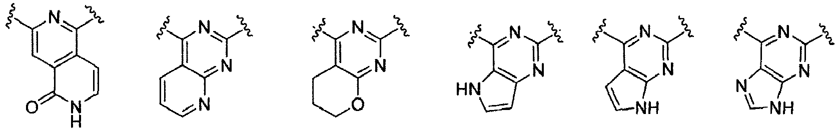 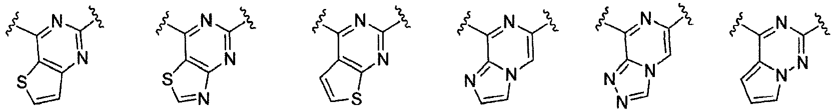 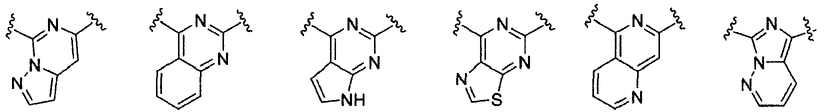 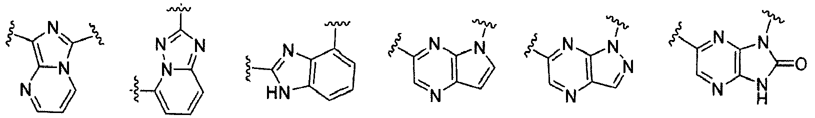 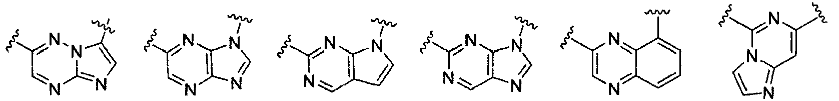 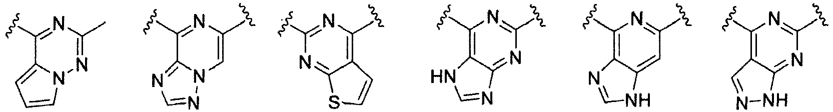 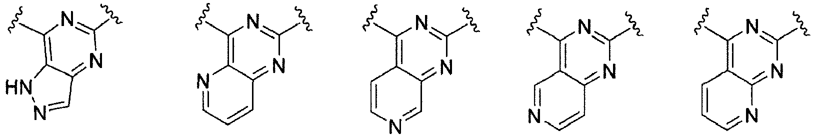 and 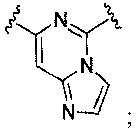
ring B is selected from the following group: 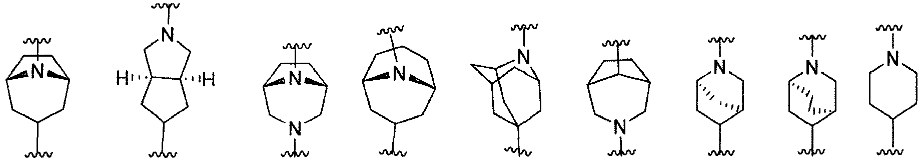 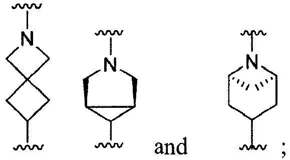
ring C is selected from the following group: 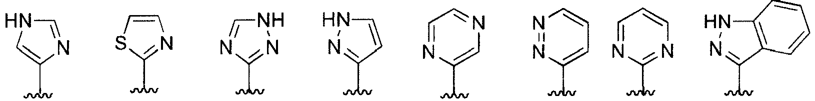 and

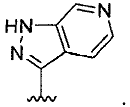

29. The compound of general formula (I), the stereoisomer thereof or the pharmaceutically acceptable salt thereof according to any one of claims 10-21 and 28, wherein,
L₁ is C₃₋₈ cycloalkyl, C₃₋₈ heterocycloalkyl, -(CH₂)ₙ₁-, -NRₐₐ(CH₂)ₙ₁-, -(CH₂)ₙ₁S(O)ₘ₁- or - (CH₂)ₙ₁S(O)ₘ₁NRₐₐ-;
L2 is selected from bond, oxygen atom, -CRₐₐR_{bb}-, -(CH₂)ₙ₁C(O)-, -NR₄- or -(CH₂)ₙ₁S(O)ₘ₁-;
L₃ is selected from bond, -NRₐₐ- or -C(O)NRₐₐ-;
R₁ is selected from cyano, -(CH₂)ₙ₁Rₐₐ, C₃₋₈cycloalkyl or 3-10 membered heterocyclyl, wherein the cycloalkyl and heterocyclyl are optionally further substituted with one or more substituents selected from hydrogen atom or cyano;
R₂ is selected from hydrogen atom, halogen, cyano, hydroxyl, oxo, C₁₋₆ alkyl, C₁₋₆ alkoxy or 3-10 membered heterocyclyl; preferably hydrogen atom, halogen, cyano, hydroxyl, oxo, C₁₋₃ alkoxy, C₁₋₃ alkyl or 3-8 membered heterocyclyl; more preferably hydrogen atom, fluorine, cyano, hydroxyl, oxo, methoxy, methyl or morpholinyl;
R₃ is selected from hydrogen atom, cyano, halogen, C₁₋₆ alkyl, C₁₋₆ haloalkyl, C₁₋₆ hydroxyalkyl, C₃₋₈ cycloalkyl, 3-10 membered heterocyclyl, -(CH₂)ₙ₁ORₐₐ, -C(O)NRₐₐR_{bb} or - (CH₂)ₙ₁S(O)ₘ₁Rₐₐ, wherein the cycloalkyl and heterocyclyl are optionally further substituted with one or more substituents selected from hydrogen atom, C₁₋₆ alkyl or hydroxyl;
R₄ is selected from hydrogen atom or methyl;
Rₐₐ, R_{bb}, R_{cc} and R_{dd} are identical or different and are each independently selected from hydrogen atom, C₁₋₆ alkyl, cyano, hydroxyl, amino or 3-10 membered heterocyclyl; wherein the C₁₋₆ alkyl, amino and 3-10 membered heterocyclyl are optionally further substituted with hydrogen atom, cyano, hydroxyl or 5-10 membered heteroaryl.

30. The compound of general formula (I), the stereoisomer thereof or the pharmaceutically acceptable salt thereof according to any one of claims 9 to 28, wherein,
L₁ is C₃₋₈ cycloalkyl, 3-10 membered heterocyclyl, -(CH₂)ₙ₁-, -(CH₂)ₙ₁C(O)(CH₂)ₘ₁-, - (CH₂)ₙ₁C(O)(CH₂)ₘ₁NRₐₐ(CH₂)ₘ₂-, -NRₐₐ(CH₂)ₙ₁-, -(CH₂)ₙ₁S(O)ₘ₁- or -(CH₂)ₙ₁S(O)ₘ₁NRₐₐ-;
L2 is selected from bond, oxygen atom, -CRₐₐR_{bb}-, -(CH₂)ₙ₁C(O)-, -NR₄- or -(CH₂)ₙ₁S(O)ₘ₁-;
L₃ is selected from bond, -NRₐₐ- or -C(O)NRₐₐ-;
R₁ is selected from cyano, -(CH₂)ₙ₁Rₐₐ, -(CH₂)ₙ₁C(O)Rₐₐ, C₃₋₈ cycloalkyl, 3-10 membered heterocyclyl, C₆₋₁₀ aryl or 5-10 membered heteroaryl, wherein the C₃₋₈ cycloalkyl, 3-10 membered heterocyclyl, C₆₋₁₀ aryl and 5-14 membered heteroaryl are optionally further substituted with one or more substituents selected from hydrogen atom, cyano, C₁₋₆ alkyl, or C₁₋₆ alkoxy;
R₂ is selected from hydrogen atom, halogen, cyano, hydroxyl, oxo, C₁₋₆ alkyl, C₁₋₆ alkoxy, C₃₋₈ cycloalkyl, 3-10 membered heterocyclyl, C₆₋₁₂ aryl, 5-10 membered heteroaryl, -(CH₂)ₙ₁Rₐₐ, -O(CH₂)ₙ₁Rₐₐ, -S(CH₂)ₙ₁Rₐₐ or -NRₐₐ(CH₂)ₙ₁R_{bb}, wherein the C₃₋₈ cycloalkyl, 3-10 membered heterocyclyl, C₆₋₁₀ aryl and 5-10 membered heteroaryl are optionally further substituted with one or more substituents selected from hydrogen atom, cyano, C₁₋₆ alkyl, C₁₋₆ alkoxy, C₃₋₈ cycloalkyl or -NR_{cc}R_{dd};
R₃ is selected from hydrogen atom, cyano, halogen, C₁₋₆ alkyl, C₁₋₆ haloalkyl, C₁₋₆ hydroxyalkyl, C₃₋₈ cycloalkyl, 3-10 membered heterocyclyl, -(CH₂)ₙ₁ORₐₐ, -C(O)NRₐₐR_{bb} or - (CH₂)ₙ₁S(O)ₘ₁Rₐₐ, wherein the cycloalkyl and heterocyclyl are optionally further substituted with one or more substituents selected from hydrogen atom, C₁₋₆ alkyl or hydroxyl;
R₄ is selected from hydrogen atom or methyl;
Rₐₐ, R_{bb}, Rcc and Rdd are identical or different and are each independently selected from hydrogen atom, C₁₋₆ alkyl, C₁₋₆ haloalkyl, cyano, hydroxyl, amino, C₃₋₈ cycloalkyl, 3-10 membered heterocyclyl, C₆₋₁₀ aryl or 5-10 membered heteroaryl; wherein the C₁₋₆ alkyl, C₁₋₆ haloalkyl, amino, C₃₋₈ cycloalkyl, 3-10 membered heterocyclyl, C₆₋₁₂ aryl and 5-10 membered heteroaryl are optionally further substituted with one or more substituents selected from hydrogen atom, cyano, hydroxyl, amino, aminoalkyl, C₁₋₆ alkyl, C₁₋₆ alkoxy, C₃₋₈ cycloalkyl, 3-10 membered heterocyclyl, C₆₋₁₀ aryl or 5-10 membered heteroaryl.

31. The compound of general formula (I), the stereoisomer thereof or the pharmaceutically acceptable salt thereof according to any one of claims 1-30, wherein, the compound of the general formula (I) is selected from the following compounds:

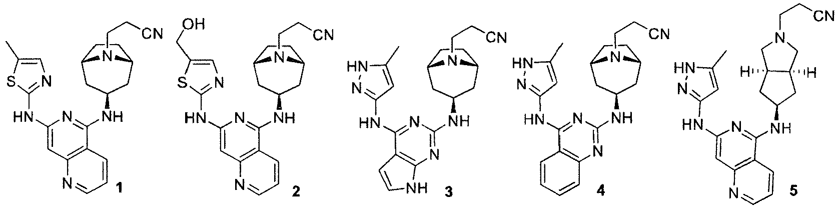

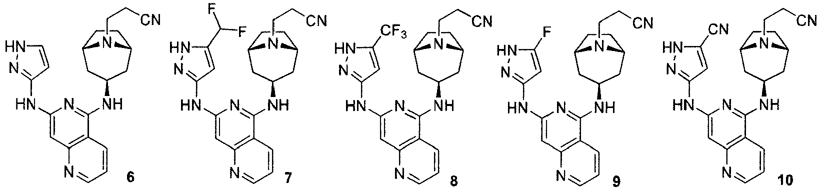

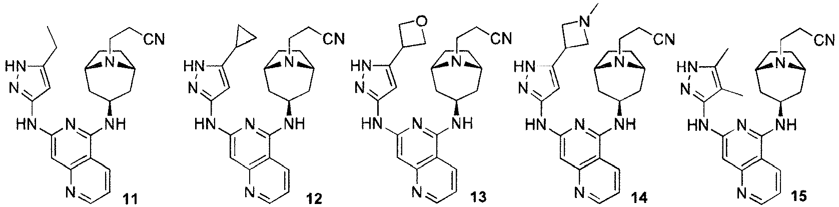

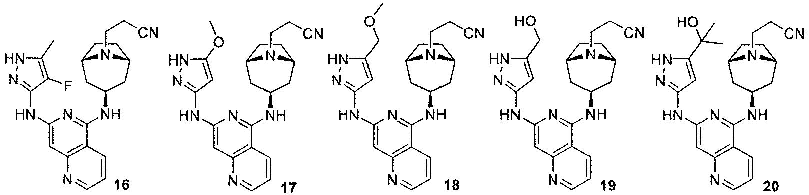

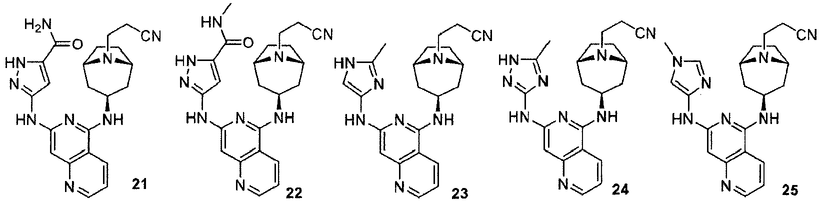

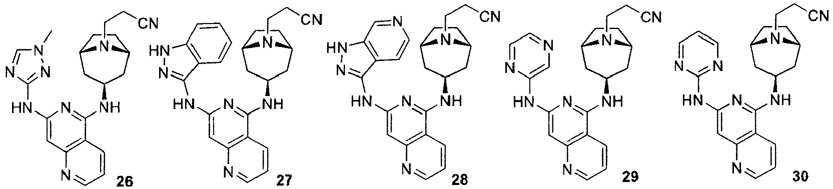

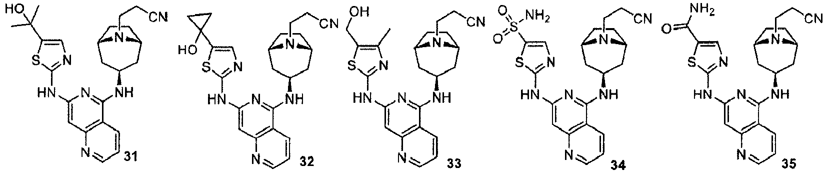

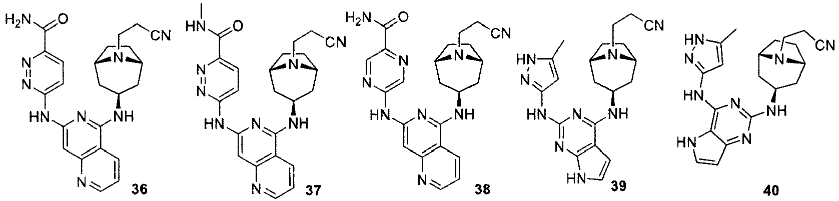

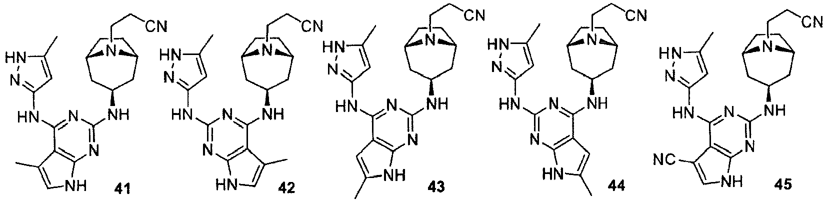

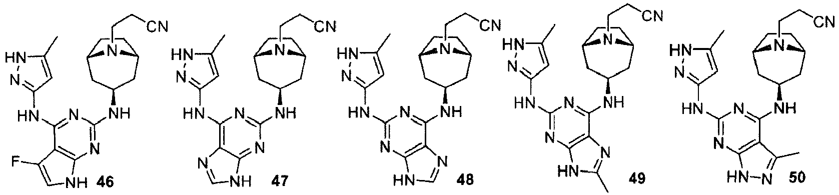

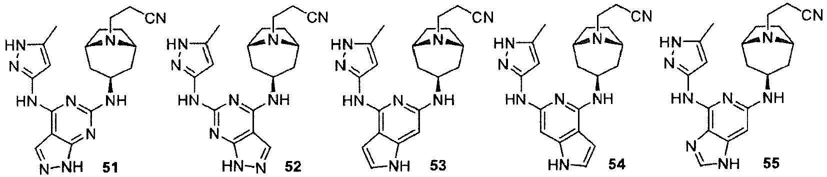

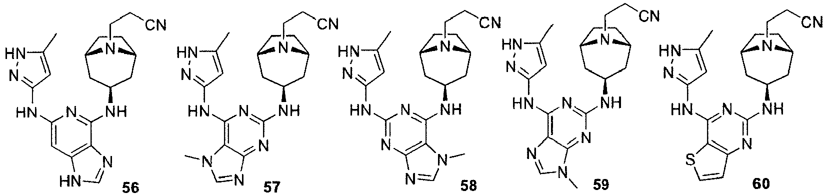

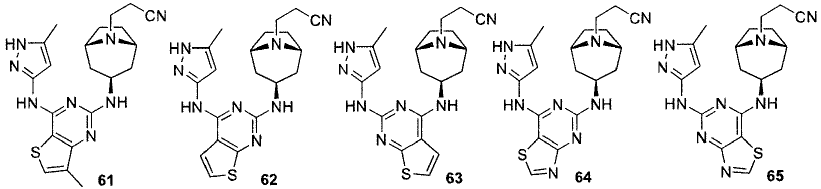

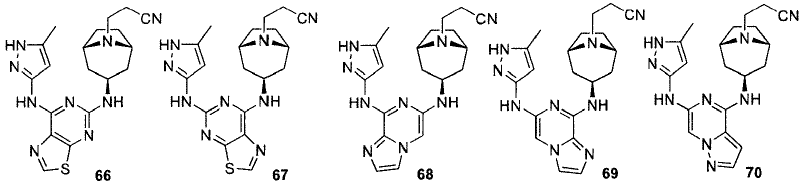

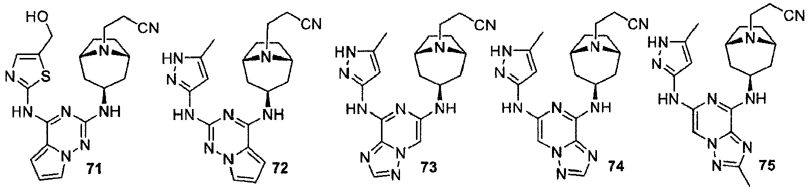

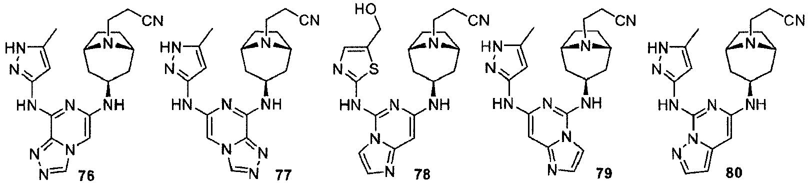

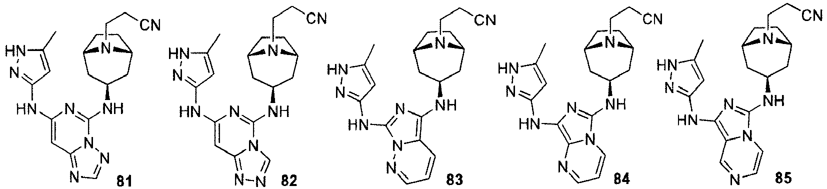

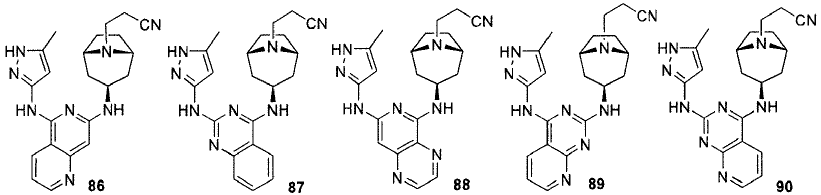

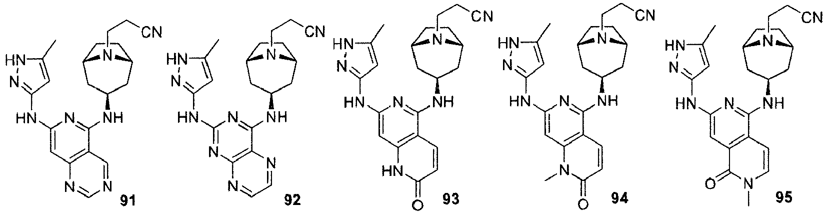

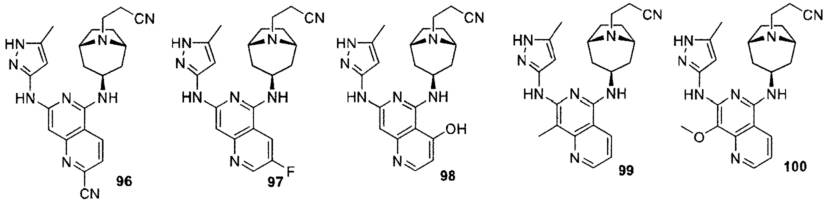

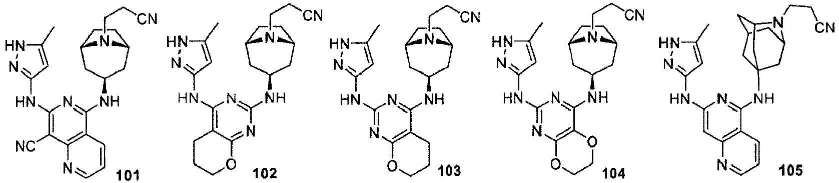

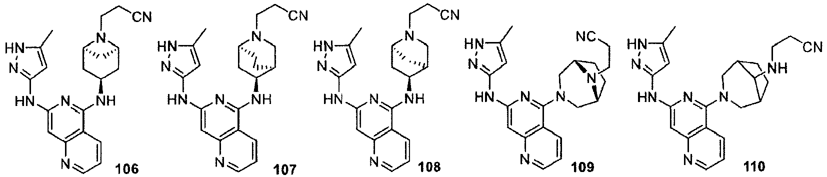

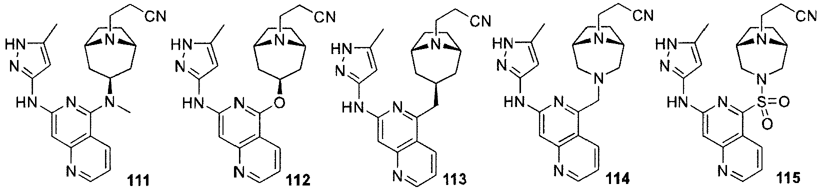

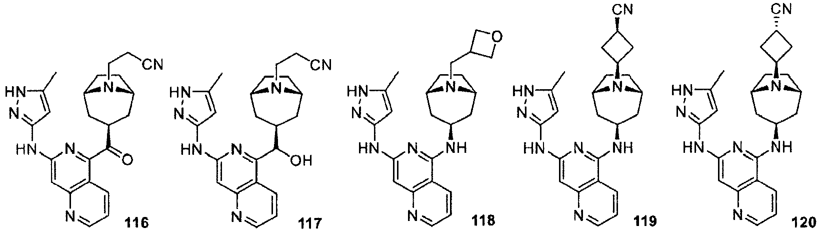

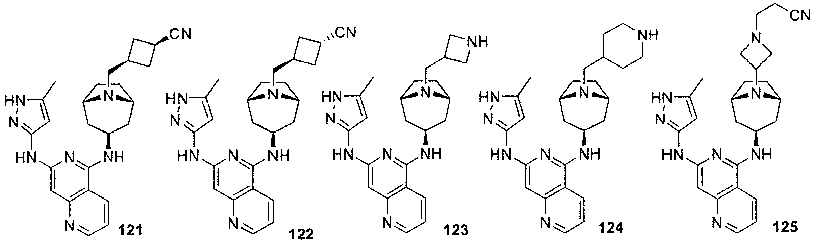

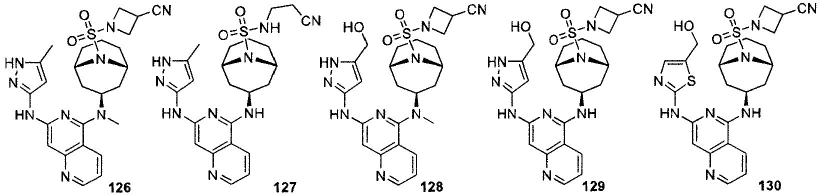

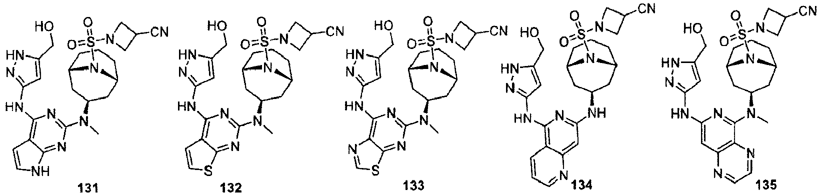

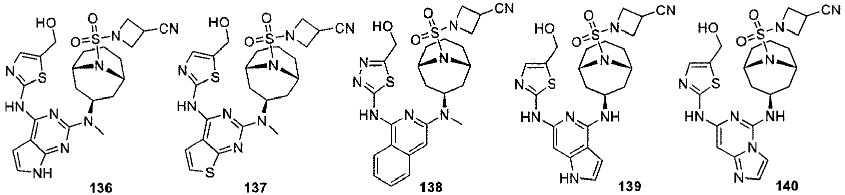

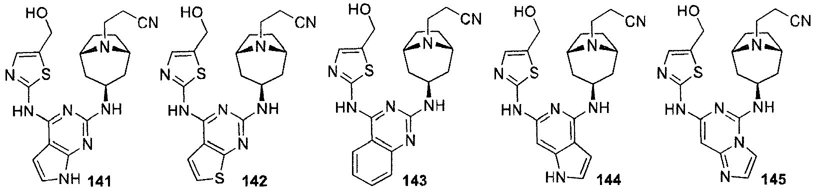

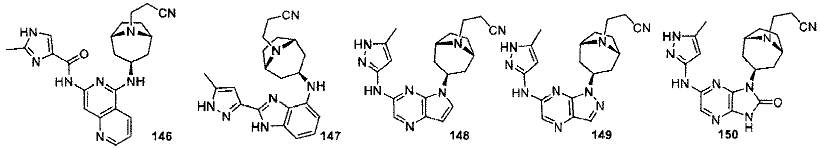

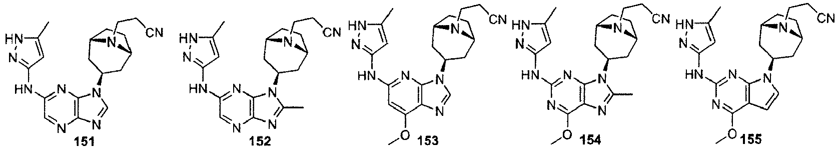

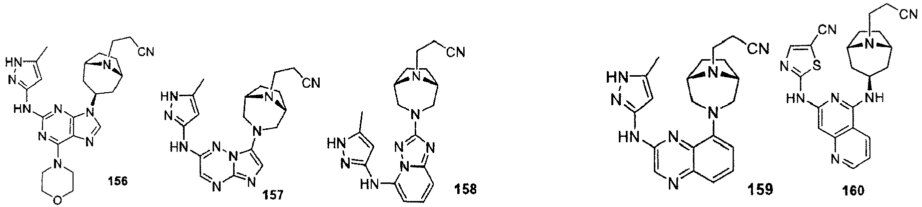

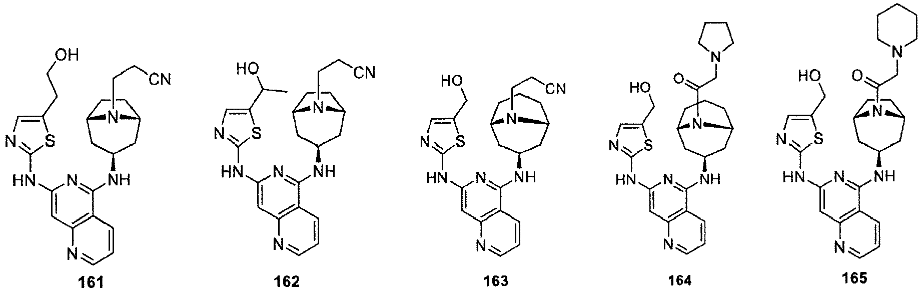

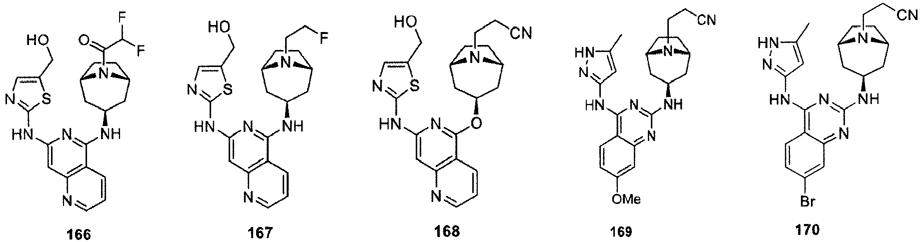

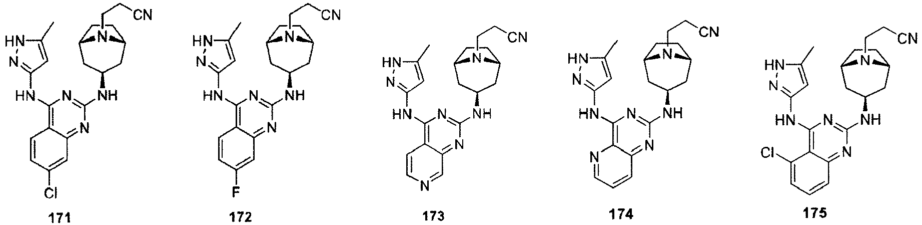

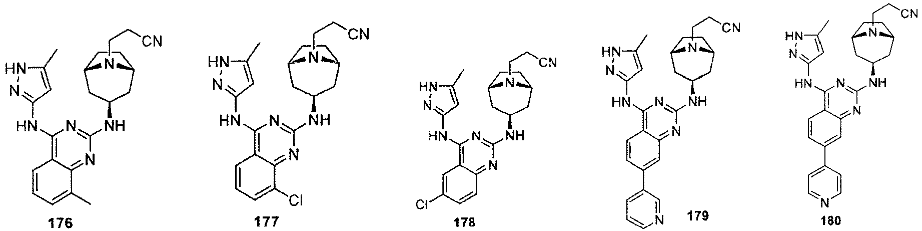

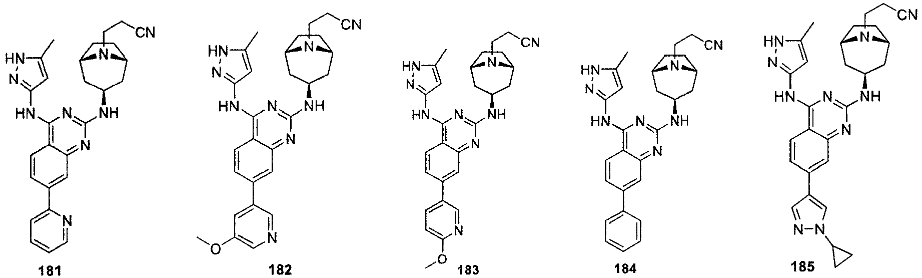

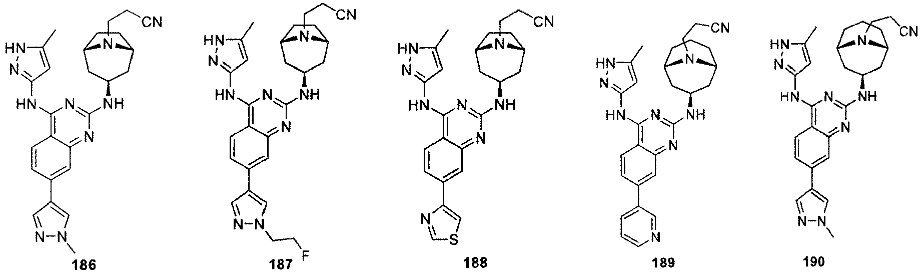

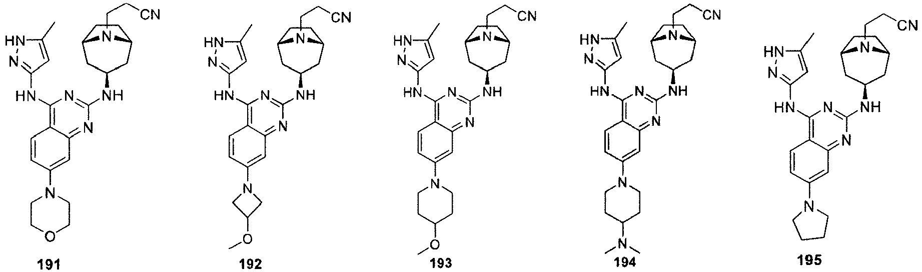

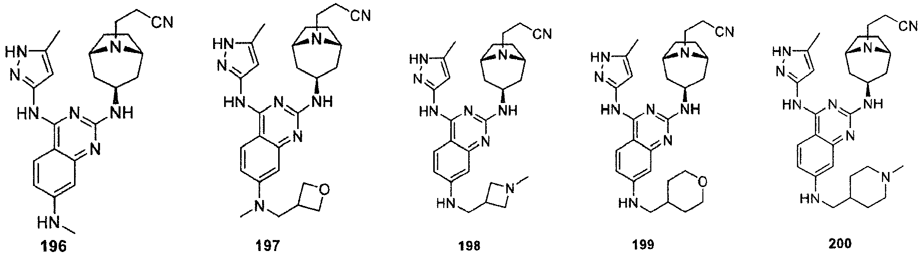

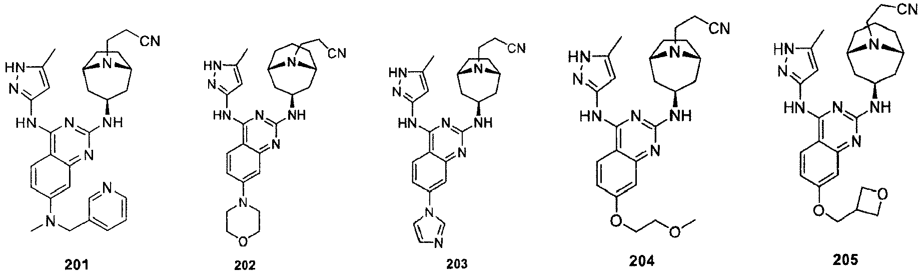

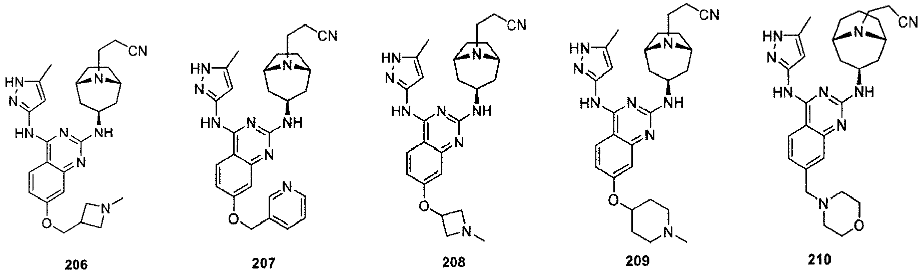

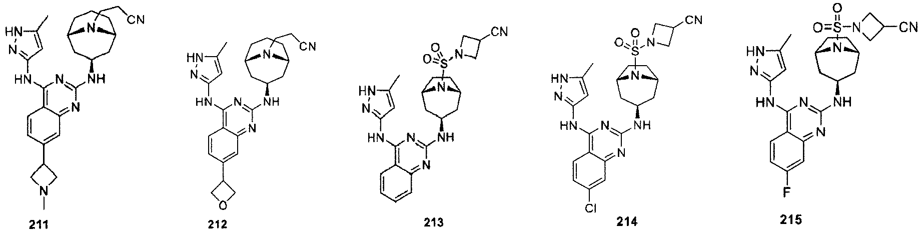

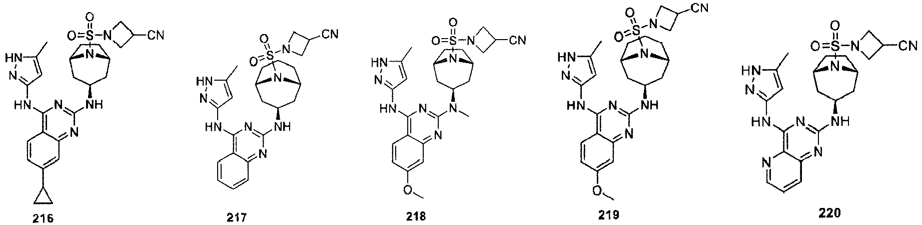

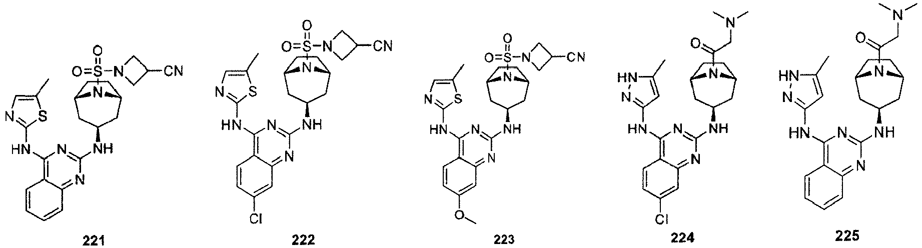

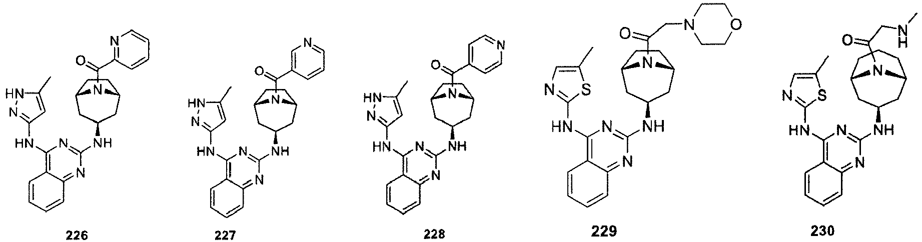

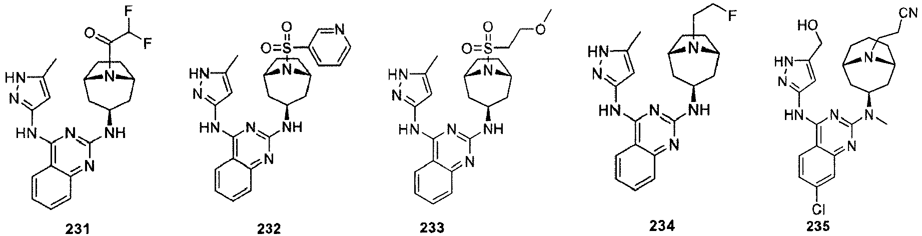

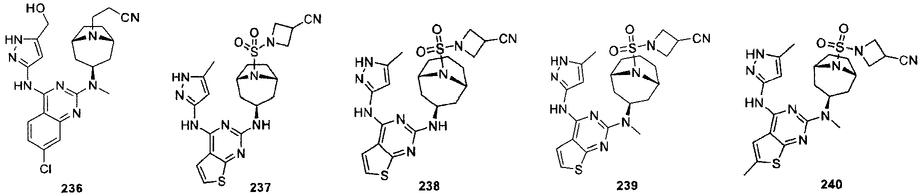

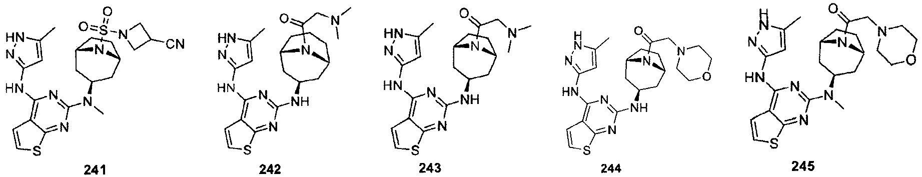

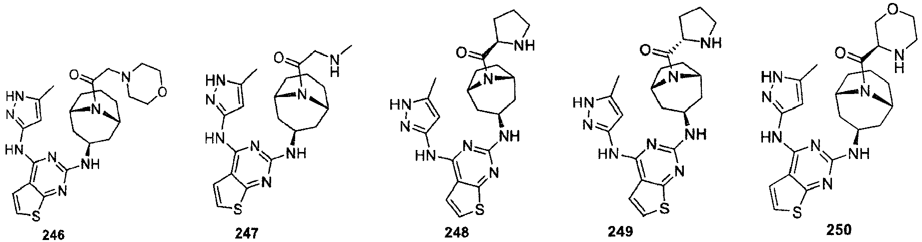

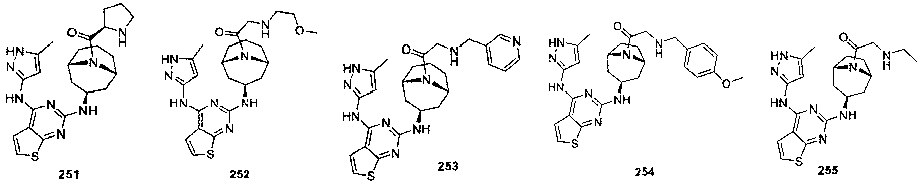

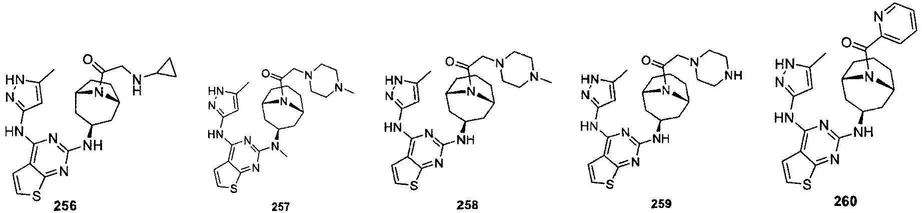

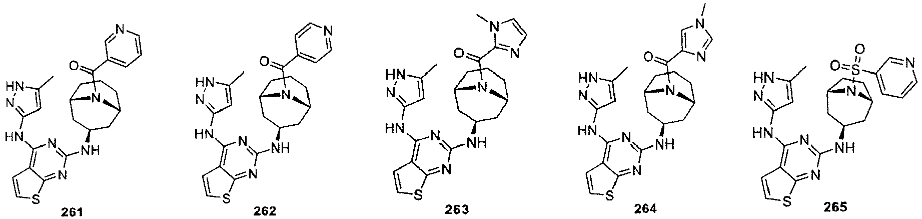

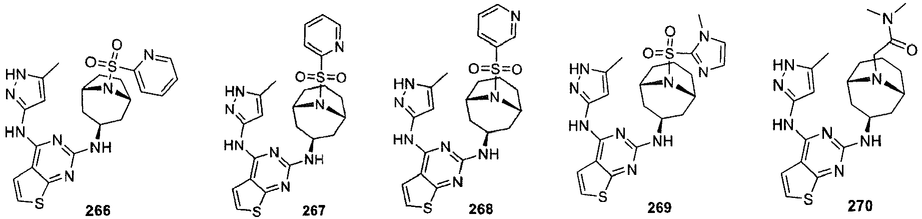

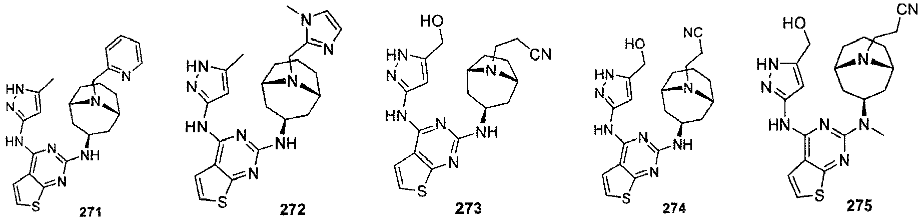

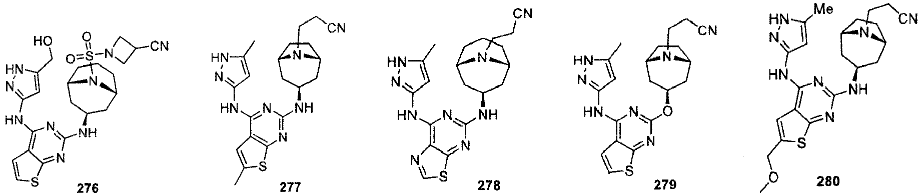

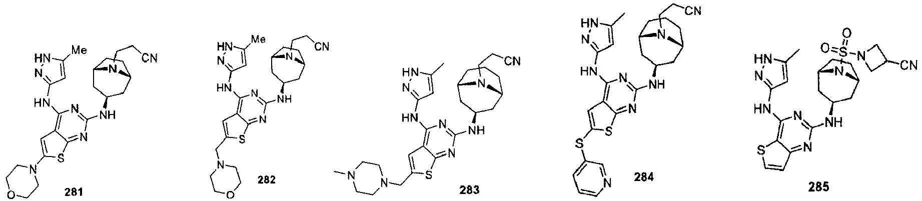

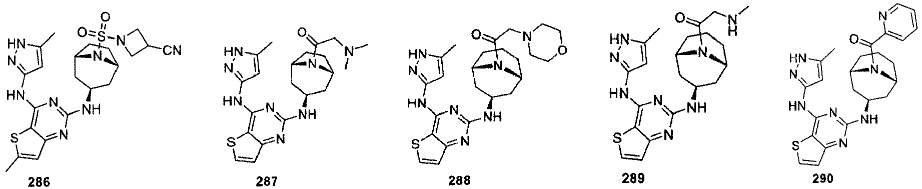

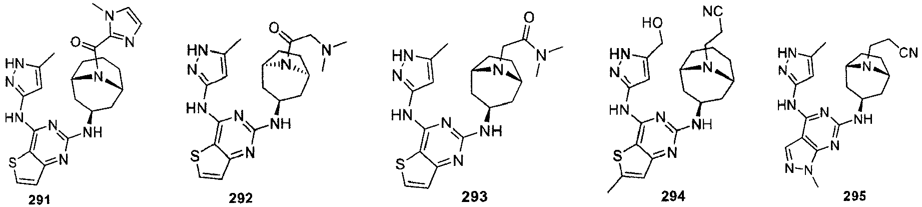

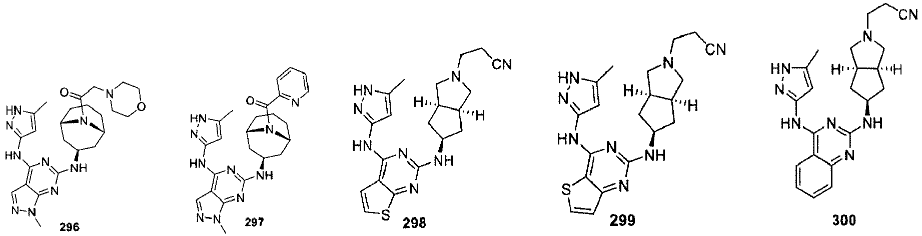

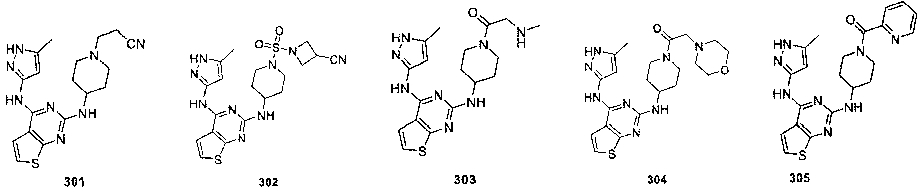

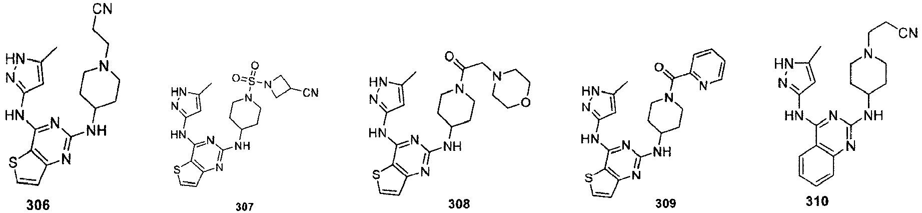

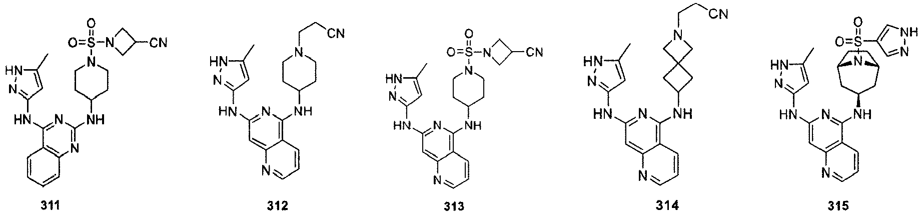

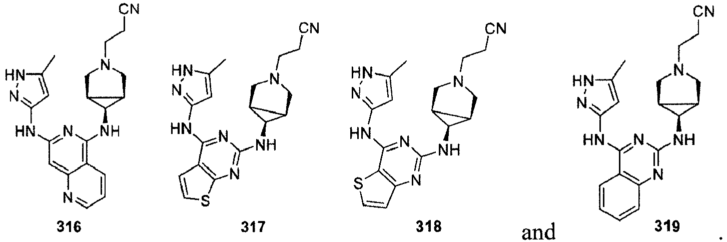

32. A pharmaceutical composition, comprising a therapeutically effective dose of the compound of general formula (I), the stereoisomer thereof or the pharmaceutically acceptable salt thereof as defined in any one of claims 8-31 and one or more pharmaceutically acceptable carriers, diluents or excipients.

33. A use of the compound of general formula (I), the stereoisomer thereof or the pharmaceutically acceptable salt thereof as defined in any one of claims 8-31, or the pharmaceutical composition as defined in claim 32 in the manufacture of a JAK kinase inhibitor medicament.

34. A use of the compound of general formula (I), the stereoisomer thereof or the pharmaceutically acceptable salt thereof as defined in any one of claims 8-31, or the pharmaceutical composition as defined in claim 32 in the manufacture of a medicament for the treatment of an inflammatory disease and a neoplastic disease, wherein the inflammatory disease is selected from rheumatoid arthritis, dermatitis, psoriasis, inflammatory bowel disease; the neoplastic disease is selected from myelofibrosis, polycythemia vera and essential thrombocythemia, acute myelomonocytic leukemia, acute lymphocytic leukemia, ductal carcinoma of the breast and non-small cell lung cancer, wherein gastrointestinal inflammatory disease is chronic inflammatory disease of the intestine, further preferably ulcerative colitis and Crohn's disease.

35. A pharmaceutical composition, comprising the compound of general formula (I), the stereoisomer thereof or the pharmaceutically acceptable salt thereof as defined in any one of claims 1-31 and one or more pharmaceutically acceptable carriers, diluents or excipients, the compound of general formula (I), the stereoisomer thereof or the pharmaceutically acceptable salt thereof may be present in a therapeutically effective amount.

36. A use of the compound of general formula (I), the stereoisomer thereof or the pharmaceutically acceptable salt thereof as defined in any one of claims 1-31, or the pharmaceutical composition as defined in claim 35 in the manufacture of a JAK kinase inhibitor.

37. A use of the compound of general formula (I), the stereoisomer thereof or the pharmaceutically acceptable salt thereof as defined in any one of claims 1-31, or the pharmaceutical composition as defined in claim 35 in the manufacture of a medicament for the prevention and/or treatment of a JAK kinase related disease;
the JAK kinase related disease is preferably an inflammatory disease and/or a neoplastic disease;
the inflammatory disease is preferably selected from rheumatoid arthritis, dermatitis, psoriasis, or inflammatory bowel disease; wherein gastrointestinal inflammatory disease is preferably chronic inflammatory disease of the intestine, further preferably ulcerative colitis and Crohn's disease;
the neoplastic disease is preferably selected from myelofibrosis, polycythemia vera and essential thrombocythemia, acute myelomonocytic leukemia, acute lymphocytic leukemia, ductal carcinoma of the breast or non-small cell lung cancer.

38. A use of the compound of general formula (I), the stereoisomer thereof or the pharmaceutically acceptable salt thereof as defined in any one of claims 1-31, or the pharmaceutical composition as defined in claim 35 in the manufacture of a medicament;
the medicament is preferably a medicament for the treatment of an inflammatory disease and/or a neoplastic disease;
the inflammatory disease is preferably selected from rheumatoid arthritis, dermatitis, psoriasis, inflammatory bowel disease; wherein gastrointestinal inflammatory disease is preferably chronic inflammatory disease of the intestine, further preferably ulcerative colitis or Crohn's disease;
the neoplastic disease is preferably selected from myelofibrosis, polycythemia vera and essential thrombocythemia, acute myelomonocytic leukemia, acute lymphocytic leukemia, ductal carcinoma of the breast or non-small cell lung cancer.

39. A method for the treatment of an inflammatory disease or a neoplastic disease, comprising administering to a patient a therapeutically effective dose of the compound of general formula (I), the stereoisomer thereof or the pharmaceutically acceptable salt thereof as defined in any one of claims 1-31, or the pharmaceutical composition as defined in claim 35,
the inflammatory disease is preferably rheumatoid arthritis, dermatitis, psoriasis, inflammatory bowel disease; wherein gastrointestinal inflammatory disease is preferably chronic inflammatory disease of the intestine, further preferably ulcerative colitis and Crohn's disease;
the neoplastic disease is preferably myelofibrosis, polycythemia vera and essential thrombocythemia, acute myelomonocytic leukemia, acute lymphocytic leukemia, ductal carcinoma of the breast and non-small cell lung cancer.

40. A method for the prevention and/or treatment of a condition mediated by JAK kinase, comprising administering to a patient a therapeutically effective dose of the compound of general formula (I), the stereoisomer thereof or the pharmaceutically acceptable salt thereof as defined in any one of claims 1-31, or the pharmaceutical composition as defined in claim 35,
the condition mediated by JAK kinase is preferably an inflammatory disease or a neoplastic disease;
the inflammatory disease is preferably selected from rheumatoid arthritis, dermatitis, psoriasis, or inflammatory bowel disease; wherein gastrointestinal inflammatory disease is preferably chronic inflammatory disease of the intestine, further preferably ulcerative colitis and Crohn's disease;
the neoplastic disease is preferably selected from myelofibrosis, polycythemia vera and essential thrombocythemia, acute myelomonocytic leukemia, acute lymphocytic leukemia, ductal carcinoma of the breast or non-small cell lung cancer.
